# EUROPEAN PATENT APPLICATION

(11) **EP 4 834 897 A1**
(43) Date of publication of application: **04.11.2026**
(21) Application number: 24911291.3
(22) Date of filing: 26.12.2024
(51) Int. Cl.: C07D 401/14, C07D 401/04, C07D 413/14, C07D 491/107, C07D 487/10, C07D 498/10, C07D 419/14, C07D 417/14, C07D 409/14, C07D 405/14, A61K 31/45, A61K 31/454, A61K 31/4545, A61K 31/497, A61K 31/538, A61P 35/00, A61P 37/00, A61P 25/00, A61P 29/00

(54) **COMPOUNDS WITH TWO AZAHETEROCYCLES SUBSTITUTED ISOINDOLINONE SKELETON AND USES THEREOF**

(30) Priority: 26.12.2023 CN 202311811318
(71) Applicant: Gluetacs Therapeutics (Shanghai) Co., Ltd., Shanghai 201306 (CN)
(72) Inventor: YANG, Xiaobao, Shanghai 201306 (CN); REN, Chaowei, Shanghai 201306 (CN); SUN, Renhong, Shanghai 201306 (CN); ZHAO, Baoyin, Shanghai 201306 (CN); FU, Xuhong, Shanghai 201306 (CN); LI, Yan, Shanghai 201306 (CN); ZHOU, Yuedong, Shanghai 201306 (CN); YANG, Sen, Shanghai 201306 (CN); DENG, Meigui, Shanghai 201306 (CN); JIANG, Suping, Shanghai 201306 (CN); REN, Yinbo, Shanghai 201306 (CN)
(74) Representative: August Debouzy
(86) International application number: PCT/CN2024/142598
(87) International publication number: WO 2025/140369

(57) **Abstract**

The present disclosure provides a compound of Formula (I) or salts, enantiomers, stereoisomers, solvates, or polymorphs thereof and uses thereof. The present disclosure also provides pharmaceutical compositions comprising, as an active ingredient, the compound of Formula (I) or salts, enantiomers, stereoisomers, solvates, or polymorphs thereof and uses thereof. The series of compounds designed and synthesized in the present disclosure can effectively prevent or treat diseases or disorders associated with the cereblon protein, including tumors or cancers.

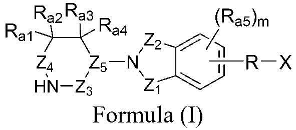

## Description

### Technical Field

The present disclosure relates to a compound of Formula (I) or salts, enantiomers, diastereoisomers, solvates, or polymorphs thereof and uses thereof, especially their use in the prevention or treatment of diseases or disorders associated with cereblon protein, including tumors or cancers.

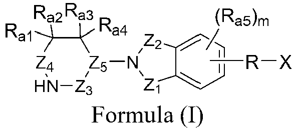

### Background

Immunomodulatory drugs (IMiDs) of the phthalimide class, such as thalidomide and lenalidomide, have shown remarkable efficacy in treating multiple myeloma and autoimmune diseases. However, it was not until 2010 that their direct binding target was identified as the E3 ubiquitin ligase cereblon (CRBN). Subsequent studies confirmed that upon binding to CRBN, these drugs act as molecular glues, recruiting substrate proteins (e.g., the transcription factors IKZF1/3) and inducing protein-protein interactions between CRBN and the substrates. This leads to the ubiquitination of the substrate proteins, which are then recognized and degraded by the proteasome, thereby eliciting pharmacological effects such as antitumor and immunomodulatory activities. Molecular glue degraders directly target and degrade specific proteins, offering potential advantages such as targeting "undruggable" proteins. Moreover, molecular glues typically exhibit low molecular weight and favorable drug-like properties, making their development highly challenging. Based on the CRBN E3 ubiquitin ligase and the molecular glue degradation mechanism, a series of compounds have been developed, including the approved pomalidomide and several candidates currently in clinical trials, such as Bristol Myers Squibb's CC-122, CC-220, CC-90009, CC-99282, CC-92480, and BMS-986470; Novartis's DKY709; C4 Therapeutics's CFT7455; and Monte Rosa's MRT-6160. The substrates degraded by these molecular glues have also expanded from the initially identified transcription factors IKZF1/3 to casein kinase 1α (CK1α), zinc finger protein 91 (ZFP91), WIZ, transcription factor IKZF2, translation factor GSPT1, and immune disease target Vav1, among others. Degradation of these protein substrates enables molecular glues to exert immunomodulatory, antiinflammatory, and antitumor activities. Currently, the structural novelty of designed molecular glue candidates remains quite limited, making the diversification of molecular scaffolds and the development of novel molecular glues a key research focus in the field.

Therefore, the present application designs and develops a series of novel molecular glue degraders based on CRBN E3 ubiquitin ligase, which can be used for treating and/or preventing a disease or disorder mediated by or associated with a degradation protein.

### Summary of Invention

In view of the foregoing, the objectvies of the present disclosure are to provide provide novel protein degradation compounds having two azaheterocycles substituted isoindolinone skeleton, methods for their preparation, uses thereof, and methods of using the same.

To achieve the above objectives and other related goals, in one aspect, the present disclosure provides a compound of Formula (I): 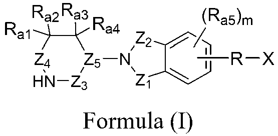 or salts, stereoisomers (including enantiomers and diastereoisomer), solvates, or polymorphs thereof,
wherein Z₁ represents C(O), C(S), CH₂, or CD₂, and Z₂, Z₃, and Z₄ each independently represent C(O) or C(S);
Z₅ represents CH or N;
Rₐ₁, Rₐ₂, Rₐ₃, and Rₐ₄ each independently represent hydrogen, deuterium, halogen, C₁₋₆ alkyl, halogenated C₁₋₆ alkyl, deuterated C₁₋₆ alkyl, C₁₋₆ alkoxy, deuterated C₁₋₆ alkoxy or halogenated C₁₋₆ alkoxy;
(Rₐ₅)ₘ indicates that the isoindoline ring to which it is attached is optionally substituted with m Rₐ₅, with each Rₐ₅ being identical or different and each independently representing deuterium, halogen, hydroxy, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkenyl, or C₂₋₆ alkynyl;
m represents an integer of 0, 1, 2, or 3;
R represents C(O) or optionally substituted linear or branched C₁₋₅ alkylene;
X represents: 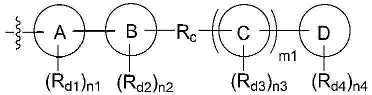
   wherein ring A represents nitrogen-containing heterocyclylene, and (R_{d1})ₙ₁ indicates that the ring A is optionally substituted with n1 R_{d1} groups, wherein each R_{d1} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n1 represents an integer of 0-20;
   ring B represents nitrogen-containing heterocyclylene, and (Ra₂)ₙ₂ indicates that the ring B is optionally substituted with n2 R_{d2} groups, wherein each R_{d2} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n2 represents an integer of 0-20;
   R_{c} represents C(O), CR_{c1}R_{c2}, or a bond, wherein R_{c1} and R_{c2} each independently represent H, deuterium, halogen, optionally substituted linear or branched alkyl, optionally substituted cycloalkyl, optionally substituted heterocyclyl, optionally substituted aryl, or optionally substituted heteroaryl;
   ring C represents cycloalkylene, heterocyclylene, arylene, or heteroarylene, m1 represents an integer of 0 or 1, and (R_{d3})ₙ₃ indicates that the ring C is optionally substituted with n3 R_{d3} groups, wherein each R_{d3} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n3 represents an integer of 0-20; and
   ring D represents heterocyclyl, cycloalkyl, aryl, or heteroaryl, and (R_{d4})ₙ₄ indicates that ring D is optionally substituted with n4 R_{d4} groups, and each R_{d4} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n4 represents an integer of 0-20.

In a further aspect, the present disclosure provides a pharmaceutical composition comprising the compound of Formula (I) or pharmaceutically acceptable salts, stereoisomers (including enantiomers and diastereomers), solvates, isotopically enriched analogs, prodrugs, or polymorphs thereof, and at least one pharmaceutically acceptable carrier.

In a further aspect, the present disclosure further provides a medicine kit or reagent kit comprising: the compound of Formula (I) or a pharmaceutically acceptable salt, or the pharmaceutical composition comprising the same.

In a further aspect, the present disclosure provides the compound of Formula (I) or pharmaceutically acceptable salts, enantiomers, diastereomers, solvates, prodrugs, or polymorphs thereof, for use as a medicament.

In a further aspect, the present disclosure provides the compound of Formula (I) or pharmaceutically acceptable salts, stereoisomers (including enantiomers and diastereomers), solvates, isotopically enriched analogs, prodrugs, or polymorphs thereof, or the pharmaceutical composition of the present disclosure, for use in the treatment or prevention of a disease or disorder associated with cereblon protein.

In a further aspect, the present disclosure provides the compound of Formula (I) or pharmaceutically acceptable salts, stereoisomers (including enantiomers and diastereomers), solvates, isotopically enriched analogs, prodrugs, or polymorphs thereof, or the pharmaceutical composition of the present disclosure, for use in the treatment or prevention of a disease or disorder selected from the group consisting of: tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, and diabetes.

In a further aspect, the present disclosure provides the use of the compound of Formula (I) or pharmaceutically acceptable salts, stereoisomers (including enantiomers and diastereomers), solvates, isotopically enriched analogs, prodrugs, or polymorphs thereof, or the pharmaceutical composition of the present disclosure, for the manufacture of a medicament for the prevention or treatment of a disease or disorder associated with cereblon protein.

In a further aspect, the present disclosure provides the use of the compound of Formula (I) or pharmaceutically acceptable salts, stereoisomers (including enantiomers and diastereomers), solvates, isotopically enriched analogs, prodrugs, or polymorphs thereof, or the pharmaceutical composition of the present disclosure, for the manufacture of a medicament for the prevention or treatment of a disease or disorder selected from the group consisting of: tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, and diabetes.

In a further aspect, the present disclosure provides a method for treating or preventing a disease or disorder associated with cereblon protein, comprising administering to the subject a therapeutically effective amount of the compound of Formula (I) or pharmaceutically acceptable salts, stereoisomers (including enantiomers and diastereomers), solvates, isotopically enriched analogs, prodrugs, or polymorphs thereof, or the pharmaceutical composition of the present disclosure.

In a further aspect, the present disclosure provides a method for treating or preventing a disease or disorder in a subject, comprising administering to the subject a therapeutically effective amount of the compound of Formula (I) or pharmaceutically acceptable salts thereof, or the pharmaceutical composition of the present disclosure, wherein the disease or disorder is selected from the group consisting of: tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, and diabetes.

In a further aspect, the present disclosure provides a method for preparing the compound of Formula (I), comprising reacting a compound of Formula (M1) with a compound of Formula (M2) to obtain the compound of Formula (I): 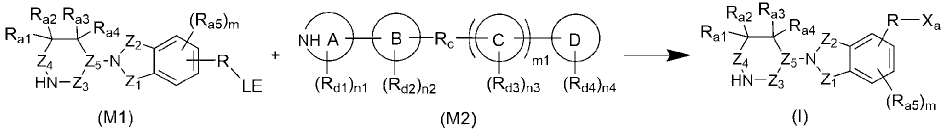
wherein Rₐ₁, Rₐ₂, Rₐ₃, Rₐ₄, (Rₐ₅)ₘ, Z₁, Z₂, Z₃, Z₄, Z₅, nitrogen-containing heterocycle A, (R_{d1})ₙ₁, ring B, (R_{d2})ₙ₂, R_{c}, ring C, m1, (R_{d3})ₙ₃, ring D, and (R_{d4})ₙ₄ are as defined in the compound of Formula (I) and its various sub-embodiments in the present disclosure; and
R represents optionally substituted linear or branched C₁₋₅ alkylene;
LE represents Cl, Br, I, methanesulfonyloxy, p-toluenesulfonyloxy, o-nitrobenzenesulfonyl, C(O)Cl, or COOH; and
Xₐ of the compound of Formula (I) correspondingly represents a structure represented by the following formula: 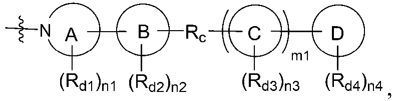
wherein nitrogen-containing heterocyclylene A is a divalent group obtained by removing a hydrogen atom from the nitrogen atom of said nitrogen-containing heterocycle A, wherein said nitrogen-containing heterocyclylene A represents a 4- to 30-membered nitrogen-containing heterocyclylene (preferably, 4- to 20-membered nitrogen-containing heterocyclylene; more preferably, 4- to 15-membered nitrogen-containing heterocyclylene), and said nitrogen-containing heterocycle A represents a 4- to 30-membered nitrogen-containing heterocycle (preferably, 4- to 20-membered nitrogen-containing heterocycle; more preferably, 4- to 15-membered nitrogen-containing heterocycle), and
(R_{d1})ₙ₁, ring B, (R_{d2})ₙ₂, R_{c}, ring C, m1, (R_{d3})ₙ₃, ring D, and (R_{d4})ₙ₄ are as defined in the compound of Formula (I) and its various sub-embodiments in the present disclosure.

### Detailed Description of the Invention

The following detailed description is provided as exemplary specific embodiments to assist those skilled in the art in understanding and practicing the present disclosure. It should be appreciated, however, that such description is not intended to limit the scope of the present disclosure, and that various modifications and changes may be made to the specific embodiments described in the present disclosure without departing from the spirit and scope of the present disclosure. Such changes and modifications are to be understood as being included within the scope of the present invention as defined by the appended claims.

### I. Compounds

### Compounds of Formula (I)

The present disclosure provides a compound of Formula (I) or salts (including pharmaceutically acceptable salts), stereoisomers (including enantiomers and diastereomers), solvates, isotopically enriched analogs, prodrugs, or polymorphs thereof: 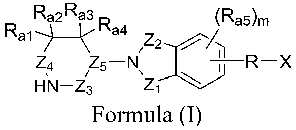 wherein Z₁, Z₂, Z₃, Z₄, Z₅, Rₐ₁, Rₐ₂, Rₐ₃, Rₐ₄, (Rₐ₅)ₘ, R, and X are as defined in the compound of Formula (I) and its various sub-embodiments in the present disclosure.

The compounds of Formula (I) of the present disclosure, or salts thereof (including pharmaceutically acceptable salts), stereoisomers (including enantiomers), solvates, isotopically enriched analogs, prodrugs, or polymorphs thereof, exhibit binding affinity to CRBN, facilitate the recruitment of substrate proteins, and can act as molecular glues binding to the CRBN E3 ubiquitin ligase. These compounds of Formula (I), or salts thereof (including pharmaceutically acceptable salts), stereoisomers (including enantiomers), solvates, isotopically enriched analogs, prodrugs, or polymorphs thereof, can also degrade substrate proteins (e.g., IKZF1/2/3/4 proteins, WEE1 protein, CK1α protein, GSPT1 protein, ZFP91 protein, etc.). The compounds of Formula (I) of the present disclosure, or salts thereof (including pharmaceutically acceptable salts), stereoisomers (including enantiomers), solvates, isotopically enriched analogs, prodrugs, or polymorphs thereof, has antitumor activity or excellent pharmacokinetic properties, rendering them suitable for use as therapeutic agents for tumor patients.

In some embodiments of the present disclosure, Rₐ₁, Rₐ₂, Rₐ₃, and Rₐ₄ are identical or different and each independently represent H, deuterium (i.e., D), C₁₋₆ alkyl (e.g., C₁₋₃ alkyl, such as methyl, ethyl, or propyl), halogenated C₁₋₆ alkyl (e.g., halogenated C₁₋₄ alkyl, such as trifluoromethyl), deuterated C₁₋₆ alkyl, C₁₋₆ alkoxy (e.g., C₁₋₄ alkoxy, such as methoxy), deuterated C₁₋₆ alkoxy, or halogenated C₁₋₆ alkoxy (e.g., halogenated C₁₋₄ alkoxy, such as trifluoromethoxy). In some sub-embodiments of the present disclosure, Rₐ₁, Rₐ₂, Rₐ₃, and Rₐ₄ each independently represent H.

In some embodiments of the present disclosure, Z₁ represents C(O), C(S), CH₂, or CD₂.

In some embodiments of the present disclosure, Z₂, Z₃, and Z₄ represent C(O), or C(S).

In some embodiments of the present disclosure, Z₁, Z₂, Z₃, and Z₄ represent C(O).

In some embodiments of the present disclosure, Z₁ represents CH₂, and Z₂, Z₃, and Z₄ represent C(O).

In some embodiments of the present disclosure, Z₁ represents CH₂, Z₂ represents C(S), and Z₃ and Z₄ represent C(O).

In some embodiments of the present disclosure, Z₁ represents CH₂, Z₃ represents C(S), and Z₂ and Z₄ represent C(O).

In some embodiments of the present disclosure, Z₁ represents CH₂, Z₄ represents C(S), and Z₂ and Z₃ represent C(O).

In some embodiments of the present disclosure, Z₁ represents CH₂, Z₂ and Z₄ represent C(S), and Z₃ represents C(O).

In some embodiments of the present disclosure, Z₁ represents CH₂, Z₂ and Z₃ represent C(S), and Z₄ represents C(O).

In some embodiments of the present disclosure, Z₁ represents CH₂, and Z₂, Z₃, and Z₄ represent C(S).

In some embodiments of the present disclosure, Z₁ represents C(S), and Z₂, Z₃, and Z₄ represent C(O).

In some embodiments of the present disclosure, Z₂ represents C(S), and Z₁, Z₃, and Z₄ represent C(O).

In some embodiments of the present disclosure, Z₃ represents C(S), and Z₁, Z₂, and Z₄ represent C(O).

In some embodiments of the present disclosure, Z₄ represents C(S), and Z₁, Z₂, and Z₃ represent C(O).

In some embodiments of the present disclosure, Z₁ and Z₂ represent C(S), and Z₃ and Z₄ represent C(O).

In some embodiments of the present disclosure, Z₁ and Z₃ represent C(S), and Z₂ and Z₄ represent C(O).

In some embodiments of the present disclosure, Z₁ and Z₄ represent C(S), and Z₂ and Z₃ represent C(O).

In some embodiments of the present disclosure, Z₁, Z₂, and Z₃ represent C(S), and Z₄ represents C(O).

In some embodiments of the present disclosure, Z₁, Z₂, and Z₄ represent C(S), and Z₃ represents C(O).

In some embodiments of the present disclosure, Z₁, Z₃, and Z₄ represent C(S), and Z₂ represents C(O).

In some embodiments of the present disclosure, Z₁, Z₂, Z₃, and Z₄ represent C(S).

In some embodiments of the present disclosure, Z₅ represents CH or N.

In some embodiments of the present disclosure, (Rₐ₅)ₘ indicates that the isoindoline ring in Formula (I) to which it is attached is optionally substituted with m Rₐ₅, wherein each Rₐ₅ is identical or different and each independently represents deuterium, halogen (e.g., fluorine, chlorine, bromine, or iodine), hydroxy, mercapto, nitro, amino, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂-etc.), C₁₋₆ alkoxy (e.g., C₁₋₄ alkoxy, such as methoxy, ethoxy, propoxy, isopropoxy, n-butoxy, sec-butoxy, or tert-butoxy), deuterated C₁₋₆ alkoxy, halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), C₂₋₆ alkenyl (e.g., vinyl), or C₂₋₆ alkynyl (e.g., ethynyl), and m represents an integer of 0, 1, 2, or 3. In some sub-embodiments of the present disclosure, m represents an integer of 0, 1 or 2. In some sub-embodiments of the present disclosure, each Rₐ₅ is identical or different and each independently represents deuterium, halogen (e.g., fluorine, chlorine, bromine, or iodine), hydroxy, mercapto, nitro, amino, cyano, C₁₋₄ alkyl (e.g., C₁₋₃ alkyl, such as methyl, ethyl, propyl, isopropyl, butyl, see-butyl, or tert-butyl), halogenated C₁₋₄ alkyl (e.g., halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₄ alkoxy (e.g., C₁₋₃ alkoxy, such as methoxy, ethoxy, propoxy, isopropoxy, n-butoxy, sec-butoxy, or tert-butoxy), or halogenated C₁₋₄ alkoxy (e.g., halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-).

In the embodiments of the present disclosure, the number of substituents is not theoretically limited in any way, or is automatically limited by the size of the building units.

In some embodiments of the present disclosure, R represents C(O) or optionally substituted linear or branched C₁₋₅ alkylene (e.g., C₁₋₄ alkylene, C₁₋₃ alkylene, C₁₋₂ alkylene, or methylene). The C₁₋₅ alkylene is optionally substituted with a substituent(s) selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, and C₂₋₆ alkenyl.

In some embodiments of the present disclosure, R represents C(O).

In some embodiments of the present disclosure, R represents -CH₂-, -(CH₂)₂-, -(CH₂)₃-, - (CH₂)₄-, or -(CH₂)₅-; wherein the above groups are optionally substituted with a substituent selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, and C₂₋₆ alkenyl.

In some embodiments of the present disclosure, X represents: 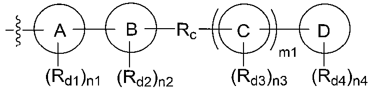
wherein ring A represents nitrogen-containing heterocyclylene, and (R_{d1})ₙ₁ indicates that the ring A is optionally substituted with n1 R_{d1} groups, wherein each R_{d1} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n1 represents an integer of 0-20;
ring B represents nitrogen-containing heterocyclylene, and (R_{d2})ₙ₂ indicates that the ring B is optionally substituted with n2 R_{d2} groups, wherein each R_{d2} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n2 represents an integer of 0-20;
R_{c} represents C(O), CR_{c1}R_{c2}, or a bond, wherein R_{c1} and R_{c2} each independently represent H, deuterium, halogen, optionally substituted linear or branched alkyl, optionally substituted cycloalkyl, optionally substituted heterocyclyl, optionally substituted aryl, or optionally substituted heteroaryl;
ring C represents cycloalkylene, heterocyclylene, arylene, or heteroarylene, m1 represents an integer of 0 or 1, and (R_{d3})ₙ₃ indicates that the ring C is optionally substituted with n3 R_{d3} groups, wherein each R_{d3} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n3 represents an integer of 0-20; and
ring D represents heterocyclyl, cycloalkyl, aryl, or heteroaryl, and (R_{d4})ₙ₄ indicates that the ring D is optionally substituted with n4 R_{d4} groups, and each R_{d4} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n4 represents an integer of 0-20.

In some embodiments of the present disclosure, ring A represents 4- to 30-membered nitrogen-containing heterocyclylene (including 4- to 25-membered nitrogen-containing heterocyclylene, 4- to 20-membered nitrogen-containing heterocyclylene, 4- to 15-membered nitrogen-containing heterocyclylene, and 5- to 20-membered nitrogen-containing heterocyclylene). Ring A is optionally substituted with n1 R_{d1} groups, each R_{d1} independently being deuterium, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), halogen (e.g., fluorine, chlorine, bromine, or iodine), amino, hydroxy, mercapto, cyano, oxo, C₂₋₆ alkynyl (e.g., ethynyl), or C₂₋₆ alkenyl (e.g., vinyl), and n1 represents an integer of 0, 1, 2, 3, 4, 5, 6, 7, 8, 9, 10, 11, 12, 13, 14, 15, 16, 17, 18, 19, or 20.

In some embodiments of the present disclosure, ring A represents azetidinylene, pyrrolidinylene, imidazolidinylene, pyrazolidylene, piperidinylene, piperazinylene, azacycloheptanylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, quinuclidinylene, 2,6-diazaspiro[3.3]heptanylene, 2,7-diazaspiro[3.5]nonanylene, 2,8-diazaspiro[4.5]decanylene, 3,9-diazaspiro[5.5]undecanylene, 3-azaspiro[5.5]undecanylene, 7-azaspiro[3.5]nonanylene, 8-azaspiro[4.5]decanylene, or octahydropyrrolo[3,4-c]pyrrolylene, each optionally substituted with one or more (e.g., 1-20, 1-15, 1-10, 1-5, 1-4, or 1-3) substituents selected from the group consisting of deuterium, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl, halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), halogen (e.g., fluorine, chlorine, bromine, or iodine), amino, hydroxy, mercapto, cyano, oxo, C₂₋₆ alkynyl (e.g., ethynyl), and C₂₋₆ alkenyl (e.g., vinyl).

In some embodiments of the present disclosure, ring B represents 4- to 30-membered nitrogen-containing heterocyclylene (including 4- to 25-membered nitrogen-containing heterocyclylene, 4- to 20-membered nitrogen-containing heterocyclylene, 4- to 15-membered nitrogen-containing heterocyclylene, and 5- to 20-membered nitrogen-containing heterocyclylene). Ring B is optionally substituted with n2 R_{d2} groups, each R_{d2} independently being deuterium, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), halogen (e.g., fluorine, chlorine, bromine, or iodine), amino, hydroxy, mercapto, cyano, oxo, C₂₋₆ alkynyl (e.g., ethynyl), or C₂₋₆ alkenyl (e.g., vinyl), and n2 represents an integer of 0, 1, 2, 3, 4, 5, 6, 7, 8, 9, 10, 11, 12, 13, 14, 15, 16, 17, 18, 19, or 20.

In some embodiments of the present disclosure, ring B represents azetidinylene, pyrrolidinylene, imidazolidinylene, pyrazolidylene, piperidinylene, piperazinylene, azacycloheptanylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, quinuclidinylene, 2,6-diazaspiro[3.3]heptanylene, 2,7-diazaspiro[3.5]nonanylene, 2,8-diazaspiro[4.5]decanylene, 3,9-diazaspiro[5.5]undecanylene, 3-azaspiro[5.5]undecanylene, 7-azaspiro[3.5]nonanylene, 8-azaspiro[4.5]decanylene, or octahydropyrrolo[3,4-c]pyrrolylene, each optionally substituted with one or more (e.g., 1-20, 1-15, 1-10, 1-5, 1-4, or 1-3) substituents selected from the group consisting of deuterium, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl, halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), halogen (e.g., fluorine, chlorine, bromine, or iodine), amino, hydroxy, mercapto, cyano, oxo, C₂₋₆ alkynyl (e.g., ethynyl), and C₂₋₆ alkenyl (e.g., vinyl).

In some embodiments of the present disclosure, R_{c} represents C(O).

In some embodiments of the present disclosure, R_{c} represents a bond. When R_{c} represents a bond, ring B is directly connected to ring C.

In some embodiments of the present disclosure, R_{c} represents CR_{c1}R_{c2}, wherein R_{c1} and R_{c2} each independently represent H, deuterium, halogen, optionally substituted linear or branched C₁₋₁₀ alkyl (e.g., optionally substituted linear or branched C₁₋₆ alkyl, or optionally substituted linear or branched C₁₋₃ alkyl), optionally substituted C₃₋₃₀ cycloalkyl (e.g., optionally substituted C₃₋₂₀ cycloalkyl, or optionally substituted C₃₋₁₅ cycloalkyl), optionally substituted C₅₋₃₀ aryl (e.g., optionally substituted C₅₋₂₀ aryl, or optionally substituted C₅₋₁₅ aryl), optionally substituted 4- to 30-membered heterocyclyl (e.g., optionally substituted 4- to 20-membered heterocyclyl, or optionally substituted 4- to 15-membered heterocyclyl), or optionally substituted 5- to 30-membered heteroaryl (e.g., optionally substituted 5- to 20-membered heteroaryl, or optionally substituted 5- to 15-membered heteroaryl). In some embodiments, examples of linear or branched C₁₋₁₀ alkyl include, but are not limited to, methyl, ethyl, propyl, isopropyl, butyl, isobutyl, sec-butyl, tert-butyl, pentyl, isopentyl, neopentyl, tert-pentyl, hexyl, heptyl, and octyl. In some embodiments, the linear or branched C₁₋₁₀ alkyl is optionally substituted with one or more (e.g., 1-20, 1-15, 1-10, 1-6, 1-4, 1-3, 2-6, or 1) substituents selected from the group consisting of deuterium, halogen, amino, hydroxy, mercapto, cyano, nitro, oxo, C₁₋₆ alkyl, deuterated C₁₋₆ alkyl, halogenated C₁₋₆ alkyl, C₁₋₆ alkoxy, halogenated C₁₋₆ alkoxy, C₂₋₆ alkynyl, and C₂₋₆ alkenyl. In some embodiments, examples of C₃₋₃₀ cycloalkyl include, but are not limited to, cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., C₅-C₂₀ spiro-cycloalkyl, such as spiro[3.3]heptyl, spiro[2.5]octyl, spiro[3.5]nonyl, spiro[4.4]nonyl, spiro[4.5]decyl, and spiro[5.5]undecyl), *p*-menthanyl, *m*-menthanyl, or bridged cycloalkyl (e.g., C₆-C₂₀ bridged cycloalkyl, such as adamantanyl, noradamantanyl, bornyl, norbornyl, bicyclo[2.2.1]heptanyl, 2-oxobicyclo[2.2.1]heptyl, or bicyclo[2.2.1]heptenyl). In some embodiments, the C₃₋₃₀ cycloalkyl is optionally substituted with one or more (e.g., 1-20, 1-15, 1-10, 1-6, 1-4, 1-3, 2-6, or 1) substituents selected from the group consisting of deuterium, halogen, hydroxy, mercapto, nitro, amino, cyano, oxo, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkenyl, and C₂₋₆ alkynyl. In some embodiments, examples of 4- to 30-membered heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, tetrahydropyridinyl, dihydroxy-piperidinyl, difluoro-piperidinyl, morpholinyl, thiomorpholinyl, azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), and azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl. In some embodiments, 4- to 30-membered heterocyclyl is optionally substituted with one or more (e.g., 1-20, 1-15, 1-10, 1-6, 1-4, 1-3, 2-6, or 1) substituents selected from the group consisting of deuterium, halogen, hydroxy, mercapto, amino, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₂₋₆ alkenyl, and C₂₋₆ alkynyl. In some embodiments, examples of C₅₋₃₀ aryl include, but are not limited to, phenyl or naphthyl. In some embodiments, the C₅₋₃₀ aryl is optionally substituted with one or more (e.g., 1-20, 1-15, 1-10, 1-6, 1-4, 1-3, 2-6, or 1) substituents selected from the group consisting of deuterium, halogen, hydroxy, mercapto, nitro, amino, cyano, C₁₋₆ alkyl, halogenated C₁₋₆ alkyl, deuterated C₁₋₆ alkyl, C₁₋₆ alkoxy, halogenated C₁₋₆ alkoxy, C₂₋₆ alkenyl, and C₂₋₆ alkynyl. In some embodiments, examples of 5- to 30-membered heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, indolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl,quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno[2,3-d]pyrimidinyl. In some embodiments, the 5- to 30-membered heteroaryl is optionally substituted with one or more (e.g., 1-20, 1-15, 1-10, 1-6, 1-4, 1-3, 2-6, or 1) substituents selected from the group consisting of deuterium, halogen, hydroxy, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkenyl, and C₂₋₆ alkynyl.

In some embodiments of the present disclosure, m1 represents an integer of 1, and ring C represents 4- to 30-membered heterocyclylene (e.g., 4- to 20-membered heterocyclylene, or 4- to 15-membered heterocyclylene), C₃₋₃₀ cycloalkylene (e.g., C₃₋₂₀ cycloalkylene, or C₃₋₁₅ cycloalkylene), C₅₋₃₀ arylene (e.g., C₅₋₂₀ arylene, or C₅₋₁₅ arylene), or 5- to 30-membered heteroarylene (e.g., 5- to 20-membered heteroarylene, or 5- to 15-membered heteroarylene). Ring C is optionally substituted with n3 R_{d3} groups, wherein each R_{d3} independently represents deuterium, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl, halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), halogen (e.g., fluorine, chlorine, bromine, or iodine), amino, hydroxy, mercapto, cyano, oxo, C₂₋₆ alkynyl (e.g., ethynyl), or C₂₋₆ alkenyl (e.g., vinyl), and n3 represents an integer of 0, 1, 2, 3, 4, 5, 6, 7, 8, 9, 10, 11, 12, 13, 14, 15, 16, 17, 18, 19, or 20.

In some embodiments, ring C represents 4- to 30-membered heterocyclylene, examples of which include but are not limited to 4- to 20-membered, 4- to 15-membered, 4- to 12-membered, 4-to 11-membered, 4- to 10-membered, 4- to 9-membered, 4- to 8-membered, 4- to 7-membered, 4- to 6-membered, 5- to 15-membered, and 5- to 9-membered heterocyclylene, such as azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, azacycloheptanylene, azacyclooctylene, diazacycloheptanylene (e.g., 1,4-diazacycloheptanylene, 4,5-diazacycloheptanylene, 1,3-diazacycloheptanylene), diazacyclooctylene, bridged heterocyclylene (e.g., 6- to 20-membered bridged heterocyclylene, such as 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and quinuclidinylene), azaspirocycloalkylene (e.g., 5- to 20-membered azaspirocycloalkylene, such as 2,6-diazaspiro[3.3]heptanylene, 2,7-diazaspiro[3.5]nonanylene, 2,8-diazaspiro[4.5]decanylene, 3,9-diazaspiro[5.5]undecanylene, 3-azaspiro[5.5]undecanylene, and 7-azaspiro[3.5]nonanylene), or octahydropyrrolo[3,4-c]pyrrolylene. The heterocyclylene is optionally substituted with one or more (e.g., 1-20, 1-15, 1-10, 1-6, 1-4, 1-3, 2-6, or 1) substituents selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, hydroxy-substituted C₁₋₆ alkyl, amino-substituted C₁₋₆ alkyl, C₁₋₆ alkoxy, deuterated C₁₋₆ alkoxy, halogenated C₁₋₆ alkoxy, C₂₋₆ alkenyl, and C₂₋₆ alkynyl.

In some embodiments, ring C represents C₃₋₃₀ cycloalkylene, examples of which include but are not limited to C₃₋₂₀ cycloalkylene, C₃₋₁₅ cycloalkylene, and C₃₋₁₁ cycloalkylene, such as cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, spiro-cycloalkylene (e.g., C₅-C₂₀ spiro-cycloalkylene and C₅₋₁₅ spiro-cycloalkylene, such as spiro[3.3]heptylene, spiro[2.5]octylene, spiro[3.5]nonylene, spiro[4.4]nonylene, spiro[4.5]decylene, and spiro[5.5]undecylene), *p*-menthanylene, *m*-menthanylene, or bridged cycloalkylene (e.g., C₆-C₂₀ bridged cycloalkylene, such as adamantanylene, noradamantanylene, bornylene, norbornylene, bicyclo[2.2.1]heptylene, 2-oxobicyclo[2.2.1]heptylene, and bicyclo[2.2.1]heptentylene). The cycloalkylene is optionally substituted with one or more (e.g., 1-20, 1-15, 1-10, 1-6, 1-4, 1-3, 2-6, or 1) substituents selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, hydroxy-substituted C₁₋₆ alkyl, amino-substituted C₁₋₆ alkyl, C₁₋₆ alkoxy, deuterated C₁₋₆ alkoxy, halogenated C₁₋₆ alkoxy, C₂₋₆ alkenyl, and C₂₋₆ alkynyl.

In some embodiments, ring C represents C₅₋₃₀ arylene, examples of which include but are not limited to C₅₋₂₀ arylene, C₆₋₂₀ arylene, C₅₋₁₅ arylene, and C₆₋₁₅ arylene, such as phenylene or naphthylene. The arylene is optionally substituted with one or more (e.g., 1-6, 1-4, 1-3, 2-6, or 1) substituents selected from the group consisting of deuterium, hydroxy, amino, mercapto, nitro, halogen, cyano, C₁₋₆ alkyl, halogenated C₁₋₆ alkyl, deuterated C₁₋₆ alkyl, hydroxy-substituted C₁₋₆ alkyl, amino-substituted C₁₋₆ alkyl, C₁₋₆ alkoxy, deuterated C₁₋₆ alkoxy, halogenated C₁₋₆ alkoxy, C₂₋₆ alkenyl, and C₂₋₆ alkynyl.

In some embodiments, ring C represents 5- to 30-membered heteroarylene, examples of which include but are not limited to 5- to 20-membered heteroarylene, 5- to 15-membered, 5- to 10-membered, 5- to 9-membered, 5- to 8-membered, 5- to 7-membered, and 5- to 6-membered heteroarylene, such as furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, triazinylene, indolylene, isoindolylene, indolinylene, benzofuranylene, chromanylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, 2-oxo-2,3-dihydro-1H-benzo[d]imidazolylene, benzo[b][1,4]oxazinylene, 3,4-dihydro-2H-benzo[b][1,4]oxazinylene, quinolinylene, isoquinolinylene, 1,2,3,4-tetrahydroquinolinylene, naphthyridinylene, cinnolinylene, quinazolinylene, quinoxalinylene, 1,2,3,4-tetrahydroquinoxalinylene, phthalazinylene, 5,6,7,8-tetrahydro-[1,2,4]triazolo[4,3-a]pyrazinylene, 4,5,6,7-tetrahydrothieno[3,2-c]pyridinylene, 5-oxo-6,7-dihydrothieno[3,2-d]pyrimidinylene, thieno[2,3-d]pyrimidinylene, thieno[3,2-d]pyrimidinylene, isoxazolo[4,5-c]pyridinylene, isoxazolo[4,5-c]pyrimidinylene, isoxazolo[4,5-d]pyrimidinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, imidazo[2,1-b]thiazolylene, 5,6,7,8-tetrahydrobenzo[4,5]thieno[2,3-d]pyrimidinylene, or 6,7-dihydro-5H-cyclopenta[4,5]thieno[2,3-d]pyrimidinylene. The heteroarylene is optionally substituted with one or more (e.g., 1-20, 1-15, 1-10, 1-6, 1-4, 1-3, 2-6, or 1) substituents selected from the group consisting of deuterium, halogen, hydroxy, mercapto, nitro, amino, cyano, C₁₋₆ alkyl, halogenated C₁₋₆ alkyl, deuterated C₁₋₆ alkyl, hydroxy-substituted C₁₋₆ alkyl, amino-substituted C₁₋₆ alkyl, C₁₋₆ alkoxy, deuterated C₁₋₆ alkoxy, halogenated C₁₋₆ alkoxy, C₂₋₆ alkenyl, and C₂₋₆ alkynyl.

In some embodiments of the present disclosure, when m1 represents 0 (i.e., ring C is absent), R_{c} is directly bonded to ring D.

In some embodiments of the present disclosure, ring D represents C₃₋₃₀ cycloalkyl (e.g., C₃₋₂₀ cycloalkyl, or C₃₋₁₅ cycloalkyl), 4- to 30-membered heterocyclyl (e.g., 4- to 20-membered heterocyclyl, or 4- to 15-membered heterocyclyl), C₅₋₃₀ aryl (e.g., C₅₋₂₀ aryl, or C₅₋₁₅ aryl), or 5- to 30-membered heteroaryl (e.g., 5- to 20-membered heteroaryl, or 5- to 15-membered heteroaryl). Ring D is optionally substituted with n4 R_{d4} groups, and each R_{d4} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n4 represents an integer of 0, 1, 2, 3, 4, 5, 6, 7, 8, 9, 10, 11, 12, 13, 14, 15, 16, 17, 18, 19, or 20.

In some embodiments, ring D represents C₃₋₃₀ cycloalkyl, examples of which include but are not limited to C₃₋₂₀ cycloalkyl, C₃₋₁₅ cycloalkyl, and C₃₋₁₁ cycloalkyl, such as cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., C₅-C₂₀ spiro-cycloalkyl, such as spiro[3.3]heptyl, spiro[2.5]octyl, spiro[3.5]nonyl, spiro[4.4]nonyl, spiro[4.5]decyl, and spiro[5.5]undecyl), *p*-menthanyl, *m*-menthanyl, or bridged cycloalkyl (e.g., C₆-C₂₀ bridged cycloalkyl, such as adamantanyl, noradamantanyl, bornyl, norbornyl, bicyclo[2.2.1]heptanyl, 2-oxobicyclo[2.2.1]heptyl, or bicyclo[2.2.1]heptenyl). The cycloalkyl is optionally substituted with one or more (e.g., 1-20, 1-15, 1-10, 1-6, 1-4, 1-3, 2-6, or 1) substituents selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, and C₂₋₆ alkenyl.

In some embodiments, ring D represents 4- to 30-membered heterocyclyl, examples of which include but are not limited to 4- to 20-membered, 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, and 5- to 9-membered heterocyclyl, such as azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, tetrahydropyridinyl, dihydroxy-piperidinyl, difluoro-piperidinyl, morpholinyl, thiomorpholinyl, azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), and azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), and octahydropyrrolo[3,4-c]pyrrolyl. The heterocyclyl is optionally substituted with one or more (e.g., 1-20, 1-15, 1-10, 1-6, 1-4, 1-3, 2-6, or 1) substituents selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, and C₂₋₆ alkenyl.

In some embodiments, ring D represents C₅₋₃₀ aryl, examples of which include but are not limited to C₅₋₂₀ aryl, C₆₋₂₀ aryl, C₅₋₁₅ aryl, and C₆₋₁₅ aryl, such as phenyl or naphthyl, optionally substituted with one or more (e.g., 1-7, 1-6, 1-4, 1-3, 2-6, or 1) substituent(s) selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, C₂₋₆ alkynyl, and C₂₋₆ alkenyl.

In some embodiments, ring D represents 5- to 30-membered heteroaryl, examples of which include but are not limited to 5- to 20-membered, 5- to 15-membered, 5- to 10-membered, 5- to 9-membered, 5- to 8-membered, 5- to 7-membered, and 5- to 6-membered heteroaryl, such as furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, indolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl,quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno[2,3-d]pyrimidinyl. The heteroaryl is optionally substituted with one or more (e.g., 1-20, 1-15, 1-10, 1-6, 1-4, 1-3, 2-6, or 1) substituents selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, C₂₋₆ alkynyl, and C₂₋₆ alkenyl.

In some embodiments of the present disclosure, X represents: 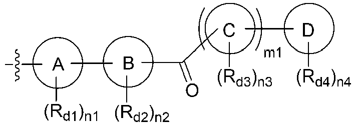
wherein ring A represents nitrogen-containing heterocyclylene (including 4- to 30-membered nitrogen-containing heterocyclylene, 4- to 25-membered nitrogen-containing heterocyclylene, 4- to 20-membered nitrogen-containing heterocyclylene, 4- to 15-membered nitrogen-containing heterocyclylene, and 5- to 20-membered nitrogen-containing heterocyclylene), and (R_{d1})ₙ₁ indicates that the ring A is optionally substituted with n1 R_{d1} groups, wherein each R_{d1} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n1 represents an integer of 0, 1, 2, 3, 4, 5, 6, 7, 8, 9, 10, 11, 12, 13, 14, 15, 16, 17, 18, 19, or 20;
ring B represents nitrogen-containing heterocyclylene (including 4- to 30-membered nitrogen-containing heterocyclylene, 4- to 25-membered nitrogen-containing heterocyclylene, 4- to 20-membered nitrogen-containing heterocyclylene, 4- to 15-membered nitrogen-containing heterocyclylene, and 5- to 20-membered nitrogen-containing heterocyclylene), and (Ra₂)ₙ₂ indicates that the ring B is optionally substituted with n2 R_{d2} groups, wherein each R_{d2} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n2 represents an integer of 0, 1, 2, 3, 4, 5, 6, 7, 8, 9, 10, 11, 12, 13, 14, 15, 16, 17, 18, 19, or 20;
R_{c} represents C(O), CR_{c1}R_{c2}, or a bond, wherein R_{c1} and R_{c2} each independently represent H, deuterium, halogen, optionally substituted linear or branched alkyl (e.g., optionally substituted linear or branched C₁₋₁₀ alkyl, optionally substituted linear or branched C₁₋₆ alkyl, or optionally substituted linear or branched C₁₋₃ alkyl), optionally substituted cycloalkyl (e.g., optionally substituted C₃₋₃₀ cycloalkyl, optionally substituted C₃₋₂₀ cycloalkyl, or optionally substituted C₃₋₁₅ cycloalkyl), optionally substituted heterocyclyl (e.g., optionally substituted 4- to 30-membered heterocyclyl, optionally substituted 4- to 20-membered heterocyclyl, or optionally substituted 4- to 15-membered heterocyclyl), optionally substituted aryl (e.g., optionally substituted C₅₋₃₀ aryl, optionally substituted C₅₋₂₀ aryl, or optionally substituted C₅₋₁₅ aryl), or optionally substituted heteroaryl (e.g., optionally substituted 5- to 30-membered heteroaryl, optionally substituted 5- to 20-membered heteroaryl, or optionally substituted 5- to 15-membered heteroaryl);
ring C represents cycloalkylene (e.g., C₃₋₃₀ cycloalkylene, C₃₋₂₀ cycloalkylene, or C₃₋₁₅ cycloalkylene), heterocyclylene (e.g., 4- to 30-membered heterocyclylene, 4- to 20-membered heterocyclylene, or 4- to 15-membered heterocyclylene), arylene (e.g., C₅₋₃₀ arylene, C₅₋₂₀ arylene, or C₅₋₁₅ arylene), or heteroarylene (e.g., 5- to 30-membered heteroarylene, 5- to 20-membered heteroarylene, or 5- to 15-membered heteroarylene), m1 represents an integer of 0 or 1, and (R_{d3})ₙ₃ indicates that the ring C is optionally substituted with n3 R_{d3} groups, wherein each R_{d3} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n3 represents an integer of 0, 1, 2, 3, 4, 5, 6, 7, 8, 9, 10, 11, 12, 13, 14, 15, 16, 17, 18, 19, or 20; and
ring D represents heterocyclyl (e.g., 4- to 30-membered heterocyclyl, 4- to 20-membered heterocyclyl, or 4- to 15-membered heterocyclyl), cycloalkyl (e.g., C₃₋₃₀ cycloalkyl, C₃₋₂₀ cycloalkyl, or C₃₋₁₅ cycloalkyl), aryl (e.g., C₅₋₃₀ aryl, C₅₋₂₀ aryl, or C₅₋₁₅ aryl), or heteroaryl (e.g., 5- to 30-membered heteroaryl, 5- to 20-membered heteroaryl, or 5- to 15-membered heteroaryl), and (R_{d4})ₙ₄ indicates that the ring D is optionally substituted with n4 R_{d4} groups, wherein each R_{d4} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n4 represents an integer of 0, 1, 2, 3, 4, 5, 6, 7, 8, 9, 10, 11, 12, 13, 14, 15, 16, 17, 18, 19, or 20.

In some embodiments of the present disclosure, X represents: 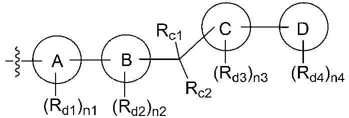
wherein ring A represents nitrogen-containing heterocyclylene (including 4- to 30-membered nitrogen-containing heterocyclylene, 4- to 25-membered nitrogen-containing heterocyclylene, 4- to 20-membered nitrogen-containing heterocyclylene, 4- to 15-membered nitrogen-containing heterocyclylene, and 5- to 20-membered nitrogen-containing heterocyclylene), and (R_{d1})ₙ₁ indicates that the ring A is optionally substituted with n1 R_{d1} groups, wherein each R_{d1} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n1 represents an integer of 0, 1, 2, 3, 4, 5, 6, 7, 8, 9, 10, 11, 12, 13, 14, 15, 16, 17, 18, 19, or 20;
ring B represents nitrogen-containing heterocyclylene (including 4- to 30-membered nitrogen-containing heterocyclylene, 4- to 25-membered nitrogen-containing heterocyclylene, 4- to 20-membered nitrogen-containing heterocyclylene, 4- to 15-membered nitrogen-containing heterocyclylene, and 5- to 20-membered nitrogen-containing heterocyclylene), and (Ra₂)ₙ₂ indicates that the ring B is optionally substituted with n2 R_{d2} groups, wherein each R_{d2} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n2 represents an integer of 0, 1, 2, 3, 4, 5, 6, 7, 8, 9, 10, 11, 12, 13, 14, 15, 16, 17, 18, 19, or 20;
R_{c1} and R_{c2} each independently represent H, deuterium, halogen, optionally substituted linear or branched alkyl (e.g., optionally substituted linear or branched C₁₋₁₀ alkyl, optionally substituted linear or branched C₁₋₆ alkyl, or optionally substituted linear or branched C₁₋₃ alkyl), optionally substituted cycloalkyl (e.g., optionally substituted C₃₋₃₀ cycloalkyl, optionally substituted C₃₋₂₀ cycloalkyl, or optionally substituted C₃₋₁₅ cycloalkyl), optionally substituted heterocyclyl (e.g., optionally substituted 4- to 30-membered heterocyclyl, optionally substituted 4- to 20-membered heterocyclyl, or optionally substituted 4- to 15-membered heterocyclyl), optionally substituted aryl (e.g., optionally substituted C₅₋₃₀ aryl, optionally substituted C₅₋₂₀ aryl, or optionally substituted C₅₋₁₅ aryl), or optionally substituted heteroaryl (e.g., optionally substituted 5- to 30-membered heteroaryl, optionally substituted 5- to 20-membered heteroaryl, or optionally substituted 5- to 15-membered heteroaryl);
ring C represents cycloalkylene (e.g., C₃₋₃₀ cycloalkylene, C₃₋₂₀ cycloalkylene, or C₃₋₁₅ cycloalkylene), heterocyclylene (e.g., 4- to 30-membered heterocyclylene, 4- to 20-membered heterocyclylene, or 4- to 15-membered heterocyclylene), arylene (e.g., C₅₋₃₀ arylene, C₅₋₂₀ arylene, or C₅₋₁₅ arylene), or heteroarylene (e.g., 5- to 30-membered heteroarylene, 5- to 20-membered heteroarylene, or 5- to 15-membered heteroarylene), m1 represents an integer of 0 or 1, and (R_{d3})ₙ₃ indicates that the ring C is optionally substituted with n3 R_{d3} groups, wherein each R_{d3} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n3 represents an integer of 0, 1, 2, 3, 4, 5, 6, 7, 8, 9, 10, 11, 12, 13, 14, 15, 16, 17, 18, 19, or 20; and
ring D represents heterocyclyl (e.g., 4- to 30-membered heterocyclyl, 4- to 20-membered heterocyclyl, or 4- to 15-membered heterocyclyl), cycloalkyl (e.g., C₃₋₃₀ cycloalkyl, C₃₋₂₀ cycloalkyl, or C₃₋₁₅ cycloalkyl), aryl (e.g., C₅₋₃₀ aryl, C₅₋₂₀ aryl, or C₅₋₁₅ aryl), or heteroaryl (e.g., 5- to 30-membered heteroaryl, 5- to 20-membered heteroaryl, or 5- to 15-membered heteroaryl), and (R_{d4})ₙ₄ indicates that the ring D is optionally substituted with n4 R_{d4} groups, wherein each R_{d4} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n4 represents an integer of 0, 1, 2, 3, 4, 5, 6, 7, 8, 9, 10, 11, 12, 13, 14, 15, 16, 17, 18, 19, or 20.

In some embodiments of the present disclosure, X represents: 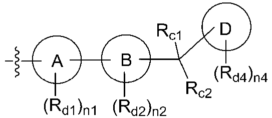
wherein ring A represents nitrogen-containing heterocyclylene (including 4- to 30-membered nitrogen-containing heterocyclylene, 4- to 25-membered nitrogen-containing heterocyclylene, 4- to 20-membered nitrogen-containing heterocyclylene, 4- to 15-membered nitrogen-containing heterocyclylene, and 5- to 20-membered nitrogen-containing heterocyclylene), and (R_{d1})ₙ₁ indicates that the ring A is optionally substituted with n1 R_{d1} groups, wherein each R_{d1} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n1 represents an integer of 0, 1, 2, 3, 4, 5, 6, 7, 8, 9, 10, 11, 12, 13, 14, 15, 16, 17, 18, 19, or 20;
ring B represents nitrogen-containing heterocyclylene (including 4- to 30-membered nitrogen-containing heterocyclylene, 4- to 25-membered nitrogen-containing heterocyclylene, 4- to 20-membered nitrogen-containing heterocyclylene, 4- to 15-membered nitrogen-containing heterocyclylene, and 5- to 20-membered nitrogen-containing heterocyclylene), and (R_{d2})ₙ₂ indicates that the ring B is optionally substituted with n2 R_{d2} groups, wherein each R_{d2} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n2 represents an integer of 0, 1, 2, 3, 4, 5, 6, 7, 8, 9, 10, 11, 12, 13, 14, 15, 16, 17, 18, 19, or 20;
R_{c1} represents H, deuterium, halogen, or optionally substituted linear or branched alkyl (e.g., optionally substituted linear or branched C₁₋₁₀ alkyl, optionally substituted linear or branched C₁₋₆ alkyl, or optionally substituted linear or branched C₁₋₃ alkyl);
R_{c2} represents H, deuterium, halogen, optionally substituted linear or branched alkyl (e.g., optionally substituted linear or branched C₁₋₁₀ alkyl, optionally substituted linear or branched C₁₋₆ alkyl, or optionally substituted linear or branched C₁₋₃ alkyl), optionally substituted cycloalkyl (e.g., optionally substituted C₃₋₃₀ cycloalkyl, optionally substituted C₃₋₂₀ cycloalkyl, or optionally substituted C₃₋₁₅ cycloalkyl), optionally substituted heterocyclyl (e.g., optionally substituted 4- to 30-membered heterocyclyl, optionally substituted 4- to 20-membered heterocyclyl, or optionally substituted 4- to 15-membered heterocyclyl), optionally substituted aryl (e.g., optionally substituted C₅₋₃₀ aryl, optionally substituted C₅₋₂₀ aryl, or optionally substituted C₅₋₁₅ aryl), or optionally substituted heteroaryl (e.g., optionally substituted 5- to 30-membered heteroaryl, optionally substituted 5- to 20-membered heteroaryl, or optionally substituted 5- to 15-membered heteroaryl); and
ring D represents heterocyclyl (e.g., 4- to 30-membered heterocyclyl, 4- to 20-membered heterocyclyl, or 4- to 15-membered heterocyclyl), cycloalkyl (e.g., C₃₋₃₀ cycloalkyl, C₃₋₂₀ cycloalkyl, or C₃₋₁₅ cycloalkyl), aryl (e.g., C₅₋₃₀ aryl, C₅₋₂₀ aryl, or C₅₋₁₅ aryl), or heteroaryl (e.g., 5- to 30-membered heteroaryl, 5- to 20-membered heteroaryl, or 5- to 15-membered heteroaryl), and (R_{d4})ₙ₄ indicates that the ring D is optionally substituted with n4 R_{d4} groups, wherein each R_{d4} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n4 represents an integer of 0, 1, 2, 3, 4, 5, 6, 7, 8, 9, 10, 11, 12, 13, 14, 15, 16, 17, 18, 19, or 20.

In some embodiments of the present disclosure, X represents: 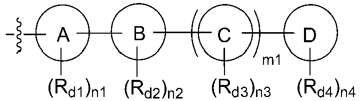
ring A represents nitrogen-containing heterocyclylene (including 4- to 30-membered nitrogen-containing heterocyclylene, 4- to 25-membered nitrogen-containing heterocyclylene, 4- to 20-membered nitrogen-containing heterocyclylene, 4- to 15-membered nitrogen-containing heterocyclylene, and 5- to 20-membered nitrogen-containing heterocyclylene), and (R_{d1})ₙ₁ indicates that the ring A is optionally substituted with n1 R_{d1} groups, wherein each R_{d1} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n1 represents an integer of 0, 1, 2, 3, 4, 5, 6, 7, 8, 9, 10, 11, 12, 13, 14, 15, 16, 17, 18, 19, or 20;
ring B represents nitrogen-containing heterocyclylene (including 4- to 30-membered nitrogen-containing heterocyclylene, 4- to 25-membered nitrogen-containing heterocyclylene, 4- to 20-membered nitrogen-containing heterocyclylene, 4- to 15-membered nitrogen-containing heterocyclylene, and 5- to 20-membered nitrogen-containing heterocyclylene), and (R_{d2})ₙ₂ indicates that the ring B is optionally substituted with n2 R_{d2} groups, wherein each R_{d2} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n2 represents an integer of 0, 1, 2, 3, 4, 5, 6, 7, 8, 9, 10, 11, 12, 13, 14, 15, 16, 17, 18, 19, or 20;
ring C represents cycloalkylene (e.g., C₃₋₃₀ cycloalkylene, C₃₋₂₀ cycloalkylene, or C₃₋₁₅ cycloalkylene), heterocyclylene (e.g., 4- to 30-membered heterocyclylene, 4- to 20-membered heterocyclylene, or 4- to 15-membered heterocyclylene), arylene (e.g., C₅₋₃₀ arylene, C₅₋₂₀ arylene, or C₅₋₁₅ arylene), or heteroarylene (e.g., 5- to 30-membered heteroarylene, 5- to 20-membered heteroarylene, or 5- to 15-membered heteroarylene), m1 represents an integer of 0 or 1, and (R_{d3})ₙ₃ indicates that the ring C is optionally substituted with n3 R_{d3} groups, wherein each R_{d3} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n3 represents an integer of 0, 1, 2, 3, 4, 5, 6, 7, 8, 9, 10, 11, 12, 13, 14, 15, 16, 17, 18, 19, or 20; and
ring D represents heterocyclyl (e.g., 4- to 30-membered heterocyclyl, 4- to 20-membered heterocyclyl, or 4- to 15-membered heterocyclyl), cycloalkyl (e.g., C₃₋₃₀ cycloalkyl, C₃₋₂₀ cycloalkyl, or C₃₋₁₅ cycloalkyl), aryl (e.g., C₅₋₃₀ aryl, C₅₋₂₀ aryl, or C₅₋₁₅ aryl), or heteroaryl (e.g., 5- to 30-membered heteroaryl, 5- to 20-membered heteroaryl, or 5- to 15-membered heteroaryl), and (R_{d4})ₙ₄ indicates that the ring D is optionally substituted with n4 R_{d4} groups, wherein each R_{d4} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n4 represents an integer of 0, 1, 2, 3, 4, 5, 6, 7, 8, 9, 10, 11, 12, 13, 14, 15, 16, 17, 18, 19, or 20.

In some embodiments of the present disclosure, examples of X include but are not limited to:

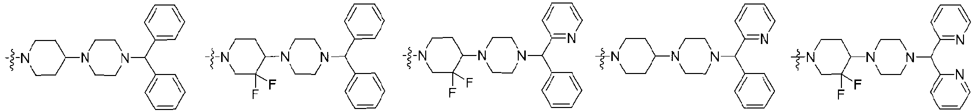

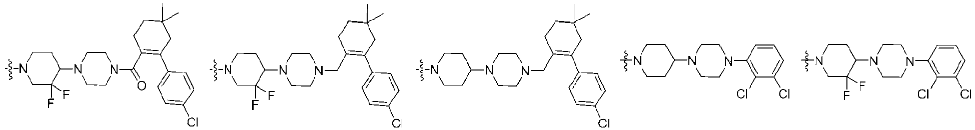

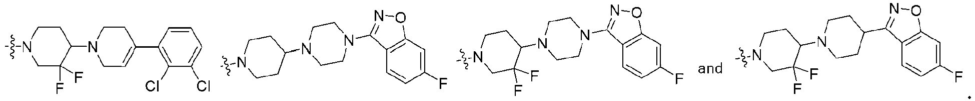

In some embodiments of the present disclosure, the compound of Formula (I) is also represented by Formula (I-1), Formula (I-2), Formula (I-3), or Formula (I-4): 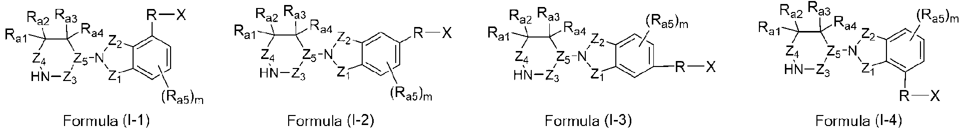 wherein Rₐ₁, Rₐ₂, Rₐ₃, Rₐ₄, Z₁, Z₂, Z₃, Z₄, Z₅, (Rₐ₅)ₘ, R, and X are as defined in the compound of Formula (I) and its various embodiments in the present disclosure.

In some embodiments of the present disclosure, the compound of Formula (I) is also represented by Formula (I-5): 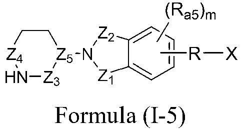 wherein Z₁, Z₂, Z₃, Z₄, Z₅, (Rₐ₅)ₘ, R, and X are as defined in the compound of Formula (I) and its various embodiments in the present disclosure.

In some embodiments of the present disclosure, the compound of Formula (I-5) is also represented by Formula (I-5-1): 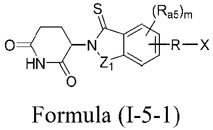 wherein Z₁, (Rₐ₅)ₘ, R, and X are as defined in the compound of Formula (I) and its various embodiments in the present disclosure.

In some embodiments of the present disclosure, the compound of Formula (I-5) is also represented by Formula (I-5-1A): 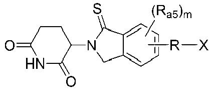 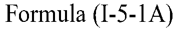 wherein (Rₐ₅)ₘ, R, and X are as defined in the compound of Formula (I) and its various embodiments in the present disclosure.

In some embodiments of the present disclosure, the compound of Formula (I-5) is also represented by Formula (I-5-1B): 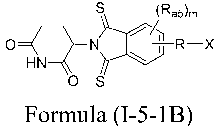 wherein (Rₐ₅)ₘ, R, L, and X are as defined in the compound of Formula (I) and its various embodiments in the present disclosure.

In some embodiments of the present disclosure, the compound of Formula (I-5) is also represented by Formula (I-5-2): 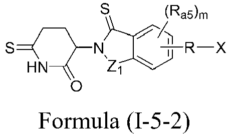 wherein Z₁, (Rₐ₅)ₘ, R, L, and X are as defined in the compound of Formula (I) and its various embodiments in the present disclosure.

In some embodiments of the present disclosure, the compound of Formula (I-5) is also represented by Formula (I-5-2A): 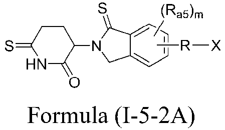 wherein (Rₐ₅)ₘ, R, L, and X are as defined in the compound of Formula (I) and its various embodiments in the present disclosure.

In some embodiments of the present disclosure, the compound of Formula (I-5) is also represented by Formula (I-5-2B): 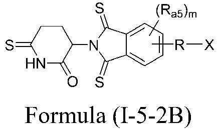 wherein (Rₐ₅)ₘ, R, L, and X are as defined in the compound of Formula (I) and its various embodiments in the present disclosure.

In some embodiments of the present disclosure, the compound of Formula (I-5) is also represented by Formula (I-5-3): 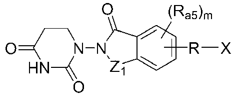 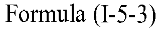 wherein Z₁, (Rₐ₅)ₘ, R, and X are as defined in the compound of Formula (I) and its various embodiments in the present disclosure.

Preferably the compounds of the present invention and their salts (especially pharmaceutically acceptable salts, such as hydrochloride, etc.), enantiomers, diastereomers, solvates, or polymorphs thereof in Table 1 below are provided:

**Table 1. The compounds of the present invention**

| Compo und No. | Structure of the compounds | The compounds' name | Chinese translation of the compounds' name |
|---|---|---|---|
| GT-04329 | 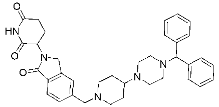 | 3-(5-((4-(4-benzhydrylpiperazin-1-yl)piperidin-1-yl)methyl)-1-oxoisoindolin-2-yl)piperidine-2,6-dione | 3-(5-((4-(4-benzhydrylpiperazin-1-yl)piperidin-1-yl)methyl)-1-oxoisoindolin-2-yl)piperidine-2,6-dione |
| GT-04330 | 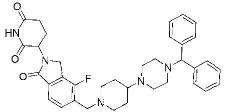 | 3-(5-((4-(4-benzhydrylpiperazin-1-yl)piperidin-1-yl)methyl)-4-fluoro-1-oxoisoindolin-2-yl)piperidine-2,6-dione | 3-(5-((4-(4-benzhydrylpiperazin-1-yl)piperidin-1-yl)methyl)-4-fluoro-1-oxoisoindolin-2-yl)piperidine-2,6-dione |
| GT-04331 | 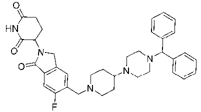 | 3-(5-((4-(4-benzhydrylpiperazin-1-yl)piperidin-1-yl)methyl)-6-fluoro-1-oxoisoindolin-2-yl)piperidine-2,6-dione | 3-(5-((4-(4-benzhydrylpiperazin-1-yl)piperidin-1-yl)methyl)-6-fluoro-1-oxoisoindolin-2-yl)piperidine-2,6-dione |
| GT-04597 | 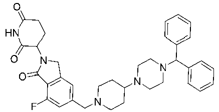 | 3-(5-((4-(4-benzhydrylpiperazin-1-yl)piperidin-1-yl)methyl)-7-fluoro-1-oxoisoindolin-2-yl)piperidine-2,6-dione | 3-(5-((4-(4-benzhydrylpiperazin-1-yl)piperidin-1-yl)methyl)-7-fluoro-1-oxoisoindolin-2-yl)piperidine-2,6-dione |
| GT-04598 | 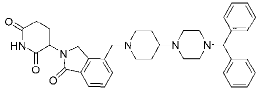 | 3-(4-((4-(4-benzhydrylpiperazin-1-yl)piperidin-1-yl)methyl)-1-oxoisoindolin-2-yl)piperidine-2,6-dione | 3-(4-((4-(4-benzhydrylpiperazin-1-yl)piperidin-1-yl)methyl)-1-oxoisoindolin-2-yl)piperidine-2,6-dione |
| GT-04599 | 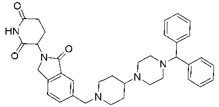 | 3-(6-((4-(4-benzhydrylpiperazin-1-yl)piperidin-1-yl)methyl)-1-oxoisoindolin-2-yl)piperidine-2,6-dione | 3-(6-((4-(4-benzhydrylpiperazin-1-yl)piperidin-1-yl)methyl)-1-oxoisoindolin-2-yl)piperidine-2,6-dione |
| GT-09194 | 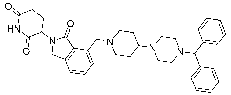 | 3-(7-((4-(4-benzhydrylpiperazin-1-yl)piperidin-1-yl)methyl)-1-oxoisoindolin-2-yl)piperidine-2,6-dione | 3-(7-((4-(4-benzhydrylpiperazin-1-yl)piperidin-1-yl)methyl)-1-oxoisoindolin-2-yl)piperidine-2,6-dione |
| GT-09195 | 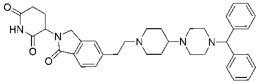 | 3-(5-(2-(4-(4-benzhydrylpiperazin-1-yl)piperidin-1-yl)ethyl)-1-oxoisoindolin-2-yl)piperidine-2,6-dione | 3-(5-(2-(4-(4-benzhydrylpiperazin-1-yl)piperidin-1-yl)ethyl)-1-oxoisoindolin-2-yl)piperidine-2,6-dione |
| GT-09196 | 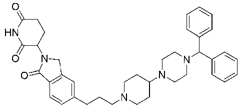 | 3-(5-(3-(4-(4-benzhydrylpiperazin-1-yl)piperidin-1-yl)propyl)-1-oxoisoindolin-2-yl)piperidine-2,6-dione | 3-(5-(3-(4-(4-benzhydrylpiperazin-1-yl)piperidin-1-yl)propyl)-1-oxoisoindolin-2-yl)piperidine-2,6-dione |
| GT-09335 | 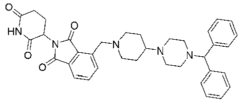 | 4-((4-(4-benzhydrylpiperazin-1-yl)piperidin-1-yl)methyl)-2-(2,6-dioxopiperidin-3-yl)isoindoline-1,3-dione | 4-((4-(4-benzhydrylpiperazin-1-yl)piperidin-1-yl)methyl)-2-(2,6-dioxopiperidin-3-yl)isoindoline-1,3-dione |
| GT-09334 | 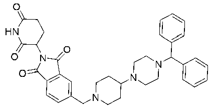 | 5-((4-(4-benzhydrylpiperazin-1-yl)piperidin-1-yl)methyl)-2-(2,6-dioxopiperidin-3-yl)isoindoline-1,3-dione | 5-((4-(4-benzhydrylpiperazin-1-yl)piperidin-1-yl)methyl)-2-(2,6-dioxopiperidin-3-yl)isoindoline-1,3-dione |
| GT-04376 | 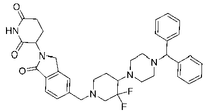 | 3-(5-((4-(4-benzhydrylpiperazin-1-yl)-3,3-difluoropiperidin-1-yl)methyl)-1-oxoisoindolin-2-yl)piperidine-2,6-dione | 3-(5-((4-(4-benzhydrylpiperazin-1-yl)-3,3-difluoropiperidin-1-yl)methyl)-1-oxoisoindolin-2-yl)piperidine-2,6-dione |
| GT-04377 | 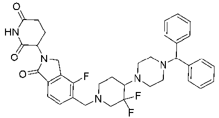 | 3-(5-((4-(4-benzhydrylpiperazin-1-yl)-3,3-difluoropiperidin-1-yl)methyl)-4-fluoro-1-oxoisoindolin-2-yl)piperidine-2,6-dione | 3-(5-((4-(4-benzhydrylpiperazin-1-yl)-3,3-difluoropiperidin-1-yl)methyl)-4-fluoro-1-oxoisoindolin-2-yl)piperidine-2,6-dione |
| GT-04378 | 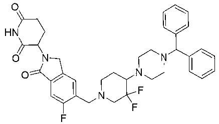 | 3-(5-((4-(4-benzhydrylpiperazin-1-yl)-3,3-difluoropiperidin-1-yl)methyl)-6-fluoro-1-oxoisoindolin-2-yl)piperidine-2,6-dione | 3-(5-((4-(4-benzhydrylpiperazin-1-yl)-3,3-difluoropiperidin-1-yl)methyl)-6-fluoro-1-oxoisoindolin-2-yl)piperidine-2,6-dione |
| GT-04379 | 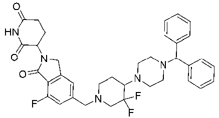 | 3-(5-((4-(4-benzhydrylpiperazin-1-yl)-3,3-difluoropiperidin-1-yl)methyl)-7-fluoro-1-oxoisoindolin-2-yl)piperidine-2,6-dione | 3-(5-((4-(4-benzhydrylpiperazin-1-yl)-3,3-difluoropiperidin-1-yl)methyl)-7-fluoro-1-oxoisoindolin-2-yl)piperidine-2,6-dione |
| GT-04380 | 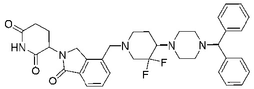 | 3-(4-((4-(4-benzhydrylpiperazin-1-yl)-3,3-difluoropiperidin-1-yl)methyl)-1-oxoisoindolin-2-yl)piperidine-2,6-dione | 3-(4-((4-(4-benzhydrylpiperazin-1-yl)-3,3-difluoropiperidin-1-yl)methyl)-1-oxoisoindolin-2-yl)piperidine-2,6-dione |
| GT-09188 | 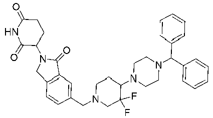 | 3-(6-((4-(4-benzhydrylpiperazin-1-yl)-3,3-difluoropiperidin-1-yl)methyl)-1-oxoisoindolin-2-yl)piperidine-2,6-dione | 3-(6-((4-(4-benzhydrylpiperazin-1-yl)-3,3-difluoropiperidin-1-yl)methyl)-1-oxoisoindolin-2-yl)piperidine-2,6-dione |
| GT-09189 | 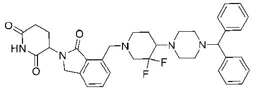 | 3-(7-((4-(4-benzhydrylpiperazin-1-yl)-3,3-difluoropiperidin-1-yl)methyl)-1-oxoisoindolin-2-yl)piperidine-2,6-dione | 3-(7-((4-(4-benzhydrylpiperazin-1-yl)-3,3-difluoropiperidin-1-yl)methyl)-1-oxoisoindolin-2-yl)piperidine-2,6-dione |
| GT-09190 | 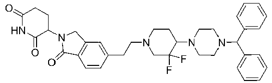 | 3-(5-(2-(4-(4-benzhydrylpiperazin-1-yl)-3,3-difluoropiperidin-1-yl)ethyl)-1-oxoisoindolin-2-yl)piperidine-2,6-dione | 3-(5-(2-(4-(4-benzhydrylpiperazin-1-yl)-3,3-difluoropiperidin-1-yl)ethyl)-1-oxoisoindolin-2-yl)piperidine-2,6-dione |
| GT-09191 | 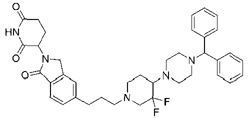 | 3-(5-(3-(4-(4-benzhydrylpiperazin-1-yl)-3,3-difluoropiperidin-1-yl)propyl)-1-oxoisoindolin-2-yl)piperidine-2,6-dione | 3-(5-(3-(4-(4-benzhydrylpiperazin-1-yl)-3,3-difluoropiperidin-1-yl)propyl)-1-oxoisoindolin-2-yl)piperidine-2,6-dione |
| GT-09193 | 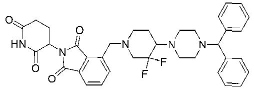 | 4-((4-(4-benzhydrylpiperazin-1-yl)-3,3-difluoropiperidin-1-yl)methyl)-2-(2,6-dioxopiperidin-3-yl)isoindoline-1,3-dione | 4-((4-(4-benzhydrylpiperazin-1-yl)-3,3-difluoropiperidin-1-yl)methyl)-2-(2,6-dioxopiperidin-3-yl)isoindoline-1,3-dione |
| GT-09192 | 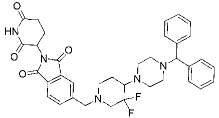 | 5-((4-(4-benzhydrylpiperazin-1-yl)-3,3-difluoropiperidin-1-yl)methyl)-2-(2,6-dioxopiperidin-3-yl)isoindoline-1,3-dione | 5-((4-(4-benzhydrylpiperazin-1-yl)-3,3-difluoropiperidin-1-yl)methyl)-2-(2,6-dioxopiperidin-3-yl)isoindoline-1,3-dione |
| GT-05037 | 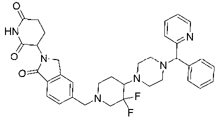 | 3-(5-((3,3-difluoro-4-(4-(phenyl(pyridin-2-yl)methyl)piperazin-1-yl)piperidin-1-yl)methyl)-1-oxoisoindolin-2-yl)piperidine-2,6-dione | 3-(5-((3,3-difluoro-4-(4-(phenyl(pyridin-2-yl)methyl)piperazin-1-yl)piperidin-1-yl)methyl)-1-oxoisoindolin-2-yl)piperidine-2,6-dione |
| GT-09336 | 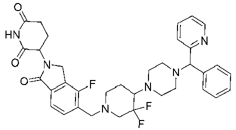 | 3-(5-((3,3-difluoro-4-(4-(phenyl(pyridin-2-yl)methyl)piperazin-1-yl)piperidin-1-yl)methyl)-4-fluoro-1-oxoisoindolin-2-yl)piperidine-2,6-dione | 3-(5-((3,3-difluoro-4-(4-(phenyl(pyridin-2-yl)methyl)piperazin-1-yl)piperidin-1-yl)methyl)-4-fluoro-1-oxoisoindolin-2-yl)piperidine-2,6-dione |
| GT-09337 | 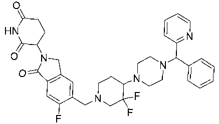 | 3-(5-((3,3-difluoro-4-(4-(phenyl(pyridin-2-yl)methyl)piperazin-1-yl)piperidin-1-yl)methyl)-6-fluoro-1-oxoisoindolin-2-yl)piperidine-2,6-dione | 3-(5-((3,3-difluoro-4-(4-(phenyl(pyridin-2-yl)methyl)piperazin-1-yl)piperidin-1-yl)methyl)-6-fluoro-1-oxoisoindolin-2-yl)piperidine-2,6-dione |
| GT-09338 | 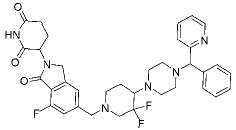 | 3-(5-((3,3-difluoro-4-(4-(phenyl(pyridin-2-yl)methyl)piperazin-1-yl)piperidin-1-yl)methyl)-7-fluoro-1-oxoisoindolin-2-yl)piperidine-2,6-dione | 3-(5-((3,3-difluoro-4-(4-(phenyl(pyridin-2-yl)methyl)piperazin-1-yl)piperidin-1-yl)methyl)-7-fluoro-1-oxoisoindolin-2-yl)piperidine-2,6-dione |
| GT-09339 | 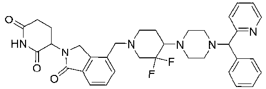 | 3-(4-((3,3-difluoro-4-(4-(phenyl(pyridin-2-yl)methyl)piperazin-1-yl)piperidin-1-yl)methyl)-1-oxoisoindolin-2-yl)piperidine-2,6-dione | 3-(4-((3,3-difluoro-4-(4-(phenyl(pyridin-2-yl)methyl)piperazin-1-yl)piperidin-1-yl)methyl)-1-oxoisoindolin-2-yl)piperidine-2,6-dione |
| GT-09340 | 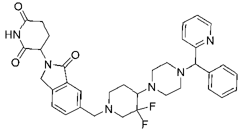 | 3-(6-((3,3-difluoro-4-(4-(phenyl(pyridin-2-yl)methyl)piperazin-1-yl)piperidin-1-yl)methyl)-1-oxoisoindolin-2-yl)piperidine-2,6-dione | 3-(6-((3,3-difluoro-4-(4-(phenyl(pyridin-2-yl)methyl)piperazin-1-yl)piperidin-1-yl)methyl)-1-oxoisoindolin-2-yl)piperidine-2,6-dione |
| GT-09341 | 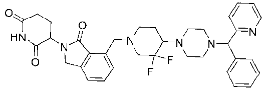 | 3-(7-((3,3-difluoro-4-(4-(phenyl(pyridin-2-yl)methyl)piperazin-1-yl)piperidin-1-yl)methyl)-1-oxoisoindolin-2-yl)piperidine-2,6-dione | 3-(7-((3,3-difluoro-4-(4-(phenyl(pyridin-2-yl)methyl)piperazin-1-yl)piperidin-1-yl)methyl)-1-oxoisoindolin-2-yl)piperidine-2,6-dione |
| GT-09342 | 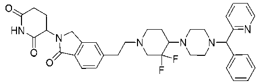 | 3-(5-(2-(3,3-difluoro-4-(4-(phenyl(pyridin-2-yl)methyl)piperazin-1-yl)piperidin-1-yl)ethyl)-1-oxoisoindolin-2-yl)piperidine-2,6-dione | 3-(5-(2-(3,3-difluoro-4-(4-(phenyl(pyridin-2-yl)methyl)piperazin-1-yl)piperidin-1-yl)ethyl)-1-oxoisoindolin-2-yl)piperidine-2,6-dione |
| GT-09343 | 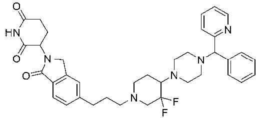 | 3-(5-(3-(3,3-difluoro-4-(4-(phenyl(pyridin-2-yl)methyl)piperazin-1-yl)piperidin-1-yl)propyl)-1-oxoisoindolin-2-yl)piperidine-2,6-dione | 3-(5-(3-(3,3-difluoro-4-(4-(phenyl(pyridin-2-yl)methyl)piperazin-1-yl)piperidin-1-yl)propyl)-1-oxoisoindolin-2-yl)piperidine-2,6-dione |
| GT-09345 | 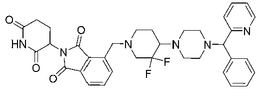 | 4-((3,3-difluoro-4-(4-(phenyl(pyridin-2-yl)methyl)piperazin-1-yl)piperidin-1-yl)methyl)-2-(2,6-dioxopiperidin-3-yl)isoindoline-1,3-dione | 4-((3,3-difluoro-4-(4-(phenyl(pyridin-2-yl)methyl)piperazin-1-yl)piperidin-1-yl)methyl)-2-(2,6-dioxopiperidin-3-yl)isoindoline-1,3-dione |
| GT-09344 | 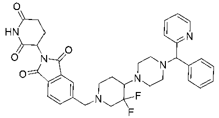 | 5-((3,3-difluoro-4-(4-(phenyl(pyridin-2-yl)methyl)piperazin-1-yl)piperidin-1-yl)methyl)-2-(2,6-dioxopiperidin-3-yl)isoindoline-1,3-dione | 5-((3,3-difluoro-4-(4-(phenyl(pyridin-2-yl)methyl)piperazin-1-yl)piperidin-1-yl)methyl)-2-(2,6-dioxopiperidin-3-yl)isoindoline-1,3-dione |
| GT-04595 | 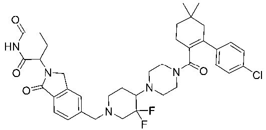 | 3-(5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-oxoisoindolin-2-yl)piperidine-2,6-dione | 3-(5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-oxoisoindolin-2-yl)piperidine-2,6-dione |
| GT-04596 | 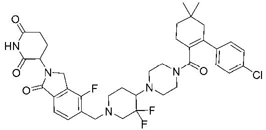 | 3-(5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-4-fluoro-1-oxoisoindolin-2-yl)piperidine-2,6-dione | 3-(5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-4-fluoro-1-oxoisoindolin-2-yl)piperidine-2,6-dione |
| GT-09346 | 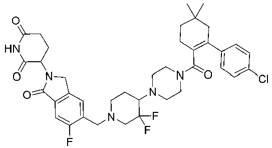 | 3-(5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-6-fluoro-1-oxoisoindolin-2-yl)piperidine-2,6-dione | 3-(5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-6-fluoro-1-oxoisoindolin-2-yl)piperidine-2,6-dione |
| GT-09347 | 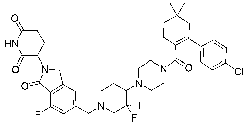 | 3-(5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-7-fluoro-1-oxoisoindolin-2-yl)piperidine-2,6-dione | 3-(5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-7-fluoro-1-oxoisoindolin-2-yl)piperidine-2,6-dione |
| GT-09348 | 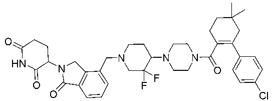 | 3-(4-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-oxoisoindolin-2-yl)piperidine-2,6-dione | 3-(4-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-oxoisoindolin-2-yl)piperidine-2,6-dione |
| GT-09349 | 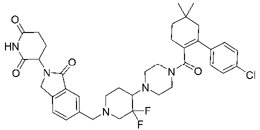 | 3-(6-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-oxoisoindolin-2-yl)piperidine-2,6-dione | 3-(6-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-oxoisoindolin-2-yl)piperidine-2,6-dione |
| GT-09350 | 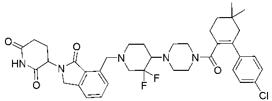 | 3-(7-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-oxoisoindolin-2-yl)piperidine-2,6-dione | 3-(7-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-oxoisoindolin-2-yl)piperidine-2,6-dione |
| GT-09351 | 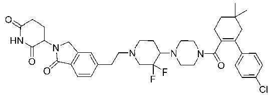 | 3-(5-(2-(4-(4-(4'-chloro-5,5-dimethyl-3,4,5,6-tetrahydro-[1,1'-biphenyl]-2-carbonyl)piperazin-1-yl)-3,3-difluoropiperidin-1-yl)ethyl)-1-oxoisoindolin-2-yl)piperidine-2,6-dione | 3-(5-(2-(4-(4-(4'-chloro-5,5-dimethyl-3,4,5,6-tetrahydro-[1,1'-biphenyl]-2-carbonyl)piperazin-1-yl)-3,3-difluoropiperidin-1-yl)ethyl)-1-oxoisoindolin-2-yl)piperidine-2,6-dione |
| GT-09378 | 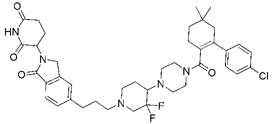 | 3-(5-(3-(4-(4-(4'-chloro-5,5-dimethyl-3,4,5,6-tetrahydro-[1,1'-biphenyl]-2-carbonyl)piperazin-1-yl)-3,3-difluoropiperidin-1-yl)propyl)-1-oxoisoindolin-2-yl)piperidine-2,6-dione | 3-(5-(3-(4-(4-(4'-chloro-5,5-dimethyl-3,4,5,6-tetrahydro-[1,1'-biphenyl]-2-carbonyl)piperazin-1-yl)-3,3-difluoropiperidin-1-yl)propyl)-1-oxoisoindolin-2-yl)piperidine-2,6-dione |
| GT-09352 | 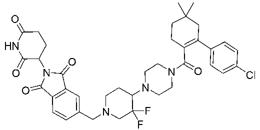 | 5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-2-(2,6-dioxopiperidin-3-yl)isoindoline-1,3-dione | 5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-2-(2,6-dioxopiperidin-3-yl)isoindoline-1,3-dione |
| GT-09353 | 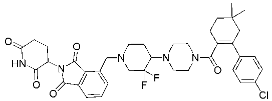 | 4-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-2-(2,6-dioxopiperidin-3-yl)isoindoline-1,3-dione | 4-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-2-(2,6-dioxopiperidin-3-yl)isoindoline-1,3-dione |
| GT-05160 | 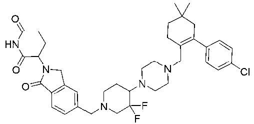 | 3-(5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-oxoisoindolin-2-yl)piperidine-2,6-dione | 3-(5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-oxoisoindolin-2-yl)piperidine-2,6-dione |
| GT-09379 | 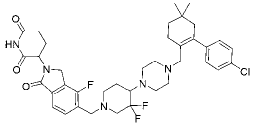 | 3-(5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-4-fluoro-1-oxoisoindolin-2-yl)piperidine-2,6-dione | 3-(5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-4-fluoro-1-oxoisoindolin-2-yl)piperidine-2,6-dione |
| GT-09380 | 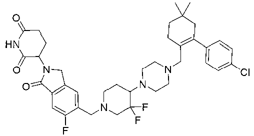 | 3-(5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-6-fluoro-1-oxoisoindolin-2-yl)piperidine-2,6-dione | 3-(5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-6-fluoro-1-oxoisoindolin-2-yl)piperidine-2,6-dione |
| GT-09381 | 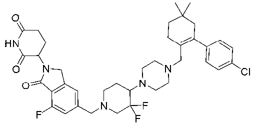 | 3-(5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-7-fluoro-1-oxoisoindolin-2-yl)piperidine-2,6-dione | 3-(5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-7-fluoro-1-oxoisoindolin-2-yl)piperidine-2,6-dione |
| GT-09382 | 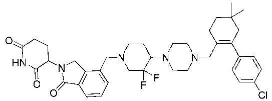 | 3-(4-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-oxoisoindolin-2-yl)piperidine-2,6-dione | 3-(4-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-oxoisoindolin-2-yl)piperidine-2,6-dione |
| GT-09383 | 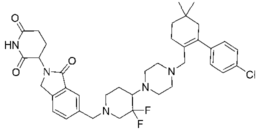 | 3-(6-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-oxoisoindolin-2-yl)piperidine-2,6-dione | 3-(6-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-oxoisoindolin-2-yl)piperidine-2,6-dione |
| GT-09384 | 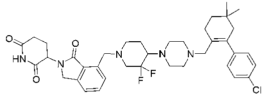 | 3-(7-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-oxoisoindolin-2-yl)piperidine-2,6-dione | 3-(7-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-oxoisoindolin-2-yl)piperidine-2,6-dione |
| GT-09385 | 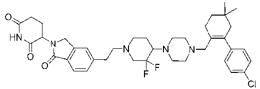 | 3-(5-(2-(4-(4-((4'-chloro-5,5-dimethyl-3,4,5,6-tetrahydro-[1,1'-biphenyl]-2-yl)methyl)piperazin-1-yl)-3,3-difluoropiperidin-1-yl)ethyl)-1-oxoisoindolin-2-yl)piperidine-2,6-dione | 3-(5-(2-(4-(4-((4'-chloro-5,5-dimethyl-3,4,5,6-tetrahydro-[1,1'-biphenyl]-2-yl)methyl)piperazin-1-yl)-3,3-difluoropiperidin-1-yl)ethyl)-1-oxoisoindolin-2-yl)piperidine-2,6-dione |
| GT-09386 | 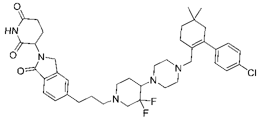 | 3-(5-(3-(4-(4-((4'-chloro-5,5-dimethyl-3,4,5,6-tetrahydro-[1,1'-biphenyl]-2-yl)methyl)piperazin-1-yl)-3,3-difluoropiperidin-1-yl)propyl)-1-oxoisoindolin-2-yl)piperidine-2,6-dione | 3-(5-(3-(4-(4-((4'-chloro-5,5-dimethyl-3,4,5,6-tetrahydro-[1,1'-biphenyl]-2-yl)methyl)piperazin-1-yl)-3,3-difluoropiperidin-1-yl)propyl)-1-oxoisoindolin-2-yl)piperidine-2,6-dione |
| GT-09387 | 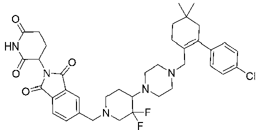 | 5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-2-(2,6-dioxopiperidin-3-yl)isoindoline-1,3-dione | 5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-2-(2,6-dioxopiperidin-3-yl)isoindoline-1,3-dione |
| GT-09388 | 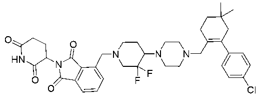 | 4-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-2-(2,6-dioxopiperidin-3-yl)isoindoline-1,3-dione | 4-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-2-(2,6-dioxopiperidin-3-yl)isoindoline-1,3-dione |
| GT-04604 | 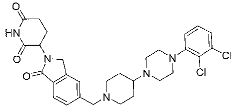 | 3-(5-((4-(4-(2,3-dichlorophenyl)piperazin-1-yl)piperidin-1-yl)methyl)-1-oxoisoindolin-2-yl)piperidine-2,6-dione | 3-(5-((4-(4-(2,3-dichlorophenyl)piperazin-1-yl)piperidin-1-yl)methyl)-1-oxoisoindolin-2-yl)piperidine-2,6-dione |
| GT-04605 | 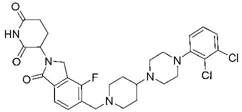 | 3-(5-((4-(4-(2,3-dichlorophenyl)piperazin-1-yl)piperidin-1-yl)methyl)-4-fluoro-1-oxoisoindolin-2-yl)piperidine-2,6-dione | 3-(5-((4-(4-(2,3-dichlorophenyl)piperazin-1-yl)piperidin-1-yl)methyl)-4-fluoro-1-oxoisoindolin-2-yl)piperidine-2,6-dione |
| GT-04606 | 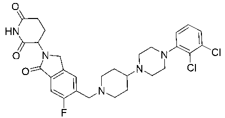 | 3-(5-((4-(4-(2,3-dichlorophenyl)piperazin-1-yl)piperidin-1-yl)methyl)-6-fluoro-1-oxoisoindolin-2-yl)piperidine-2,6-dione | 3-(5-((4-(4-(2,3-dichlorophenyl)piperazin-1-yl)piperidin-1-yl)methyl)-6-fluoro-1-oxoisoindolin-2-yl)piperidine-2,6-dione |
| GT-04607 | 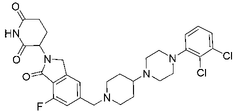 | 3-(5-((4-(4-(2,3-dichlorophenyl)piperazin-1-yl)piperidin-1-yl)methyl)-7-fluoro-1-oxoisoindolin-2-yl)piperidine-2,6-dione | 3-(5-((4-(4-(2,3-dichlorophenyl)piperazin-1-yl)piperidin-1-yl)methyl)-7-fluoro-1-oxoisoindolin-2-yl)piperidine-2,6-dione |
| GT-04609 | 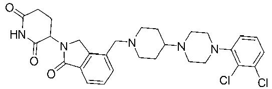 | 3-(4-((4-(4-(2,3-dichlorophenyl)piperazin-1-yl)piperidin-1-yl)methyl)-1-oxoisoindolin-2-yl)piperidine-2,6-dione | 3-(4-((4-(4-(2,3-dichlorophenyl)piperazin-1-yl)piperidin-1-yl)methyl)-1-oxoisoindolin-2-yl)piperidine-2,6-dione |
| GT-04608 | 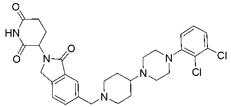 | 3-(6-((4-(4-(2,3-dichlorophenyl)piperazin-1-yl)piperidin-1-yl)methyl)-1-oxoisoindolin-2-yl)piperidine-2,6-dione | 3-(6-((4-(4-(2,3-dichlorophenyl)piperazin-1-yl)piperidin-1-yl)methyl)-1-oxoisoindolin-2-yl)piperidine-2,6-dione |
| GT-09389 | 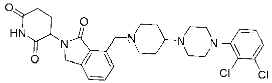 | 3-(7-((4-(4-(2,3-dichlorophenyl)piperazin-1-yl)piperidin-1-yl)methyl)-1-oxoisoindolin-2-yl)piperidine-2,6-dione | 3-(7-((4-(4-(2,3-dichlorophenyl)piperazin-1-yl)piperidin-1-yl)methyl)-1-oxoisoindolin-2-yl)piperidine-2,6-dione |
| GT-09390 | 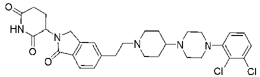 | 3-(5-(2-(4-(4-(2,3-dichlorophenyl)piperazin-1-yl)piperidin-1-yl)ethyl)-1-oxoisoindolin-2-yl)piperidine-2,6-dione | 3-(5-(2-(4-(4-(2,3-dichlorophenyl)piperazin-1-yl)piperidin-1-yl)ethyl)-1-oxoisoindolin-2-yl)piperidine-2,6-dione |
| GT-09391 | 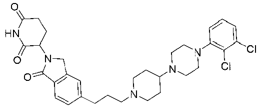 | 3-(5-(3-(4-(4-(2,3-dichlorophenyl)piperazin-1-yl)piperidin-1-yl)propyl)-1-oxoisoindolin-2-yl)piperidine-2,6-dione | 3-(5-(3-(4-(4-(2,3-dichlorophenyl)piperazin-1-yl)piperidin-1-yl)propyl)-1-oxoisoindolin-2-yl)piperidine-2,6-dione |
| GT-09393 | 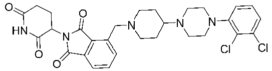 | 4-((4-(4-(2,3-dichlorophenyl)piperazin-1-yl)piperidin-1-yl)methyl)-2-(2,6-dioxopiperidin-3-yl)isoindoline-1,3-dione | 4-((4-(4-(2,3-dichlorophenyl)piperazin-1-yl)piperidin-1-yl)methyl)-2-(2,6-dioxopiperidin-3-yl)isoindoline-1,3-dione |
| GT-09392 | 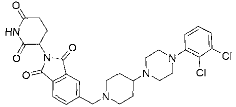 | 5-((4-(4-(2,3-dichlorophenyl)piperazin-1-yl)piperidin-1-yl)methyl)-2-(2,6-dioxopiperidin-3-yl)isoindoline-1,3-dione | 5-((4-(4-(2,3-dichlorophenyl)piperazin-1-yl)piperidin-1-yl)methyl)-2-(2,6-dioxopiperidin-3-yl)isoindoline-1,3-dione |
| GT-05212 | 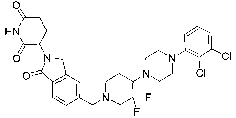 | 3-(5-((4-(4-(2,3-dichlorophenyl)piperazin-1-yl)-3,3-difluoropiperidin-1-yl)methyl)-1-oxoisoindolin-2-yl)piperidine-2,6-dione | 3-(5-((4-(4-(2,3-dichlorophenyl)piperazin-1-yl)-3,3-difluoropiperidin-1-yl)methyl)-1-oxoisoindolin-2-yl)piperidine-2,6-dione |
| GT-09417 | 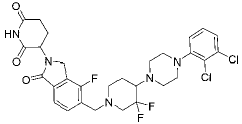 | 3-(5-((4-(4-(2,3-dichlorophenyl)piperazin-1-yl)-3,3-difluoropiperidin-1-yl)methyl)-4-fluoro-1-oxoisoindolin-2-yl)piperidine-2,6-dione | 3-(5-((4-(4-(2,3-dichlorophenyl)piperazin-1-yl)-3,3-difluoropiperidin-1-yl)methyl)-4-fluoro-1-oxoisoindolin-2-yl)piperidine-2,6-dione |
| GT-09418 | 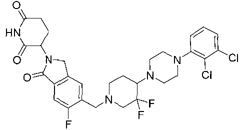 | 3-(5-((4-(4-(2,3-dichlorophenyl)piperazin-1-yl)-3,3-difluoropiperidin-1-yl)methyl)-6-fluoro-1-oxoisoindolin-2-yl)piperidine-2,6-dione | 3-(5-((4-(4-(2,3-dichlorophenyl)piperazin-1-yl)-3,3-difluoropiperidin-1-yl)methyl)-6-fluoro-1-oxoisoindolin-2-yl)piperidine-2,6-dione |
| GT-09419 | 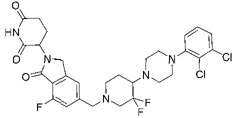 | 3-(5-((4-(4-(2,3-dichlorophenyl)piperazin-1-yl)-3,3-difluoropiperidin-1-yl)methyl)-7-fluoro-1-oxoisoindolin-2-yl)piperidine-2,6-dione | 3-(5-((4-(4-(2,3-dichlorophenyl)piperazin-1-yl)-3,3-difluoropiperidin-1-yl)methyl)-7-fluoro-1-oxoisoindolin-2-yl)piperidine-2,6-dione |
| GT-09420 | 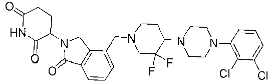 | 3-(4-((4-(4-(2,3-dichlorophenyl)piperazin-1-yl)-3,3-difluoropiperidin-1-yl)methyl)-1-oxoisoindolin-2-yl)piperidine-2,6-dione | 3-(4-((4-(4-(2,3-dichlorophenyl)piperazin-1-yl)-3,3-difluoropiperidin-1-yl)methyl)-1-oxoisoindolin-2-yl)piperidine-2,6-dione |
| GT-09421 | 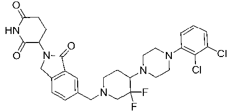 | 3-(6-((4-(4-(2,3-dichlorophenyl)piperazin-1-yl)-3,3-difluoropiperidin-1-yl)methyl)-1-oxoisoindolin-2-yl)piperidine-2,6-dione | 3-(6-((4-(4-(2,3-dichlorophenyl)piperazin-1-yl)-3,3-difluoropiperidin-1-yl)methyl)-1-oxoisoindolin-2-yl)piperidine-2,6-dione |
| GT-09422 | 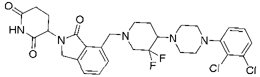 | 3-(7-((4-(4-(2,3-dichlorophenyl)piperazin-1-yl)-3,3-difluoropiperidin-1-yl)methyl)-1-oxoisoindolin-2-yl)piperidine-2,6-dione | 3-(7-((4-(4-(2,3-dichlorophenyl)piperazin-1-yl)-3,3-difluoropiperidin-1-yl)methyl)-1-oxoisoindolin-2-yl)piperidine-2,6-dione |
| GT-09430 | 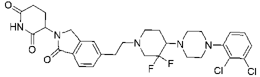 | 3-(5-(2-(4-(4-(2,3-dichlorophenyl)piperazin-1-yl)-3,3-difluoropiperidin-1-yl)ethyl)-1-oxoisoindolin-2-yl)piperidine-2,6-dione | 3-(5-(2-(4-(4-(2,3-dichlorophenyl)piperazin-1-yl)-3,3-difluoropiperidin-1-yl)ethyl)-1-oxoisoindolin-2-yl)piperidine-2,6-dione |
| GT-09431 | 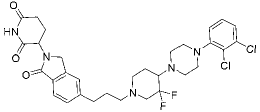 | 3-(5-(3-(4-(4-(2,3-dichlorophenyl)piperazin-1-yl)-3,3-difluoropiperidin-1-yl)propyl)-1-oxoisoindolin-2-yl)piperidine-2,6-dione | 3-(5-(3-(4-(4-(2,3-dichlorophenyl)piperazin-1-yl)-3,3-difluoropiperidin-1-yl)propyl)-1-oxoisoindolin-2-yl)piperidine-2,6-dione |
| GT-09433 | 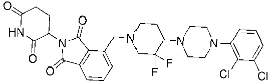 | 4-((4-(4-(2,3-dichlorophenyl)piperazin-1-yl)-3,3-difluoropiperidin-1-yl)methyl)-2-(2,6-dioxopiperidin-3-yl)isoindoline-1,3-dione | 4-((4-(4-(2,3-dichlorophenyl)piperazin-1-yl)-3,3-difluoropiperidin-1-yl)methyl)-2-(2,6-dioxopiperidin-3-yl)isoindoline-1,3-dione |
| GT-09432 | 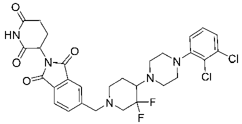 | 5-((4-(4-(2,3-dichlorophenyl)piperazin-1-yl)-3,3-difluoropiperidin-1-yl)methyl)-2-(2,6-dioxopiperidin-3-yl)isoindoline-1,3-dione | 5-((4-(4-(2,3-dichlorophenyl)piperazin-1-yl)-3,3-difluoropiperidin-1-yl)methyl)-2-(2,6-dioxopiperidin-3-yl)isoindoline-1,3-dione |
| GT-05333 | 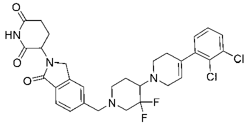 | 3-(5-((4-(4-(2,3-dichlorophenyl)-3,6-dihydropyridin-1(2H)-yl)-3,3-difluoropiperidin-1-yl)methyl)-1-oxoisoindolin-2-yl)piperidine-2,6-dione | 3-(5-((4-(4-(2,3-dichlorophenyl)-3,6-dihydropyridin-1(2H)-yl)-3,3-difluoropiperidin-1-yl)methyl)-1-oxoisoindolin-2-yl)piperidine-2,6-dione |
| GT-09599 | 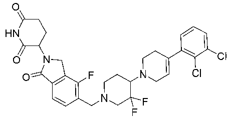 | 3-(5-((4-(4-(2,3-dichlorophenyl)-3,6-dihydropyridin-1(2H)-yl)-3,3-difluoropiperidin-1-yl)methyl)-4-fluoro-1-oxoisoindolin-2-yl)piperidine-2,6-dione | 3-(5-((4-(4-(2,3-dichlorophenyl)-3,6-dihydropyridin-1(2H)-yl)-3,3-difluoropiperidin-1-yl)methyl)-4-fluoro-1-oxoisoindolin-2-yl)piperidine-2,6-dione |
| GT-09600 | 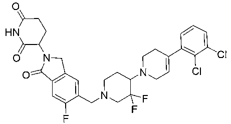 | 3-(5-((4-(4-(2,3-dichlorophenyl)-3,6-dihydropyridin-1(2H)-yl)-3,3-difluoropiperidin-1-yl)methyl)-6-fluoro-1-oxoisoindolin-2-yl)piperidine-2,6-dione | 3-(5-((4-(4-(2,3-dichlorophenyl)-3,6-dihydropyridin-1(2H)-yl)-3,3-difluoropiperidin-1-yl)methyl)-6-fluoro-1-oxoisoindolin-2-yl)piperidine-2,6-dione |
| GT-09601 | 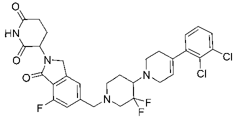 | 3-(5-((4-(4-(2,3-dichlorophenyl)-3,6-dihydropyridin-1(2H)-yl)-3,3-difluoropiperidin-1-yl)methyl)-7-fluoro-1-oxoisoindolin-2-yl)piperidine-2,6-dione | 3-(5-((4-(4-(2,3-dichlorophenyl)-3,6-dihydropyridin-1(2H)-yl)-3,3-difluoropiperidin-1-yl)methyl)-7-fluoro-1-oxoisoindolin-2-yl)piperidine-2,6-dione |
| GT-09602 | 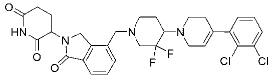 | 3-(4-((4-(4-(2,3-dichlorophenyl)-3,6-dihydropyridin-1(2H)-yl)-3,3-difluoropiperidin-1-yl)methyl)-1-oxoisoindolin-2-yl)piperidine-2,6-dione | 3-(4-((4-(4-(2,3-dichlorophenyl)-3,6-dihydropyridin-1(2H)-yl)-3,3-difluoropiperidin-1-yl)methyl)-1-oxoisoindolin-2-yl)piperidine-2,6-dione |
| GT-09635 | 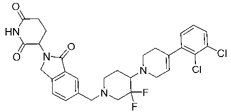 | 3-(6-((4-(4-(2,3-dichlorophenyl)-3,6-dihydropyridin-1(2H)-yl)-3,3-difluoropiperidin-1-yl)methyl)-1-oxoisoindolin-2-yl)piperidine-2,6-dione | 3-(6-((4-(4-(2,3-dichlorophenyl)-3,6-dihydropyridin-1(2H)-yl)-3,3-difluoropiperidin-1-yl)methyl)-1-oxoisoindolin-2-yl)piperidine-2,6-dione |
| GT-09636 | 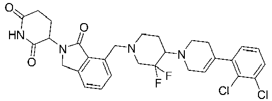 | 3-(7-((4-(4-(2,3-dichlorophenyl)-3,6-dihydropyridin-1(2H)-yl)-3,3-difluoropiperidin-1-yl)methyl)-1-oxoisoindolin-2-yl)piperidine-2,6-dione | 3-(7-((4-(4-(2,3-dichlorophenyl)-3,6-dihydropyridin-1(2H)-yl)-3,3-difluoropiperidin-1-yl)methyl)-1-oxoisoindolin-2-yl)piperidine-2,6-dione |
| GT-09637 | 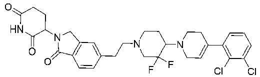 | 3-(5-(2-(4-(4-(2,3-dichlorophenyl)-3,6-dihydropyridin-1(2H)-yl)-3,3-difluoropiperidin-1-yl)ethyl)-1-oxoisoindolin-2-yl)piperidine-2,6-dione | 3-(5-(2-(4-(4-(2,3-dichlorophenyl)-3,6-dihydropyridin-1(2H)-yl)-3,3-difluoropiperidin-1-yl)ethyl)-1-oxoisoindolin-2-yl)piperidine-2,6-dione |
| GT-09638 | 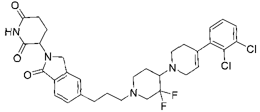 | 3-(5-(3-(4-(4-(2,3-dichlorophenyl)-3,6-dihydropyridin-1(2H)-yl)-3,3-difluoropiperidin-1-yl)propyl)-1-oxoisoindolin-2-yl)piperidine-2,6-dione | 3-(5-(3-(4-(4-(2,3-dichlorophenyl)-3,6-dihydropyridin-1(2H)-yl)-3,3-difluoropiperidin-1-yl)propyl)-1-oxoisoindolin-2-yl)piperidine-2,6-dione |
| GT-09640 | 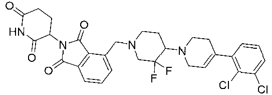 | 4-((4-(4-(2,3-dichlorophenyl)-3,6-dihydropyridin-1(2H)-yl)-3,3-difluoropiperidin-1-yl)methyl)-2-(2,6-dioxopiperidin-3-yl)isoindoline-1,3-dione | 4-((4-(4-(2,3-dichlorophenyl)-3,6-dihydropyridin-1(2H)-yl)-3,3-difluoropiperidin-1-yl)methyl)-2-(2,6-dioxopiperidin-3-yl)isoindoline-1,3-dione |
| GT-09639 | 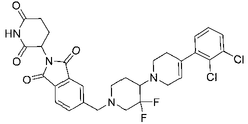 | 5-((4-(4-(2,3-dichlorophenyl)-3,6-dihydropyridin-1(2H)-yl)-3,3-difluoropiperidin-1-yl)methyl)-2-(2,6-dioxopiperidin-3-yl)isoindoline-1,3-dione | 5-((4-(4-(2,3-dichlorophenyl)-3,6-dihydropyridin-1(2H)-yl)-3,3-difluoropiperidin-1-yl)methyl)-2-(2,6-dioxopiperidin-3-yl)isoindoline-1,3-dione |
| GT-04989 | 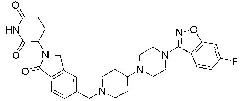 | 3-(5-((4-(4-(6-fluorobenzo[d]isoxazol-3-yl)piperazin-1-yl)piperidin-1-yl)methyl)-1-oxoisoindolin-2-yl)piperidine-2,6-dione | 3-(5-((4-(4-(6-fluorobenzo[d]isoxazol-3-yl)piperazin-1-yl)piperidin-1-yl)methyl)-1-oxoisoindolin-2-yl)piperidine-2,6-dione |
| GT-09641 | 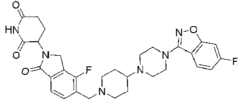 | 3-(4-fluoro-5-((4-(4-(6-fluorobenzo[d]isoxazol-3-yl)piperazin-1-yl)piperidin-1-yl)methyl)-1-oxoisoindolin-2-yl)piperidine-2,6-dione | 3-(4-fluoro-5-((4-(4-(6-fluorobenzo[d]isoxazol-3-yl)piperazin-1-yl)piperidin-1-yl)methyl)-1-oxoisoindolin-2-yl)piperidine-2,6-dione |
| GT-09642 | 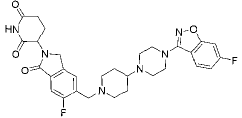 | 3-(6-fluoro-5-((4-(4-(6-fluorobenzo[d]isoxazol-3-yl)piperazin-1-yl)piperidin-1-yl)methyl)-1-oxoisoindolin-2-yl)piperidine-2,6-dione | 3-(6-fluoro-5-((4-(4-(6-fluorobenzo[d]isoxazol-3-yl)piperazin-1-yl)piperidin-1-yl)methyl)-1-oxoisoindolin-2-yl)piperidine-2,6-dione |
| GT-09643 | 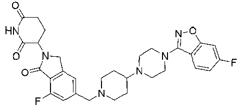 | 3-(7-fluoro-5-((4-(4-(6-fluorobenzo[d]isoxazol-3-yl)piperazin-1-yl)piperidin-1-yl)methyl)-1-oxoisoindolin-2-yl)piperidine-2,6-dione | 3-(7-fluoro-5-((4-(4-(6-fluorobenzo[d]isoxazol-3-yl)piperazin-1-yl)piperidin-1-yl)methyl)-1-oxoisoindolin-2-yl)piperidine-2,6-dione |
| GT-09644 | 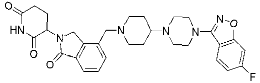 | 3-(4-((4-(4-(6-fluorobenzo[d]isoxazol-3-yl)piperazin-1-yl)piperidin-1-yl)methyl)-1-oxoisoindolin-2-yl)piperidine-2,6-dione | 3-(4-((4-(4-(6-fluorobenzo[d]isoxazol-3-yl)piperazin-1-yl)piperidin-1-yl)methyl)-1-oxoisoindolin-2-yl)piperidine-2,6-dione |
| GT-09645 | 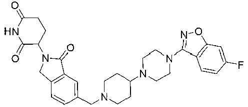 | 3-(6-((4-(4-(6-fluorobenzo[d]isoxazol-3-yl)piperazin-1-yl)piperidin-1-yl)methyl)-1-oxoisoindolin-2-yl)piperidine-2,6-dione | 3-(6-((4-(4-(6-fluorobenzo[d]isoxazol-3-yl)piperazin-1-yl)piperidin-1-yl)methyl)-1-oxoisoindolin-2-yl)piperidine-2,6-dione |
| GT-09646 | 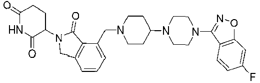 | 3-(7-((4-(4-(6-fluorobenzo[d]isoxazol-3-yl)piperazin-1-yl)piperidin-1-yl)methyl)-1-oxoisoindolin-2-yl)piperidine-2,6-dione | 3-(7-((4-(4-(6-fluorobenzo[d]isoxazol-3-yl)piperazin-1-yl)piperidin-1-yl)methyl)-1-oxoisoindolin-2-yl)piperidine-2,6-dione |
| GT-09647 | 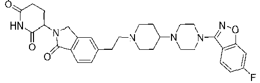 | 3-(5-(2-(4-(4-(6-fluorobenzo[d]isoxazol-3-yl)piperazin-1-yl)piperidin-1-yl)ethyl)-1-oxoisoindolin-2-yl)piperidine-2,6-dione | 3-(5-(2-(4-(4-(6-fluorobenzo[d]isoxazol-3-yl)piperazin-1-yl)piperidin-1-yl)ethyl)-1-oxoisoindolin-2-yl)piperidine-2,6-dione |
| GT-09648 | 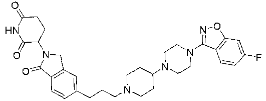 | 3-(5-(3-(4-(4-(6-fluorobenzo[d]isoxazol-3-yl)piperazin-1-yl)piperidin-1-yl)propyl)-1-oxoisoindolin-2-yl)piperidine-2,6-dione | 3-(5-(3-(4-(4-(6-fluorobenzo[d]isoxazol-3-yl)piperazin-1-yl)piperidin-1-yl)propyl)-1-oxoisoindolin-2-yl)piperidine-2,6-dione |
| GT-09650 | 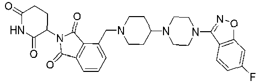 | 2-(2,6-dioxopiperidin-3-yl)-4-((4-(4-(6-fluorobenzo[d]isoxazol-3-yl)piperazin-1-yl)piperidin-1-yl)methyl)isoindoline-1,3-dione | 2-(2,6-dioxopiperidin-3-yl)-4-((4-(4-(6-fluorobenzo[d]isoxazol-3-yl)piperazin-1-yl)piperidin-1-yl)methyl)isoindoline-1,3-dione |
| GT-09649 | 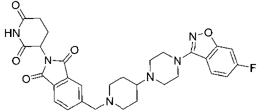 | 2-(2,6-dioxopiperidin-3-yl)-5-((4-(4-(6-fluorobenzo[d]isoxazol-3-yl)piperazin-1-yl)piperidin-1-yl)methyl)isoindoline-1,3-dione | 2-(2,6-dioxopiperidin-3-yl)-5-((4-(4-(6-fluorobenzo[d]isoxazol-3-yl)piperazin-1-yl)piperidin-1-yl)methyl)isoindoline-1,3-dione |
| GT-04931 | 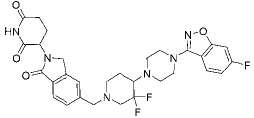 | 3-(5-((3,3-difluoro-4-(4-(6-fluorobenzo[d]isoxazol-3-yl)piperazin-1-yl)piperidin-1-yl)methyl)-1-oxoisoindolin-2-yl)piperidine-2,6-dione | 3-(5-((3,3-difluoro-4-(4-(6-fluorobenzo[d]isoxazol-3-yl)piperazin-1-yl)piperidin-1-yl)methyl)-1-oxoisoindolin-2-yl)piperidine-2,6-dione |
| GT-09655 | 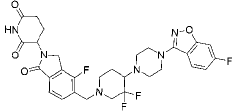 | 3-(5-((3,3-difluoro-4-(4-(6-fluorobenzo[d]isoxazol-3-yl)piperazin-1-yl)piperidin-1-yl)methyl)-4-fluoro-1-oxoisoindolin-2-yl)piperidine-2,6-dione | 3-(5-((3,3-difluoro-4-(4-(6-fluorobenzo[d]isoxazol-3-yl)piperazin-1-yl)piperidin-1-yl)methyl)-4-fluoro-1-oxoisoindolin-2-yl)piperidine-2,6-dione |
| GT-09656 | 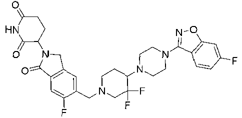 | 3-(5-((3,3-difluoro-4-(4-(6-fluorobenzo[d]isoxazol-3-yl)piperazin-1-yl)piperidin-1-yl)methyl)-6-fluoro-1-oxoisoindolin-2-yl)piperidine-2,6-dione | 3-(5-((3,3-difluoro-4-(4-(6-fluorobenzo[d]isoxazol-3-yl)piperazin-1-yl)piperidin-1-yl)methyl)-6-fluoro-1-oxoisoindolin-2-yl)piperidine-2,6-dione |
| GT-09657 | 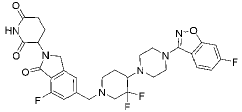 | 3-(5-((3,3-difluoro-4-(4-(6-fluorobenzo[d]isoxazol-3-yl)piperazin-1-yl)piperidin-1-yl)methyl)-7-fluoro-1-oxoisoindolin-2-yl)piperidine-2,6-dione | 3-(5-((3,3-difluoro-4-(4-(6-fluorobenzo[d]isoxazol-3-yl)piperazin-1-yl)piperidin-1-yl)methyl)-7-fluoro-1-oxoisoindolin-2-yl)piperidine-2,6-dione |
| GT-09658 | 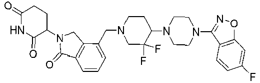 | 3-(4-((3,3-difluoro-4-(4-(6-fluorobenzo[d]isoxazol-3-yl)piperazin-1-yl)piperidin-1-yl)methyl)-1-oxoisoindolin-2-yl)piperidine-2,6-dione | 3-(4-((3,3-difluoro-4-(4-(6-fluorobenzo[d]isoxazol-3-yl)piperazin-1-yl)piperidin-1-yl)methyl)-1-oxoisoindolin-2-yl)piperidine-2,6-dione |
| GT-09659 | 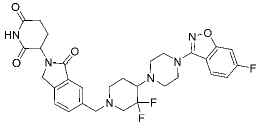 | 3-(6-((3,3-difluoro-4-(4-(6-fluorobenzo[d]isoxazol-3-yl)piperazin-1-yl)piperidin-1-yl)methyl)-1-oxoisoindolin-2-yl)piperidine-2,6-dione | 3-(6-((3,3-difluoro-4-(4-(6-fluorobenzo[d]isoxazol-3-yl)piperazin-1-yl)piperidin-1-yl)methyl)-1-oxoisoindolin-2-yl)piperidine-2,6-dione |
| GT-09660 | 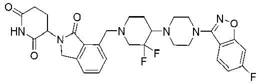 | 3-(7-((3,3-difluoro-4-(4-(6-fluorobenzo[d]isoxazol-3-yl)piperazin-1-yl)piperidin-1-yl)methyl)-1-oxoisoindolin-2-yl)piperidine-2,6-dione | 3-(7-((3,3-difluoro-4-(4-(6-fluorobenzo[d]isoxazol-3-yl)piperazin-1-yl)piperidin-1-yl)methyl)-1-oxoisoindolin-2-yl)piperidine-2,6-dione |
| GT-09661 | 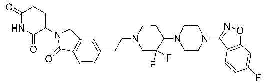 | 3-(5-(2-(3,3-difluoro-4-(4-(6-fluorobenzo[d]isoxazol-3-yl)piperazin-1-yl)piperidin-1-yl)ethyl)-1-oxoisoindolin-2-yl)piperidine-2,6-dione | 3-(5-(2-(3,3-difluoro-4-(4-(6-fluorobenzo[d]isoxazol-3-yl)piperazin-1-yl)piperidin-1-yl)ethyl)-1-oxoisoindolin-2-yl)piperidine-2,6-dione |
| GT-09662 | 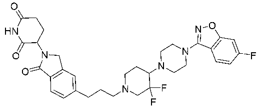 | 3-(5-(3-(3,3-difluoro-4-(4-(6-fluorobenzo[d]isoxazol-3-yl)piperazin-1-yl)piperidin-1-yl)propyl)-1-oxoisoindolin-2-yl)piperidine-2,6-dione | 3-(5-(3-(3,3-difluoro-4-(4-(6-fluorobenzo[d]isoxazol-3-yl)piperazin-1-yl)piperidin-1-yl)propyl)-1-oxoisoindolin-2-yl)piperidine-2,6-dione |
| GT-09664 | 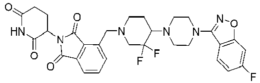 | 4-((3,3-difluoro-4-(4-(6-fluorobenzo[d]isoxazol-3-yl)piperazin-1-yl)piperidin-1-yl)methyl)-2-(2,6-dioxopiperidin-3-yl)isoindoline-1,3-dione | 4-((3,3-difluoro-4-(4-(6-fluorobenzo[d]isoxazol-3-yl)piperazin-1-yl)piperidin-1-yl)methyl)-2-(2,6-dioxopiperidin-3-yl)isoindoline-1,3-dione |
| GT-09663 | 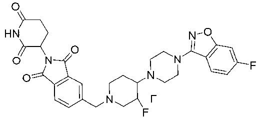 | 5-((3,3-difluoro-4-(4-(6-fluorobenzo[d]isoxazol-3-yl)piperazin-1-yl)piperidin-1-yl)methyl)-2-(2,6-dioxopiperidin-3-yl)isoindoline-1,3-dione | 5-((3,3-difluoro-4-(4-(6-fluorobenzo[d]isoxazol-3-yl)piperazin-1-yl)piperidin-1-yl)methyl)-2-(2,6-dioxopiperidin-3-yl)isoindoline-1,3-dione |
| GT-04932 | 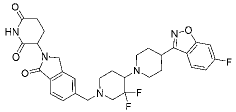 | 3-(5-((3',3'-difluoro-4-(6-fluorobenzo[d]isoxazol-3-yl)-[1,4'-bipiperidin]-1'-yl)methyl)-1-oxoisoindolin-2-yl)piperidine-2,6-dione | 3-(5-((3',3'-difluoro-4-(6-fluorobenzo[d]isoxazol-3-yl)-[1,4'-bipiperidin]-1'-yl)methyl)-1-oxoisoindolin-2-yl)piperidine-2,6-dione |
| GT-09665 | 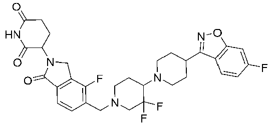 | 3-(5-((3',3'-difluoro-4-(6-fluorobenzo[d]isoxazol-3-yl)-[1,4'-bipiperidin]-1'-yl)methyl)-4-fluoro-1-oxoisoindolin-2-yl)piperidine-2,6-dione | 3-(5-((3',3'-difluoro-4-(6-fluorobenzo[d]isoxazol-3-yl)-[1,4'-bipiperidin]-1'-yl)methyl)-4-fluoro-1-oxoisoindolin-2-yl)piperidine-2,6-dione |
| GT-09666 | 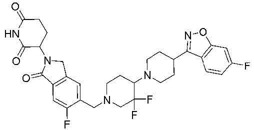 | 3-(5-((3',3'-difluoro-4-(6-fluorobenzo[d]isoxazol-3-yl)-[1,4'-bipiperidin]-1'-yl)methyl)-6-fluoro-1-oxoisoindolin-2-yl)piperidine-2,6-dione | 3-(5-((3',3'-difluoro-4-(6-fluorobenzo[d]isoxazol-3-yl)-[1,4'-bipiperidin]-1'-yl)methyl)-6-fluoro-1-oxoisoindolin-2-yl)piperidine-2,6-dione |
| GT-09667 | 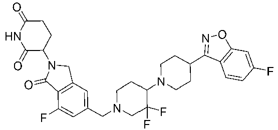 | 3-(5-((3',3'-difluoro-4-(6-fluorobenzo[d]isoxazol-3-yl)-[1,4'-bipiperidin]-1'-yl)methyl)-7-fluoro-1-oxoisoindolin-2-yl)piperidine-2,6-dione | 3-(5-((3',3'-difluoro-4-(6-fluorobenzo[d]isoxazol-3-yl)-[1,4'-bipiperidin]-1'-yl)methyl)-7-fluoro-1-oxoisoindolin-2-yl)piperidine-2,6-dione |
| GT-09668 | 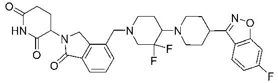 | 3-(4-((3',3'-difluoro-4-(6-fluorobenzo[d]isoxazol-3-yl)-[1,4'-bipiperidin]-1'-yl)methyl)-1-oxoisoindolin-2-yl)piperidine-2,6-dione | 3-(4-((3',3'-difluoro-4-(6-fluorobenzo[d]isoxazol-3-yl)-[1,4'-bipiperidin]-1'-yl)methyl)-1-oxoisoindolin-2-yl)piperidine-2,6-dione |
| GT-09669 | 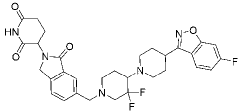 | 3-(6-((3',3'-difluoro-4-(6-fluorobenzo[d]isoxazol-3-yl)-[1,4'-bipiperidin]-1'-yl)methyl)-1-oxoisoindolin-2-yl)piperidine-2,6-dione | 3-(6-((3',3'-difluoro-4-(6-fluorobenzo[d]isoxazol-3-yl)-[1,4'-bipiperidin]-1'-yl)methyl)-1-oxoisoindolin-2-yl)piperidine-2,6-dione |
| GT-09670 | 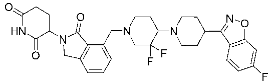 | 3-(7-((3',3'-difluoro-4-(6-fluorobenzo[d]isoxazol-3-yl)-[1,4'-bipiperidin]-1'-yl)methyl)-1-oxoisoindolin-2-yl)piperidine-2,6-dione | 3-(7-((3',3'-difluoro-4-(6-fluorobenzo[d]isoxazol-3-yl)-[1,4'-bipiperidin]-1'-yl)methyl)-1-oxoisoindolin-2-yl)piperidine-2,6-dione |
| GT-09671 | 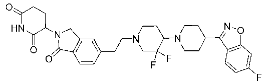 | 3-(5-(2-(3',3'-difluoro-4-(6-fluorobenzo[d]isoxazol-3-yl)-[1,4'-bipiperidin]-1'-yl)ethyl)-1-oxoisoindolin-2-yl)piperidine-2,6-dione | 3-(5-(2-(3',3'-difluoro-4-(6-fluorobenzo[d]isoxazol-3-yl)-[1,4'-bipiperidin]-1'-yl)ethyl)-1-oxoisoindolin-2-yl)piperidine-2,6-dione |
| GT-09672 | 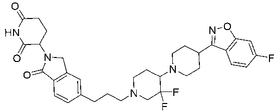 | 3-(5-(3-(3',3'-difluoro-4-(6-fluorobenzo[d]isoxazol-3-yl)-[1,4'-bipiperidin]-1'-yl)propyl)-1-oxoisoindolin-2-yl)piperidine-2,6-dione | 3-(5-(3-(3',3'-difluoro-4-(6-fluorobenzo[d]isoxazol-3-yl)-[1,4'-bipiperidin]-1'-yl)propyl)-1-oxoisoindolin-2-yl)piperidine-2,6-dione |
| GT-09674 | 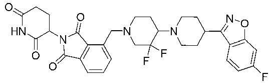 | 4-((3',3'-difluoro-4-(6-fluorobenzo[d]isoxazol-3-yl)-[1,4'-bipiperidin]-1'-yl)methyl)-2-(2,6-dioxopiperidin-3-yl)isoindoline-1,3-dione | 4-((3',3'-difluoro-4-(6-fluorobenzo[d]isoxazol-3-yl)-[1,4'-bipiperidin]-1'-yl)methyl)-2-(2,6-dioxopiperidin-3-yl)isoindoline-1,3-dione |
| GT-09673 | 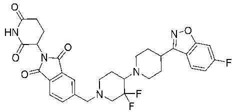 | 5-((3',3'-difluoro-4-(6-fluorobenzo[d]isoxazol-3-yl)-[1,4'-bipiperidin]-1'-yl)methyl)-2-(2,6-dioxopiperidin-3-yl)isoindoline-1,3-dione | 5-((3',3'-difluoro-4-(6-fluorobenzo[d]isoxazol-3-yl)-[1,4'-bipiperidin]-1'-yl)methyl)-2-(2,6-dioxopiperidin-3-yl)isoindoline-1,3-dione |
| GT-09944 | 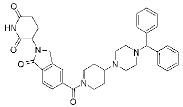 | 3-(5-(4-(4-benzhydrylpiperazin-1-yl)piperidine-1-carbonyl)-1-oxoisoindolin-2-yl)piperidine-2,6-dione | 3-(5-(4-(4-benzhydrylpiperazin-1-yl)piperidine-1-carbonyl)-1-oxoisoindolin-2-yl)piperidine-2,6-dione |
| GT-09946 | 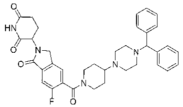 | 3-(5-(4-(4-benzhydrylpiperazin-1-yl)piperidine-1-carbonyl)-6-fluoro-1-oxoisoindolin-2-yl)piperidine-2,6-dione | 3-(5-(4-(4-benzhydrylpiperazin-1-yl)piperidine-1-carbonyl)-6-fluoro-1-oxoisoindolin-2-yl)piperidine-2,6-dione |
| GT-09945 | 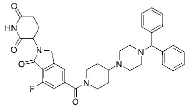 | 3-(5-(4-(4-benzhydrylpiperazin-1-yl)piperidine-1-carbonyl)-7-fluoro-1-oxoisoindolin-2-yl)piperidine-2,6-dione | 3-(5-(4-(4-benzhydrylpiperazin-1-yl)piperidine-1-carbonyl)-7-fluoro-1-oxoisoindolin-2-yl)piperidine-2,6-dione |
| GT-09947 | 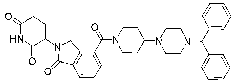 | 3-(4-(4-(4-benzhydrylpiperazin-1-yl)piperidine-1-carbonyl)-1-oxoisoindolin-2-yl)piperidine-2,6-dione | 3-(4-(4-(4-benzhydrylpiperazin-1-yl)piperidine-1-carbonyl)-1-oxoisoindolin-2-yl)piperidine-2,6-dione |
| GT-09948 | 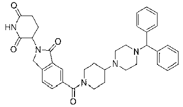 | 3-(6-(4-(4-benzhydrylpiperazin-1-yl)piperidine-1-carbonyl)-1-oxoisoindolin-2-yl)piperidine-2,6-dione | 3-(6-(4-(4-benzhydrylpiperazin-1-yl)piperidine-1-carbonyl)-1-oxoisoindolin-2-yl)piperidine-2,6-dione |
| GT-09949 | 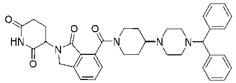 | 3-(7-(4-(4-benzhydrylpiperazin-1-yl)piperidine-1-carbonyl)-1-oxoisoindolin-2-yl)piperidine-2,6-dione | 3-(7-(4-(4-benzhydrylpiperazin-1-yl)piperidine-1-carbonyl)-1-oxoisoindolin-2-yl)piperidine-2,6-dione |
| GT-09950 | 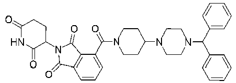 | 4-(4-(4-benzhydrylpiperazin-1-yl)piperidine-1-carbonyl)-2-(2,6-dioxopiperidin-3-yl)isoindoline-1,3-dione | 4-(4-(4-benzhydrylpiperazin-1-yl)piperidine-1-carbonyl)-2-(2,6-dioxopiperidin-3-yl)isoindoline-1,3-dione |
| GT-09951 | 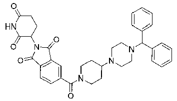 | 5-(4-(4-benzhydrylpiperazin-1-yl)piperidine-1-carbonyl)-2-(2,6-dioxopiperidin-3-yl)isoindoline-1,3-dione | 5-(4-(4-benzhydrylpiperazin-1-yl)piperidine-1-carbonyl)-2-(2,6-dioxopiperidin-3-yl)isoindoline-1,3-dione |
| GT-09675 | 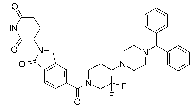 | 3-(5-(4-(4-benzhydrylpiperazin-1-yl)-3,3-difluoropiperidine-1-carbonyl)-1-oxoisoindolin-2-yl)piperidine-2,6-dione | 3-(5-(4-(4-benzhydrylpiperazin-1-yl)-3,3-difluoropiperidine-1-carbonyl)-1-oxoisoindolin-2-yl)piperidine-2,6-dione |
| GT-09710 | 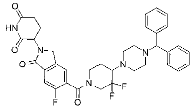 | 3-(5-(4-(4-benzhydrylpiperazin-1-yl)-3,3-difluoropiperidine-1-carbonyl)-6-fluoro-1-oxoisoindolin-2-yl)piperidine-2,6-dione | 3-(5-(4-(4-benzhydrylpiperazin-1-yl)-3,3-difluoropiperidine-1-carbonyl)-6-fluoro-1-oxoisoindolin-2-yl)piperidine-2,6-dione |
| GT-09676 | 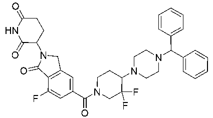 | 3-(5-(4-(4-benzhydrylpiperazin-1-yl)-3,3-difluoropiperidine-1-carbonyl)-7-fluoro-1-oxoisoindolin-2-yl)piperidine-2,6-dione | 3-(5-(4-(4-benzhydrylpiperazin-1-yl)-3,3-difluoropiperidine-1-carbonyl)-7-fluoro-1-oxoisoindolin-2-yl)piperidine-2,6-dione |
| GT-09678 | 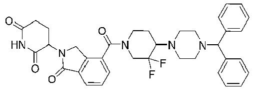 | 3-(4-(4-(4-benzhydrylpiperazin-1-yl)-3,3-difluoropiperidine-1-carbonyl)-1-oxoisoindolin-2-yl)piperidine-2,6-dione | 3-(4-(4-(4-benzhydrylpiperazin-1-yl)-3,3-difluoropiperidine-1-carbonyl)-1-oxoisoindolin-2-yl)piperidine-2,6-dione |
| GT-09677 | 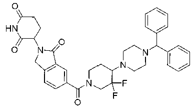 | 3-(6-(4-(4-benzhydrylpiperazin-1-yl)-3,3-difluoropiperidine-1-carbonyl)-1-oxoisoindolin-2-yl)piperidine-2,6-dione | 3-(6-(4-(4-benzhydrylpiperazin-1-yl)-3,3-difluoropiperidine-1-carbonyl)-1-oxoisoindolin-2-yl)piperidine-2,6-dione |
| GT-09711 | 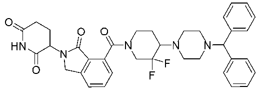 | 3-(7-(4-(4-benzhydrylpiperazin-1-yl)-3,3-difluoropiperidine-1-carbonyl)-1-oxoisoindolin-2-yl)piperidine-2,6-dione | 3-(7-(4-(4-benzhydrylpiperazin-1-yl)-3,3-difluoropiperidine-1-carbonyl)-1-oxoisoindolin-2-yl)piperidine-2,6-dione |
| GT-09712 | 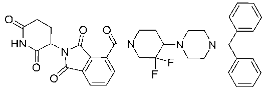 | 4-(4-(4-benzhydrylpiperazin-1-yl)-3,3-difluoropiperidine-1-carbonyl)-2-(2,6-dioxopiperidin-3-yl)isoindoline-1,3-dione | 4-(4-(4-benzhydrylpiperazin-1-yl)-3,3-difluoropiperidine-1-carbonyl)-2-(2,6-dioxopiperidin-3-yl)isoindoline-1,3-dione |
| GT-09713 | 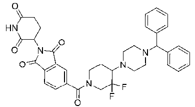 | 5-(4-(4-benzhydrylpiperazin-1-yl)-3,3-difluoropiperidine-1-carbonyl)-2-(2,6-dioxopiperidin-3-yl)isoindoline-1,3-dione | 5-(4-(4-benzhydrylpiperazin-1-yl)-3,3-difluoropiperidine-1-carbonyl)-2-(2,6-dioxopiperidin-3-yl)isoindoline-1,3-dione |
| GT-09714 | 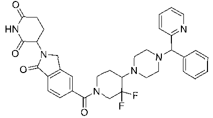 | 3-(5-(3,3-difluoro-4-(4-(phenyl(pyridin-2-yl)methyl)piperazin-1-yl)piperidine-1-carbonyl)-1-oxoisoindolin-2-yl)piperidine-2,6-dione | 3-(5-(3,3-difluoro-4-(4-(phenyl(pyridin-2-yl)methyl)piperazin-1-yl)piperidine-1-carbonyl)-1-oxoisoindolin-2-yl)piperidine-2,6-dione |
| GT-09716 | 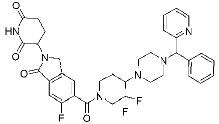 | 3-(5-(3,3-difluoro-4-(4-(phenyl(pyridin-2-yl)methyl)piperazin-1-yl)piperidine-1-carbonyl)-6-fluoro-1-oxoisoindolin-2-yl)piperidine-2,6-dione | 3-(5-(3,3-difluoro-4-(4-(phenyl(pyridin-2-yl)methyl)piperazin-1-yl)piperidine-1-carbonyl)-6-fluoro-1-oxoisoindolin-2-yl)piperidine-2,6-dione |
| GT-09715 | 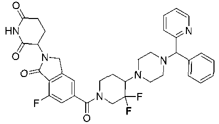 | 3-(5-(3,3-difluoro-4-(4-(phenyl(pyridin-2-yl)methyl)piperazin-1-yl)piperidine-1-carbonyl)-7-fluoro-1-oxoisoindolin-2-yl)piperidine-2,6-dione | 3-(5-(3,3-difluoro-4-(4-(phenyl(pyridin-2-yl)methyl)piperazin-1-yl)piperidine-1-carbonyl)-7-fluoro-1-oxoisoindolin-2-yl)piperidine-2,6-dione |
| GT-09717 | 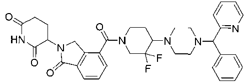 | 3-(4-(3,3-difluoro-4-(4-(phenyl(pyridin-2-yl)methyl)piperazin-1-yl)piperidine-1-carbonyl)-1-oxoisoindolin-2-yl)piperidine-2,6-dione | 3-(4-(3,3-difluoro-4-(4-(phenyl(pyridin-2-yl)methyl)piperazin-1-yl)piperidine-1-carbonyl)-1-oxoisoindolin-2-yl)piperidine-2,6-dione |
| GT-09718 | 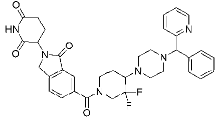 | 3-(6-(3,3-difluoro-4-(4-(phenyl(pyridin-2-yl)methyl)piperazin-1-yl)piperidine-1-carbonyl)-1-oxoisoindolin-2-yl)piperidine-2,6-dione | 3-(6-(3,3-difluoro-4-(4-(phenyl(pyridin-2-yl)methyl)piperazin-1-yl)piperidine-1-carbonyl)-1-oxoisoindolin-2-yl)piperidine-2,6-dione |
| GT-09719 | 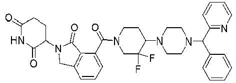 | 3-(7-(3,3-difluoro-4-(4-(phenyl(pyridin-2-yl)methyl)piperazin-1-yl)piperidine-1-carbonyl)-1-oxoisoindolin-2-yl)piperidine-2,6-dione | 3-(7-(3,3-difluoro-4-(4-(phenyl(pyridin-2-yl)methyl)piperazin-1-yl)piperidine-1-carbonyl)-1-oxoisoindolin-2-yl)piperidine-2,6-dione |
| GT-09720 | 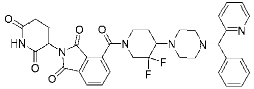 | 4-(3,3-difluoro-4-(4-(phenyl(pyridin-2-yl)methyl)piperazin-1-yl)piperidine-1-carbonyl)-2-(2,6-dioxopiperidin-3-yl)isoindoline-1,3-dione | 4-(3,3-difluoro-4-(4-(phenyl(pyridin-2-yl)methyl)piperazin-1-yl)piperidine-1-carbonyl)-2-(2,6-dioxopiperidin-3-yl)isoindoline-1,3-dione |
| GT-09721 | 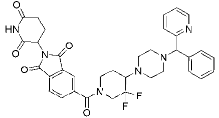 | 5-(3,3-difluoro-4-(4-(phenyl(pyridin-2-yl)methyl)piperazin-1-yl)piperidine-1-carbonyl)-2-(2,6-dioxopiperidin-3-yl)isoindoline-1,3-dione | 5-(3,3-difluoro-4-(4-(phenyl(pyridin-2-yl)methyl)piperazin-1-yl)piperidine-1-carbonyl)-2-(2,6-dioxopiperidin-3-yl)isoindoline-1,3-dione |
| GT-09722 | 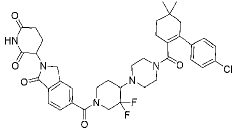 | 3-(5-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-1-oxoisoindolin-2-yl)piperidine-2,6-dione | 3-(5-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-1-oxoisoindolin-2-yl)piperidine-2,6-dione |
| GT-09724 | 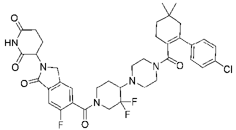 | 3-(5-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-6-fluoro-1-oxoisoindolin-2-yl)piperidine-2,6-dione | 3-(5-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-6-fluoro-1-oxoisoindolin-2-yl)piperidine-2,6-dione |
| GT-09723 | 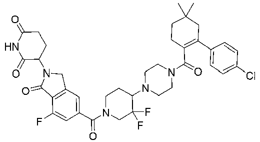 | 3-(5-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-7-fluoro-1-oxoisoindolin-2-yl)piperidine-2,6-dione | 3-(5-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-7-fluoro-1-oxoisoindolin-2-yl)piperidine-2,6-dione |
| GT-09733 | 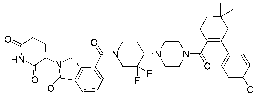 | 3-(4-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-1-oxoisoindolin-2-yl)piperidine-2,6-dione | 3-(4-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-1-oxoisoindolin-2-yl)piperidine-2,6-dione |
| GT-09725 | 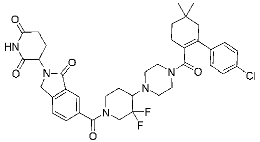 | 3-(6-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-1-oxoisoindolin-2-yl)piperidine-2,6-dione | 3-(6-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-1-oxoisoindolin-2-yl)piperidine-2,6-dione |
| GT-09726 | 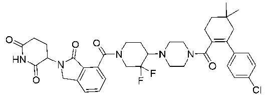 | 3-(7-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-1-oxoisoindolin-2-yl)piperidine-2,6-dione | 3-(7-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-1-oxoisoindolin-2-yl)piperidine-2,6-dione |
| GT-09727 | 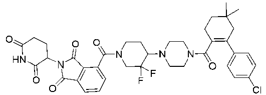 | 4-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-2-(2,6-dioxopiperidin-3-yl)isoindoline-1,3-dione | 4-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-2-(2,6-dioxopiperidin-3-yl)isoindoline-1,3-dione |
| GT-09728 | 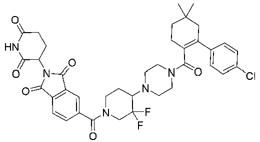 | 5-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-2-(2,6-dioxopiperidin-3-yl)isoindoline-1,3-dione | 5-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-2-(2,6-dioxopiperidin-3-yl)isoindoline-1,3-dione |
| GT-09729 | 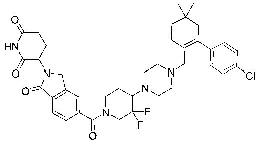 | 3-(5-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-1-oxoisoindolin-2-yl)piperidine-2,6-dione | 3-(5-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-1-oxoisoindolin-2-yl)piperidine-2,6-dione |
| GT-09731 | 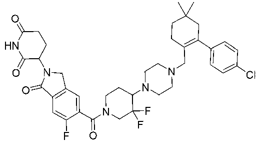 | 3-(5-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-6-fluoro-1-oxoisoindolin-2-yl)piperidine-2,6-dione | 3-(5-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-6-fluoro-1-oxoisoindolin-2-yl)piperidine-2,6-dione |
| GT-09730 | 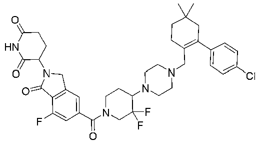 | 3-(5-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-7-fluoro-1-oxoisoindolin-2-yl)piperidine-2,6-dione | 3-(5-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-7-fluoro-1-oxoisoindolin-2-yl)piperidine-2,6-dione |
| GT-09732 | 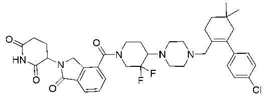 | 3-(4-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-1-oxoisoindolin-2-yl)piperidine-2,6-dione | 3-(4-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-1-oxoisoindolin-2-yl)piperidine-2,6-dione |
| GT-09828 | 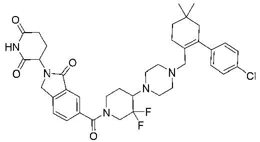 | 3-(6-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-1-oxoisoindolin-2-yl)piperidine-2,6-dione | 3-(6-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-1-oxoisoindolin-2-yl)piperidine-2,6-dione |
| GT-09829 | 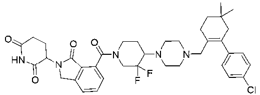 | 3-(7-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-1-oxoisoindolin-2-yl)piperidine-2,6-dione | 3-(7-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-1-oxoisoindolin-2-yl)piperidine-2,6-dione |
| GT-09830 | 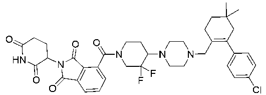 | 4-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-2-(2,6-dioxopiperidin-3-yl)isoindoline-1,3-dione | 4-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-2-(2,6-dioxopiperidin-3-yl)isoindoline-1,3-dione |
| GT-09831 | 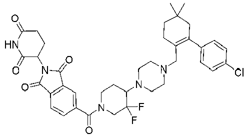 | 5-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-2-(2,6-dioxopiperidin-3-yl)isoindoline-1,3-dione | 5-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-2-(2,6-dioxopiperidin-3-yl)isoindoline-1,3-dione |
| GT-09832 | 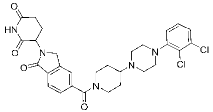 | 3-(5-(4-(4-(2,3-dichlorophenyl)piperazin-1-yl)piperidine-1-carbonyl)-1-oxoisoindolin-2-yl)piperidine-2,6-dione | 3-(5-(4-(4-(2,3-dichlorophenyl)piperazin-1-yl)piperidine-1-carbonyl)-1-oxoisoindolin-2-yl)piperidine-2,6-dione |
| GT-09834 | 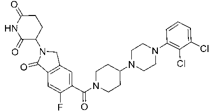 | 3-(5-(4-(4-(2,3-dichlorophenyl)piperazin-1-yl)piperidine-1-carbonyl)-6-fluoro-1-oxoisoindolin-2-yl)piperidine-2,6-dione | 3-(5-(4-(4-(2,3-dichlorophenyl)piperazin-1-yl)piperidine-1-carbonyl)-6-fluoro-1-oxoisoindolin-2-yl)piperidine-2,6-dione |
| GT-09833 | 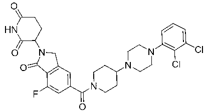 | 3-(5-(4-(4-(2,3-dichlorophenyl)piperazin-1-yl)piperidine-1-carbonyl)-7-fluoro-1-oxoisoindolin-2-yl)piperidine-2,6-dione | 3-(5-(4-(4-(2,3-dichlorophenyl)piperazin-1-yl)piperidine-1-carbonyl)-7-fluoro-1-oxoisoindolin-2-yl)piperidine-2,6-dione |
| GT-09835 | 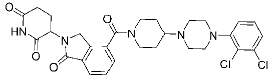 | 3-(4-(4-(4-(2,3-dichlorophenyl)piperazin-1-yl)piperidine-1-carbonyl)-1-oxoisoindolin-2-yl)piperidine-2,6-dione | 3-(4-(4-(4-(2,3-dichlorophenyl)piperazin-1-yl)piperidine-1-carbonyl)-1-oxoisoindolin-2-yl)piperidine-2,6-dione |
| GT-09836 | 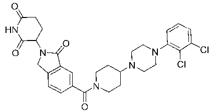 | 3-(6-(4-(4-(2,3-dichlorophenyl)piperazin-1-yl)piperidine-1-carbonyl)-1-oxoisoindolin-2-yl)piperidine-2,6-dione | 3-(6-(4-(4-(2,3-dichlorophenyl)piperazin-1-yl)piperidine-1-carbonyl)-1-oxoisoindolin-2-yl)piperidine-2,6-dione |
| GT-09837 | 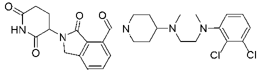 | 3-(7-(4-(4-(2,3-dichlorophenyl)piperazin-1-yl)piperidine-1-carbonyl)-1-oxoisoindolin-2-yl)piperidine-2,6-dione | 3-(7-(4-(4-(2,3-dichlorophenyl)piperazin-1-yl)piperidine-1-carbonyl)-1-oxoisoindolin-2-yl)piperidine-2,6-dione |
| GT-09838 | 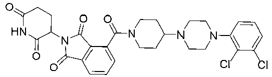 | 4-(4-(4-(2,3-dichlorophenyl)piperazin-1-yl)piperidine-1-carbonyl)-2-(2,6-dioxopiperidin-3-yl)isoindoline-1,3-dione | 4-(4-(4-(2,3-dichlorophenyl)piperazin-1-yl)piperidine-1-carbonyl)-2-(2,6-dioxopiperidin-3-yl)isoindoline-1,3-dione |
| GT-09839 | 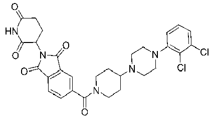 | 5-(4-(4-(2,3-dichlorophenyl)piperazin-1-yl)piperidine-1-carbonyl)-2-(2,6-dioxopiperidin-3-yl)isoindoline-1,3-dione | 5-(4-(4-(2,3-dichlorophenyl)piperazin-1-yl)piperidine-1-carbonyl)-2-(2,6-dioxopiperidin-3-yl)isoindoline-1,3-dione |
| GT-09840 | 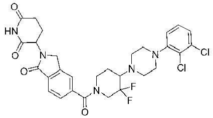 | 3-(5-(4-(4-(2,3-dichlorophenyl)piperazin-1-yl)-3,3-difluoropiperidine-1-carbonyl)-1-oxoisoindolin-2-yl)piperidine-2,6-dione | 3-(5-(4-(4-(2,3-dichlorophenyl)piperazin-1-yl)-3,3-difluoropiperidine-1-carbonyl)-1-oxoisoindolin-2-yl)piperidine-2,6-dione |
| GT-09842 | 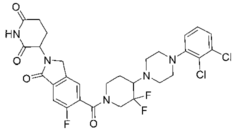 | 3-(5-(4-(4-(2,3-dichlorophenyl)piperazin-1-yl)-3,3-difluoropiperidine-1-carbonyl)-6-fluoro-1-oxoisoindolin-2-yl)piperidine-2,6-dione | 3-(5-(4-(4-(2,3-dichlorophenyl)piperazin-1-yl)-3,3-difluoropiperidine-1-carbonyl)-6-fluoro-1-oxoisoindolin-2-yl)piperidine-2,6-dione |
| GT-09841 | 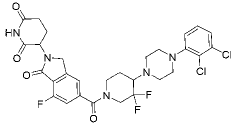 | 3-(5-(4-(4-(2,3-dichlorophenyl)piperazin-1-yl)-3,3-difluoropiperidine-1-carbonyl)-7-fluoro-1-oxoisoindolin-2-yl)piperidine-2,6-dione | 3-(5-(4-(4-(2,3-dichlorophenyl)piperazin-1-yl)-3,3-difluoropiperidine-1-carbonyl)-7-fluoro-1-oxoisoindolin-2-yl)piperidine-2,6-dione |
| GT-09843 | 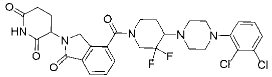 | 3-(4-(4-(4-(2,3-dichlorophenyl)piperazin-1-yl)-3,3-difluoropiperidine-1-carbonyl)-1-oxoisoindolin-2-yl)piperidine-2,6-dione | 3-(4-(4-(4-(2,3-dichlorophenyl)piperazin-1-yl)-3,3-difluoropiperidine-1-carbonyl)-1-oxoisoindolin-2-yl)piperidine-2,6-dione |
| GT-09844 | 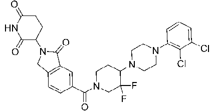 | 3-(6-(4-(4-(2,3-dichlorophenyl)piperazin-1-yl)-3,3-difluoropiperidine-1-carbonyl)-1-oxoisoindolin-2-yl)piperidine-2,6-dione | 3-(6-(4-(4-(2,3-dichlorophenyl)piperazin-1-yl)-3,3-difluoropiperidine-1-carbonyl)-1-oxoisoindolin-2-yl)piperidine-2,6-dione |
| GT-09845 | 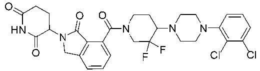 | 3-(7-(4-(4-(2,3-dichlorophenyl)piperazin-1-yl)-3,3-difluoropiperidine-1-carbonyl)-1-oxoisoindolin-2-yl)piperidine-2,6-dione | 3-(7-(4-(4-(2,3-dichlorophenyl)piperazin-1-yl)-3,3-difluoropiperidine-1-carbonyl)-1-oxoisoindolin-2-yl)piperidine-2,6-dione |
| GT-09846 | 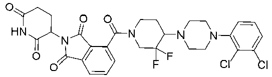 | 4-(4-(4-(2,3-dichlorophenyl)piperazin-1-yl)-3,3-difluoropiperidine-1-carbonyl)-2-(2,6-dioxopiperidin-3-yl)isoindoline-1,3-dione | 4-(4-(4-(2,3-dichlorophenyl)piperazin-1-yl)-3,3-difluoropiperidine-1-carbonyl)-2-(2,6-dioxopiperidin-3-yl)isoindoline-1,3-dione |
| GT-09847 | 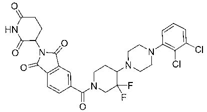 | 5-(4-(4-(2,3-dichlorophenyl)piperazin-1-yl)-3,3-difluoropiperidine-1-carbonyl)-2-(2,6-dioxopiperidin-3-yl)isoindoline-1,3-dione | 5-(4-(4-(2,3-dichlorophenyl)piperazin-1-yl)-3,3-difluoropiperidine-1-carbonyl)-2-(2,6-dioxopiperidin-3-yl)isoindoline-1,3-dione |
| GT-09952 | 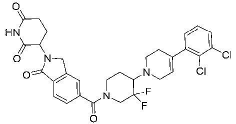 | 3-(5-(4-(4-(2,3-dichlorophenyl)-3,6-dihydropyridin-1(2H)-yl)-3,3-difluoropiperidine-1-carbonyl)-1-oxoisoindolin-2-yl)piperidine-2,6-dione | 3-(5-(4-(4-(2,3-dichlorophenyl)-3,6-dihydropyridin-1(2H)-yl)-3,3-difluoropiperidine-1-carbonyl)-1-oxoisoindolin-2-yl)piperidine-2,6-dione |
| GT-09954 | 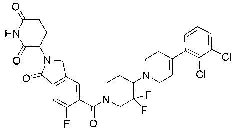 | 3-(5-(4-(4-(2,3-dichlorophenyl)-3,6-dihydropyridin-1(2H)-yl)-3,3-difluoropiperidine-1-carbonyl)-6-fluoro-1-oxoisoindolin-2-yl)piperidine-2,6-dione | 3-(5-(4-(4-(2,3-dichlorophenyl)-3,6-dihydropyridin-1(2H)-yl)-3,3-difluoropiperidine-1-carbonyl)-6-fluoro-1-oxoisoindolin-2-yl)piperidine-2,6-dione |
| GT-09953 | 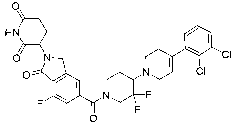 | 3-(5-(4-(4-(2,3-dichlorophenyl)-3,6-dihydropyridin-1(2H)-yl)-3,3-difluoropiperidine-1-carbonyl)-7-fluoro-1-oxoisoindolin-2-yl)piperidine-2,6-dione | 3-(5-(4-(4-(2,3-dichlorophenyl)-3,6-dihydropyridin-1(2H)-yl)-3,3-difluoropiperidine-1-carbonyl)-7-fluoro-1-oxoisoindolin-2-yl)piperidine-2,6-dione |
| GT-09955 | 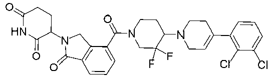 | 3-(4-(4-(4-(2,3-dichlorophenyl)-3,6-dihydropyridin-1(2H)-yl)-3,3-difluoropiperidine-1-carbonyl)-1-oxoisoindolin-2-yl)piperidine-2,6-dione | 3-(4-(4-(4-(2,3-dichlorophenyl)-3,6-dihydropyridin-1(2H)-yl)-3,3-difluoropiperidine-1-carbonyl)-1-oxoisoindolin-2-yl)piperidine-2,6-dione |
| GT-09956 | 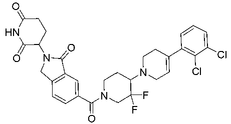 | 3-(6-(4-(4-(2,3-dichlorophenyl)-3,6-dihydropyridin-1(2H)-yl)-3,3-difluoropiperidine-1-carbonyl)-1-oxoisoindolin-2-yl)piperidine-2,6-dione | 3-(6-(4-(4-(2,3-dichlorophenyl)-3,6-dihydropyridin-1(2H)-yl)-3,3-difluoropiperidine-1-carbonyl)-1-oxoisoindolin-2-yl)piperidine-2,6-dione |
| GT-09957 | 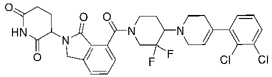 | 3-(7-(4-(4-(2,3-dichlorophenyl)-3,6-dihydropyridin-1(2H)-yl)-3,3-difluoropiperidine-1-carbonyl)-1-oxoisoindolin-2-yl)piperidine-2,6-dione | 3-(7-(4-(4-(2,3-dichlorophenyl)-3,6-dihydropyridin-1(2H)-yl)-3,3-difluoropiperidine-1-carbonyl)-1-oxoisoindolin-2-yl)piperidine-2,6-dione |
| GT-09958 | 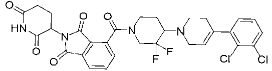 | 4-(4-(4-(2,3-dichlorophenyl)-3,6-dihydropyridin-1(2H)-yl)-3,3-difluoropiperidine-1-carbonyl)-2-(2,6-dioxopiperidin-3-yl)isoindoline-1,3-dione | 4-(4-(4-(2,3-dichlorophenyl)-3,6-dihydropyridin-1(2H)-yl)-3,3-difluoropiperidine-1-carbonyl)-2-(2,6-dioxopiperidin-3-yl)isoindoline-1,3-dione |
| GT-09959 | 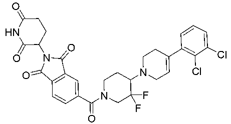 | 5-(4-(4-(2,3-dichlorophenyl)-3,6-dihydropyridin-1(2H)-yl)-3,3-difluoropiperidine-1-carbonyl)-2-(2,6-dioxopiperidin-3-yl)isoindoline-1,3-dione | 5-(4-(4-(2,3-dichlorophenyl)-3,6-dihydropyridin-1(2H)-yl)-3,3-difluoropiperidine-1-carbonyl)-2-(2,6-dioxopiperidin-3-yl)isoindoline-1,3-dione |
| GT-09848 | 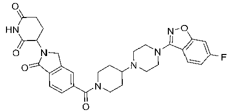 | 3-(5-(4-(4-(6-fluorobenzo[d]isoxazol-3-yl)piperazin-1-yl)piperidine-1-carbonyl)-1-oxoisoindolin-2-yl)piperidine-2,6-dione | 3-(5-(4-(4-(6-fluorobenzo[d]isoxazol-3-yl)piperazin-1-yl)piperidine-1-carbonyl)-1-oxoisoindolin-2-yl)piperidine-2,6-dione |
| GT-09850 | 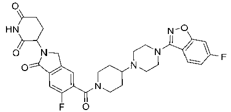 | 3-(6-fluoro-5-(4-(4-(6-fluorobenzo[d]isoxazol-3-yl)piperazin-1-yl)piperidine-1-carbonyl)-1-oxoisoindolin-2-yl)piperidine-2,6-dione | 3-(6-fluoro-5-(4-(4-(6-fluorobenzo[d]isoxazol-3-yl)piperazin-1-yl)piperidine-1-carbonyl)-1-oxoisoindolin-2-yl)piperidine-2,6-dione |
| GT-09849 | 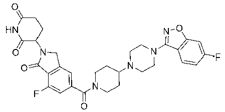 | 3-(7-fluoro-5-(4-(4-(6-fluorobenzo[d]isoxazol-3-yl)piperazin-1-yl)piperidine-1-carbonyl)-1-oxoisoindolin-2-yl)piperidine-2,6-dione | 3-(7-fluoro-5-(4-(4-(6-fluorobenzo[d]isoxazol-3-yl)piperazin-1-yl)piperidine-1-carbonyl)-1-oxoisoindolin-2-yl)piperidine-2,6-dione |
| GT-09851 | 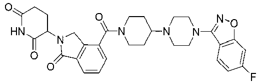 | 3-(4-(4-(4-(6-fluorobenzo[d]isoxazol-3-yl)piperazin-1-yl)piperidine-1-carbonyl)-1-oxoisoindolin-2-yl)piperidine-2,6-dione | 3-(4-(4-(4-(6-fluorobenzo[d]isoxazol-3-yl)piperazin-1-yl)piperidine-1-carbonyl)-1-oxoisoindolin-2-yl)piperidine-2,6-dione |
| GT-09896 | 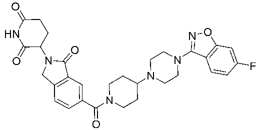 | 3-(6-(4-(4-(6-fluorobenzo[d]isoxazol-3-yl)piperazin-1-yl)piperidine-1-carbonyl)-1-oxoisoindolin-2-yl)piperidine-2,6-dione | 3-(6-(4-(4-(6-fluorobenzo[d]isoxazol-3-yl)piperazin-1-yl)piperidine-1-carbonyl)-1-oxoisoindolin-2-yl)piperidine-2,6-dione |
| GT-09897 | 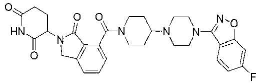 | 3-(7-(4-(4-(6-fluorobenzo[d]isoxazol-3-yl)piperazin-1-yl)piperidine-1-carbonyl)-1-oxoisoindolin-2-yl)piperidine-2,6-dione | 3-(7-(4-(4-(6-fluorobenzo[d]isoxazol-3-yl)piperazin-1-yl)piperidine-1-carbonyl)-1-oxoisoindolin-2-yl)piperidine-2,6-dione |
| GT-09898 | 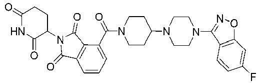 | 2-(2,6-dioxopiperidin-3-yl)-4-(4-(4-(6-fluorobenzo[d]isoxazol-3-yl)piperazin-1-yl)piperidine-1-carbonyl)isoindoline-1,3-dione | 2-(2,6-dioxopiperidin-3-yl)-4-(4-(4-(6-fluorobenzo[d]isoxazol-3-yl)piperazin-1-yl)piperidine-1-carbonyl)isoindoline-1,3-dione |
| GT-09899 | 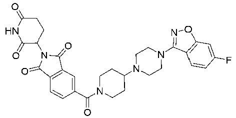 | 2-(2,6-dioxopiperidin-3-yl)-5-(4-(4-(6-fluorobenzo[d]isoxazol-3-yl)piperazin-1-yl)piperidine-1-carbonyl)isoindoline-1,3-dione | 2-(2,6-dioxopiperidin-3-yl)-5-(4-(4-(6-fluorobenzo[d]isoxazol-3-yl)piperazin-1-yl)piperidine-1-carbonyl)isoindoline-1,3-dione |
| GT-09900 | 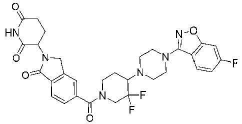 | 3-(5-(3,3-difluoro-4-(4-(6-fluorobenzo[d]isoxazol-3-yl)piperazin-1-yl)piperidine-1-carbonyl)-1-oxoisoindolin-2-yl)piperidine-2,6-dione | 3-(5-(3,3-difluoro-4-(4-(6-fluorobenzo[d]isoxazol-3-yl)piperazin-1-yl)piperidine-1-carbonyl)-1-oxoisoindolin-2-yl)piperidine-2,6-dione |
| GT-09902 | 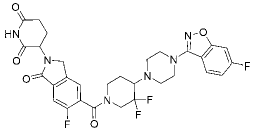 | 3-(5-(3,3-difluoro-4-(4-(6-fluorobenzo[d]isoxazol-3-yl)piperazin-1-yl)piperidine-1-carbonyl)-6-fluoro-1-oxoisoindolin-2-yl)piperidine-2,6-dione | 3-(5-(3,3-difluoro-4-(4-(6-fluorobenzo[d]isoxazol-3-yl)piperazin-1-yl)piperidine-1-carbonyl)-6-fluoro-1-oxoisoindolin-2-yl)piperidine-2,6-dione |
| GT-09901 | 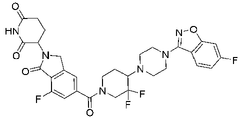 | 3-(5-(3,3-difluoro-4-(4-(6-fluorobenzo[d]isoxazol-3-yl)piperazin-1-yl)piperidine-1-carbonyl)-7-fluoro-1-oxoisoindolin-2-yl)piperidine-2,6-dione | 3-(5-(3,3-difluoro-4-(4-(6-fluorobenzo[d]isoxazol-3-yl)piperazin-1-yl)piperidine-1-carbonyl)-7-fluoro-1-oxoisoindolin-2-yl)piperidine-2,6-dione |
| GT-09903 | 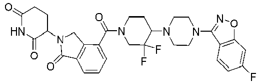 | 3-(4-(3,3-difluoro-4-(4-(6-fluorobenzo[d]isoxazol-3-yl)piperazin-1-yl)piperidine-1-carbonyl)-1-oxoisoindolin-2-yl)piperidine-2,6-dione | 3-(4-(3,3-difluoro-4-(4-(6-fluorobenzo[d]isoxazol-3-yl)piperazin-1-yl)piperidine-1-carbonyl)-1-oxoisoindolin-2-yl)piperidine-2,6-dione |
| GT-09904 | 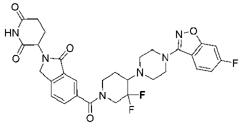 | 3-(6-(3,3-difluoro-4-(4-(6-fluorobenzo[d]isoxazol-3-yl)piperazin-1-yl)piperidine-1-carbonyl)-1-oxoisoindolin-2-yl)piperidine-2,6-dione | 3-(6-(3,3-difluoro-4-(4-(6-fluorobenzo[d]isoxazol-3-yl)piperazin-1-yl)piperidine-1-carbonyl)-1-oxoisoindolin-2-yl)piperidine-2,6-dione |
| GT-09905 | 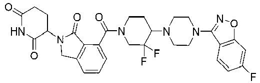 | 3-(7-(3,3-difluoro-4-(4-(6-fluorobenzo[d]isoxazol-3-yl)piperazin-1-yl)piperidine-1-carbonyl)-1-oxoisoindolin-2-yl)piperidine-2,6-dione | 3-(7-(3,3-difluoro-4-(4-(6-fluorobenzo[d]isoxazol-3-yl)piperazin-1-yl)piperidine-1-carbonyl)-1-oxoisoindolin-2-yl)piperidine-2,6-dione |
| GT-09906 | 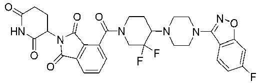 | 4-(3,3-difluoro-4-(4-(6-fluorobenzo[d]isoxazol-3-yl)piperazin-1-yl)piperidine-1-carbonyl)-2-(2,6-dioxopiperidin-3-yl)isoindoline-1,3-dione | 4-(3,3-difluoro-4-(4-(6-fluorobenzo[d]isoxazol-3-yl)piperazin-1-yl)piperidine-1-carbonyl)-2-(2,6-dioxopiperidin-3-yl)isoindoline-1,3-dione |
| GT-09907 | 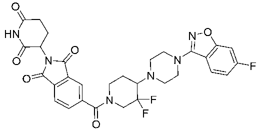 | 5-(3,3-difluoro-4-(4-(6-fluorobenzo[d]isoxazol-3-yl)piperazin-1-yl)piperidine-1-carbonyl)-2-(2,6-dioxopiperidin-3-yl)isoindoline-1,3-dione | 5-(3,3-difluoro-4-(4-(6-fluorobenzo[d]isoxazol-3-yl)piperazin-1-yl)piperidine-1-carbonyl)-2-(2,6-dioxopiperidin-3-yl)isoindoline-1,3-dione |
| GT-09908 | 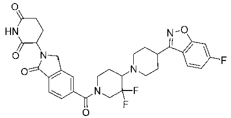 | 3-(5-(3',3'-difluoro-4-(6-fluorobenzo[d]isoxazol-3-yl)-[1,4'-bipiperidine]-1'-carbonyl)-1-oxoisoindolin-2-yl)piperidine-2,6-dione | 3-(5-(3',3'-difluoro-4-(6-fluorobenzo[d]isoxazol-3-yl)-[1,4'-bipiperidine]-1'-carbonyl)-1-oxoisoindolin-2-yl)piperidine-2,6-dione |
| GT-09910 | 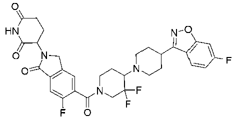 | 3-(5-(3',3'-difluoro-4-(6-fluorobenzo[d]isoxazol-3-yl)-[1,4'-bipiperidine]-1'-carbonyl)-6-fluoro-1-oxoisoindolin-2-yl)piperidine-2,6-dione | 3-(5-(3',3'-difluoro-4-(6-fluorobenzo[d]isoxazol-3-yl)-[1,4'-bipiperidine]-1'-carbonyl)-6-fluoro-1-oxoisoindolin-2-yl)piperidine-2,6-dione |
| GT-09909 | 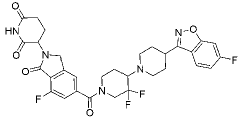 | 3-(5-(3',3'-difluoro-4-(6-fluorobenzo[d]isoxazol-3-yl)-[1,4'-bipiperidine]-1'-carbonyl)-7-fluoro-1-oxoisoindolin-2-yl)piperidine-2,6-dione | 3-(5-(3',3'-difluoro-4-(6-fluorobenzo[d]isoxazol-3-yl)-[1,4'-bipiperidine]-1'-carbonyl)-7-fluoro-1-oxoisoindolin-2-yl)piperidine-2,6-dione |
| GT-09911 | 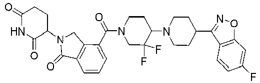 | 3-(4-(3',3'-difluoro-4-(6-fluorobenzo[d]isoxazol-3-yl)-[1,4'-bipiperidine]-1'-carbonyl)-1-oxoisoindolin-2-yl)piperidine-2,6-dione | 3-(4-(3',3'-difluoro-4-(6-fluorobenzo[d]isoxazol-3-yl)-[1,4'-bipiperidine]-1'-carbonyl)-1-oxoisoindolin-2-yl)piperidine-2,6-dione |
| GT-09912 | 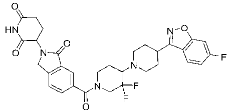 | 3-(6-(3',3'-difluoro-4-(6-fluorobenzo[d]isoxazol-3-yl)-[1,4'-bipiperidine]-1'-carbonyl)-1-oxoisoindolin-2-yl)piperidine-2,6-dione | 3-(6-(3',3'-difluoro-4-(6-fluorobenzo[d]isoxazol-3-yl)-[1,4'-bipiperidine]-1'-carbonyl)-1-oxoisoindolin-2-yl)piperidine-2,6-dione |
| GT-09913 | 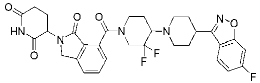 | 3-(7-(3',3'-difluoro-4-(6-fluorobenzo[d]isoxazol-3-yl)-[1,4'-bipiperidine]-1'-carbonyl)-1-oxoisoindolin-2-yl)piperidine-2,6-dione | 3-(7-(3',3'-difluoro-4-(6-fluorobenzo[d]isoxazol-3-yl)-[1,4'-bipiperidine]-1'-carbonyl)-1-oxoisoindolin-2-yl)piperidine-2,6-dione |
| GT-09914 | 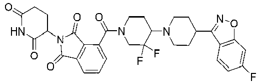 | 4-(3',3'-difluoro-4-(6-fluorobenzo[d]isoxazol-3-yl)-[1,4'-bipiperidine]-1'-carbonyl)-2-(2,6-dioxopiperidin-3-yl)isoindoline-1,3-dione | 4-(3',3'-difluoro-4-(6-fluorobenzo[d]isoxazol-3-yl)-[1,4'-bipiperidine]-1'-carbonyl)-2-(2,6-dioxopiperidin-3-yl)isoindoline-1,3-dione |
| GT-09915 | 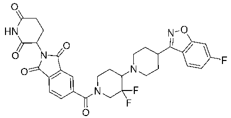 | 5-(3',3'-difluoro-4-(6-fluorobenzo[d]isoxazol-3-yl)-[1,4'-bipiperidine]-1'-carbonyl)-2-(2,6-dioxopiperidin-3-yl)isoindoline-1,3-dione | 5-(3',3'-difluoro-4-(6-fluorobenzo[d]isoxazol-3-yl)-[1,4'-bipiperidine]-1'-carbonyl)-2-(2,6-dioxopiperidin-3-yl)isoindoline-1,3-dione |
| GT-09941 | 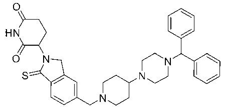 | 3-(5-((4-(4-benzhydrylpiperazin-1-yl)piperidin-1-yl)methyl)-1-thioxoisoindolin-2-yl)piperidine-2,6-dione | 3-(5-((4-(4-benzhydrylpiperazin-1-yl)piperidin-1-yl)methyl)-1-thioxoisoindolin-2-yl)piperidine-2,6-dione |
| GT-09938 | 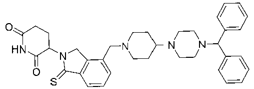 | 3-(4-((4-(4-benzhydrylpiperazin-1-yl)piperidin-1-yl)methyl)-1-thioxoisoindolin-2-yl)piperidine-2,6-dione | 3-(4-((4-(4-benzhydrylpiperazin-1-yl)piperidin-1-yl)methyl)-1-thioxoisoindolin-2-yl)piperidine-2,6-dione |
| GT-09862 | 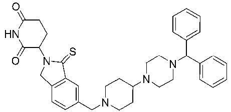 | 3-(6-((4-(4-benzhydrylpiperazin-1-yl)piperidin-1-yl)methyl)-1-thioxoisoindolin-2-yl)piperidine-2,6-dione | 3-(6-((4-(4-benzhydrylpiperazin-1-yl)piperidin-1-yl)methyl)-1-thioxoisoindolin-2-yl)piperidine-2,6-dione |
| GT-05105 | 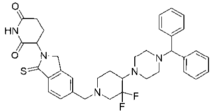 | 3-(5-((4-(4-benzhydrylpiperazin-1-yl)-3,3-difluoropiperidin-1-yl)methyl)-1-thioxoisoindolin-2-yl)piperidine-2,6-dione | 3-(5-((4-(4-benzhydrylpiperazin-1-yl)-3,3-difluoropiperidin-1-yl)methyl)-1-thioxoisoindolin-2-yl)piperidine-2,6-dione |
| GT-09617 | 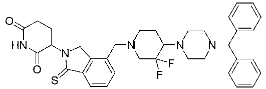 | 3-(4-((4-(4-benzhydrylpiperazin-1-yl)-3,3-difluoropiperidin-1-yl)methyl)-1-thioxoisoindolin-2-yl)piperidine-2,6-dione | 3-(4-((4-(4-benzhydrylpiperazin-1-yl)-3,3-difluoropiperidin-1-yl)methyl)-1-thioxoisoindolin-2-yl)piperidine-2,6-dione |
| GT-09691 | 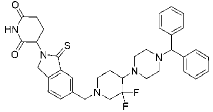 | 3-(6-((4-(4-benzhydrylpiperazin-1-yl)-3,3-difluoropiperidin-1-yl)methyl)-1-thioxoisoindolin-2-yl)piperidine-2,6-dione | 3-(6-((4-(4-benzhydrylpiperazin-1-yl)-3,3-difluoropiperidin-1-yl)methyl)-1-thioxoisoindolin-2-yl)piperidine-2,6-dione |
| GT-05208 | 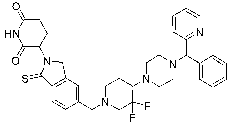 | 3-(5-((3,3-difluoro-4-(4-(phenyl(pyridin-2-yl)methyl)piperazin-1-yl)piperidin-1-yl)methyl)-1-thioxoisoindolin-2-yl)piperidine-2,6-dione | 3-(5-((3,3-difluoro-4-(4-(phenyl(pyridin-2-yl)methyl)piperazin-1-yl)piperidin-1-yl)methyl)-1-thioxoisoindolin-2-yl)piperidine-2,6-dione |
| GT-09618 | 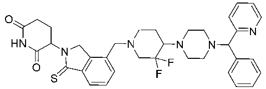 | 3-(4-((3,3-difluoro-4-(4-(phenyl(pyridin-2-yl)methyl)piperazin-1-yl)piperidin-1-yl)methyl)-1-thioxoisoindolin-2-yl)piperidine-2,6-dione | 3-(4-((3,3-difluoro-4-(4-(phenyl(pyridin-2-yl)methyl)piperazin-1-yl)piperidin-1-yl)methyl)-1-thioxoisoindolin-2-yl)piperidine-2,6-dione |
| GT-05042 | 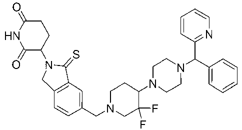 | 3-(6-((3,3-difluoro-4-(4-(phenyl(pyridin-2-yl)methyl)piperazin-1-yl)piperidin-1-yl)methyl)-1-thioxoisoindolin-2-yl)piperidine-2,6-dione | 3-(6-((3,3-difluoro-4-(4-(phenyl(pyridin-2-yl)methyl)piperazin-1-yl)piperidin-1-yl)methyl)-1-thioxoisoindolin-2-yl)piperidine-2,6-dione |
| GT-09679 | 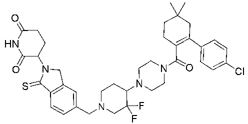 | 3-(5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-thioxoisoindolin-2-yl)piperidine-2,6-dione | 3-(5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-thioxoisoindolin-2-yl)piperidine-2,6-dione |
| GT-09619 | 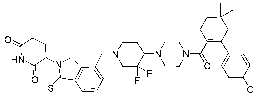 | 3-(4-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-thioxoisoindolin-2-yl)piperidine-2,6-dione | 3-(4-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-thioxoisoindolin-2-yl)piperidine-2,6-dione |
| GT-09692 | 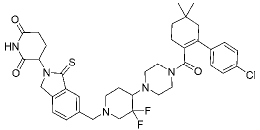 | 3-(6-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-thioxoisoindolin-2-yl)piperidine-2,6-dione | 3-(6-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-thioxoisoindolin-2-yl)piperidine-2,6-dione |
| GT-09680 | 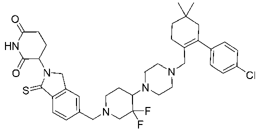 | 3-(5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-thioxoisoindolin-2-yl)piperidine-2,6-dione | 3-(5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-thioxoisoindolin-2-yl)piperidine-2,6-dione |
| GT-09576 | 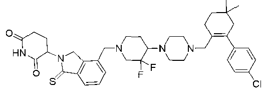 | 3-(4-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-thioxoisoindolin-2-yl)piperidine-2,6-dione | 3-(4-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-thioxoisoindolin-2-yl)piperidine-2,6-dione |
| GT-09863 | 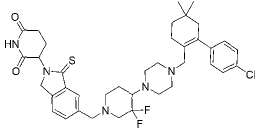 | 3-(6-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-thioxoisoindolin-2-yl)piperidine-2,6-dione | 3-(6-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-thioxoisoindolin-2-yl)piperidine-2,6-dione |
| GT-09934 | 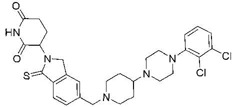 | 3-(5-((4-(4-(2,3-dichlorophenyl)piperazin-1-yl)piperidin-1-yl)methyl)-1-thioxoisoindolin-2-yl)piperidine-2,6-dione | 3-(5-((4-(4-(2,3-dichlorophenyl)piperazin-1-yl)piperidin-1-yl)methyl)-1-thioxoisoindolin-2-yl)piperidine-2,6-dione |
| GT-09939 | 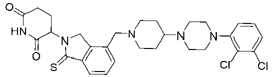 | 3-(4-((4-(4-(2,3-dichlorophenyl)piperazin-1-yl)piperidin-1-yl)methyl)-1-thioxoisoindolin-2-yl)piperidine-2,6-dione | 3-(4-((4-(4-(2,3-dichlorophenyl)piperazin-1-yl)piperidin-1-yl)methyl)-1-thioxoisoindolin-2-yl)piperidine-2,6-dione |
| GT-09935 | 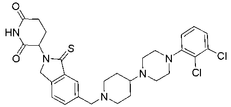 | 3-(6-((4-(4-(2,3-dichlorophenyl)piperazin-1-yl)piperidin-1-yl)methyl)-1-thioxoisoindolin-2-yl)piperidine-2,6-dione | 3-(6-((4-(4-(2,3-dichlorophenyl)piperazin-1-yl)piperidin-1-yl)methyl)-1-thioxoisoindolin-2-yl)piperidine-2,6-dione |
| GT-09687 | 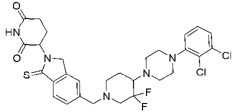 | 3-(5-((4-(4-(2,3-dichlorophenyl)piperazin-1-yl)-3,3-difluoropiperidin-1-yl)methyl)-1-thioxoisoindolin-2-yl)piperidine-2,6-dione | 3-(5-((4-(4-(2,3-dichlorophenyl)piperazin-1-yl)-3,3-difluoropiperidin-1-yl)methyl)-1-thioxoisoindolin-2-yl)piperidine-2,6-dione |
| GT-09575 | 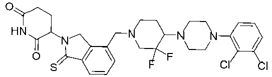 | 3-(4-((4-(4-(2,3-dichlorophenyl)piperazin-1-yl)-3,3-difluoropiperidin-1-yl)methyl)-1-thioxoisoindolin-2-yl)piperidine-2,6-dione | 3-(4-((4-(4-(2,3-dichlorophenyl)piperazin-1-yl)-3,3-difluoropiperidin-1-yl)methyl)-1-thioxoisoindolin-2-yl)piperidine-2,6-dione |
| GT-09693 | 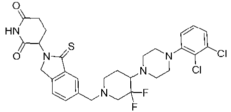 | 3-(6-((4-(4-(2,3-dichlorophenyl)piperazin-1-yl)-3,3-difluoropiperidin-1-yl)methyl)-1-thioxoisoindolin-2-yl)piperidine-2,6-dione | 3-(6-((4-(4-(2,3-dichlorophenyl)piperazin-1-yl)-3,3-difluoropiperidin-1-yl)methyl)-1-thioxoisoindolin-2-yl)piperidine-2,6-dione |
| GT-09688 | 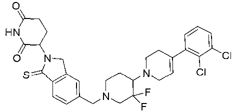 | 3-(5-((4-(4-(2,3-dichlorophenyl)-3,6-dihydropyridin-1(2H)-yl)-3,3-difluoropiperidin-1-yl)methyl)-1-thioxoisoindolin-2-yl)piperidine-2,6-dione | 3-(5-((4-(4-(2,3-dichlorophenyl)-3,6-dihydropyridin-1(2H)-yl)-3,3-difluoropiperidin-1-yl)methyl)-1-thioxoisoindolin-2-yl)piperidine-2,6-dione |
| GT-09614 | 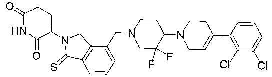 | 3-(4-((4-(4-(2,3-dichlorophenyl)-3,6-dihydropyridin-1(2H)-yl)-3,3-difluoropiperidin-1-yl)methyl)-1-thioxoisoindolin-2-yl)piperidine-2,6-dione | 3-(4-((4-(4-(2,3-dichlorophenyl)-3,6-dihydropyridin-1(2H)-yl)-3,3-difluoropiperidin-1-yl)methyl)-1-thioxoisoindolin-2-yl)piperidine-2,6-dione |
| GT-09694 | 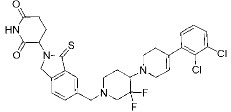 | 3-(6-((4-(4-(2,3-dichlorophenyl)-3,6-dihydropyridin-1(2H)-yl)-3,3-difluoropiperidin-1-yl)methyl)-1-thioxoisoindolin-2-yl)piperidine-2,6-dione | 3-(6-((4-(4-(2,3-dichlorophenyl)-3,6-dihydropyridin-1(2H)-yl)-3,3-difluoropiperidin-1-yl)methyl)-1-thioxoisoindolin-2-yl)piperidine-2,6-dione |
| GT-09861 | 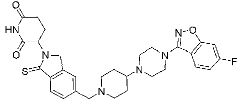 | 3-(5-((4-(4-(6-fluorobenzo[d]isoxazol-3-yl)piperazin-1-yl)piperidin-1-yl)methyl)-1-thioxoisoindolin-2-yl)piperidine-2,6-dione | 3-(5-((4-(4-(6-fluorobenzo[d]isoxazol-3-yl)piperazin-1-yl)piperidin-1-yl)methyl)-1-thioxoisoindolin-2-yl)piperidine-2,6-dione |
| GT-09940 | 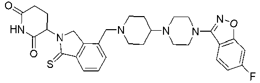 | 3-(4-((4-(4-(6-fluorobenzo[d]isoxazol-3-yl)piperazin-1-yl)piperidin-1-yl)methyl)-1-thioxoisoindolin-2-yl)piperidine-2,6-dione | 3-(4-((4-(4-(6-fluorobenzo[d]isoxazol-3-yl)piperazin-1-yl)piperidin-1-yl)methyl)-1-thioxoisoindolin-2-yl)piperidine-2,6-dione |
| GT-09864 | 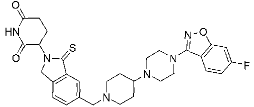 | 3-(6-((4-(4-(6-fluorobenzo[d]isoxazol-3-yl)piperazin-1-yl)piperidin-1-yl)methyl)-1-thioxoisoindolin-2-yl)piperidine-2,6-dione | 3-(6-((4-(4-(6-fluorobenzo[d]isoxazol-3-yl)piperazin-1-yl)piperidin-1-yl)methyl)-1-thioxoisoindolin-2-yl)piperidine-2,6-dione |
| GT-09689 | 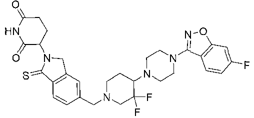 | 3-(5-((3,3-difluoro-4-(4-(6-fluorobenzo[d]isoxazol-3-yl)piperazin-1-yl)piperidin-1-yl)methyl)-1-thioxoisoindolin-2-yl)piperidine-2,6-dione | 3-(5-((3,3-difluoro-4-(4-(6-fluorobenzo[d]isoxazol-3-yl)piperazin-1-yl)piperidin-1-yl)methyl)-1-thioxoisoindolin-2-yl)piperidine-2,6-dione |
| GT-09615 | 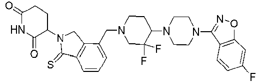 | 3-(4-((3,3-difluoro-4-(4-(6-fluorobenzo[d]isoxazol-3-yl)piperazin-1-yl)piperidin-1-yl)methyl)-1-thioxoisoindolin-2-yl)piperidine-2,6-dione | 3-(4-((3,3-difluoro-4-(4-(6-fluorobenzo[d]isoxazol-3-yl)piperazin-1-yl)piperidin-1-yl)methyl)-1-thioxoisoindolin-2-yl)piperidine-2,6-dione |
| GT-09695 | 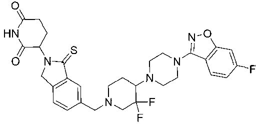 | 3-(6-((3,3-difluoro-4-(4-(6-fluorobenzo[d]isoxazol-3-yl)piperazin-1-yl)piperidin-1-yl)methyl)-1-thioxoisoindolin-2-yl)piperidine-2,6-dione | 3-(6-((3,3-difluoro-4-(4-(6-fluorobenzo[d]isoxazol-3-yl)piperazin-1-yl)piperidin-1-yl)methyl)-1-thioxoisoindolin-2-yl)piperidine-2,6-dione |
| GT-09690 | 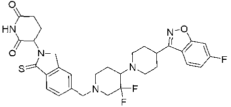 | 3-(5-((3',3'-difluoro-4-(6-fluorobenzo[d]isoxazol-3-yl)-[1,4'-bipiperidin]-1'-yl)methyl)-1-thioxoisoindolin-2-yl)piperidine-2,6-dione | 3-(5-((3',3'-difluoro-4-(6-fluorobenzo[d]isoxazol-3-yl)-[1,4'-bipiperidin]-1'-yl)methyl)-1-thioxoisoindolin-2-yl)piperidine-2,6-dione |
| GT-09616 | 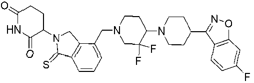 | 3-(4-((3',3'-difluoro-4-(6-fluorobenzo[d]isoxazol-3-yl)-[1,4'-bipiperidin]-1'-yl)methyl)-1-thioxoisoindolin-2-yl)piperidine-2,6-dione | 3-(4-((3',3'-difluoro-4-(6-fluorobenzo[d]isoxazol-3-yl)-[1,4'-bipiperidin]-1'-yl)methyl)-1-thioxoisoindolin-2-yl)piperidine-2,6-dione |
| GT-09936 | 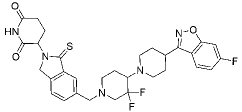 | 3-(6-((3',3'-difluoro-4-(6-fluorobenzo[d]isoxazol-3-yl)-[1,4'-bipiperidin]-1'-yl)methyl)-1-thioxoisoindolin-2-yl)piperidine-2,6-dione | 3-(6-((3',3'-difluoro-4-(6-fluorobenzo[d]isoxazol-3-yl)-[1,4'-bipiperidin]-1'-yl)methyl)-1-thioxoisoindolin-2-yl)piperidine-2,6-dione |
| GT-04941 | 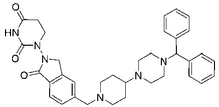 | 1-(5-((4-(4-benzhydrylpiperazin-1-yl)piperidin-1-yl)methyl)-1-oxoisoindolin-2-yl)dihydropyrimidine-2,4(1H,3H)-dione | 1-(5-((4-(4-benzhydrylpiperazin-1-yl)piperidin-1-yl)methyl)-1-oxoisoindolin-2-yl)dihydropyrimidine-2,4(1H,3H)-dione |
| GT-05255 | 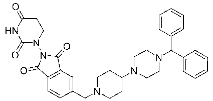 | 5-((4-(4-benzhydrylpiperazin-1-yl)piperidin-1-yl)methyl)-2-(2,4-dioxotetrahydropyrimidin-1(2H)-yl)isoindoline-1,3-dione | 5-((4-(4-benzhydrylpiperazin-1-yl)piperidin-1-yl)methyl)-2-(2,4-dioxotetrahydropyrimidin-1(2H)-yl)isoindoline-1,3-dione |
| GT-04942 | 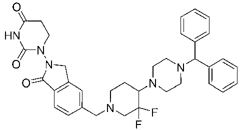 | 1-(5-((4-(4-benzhydrylpiperazin-1-yl)-3,3-difluoropiperidin-1-yl)methyl)-1-oxoisoindolin-2-yl)dihydropyrimidine-2,4(1H,3H)-dione | 1-(5-((4-(4-benzhydrylpiperazin-1-yl)-3,3-difluoropiperidin-1-yl)methyl)-1-oxoisoindolin-2-yl)dihydropyrimidine-2,4(1H,3H)-dione |
| GT-05256 | 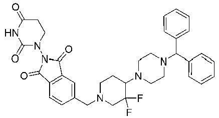 | 5-((4-(4-benzhydrylpiperazin-1-yl)-3,3-difluoropiperidin-1-yl)methyl)-2-(2,4-dioxotetrahydropyrimidin-1(2H)-yl)isoindoline-1,3-dione | 5-((4-(4-benzhydrylpiperazin-1-yl)-3,3-difluoropiperidin-1-yl)methyl)-2-(2,4-dioxotetrahydropyrimidin-1(2H)-yl)isoindoline-1,3-dione |
| GT-05082 | 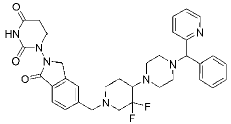 | 1-(5-((3,3-difluoro-4-(4-(phenyl(pyridin-2-yl)methyl)piperazin-1-yl)piperidin-1-yl)methyl)-1-oxoisoindolin-2-yl)dihydropyrimidine-2,4(1H,3H)-dione | 1-(5-((3,3-difluoro-4-(4-(phenyl(pyridin-2-yl)methyl)piperazin-1-yl)piperidin-1-yl)methyl)-1-oxoisoindolin-2-yl)dihydropyrimidine-2,4(1H,3H)-dione |
| GT-05257 | 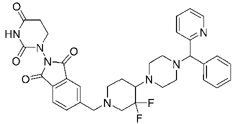 | 5-((3,3-difluoro-4-(4-(phenyl(pyridin-2-yl)methyl)piperazin-1-yl)piperidin-1-yl)methyl)-2-(2,4-dioxotetrahydropyrimidin-1(2H)-yl)isoindoline-1,3-dione | 5-((3,3-difluoro-4-(4-(phenyl(pyridin-2-yl)methyl)piperazin-1-yl)piperidin-1-yl)methyl)-2-(2,4-dioxotetrahydropyrimidin-1(2H)-yl)isoindoline-1,3-dione |
| GT-05335 | 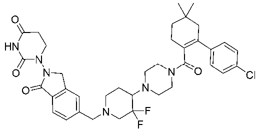 | 1-(5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-oxoisoindolin-2-yl)dihydropyrimidine-2,4(1H,3H)-dione | 1-(5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-oxoisoindolin-2-yl)dihydropyrimidine-2,4(1H,3H)-dione |
| GT-05271 | 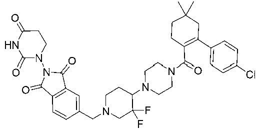 | 5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-2-(2,4-dioxotetrahydropyrimidin-1(2H)-yl)isoindoline-1,3-dione | 5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-2-(2,4-dioxotetrahydropyrimidin-1(2H)-yl)isoindoline-1,3-dione |
| GT-05334 | 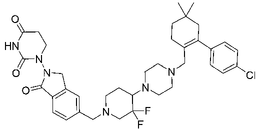 | 1-(5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-oxoisoindolin-2-yl)dihydropyrimidine-2,4(1H,3H)-dione | 1-(5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-oxoisoindolin-2-yl)dihydropyrimidine-2,4(1H,3H)-dione |
| GT-05270 | 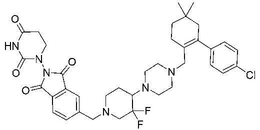 | 5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-2-(2,4-dioxotetrahydropyrimidin-1(2H)-yl)isoindoline-1,3-dione | 5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-2-(2,4-dioxotetrahydropyrimidin-1(2H)-yl)isoindoline-1,3-dione |
| GT-09355 | 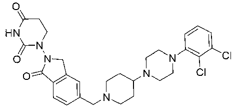 | 1-(5-((4-(4-(2,3-dichlorophenyl)piperazin-1-yl)piperidin-1-yl)methyl)-1-oxoisoindolin-2-yl)dihydropyrimidine-2,4(1H,3H)-dione | 1-(5-((4-(4-(2,3-dichlorophenyl)piperazin-1-yl)piperidin-1-yl)methyl)-1-oxoisoindolin-2-yl)dihydropyrimidine-2,4(1H,3H)-dione |
| GT-09357 | 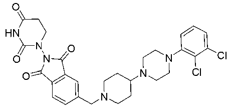 | 5-((4-(4-(2,3-dichlorophenyl)piperazin-1-yl)piperidin-1-yl)methyl)-2-(2,4-dioxotetrahydropyrimidin-1(2H)-yl)isoindoline-1,3-dione | 5-((4-(4-(2,3-dichlorophenyl)piperazin-1-yl)piperidin-1-yl)methyl)-2-(2,4-dioxotetrahydropyrimidin-1(2H)-yl)isoindoline-1,3-dione |
| GT-09205 | 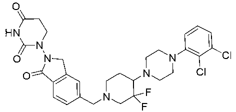 | 1-(5-((4-(4-(2,3-dichlorophenyl)piperazin-1-yl)-3,3-difluoropiperidin-1-yl)methyl)-1-oxoisoindolin-2-yl)dihydropyrimidine-2,4(1H,3H)-dione | 1-(5-((4-(4-(2,3-dichlorophenyl)piperazin-1-yl)-3,3-difluoropiperidin-1-yl)methyl)-1-oxoisoindolin-2-yl)dihydropyrimidine-2,4(1H,3H)-dione |
| GT-05258 | 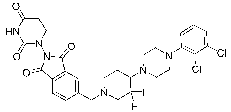 | 5-((4-(4-(2,3-dichlorophenyl)piperazin-1-yl)-3,3-difluoropiperidin-1-yl)methyl)-2-(2,4-dioxotetrahydropyrimidin-1(2H)-yl)isoindoline-1,3-dione | 5-((4-(4-(2,3-dichlorophenyl)piperazin-1-yl)-3,3-difluoropiperidin-1-yl)methyl)-2-(2,4-dioxotetrahydropyrimidin-1(2H)-yl)isoindoline-1,3-dione |
| GT-09206 | 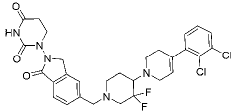 | 1-(5-((4-(4-(2,3-dichlorophenyl)-3,6-dihydropyridin-1(2H)-yl)-3,3-difluoropiperidin-1-yl)methyl)-1-oxoisoindolin-2-yl)dihydropyrimidine-2,4(1H,3H)-dione | 1-(5-((4-(4-(2,3-dichlorophenyl)-3,6-dihydropyridin-1(2H)-yl)-3,3-difluoropiperidin-1-yl)methyl)-1-oxoisoindolin-2-yl)dihydropyrimidine-2,4(1H,3H)-dione |
| GT-09358 | 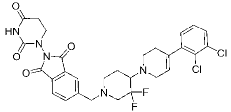 | 5-((4-(4-(2,3-dichlorophenyl)-3,6-dihydropyridin-1(2H)-yl)-3,3-difluoropiperidin-1-yl)methyl)-2-(2,4-dioxotetrahydropyrimidin-1(2H)-yl)isoindoline-1,3-dione | 5-((4-(4-(2,3-dichlorophenyl)-3,6-dihydropyridin-1(2H)-yl)-3,3-difluoropiperidin-1-yl)methyl)-2-(2,4-dioxotetrahydropyrimidin-1(2H)-yl)isoindoline-1,3-dione |
| GT-09356 | 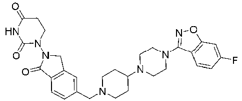 | 1-(5-((4-(4-(6-fluorobenzo[d]isoxazol-3-yl)piperazin-1-yl)piperidin-1-yl)methyl)-1-oxoisoindolin-2-yl)dihydropyrimidine-2,4(1H,3H)-dione | 1-(5-((4-(4-(6-fluorobenzo[d]isoxazol-3-yl)piperazin-1-yl)piperidin-1-yl)methyl)-1-oxoisoindolin-2-yl)dihydropyrimidine-2,4(1H,3H)-dione |
| GT-09359 | 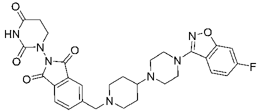 | 2-(2,4-dioxotetrahydropyrimidin-1(2H)-yl)-5-((4-(4-(6-fluorobenzo[d]isoxazol-3-yl)piperazin-1-yl)piperidin-1-yl)methyl)isoindoline-1,3-dione | 2-(2,4-dioxotetrahydropyrimidin-1(2H)-yl)-5-((4-(4-(6-fluorobenzo[d]isoxazol-3-yl)piperazin-1-yl)piperidin-1-yl)methyl)isoindoline-1,3-dione |
| GT-09207 | 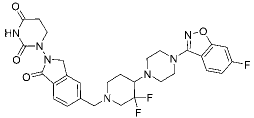 | 1-(5-((3,3-difluoro-4-(4-(6-fluorobenzo[d]isoxazol-3-yl)piperazin-1-yl)piperidin-1-yl)methyl)-1-oxoisoindolin-2-yl)dihydropyrimidine-2,4(1H,3H)-dione | 1-(5-((3,3-difluoro-4-(4-(6-fluorobenzo[d]isoxazol-3-yl)piperazin-1-yl)piperidin-1-yl)methyl)-1-oxoisoindolin-2-yl)dihydropyrimidine-2,4(1H,3H)-dione |
| GT-09360 | 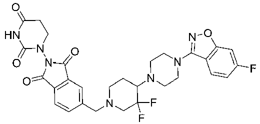 | 5-((3,3-difluoro-4-(4-(6-fluorobenzo[d]isoxazol-3-yl)piperazin-1-yl)piperidin-1-yl)methyl)-2-(2,4-dioxotetrahydropyrimidin-1(2H)-yl)isoindoline-1,3-dione | 5-((3,3-difluoro-4-(4-(6-fluorobenzo[d]isoxazol-3-yl)piperazin-1-yl)piperidin-1-yl)methyl)-2-(2,4-dioxotetrahydropyrimidin-1(2H)-yl)isoindoline-1,3-dione |
| GT-09208 | 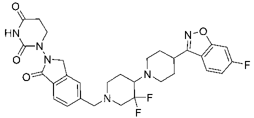 | 1-(5-((3',3'-difluoro-4-(6-fluorobenzo[d]isoxazol-3-yl)-[1,4'-bipiperidin]-1'-yl)methyl)-1-oxoisoindolin-2-yl)dihydropyrimidine-2,4(1H,3H)-dione | 1-(5-((3',3'-difluoro-4-(6-fluorobenzo[d]isoxazol-3-yl)-[1,4'-bipiperidin]-1'-yl)methyl)-1-oxoisoindolin-2-yl)dihydropyrimidine-2,4(1H,3H)-dione |
| GT-09361 | 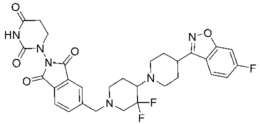 | 5-((3',3'-difluoro-4-(6-fluorobenzo[d]isoxazol-3-yl)-[1,4'-bipiperidin]-1'-yl)methyl)-2-(2,4-dioxotetrahydropyrimidin-1(2H)-yl)isoindoline-1,3-dione | 5-((3',3'-difluoro-4-(6-fluorobenzo[d]isoxazol-3-yl)-[1,4'-bipiperidin]-1'-yl)methyl)-2-(2,4-dioxotetrahydropyrimidin-1(2H)-yl)isoindoline-1,3-dione |

### II. Other Forms of Compounds (including salts, enantiomers, stereoisomers, solvates, isotopically enriched analogs, prodrugs, or polymorphs of compounds)

The compounds of the present disclosure have the structures of any one of Formula (I), Formula (I-1), Formula (I-2), Formula (I-3), Formula (I-4), Formula (I-5), Formula (I-5-1), Formula (I-5-1A), Formula (I-5-1B), Formula (I-5-2), Formula (I-5-2A), Formula (I-5-2B), or Formula (I-5-3). Unless otherwise specified, all references to the compounds of the present disclosure also include compounds of any one of Formula (I), Formula (I-1), Formula (I-2), Formula (I-3), Formula (I-4), Formula (I-5), Formula (I-5-1), Formula (I-5-1A), Formula (I-5-1B), Formula (I-5-2), Formula (I-5-2A), Formula (I-5-2B), or Formula (I-5-3) and specific compounds within the scope of these general formulae.

It should be recognized that the compounds of the present disclosure (including the compounds of Formula (I), Formula (I-1), Formula (I-2), Formula (I-3), Formula (I-4), Formula (I-5), Formula (I-5-1), Formula (I-5-1A), Formula (I-5-1B), Formula (I-5-2), Formula (I-5-2A), Formula (I-5-2B), or Formula (I-5-3)) may have a stereo-configuration and thus can exist in more than one stereoisomeric form. The present disclosure also relates to optically enriched compounds having a stereo-configuration, e.g., greater than about 90% enantiomeric/diastereomeric excess ("ee"), such as about 95% ee or 97% ee, or greater than 99% ee, and mixtures thereof, including racemic mixtures. As used herein, "optically enriched" means that a mixture of enantiomers consists of a significantly greater proportion of one enantiomer, and can be described by enantiomeric excess (ee%). Purification of isomers and separation of mixtures of isomers can be accomplished by standard techniques known in the art (e.g., column chromatography, preparative TLC, preparative HPLC, asymmetric synthesis (e.g., by using chiral intermediates) and/or or chiral resolution, etc.).

In some embodiments, polymorph forms or salts of the compounds of the present disclosure are also provided. Salts of the compounds of the present disclosure may be pharmaceutically acceptable salts including, but not limited to, hydrohalide (including hydrochloride, hydrobromide), sulfate, citrate, maleate, besylate, glycolate, α-D-glucoheptonate, D-gluconate, L-lactate, L-malate, malonate, mandelate, phosphate, propionate, succinate, tartrate, tosylate, valerate, palmitate, sebacate, stearate, laurate, acetate, adipate, carbonate, 4-chlorobenzenesulfonate, edisylate, fumarate, D-glucuronate, α-ketoglutarate, hippurate, 2-hydroxyethanesulfonate, lactobionate, 2-naphthalenesulfonate, oleate, cholate, terephthalate, L-ascorbate, camphorate, ethanesulfonate, formate, hydrobromide, methanesulfonate, oxalate, benzoate, salicylate, pyruvate, dihydrogen phosphate, pyrophosphate, metaphosphate, *p*-chlorobenzenesulfonate, 1,5-naphthalenedisulfonate, 3-hydroxy-2-naphthoate, 1-hydroxy-2-naphthoate, 2-naphthalenesulfonate, trifluoroacetate, and hydroxyacetate, etc., of the compounds of the present disclosure. The compounds of the present disclosure can exist in non-solvated or solvated forms with pharmaceutically acceptable solvents such as water, ethanol, and the like. In some embodiments, the compounds of the present disclosure can be prepared as prodrugs or precursor drugs. Prodrugs can be converted into the parent drug in vivo to exert their therapeutic effects. In some embodiments, isotopically-labeled compounds of the present disclosure are also provided, examples of which include deuterium (D or ²H).

### III. Compositions/Formulations

In some embodiments, the present disclosure provides a pharmaceutical composition comprising as active ingredient the compound of the present disclosure or a pharmaceutically acceptable salt, solvate, isotopically enriched analog, polymorph, prodrug, stereoisomer (including enantiomer), or mixture of stereoisomers thereof, and at least one pharmaceutically acceptable carrier.

In some embodiments, pharmaceutically acceptable carriers include, but are not limited to, fillers, stabilizers, dispersants, suspending agents, diluents, excipients, thickeners, colorants, solvents, or encapsulating materials. Each carrier must be "acceptable" in the sense of being compatible with the other ingredients of the formulation (including the compounds useful in the present disclosure) and not injurious to the patient. Some examples of materials that can be used as pharmaceutically acceptable carriers include: sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; polyethylene oxide, polyvinylpyrrolidone, polyacrylamide, poloxamer; and other common non-toxic compatible substances used in pharmaceutical formulations.

The pharmaceutical composition of the present disclosure can further comprise at least one second therapeutic agent, e.g., an anticancer agent. The second therapeutic agent may be used in combination with the compounds of Formula (I) described in the present disclosure to treat the diseases or disorders as disclosed herein. The second therapeutic agent includes, but is not limited to, chemotherapeutic agents, immunotherapeutic agents, gene therapy agents, and the like.

The pharmaceutical composition of the present disclosure comprising, as an active ingredient, the compounds of Formula (I) of the present disclosure or a pharmaceutically acceptable salt thereof can be formulated into any suitable formulations such as sprays, patches, tablets (such as conventional tablets, dispersible tablets, orally disintegrating tablets), capsules (such as soft capsules, hard capsules, enteric-coated capsules), dragees, troches, powders, granules, powder injections, suppositories, or liquid formulations (such as suspensions (e.g., aqueous or oily suspensions), solutions, emulsions, or syrups), or conventional injection dosage forms such as injectable solutions (e.g., sterile injectable solutions formulated according to methods known in the art using water, Ringer's solution, or isotonic sodium chloride solution or the like as a vehicle or solvent) or lyophilized injectable formulation and the like, depending upon a suitable route of administration (including, but not limited to, nasal administration, inhalation administration, topical administration, oral administration, oral mucosal administration, rectal administration, intrapleural administration, intraperitoneal administration, vaginal administration, intramuscular administration, subcutaneous administration, transdermal administration, epidural administration, intrathecal administration, and intravenous administration). Those skilled in the art can also formulate the compounds of Formula (I) of the present disclosure into conventional, dispersible, chewable, orally disintegrating or rapidly dissolving formulations, or sustained-release capsules or controlled-release capsules as needed.

The compounds of Formula (I) of the present disclosure, as an active ingredient, is contained in the pharmaceutically acceptable carrier or diluent in an amount sufficient to deliver to a subject a therapeutically effective amount for the indication to be treated, without causing serious toxic effects in the subject treated. A dosage of the active compound for all diseases or disorders mentioned herein ranges, for example, from about 5ng/kg to 500mg/kg, from about 10ng/kg to 300mg/kg per day, such as from 0.1 to 100 mg/kg, or from 0.5 to about 25mg per kilogram body weight of the subject per day.

The compounds of Formula (I) of the present disclosure or pharmaceutically acceptable salts thereof may be conveniently administered in any suitable unit dosage form. Suitable unit dosage form specifications include, but are not limited to, less than 1mg, 1mg to 3000mg, 5mg to 1000mg, for example, 5 to 500mg, 25 to 250mg of active ingredient per unit dosage form.

### IV. Kits/Packaged Products

The compounds of Formula (I) of the present disclosure or a pharmaceutically acceptable salt, solvate, isotopically enriched analog, polymorph, prodrug, stereoisomer (including enantiomer), or mixture of stereoisomers thereof is used as a medicament. The medicament of the present disclosure or the pharmaceutical composition of the present disclosure may be presented in a kit/packaged product. The kit/packaged product may include a package or container including, but not limited to, ampoules, blister packs, pharmaceutical plastic bottles, vials, pharmaceutical glass bottles, containers, syringes, laminated flexible packaging, co-extruded film infusion containers, test tubes and dispensing devices, and the like. The kit/packaged product may contain instructions for use of the product.

### V. Treatment Methods and Uses

The compounds of Formula (I) or a pharmaceutically acceptable salt, solvate, isotopically enriched analog, polymorph, prodrug, stereoisomer (including enantiomer), or mixture of stereoisomers thereof can be used as a medicament. Especially the compound of Formula (I) of the present disclosure or a pharmaceutically acceptable salt, solvate, isotopically enriched analog, polymorph, prodrug, stereoisomer (including enantiomer), or mixture of stereoisomers thereof can be used for the manufacture of a medicament for the prevention and/or treatment of a disease or disorder. The disease or disorder includes a disease or disorder associated with a cereblon protein.

In some embodiments, the disease or disorder comprises: tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, and diabetes.

In some embodiments, the disease or disorder includes, but is not limited to, myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myeloid leukemia (CML), chronic myelogenous leukemia, acute lymphoblastic leukemia (ALL), chronic lymphocytic leukemia (CLL), B-cell chronic lymphocytic leukemia, leukemia-associated anemia, acute B-lymphoblastic leukemia, T-lymphoblastic leukemia, acute T-lymphoblastic leukemia, lymphoma cell leukemia, monocytic leukemia, myelomonocytic leukemia; lymphoma, including diffuse large B-cell lymphoma, non-Hodgkin's lymphoma, Hodgkin's lymphoma, anaplastic lymphoma, anaplastic large cell lymphoma, immunoblastic T-Cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; Burkitt's lymphoma (Burkitt's lymphoma); thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, psoriatic arthritis, systemic lupus erythematosus, multiple sclerosis, recurrent oral ulcers, Kawasaki disease, polymyositis/dermatomyositis, Sjogren's syndrome, atopic dermatitis, hidradenitis suppurativa, gout, type I diabetes, urticaria, inflammatory bowel disease (including Crohn's disease and ulcerative colitis); keratoconjunctivitis; inflammatory diseases, including pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; and acute liver failure.

The present disclosure provides a method for preventing and/or treating a disease or disorder in a subject, comprising administering to the subject a therapeutically effective amount of the compounds of Formula (I) of the present disclosure or a pharmaceutically acceptable salt thereof, or the pharmaceutical composition of the present disclosure comprising as an active ingredient the compounds of Formula (I) of the present disclosure or a pharmaceutically acceptable salt thereof. In some embodiments, In some embodiments, the disease or disorder includes a disease or disorder associated with a cereblon protein. The disease or disorder is as defined herein.

In the method for preventing and/or treating a disease or disorder (associated with a cereblon protein) in a subject, the compound of Formula (I) of the present disclosure or the pharmaceutical composition comprising as an active ingredient the compound of Formula (I) of the present disclosure is administered to the subject through at least one mode of administration selected from the group consisting of nasal administration, inhalation administration, topical administration, oral administration, oral mucosal administration, rectal administration, pleural cavity administration, peritoneal administration, intramuscular administration, subcutaneous, transdermal, epidural, intrathecal, and intravenous administration.

The term "treatment" or "treating" refers to administering to a subject the compound of Formula (I) of the present disclosure, or a pharmaceutically acceptable salt thereof, or the pharmaceutical composition comprising, as an active ingredient, the compound of Formula (I) of the present disclosure or a pharmaceutically acceptable salt thereof, to mitigate (alleviate) undesirable diseases or conditions, such as the development of a cancer or tumor. The beneficial or desired clinical results of the present disclosure include, but are not limited to: alleviating symptoms, reducing the severity of the disease, stabilizing the state of the disease, slowing down or delaying the progression of the disease, improving or alleviating the condition, and alleviating the disease.

A "therapeutic effective amount" of the compound of the present disclosure depends on a variety of factors, including the activity of the specific compound used, the metabolic stability of the compound and the duration of its action, the age, sex and weight of the patient, the patient's current medical condition, the route and duration of administration, the excretion rate, the combined administration of additional drugs, and the progression of the diseases or conditions of the patient being treated. Those skilled in the art will be able to determine appropriate dosages based on these and other factors.

It is to be understood that the choice of using one or more active compounds and/or compositions and their dosage depends on the basic situations of the individual (which should generally render the individual situation to achieve the best effect). Dosing and dosing regimens should be within the ability of those skilled in the art, and the appropriate dosage depends on many factors including the knowledge and ability of the physicians, veterinarians or researchers (see e.g., Jun Li (chief editor), "Clinical Pharmacology", 4th edition, People's Public Health Press, 2008).

As used herein, the term "patient" or "subject" to be treated refers to animal, for example mammal, including but not limited to primate (such as human being), cow, sheep, goat, horse, dog, cat, rabbit, guinea pig, rat, mice, etc.

### VI. Preparation Method

The compound of Formula (I) of the present disclosure may be prepared by reacting a compound of Formula (M1) with a compound of Formula (M2): 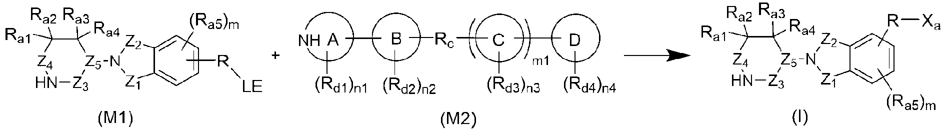
wherein Rₐ₁, Rₐ₂, Rₐ₃, Rₐ₄, (Rₐ₅)ₘ, Z₁, Z₂, Z₃, Z₄, Z₅, (R_{d1})ₙ₁, ring B, (R_{d2})ₙ₂, R_{c}, ring C, m1, (R_{d3})ₙ₃, ring D, and (R_{d4})ₙ₄ are as defined in the compound of Formula (I) and its various sub-embodiments in the present disclosure;
nitrogen-containing heterocycle A represents a 4- to 30-membered nitrogen-containing heterocycle (preferably a 4- to 20-membered nitrogen-containing heterocycle; more preferably a 4- to 15-membered nitrogen-containing heterocycle);
R represents optionally substituted linear or branched C₁₋₅ alkylene;
LE represents Cl, Br, I, methanesulfonyloxy, p-toluenesulfonyloxy, o-nitrobenzenesulfonyl, C(O)Cl, or COOH; and
Xₐ of the compound of Formula (I) correspondingly represents a structure represented by the following formula: 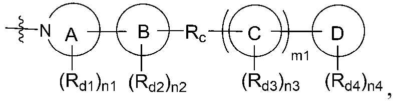
wherein nitrogen-containing heterocyclylene A represents 4- to 30-membered nitrogen-containing heterocyclylene (preferably, 4- to 20-membered nitrogen-containing heterocyclylene; more preferably, 4- to 15-membered nitrogen-containing heterocyclylene),
(R_{d1})ₙ₁, ring B, (R_{d2})ₙ₂, R_{c}, ring C, m1, (R_{d3})ₙ₃, ring D, and (R_{d4})ₙ₄ are as defined in the compound of Formula (I) and its various sub-embodiments in the present disclosure.

In some embodiments of the method for preparing the compound of Formula (I) of the present disclosure, R represents -CH₂-, -(CH₂)₂-, -(CH₂)₃-, -(CH₂)₄-, or -(CH₂)₅-; wherein the above-mentioned group is optionally substituted with a substituent selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, and C₂₋₆ alkenyl.

In some embodiments of the method for preparing the compound of Formula (I) of the present disclosure, the nitrogen-containing heterocycle A, after removal of the hydrogen atom on the nitrogen atom through a reaction, gives a nitrogen-containing heterocyclylene A. In some embodiments, the nitrogen-containing heterocycle A represents 4- to 30-membered nitrogen-containing heterocycle (preferably, 4- to 20-membered nitrogen-containing heterocycle; more preferably, 4- to 15-membered nitrogen-containing heterocycle). The nitrogen-containing heterocyclylene A represents 4- to 30-membered nitrogen-containing heterocyclylene (preferably, 4-to 20-membered nitrogen-containing heterocyclylene; more preferably, 4- to 15-membered nitrogen-containing heterocyclylene).

In some embodiments of the method for preparing the compound of Formula (I) of the present disclosure, when LE represents Cl, Br, I, methanesulfonyloxy, p-toluenesulfonyloxy, or o-nitrobenzenesulfonyl, the compound of Formula (M1) and the compound of Formula (M2) undergo an amine alkylation reaction. In some embodiments, the amine alkylation reaction may be carried out, for example, in the presence of an organic base and sodium iodide at a temperature ranging from room temperature to 80°C (e.g., 40°C to 60°C, 40°C to 50°C, or 50°C to 60°C). Examples of the organic base include, but are not limited to, DIEA or triethylamine. In some embodiments, the molar ratio of the compound of Formula (M1) to the compound of Formula (M2) may be, for example, 1:1.0 to 2, 1:1.1 to 2, 1:1.1 to 1.5, 1:1.1 to 1.2, or 1:1.2 to 1.3, and the like.

In some embodiments of the method for preparing the compound of Formula (I) of the present disclosure, when LE represents COOH, the compound of Formula (M1) and the compound of Formula (M2) undergo an amide condensation reaction. In some embodiments, the amide condensation reaction may be carried out, for example, in the presence of an organic base and HATU at room temperature. Examples of the organic base include, but are not limited to, DIEA or triethylamine. In some embodiments, the molar ratio of the compound of Formula (M1) to the compound of Formula (M2) may be, for example, 1:1.0 to 2, 1:1.1 to 2, 1:1.1 to 1.5, 1:1.1 to 1.2, or 1:1.2 to 1.3, and the like.

In some embodiments of the method for preparing the compound of Formula (I) of the present disclosure, when the group LE represents methanesulfonyloxy, the compound of Formula (M1) is prepared by reacting a compound of Formula (M3) with methanesulfonic anhydride or methanesulfonyl chloride: 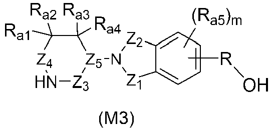 wherein Rₐ₁, Rₐ₂, Rₐ₃, Rₐ₄, (Rₐ₅)ₘ, Z₁, Z₂, Z₃, Z₄, Z₅, and R are as defined in the compound of Formula (I) and its various sub-embodiments in the present disclosure. In some embodiments, R may represent CH₂.

In some embodiments of the method for preparing the compound of Formula (I) of the present disclosure, when the group LE represents Cl, Br or I, the compound of Formula (M1) may be prepared by reacting a compound of Formula (M3) with a hydrohalic acid through a halogenation reaction: 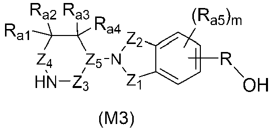 wherein Rₐ₁, Rₐ₂, Rₐ₃, Rₐ₄, (Rₐ₅)ₘ, Z₁, Z₂, Z₃, Z₄, Z₅, and R are as defined in the compound of Formula (I) and its various sub-embodiments in the present disclosure. In some embodiments, R may represent CH₂.

In some embodiments of the method for preparing the compound of Formula (I) of the present disclosure, the compound of Formula (M3), wherein R represents CH₂, may be prepared by subjecting a compound of Formula (M4) to a coupling reaction with (tributylstannyl)methanol in the presence of a palladium catalyst: 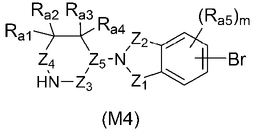 wherein Rₐ₁, Rₐ₂, Rₐ₃, Rₐ₄, (Rₐ₅)ₘ, Z₁, Z₂, Z₃, Z₄, and Z₅ are as defined in the compound of Formula (I) and its various sub-embodiments in the present disclosure.

In some embodiments, the coupling reaction may be carried out, for example, in the presence of tetrakis(triphenylphosphine)palladium and 1,4-dioxane (or DMF) at a temperature ranging from 40°C to 120°C.

In some embodiments, when Z₅ in the compound of Formula (M4) represents N, and Zₗ represents C(O), CH₂, or CD₂, and Z₂, Z₃, and Z₄ each independently represent C(O), the compound of Formula (M4) is prepared by subjecting a compound of Formula (M5) and a compound of Formula (M6) to an amine-ester exchange reaction: 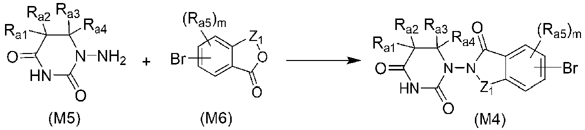 wherein Rₐ₁, Rₐ₂, Rₐ₃, Rₐ₄, and (Rₐ₅)ₘ are as defined in the compound of Formula (I) and its various sub-embodiments in the present disclosure.

In some embodiments, the amine-ester exchange reaction may be carried out in the presence of CH₃ONa and methanol at room temperature. Alternatively, the amine-ester exchange reaction may also be carried out in the presence of CH₃OLi or Cs₂CO₃ and methanol at a temperature ranging from 40°C to 90°C, or in the presence of DBU and THF at a temperature ranging from 40°C to 90°C.

In some embodiments, when Z₁ in the compound of Formula (M4) represents C(O), C(S), CH₂, or CD₂; Z₂, Z₃, and Z₄ each independently represent C(O) or C(S); and at least one of Z₁, Z₂, Z₃, and Z₄ represents C(S), the compound of Formula (M4) is prepared by subjecting a compound of Formula (M7) to a thionation reaction with a thionating reagent: 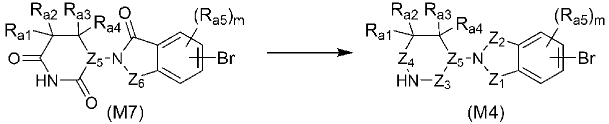 wherein Z₆ in the compound of Formula (M7) represents C(O), CH₂, or CD₂; and the Rₐ₁, Rₐ₂, Rₐ₃, Rₐ₄, and (Rₐ₅)ₘ are as defined in the compound of Formula (I) and its various sub-embodiments in the present disclosure. When Z₆ in the starting material compound of Formula (M7) represents CH₂ or CD₂, the corresponding Z₁ in the product thiocarbonyl compound of Formula (M4) represents CH₂ or CD₂. When Z₆ in the starting material compound of Formula (M7) represents C(O), the corresponding Z₁ in the product thiocarbonyl compound of Formula (M4) represents C(O) or C(S).

In some embodiments, examples of the thionating reagent include, but are not limited to, carbon disulfide, hexamethyldisilathiane, sulfur, thiourea, hydrogen sulfide, phosphorus pentasulfide, Lawesson's Reagent (CAS No.:19172-47-5), Belleau's reagent (CAS No.: 88816-02-8), and Davy's reagent (CAS No.: 82737-61-9) etc.

In some embodiments, the compound of Formula (M7) is reacted with the thionating reagent (e.g., Lawesson's reagent) in a solvent (e.g., 1,4-dioxane) at a temperature ranging from 40°C to 150°C to give the thiocarbonyl compound of Formula (M4).

In some embodiments, at least one of Z₁, Z₂, Z₃, and Z₄ in the thiocarbonyl compound of Formula (M4) represents C(S). In some embodiments, at least two of Z₁, Z₂, Z₃, and Z₄ in the thiocarbonyl compound of Formula (M4) represent C(S). In some embodiments, at least three of Z₁, Z₂, Z₃, and Z₄ represent C(S). In some embodiments, all of Z₁, Z₂, Z₃, and Z₄ represent C(S).

### VII. Definitions

Unless otherwise specified, the following words, phrases and symbols used herein generally have the meanings as described below.

In general, the nomenclature used herein (including the IUPAC nomenclature) and the laboratory procedures described below (including those used in cell culture, organic chemistry, analytical chemistry, and pharmacology, etc.) are those well-known and commonly used in the art. Unless otherwise defined, all scientific and technical terms used herein in connection with the present disclosure described herein have the same meaning as commonly understood by one skill in the art. In addition, the use of the word "a" or "an" when used in conjunction with the term "comprising" or a noun in the claims and/or the specification may mean "one", but it is also consistent with the meaning of "one or more", "at least one", and "one or more than one". Similarly, the terms "another" or "other" can mean at least a second or more.

It should be understood that whenever the term "comprise" or "include" is used herein to describe various aspects, other similar aspects described by "consisting of" and/or "consisting essentially of' are also provided.

As used herein, the term "about" used alone or in combination refers to approximately, roughly, nearly, or around. When the term "about" is used in conjunction with a numerical range, it modifies that range by extending the boundaries above and below the stated numerical value. In general, the term "about" can modify a numerical value above and below the stated value by an upward or downward (increasing or decreasing) variation, e.g., 10%, 5%, 2%, or 1%.

As used herein, the wording "...represents a bond" used alone or in combination means that the referenced group is a bond linker (that is, the referenced group is absent). For example, the wording "R_{c} represents a bond" means that R_{c} is a bond linker. In other words, when R_{c} represents a bond, the ring B in the structure of Formula (I) is directly connected to the ring C in the structure of Formula (I).

In this document, the term "optionally substituted" used alone or in combination means that the referenced group may be unsubstituted or substituted with one or more substituents as defined here. Herein, the wording "optionally substituted with..." and "unsubstituted or substituted" can be used interchangeably. The term "substituted" generally represents the replacement of one or more hydrogen atoms in the referenced structure by identical or different specific substituents. The number of substituents is not theoretically limited in any way, or is automatically limited by the size of the building units (i.e., the total number of replaceable hydrogen atoms in the building units), or as clearly defined herein.

Herein, a bond interrupted by a wavy line shows the point of attachment of the depicted group to the rest of the molecule. For example, the monovalent group X depicted below 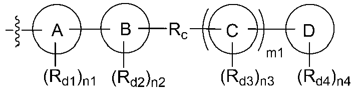 shows the point of attachment of ring A in said group X to group R in the structure of Formula (I).

As used herein, the term "one or more" in the expression "substituted with one or more substituents selected from the group consisting of ...", whether used alone or in combination, means that some or all of the hydrogen atoms of the referenced group are replaced by a substituent or substituents. The number of substituents includes but is not limited to 1 to 40, such as 1 to 30, 1 to 25, 1 to 20, 1 to 15, 1 to 10, 1 to 5, 1 to 4, 1 to 3, 1 to 2, or 1. The number of hydrogens to be replaced is in principle not limited in any way, or is automatically limited by the size of the building unit. For example, where the referenced group is a methyl group, the number of substituents may be 1 to 3.

As used herein, the term "deuterated" used alone or in combination, indicates that one or more hydrogen atoms in the referenced group are replaced by deuterium atoms.

As used herein, the term "oxo" or "oxo group" used alone or in combination, refers to =O.

As used herein, the term "C(O)" or "C(=O)" used alone or in combination, refers to a carbonyl group.

As used herein, the term "thiocarbonyl", whether used alone or in combination, refers to C(S) or C(=S).

As used herein, the term "halogen atom" or "halogen", used alone or in combination, refers to fluorine, chlorine, bromine, or iodine.

As used herein, the term "alkyl", used alone or in combination, refers to a linear or branched alkyl group. The term "Cₓ-C_{y} alkyl" or "C_{x-y} alkyl" (x and y each being an integer) refers to a linear or branched alkyl group containing from x to y carbon atoms. The term "C₁₋₁₀ alkyl" used alone or in combination in the present disclosure refers to a linear or branched alkyl group containing from 1 to 10 carbon atoms. Examples of the C₁₋₁₀ alkyl of the present disclosure may include a C₁₋₉ alkyl, C₁₋₈ alkyl, C₂₋₈ alkyl, C₁₋₇ alkyl, C₁₋₆ alkyl, C₁₋₅ alkyl, C₁₋₄ alkyl, C₁-C₃ alkyl, and C₁-C₂ alkyl. Representative examples include methyl, ethyl, propyl, isopropyl, butyl, isobutyl, sec-butyl, tert-butyl, pentyl, isopentyl, neopentyl, tert-pentyl, and hexyl. The term "C₁₋₃ alkyl" or "C₁-C₃ alkyl" in the present disclosure refers to an alkyl group containing from 1 to 3 carbon atoms, and representative examples thereof include methyl, ethyl, propyl, and isopropyl. In the present disclosure, the "alkyl" is optionally substituted, and the substituents are optionally one or more (e.g., 1-10, 1-6, 1-5, 1-4, or 1-3) substituents selected from the group consisting of e.g., halogen, hydroxyl, cyano, C₁₋₃ alkyl, C₁₋₃ alkoxy, halogenated C₁₋₆ alkoxy, halogenated C₁₋₆ alkyl (e.g., trifluoromethyl), C₃₋₆ cycloalkyl, and 4- to 7-membered heterocyclyl.

As used herein, the term "halogenated alkyl", used alone or in combination, refers to a linear or branched alkyl group substituted with one or more halogens, wherein one or more hydrogen atoms of the alkyl group are replaced with one or more halogens. The term "halogenated Cₓ₋C_{y} alkyl" or "halogenated C_{x-y} alkyl" (x and y are each an integer) refers to a linear or branched alkyl containing from x to y carbon atoms substituted with one or more halogens. The term "halogenated C₁₋₁₀ alkyl" used alone or in combination in the present disclosure refers to a linear or branched alkyl group containing from 1 to 10 carbon atoms substituted with one or more halogens. Examples of the halogenated C₁₋₁₀ alkyl group of the present disclosure include halogenated C₁₋₉ alkyl group, e.g., halogenated C₁₋₈ alkyl group, halogenated C₂₋₈ alkyl group, halogenated C₁₋₇ alkyl group, halogenated C₁₋₆ alkyl, halogenated C₁₋₅ alkyl, or halogenated C₁₋₄ alkyl. Representative examples include halomethyl, haloethyl, halopropyl, haloisopropyl, halobutyl, haloisobutyl, halo-sec-butyl, halo-tert-butyl, halopentyl, haloisoamyl, haloneopentyl, halo-tert-pentyl, halohexyl, haloheptyl, halooctyl, halononyl, and halodecyl. The term "halogenated C₁₋₃ alkyl" or "halogenated C₁₋C₃ alkyl" of the present disclosure refers to an alkyl group containing from 1 to 3 carbon atoms substituted with one or more halogens, and its representative examples include halomethyl (e.g., trifluoromethyl), haloethyl, halopropyl and haloisopropyl.

As used herein, the term "deuterated alkyl", used alone or in combination, refers to a linear or branched alkyl group substituted with one or more deuterium atoms, wherein one or more hydrogen atoms of the alkyl group are replaced with one or more deuterium atoms. The term "deuterated Cₓ₋C_{y} alkyl" or "deuterated C_{x-y} alkyl" (x and y are each an integer) refers to a linear or branched alkyl containing from x to y carbon atoms substituted with one or more deuterium atoms. The term "deuterated C₁₋₁₀ alkyl" used alone or in combination in the present disclosure refers to a linear or branched alkyl group containing from 1 to 10 carbon atoms substituted with one or more deuterium atoms. Examples of the deuterated C₁₋₁₀ alkyl group of the present disclosure include deuterated C₁₋₉ alkyl group, e.g., deuterated C₁₋₈ alkyl group, deuterated C₂₋₈ alkyl group, deuterated C₁₋₇ alkyl group, deuterated C₁₋₆ alkyl, deuterated C₁₋₅ alkyl, or deuterated C₁₋₄ alkyl. Representative examples include perdeuterated methyl (CD₃), perdeuterated ethyl (CD₃CD₂), perdeuterated propyl, perdeuterated isopropyl, perdeuterated butyl, perdeuterated isobutyl, perdeuterated sec-butyl, perdeuterated tert-butyl, perdeuterated pentyl, perdeuterated isopentyl, perdeuterated neopentyl, perdeuterated tert-pentyl, and perdeuterated hexyl. The term "deuterated C₁₋₃ alkyl" or "deuterated C₁₋C₃ alkyl" of the present disclosure refers to an alkyl group containing from 1 to 3 carbon atoms substituted with one or more deuterium atoms, and its representative examples include perdeuterated methyl (CD₃) and perdeuterated ethyl (CD₃CD₂).

As used herein, the term "alkylene" (which is used interchangeably with "alkylene chain"), used alone or in combination, refers to a linear or branched divalent saturated hydrocarbon group composed of carbon and hydrogen atoms, which is the divalent form of a linear or branched alkyl group. The term "Cₓ-C_{y} alkylene" or "C_{x-y} alkylene" (x and y each being an integer) refers to a linear or branched alkylene group containing from x to y carbon atoms. The term "C₁-C₅ alkylene" as used herein, whether alone or in combination, refers to a linear or branched alkylene containing 1 to 5 carbon atoms, examples of which include, but are not limited to, C₁-C₄ alkylene, C₁-C₃ alkylene, and C₁-C₂ alkylene. Representative examples include, but are not limited to, methylene (i.e., -CH₂-), ethylene (e.g., -CH₂CH₂-), propylene, isopropylene, butylene, isobutylene, sec-butylene, tert-butylene, pentylene, isopentylene, neopentylene, and tert-pentylene. In the present disclosure, the "alkylene" is optionally substituted, and the substituents are optionally one or more (e.g., 1-10, 1-6, 1-5, 1-4, or 1-3) substituents selected from the group consisting of e.g., deuterium, hydroxy, amino, mercapto, nitro, halogen, cyano, oxo, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, and C₂₋₆ alkenyl.

As used herein, the term "alkoxy", used alone or in combination, refers to a linear or branched alkoxy group having structural formula of alkyl-O-. Optionally, the alkyl portion of the alkoxy group may contain 1-10 (e.g., 1-6, 1-4, or 1-3) carbon atoms. Representative examples of "alkoxy" include, but are not limited to, methoxy, ethoxy, propoxy, isopropoxy, butoxy, isobutoxy, tert-butoxy, pentyloxy, 2-pentyloxy, isopentyloxy, neopentyloxy, hexyloxy, 2-hexyloxy, 3-hexyloxy, 3-methylpentyloxy, etc. The term "C₁-C₆ alkoxy" or "C₁₋₆ alkoxy" refers to a linear or branched alkoxy group containing from 1 to 6 carbon atoms. Representative examples of C₁₋₆ alkoxy include, but are not limited to, methoxy, ethoxy, propoxy, isopropoxy, butoxy, pentyloxy, and hexyloxy.

As used herein, the term "halogenated alkoxy", used alone or in combination, refers to an alkoxy group substituted with one or more halogen atoms. Optionally, the alkyl portion of the alkoxy group may contain 1-10 (e.g., 1-6, 1-4, or 1-3) carbon atoms. Examples of "halogenated alkoxy" include halogenated C₁₋₆ alkoxy or halogenated C₁₋₄ alkoxy. Representative examples include, but are not limited to, F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-.

As used herein, the term "heteroaryl", used alone or in combination, refers to a 5- to 30-membered (e.g., 5- to 20-membered, 5- to 15-membered, 5- to 12-membered, 5- to 11-membered, 5-to 10-membered, 5- to 9-membered, 5- to 8-membered, 5- to 7-membered, 5- to 6-membered, 6- to 15-membered, 6- to 9-membered, or 6- to 20-membered) monocyclic, bicyclic, or polycyclic cyclic hydrocarbon radical containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroaryl groups include bicyclic, tricyclic or tetracyclic heteroaryl groups, which contain one aromatic ring having one or more (e.g., 1-4, 1-3, 1-2, or 1) heteroatoms independently selected from O, S and N, and the remaining rings which may be a saturated, partially unsaturated or aromatic ring and can be carbocyclic ring or contain one or more (e.g., 1-4, 1-3, 1-2, or 1) heteroatoms independently selected from O, S and N. Examples of monocyclic heteroaryl groups include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, tetrazolyl, and triazinyl. Examples of bicyclic heteroaryl groups include, but are not limited to, indolyl, isoindolyl, isoindolinyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, oxazolopyridyl, furopyridyl, pteridyl, purinyl, pyridopyridyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, chromanyl, and 6,7-dihydrothieno[3,2-d]pyrimidinyl. Examples of tricyclic or polycyclic heteroaryl groups include, but are not limited to, acridinyl, benzindolyl, carbazolyl, dibenzofuranyl, xanthyl, 5,6,7,8-tetrahydrobenzo[4,5]thieno[2,3-d]pyrimidinyl, and 6,7-dihydro-5H-cyclopenta[4,5]thieno[2,3-d]pyrimidinyl. The heteroaryl group may be unsubstituted or substituted. A substituted heteroaryl refers to heteroaryl substituted one or more times (e.g., 1-4, 1-3, or 1-2 times) by a substituent(s) optionally selected from the group consisting of e.g., deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, and C₂₋₆ alkenyl.

As used herein, the term "heteroarylene", used alone or in combination, refers to a 5- to 30-membered (e.g., 5- to 20-membered, 5- to 15-membered, 5- to 12-membered, 5- to 11-membered, 5-to 10-membered, 5- to 9-membered, 5- to 8-membered, 5- to 7-membered, 5- to 6-membered, 6- to 15-membered, 6- to 9-membered, or 6- to 20-membered) bivalent monocyclic, bicyclic, or polycyclic cyclic hydrocarbon radical containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroarylene groups include bicyclic, tricyclic, tetracyclic or polycyclic heteroarylene groups, which contain one aromatic ring having one or more (e.g., 1-4, 1-3, 1-2, or 1) heteroatoms independently selected from O, S and N, and the remaining ring(s) which may be a saturated, partially unsaturated or aromatic ring and can be carbocyclic ring or contain one or more (e.g., 1-4, 1-3, 1-2, or 1) heteroatoms independently selected from O, S and N. Examples of monocyclic heteroarylene groups include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, tetrazolylene, and triazinylene. Examples of bicyclic heteroarylene include, but are not limited to, indolylene, isoindolylene, isoindolinylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, oxazolopyridylene, furopyridylene, pteridylene, purinylene, pyridopyridylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, imidazo[2,1-b]thiazolylene, chromanylene, and 6,7-dihydrothieno[3,2-d]pyrimidinylene. Examples of tricyclic or polycyclic heteroarylene include, but are not limited to, acridinylene, benzindolylene, carbazolylene, dibenzofuranylene, xanthylene, 5,6,7,8-tetrahydrobenzo[4,5]thieno[2,3-d]pyrimidinylene, and 6,7-dihydro-5H-cyclopenta[4,5]thieno[2,3-d]pyrimidinylene. The heteroarylene may be unsubstituted or substituted. A substituted heteroarylene refers to heteroarylene substituted one or more times (e.g., 1-4, 1-3, or 1-2 times) by a substituent(s) optionally selected from the group consisting of e.g., deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, and C₂₋₆ alkenyl.

As used herein, the term "aryl", used alone or in combination, refers to a monovalent aromatic hydrocarbon group containing from 5 to 30 (e.g., from 5 to 20, from 5 to 15, from 5 to 12, from 5 to 10, from 5 to 9, from 5 to 8, from 5 to 7, from 5 to 6, from 6 to 15, from 6 to 9, or from 6 to 20) carbon atoms and optionally one or more fused rings, such as phenyl group, naphthyl group, or fluorenyl group. As used herein, the "aryl" is optionally substituted. A substituted aryl group refers to an aryl group substituted one or more times (e.g., 1-4, 1-3, or 1-2 times) with a substituent(s). For example, aryl is mono-, di-, tri-, or poly-substituted with a substituent(s) optionally selected from the group consisting of e.g., deuterium, hydroxy, amino, mercapto, nitro, halogen, cyano, oxo, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, and C₂₋₆ alkenyl.

As used herein, the term "arylene", used alone or in combination, refers to a divalent aromatic hydrocarbon group containing from 5 to 30 (e.g., from 5 to 20, from 5 to 15, from 5 to 12, from 5 to 10, from 5 to 9, from 5 to 8, from 5 to 7, from 5 to 6, from 6 to 15, from 6 to 9, or from 6 to 20) carbon atoms and optionally one or more fused rings, such as phenylene (e.g., 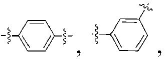 and 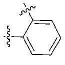 ), naphthylene, or fluorenylene. As used herein, the "arylene" is optionally substituted. A substituted arylene refers to an arylene group substituted one or more times (e.g., 1-4, 1-3, or 1-2 times) with a substituent(s). For example, arylene is mono-, di-, tri-, or poly-substituted with a substituent(s) optionally selected from the group consisting of e.g., deuterium, hydroxy, amino, mercapto, nitro, halogen, cyano, oxo, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, and C₂₋₆ alkenyl.

As used herein, the term "cycloalkyl", used alone or in combination, refers to a saturated or partially unsaturated (i.e., containing one or more double bonds but not fully conjugated) monocyclic or bicyclic or tricyclic or polycyclic cyclic hydrocarbon radical, and the number of carbon atoms thereof includes, but is not limited to, from 3 to 30 carbon atoms (i.e., C₃₋₃₀ cycloalkyl), or from 3 to 25 carbon atoms (i.e., C₃₋₂₅ cycloalkyl), or from 3 to 20 carbon atoms (i.e., C₃₋₂₀ cycloalkyl), or from 3 to 15 carbon atoms (i.e., C₃₋₁₅ cycloalkyl), or from 3 to 12 carbon atoms (i.e., C₃₋₁₂ cycloalkyl), or from 3 to 11 carbon atoms (i.e., C₃₋₁₁ cycloalkyl), or from 3 to 10 carbon atoms (i.e., C₃₋₁₀ cycloalkyl), or from 3 to 8 carbon atoms ( i.e., C₃₋₈ cycloalkyl), or from 3 to 7 carbon atoms (i.e., C₃₋₇ cycloalkyl), or from 3 to 6 carbon atoms (i.e., C₃₋₆ cycloalkyl), or from 4 to 20 carbon atoms ( i.e., C₄₋₂₀ cycloalkyl), or from 4 to 15 carbon atoms ( i.e., C₄₋₁₅ cycloalkyl), or from 4 to 12 carbon atoms (i.e., C₄₋₁₂ cycloalkyl), or from 4 to 10 carbon atoms (i.e., C₄₋₁₀ cycloalkyl). The term "cycloalkyl" includes monocyclic, bicyclic, tricyclic, and polycyclic cyclic hydrocarbon radical having from 3 to 30 carbon atoms. Examples of the term "cycloalkyl" include, but are not limited to, monocyclic cycloalkyl, bridged cycloalkyl (e.g., C₅₋₃₀ bridged cycloalkyl, C₅₋₂₀ bridged cycloalkyl, C₅₋₁₅ bridged cycloalkyl, and C₇₋₁₅ bridged cycloalkyl), fused cycloalkyl (e.g., C₅₋₃₀ fused cycloalkyl, C₅₋₂₀ fused cycloalkyl, C₅₋₁₅ fused cycloalkyl, C₆₋₃₀ fused cycloalkyl, C₆₋₂₀ fused cycloalkyl, C₆₋₁₅ fused cycloalkyl, C₇₋₃₀ fused cycloalkyl, C₇₋₂₀ fused cycloalkyl, C₇₋₁₅ fused cycloalkyl, and C₈₋₁₅ fused cycloalkyl), and spiro-cycloalkyl (e.g., C₅₋₃₀ spiro-cycloalkyl, C₅₋₂₀ spiro-cycloalkyl, C₅₋₁₅ spiro-cycloalkyl, C₆₋₃₀ spiro-cycloalkyl, C₆₋₂₀ spiro-cycloalkyl, C₆₋₁₅ spiro-cycloalkyl, C₇₋₃₀ spiro-cycloalkyl, C₇₋₂₀ spiro-cycloalkyl, C₇₋₁₅ spiro-cycloalkyl, and C₈₋₁₅ spiro-cycloalkyl). Representative examples of monocyclic cycloalkyl groups include, but are not limited to, cyclopropyl, cyclobutyl, cyclopentyl, cyclopentenyl, cyclohexyl, cyclohexenyl, cycloheptyl, and cyclooctyl. Examples of fused cycloalkyl, spiro-cycloalkyl, and bridged cycloalkyl groups include, but are not limited to, decalinyl, octahydropentalenyl, octahydro-1H-indenyl, C₅₋₂₀ spiro-cycloalkyl, C₅₋₁₅ spiro-cycloalkyl, adamantanyl, noradamantanyl, bornyl, and norbornyl (also named as bicyclo[2.2.1]heptyl by the IUPAC system). As used herein, the "cycloalkyl" is optionally mono- or poly-substituted, such as, but not limited to, 2,2-, 2,3-, 2,4-, 2,5-, or 2,6-disubstituted cyclohexyl. The substituents of the substituted "cycloalkyl" are optionally one or more (e.g., 1-5, 1-4, 1-3, 1-2, or 1) substituents selected from the group consisting of e.g., deuterium, hydroxy, amino, mercapto, nitro, halogen, cyano, oxo, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, and C₂₋₆ alkenyl. Examples of the terms "C₃₋₆ cycloalkyl" include, but are not limited to, cyclopropyl, cyclobutyl, cyclopentyl, cyclopentenyl, and cyclohexyl.

As used herein, the term "C_{x-y} spiro-cycloalkyl" (x and y each being an integer), used alone or in combination, refers to a spiro-cycloalkyl group containing from x to y carbon atoms. As used herein, the term "C₅₋₃₀ spiro-cycloalkyl", used alone or in combination, refers to a spiro-cycloalkyl group containing from 5 to 30 carbon atoms (e.g., but not limited to, 5-20, 5-15, 7-20, 7-15, 5-11, 5-10, and 7-9 carbon atoms). The term "C₅₋₃₀ spirocycloalkyl" includes "C₅₋₂₀ spirocycloalkyl", "C₅₋₁₅ spirocycloalkyl", "C₇₋₁₅ spirocycloalkyl" and "C₇₋₂₀ spirocycloalkyl", and representative examples of which include, but are not limited to, spiro[3.3]heptyl, spiro[2.5]octyl, spiro[3.5]nonyl, spiro[4.4]nonyl, spiro[4.5]decyl, and spiro[5.5]undecyl. The "C₅₋₃₀ spiro-cycloalkyl" is optionally further substituted with one or more (e.g., 1-10, 1-6, 1-5, 1-4, or 1-3) substituents selected from the group consisting of e.g., deuterium, hydroxy, amino, mercapto, nitro, halogen, cyano, oxo, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, and C₂₋₆ alkenyl.

As used herein, the term "C_{x-y} bridged cycloalkyl" (where x and y are integers) used alone or in combination, refers to a bridged cycloalkyl group containing x to y carbon atoms. In the present invention, the term "C₅₋₃₀ bridged cycloalkyl" used alone or in combination, refers to a bridged cycloalkyl group containing from 5 to 30 carbon atoms (e.g., but not limited to, 5-20, 6-20, 7-20, 5-15, 7-15, 5-11, 5-10, and 7-9 carbon atoms). The term "C₅₋₃₀ bridged cycloalkyl" includes "C₅₋₂₀ bridged cycloalkyl", "C₆₋₂₀ bridged cycloalkyl", "C₇₋₂₀ bridged cycloalkyl", "C₅₋₁₅ bridged cycloalkyl", and "C₇₋₁₅ bridged cycloalkyl", and representative examples of which include, but are not limited to, adamantanyl, noradamantanyl, bornyl, norbornanyl (systematically named bicyclo[2.2.1]heptanyl), 2-oxobicyclo[2.2.1]heptanyl, bicyclo[2.2.1]heptenyl, and cubanyl. Said " C₅₋₃₀ bridged cycloalkyl" is optionally substituted with 1-10 (e.g., 1-6, 1-5, 1-4, or 1-3) substituents selected from the group consisting of e.g., deuterium, hydroxy, amino, mercapto, nitro, halogen, cyano, oxo, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, and C₂₋₆ alkenyl.

As used herein, the term "cycloalkylene", used alone or in combination, refers to a saturated or partially unsaturated (i.e., containing one or more double bonds but not fully conjugated) monocyclic, bicyclic, tricyclic or polycyclic divalent cyclic hydrocarbon radical, and the number of carbon atoms thereof includes, but is not limited to, from 3 to 30 carbon atoms (i.e., C₃₋₃₀ cycloalkylene), or from 3 to 25 carbon atoms (i.e., C₃₋₂₅ cycloalkylene), from 3 to 20 carbon atoms (i.e., C₃₋₂₀ cycloalkylene), or from 3 to 15 carbon atoms (i.e., C₃₋₁₅ cycloalkylene), or from 3 to 12 carbon atoms (i.e., C₃₋₁₂ cycloalkylene), or from 3 to 11 carbon atoms (i.e., C₃₋₁₁ cycloalkylene), or from 3 to 10 carbon atoms (i.e., C₃₋₁₀ cycloalkylene), or from 3 to 8 carbon atoms ( i.e., C₃₋₈ cycloalkylene), or from 3 to 7 carbon atoms (i.e., C₃₋₇ cycloalkylene), or from 3 to 6 carbon atoms (i.e., C₃₋₆ cycloalkylene), or from 4 to 20 carbon atoms (i.e., C₄₋₂₀ cycloalkylene), or from 4 to 15 carbon atoms ( i.e., C₄₋₁₅ cycloalkylene), or from 4 to 12 carbon atoms (i.e., C₄₋₁₂ cycloalkylene), or from 4 to 10 carbon atoms (i.e., C₄₋₁₀ cycloalkylene). The term "cycloalkylene" includes monocyclic, bicyclic, tricyclic and polycyclic divalent cyclic hydrocarbon radical having from 3 to 30 carbon atoms. Examples of the term "cycloalkylene" include, but are not limited to, monocyclic cycloalkylene, bridged cycloalkylene (e.g., C₅₋₃₀ bridged cycloalkylene, C₅₋₂₀ bridged cycloalkylene, C₅₋₁₅ bridged cycloalkylene, and C₇₋₁₅ bridged cycloalkylene), fused cycloalkylene (e.g., C₅₋₃₀ fused cycloalkylene, C₅₋₂₀ fused cycloalkylene, C₅₋₁₅ fused cycloalkylene, C₆₋₃₀ fused cycloalkylene, C₆₋₂₀ fused cycloalkylene, C₆₋₁₅ fused cycloalkylene, C₇₋₃₀ fused cycloalkylene, C₇₋₂₀ fused cycloalkylene, C₇₋₁₅ fused cycloalkylene, and C₈₋₁₅ fused cycloalkylene), and spiro-cycloalkylene (e.g., C₅₋₃₀ spiro-cycloalkylene, C₅₋₂₀ spiro-cycloalkylene, C₅₋₁₅ spiro-cycloalkylene, C₆₋₃₀ spiro-cycloalkylene, C₆₋₂₀ spiro-cycloalkylene, C₆₋₁₅ spiro-cycloalkylene, C₇₋₃₀ spiro-cycloalkylene, C₇₋₂₀ spiro-cycloalkylene, C₇₋₁₅ spiro-cycloalkylene, and C₈₋₁₅ spiro-cycloalkylene). Representative examples of monocyclic cycloalkylene groups include, but are not limited to, cyclopropylene, cyclobutylene, cyclopentylene, cyclopentenylene, cyclohexylene, cyclohexenylene, cycloheptylene, and cyclooctylene. Examples of fused cycloalkylene, spiro-cycloalkylene, and bridged cycloalkylene groups include, but are not limited to, decalinylene, octahydropentalenylene, octahydro-1H-indenylene, 2,3-dihydro-1H-indenylene, C₅₋₂₀ spiro-cycloalkylene (e.g., C₅₋₁₅ spiro-cycloalkylene), adamantanylene, noradamantanylene, and norbornylene also named as bicyclo[2.2.1]heptylene by the IUPAC system). As used herein, the "cycloalkylene" is optionally mono- or poly-substituted, such as, but not limited to, 2,2-, 2,3-, 2,4-, 2,5-, or 2,6-disubstituted cyclohexylene. The substituents of the substituted "cycloalkylene" are optionally one or more (e.g., 1-5, 1-4, 1-3, 1-2, or 1) substituents selected from the group consisting of deuterium, hydroxy, amino, mercapto, nitro, halogen, cyano, oxo, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, and C₂₋₆ alkenyl.

As used herein, the term "C_{x-y} spiro-cycloalkylene" (x and y each being an integer), used alone or in combination, refers to a spiro-cycloalkylene group containing from x to y carbon atoms. As used herein, the term "C₅₋₃₀ spiro-cycloalkylene", used alone or in combination, refers to a spiro-cycloalkylene group containing from 5 to 30 carbon atoms (e.g., 5-20, 5-15, 7-20, 7-15, 5-11, 5-10, or 7-9 carbon atoms). The term "C₅₋₃₀ spiro-cycloalkylene" includes "C₅₋₂₀ spiro-cycloalkylene", "C₅₋₁₅ spiro-cycloalkylene", "C₇₋₁₅ spiro-cycloalkylene", and "C₇₋₂₀ spiro-cycloalkylene", and representative examples of which include, but are not limited to, spiro[3.3]heptylene, spiro[2.5]octylene, spiro[3.5]nonylene, spiro[4.4]nonylene, spiro[4.5]decylene, and spiro[5.5]undecylene. The "C₅₋₃₀ spiro-cycloalkylene" is optionally further substituted with one or more (e.g., 1-10, 1-6, 1-5, 1-4, or 1-3) substituents selected from the group consisting of deuterium, hydroxy, amino, mercapto, nitro, halogen, cyano, oxo, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, and C₂₋₆ alkenyl.

As used herein, the term "C_{x-y} bridged cycloalkylene" (where x and y are integers) used alone or in combination, refers to a bridged cycloalkylene group containing x to y carbon atoms. In the present invention, the term "C₅₋₃₀ bridged cycloalkylene" used alone or in combination, refers to a bridged cycloalkylene group containing 5 to 30 (e.g., but not limited to, 5-20, 6-20, 7-20, 5-15, 7-15, 5-11, 5-10, and 7-9) carbon atoms. The term "C₅₋₃₀ bridged cycloalkylene" includes "C₅₋₂₀ bridged cycloalkylene", "C₆₋₂₀ bridged cycloalkylene", "C₇₋₂₀ bridged cycloalkylene", "C₅₋₁₅ bridged cycloalkylene", and "C₇₋₁₅ bridged cycloalkylene", and representative examples of which include, but are not limited to, adamantanylene, noradamantanylene, bornylene, bicyclo[2.2.1]heptanylene, 2-oxobicyclo[2.2.1]heptanylene, bicyclo[2.2.1]heptenylene, and cubanylene. Said "C₅₋₃₀ bridged cycloalkylene" is optionally substituted with one or more (e.g., 1-10, 1-6, 1-5, 1-4, or 1-3) substituents selected from the group consisting of deuterium, hydroxy, amino, mercapto, nitro, halogen, cyano, oxo, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, and C₂₋₆ alkenyl.

As used herein, the term "heterocyclyl" or "heterocyclic group", used alone or in combination, refers to a 4- to 30-membered (e.g., 4- to 30-membered, 4- to 25-membered, 4- to 20-membered, 4- to 15-membered, 4- to 14-membered, 4- to 13-membered, 4- to 12-membered, 4- to 11-membered, 4- to 10-membered, 4- to 9-membered, 4- to 8-membered, 4- to 7-membered, 4- to 6-membered, 4- to 5-membered, 5- to 9-membered, 5- to 30-membered, 5- to 20-membered, or 5- to 15-membered) saturated or partially unsaturated (i.e., containing one or more double bonds but not fully conjugated) monocyclic, bicyclic, tricyclic or polycyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. Examples of the heterocyclyl group include, but not limited to, monocyclic heterocyclyl (e.g., 4- to 30-membered monocyclic heterocyclyl, and 4- to 20-membered monocyclic heterocyclyl), bridged heterocyclyl (e.g., 5- to 30-membered bridged heterocyclyl, 5- to 20-membered bridged heterocyclyl, 7- to 20-membered bridged heterocyclyl, and 7- to 15-membered bridged heterocyclyl), fused heterocyclyl (e.g., 5- to 30-membered fused heterocyclyl, and 5- to 20-membered fused heterocyclyl), and spiro-heterocyclyl groups (e.g., 5- to 30-membered spiro-heterocyclyl, and 5- to 20-membered spiro-heterocyclyl). Representative examples of the monocyclic heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl (e.g., 1,4-diazacycloheptan-1-yl), and diazacyclooctyl. Examples of bridged heterocyclyl, fused heterocyclyl and spiro-heterocyclyl groups include, but are not limited to, 6-azabicyclo[3.1.1]heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo[3.2.1]octan-8-yl, 3,8-diazabicyclo[3.2.1]octan-3-yl, 2,5-diazabicyclo[2.2.2]octan-2-yl, octahydro-1H-indolyl, and azaspirocycloalkyl (e.g., 5- to 20-membered azaspirocycloalkyl, such as 3-azaspiro[5.5]undecan-3-yl). The heterocyclyl may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, oxo, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, and C₂₋₆ alkenyl.

As used herein, the term "nitrogen-containing heterocyclyl", used alone or in combination, refers to 4- to 30-membered (e.g., 4- to 30-membered, 4- to 25-membered, 4- to 20-membered, 4- to 15-membered, 4- to 14-membered, 4- to 13-membered, 4- to 12-membered, 4- to 11-membered, 4- to 10-membered, 4- to 9-membered, 4- to 8-membered, 4- to 7-membered, 4- to 6-membered, 4- to 5-membered, 5- to 9-membered, 5- to 30-membered, 5- to 20-membered, or 5- to 15-membered) saturated or partially unsaturated (i.e., containing one or more double bonds but not fully conjugated) monocyclic, bicyclic, tricyclic or polycyclic cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Examples of the nitrogen-containing heterocyclyl group include, but not limited to, nitrogen-containing monocyclic heterocyclyl (e.g., 4- to 30-membered nitrogen-containing monocyclic heterocyclyl, and 4- to 20-membered nitrogen-containing monocyclic heterocyclyl), nitrogen-containing bridged heterocyclyl (e.g., 5- to 30-membered nitrogen-containing bridged heterocyclyl, 5- to 20-membered nitrogen-containing bridged heterocyclyl, 5- to 15-membered nitrogen-containing bridged heterocyclyl, 7- to 20-membered nitrogen-containing bridged heterocyclyl, and 7- to 15-membered nitrogen-containing bridged heterocyclyl), nitrogen-containing fused heterocyclyl (e.g., 5- to 30-membered nitrogen-containing fused heterocyclyl, and 5- to 20-membered nitrogen-containing fused heterocyclyl), and nitrogen-containing spiro-heterocyclyl groups (e.g., 5- to 30-membered nitrogen-containing spiro-heterocyclyl, and 5- to 20-membered nitrogen-containing spiro-heterocyclyl). Representative examples of the nitrogen-containing monocyclic heterocyclyl include, but are not limited to, azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, azacyclooctyl, diazacycloheptanyl (e.g., 1,4-diazacycloheptan-1-yl), and diazacyclooctyl. Examples of nitrogen-containing bridged heterocyclyl, nitrogen-containing fused heterocyclyl and nitrogen-containing spiro-heterocyclyl groups include, but are not limited to, 6-azabicyclo[3.1.1]heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo[3.2.1]octan-8-yl, 3,8-diazabicyclo[3.2.1]octan-3-yl, 2,5-diazabicyclo[2.2.2]octan-2-yl, octahydro-1H-indolyl, and azaspirocycloalkyl (e.g., 5- to 20-membered azaspirocycloalkyl, such as 3-azaspiro[5.5]undecan-3-yl). The nitrogen-containing heterocyclyl may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, oxo, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, and C₂₋₆ alkenyl.

As used herein, the term "heterocyclylene", used alone or in combination, refers to a 4- to 30-membered (e.g., 4- to 30-membered, 4- to 25-membered, 4- to 20-membered, 4- to 15-membered, 4- to 14-membered, 4- to 13-membered, 4- to 12-membered, 4- to 11-membered, 4- to 10-membered, 4- to 9-membered, 4- to 8-membered, 4- to 7-membered, 4- to 6-membered, 4- to 5-membered, 5- to 9-membered, 5- to 30-membered, 5- to 20-membered, or 5- to 15-membered) saturated or partially unsaturated (i.e., containing one or more double bonds but not fully conjugated) monocyclic, bicyclic, tricyclic or polycyclic bivalent cyclic hydrocarbon group containing one or more (e.g., from 1 to 5, or from 1 to 4, from 1 to 3, from 1 to 2, or 1) heteroatoms independently selected from sulfur, oxygen, and nitrogen. Examples of the heterocyclylene groups include, but are not limited to, monocyclic heterocyclylene (e.g., 4- to 30-membered monocyclic heterocyclylene, and 4- to 20-membered monocyclic heterocyclylene), bridged heterocyclylene (e.g., 5- to 30-membered bridged heterocyclylene, 5- to 20-membered bridged heterocyclylene, 7- to 20-membered bridged heterocyclylene, and 7- to 15-membered bridged heterocyclylene), fused heterocyclylene (e.g., 5- to 30-membered fused heterocyclylene, and 5- to 20-membered fused heterocyclylene), and spiro-heterocyclylene groups (e.g., 5- to 30-membered spiro-heterocyclylene, and 5- to 20-membered spiro-heterocyclylene). Representative examples of the monocyclic heterocyclylene include, but are not limited to, azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, diazacycloheptanylene (e.g., 1,4-diazacycloheptanylene, 4,5-diazacycloheptanylene, 1,3-diazacycloheptanylene), and diazacyclooctylene. Examples of the bridged heterocyclylene, fused heterocyclylene and spiro-heterocyclylene groups include, but are not limited to, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, octahydro-1H-indolylene, and azaspirocycloalkylene (e.g., 5- to 20-membered azaspirocycloalkylene, such as 3-azaspiro[5.5]undecanylene). The heterocyclylene may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, oxo, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, and C₂₋₆ alkenyl.

As used herein, the term "nitrogen-containing heterocyclylene", used alone or in combination, refers to a 4- to 30-membered (e.g., 4- to 30-membered, 4- to 25-membered, 4- to 20-membered, 4- to 15-membered, 4- to 14-membered, 4- to 12-membered, 4- to 11-membered, 4- to 10-membered, 4- to 9-membered, 4- to 8-membered, 4- to 7-membered, 4- to 6-membered, 4- to 5-membered, 5- to 9-membered, 5- to 30-membered, 5- to 20-membered, or 5- to 15-membered) saturated or partially unsaturated (i.e., containing one or more double bonds but not fully conjugated) monocyclic, bicyclic, tricyclic or polycyclic bivalent cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Examples of the nitrogen-containing heterocyclylene group include, but not limited to, nitrogen-containing monocyclic heterocyclylene (e.g., 4- to 30-membered nitrogen-containing monocyclic heterocyclylene, and 4- to 20-membered nitrogen-containing monocyclic heterocyclylene), nitrogen-containing bridged heterocyclylene (e.g., 5- to 30-membered nitrogen-containing bridged heterocyclylene, 5- to 20-membered nitrogen-containing bridged heterocyclylene, 7- to 20-membered nitrogen-containing bridged heterocyclylene, and 7- to 15-membered nitrogen-containing bridged heterocyclylene), nitrogen-containing fused heterocyclylene (e.g., 5- to 30-membered nitrogen-containing fused heterocyclylene, and 5- to 20-membered nitrogen-containing fused heterocyclylene), and nitrogen-containing spiro-heterocyclylene (e.g., 5- to 30-membered nitrogen-containing spiro-heterocyclylene, and 5- to 20-membered nitrogen-containing spiro-heterocyclylene). Representative examples of the nitrogen-containing monocyclic heterocyclylene include, but are not limited to, azetidinylene, pyrrolidinylene, imidazolidinylene, pyrazolidylene, oxazolidinylene, thiazolidinylene, piperidinylene, piperazinylene, morpholinylene, thiomorpholinylene, azacycloheptanylene, azacyclooctylene, diazacycloheptanylene (e.g., 1,4-diazacycloheptanylene, 4,5-diazacycloheptanylene, 1,3-diazacycloheptanylene), and diazacyclooctylene. Examples of nitrogen-containing bridged heterocyclylene, nitrogen-containing fused heterocyclylene, and nitrogen-containing spiro-heterocyclylene groups include, but are not limited to, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, octahydro-1H-indolylene, and azaspirocycloalkylene (e.g., 5- to 20-membered azaspirocycloalkylene, such as 3-azaspiro[5.5]undecanylene). The nitrogen-containing heterocyclylene may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, oxo, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, and C₂₋₆ alkenyl.

As used herein, the term "alkynyl", used alone or in combination, refers to a linear or branched monovalent hydrocarbon group containing from 2 to 8 (e.g., from 2 to 6, from 2 to 5, from 2 to 4, or preferably 2) carbon atoms and having one or more (e.g., from 1 to 3, from 1 to 2, or 1) carbon-carbon triple bonds. Examples of alkynyl include C₂₋₈ alkynyl, C₂₋₆ alkynyl, or C₂₋₄ alkynyl, with representative but non-limiting examples including ethynyl, 1-propynyl, 1-butynyl, and 1,3-diynyl.

As used herein, the term "alkenyl", used alone or in combination, refers to a linear or branched monovalent hydrocarbon group containing from 2 to 8 (e.g., from 2 to 6, from 2 to 5, from 2 to 4, from 2 to 3, or 2) carbon atoms and having one or more (e.g., from 1 to 3, from 1 to 2, or 1) carbon-carbon double bonds. Examples of "alkenyl" include C₂₋₈ alkenyl, C₂₋₆ alkenyl or C₂₋₄ alkenyl, with representative but non-limiting examples including, vinyl (e.g., CH₂=CH-), 1-propenyl, allyl, 1-butenyl, 2-butenyl, 3-butenyl, isobutenyl, pentenyl, n-pent-2,4-dienyl, 1-methyl-but-1-enyl, 2-methyl-but-1-enyl, 3-methyl-but-1-enyl, 1-methyl-but-2-enyl, 2-methyl-but-2-enyl, 3-methyl-but-2-enyl, 1-methyl-but-3-enyl, 2-methyl-but-3-enyl, 3-methyl-but-3-enyl, and hexenyl.

As used herein, "bornylane" or "bornane" (also known as 1,7,7-trimethylbicyclo[2.2.1]heptane; camphane) has a definition known to those skilled in the art. As used herein, "camphanyl" or "bornyl" refers to a monovalent group of bornane, i.e., the group remaining after any one of the hydrogens in bornane is removed. Representative examples of "bornyl" include, but are not limited to, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl,

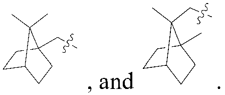

As used herein, "bicyclo[2.2.1]heptane" also known as "norbornane", has a definition known to those skilled in the art. As used herein, "bicyclo[2.2.1]heptyl" or "norbornyl" refers to a monovalent group of bicyclo[2.2.1]heptane, i.e., the group remaining after any hydrogen in bicyclo[2.2.1]heptane is removed. Representative examples of "bicyclo[2.2.1]heptyl" include, but are not limited to, bicyclo[2.2.1]heptan-2-yl, bicyclo[2.2.1]heptan-3-yl, bicyclo[2.2.1]heptan-4-yl, bicyclo[2.2.1]heptan-5-yl, or bicyclo[2.2.1]heptan-6-yl.

As used herein, the term "bicyclo[2.2.1]heptene" has a definition known to those skilled in the art. As used herein, "bicyclo[2.2.1]heptenyl" refers to a monovalent group of bicyclo[2.2.1]heptene, i.e., the group remaining after any hydrogen in bicyclo[2.2.1]heptene is removed. Representative examples of "bicyclo[2.2.1]heptenyl" include, but are not limited to, bicyclo[2.2.1]hept-5-en-2-yl, bicyclo[2.2.1]hept-5-en- 3-yl, or bicyclo[2.2.1]hept-5-en-7-yl.

As used herein, "adamantane" (also known as tricyclo[3.3.1.1^{3,7}]decane) has a definition known to those skilled in the art, and its structural formula is e.g., as follows: 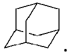 As used herein, "adamantanyl" refers to a monovalent group of adamantane, that is, the group remaining after any hydrogen in adamantane is removed. Representative examples of "adamantanyl" include, but are not limited to, 1-adamantanyl, 2-adamantanyl, 3-adamantanyl, 4-adamantanyl, 5-adamantanyl, 6-adamantanyl, 7-adamantanyl, 8-adamantanyl, 9-adamantanyl, or 10-adamantanyl.

As used herein, "noradamantane" has a definition known to those skilled in the art, and its structural formula is e.g., as follows: 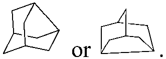 As used herein, "noradamantanyl" refers to a monovalent group of noradamantane, that is, the group remaining after any hydrogen in noradamantane is removed. Representative examples of "noradamantanyl" include, but are not limited to, 1-noradamantanyl, 2-noradamantanyl, 3-noradamantanyl, 4-noradamantanyl, 5-noradamantanyl, 6-noradamantanyl, 7-noradamantanyl, 8-noradamantanyl or 9-noradamantanyl.

As used herein, "adamantanamine" has the definitions known to those skilled in the art, namely referring to an adamantane having an amino substituent, wherein the amino substituent can replace a hydrogen on a carbon at any position in the adamantane. An example of "adamantanamine" can be adamantan-1-amine (corresponding English chemical name is adamantan-1-amine or Tricyclo[3.3.1.1^{3,7}]decan-1-amine; CAS No.: 768-94-5), with the following structural Formula:

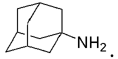

Salts or pharmaceutically acceptable salts, enantiomers, stereoisomers, solvates, polymorphs of the compounds of Formula (I) of the present disclosure are also encompassed within the scope of the present invention.

In all embodiments of the present disclosure, the salts or pharmaceutically acceptable salts of the compounds of Formula (I) refer to non-toxic inorganic or organic acid and/or base addition salts. Examples include: sulfates, hydrohalides (including hydrochlorides and hydrobromates), maleate, sulfonate, citrate, lactate, lactobionate, L-tartrate, fumarate, L-malate, L-lactate, α-ketoglutarate, hippurate, D-glucuronate, D-gluconate, α-D-glucoheptonate, glycolate, mucate, L-ascorbate, orotate, picrate, glycinate, alaninate, arginate, cinnamate, laurate, pamoate, sebacate, besylate, methanesulfonate, ethanesulfonate, edisylate, formate, acetate, 2,2-dichloroacetate, trimethylacetate, propionate, valerate, palmitate, triphenylacetate, 2-ethyl-butane-1,4-dioate, iodate, nicotinate, L-pyroglutamate, L-prolinate, ferulate, 2-hydroxyethanesulfonate, nitrate, gentisate, cholate, salicylate, terephthalate, glutarate, adipate, stearate, oleate, undecylenate, camphorate, camsylate, dodecylsulfate, phosphate, thiocyanate, dihydrogen phosphate, pyrophosphate, metaphosphate, oxalate, carbonate, malonate, benzoate, mandelate, succinate, pyruvate, 4-chlorobenzenesulfonate, 1,5-naphthalenedisulfonate, 3-hydroxy-2-naphthoate, 1-hydroxy-2-naphthoate, 2-naphthalenesulfonate, glycolate, trifluoroacetate, terephthalate, or 4-methylbenzenesulfonate, etc.

"Pharmaceutically acceptable carrier" refers to a pharmaceutically acceptable material, such as a filler, stabilizer, dispersant, suspending agent, diluent, excipient, thickener, solvent, or encapsulating material, with which the useful compounds according to the present disclosure are carried or transported into or administered to a patient so that they can perform their intended function. Generally, such constructs are carried or transported from one organ or part of the body to another organ or part of the body. The carrier is compatible with the other ingredients of the formulation, including the compounds useful in the present disclosure, and is not harmful to the patient, and the carrier must be "acceptable". Some examples of materials that can be used as pharmaceutically acceptable carriers include sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; and other common non-toxic compatible substances used in pharmaceutical formulations.

As used herein, the term "room temperature" refers to the ambient temperature, such as 20-30°C.

As used herein, "stereoisomer" refers to a compound with the same chemical structural formula, but a different arrangement of atoms or groups in space. Stereoisomers include enantiomers, diastereomers, conformational isomers (rotational isomers), geometric isomers (cis/trans isomers), atropisomers, and so on.

As used herein, the term "solvate" refers to an association or complex formed by the interaction between one or more solvent molecules and the compounds of the present invention. Examples of solvents include water, isopropanol, ethanol, methanol, DMSO, ethyl acetate, acetic acid and ethanolamine. The term "hydrate" means that a complex formed with water.

As used herein, the term "chiral" refers to a molecule that is nonsuperimposable on its mirror image; whereas "achiral" refers to a molecule that can be superimposed on its mirror image.

As used herein, the term "enantiomers" refers to two isomers of a compound that are nonsuperimposable mirror images.

As used herein, the term "diastereomers" refers to stereoisomers that have two or more chiral centers but are not mirror images of each other. The diastereomers have different physical properties, such as melting point, boiling point, spectral properties and reactivity. The diastereomeric mixture can be separated by high-resolution analytical operations such as electrophoresis and chromatography, such as HPLC.

As used herein, "p-menthane" has the definitions known to those skilled in the art, and its structural formula is e.g., as follows: 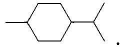 As used herein, "p-menthanyl" refers to a monovalent group of p-menthane, that is, the group remaining after any hydrogen in p-menthane is removed. Representative examples of "p-menthanyl" include, but are not limited to,

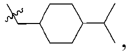

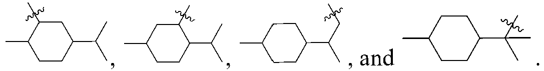

As used herein, "m-menthane" has the definitions known to those skilled in the art, and its structural formula is e.g., as follows: 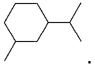 As used herein, "m-menthanyl" refers to a monovalent group of m-menthane, that is, the group remaining after any hydrogen in m-menthane is removed. Representative examples of "m-menthanyl" include, but are not limited to,

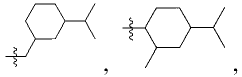

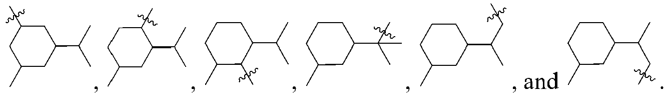

As used herein, "Quinuclidine" (also known as 1-azabicyclo[2.2.2]octane) has the definitions known to those skilled in the art, and its structural formula is e.g., as follows: 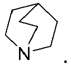 As used herein, "quinuclidinyl" refers to a monovalent group of Quinuclidine, that is, the group remaining after any hydrogen in Quinuclidine is removed. Representative examples of "quinuclidinyl" include, but are not limited to,

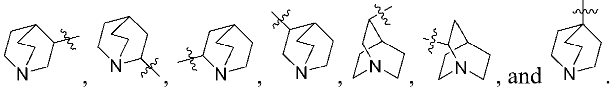

### Description of the Drawings

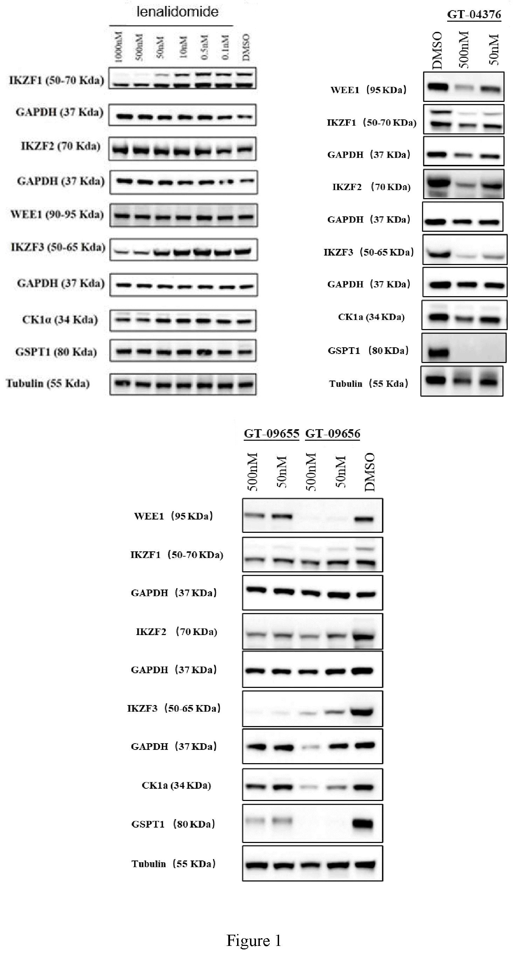

FIG. 1 shows the Western Blot results of target substrate protein degradation induced by the compounds of the present invention in hPBMCs.

### Examples

In the following description, numerous specific details are set forth in order to provide a thorough understanding of the present disclosure. The present disclosure may be practiced without some or all of these specific details. In other cases, well-known process operations have not been described in detail in order not to unnecessarily obscure the present disclosure. Although the present disclosure will be described in conjunction with specific embodiments, it should be understood that this is not intended to limit the present disclosure to these embodiments.

The following abbreviations are used throughout the specification and examples:
- AcOH: acetic acid
- Boc: t-Butyloxy carbonyl
- DCM: dichloromethane
- DIEA or DIPEA: N, N-diisopropylethylamine
- DMF: N,N-dimethylformamide
- DMSO: dimethyl sulfoxide
- EA: ethyl acetate
- ESI: electrospray ionization
- equiv: equivalent
- EtOH: ethanol
- EtOAc: ethyl acetate
- HPLC: high performance liquid chromatography
- HRMS: high resolution mass spectrometry
- LC-MS: liquid chromatography-mass spectrometry
- LRMS: low resolution mass spectrometry
- LC: liquid chromatography
- Me: methyl
- MeCN: acetonitrile
- MeOH: methanol
- MS: mass spectrometry
- MsCl: methanesulfonyl chloride
- MsO-: methanesulfonyloxy
- Ms₂O: methanesulfonic anhydride
- ¹H NMR: Proton nuclear magnetic resonance
- MeO-: methoxy
- ONs: o-nitrobenzenesulfonyl
- rt: room temperature
- tBu: tert-butyl
- TEA: triethylamine
- TFA: trifluoroacetic acid
- T_{f}O-: trifluoromethanesulfonate
- TLC: thin layer chromatography
- TMS: tetramethylsilane
- TsO-: tosyloxy
- Xantphos: 4,5-bis(diphenylphosphino)-9,9-dimethylxanthene
- X-Phos: 2-dicyclohexylphosphino-2',4',6'-triisopropylbiphenyl

In the present disclosure, the ¹H NMR spectrum was recorded on a Bruker-500MHz nuclear magnetic resonance instrument, by using, as a solvent, CD₃OD (δ = 3.31 ppm) containing 0.1% TMS (as an internal standard); or using, as a solvent, CDCl₃ (δ = 7.26 ppm) containing 0.1% TMS (as an internal standard); or using, as a solvent, DMSO-*d*₆ (δ = 2.50 ppm) containing 0.03% TMS (as an internal standard). LC-MS spectra were recorded on a Sciex API 2000 mass spectrometer equipped with an Agilent 1100 binary pump, DAD and ELSD detectors, or on an Agilent 1260-6125B single quadrupole LC-MS system equipped with an Agilent 1260 quaternary pump, DAD and ELSD detectors. HPLC preparative purification was performed using a SHIMADZU LC-20AP system. HPLC purity was determined using either a SHIMADZU LC-30AP or Waters 1525 system. All reactions were carried out under ambient atmosphere unless otherwise specified. Reaction progress was monitored by TLC or LC-MS.

Solvents and reagents are processed as follows: DCM, DMF, anhydrous EtOH and anhydrous MeOH used in the reactions were purchased from Sinopharm Group; and preparative HPLC was performed using HPLC-grade CH₃CN and deionized water. Unless otherwise specified, other reaction substrates, reagents and chemicals are either commercially available or may be synthesized by methods known in the art.

Unless otherwise specified, the materials and reagents used in the following examples are commercially available and used directly, or can be synthesized by using methods known in the art.

### General synthesis methods

Compounds and/or pharmaceutically acceptable salts thereof of the present disclosure can be synthesized using commercially available raw materials by synthetic techniques known in the art. The synthetic schemes described below illustrate the preparation of most compounds. The starting materials or reagents used in each scheme can be purchased from commercial sources or prepared by methods known to those skilled in the art. One skilled in the art can prepare the salts, racemates, enantiomers, phosphates, sulfates, hydrochlorides and prodrug forms of the compounds of Formula (I) of the present disclosure according to routine techniques in the art.

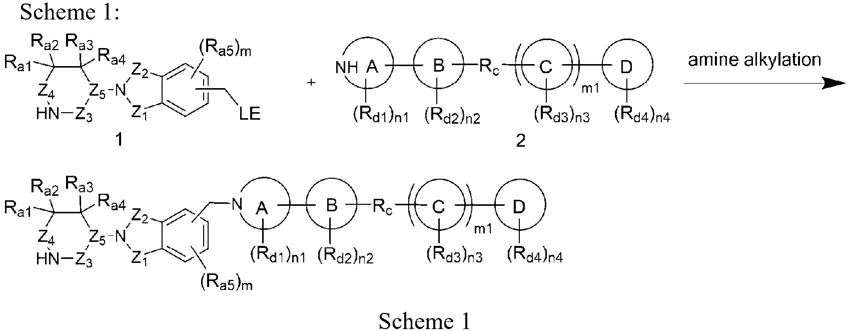

In Scheme 1, Rₐ₁, Rₐ₂, Rₐ₃, Rₐ₄, (Rₐ₅)ₘ, Z₁, Z₂, Z₃, Z₄, Z₅, nitrogen-containing heterocycle A, (R_{d1})ₙ₁, ring B, (R_{d2})ₙ₂, R_{c}, ring C, m1, (R_{d3})ₙ₃, ring D, and (R_{d4})ₙ₄ are as defined in the compound of Formula (I) and its various sub-embodiments in the present disclosure. The group LE of Substrate 1 represents Cl, Br, I, OMs, Ots, or ONs. Substrate 2 is a compound corresponding to the moiety X containing a nitrogen-containing heterocycle in the compound of Formula (I).

The amine alkylation reaction in Scheme 1 may be carried out, for example, in the presence of DIEA and sodium iodide, or triethylamine and sodium iodide, at a temperature ranging from room temperature to 80 °C (e.g., 40 °C to 60 °C, 40 °C to 50 °C, or 50 °C to 60 °C). The molar ratio of Substrate 1 to Substrate 2 may be, for example, 1:1.1 to 2, 1:1.1 to 1.5, 1:1.1 to 1.2, or 1:1.2 to 1.3, and the like.

For example, the specific procedure for Scheme 1 may be as follows:

To a solution of bromo-substituted substrate 1 (1.3 eq.) and substrate 2 (1.0 eq.) in anhydrous DMF (2 mL) are added triethylamine (3.0 eq.) and sodium iodide (1.0 eq.). The reaction mixture is stirred at 50 °C for 2 hours. After monitoring the reaction via LCMS and confirming its completion, the reaction mixture is filtered, and the filtrate is separated and purified by high-performance liquid chromatography to give the target compound.

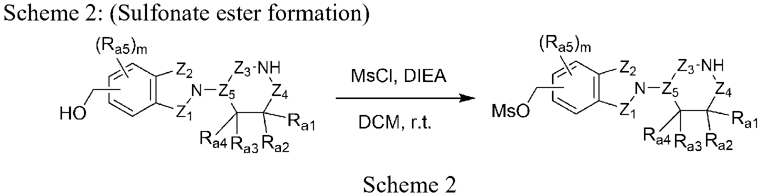

In Scheme 2, Rₐ₁, Rₐ₂, Rₐ₃, Rₐ₄, (Rₐ₅)ₘ, Z₁, Z₂, Z₃, Z₄, and Z₅ are as defined in the compound of Formula (I) and its various sub-embodiments in the present disclosure.

In Scheme 2, the substituent -CH₂-OH of the reaction substrate may be at the 4-, 5-, 6- or 7-position on the benzene ring of the isoindolinyl group, and the substituent -CH₂-OMs of the resulting products 2-2 and 2-3 is correspondingly at the 4-, 5-, 6- or 7-position on the benzene ring of the isoindolinyl group.

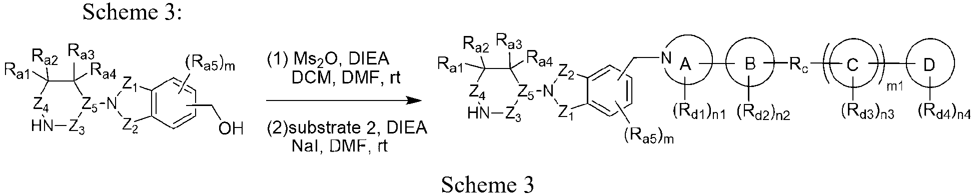

In Scheme 3, Rₐ₁, Rₐ₂, Rₐ₃, Rₐ₄, (Rₐ₅)ₘ, Z₁, Z₂, Z₃, Z₄, Z₅, nitrogen-containing heterocycle A, (R_{d1})ₙ₁, ring B, (Ra₂)ₙ₂, R_{c}, ring C, m1, (R_{d3})ₙ₃, ring D, and (R_{d4})ₙ₄ are as defined in the compound of Formula (I) and its various sub-embodiments in the present disclosure. The substrate 2 in step (2) is the same compound as substrate 2 in Scheme 1.

For example, the specific procedure for Scheme 3 may be as follows:

To a solution of hydroxyl substrate (1.0 eq.) in anhydrous DMF (0.5 mL) and dichloromethane (5 mL) are added diisopropylethylamine (3.0 eq.) and methanesulfonic anhydride (0.9 eq.). The reaction mixture is stirred at room temperature for 5 minutes. After monitoring the reaction via TLC and confirming its completion, a solution of the corresponding amine substrate (1.0 eq.) in DMF (0.5 mL) is added to the reaction mixture, and the mixture is then stirred at room temperature for 3 hours. After monitoring the reaction via LCMS and confirming its completion, the reaction mixture is filtered and concentrated, and the residue is separated and purified by high-performance liquid chromatography to give the target compound.

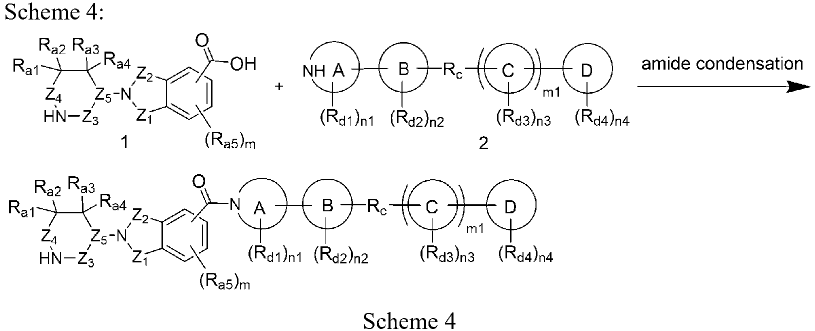

In Scheme 4, Rₐ₁, Rₐ₂, Rₐ₃, Rₐ₄, (Rₐ₅)ₘ, Z₁, Z₂, Z₃, Z₄, Z₅, nitrogen-containing heterocycle A, (R_{d1})ₙ₁, ring B, (Ra₂)ₙ₂, R_{c}, ring C, m1, (R_{d3})ₙ₃, ring D, and (R_{d4})ₙ₄ are as defined in the compound of Formula (I) and its various sub-embodiments in the present disclosure.

The specific procedure for Scheme 4 may be as follows:

To a solution of carboxylic acid substrate 1 (1.1 eq.) and substrate 2 (1.0 eq.) in anhydrous DMF (2 mL) are added triethylamine (3.0 eq.) and HATU (1.3 eq.). The reaction mixture is stirred at room temperature for 2 hours. After monitoring the reaction via LCMS and confirming its completion, the reaction mixture is filtered, and the filtrate is separated and purified by high-performance liquid chromatography to give the target compound.

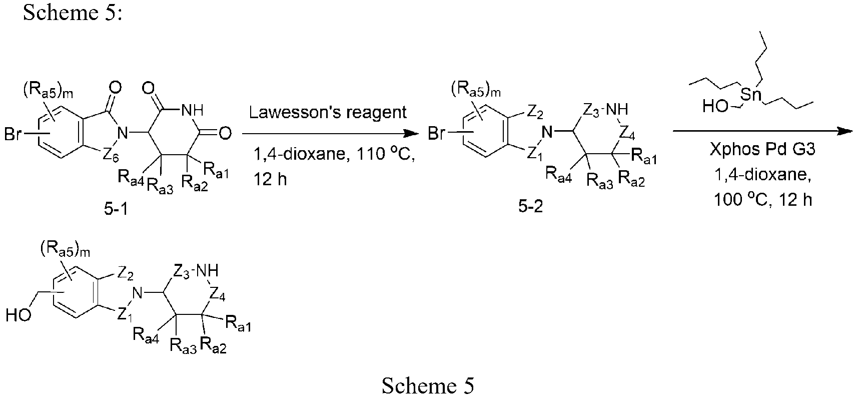

In Scheme 5, Z₆ represents C(O), CH₂, or CD₂. Z₁ represents C(O), C(S), CH₂, or CD₂, and Z₂, Z₃, and Z₄ each independently represents C(O) or C(S), wherein at least one of Z₁, Z₂, Z₃, and Z₄ represents C(S). When Z₆ represents C(O), Z₁ represents C(O) or C(S). When Z₆ represents CH₂ or CD₂, Z₁ correspondingly represents CH₂ or CD₂.

The thionation reagent Lawesson's reagent in Scheme 5 may also be replaced with other suitable thionation reagents, such as, but not limited to, carbon disulfide, hexamethyldisilthiane, sulfur, thiourea, hydrogen sulfide, phosphorus pentasulfide, Belleau's reagent, and Davy's reagent, and the like.

In Scheme 5, the substituent Br of the reaction substrate 5-1 in step 1 may be at the 4-, 5-, 6-, or 7-position on the benzene ring of the isoindolinyl group, and the substituent Br of the resulting intermediate product and the substituent -CH₂-OMs of the product of step 2 are correspondingly at the 4-, 5-, 6-, or 7-position on the benzene ring of the isoindolinyl group. Rₐ₁, Rₐ₂, Rₐ₃, Rₐ₄, and (Rₐ₅)ₘ are as defined in the compound of Formula (I) and its various sub-embodiments in the present disclosure.

In some embodiments of scheme 5, at least one of Z₁, Z₂, Z₃, and Z₄ in the compound of Formula (I) represents C(S). In some embodiments, at least two of Z₁, Z₂, Z₃, and Z₄ in the compound of Formula (I) represent C(S). In some embodiments, at least three of Z₁, Z₂, Z₃, and Z₄ represent C(S). In some embodiments, all of Z₁, Z₂, Z₃, and Z₄ represent C(S).

Depending upon the target compounds, the above schemes and their reaction substrates, reaction conditions (including reaction dosage, temperature, duration, etc.), work up, etc. can be appropriately modified and adjusted by techniques and methods well known to those skilled in the art to obtain the desired target compounds. The obtained target compounds can be further modified by the substituents and the like to obtain subsequent target compounds through methods well known to those skilled in the art.

### Examples

### Intermediate Example 1: Preparation of 3-(5-(hydroxymethyl)-1-thioxoisoindolin-2-yl)piperidine-2,6-dione (GTC00170)

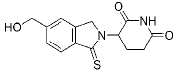

The target compound (GTC00170) was prepared by referring to the method of Scheme 5.

Step 1: To a solution of 3-(5-bromo-1-oxoisoindolin-2-yl)piperidine-2,6-dione (20 g, 61.891 mmol) in 1,4-dioxane (200 mL) was added Lawesson's reagent (11.26 g, 27.851 mmol). The reaction mixture was stirred at 110°C for 12 hours. After monitoring the reaction via TLC and confirming its completion, the reaction mixture was concentrated, and the residue was purified by column chromatography to afford 3-(5-bromo-1-thioxoisoindolin-2-yl)piperidine-2,6-dione (10 g, 29.480 mmol, yield 47.64%) as an off-white solid.

Step 2: To a solution of 3-(5-bromo-1-thioxoisoindolin-2-yl)piperidine-2,6-dione (6 g, 17.688 mmol) and (tributyl-λ⁴-stannyl)methanol (8.52 g, 26.532 mmol) in 1,4-dioxane (60 mL) was added XPhos Pd G3 (0.1 g, 0.147 mmol). The reaction mixture was stirred at 100°C for 3 hours under an argon atmosphere. The reaction mixture was concentrated, and the residue was purified by column chromatography to afford 3-(5-(hydroxymethyl)-1-thioxoisoindolin-2-yl)piperidine-2,6-dione (1 g, yield 19.46%) as a gray solid. ¹H NMR (400 MHz, DMSO) δ 11.17 (s, 1H), 7.91 (d, J = 7.9 Hz, 1H), 7.66 (s, 1H), 7.55 (d, J = 8.0 Hz, 1H), 6.09 - 5.91 (m, 1H), 5.49 (t, J = 5.7 Hz, 1H), 4.91 - 4.67 (m, 4H), 3.13 - 2.93 (m, 1H), 2.79 - 2.58 (m, 2H), 2.16 (dd, J = 8.9, 3.6 Hz, 1H). LCMS (ESI) calcd. for C₁₄H₁₅N₂O₃S⁺ [M+H]+: 291.08, found, 291.0.

### Intermediate Example 2: Preparation of 3-(4-(hydroxymethyl)-1-thioxoisoindolin-2-yl)piperidine-2,6-dione (GTC00504)

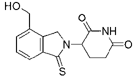

With reference to the method of Scheme 5 or Intermediate Example 1, the target compound (GTC00504) was obtained as a gray solid (0.8 g, yield 33.32%). ¹HNMR (400 MHz, DMSO) δ 11.12 (s, 1H), 7.80 (d, J = 7.4 Hz, 1H), 7.61 (d, J = 7.4 Hz, 1H), 7.53 (t, J = 7.6 Hz, 1H), 5.98 (d, J = 8.3 Hz, 1H), 5.37 (d, J = 5.4 Hz, 1H), 4.79 (dd, J = 49.7, 20.2 Hz, 2H), 4.65 (d, J = 4.1 Hz, 2H), 2.96 (s, 1H), 2.64 (d, J = 18.4 Hz, 2H), 2.09 (dd, J = 8.9, 3.5 Hz, 1H). LCMS (ESI) calcd. for C₁₄H₁₅N₂O₃S⁺ [M+H]+: 291.08, found, 291.0.

### Intermediate Example 3: Preparation of 3-(6-(hydroxymethyl)-1-thioxoisoindolin-2-yl)piperidine-2,6-dione (GTC00505)

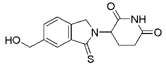

With reference to the method of Scheme 5 or Intermediate Example 1, the target compound (GTC00505) was obtained as a gray solid (1 g, yield 38.91%). ¹HNMR (400 MHz, DMSO) δ 11.06 (d, J = 31.4 Hz, 1H), 7.86 (d, J = 19.3 Hz, 1H), 7.60 (d, J = 7.8 Hz, 2H), 5.95 (d, J = 8.9 Hz, 1H), 5.41 (t, J = 5.7 Hz, 1H), 4.82 - 4.60 (m, 4H), 3.00 - 2.90 (m, 1H), 2.64 (d, J = 17.9 Hz, 1H), 2.54 (s, 1H), 2.16 - 2.05 (m, 1H). LCMS (ESI) calcd. for C₁₄H₁₅N₂O₃S⁺ [M+H]+: 291.08, found, 291.0.

### Intermediate Example 4: Preparation of (2-(2,6-dioxopiperidin-3-yl)-1-thioxoisoindolin-5-yl)methyl methanesulfonate (GTC00505)

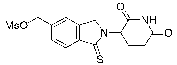

With reference to the method of Scheme 5 or Intermediate Example 1, the target compound (GTC00505) was obtained as a gray solid (1 g, yield 38.91%). ¹HNMR (400 MHz, DMSO) δ 11.06 (d, J = 31.4 Hz, 1H), 7.86 (d, J = 19.3 Hz, 1H), 7.60 (d, J = 7.8 Hz, 2H), 5.95 (d, J = 8.9 Hz, 1H), 5.41 (t, J = 5.7 Hz, 1H), 4.82 - 4.60 (m, 4H), 3.00 - 2.90 (m, 1H), 2.64 (d, J = 17.9 Hz, 1H), 2.54 (s, 1H), 2.16 - 2.05 (m, 1H). LCMS (ESI) calcd. for C₁₄H₁₅N₂O₃S⁺ [M+H]+: 291.08, found, 291.0.

### Example 1: Preparation of 3-(5-((4-(4-benzhydrylpiperazin-1-yl)piperidin-1-yl)methyl)-1-oxoisoindolin-2-yl)piperidine-2,6-dione (GT-04329)

With reference to the method of Scheme 1, the target compound (GT-04329) was obtained as a white solid (28 mg, yield 37%). ¹H NMR (400 MHz, MeOD) δ 7.82 (d, J = 7.8 Hz, 1H), 7.74 (s, 1H), 7.62 (d, J = 7.7 Hz, 1H), 7.61 - 7.50 (m, 4H), 7.32 - 7.29 (m, 4H), 7.25 - 7.22 (m, 2H), 5.08 (dd, J = 13.3, 5.2 Hz, 1H), 5.04 - 4.88 (m, 1H), 4.48 - 4.40 (m, 4H), 3.60 - 3.56 (m, 2H), 3.51 - 3.42 (m, 5H), 3.18 - 3.01 (m, 5H), 2.88 - 2.67 (m, 2H), 2.48 - 2.23 (m, 4H), 2.17 - 1.97 (m, 3H). LCMS (ESI) calcd. for C₃₆H₄₂N₅O₃⁺ [M+H]⁺: 592.23, found, 592.3.

### Example 2: Preparation of 3-(5-((4-(4-benzhydrylpiperazin-1-yl)piperidin-1-yl)methyl)-4-fluoro-1-oxoisoindolin-2-yl)piperidine-2,6-dione (GT-04330)

With reference to the method of Scheme 1, the target compound (GT-04330) was obtained as a white solid (34 mg, yield 43%). ¹H NMR (400 MHz, MeOD) δ 7.76 - 7.70 (m, 1H), 7.66 (d, J = 7.8 Hz, 1H), 7.58 - 7.55 (m, 4H), 7.33 - 7.29 (m, 4H), 7.25 - 7.22 (m, 2H), 5.08 (dd, J = 13.3, 5.2 Hz, 1H), 5.04 - 4.89 (m, 1H), 4.60 - 4.48 (m, 4H), 3.70 - 3.61 (m, 2H), 3.59 - 3.40 (m, 5H), 3.20 - 2.98 (m, 6H), 2.91 - 2.75 (m, 1H), 2.73 - 2.66 (m, 1H), 2.51 - 2.39 (m, 1H), 2.38 - 2.31 (m, 2H), 2.17 - 1.95 (m, 3H). LCMS (ESI) calcd. for C₃₆H₄₁FN₅O₃⁺ [M+H]⁺: 610.32, found, 610.3.

### Example 3: Preparation of 3-(5-((4-(4-benzhydrylpiperazin-1-yl)piperidin-1-yl)methyl)-6-fluoro-1-oxoisoindolin-2-yl)piperidine-2,6-dione (GT-04331)

With reference to the method of Scheme 1, the target compound (GT-04331) was obtained as a white solid (34 mg, yield 43%). ¹H NMR (400 MHz, MeOD) δ 7.82 (d, J = 6.0 Hz, 1H), 7.65 - 7.50 (m, 5H), 7.35 - 7.29 (m, 4H), 7.25 - 7.22 (m, 2H), 5.08 (dd, J = 13.3, 5.2 Hz, 1H), 5.04 - 4.89 (m, 1H), 4.55 - 4.36 (m, 4H), 3.68 - 3.62 (m, 2H), 3.57 - 3.38 (m, 5H), 3.20 - 2.94 (m, 6H), 2.88 - 2.75 (m, 1H), 2.72 - 2.66 (m, 1H), 2.49 - 2.26 (m, 3H), 2.12 - 2.03 (m, 3H). LCMS (ESI) calcd. for C₃₆H₄₁FN₅O₃⁺ [M+H]⁺: 610.32, found, 610.3.

### Example 4: Preparation of 3-(5-((4-(4-benzhydrylpiperazin-1-yl)piperidin-1-yl)methyl)-7-fluoro-1-oxoisoindolin-2-yl)piperidine-2,6-dione (GT-04597)

With reference to the method of Scheme 1, the target compound (GT-04597) was obtained as a white solid (39 mg, yield 45%). ¹H NMR (400 MHz, MeOD) δ 7.54 (s, 1H), 7.52 - 7.40 (m, 4H), 7.37 (d, J = 9.6 Hz, 1H), 7.27 (t, J = 6.9 Hz, 4H), 7.23 - 7.16 (m, 2H), 5.05 (dd, J = 13.3, 5.1 Hz, 1H), 4.71 (s, 2H), 4.48 (q, J = 17.9 Hz, 2H), 4.39 (s, 2H), 3.60 - 3.54 (m, 2H), 3.41 - 3.23 (m, 5H), 3.14 - 3.06 (m, 3H), 3.01 - 2.92 (m, 1H), 2.89 - 2.76 (m, 2H), 2.69 (d, J = 15.5 Hz, 1H), 2.48 - 2.36 (m, 1H), 2.36 - 2.22 (m, 2H), 2.16 - 1.88 (m, 3H). LCMS (ESI) calcd. for C₃₆H₄₁FN₅O₃⁺ [M+H]⁺: 610.32, found, 610.3.

### Example 5: Preparation of 3-(4-((4-(4-benzhydrylpiperazin-1-yl)piperidin-1-yl)methyl)-1-oxoisoindolin-2-yl)piperidine-2,6-dione (GT-04598)

With reference to the method of Scheme 1, the target compound (GT-04598) was obtained as a white solid (46 mg, yield 54%). ¹H NMR (400 MHz, MeOD) δ 7.97 (d, J = 7.6 Hz, 1H), 7.90 (d, J = 7.6 Hz, 1H), 7.72 (t, J = 7.6 Hz, 1H), 7.63 - 7.52 (m, 4H), 7.41 - 7.37 (m, 4H), 7.34 - 7.21 (m, 2H), 5.24 (dd, J = 13.4, 5.1 Hz, 1H), 4.80 (d, J = 17.4 Hz, 1H), 4.67 (d, J = 17.4 Hz, 1H), 4.58 - 4.42 (m, 2H), 3.82 - 3.65 (m, 2H), 3.56 - 3.49 (m, 1H), 3.46 - 3.37 (m, 4H), 3.32 - 3.27 (m, 5H), 3.26 - 3.20 (m, 1H), 3.03 - 2.90 (m, 2H), 2.83 (d, J = 15.8 Hz, 1H), 2.61 - 2.53 (m, 1H), 2.46 - 2.38 (m, 2H), 2.31 - 2.20 (m, 1H), 2.18 - 2.09 (m, 2H). LCMS (ESI) calcd. for C₃₆H₄₂N₅O₃⁺ [M+H]⁺: 592.33, found, 592.3.

### Example 6: Preparation of 3-(6-((4-(4-benzhydrylpiperazin-1-yl)piperidin-1-yl)methyl)-1-oxoisoindolin-2-yl)piperidine-2,6-dione (GT-04599)

With reference to the method of Scheme 1, the target compound (GT-04599) was obtained as a white solid (8 mg, yield 9%). ¹H NMR (400 MHz, MeOD) δ 7.98 (s, 1H), 7.87 (d, J = 7.9 Hz, 1H), 7.77 (d, J = 7.9 Hz, 1H), 7.63 (d, J = 6.3 Hz, 4H), 7.40 (t, J = 7.2 Hz, 4H), 7.34 - 7.32 (m, 2H), 5.19 (dd, J = 13.3, 5.1 Hz, 1H), 4.64 - 4.52 (m, 4H), 3.70 - 3.67 (m, 2H), 3.61 - 3.40 (m, 6H), 3.24 - 3.05 (m, 5H), 3.03 - 2.86 (m, 2H), 2.87 - 2.75 (m, 1H), 2.59 - 2.51 (m, 1H), 2.48 - 2.41 (m, 2H), 2.28 - 2.16 (m, 1H), 2.14 - 2.03 (m, 2H). LCMS (ESI) calcd. for C₃₆H₄₂N₅O₃⁺ [M+H]⁺: 592.33, found, 592.3.

### Example 7: Preparation of 3-(5-((4-(4-benzhydrylpiperazin-1-yl)-3,3-difluoropiperidin-1-yl)methyl)-1-oxoisoindolin-2-yl)piperidine-2,6-dione (GT-04376)

With reference to the method of Scheme 1, the target compound (GT-04376) was obtained as a white solid (31 mg, yield 45%). ¹H NMR (400 MHz, MeOD) δ 7.82 (d, J = 7.8 Hz, 1H), 7.69 (s, 1H), 7.60 - 7.58 (m, 5H), 7.40 - 7.37 (m, 4H), 7.34 - 7.30 (m, 2H), 5.36 (s, 1H), 5.08 (dd, J = 13.3, 5.2 Hz, 1H), 4.56 - 4.36 (m, 4H), 3.68 - 3.61 (m, 1H), 3.53 - 3.50 (m, 1H), 3.43 - 3.31 (m, 2H), 3.14 - 3.09 (m, 9H), 2.88 - 2.78 (m, 1H), 2.73 - 2.65 (m, 1H), 2.44 - 2.31 (m, 1H), 2.17 - 2.01 (m, 3H). LCMS (ESI) calcd. for C₃₆H₄₀F₂N₅O₃⁺ [M+H]⁺: 628.31, found, 628.3.

### Example 8: Preparation of 3-(5-((4-(4-benzhydrylpiperazin-1-yl)-3,3-difluoropiperidin-1-yl)methyl)-4-fluoro-1-oxoisoindolin-2-yl)piperidine-2,6-dione (GT-04377)

With reference to the method of Scheme 1, the target compound (GT-04377) was obtained as a white solid (41 mg, yield 58%). ¹H NMR (400 MHz, MeOD) δ 7.68 - 7.62 (m, 2H), 7.59 - 7.55 (m, 4H), 7.40 - 7.36 (m, 4H), 7.34 - 7.30 (m, 2H), 5.35 (s, 1H), 5.08 (dd, J = 13.4, 5.1 Hz, 1H), 4.62 - 4.44 (m, 3H), 4.36 (brs, 2H), 3.70 - 3.62 (m, 1H), 3.53 - 3.40 (m, 1H), 3.35 - 3.28 (m, 1H), 3.16 - 3.01 (m, 9H), 2.87 - 2.78 (m, 1H), 2.74 - 2.64 (m, 1H), 2.48 - 2.38 (m, 1H), 2.16 - 1.99 (m, 3H). LCMS (ESI) calcd. for C₃₆H₃₉F₃N₅O₃⁺ [M+H]⁺: 646.30, found, 646.3.

### Example 9: Preparation of 3-(5-((4-(4-benzhydrylpiperazin-1-yl)-3,3-difluoropiperidin-1-yl)methyl)-6-fluoro-1-oxoisoindolin-2-yl)piperidine-2,6-dione (GT-04378)

With reference to the method of Scheme 1, the target compound (GT-04378) was obtained as a white solid (40 mg, yield 57%). ¹H NMR (400 MHz, MeOD) δ 7.74 (d, J = 5.9 Hz, 1H), 7.60 - 7.58 (m, 4H), 7.54 (d, J = 8.6 Hz, 1H), 7.40 - 7.36 (m, 4H), 7.35 - 7.29 (m, 2H), 5.35 (s, 1H), 5.12 - 5.03 (m, 1H), 4.53 - 4.39 (m, 2H), 4.36 (brs, 2H), 3.68 - 3.59 (m, 1H), 3.53 - 3.39 (m, 1H), 3.36 - 3.23 (m, 2H), 3.20 - 2.93 (m, 9H), 2.88 - 2.76 (m, 1H), 2.74 - 2.64 (m, 1H), 2.43 - 2.36 (m, 1H), 2.15 - 1.97 (m, 3H). LCMS (ESI) calcd. for C₃₆H₃₉F₃N₅O₃⁺ [M+H]⁺: 646.30, found, 646.3.

### Example 10: Preparation of 3-(5-((4-(4-benzhydrylpiperazin-1-yl)-3,3-difluoropiperidin-1-yl)methyl)-7-fluoro-1-oxoisoindolin-2-yl)piperidine-2,6-dione (GT-04379)

With reference to the method of Scheme 1, the target compound (GT-04379) was obtained as a white solid (35 mg, yield 50%). ¹H NMR (400 MHz, MeOD) δ 7.57 (d, J = 7.4 Hz, 4H), 7.42 - 7.36 (m, 5H), 7.34 - 7.32 (m, 2H), 7.29 - 7.23 (m, 1H), 5.35 (s, 1H), 5.03 (dd, J = 13.2, 6.6 Hz, 1H), 4.53 - 4.34 (m, 2H), 4.16 (brs, 2H), 3.48 - 3.39 (m, 1H), 3.33 - 3.22 (m, 2H), 3.20 - 2.95 (m, 9H), 2.82 - 2.77 (m, 2H), 2.74 - 2.63 (m, 1H), 2.44 - 2.33 (m, 1H), 2.10 - 1.94 (m, 3H). LCMS (ESI) calcd. for C₃₆H₃₉F₃N₅O₃⁺ [M+H]⁺: 646.30, found, 646.3.

### Example 11: Preparation of 3-(4-((4-(4-benzhydrylpiperazin-1-yl)-3,3-difluoropiperidin-1-yl)methyl)-1-oxoisoindolin-2-yl)piperidine-2,6-dione (GT-04380)

With reference to the method of Scheme 1, the target compound (GT-04380) was obtained as a white solid (19 mg, yield 28%). ¹H NMR (400 MHz, MeOD) δ 7.79 - 7.77 (m, 1H), 7.65 (t, J = 7.0 Hz, 1H), 7.60 - 7.55 (m, 4H), 7.54 - 7.50 (m, 1H), 7.40 - 7.36 (m, 4H), 7.35 - 7.28 (m, 2H), 5.35 (brs, 1H), 5.18 - 5.04 (m, 1H), 4.68 - 4.47 (m, 2H), 4.31 - 4.03 (m, 2H), 3.52 - 3.43 (m, 1H), 3.41 - 3.25 (m, 2H), 3.20 - 2.94 (m, 9H), 2.94 - 2.75 (m, 2H), 2.72 - 2.68 (m, 1H), 2.51 - 2.29 (m, 1H), 2.19 - 2.06 (m, 1H), 2.06 - 1.90 (m, 2H). LCMS (ESI) calcd. for C₃₆H₄₀F₂N₅O₃⁺ [M+H]⁺: 628.31, found, 628.3.

### Example 12: Preparation of 3-(5-((3,3-difluoro-4-(4-(phenyl(pyridin-2-yl)methyl)piperazin-1-yl)piperidin-1-yl)methyl)-1-oxoisoindolin-2-yl)piperidine-2,6-dione (GT-5037)

With reference to the method of Scheme 1, the target compound (GT-5037) was obtained as a white solid (49 mg, yield 52%). ¹H NMR (400 MHz, DMSO-d6) δ 11.00 (s, 1H), 8.73 (d, J = 4.8 Hz, 1H), 7.93 - 7.89 (m, 1H), 7.87 - 7.78 (m, 2H), 7.74 - 7.70 (m, 4H), 7.50 - 7.36 (m, 4H), 5.89 (s, 1H), 5.14 (dd, J = 13.2, 5.1 Hz, 1H), 4.52 - 4.36 (m, 4H), 3.63 - 3.52 (m, 4H), 3.47 - 3.23 (m, 4H), 3.21 - 3.12 (m, 4H), 2.98 - 2.82 (m, 2H), 2.61 (d, J = 16.8 Hz, 1H), 2.43 (dd, J = 13.2, 4.5 Hz, 1H), 2.26 - 2.09 (m, 1H), 2.03 - 1.99 (m, 2H). LCMS (ESI) calcd. for C₃₅H₃₉F₂N₆O₃⁺ [M+H]⁺: 629.30, found, 629.3.

### Example 13: Preparation of 3-(5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-oxoisoindolin-2-yl)piperidine-2,6-dione (GT-04595)

With reference to the method of Scheme 1, the target compound (GT-04595) was obtained as a white solid (16 mg, yield 35%). ¹H NMR (400 MHz, MeOD) δ 7.91 (d, J = 8.0 Hz, 1H), 7.71 (s, 1H), 7.64 (d, J = 7.8 Hz, 1H), 7.36 (d, J = 8.2 Hz, 2H), 7.28 (d, J = 8.2 Hz, 2H), 5.20 (dd, J = 13.4, 5.0 Hz, 1H), 4.64 - 4.50 (m, 2H), 4.43 - 4.25 (m, 2H), 3.65 - 3.56 (m, 2H), 3.31 - 3.09 (m, 6H), 3.01 - 2.86 (m, 3H), 2.84 - 2.78 (m, 2H), 2.72 - 2.66 (m, 1H), 2.59 - 2.44 (m, 4H), 2.28 - 2.11 (m, 2H), 2.09 - 1.93 (m, 3H), 1.62 - 1.48 (m, 2H), 1.08 (s, 3H), 1.05 (s, 3H). LCMS (ESI) calcd. for C₃₈H₄₅ClF₂N₅O₄⁺ [M+H]⁺: 708.31, found, 708.3.

### Example 14: Preparation of 3-(5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-4-fluoro-1-oxoisoindolin-2-yl)piperidine-2,6-dione (GT-04596)

With reference to the method of Scheme 1, the target compound (GT-04596) was obtained as a white solid (17 mg, yield 37%). ¹H NMR (400 MHz, MeOD) δ 7.77 - 7.68 (m, 2H), 7.37 (d, J = 8.4 Hz, 2H), 7.29 (d, J = 8.5 Hz, 2H), 5.20 (dd, J = 13.3, 5.0 Hz, 1H), 4.63 (q, J = 17.3 Hz, 2H), 4.33 - 4.21 (m, 2H), 3.66 - 3.56 (m, 2H), 3.47 - 3.39 (m, 3H), 3.29 - 3.18 (m, 2H), 3.07 - 2.88 (m, 3H), 2.85 - 2.78(m, 3H), 2.70 - 2.42 (m, 4H), 2.29 - 2.14 (m, 2H), 2.04 - 1.95 (m, 4H), 1.57 - 1.52 (m, 2H), 1.08 (s, 3H), 1.05 (s, 3H). LCMS (ESI) calcd. for C₃₈H₄₄ClF₃N₅O₄⁺ [M+H]⁺: 726.30, found, 726.3.

### Example 15: Preparation of 3-(5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-oxoisoindolin-2-yl)piperidine-2,6-dione (GT-05160)

With reference to the method of Scheme 1, the target compound (GT-05160) was obtained as a white solid (50 mg, yield 48%). ¹H NMR (400 MHz, DMSO-d6) δ 11.01 (s, 1H), 7.77 (d, J = 7.8 Hz, 1H), 7.69 - 7.64 (m, 1H), 7.60 - 7.54 (m, 1H), 7.44 (d, J = 8.3 Hz, 2H), 7.14 (d, J = 8.3 Hz, 2H), 5.13 (dd, J = 13.2, 5.1 Hz, 1H), 4.48 (d, J = 16.8 Hz, 1H), 4.35 (d, J = 16.8 Hz, 1H), 4.15 - 3.87 (m, 1H), 3.55 (brs, 3H), 3.28 - 3.20 (m, 3H), 3.05 - 2.97 (m, 4H), 2.91 - 2.82 (m, 4H), 2.66 - 2.59 (m, 4H), 2.47 - 2.36 (m, 1H), 2.34 - 2.21 (m, 3H), 2.02 (brs, 3H), 1.96 - 1.84 (m, 1H), 1.83 - 1.73 (m, 1H), 1.44(m, 2H), 0.96 (s, 6H). LCMS (ESI) calcd. for C₃₈H₄₇ClF₂N₅O₃⁺ [M+H]⁺: 694.33, found, 694.4.

### Example 16: Preparation of 3-(5-((4-(4-(2,3-dichlorophenyl)piperazin-1-yl)piperidin-1-yl)methyl)-1-oxoisoindolin-2-yl)piperidine-2,6-dione (GT-04604)

With reference to the method of Scheme 1, the target compound (GT-04604) was obtained as a white solid (29 mg, yield 33%). ¹H NMR (400 MHz, MeOD) δ 7.94 (d, J = 7.8 Hz, 1H), 7.84 (s, 1H), 7.73 (d, J = 7.6 Hz, 1H), 7.34 - 7.30 (m, 2H), 7.17 - 7.15 (m, 1H), 5.19 (dd, J = 13.7, 4.9 Hz, 1H), 4.64 - 4.46 (m, 4H), 3.79 - 3.58 (m, 7H), 3.50 - 3.43 (m, 2H), 3.20 - 3.13 (m, 4H), 3.01 - 2.86 (m, 1H), 2.84 - 2.78 (m, 1H), 2.60 - 2.45 (m, 3H), 2.25 - 2.12 (m, 3H). LCMS (ESI) calcd. for C₂₉H₃₄Cl₂N₅O₃⁺ [M+H]⁺: 570.20, found, 570.2.

### Example 17: Preparation of 3-(5-((4-(4-(2,3-dichlorophenyl)piperazin-1-yl)piperidin-1-yl)methyl)-4-fluoro-1-oxoisoindolin-2-yl)piperidine-2,6-dione (GT-04605)

With reference to the method of Scheme 1, the target compound (GT-04605) was obtained as a white solid (30 mg, yield 34%). ¹H NMR (400 MHz, MeOD) δ 7.82 - 7.77 (m, 2H), 7.35 - 7.27 (m, 2H), 7.18 - 7.16 (m, 1H), 5.19 (dd, J = 13.4, 5.2 Hz, 1H), 4.72 - 4.50 (m, 4H), 3.78 - 3.73 (m, 3H), 3.68 - 3.54 (m, 4H), 3.49 - 3.47 (m, 2H), 3.28 - 3.11 (m, 4H), 3.02 - 2.85 (m, 2H), 2.84 - 2.74 (m, 1H), 2.59 - 2.44 (m, 3H), 2.22 - 2.13 (m, 3H). LCMS (ESI) calcd. for C₂₉H₃₃Cl₂FN₅O₃⁺ [M+H]⁺: 588.19, found, 588.2.

### Example 18: Preparation of 3-(5-((4-(4-(2,3-dichlorophenyl)piperazin-1-yl)piperidin-1-yl)methyl)-6-fluoro-1-oxoisoindolin-2-yl)piperidine-2,6-dione (GT-04606)

With reference to the method of Scheme 1, the target compound (GT-04606) was obtained as a white solid (23 mg, yield 26%). ¹H NMR (400 MHz, MeOD) δ 7.93 (d, J = 6.1 Hz, 1H), 7.71 (d, J = 8.6 Hz, 1H), 7.39 - 7.28 (m, 2H), 7.19 (dd, J = 7.1, 2.5 Hz, 1H), 5.20 (dd, J = 13.3, 5.1 Hz, 1H), 4.65 - 4.46 (m, 4H), 3.83 - 3.72 (m, 3H), 3.68 - 3.59 (m, 4H), 3.52 - 3.41 (m, 2H), 3.30 - 3.12 (m, 4H), 2.97 - 2.88 (m, 1H), 2.85 - 2.78 (m, 1H), 2.57 - 2.50 (m, 3H), 2.30 - 2.13 (m, 3H). LCMS (ESI) calcd. for C₂₉H₃₃Cl₂FN₅O₃⁺ [M+H]⁺: 588.19, found, 588.2.

### Example 19: Preparation of 3-(5-((4-(4-(2,3-dichlorophenyl)piperazin-1-yl)piperidin-1-yl)methyl)-7-fluoro-1-oxoisoindolin-2-yl)piperidine-2,6-dione (GT-04607)

With reference to the method of Scheme 1, the target compound (GT-04607) was obtained as a white solid (25 mg, yield 28%). ¹H NMR (400 MHz, MeOD) δ 7.64 (s, 1H), 7.46 (d, J = 9.6 Hz, 1H), 7.33 - 7.32 (m, 2H), 7.20 - 7.16 (m, 2H), 5.21 - 5.14 (m, 1H), 4.58 (d, J = 8.2 Hz, 2H), 4.50 - 4.48 (m, 3H), 3.74 - 3.62 (m, 6H), 3.22 - 3.18 (m, 4H), 3.04 - 2.71 (m, 4H), 2.52 - 2.49 (m, 4H), 2.23 - 2.16 (m, 4H). LCMS (ESI) calcd. for C₂₉H₃₃Cl₂FN₅O₃⁺ [M+H]⁺: 588.19, found, 588.2.

### Example 20: Preparation of 3-(4-((4-(4-(2,3-dichlorophenyl)piperazin-1-yl)piperidin-1-yl)methyl)-1-oxoisoindolin-2-yl)piperidine-2,6-dione (GT-04609)

With reference to the method of Scheme 1, the target compound (GT-04609) was obtained as a white solid (8 mg, yield 10%). ¹H NMR (400 MHz, MeOD) δ 7.96 (d, J = 7.7 Hz, 1H), 7.92 (d, J = 7.7 Hz, 1H), 7.71 (t, J = 7.7 Hz, 1H), 7.35 - 7.26 (m, 2H), 7.18 - 7.16 (m, 1H), 5.23 (dd, J = 13.2, 5.2 Hz, 1H), 4.68 (d, J = 17.4 Hz, 1H), 4.52 - 4.49 (m, 2H), 3.84 - 3.66 (m, 5H), 3.59 - 3.57 (m, 2H), 3.46 - 3.32 (m, 5H), 3.24 - 3.18 (m, 2H), 3.03 - 2.89 (m, 1H), 2.84 - 2.81 (m, 1H), 2.60 - 2.47 (m, 3H), 2.38 - 2.16 (m, 3H). LCMS (ESI) calcd. for C₂₉H₃₄Cl₂N₅O₃⁺ [M+H]⁺: 570.20, found, 570.2.

### Example 21: Preparation of 3-(6-((4-(4-(2,3-dichlorophenyl)piperazin-1-yl)piperidin-1-yl)methyl)-1-oxoisoindolin-2-yl)piperidine-2,6-dione (GT-04608)

With reference to the method of Scheme 1, the target compound (GT-04608) was obtained as a white solid (5 mg, yield 6%). ¹H NMR (400 MHz, MeOD) δ 7.89 (s, 1H), 7.73 (d, J = 7.3 Hz, 1H), 7.66 (d, J = 8.1 Hz, 1H), 7.24 - 7.20 (m, 2H), 7.08 (dd, J = 6.4, 2.9 Hz, 1H), 5.08 (dd, J = 13.3, 5.2 Hz, 1H), 4.47 (dd, J = 31.5, 17.7 Hz, 2H), 4.38 - 4.32 (m, 2H), 3.61 - 3.50 (m, 2H), 3.48 - 3.25 (m, 8H), 3.06 - 2.91 (m, 3H), 2.83 - 2.78 (m, 1H), 2.74 - 2.65 (m, 1H), 2.46 - 2.39 (m, 1H), 2.38 - 2.33 (m, 2H), 2.11 - 2.07 (m, 1H), 2.04 - 1.90 (m, 2H). LCMS (ESI) calcd. for C₂₉H₃₄Cl₂N₅O₃⁺ [M+H]⁺: 570.20, found, 570.2.

### Example 22: Preparation of 3-(5-((4-(4-(2,3-dichlorophenyl)piperazin-1-yl)-3,3-difluoropiperidin-1-yl)methyl)-1-oxoisoindolin-2-yl)piperidine-2,6-dione (GT-05212)

With reference to the method of Scheme 1, the target compound (GT-05212) was obtained as a white solid (23 mg, 25%). ¹H NMR (400 MHz, DMSO-d6) δ 11.02 (s, 1H), 7.79 (d, J = 7.7 Hz, 1H), 7.75 - 7.69 (m, 2H), 7.66 - 7.58 (m, 1H), 7.39 - 7.33 (m, 2H), 7.18 - 7.16 (m, 1H), 5.14 (dd, J = 13.1, 5.2 Hz, 1H), 4.90 (s, 1H), 4.51 (d, J = 17.2 Hz, 1H), 4.38 (d, J = 17.2 Hz, 1H), 4.27 - 4.06 (m, 2H), 3.35 - 3.08 (m, 10H), 3.00 - 2.84 (m, 2H), 2.82 - 2.65 (m, 1H), 2.61 (d, J = 16.9 Hz, 1H), 2.47 - 2.38 (m, 1H), 2.33 - 2.30 (m, 1H), 2.25 - 2.09 (m, 1H), 2.08 - 1.95 (m, 1H). LCMS (ESI) calcd. for C₂₉H₃₂Cl₂F₂N₅O₃⁺ [M+H]⁺: 606.18, found, 606.2.

### Example 23: Preparation of 3-(5-((4-(4-(6-fluorobenzo[d]isoxazol-3-yl)piperazin-1-yl)piperidin-1-yl)methyl)-1-oxoisoindolin-2-yl)piperidine-2,6-dione (GT-04989)

With reference to the method of Scheme 1, the target compound (GT-04989) was obtained as a white solid (14 mg, 17%). ¹H NMR (400 MHz, DMSO-d6) δ 11.52 (s, 1H), 11.02 (s, 1H), 8.10 (dd, J = 8.9, 5.2 Hz, 1H), 7.87 - 7.81 (m, 2H), 7.74 - 7.71 (m, 1H), 7.61 (dd, J = 9.1, 2.1 Hz, 1H), 7.28 - 7.23 (m, 1H), 5.15 (dd, J = 13.4, 5.1 Hz, 1H), 4.63 - 4.33 (m, 5H), 4.24 - 4.01 (m, 2H), 3.69 - 3.39 (m, 8H), 3.08 - 3.04 (m, 1H), 3.04 - 2.85 (m, 3H), 2.61 (d, J = 15.4 Hz, 1H), 2.47 - 2.41 (m, 1H), 2.39 - 2.32 (m, 2H), 2.28 - 2.09 (m, 2H), 2.03 - 1.98 (m, 1H). LCMS (ESI) calcd. for C₃₀H₃₄FN₆O₄⁺ [M+H]⁺: 561.26, found, 561.3.

### Example 24: Preparation of 3-(5-((3,3-difluoro-4-(4-(6-fluorobenzo[d]isoxazol-3-yl)piperazin-1-yl)piperidin-1-yl)methyl)-1-oxoisoindolin-2-yl)piperidine-2,6-dione (GT-04931)

With reference to the method of Scheme 1, the target compound (GT-04931) was obtained as a white solid (23 mg, 26%). ¹H NMR (400 MHz, DMSO) δ 11.01 (s, 1H), 8.05 (dd, J = 8.9, 5.2 Hz, 1H), 7.79 (d, J = 7.5 Hz, 1H), 7.70 (dd, J = 16.6, 7.1 Hz, 1H), 7.62 - 7.56 (m, 2H), 7.21 (td, J = 9.1, 2.1 Hz, 1H), 5.14 (dd, J = 13.2, 5.1 Hz, 1H), 4.50 (d, J = 17.0 Hz, 1H), 4.37 (d, J = 17.2 Hz, 1H), 4.14 (dd, J = 48.4, 14.3 Hz, 1H), 3.83 - 3.57 (m, 6H), 3.36 - 2.99 (m, 6H), 2.99 - 2.84 (m, 2H), 2.61 (d, J = 16.6 Hz, 1H), 2.42 (dd, J = 13.3, 4.5 Hz, 2H), 2.25 - 1.93 (m, 3H). LCMS (ESI) calcd. for C₃₀H₃₂F₃N₆O₄⁺ [M+H]⁺: 597.24, found, 597.3.

### Example 25: Preparation of 3-(5-((3',3'-difluoro-4-(6-fluorobenzo[d]isoxazol-3-yl)-[1,4'-bipiperidin]-1'-yl)methyl)-1-oxoisoindolin-2-yl)piperidine-2,6-dione (GT-04932)

With reference to the method of Scheme 1, the target compound (GT-04932) was obtained as a white solid (32 mg, 36%). ¹H NMR (400 MHz, DMSO) δ 11.00 (s, 1H), 8.25 (s, 1H), 7.80 - 7.69 (m, 2H), 7.63 (s, 1H), 7.54 (d, J = 7.6 Hz, 1H), 7.34 (td, J = 9.1, 2.1 Hz, 1H), 5.13 (dd, J = 13.2, 5.0 Hz, 1H), 4.49 (d, J = 17.4 Hz, 1H), 4.36 (d, J = 17.5 Hz, 1H), 4.21 - 4.04 (m, 1H), 3.99 - 3.90 (m, 4H), 3.64 - 3.50 (m, 3H), 3.48 - 3.35 (m, 3H), 3.22 - 3.10 (m, 1H), 3.00 - 2.85 (m, 1H), 2.85 - 2.67 (m, 1H), 2.61 (d, J = 16.8 Hz, 2H), 2.47 - 2.31 (m, 3H), 2.26 - 2.14 (m, 3H), 2.07 - 1.92 (m, 1H). LCMS (ESI) calcd. for C₃₁H₃₃F₃N₅O₄⁺ [M+H]⁺: 596.25, found, 596.3.

### Example 26: Preparation of 3-(5-((4-(4-benzhydrylpiperazin-1-yl)-3,3-difluoropiperidin-1-yl)methyl)-1-thioxoisoindolin-2-yl)piperidine-2,6-dione (GT-05105)

With reference to the method of Scheme 1, the target compound (GT-05105) was obtained as a white solid (12 mg, 10%). ¹H NMR (400 MHz, DMSO-d6) δ 11.14 (s, 1H), 7.91 (d, J = 7.7 Hz, 1H), 7.88 - 7.79 (m, 4H), 7.60 (s, 1H), 7.49 (d, J = 8.3 Hz, 1H), 7.47 - 7.43 (m, 4H), 7.41 - 7.34 (m, 2H), 6.05 - 5.86 (m, 2H), 5.62 - 5.59 (m, 1H), 4.80 (d, J = 9.3 Hz, 1H), 4.70 (t, J = 10.3 Hz, 1H), 3.21 - 3.08 (m, 6H), 3.08 - 2.89 (m, 8H), 2.65 (d, J = 17.9 Hz, 2H), 2.12 - 2.06 (m, 2H), 1.97 - 1.74 (m, 2H). LCMS (ESI) calcd. for C₃₆H₄₀F₂N₅O₂S⁺ [M+H]⁺: 644.29, found, 644.3.

### Example 27: Preparation of 3-(5-((3,3-difluoro-4-(4-(phenyl(pyridin-2-yl)methyl)piperazin-1-yl)piperidin-1-yl)methyl)-1-thioxoisoindolin-2-yl)piperidine-2,6-dione (GT-05208)

With reference to the method of Scheme 1, the target compound (GT-05208) was obtained as a white solid (20 mg, 17%). ¹H NMR (400 MHz, DMSO-d6) δ 11.15 (s, 1H), 8.74 (d, J = 4.7 Hz, 1H), 7.95 - 7.91 (m, 2H), 7.84 - 7.81 (m, 1H), 7.72 - 7.67 (m, 3H), 7.63 (d, J = 7.9 Hz, 1H), 7.53 - 7.36 (m, 4H), 6.04 - 5.85 (m, 1H), 4.94 - 4.81 (m, 1H), 4.75 - 4.70 (m, 1H), 4.48 - 4.15 (m, 2H), 3.36 - 3.19 (m, 5H), 3.11 - 3.03 (m, 8H), 2.98 - 2.80 (m, 2H), 2.68 - 2.63 (m, 1H), 2.18 - 1.88 (m, 3H), 1.32 (t, J = 6.5 Hz, 1H). LCMS (ESI) calcd. for C₃₅H₃₉F₂N₆O₂S⁺ [M+H]⁺: 645.28, found, 645.3.

### Example 28: Preparation of 3-(6-((3,3-difluoro-4-(4-(phenyl(pyridin-2-yl)methyl)piperazin-1-yl)piperidin-1-yl)methyl)-1-thioxoisoindolin-2-yl)piperidine-2,6-dione (GT-05042)

With reference to the method of Scheme 1, the target compound (GT-05042) was obtained as a white solid (11 mg, 9%). ¹H NMR (400 MHz, DMSO-d6) δ 11.15 (s, 1H), 8.74 (d, J = 4.6 Hz, 1H), 7.87 - 7.82 (m, 2H), 7.71 - 7.67 (m, 4H), 7.67 - 7.62 (m, 1H), 7.49 - 7.43 (m, 4H), 5.95 - 5.86 (m, 1H), 4.90 - 4.85 (m, 1H), 4.73 - 4.62 (m, 1H), 4.48 - 4.34 (m, 2H), 3.16 - 3.05 (m, 6H), 2.98 - 2.82 (m, 4H), 2.66 (d, J = 16.7 Hz, 2H), 2.15 - 1.96 (m, 5H), 1.34 - 1.31 (m, 4H). LCMS (ESI) calcd. for C₃₅H₃₉F₂N₆O₂S⁺ [M+H]⁺: 645.28, found, 645.3.

### Example 29: Preparation of 1-(5-((4-(4-benzhydrylpiperazin-1-yl)piperidin-1-yl)methyl)-1-oxoisoindolin-2-yl)dihydropyrimidine-2,4(1H,3H)-dione (GT-04941)

With reference to the method of Scheme 1, the target compound (GT-04941) was obtained as a white solid (15 mg, 18%). ¹H NMR (400 MHz, DMSO) δ 10.69 (s, 1H), 7.91 - 7.82 (m, 2H), 7.75 (d, J = 7.4 Hz, 1H), 7.53 - 7.43 (m, 4H), 7.38 - 7.31 (m, 4H), 7.27 - 7.21 (m, 2H), 4.80 (d, J = 17.0 Hz, 1H), 4.57 (d, J = 15.6 Hz, 1H), 4.42 (brs, 2H), 3.79 (t, J = 7.0 Hz, 2H), 3.67 - 3.47 (m, 7H), 3.14 - 3.06 (m, 2H), 3.03 - 2.73 (m, 7H), 2.33 - 2.22 (m, 2H), 2.17 - 2.06 (m, 2H). LCMS (ESI) calcd. for C₃₅H₄₁N₆O₃⁺ [M+H]⁺: 593.32, found, 593.3.

### Example 30: Preparation of 5-((4-(4-benzhydrylpiperazin-1-yl)piperidin-1-yl)methyl)-2-(2,4-dioxotetrahydropyrimidin-1(2H)-yl)isoindoline-1,3-dione (GT-05255)

With reference to the method of Scheme 1, the target compound (GT-05255) was obtained as a white solid (34 mg, 37%). ¹H NMR (400 MHz, DMSO-d6) δ 10.93 (s, 1H), 8.15 - 8.10 (m, 1H), 7.65 - 7.44 (m, 4H), 7.40 - 7.31 (m, 5H), 7.31 - 7.14 (m, 3H), 4.52 - 4.41 (m, 2H), 3.82 (t, J = 6.7 Hz, 1H), 3.63 - 3.48 (m, 4H), 3.27 - 3.13 (m, 4H), 3.06 - 2.94 (m, 3H), 2.90 - 2.83 (m, 4H), 2.35 - 2.22 (m, 3H), 2.14 - 2.04 (m, 2H), 1.96 - 1.85 (m, 1H). LCMS (ESI) calcd. for C₃₅H₃₉N₆O₄⁺ [M+H]⁺: 607.30, found, 607.3.

### Example 31: Preparation of 1-(5-((4-(4-benzhydrylpiperazin-1-yl)-3,3-difluoropiperidin-1-yl)methyl)-1-oxoisoindolin-2-yl)dihydropyrimidine-2,4(1H,3H)-dione (GT-04942)

With reference to the method of Scheme 1, the target compound (GT-04942) was obtained as a white solid (22 mg, 24%). ¹H NMR (400 MHz, DMSO) δ 10.67 (s, 1H), 7.92 - 7.76 (m, 5H), 7.74 - 7.63 (m, 1H), 7.63 - 7.52 (m, 1H), 7.45 - 7.42 (m, 4H), 7.40 - 7.31 (m, 2H), 5.59 (d, J = 9.0 Hz, 1H), 4.77 (d, J = 16.4 Hz, 1H), 4.55 (d, J = 16.1 Hz, 1H), 3.80 - 3.76 (m, 3H), 3.28 - 3.19 (m, 4H), 3.14 - 3.10 (m, 3H), 3.05 - 2.96 (m, 5H), 2.93 - 2.81 (m, 2H), 2.78 - 2.72 (m, 2H), 1.99 - 1.81 (m, 2H). LCMS (ESI) calcd. for C₃₅H₃₉F₂N₆O₃⁺ [M+H]⁺: 629.30, found, 629.3.

### Example 32: Preparation of 5-((4-(4-benzhydrylpiperazin-1-yl)-3,3-difluoropiperidin-1-yl)methyl)-2-(2,4-dioxotetrahydropyrimidin-1(2H)-yl)isoindoline-1,3-dione (GT-05256)

With reference to the method of Scheme 1, the target compound (GT-05256) was obtained as a white solid (22 mg, 23%). ¹H NMR (400 MHz, DMSO-d6) δ 10.91 (s, 1H), 8.08 - 8.00 (m, 2H), 7.98 - 7.95 (m, 1H), 7.90 - 7.79 (m, 4H), 7.44 (t, J = 7.4 Hz, 4H), 7.38 - 7.35 (m, 2H), 5.59 (brs, 1H), 4.20 - 4.04 (m, 2H), 3.65 - 3.61 (m, 4H), 3.27 - 3.17 (m, 4H), 3.18 - 3.08 (m, 4H), 3.06 - 2.98 (m, 5H), 2.02 - 1.78 (m, 2H). LCMS (ESI) calcd. for C₃₅H₃₇F₂N₆O₄⁺ [M+H]⁺: 643.28, found, 643.3.

### Example 33: Preparation of 1-(5-((3,3-difluoro-4-(4-(phenyl(pyridin-2-yl)methyl)piperazin-1-yl)piperidin-1-yl)methyl)-1-oxoisoindolin-2-yl)dihydropyrimidine-2,4(1H,3H)-dione (GT-05082)

With reference to the method of Scheme 1, the target compound (GT-05082) was obtained as a white solid (80 mg, 71%). ¹H NMR (400 MHz, DMSO-d6) δ 10.69 (s, 1H), 8.74 (d, J = 4.8 Hz, 1H), 7.94 - 7.89 (m, 1H), 7.85 - 7.80 (m, 2H), 7.76 - 7.61 (m, 4H), 7.44 - 7.40 (m, 4H), 5.87 (s, 1H), 4.80 (d, J = 16.7 Hz, 1H), 4.57 (d, J = 16.5 Hz, 1H), 4.31 (brs, 2H), 3.80 - 3.77 (m, 4H), 3.23 - 3.10 (m, 4H), 3.10 - 2.97 (m, 6H), 2.88 - 2.81 (m, 2H), 2.79 - 2.73 (m, 1H), 2.19 - 1.90 (m, 2H). LCMS (ESI) calcd. for C₃₄H₃₈F₂N₇O₃⁺ [M+H]⁺: 630.30, found, 630.3.

### Example 34: Preparation of 5-((3,3-difluoro-4-(4-(phenyl(pyridin-2-yl)methyl)piperazin-1-yl)piperidin-1-yl)methyl)-2-(2,4-dioxotetrahydropyrimidin-1(2H)-yl)isoindoline-1,3-dione (GT-05257)

With reference to the method of Scheme 1, the target compound (GT-05257) was obtained as a white solid (13 mg, 13%). ¹H NMR (400 MHz, DMSO-d6) δ 10.92 (s, 1H), 8.74 (d, J = 4.7 Hz, 1H), 8.06 (s, 1H), 7.92 (t, J = 7.5 Hz, 1H), 7.76 - 7.61 (m, 4H), 7.49 - 7.40 (m, 5H), 5.86 (s, 1H), 4.30 (s, 2H), 3.80 - 3.75 (m, 4H), 3.19 - 3.00 (m, 13H), 2.09 - 1.93 (m, 2H). LCMS (ESI) calcd. for C₃₄H₃₆F₂N₇O₄⁺ [M+H]⁺: 644.28, found, 644.3.

### Example 35: Preparation of 1-(5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-oxoisoindolin-2-yl)dihydropyrimidine-2,4(1H,3H)-dione (GT-05335)

With reference to the method of Scheme 1, the target compound (GT-05335) was obtained as a white solid (25 mg, 23%). ¹H NMR (400 MHz, DMSO-d6) δ 10.68 (s, 1H), 7.82 (d, J = 7.7 Hz, 1H), 7.69 (s, 1H), 7.60 (s, 1H), 7.38 (d, J = 8.5 Hz, 2H), 7.25 (d, J = 8.4 Hz, 2H), 4.78 (d, J = 17.1 Hz, 1H), 4.56 (d, J = 16.9 Hz, 1H), 4.36 - 3.92 (m, 2H), 3.78 (t, J = 6.1 Hz, 3H), 3.25 - 3.17 (m, 7H), 3.07 - 2.95 (m, 2H), 2.94 - 2.82 (m, 2H), 2.79 - 2.73 (m, 2H), 2.44 - 2.33 (m, 3H), 2.18 - 2.00 (m, 1H), 1.96 - 1.89 (m, 2H), 1.83 - 1.72 (m, 2H), 1.50 - 1.34 (m, 2H), 1.00 (s, 3H), 0.97 (s, 3H). LCMS (ESI) calcd. for C₃₇H₄₄F₂N₆O₄⁺ [M+H]⁺: 709.31, found, 709.3.

### Example 36: Preparation of 5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-2-(2,4-dioxotetrahydropyrimidin-1(2H)-yl)isoindoline-1,3-dione (GT-05271)

With reference to the method of Scheme 1, the target compound (GT-05271) was obtained as a white solid (20 mg, 19%). ¹H NMR (400 MHz, DMSO-d6) δ 10.92 (s, 1H), 8.08 - 8.01 (m, 2H), 7.93 - 7.90 (m, 1H), 7.39 (d, J = 8.4 Hz, 2H), 7.25 (d, J = 8.4 Hz, 2H), 4.12 - 3.96 (m, 3H), 3.33 - 3.13 (m, 9H), 3.12 - 2.96 (m, 4H), 2.69 - 2.57 (m, 2H), 2.42 - 2.37 (m, 3H), 2.19 - 2.04 (m, 1H), 1.90 (d, J = 16.6 Hz, 1H), 1.82 - 1.70 (m, 3H), 1.50 - 1.35 (m, 2H), 1.00 (s, 3H), 0.97 (s, 3H). LCMS (ESI) calcd. for C₃₇H₄₂ClF₂N₆O₅⁺ [M+H]⁺: 723.29, found, 723.3.

### Example 37: Preparation of 5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-2-(2,4-dioxotetrahydropyrimidin-1(2H)-yl)isoindoline-1,3-dione (GT-05270)

With reference to the method of Scheme 1, the target compound (GT-05270) was obtained as a white solid (26 mg, 25%). ¹H NMR (400 MHz, DMSO-d6) δ 10.92 (s, 1H), 8.04 - 8.00 (m, 2H), 7.98 - 7.90 (m, 1H), 7.45 (d, J = 8.4 Hz, 2H), 7.14 (d, J = 8.4 Hz, 2H), 4.99 (s, 1H), 3.82 (t, J = 6.7 Hz, 4H), 3.27 - 3.19 (m, 6H), 3.12 - 2.96 (m, 6H), 2.86 (t, J = 6.7 Hz, 4H), 2.70 - 2.57 (m, 3H), 2.29 (brs, 2H), 2.04 (brs, 2H), 1.47 (t, J = 6.8 Hz, 2H), 0.96 (s, 6H). LCMS (ESI) calcd. for C₃₇H₄₄ClF₂N₆O₄⁺ [M+H]⁺: 709.31, found, 709.3.

### Example 38: Preparation of 5-((4-(4-(2,3-dichlorophenyl)piperazin-1-yl)-3,3-difluoropiperidin-1-yl)methyl)-2-(2,4-dioxotetrahydropyrimidin-1(2H)-yl)isoindoline-1,3-dione (GT-05258)

With reference to the method of Scheme 1, the target compound (GT-05258) was obtained as a white solid (16 mg, 17%). ¹H NMR (400 MHz, DMSO-d6) δ 10.92 (s, 1H), 8.02 (s, 2H), 7.94 - 7.92 (m, 1H), 7.36 - 7.32 (m, 2H), 7.20 - 7.17 (m, 1H), 4.99 (s, 1H), 4.11 - 3.91 (m, 2H), 3.36 - 3.17 (m, 13H), 3.13 - 3.01 (m, 3H), 2.28 -2.18 (m, 1H), 2.09 - 1.89 (m, 1H). LCMS (ESI) calcd. for C₂₈H₂₉Cl₂F₂N₆O₄⁺ [M+H]⁺: 621.16, found, 621.2.

### Example 39: Preparation of 3-(5-((4-(4-(2,3-dichlorophenyl)-3,6-dihydropyridin-1(2H)-yl)-3,3-difluoropiperidin-1-yl)methyl)-1-oxoisoindolin-2-yl)piperidine-2,6-dione (GT-05333)

With reference to the method of Scheme 1, the target compound (GT-05333) was obtained as a white solid (26 mg, yield 24%). ¹H NMR (400 MHz, DMSO) δ 11.01 (s, 1H), 7.77 (dd, *J* = 7.9, 1.7 Hz, 1H), 7.68 (d, *J* = 8.9 Hz, 1H), 7.63 - 7.58 (m, 2H), 7.38 (t, *J* = 7.9 Hz, 1H), 7.26 (dd, *J* = 7.6, 1.3 Hz, 1H), 5.75 (s, 1H), 5.13 (dd, *J* = 13.3, 5.1 Hz, 2H), 4.49 (d, *J* = 17.9 Hz, 2H), 4.37 (d, *J* = 17.9 Hz, 2H), 4.10 - 3.82 (m, 5H), 3.43 - 3.39 (m, 4H), 3.23 - 3.14 (m, 1H), 2.96 - 2.89 (m, 2H), 2.67 - 2.63 (m, 2H), 2.47 - 2.39 (m, 2H), 2.07 - 1.98 (m, 2H). LCMS (ESI) calcd. for C₃₀H₃₁Cl₂F₂N₄O₃⁺ [M+H]⁺: 603.17, found, 603.2.

### Example 40: Preparation of 3-(5-((4-(4-benzhydrylpiperazin-1-yl)piperidin-1-yl)methyl)-1-thioxoisoindolin-2-yl)piperidine-2,6-dione (GT-09941)

With reference to the method of Scheme 1, the target compound (GT-09941) was obtained as a white solid (7 mg, yield 11%). ¹H NMR (400 MHz, DMSO) δ 11.14 (s, 1H), 7.96 (d, *J* = 7.9 Hz, 1H), 7.89 (s, 1H), 7.85 (d, *J* = 8.0 Hz, 1H), 7.75 (d, *J* = 8.2 Hz, 1H), 7.60 (s, 1H), 7.49 (d, *J* = 8.1 Hz, 2H), 7.40 - 7.30 (m, 4H), 7.24 (brs, 2H), 5.95 (d, *J* = 8.9 Hz, 1H), 4.87 (d, *J* = 20.5 Hz, 1H), 4.77 (d, *J* = 20.4 Hz, 1H), 4.63 (s, 1H), 4.43 (s, 2H), 3.59 - 3.48 (m, 4H), 3.32 - 3.18 (m, 4H), 3.05 - 2.83 (m, 6H), 2.67 - 2.63 (m, 2H), 2.31 - 2.21 (m, 2H), 2.17 - 2.06 (m, 3H). LCMS (ESI) calcd. for C₃₆H₄₂N₅O₂S⁺ [M+H]⁺: 608.31, found, 608.3.

### Example 41: Preparation of 3-(4-((4-(4-benzhydrylpiperazin-1-yl)piperidin-1-yl)methyl)-1-thioxoisoindolin-2-yl)piperidine-2,6-dione (GT-09938)

With reference to the method of Scheme 1, the target compound (GT-09938) was obtained as a white solid (8 mg, yield 12%). ¹H NMR (400 MHz, DMSO) δ 11.20 (s, 1H), 7.99 (d, *J* = 7.6 Hz, 1H), 7.94 (d, *J* = 7.4 Hz, 1H), 7.67 (t, *J* = 7.8 Hz, 1H), 7.56 - 7.43 (m, 4H), 7.38 - 7.32 (m, 4H), 7.26 - 7.22 (m, 2H), 6.07 - 5.94 (m, 1H), 5.24 (d, *J* = 20.0 Hz, 1H), 4.88 (d, *J* = 20.6 Hz, 1H), 4.53 - 4.33 (m, 3H), 3.65 - 3.55 (m, 2H), 3.14 - 3.05 (m, 4H), 2.98 - 2.82 (m, 4H), 2.74 - 2.66 (m, 2H), 2.46 - 2.38 (m, 2H), 2.32 - 2.22 (m, 3H), 2.20 - 2.06 (m, 4H). LCMS (ESI) calcd. for C₃₆H₄₂N₅O₂S⁺ [M+H]⁺: 608.31, found, 608.3.

### Example 42: Preparation of 3-(6-((4-(4-benzhydrylpiperazin-1-yl)piperidin-1-yl)methyl)-1-thioxoisoindolin-2-yl)piperidine-2,6-dione (GT-09862)

With reference to the method of Scheme 1, the target compound (GT-09862) was obtained as a white solid (8 mg, yield 12%). ¹H NMR (400 MHz, DMSO) δ 11.14 (s, 1H), 8.10 (s, 1H), 7.87 (d, *J* = 7.3 Hz, 1H), 7.77 (d, *J* = 8.1 Hz, 1H), 7.51 - 7.44 (m, 4H), 7.37 - 7.31 (m, 4H), 7.25 - 7.21 (m, 2H), 6.01 - 5.86 (m, 1H), 4.88 (d, *J* = 20.7 Hz, 1H), 4.74 (d, *J* = 20.2 Hz, 1H), 4.46 (brs, 3H), 3.56 - 3.51 (m, 2H), 3.13 - 3.04 (m, 2H), 3.03 - 2.92 (m, 4H), 2.89 - 2.80 (m, 2H), 2.67 - 2.62 (m, 2H), 2.45 - 2.42 (m, 3H), 2.34 - 2.26 (m, 2H), 2.14 - 1.98 (m, 4H). LCMS (ESI) calcd. for C₃₆H₄₂N₅O₂S⁺ [M+H]⁺: 608.31, found, 608.3.

### Example 43: Preparation of 3-(4-((4-(4-benzhydrylpiperazin-1-yl)-3,3-difluoropiperidin-1-yl)methyl)-1-thioxoisoindolin-2-yl)piperidine-2,6-dione (GT-09617)

With reference to the method of Scheme 1, the target compound (GT-09617) was obtained as a white solid (12 mg, yield 16%). ¹H NMR (400 MHz, DMSO) δ 11.12 (s, 1H), 7.91 - 7.77 (m, 5H), 7.67 - 7.52 (m, 2H), 7.48 - 7.43 (m, 4H), 7.37 - 7.35 (m, 2H), 5.97 (d, *J* = 10.5 Hz, 1H), 5.60 (d, *J* = 7.3 Hz, 1H), 4.86 (d, *J* = 19.2 Hz, 1H), 4.74 (d, *J* = 18.1 Hz, 1H), 3.89 - 3.67 (m, 1H), 3.26 - 3.09 (m, 6H), 3.08 - 2.94 (m, 7H), 2.65 (d, *J* = 16.8 Hz, 2H), 2.47 - 2.39 (m, 2H), 2.15 - 2.03 (m, 1H), 1.82 - 1.73 (m, 2H). LCMS (ESI) calcd. for C₃₆H₄₀F₂N₅O₂S⁺ [M+H]⁺: 644.29, found, 644.3.

### Example 44: Preparation of 3-(6-((4-(4-benzhydrylpiperazin-1-yl)-3,3-difluoropiperidin-1-yl)methyl)-1-thioxoisoindolin-2-yl)piperidine-2,6-dione (GT-09691)

With reference to the method of Scheme 1, the target compound (GT-09691) was obtained as a white solid (14 mg, yield 20%). ¹H NMR (400 MHz, DMSO) δ 11.06 (s, 1H), 7.95 - 7.84 (m, 1H), 7.81 - 7.66 (m, 4H), 7.61 (s, 2H), 7.44 - 7.33 (m, 4H), 7.34 - 7.23 (m, 2H), 5.93 - 5.82 (m, 1H), 5.57 - 5.47 (m, 1H), 4.77 (d, *J* = 20.2 Hz, 1H), 4.63 (d, *J* = 19.9 Hz, 1H), 4.01 - 3.68 (m, 2H), 3.65 - 3.38 (m, 6H), 3.13 - 3.02 (m, 5H), 3.07 - 2.92 (m, 5H), 2.61 - 2.55 (m, 2H), 2.08 - 1.96 (m, 1H), 1.89 - 1.70 (m, 1H). LCMS (ESI) calcd. for C₃₆H₄₀F₂N₅O₂S⁺ [M+H]⁺: 644.29, found, 644.3.

### Example 45: Preparation of 3-(4-((3,3-difluoro-4-(4-(phenyl(pyridin-2-yl)methyl)piperazin-1-yl)piperidin-1-yl)methyl)-1-thioxoisoindolin-2-yl)piperidine-2,6-dione (GT-09618)

With reference to the method of Scheme 1, the target compound (GT-09618) was obtained as a white solid (15 mg, yield 20%). ¹H NMR (400 MHz, DMSO) δ 11.15 (s, 1H), 8.74 (d, *J* = 4.8 Hz, 1H), 7.93 - 7.90 (m, 2H), 7.68 (d, *J* = 7.0 Hz, 3H), 7.61 (dd, *J* = 13.9, 7.5 Hz, 2H), 7.51 - 7.37 (m, 4H), 5.98 (d, *J* = 8.0 Hz, 1H), 5.84 (s, 1H), 5.01 (dd, *J* = 33.7, 18.0 Hz, 1H), 4.80 (d, *J* = 20.1 Hz, 1H), 4.29 - 3.92 (m, 2H), 3.27 - 2.90 (m, 13H), 2.66 (d, *J* = 14.1 Hz, 2H), 2.47 - 2.38 (m, 1H), 2.17 - 2.05 (m, 1H), 2.06 - 1.82 (m, 2H). LCMS (ESI) calcd. for C₃₅H₃₉F₂N₆O₂S⁺ [M+H]⁺: 645.28, found, 645.3.

### Example 46: Preparation of 3-(5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-thioxoisoindolin-2-yl)piperidine-2,6-dione (GT-09679)

With reference to the method of Scheme 1, the target compound (GT-09679) was obtained as a white solid (11 mg, yield 14%). ¹H NMR (400 MHz, DMSO) δ 11.13 (s, 1H), 7.91 (d, *J* = 7.5 Hz, 1H), 7.70 - 7.65 (m, 1H), 7.63 - 7.50 (m, 1H), 7.37 (d, *J* = 8.4 Hz, 2H), 7.24 (d, *J* = 8.5 Hz, 2H), 6.03 - 5.90 (m, 1H), 4.84 (d, *J* = 19.7 Hz, 1H), 4.70 (d, *J* = 20.2 Hz, 1H), 3.17 - 3.08 (m, 4H), 3.02 - 2.93 (m, 4H), 2.71 - 2.61 (m, 2H), 2.42 - 2.33 (m, 6H), 2.15 - 2.00 (m, 4H), 1.96 - 1.85 (m, 2H), 1.74 - 1.59 (m, 2H), 1.51 - 1.37 (m, 3H), 0.99 (s, 3H), 0.96 (s, 3H). LCMS (ESI) calcd. for C₃₈H₄₅ClF₂N₅O₃S⁺ [M+H]⁺: 724.29, found, 724.3.

### Example 47: Preparation of 3-(4-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-thioxoisoindolin-2-yl)piperidine-2,6-dione (GT-09619)

With reference to the method of Scheme 1, the target compound (GT-09619) was obtained as a white solid (19 mg, yield 23%). ¹H NMR (400 MHz, DMSO) δ 11.15 (d, *J* = 4.5 Hz, 1H), 7.87 (d, *J* = 7.5 Hz, 1H), 7.67 - 7.50 (m, 2H), 7.37 (d, *J* = 8.4 Hz, 2H), 7.24 (d, *J* = 8.5 Hz, 2H), 6.07 - 5.91 (m, 1H), 4.86 (d, *J* = 18.8 Hz, 1H), 4.75 (d, *J* = 21.0 Hz, 1H), 3.91 - 3.74 (m, 1H), 3.27 - 3.19 (m, 7H), 3.05 - 2.86 (m, 4H), 2.72 - 2.62 (m, 2H), 2.44 - 2.29 (m, 4H), 2.19 - 2.00 (m, 3H), 1.90 (d, *J* = 17.2 Hz, 1H), 1.80 - 1.57 (m, 2H), 1.52 - 1.36 (m, 2H), 0.98 (d, *J* = 13.3 Hz, 6H). LCMS (ESI) calcd. for C₃₈H₄₅ClF₂N₅O₃S⁺ [M+H]⁺: 724.29, found, 724.3.

### Example 48: Preparation of 3-(6-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-thioxoisoindolin-2-yl)piperidine-2,6-dione (GT-09692)

With reference to the method of Scheme 1, the target compound (GT-09692) was obtained as a white solid (10 mg, yield 13%). ¹H NMR (400 MHz, DMSO) δ 11.13 (s, 1H), 7.90 - 7.85 (m, 1H), 7.72 - 7.69 (m, 2H), 7.37 (d, *J* = 8.3 Hz, 2H), 7.24 (d, *J* = 8.5 Hz, 2H), 5.98 - 5.92 (m, 1H), 4.84 (d, *J* = 20.9 Hz, 1H), 4.70 (d, *J* = 20.1 Hz, 1H), 3.31 - 3.17 (m, 6H), 3.15 - 3.02 (m, 3H), 3.03 - 2.90 (m, 3H), 2.68 - 2.62 (m, 2H), 2.43 - 2.33 (m, 4H), 2.14 - 2.04 (m, 3H), 1.90 (d, *J* = 15.5 Hz, 1H), 1.79 - 1.60 (m, 3H), 1.47 - 1.38 (m, 2H), 0.98 (d, *J* = 12.9 Hz, 6H). LCMS (ESI) calcd. for C₃₈H₄₅ClF₂N₅O₄⁺ [M+H]⁺: 708.31, found, 708.3.

### Example 49: Preparation of 3-(5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-thioxoisoindolin-2-yl)piperidine-2,6-dione (GT-09680)

With reference to the method of Scheme 1, the target compound (GT-09680) was obtained as a white solid (21 mg, yield 27%). ¹H NMR (400 MHz, DMSO) δ 11.13 (s, 1H), 7.90 (d, *J* = 8.0 Hz, 1H), 7.65 - 7.51 (m, 2H), 7.44 (d, *J* = 8.4 Hz, 2H), 7.13 (d, *J* = 8.4 Hz, 2H), 6.01 - 5.90 (m, 1H), 4.83 (d, *J* = 18.0 Hz, 1H), 4.70 (d, *J* = 20.4 Hz, 1H), 3.57 - 3.53 (m, 2H), 3.24 - 3.19 (m, 4H), 3.03 - 2.96 (m, 3H), 2.89 - 2.81 (m, 4H), 2.65 - 2.58 (m, 4H), 2.30 - 2.24 (m, 4H), 2.03 (s, 4H), 1.80 - 1.67 (m, 2H), 1.47 - 1.45 (m, 2H), 0.96 (s, 6H). LCMS (ESI) calcd. for C₃₈H₄₇ClF₂N₅O₂S⁺ [M+H]⁺: 710.31, found, 710.3.

### Example 50: Preparation of 3-(4-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-thioxoisoindolin-2-yl)piperidine-2,6-dione (GT-09576)

With reference to the method of Scheme 1, the target compound (GT-09576) was obtained as a white solid (23 mg, yield 28%). ¹H NMR (400 MHz, DMSO) δ 11.15 (s, 1H), 7.86 (d, *J* = 6.5 Hz, 1H), 7.84 - 7.77 (m, 1H), 7.60 - 7.55 (m, 1H), 7.44 (d, *J* = 8.3 Hz, 2H), 7.14 (d, *J* = 8.3 Hz, 2H), 5.97 (s, 1H), 4.90 (d, *J* = 20.5 Hz, 1H), 4.78 (d, *J* = 20.1 Hz, 1H), 3.94 - 3.64 (m, 2H), 3.55 (brs, 2H), 3.39 - 3.31 (m, 4H), 3.25 - 3.18 (m, 3H), 3.07 - 2.97 (m, 4H), 2.94 - 2.79 (m, 3H), 2.67 - 2.60 (m, 3H), 2.33 - 2.25 (m, 3H), 2.17 - 2.07 (m, 1H), 2.04 (s, 2H), 1.85 - 1.72 (m, 2H), 1.46 (t, *J* = 6.1 Hz, 2H), 0.96 (s, 6H). LCMS (ESI) calcd. for C₃₈H₄₇ClF₂N₅O₂S⁺ [M+H]⁺: 710.31, found, 710.3.

### Example 51: Preparation of 3-(6-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-thioxoisoindolin-2-yl)piperidine-2,6-dione (GT-09863)

With reference to the method of Scheme 1, the target compound (GT-09863) was obtained as a white solid (16 mg, yield 21%). ¹H NMR (400 MHz, DMSO) δ 11.13 (s, 1H), 7.99 - 7.92 (m, 1H), 7.75 - 7.62 (m, 2H), 7.44 (d, *J* = 8.3 Hz, 2H), 7.13 (d, *J* = 8.3 Hz, 2H), 5.98 - 5.94 (m, 1H), 4.84 (d, *J* = 20.2 Hz, 1H), 4.69 (d, *J* = 19.6 Hz, 1H), 3.54 (s, 2H), 3.25 - 3.17 (m, 4H), 3.07 - 2.96 (m, 4H), 2.93 - 2.75 (m, 4H), 2.71 - 2.53 (m, 6H), 2.33 - 2.25 (m, 3H), 2.15 - 2.07 (m, 1H), 2.03 (s, 3H), 1.92 - 1.63 (m, 2H), 0.96 (s, 6H). LCMS (ESI) calcd. for C₃₈H₄₇ClF₂N₅O₂S⁺ [M+H]⁺: 710.31, found, 710.3.

### Example 52: Preparation of 3-(5-((4-(4-(2,3-dichlorophenyl)piperazin-1-yl)piperidin-1-yl)methyl)-1-thioxoisoindolin-2-yl)piperidine-2,6-dione (GT-09934)

With reference to the method of Scheme 1, the target compound (GT-09934) was obtained as a white solid (8 mg, yield 12%). ¹H NMR (400 MHz, DMSO) δ 11.15 (s, 1H), 8.03 - 7.94 (m, 1H), 7.85 (d, *J* = 8.0 Hz, 1H), 7.81 - 7.69 (m, 1H), 7.42 - 7.34 (m, 2H), 7.20 (dd, *J* = 7.0, 2.3 Hz, 1H), 6.04 - 5.88 (m, 1H), 4.86 (d, *J* = 20.5 Hz, 1H), 4.76 (d, *J* = 20.2 Hz, 1H), 4.52 - 4.40 (m, 1H), 3.68 - 3.61 (m, 2H), 3.29 - 3.23 (m, 4H), 3.21 - 3.13 (m, 4H), 3.05 - 2.90 (m, 4H), 2.67 - 2.61 (m, 2H), 2.41 - 2.36 (m, 2H), 2.18 - 2.03 (m, 4H).LCMS (ESI) calcd. for C₂₉H₃₄Cl₂N₅O₂S⁺ [M+H]⁺: 586.18, found, 586.3.

### Example 53: Preparation of 3-(4-((4-(4-(2,3-dichlorophenyl)piperazin-1-yl)piperidin-1-yl)methyl)-1-thioxoisoindolin-2-yl)piperidine-2,6-dione (GT-09939)

With reference to the method of Scheme 1, the target compound (GT-09939) was obtained as a white solid (14 mg, yield 21%). ¹H NMR (400 MHz, DMSO) δ 11.21 (s, 1H), 8.07 - 7.87 (m, 2H), 7.78 - 7.62 (m, 1H), 7.40 - 7.30 (m, 2H), 7.25 - 7.14 (m, 1H), 6.02 - 5.98 (m, 1H), 5.24 (d, *J* = 18.2 Hz, 1H), 4.89 (d, *J* = 18.3 Hz, 1H), 4.53 - 4.30 (m, 2H), 3.70 - 3.55 (m, 4H), 3.17 - 3.07 (m, 4H), 2.97 - 2.82 (m, 2H), 2.74 - 2.66 (m, 2H), 2.44 - 2.39 (m, 2H), 2.30 - 2.18 (m, 3H), 2.18 - 2.04 (m, 4H). LCMS (ESI) calcd. for C₂₉H₃₄Cl₂N₅O₂S⁺ [M+H]⁺: 586.18, found, 586.3.

### Example 54: Preparation of 3-(6-((4-(4-(2,3-dichlorophenyl)piperazin-1-yl)piperidin-1-yl)methyl)-1-thioxoisoindolin-2-yl)piperidine-2,6-dione (GT-09935)

With reference to the method of Scheme 1, the target compound (GT-09935) was obtained as a white solid (8 mg, yield 12%). ¹H NMR (400 MHz, DMSO) δ 11.14 (s, 1H), 8.14 - 8.10 (m, 1H), 7.93 - 7.86 (m, 1H), 7.82 - 7.74 (m, 1H), 7.42 - 7.35 (m, 2H), 7.22 - 7.18 (m, 1H), 5.96 (d, *J* = 10.3 Hz, 2H), 4.89 (d, *J* = 20.8 Hz, 1H), 4.75 (d, *J* = 20.4 Hz, 2H), 4.55 - 4.42 (m, 2H), 3.65 - 3.54 (m, 4H), 3.24 - 3.16 (m, 6H), 3.03 - 2.92 (m, 4H), 2.67 - 2.63 (m, 2H), 2.44 - 2.33 (m, 2H), 2.18 - 2.04 (m, 3H). LCMS (ESI) calcd. for C₂₉H₃₄Cl₂N₅O₂S⁺ [M+H]⁺: 586.18, found, 586.3.

### Example 55: Preparation of 3-(5-((4-(4-(2,3-dichlorophenyl)piperazin-1-yl)-3,3-difluoropiperidin-1-yl)methyl)-1-thioxoisoindolin-2-yl)piperidine-2,6-dione (GT-09687)

With reference to the method of Scheme 1, the target compound (GT-09687) was obtained as a white solid (13 mg, yield 19%). ¹H NMR (400 MHz, DMSO) δ 11.07 (s, 1H), 7.86 (d, *J* = 8.0 Hz, 1H), 7.63 (s, 1H), 7.53 (d, *J* = 7.6 Hz, 1H), 7.29 - 7.24 (m, 2H), 7.14 - 7.05 (m, 1H), 5.89 (d, *J* = 11.3 Hz, 1H), 4.79 (d, *J* = 20.5 Hz, 1H), 4.65 (d, *J* = 20.6 Hz, 1H), 4.10 - 3.83 (m, 2H), 3.17 - 2.98 (m, 10H), 2.95 - 2.84 (m, 2H), 2.63 - 2.55 (m, 2H), 2.50 - 2.45 (m, 2H), 2.17 - 2.08 (m, 1H), 2.06 - 2.01 (m, 1H), 1.99 - 1.87 (m, 1H). LCMS (ESI) calcd. for C₂₉H₃₂Cl₂F₂N₅O₂S⁺ [M+H]⁺: 622.16, found, 622.2.

### Example 56: Preparation of 3-(4-((4-(4-(2,3-dichlorophenyl)piperazin-1-yl)-3,3-difluoropiperidin-1-yl)methyl)-1-thioxoisoindolin-2-yl)piperidine-2,6-dione (GT-09575)

With reference to the method of Scheme 1, the target compound (GT-09575) was obtained as a white solid (31 mg, yield 43%). ¹H NMR (400 MHz, DMSO) δ 11.15 (s, 1H), 7.87 (d, *J* = 7.7 Hz, 1H), 7.70 - 7.61 (m, 1H), 7.58 (d, *J* = 7.4 Hz, 1H), 7.34 (d, *J* = 5.3 Hz, 2H), 7.21 - 7.15 (m, 1H), 6.01 - 5.95 (m, 1H), 4.89 (d, *J* = 19.8 Hz, 1H), 4.77 (d, *J* = 20.9 Hz, 1H), 3.97 - 3.72 (m, 2H), 3.24 - 3.14 (m, 9H), 3.06 - 2.86 (m, 4H), 2.70 - 2.64 (m, 2H), 2.18 - 2.07 (m, 2H), 2.03 - 1.84 (m, 2H). LCMS (ESI) calcd. for C₂₉H₃₂Cl₂F₂N₅O₂S⁺ [M+H]⁺: 622.16, found, 622.2.

### Example 57: Preparation of 3-(6-((4-(4-(2,3-dichlorophenyl)piperazin-1-yl)-3,3-difluoropiperidin-1-yl)methyl)-1-thioxoisoindolin-2-yl)piperidine-2,6-dione (GT-09693)

With reference to the method of Scheme 1, the target compound (GT-09693) was obtained as a white solid (11 mg, yield 16%). 1H NMR (400 MHz, DMSO) δ 11.13 (s, 1H), 8.06 - 7.95 (m, 1H), 7.78 - 7.68 (m, 2H), 7.33 (d, *J* = 4.7 Hz, 2H), 7.16 (t, *J* = 4.8 Hz, 1H), 6.01 - 5.90 (m, 1H), 4.86 (d, *J* = 20.7 Hz, 1H), 4.72 (d, *J* = 20.1 Hz, 1H), 4.30 - 3.84 (m, 2H), 3.18 - 3.05 (m, 11H), 2.70 - 2.62 (m, 2H), 2.22 - 1.93 (m, 6H). LCMS (ESI) calcd. for C₂₉H₃₂Cl₂F₂N₅O₂S⁺ [M+H]⁺: 622.16, found, 622.2.

### Example 58: Preparation of 3-(5-((4-(4-(2,3-dichlorophenyl)-3,6-dihydropyridin-1(2H)-yl)-3,3-difluoropiperidin-1-yl)methyl)-1-thioxoisoindolin-2-yl)piperidine-2,6-dione (GT-09688)

With reference to the method of Scheme 1, the target compound (GT-09688) was obtained as a white solid (12 mg, yield 17%). ¹H NMR (400 MHz, DMSO) δ 11.14 (s, 1H), 7.92 (d, *J* = 7.8 Hz, 1H), 7.66 (s, 1H), 7.63 - 7.54 (m, 2H), 7.38 (t, *J* = 7.7 Hz, 1H), 7.26 (d, *J* = 6.2 Hz, 1H), 6.02 - 5.88 (m, 1H), 5.75 (s, 1H), 4.85 (d, *J* = 20.1 Hz, 1H), 4.71 (d, *J* = 19.7 Hz, 1H), 3.98 - 3.84 (m, 2H), 3.31 - 3.21 (m, 6H), 3.17 - 3.06 (m, 3H), 3.01 - 2.92 (m, 2H), 2.70 - 2.61 (m, 4H), 2.14 - 2.06 (m, 1H), 2.04 - 1.94 (m, 1H). LCMS (ESI) calcd. for C₃₀H₃₁Cl₂F₂N₄O₂S⁺ [M+H]⁺: 619.15, found, 619.2.

### Example 59: Preparation of 3-(4-((4-(4-(2,3-dichlorophenyl)-3,6-dihydropyridin-1(2H)-yl)-3,3-difluoropiperidin-1-yl)methyl)-1-thioxoisoindolin-2-yl)piperidine-2,6-dione (GT-09614)

With reference to the method of Scheme 1, the target compound (GT-09614) was obtained as a white solid (10 mg, yield 14%). ¹H NMR (400 MHz, DMSO) δ 11.14 (s, 1H), 7.87 (d, *J* = 7.4 Hz, 1H), 7.68 - 7.54 (m, 3H), 7.39 (t, *J* = 7.9 Hz, 1H), 7.26 (dd, *J* = 7.6, 1.2 Hz, 1H), 6.00 (d, *J* = 11.0 Hz, 1H), 5.77 (s, 1H), 4.89 (d, *J* = 18.7 Hz, 1H), 4.76 (d, *J* = 20.2 Hz, 1H), 3.97 - 3.74 (m, 4H), 3.37 - 3.19 (m, 9H), 3.08 - 2.92 (m, 2H), 2.71 - 2.65 (m, 2H), 2.17 - 2.09 (m, 1H), 2.02 - 1.93 (m, 1H). LCMS (ESI) calcd. for C₃₀H₃₁Cl₂F₂N₄O₂S⁺ [M+H]⁺: 619.15, found, 619.2.

### Example 60: Preparation of 3-(6-((4-(4-(2,3-dichlorophenyl)-3,6-dihydropyridin-1(2H)-yl)-3,3-difluoropiperidin-1-yl)methyl)-1-thioxoisoindolin-2-yl)piperidine-2,6-dione (GT-09694)

With reference to the method of Scheme 1, the target compound (GT-09694) was obtained as a white solid (10 mg, yield 15%). ¹H NMR (400 MHz, DMSO) δ 11.13 (s, 1H), 8.00 - 7.88 (m, 1H), 7.73 - 7.64 (m, 2H), 7.60 (d, *J* = 8.0 Hz, 1H), 7.38 (t, *J* = 7.8 Hz, 1H), 7.25 (d, *J* = 7.7 Hz, 1H), 6.03 - 5.89 (m, 1H), 5.74 (s, 1H), 4.85 (d, *J* = 20.6 Hz, 1H), 4.71 (d, *J* = 20.2 Hz, 1H), 4.07 - 3.84 (m, 2H), 3.65 - 3.52 (m, 2H), 3.37 - 3.31 (m, 6H), 3.32 - 3.05 (m, 4H), 2.99 - 2.92 (m, 1H), 2.69 - 2.63 (m, 2H), 2.14 - 1.99 (m, 3H). LCMS (ESI) calcd. for C₃₀H₃₁Cl₂F₂N₄O₂S⁺ [M+H]⁺: 619.15, found, 619.2.

### Example 61: Preparation of 3-(5-((4-(4-(6-fluorobenzo[d]isoxazol-3-yl)piperazin-1-yl)piperidin-1-yl)methyl)-1-thioxoisoindolin-2-yl)piperidine-2,6-dione (GT-09861)

With reference to the method of Scheme 1, the target compound (GT-09861) was obtained as a white solid (13 mg, yield 21%). ¹H NMR (400 MHz, DMSO) δ 11.15 (s, 1H), 8.09 (dd, *J* = 8.8, 5.2 Hz, 1H), 7.97 (d, *J* = 8.0 Hz, 1H), 7.89 (s, 1H), 7.77 (t, *J* = 6.8 Hz, 1H), 7.61 (dd, *J* = 8.9, 1.7 Hz, 1H), 7.26 (t, *J* = 8.5 Hz, 1H), 5.97 (d, *J* = 14.1 Hz, 1H), 4.88 (d, *J* = 20.4 Hz, 1H), 4.74 (d, *J* = 20.3 Hz, 1H), 4.45 (brs, 2H), 4.25 - 4.01 (m, 2H), 3.61 - 3.50 (m, 6H), 3.02 - 2.95 (m, 4H), 2.67 - 2.63 (m, 2H), 2.37 - 2.31 (m, 2H), 2.26 - 2.15 (m, 2H), 2.15 - 1.96 (m, 3H). LCMS (ESI) calcd. for C₃₀H₃₄FN₆O₃S⁺ [M+H]⁺: 577.24, found, 577.3.

### Example 62: Preparation of 3-(4-((4-(4-(6-fluorobenzo[d]isoxazol-3-yl)piperazin-1-yl)piperidin-1-yl)methyl)-1-thioxoisoindolin-2-yl)piperidine-2,6-dione (GT-09940)

With reference to the method of Scheme 1, the target compound (GT-09940) was obtained as a white solid (10 mg, yield 15%). ¹H NMR (400 MHz, DMSO) δ 11.21 (s, 1H), 8.12 - 8.08 (m, 1H), 8.04 - 7.90 (m, 2H), 7.68 (t, *J* = 7.7 Hz, 1H), 7.61 (dd, *J* = 9.1, 2.0 Hz, 1H), 7.26 (td, *J* = 9.0, 2.1 Hz, 1H), 6.05 - 6.00 (m, 1H), 5.25 (d, *J* = 21.4 Hz, 1H), 4.88 (d, *J* = 20.4 Hz, 1H), 4.49 - 4.36 (m, 1H), 4.22 - 4.00 (m, 2H), 3.64 - 3.50 (m, 6H), 3.21 - 3.07 (m, 4H), 3.02 - 2.89 (m, 2H), 2.74 - 2.66 (m, 2H), 2.40 - 2.33 (m, 2H), 2.29 - 2.19 (m, 2H), 2.18 - 2.06 (m, 2H). LCMS (ESI) calcd. for C₃₀H₃₄FN₆O₃S⁺ [M+H]⁺: 577.24, found, 577.3.

### Example 63: Preparation of 3-(6-((4-(4-(6-fluorobenzo[d]isoxazol-3-yl)piperazin-1-yl)piperidin-1-yl)methyl)-1-thioxoisoindolin-2-yl)piperidine-2,6-dione (GT-09864)

With reference to the method of Scheme 1, the target compound (GT-09864) was obtained as a white solid (11 mg, yield 16%). ¹H NMR (400 MHz, DMSO) δ 11.14 (s, 1H), 8.14 - 8.05 (m, 2H), 7.90 (d, *J* = 8.1 Hz, 1H), 7.78 (d, *J* = 7.9 Hz, 1H), 7.61 (dd, *J* = 9.0, 2.0 Hz, 1H), 7.26 (t, *J* = 9.0 Hz, 1H), 5.96 (d, *J* = 9.3 Hz, 1H), 4.89 (d, *J* = 20.5 Hz, 1H), 4.75 (d, *J* = 20.5 Hz, 1H), 4.49 (s, 2H), 4.22 - 4.03 (m, 2H), 3.61 - 3.49 (m, 6H), 3.04 - 2.89 (m, 4H), 2.72 - 2.67 (m, 2H), 2.41 - 2.32 (m, 3H), 2.22 - 2.04 (m, 4H). LCMS (ESI) calcd. for C₃₀H₃₄FN₆O₃S⁺ [M+H]⁺: 577.24, found, 577.3.

### Example 64: Preparation of 3-(5-((3,3-difluoro-4-(4-(6-fluorobenzo[d]isoxazol-3-yl)piperazin-1-yl)piperidin-1-yl)methyl)-1-thioxoisoindolin-2-yl)piperidine-2,6-dione (GT-09689)

With reference to the method of Scheme 1, the target compound (GT-09689) was obtained as a white solid (13 mg, yield 19%). ¹H NMR (400 MHz, DMSO) δ 11.13 (s, 1H), 8.05 (dd, *J* = 8.9, 5.3 Hz, 1H), 7.93 (d, *J* = 7.6 Hz, 1H), 7.71 (brs, 1H), 7.62 (brs, 1H), 7.56 (dd, *J* = 9.1, 2.1 Hz, 1H), 7.21 (t, *J* = 9.0 Hz, 1H), 6.04 - 5.87 (m, 1H), 4.85 (d, *J* = 20.3 Hz, 1H), 4.72 (d, *J* = 19.7 Hz, 1H), 4.19 - 3.89 (m, 2H), 3.70 - 3.56 (m, 3H), 3.58 - 3.48 (m, 6H), 3.20 - 3.03 (m, 3H), 3.01 - 2.93 (m, 2H), 2.71 - 2.61 (m, 2H), 2.19 - 1.92 (m, 3H). LCMS (ESI) calcd. for C₃₀H₃₂F₃N₆O₃S⁺ [M+H]⁺: 613.22, found, 613.3.

### Example 65: Preparation of 3-(4-((3,3-difluoro-4-(4-(6-fluorobenzo[d]isoxazol-3-yl)piperazin-1-yl)piperidin-1-yl)methyl)-1-thioxoisoindolin-2-yl)piperidine-2,6-dione (GT-09615)

With reference to the method of Scheme 1, the target compound (GT-09615) was obtained as a white solid (15 mg, yield 21%). ¹H NMR (400 MHz, DMSO) δ 11.16 (s, 1H), 8.06 (dd, *J* = 8.9, 5.3 Hz, 1H), 7.88 (d, *J* = 7.3 Hz, 1H), 7.65 (s, 1H), 7.57 (dd, *J* = 9.1, 2.1 Hz, 2H), 7.22 (t, *J* = 8.0 Hz, 1H), 6.08 - 5.91 (m, 1H), 4.91 (d, *J* = 23.1 Hz, 1H), 4.83 - 4.69 (m, 1H), 3.92 - 3.85 (m, 2H), 3.65 - 3.54 (m, 4H), 3.31 - 3.24 (m, 9H), 3.11 - 2.94 (m, 2H), 2.70 - 2.67 (m, 2H), 2.21 - 2.04 (m, 2H), 2.01 - 1.85 (m, 1H). LCMS (ESI) calcd. for C₃₀H₃₂F₃N₆O₃S⁺ [M+H]⁺: 613.22, found, 613.3.

### Example 66: Preparation of 3-(6-((3,3-difluoro-4-(4-(6-fluorobenzo[d]isoxazol-3-yl)piperazin-1-yl)piperidin-1-yl)methyl)-1-thioxoisoindolin-2-yl)piperidine-2,6-dione (GT-09695)

With reference to the method of Scheme 1, the target compound (GT-09695) was obtained as a white solid (9 mg, yield 13%). ¹H NMR (400 MHz, DMSO) δ 11.14 (s, 1H), 8.04 (dd, *J* = 8.9, 5.3 Hz, 1H), 8.00 - 7.98 (m, 1H), 7.71 (brs, 2H), 7.56 (dd, *J* = 9.1, 2.2 Hz, 1H), 7.20 (t, *J* = 7.9 Hz, 1H), 5.99 - 5.94 (m, 1H), 4.86 (d, *J* = 20.4 Hz, 1H), 4.72 (d, *J* = 20.2 Hz, 1H), 4.27 - 4.21 (m, 2H), 3.74 - 3.66 (m, 2H), 3.11 - 2.93 (m, 12H), 2.69 - 2.65 (m, 2H), 2.12 - 2.03 (m, 3H). LCMS (ESI) calcd. for C₃₀H₃₂F₃N₆O₃S⁺ [M+H]⁺: 613.22, found, 613.3.

### Example 67: Preparation of 3-(5-((3',3'-difluoro-4-(6-fluorobenzo[d]isoxazol-3-yl)-[1,4'-bipiperidin]-1'-yl)methyl)-1-thioxoisoindolin-2-yl)piperidine-2,6-dione (GT-09690)

With reference to the method of Scheme 1, the target compound (GT-09690) was obtained as a white solid (14 mg, yield 21%). ¹H NMR (400 MHz, DMSO) δ 11.13 (s, 1H), 8.18 (s, 1H), 7.90 (d, *J* = 7.9 Hz, 1H), 7.73 (dd, *J* = 9.1, 2.1 Hz, 1H), 7.63 (s, 1H), 7.54 (d, *J* = 7.5 Hz, 1H), 7.35 (dd, *J* = 10.0, 8.1 Hz, 1H), 5.98 - 5.92 (m, 1H), 4.84 (d, *J* = 19.6 Hz, 1H), 4.70 (d, *J* = 20.1 Hz, 1H), 3.94 - 3.78 (m, 2H), 3.76 - 3.65 (m, 2H), 3.60 - 3.43 (m, 6H), 3.08 - 3.03 (m, 1H), 2.98 - 2.92 (m, 1H), 2.69 - 2.63 (m, 2H), 2.45 - 2.29 (m, 4H), 2.28 - 2.16 (m, 2H), 2.15 - 2.03 (m, 2H). LCMS (ESI) calcd. for C₃₁H₃₃F₃N₅O₃S⁺ [M+H]⁺: 612.23, found, 612.2.

### Example 68: Preparation of 3-(4-((3',3'-difluoro-4-(6-fluorobenzo[d]isoxazol-3-yl)-[1,4'-bipiperidin]-1'-yl)methyl)-1-thioxoisoindolin-2-yl)piperidine-2,6-dione (GT-09616)

With reference to the method of Scheme 1, the target compound (GT-09616) was obtained as a white solid (13 mg, yield 18%). ¹H NMR (400 MHz, DMSO) δ 11.13 (s, 1H), 8.26 - 8.14 (m, 1H), 7.86 (d, *J* = 7.4 Hz, 1H), 7.73 (dd, *J* = 9.0, 1.9 Hz, 1H), 7.66 - 7.53 (m, 2H), 7.39 - 7.30 (m, 1H), 6.06 - 5.89 (m, 1H), 4.95 - 4.83 (m, 1H), 4.75 (d, *J* = 19.9 Hz, 1H), 4.16 - 3.94 (m, 1H), 3.89 - 3.61 (m, 4H), 3.60 - 3.44 (m, 4H), 3.26 - 3.12 (m, 2H), 3.01 - 2.95 (m, 2H), 2.75 - 2.62 (m, 2H), 2.46 - 2.33 (m, 3H), 2.27 - 2.19 (m, 2H), 2.17 - 1.95 (m, 2H). LCMS (ESI) calcd. for C₃₁H₃₃F₃N₅O₃S⁺ [M+H]⁺: 612.23, found, 612.2.

### Example 69: Preparation of 3-(6-((3',3'-difluoro-4-(6-fluorobenzo[d]isoxazol-3-yl)-[1,4'-bipiperidin]-1'-yl)methyl)-1-thioxoisoindolin-2-yl)piperidine-2,6-dione (GT-09936)

With reference to the method of Scheme 1, the target compound (GT-09936) was obtained as a white solid (12 mg, yield 17%). ¹H NMR (400 MHz, DMSO) δ 11.13 (s, 1H), 8.15 - 8.06 (m, 1H), 7.94 - 7.86 (m, 1H), 7.72 (d, *J* = 8.9 Hz, 1H), 7.69 - 7.62 (m, 2H), 7.38 - 7.30 (m, 1H), 5.99 - 5.91 (m, 1H), 4.83 (d, *J* = 20.6 Hz, 1H), 4.69 (d, *J* = 20.3 Hz, 1H), 3.87 - 3.82 (m, 2H), 3.28 - 3.14 (m, 6H), 3.08 - 2.90 (m, 4H), 2.71 - 2.64 (m, 2H), 2.30 - 2.22 (m, 4H), 2.14 - 2.06 (m, 2H), 2.02 - 1.98 (m, 2H). LCMS (ESI) calcd. for C₃₁H₃₃F₃N₅O₃S⁺ [M+H]⁺: 612.23, found, 612.2.

### Example 70: Preparation of 1-(5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-oxoisoindolin-2-yl)dihydropyrimidine-2,4(1H,3H)-dione (GT-05334)

With reference to the method of Scheme 1, the target compound (GT-05334) was obtained as a white solid (32 mg, yield 30%). ¹H NMR (400 MHz, DMSO-d6) δ 10.61 (s, 1H), 9.41 (s, 1H), 7.72 (d, *J* = 7.7 Hz, 1H), 7.62 - 7.54 (m, 1H), 7.54 - 7.44 (m, 1H), 7.37 (d, *J* = 8.3 Hz, 2H), 7.07 (d, *J* = 8.3 Hz, 2H), 4.70 (d, *J* = 16.2 Hz, 1H), 4.48 (d, *J* = 16.3 Hz, 1H), 4.01 - 3.80 (m, 1H), 3.73 - 3.68 (m, 3H), 3.48 (s, 2H), 3.15 (d, *J* = 10.1 Hz, 4H), 3.01 - 2.87 (m, 4H), 2.84 - 2.76 (m, 4H), 2.73 - 2.63 (m, 1H), 2.59 - 2.49 (m, 2H), 2.20 (brs, 2H), 1.97 (s, 2H), 1.86 - 1.74 (m, 1H), 1.72 - 1.65 (m, 1H), 1.39 (t, *J* = 6.1 Hz, 2H), 0.89 (s, 6H). LCMS (ESI) calcd. for C₃₇H₄₆ClF₂N₆O₃⁺ [M+H]⁺: 695.33, found, 695.3.

### Example 71: Preparation of 1-(5-((4-(4-(2,3-dichlorophenyl)piperazin-1-yl)piperidin-1-yl)methyl)-1-oxoisoindolin-2-yl)dihydropyrimidine-2,4(1H,3H)-dione (GT-09355)

With reference to the method of Scheme 1, the target compound (GT-09355) was obtained as a white solid (11 mg, yield 20%). ¹H NMR (400 MHz, DMSO) δ 10.70 (s, 1H), 7.94 - 7.82 (m, 2H), 7.83 - 7.73 (m, 1H), 7.43 - 7.31 (m, 2H), 7.22 - 7.19 (m, 1H), 4.82 (d, *J* = 16.1 Hz, 1H), 4.59 (d, *J* = 16.6 Hz, 1H), 4.45 (brs, 1H), 3.79 (t, *J* = 6.8 Hz, 2H), 3.67 - 3.58 (m, 2H), 3.51 - 3.43 (m, 4H), 3.25 - 3.17 (m, 4H), 3.04 - 2.95 (m, 2H), 2.94 - 2.82 (m, 2H), 2.81 - 2.76 (m, 1H), 2.40 - 2.30 (m, 2H), 2.29 - 2.12 (m, 2H), 2.06 - 1.92 (m, 1H). LCMS (ESI) calcd. for C₂₈H₃₃Cl₂N₆O₃⁺ [M+H]⁺: 571.20, found, 571.2.

### Example 72: Preparation of 5-((4-(4-(2,3-dichlorophenyl)piperazin-1-yl)piperidin-1-yl)methyl)-2-(2,4-dioxotetrahydropyrimidin-1(2H)-yl)isoindoline-1,3-dione (GT-09357)

With reference to the method of Scheme 1, the target compound (GT-09357) was obtained as a white solid (17 mg, white solid, yield 32%). ¹H NMR (400 MHz, DMSO) δ 10.93 (s, 1H), 8.28 (s, 1H), 8.20 - 8.04 (m, 2H), 7.39 - 7.34 (m, 2H), 7.26 - 7.15 (m, 1H), 4.65 - 4.34 (m, 2H), 3.83 (t, *J* = 6.8 Hz, 2H), 3.69 - 3.65 (m, 2H), 3.24 - 3.13 (m, 6H), 3.09 - 2.96 (m, 2H), 2.86 (t, *J* = 6.8 Hz, 2H), 2.76 (t, *J* = 6.8 Hz, 1H), 2.46 - 2.29 (m, 3H), 2.26 - 2.10 (m, 2H), 2.10 - 1.92 (m, 1H). LCMS (ESI) calcd. for C₂₈H₃₁Cl₂N₆O₄⁺ [M+H]⁺: 585.18, found, 585.2.

### Example 73: Preparation of 1-(5-((4-(4-(2,3-dichlorophenyl)piperazin-1-yl)-3,3-difluoropiperidin-1-yl)methyl)-1-oxoisoindolin-2-yl)dihydropyrimidine-2,4(1H,3H)-dione (GT-09205)

With reference to the method of Scheme 1, the target compound (GT-09205) was obtained as a white solid (43 mg, yield 57%). ¹H NMR (400 MHz, DMSO) δ 10.68 (s, 1H), 7.82 (d, *J* = 7.8 Hz, 1H), 7.71 (s, 1H), 7.62 (d, *J* = 7.7 Hz, 1H), 7.40 - 7.32 (m, 2H), 7.17 (dd, *J* = 5.7, 3.8 Hz, 1H), 4.80 (d, *J* = 16.6 Hz, 1H), 4.57 (d, *J* = 16.5 Hz, 1H), 4.10 (s, 2H), 3.81 - 3.77 (m, 4H), 3.33 - 3.14 (m, 10H), 2.90 - 2.84 (m, 2H), 2.78 (t, *J* = 5.3 Hz, 1H), 2.40 - 2.25 (m, 1H), 2.20 - 2.04 (m, 1H). LCMS (ESI) calcd. for C₂₈H₃₁Cl₂F₂N₆O₃⁺ [M+H]⁺: 607.18, found, 607.2.

### Example 74: Preparation of 1-(5-((4-(4-(2,3-dichlorophenyl)-3,6-dihydropyridin-1(2H)-yl)-3,3-difluoropiperidin-1-yl)methyl)-1-oxoisoindolin-2-yl)dihydropyrimidine-2,4(1H,3H)-dione (GT-09206)

With reference to the method of Scheme 1, the target compound (GT-09206) was obtained as a white solid (31 mg, yield 41%). ¹H NMR (400 MHz, DMSO) δ 10.68 (s, 1H), 7.81 (d, *J* = 7.8 Hz, 1H), 7.70 - 7.53 (m, 3H), 7.39 (t, *J* = 7.9 Hz, 1H), 7.26 (dd, *J* = 7.6, 1.5 Hz, 1H), 5.75 (s, 1H), 4.79 (d, *J* = 16.7 Hz, 1H), 4.56 (d, *J* = 16.7 Hz, 1H), 4.12 - 3.88 (m, 4H), 3.88 - 3.77 (m, 5H), 3.56 - 3.33 (m, 3H), 3.26 - 3.11 (m, 1H), 2.95 - 2.82 (m, 2H), 2.81 - 2.70 (m, 2H), 2.40 - 2.33 (m, 1H), 2.21 - 2.05 (m, 1H). LCMS (ESI) calcd. for C₂₉H₃₀Cl₂F₂N₅O₃⁺ [M+H]⁺: 604.17, found, 604.2.

### Example 75: Preparation of 5-((4-(4-(2,3-dichlorophenyl)-3,6-dihydropyridin-1(2H)-yl)-3,3-difluoropiperidin-1-yl)methyl)-2-(2,4-dioxotetrahydropyrimidin-1(2H)-yl)isoindoline-1,3-dione (GT-09358)

With reference to the method of Scheme 1, the target compound (GT-09358) was obtained as a white solid (17 mg, yield 31%). ¹H NMR (400 MHz, DMSO) δ 10.92 (s, 1H), 8.03 - 7.99 (m, 2H), 7.95 - 7.91 (m, 1H), 7.62 (d, *J* = 8.0 Hz, 1H), 7.39 (t, *J* = 7.9 Hz, 1H), 7.27 (d, *J* = 6.6 Hz, 1H), 5.76 (s, 1H), 4.09 - 3.87 (m, 4H), 3.82 (t, *J* = 6.7 Hz, 2H), 3.29 - 3.20 (m, 2H), 3.11 - 3.04 (m, 3H), 2.89 (s, 1H), 2.85 (t, *J* = 6.8 Hz, 2H), 2.74 (s, 1H), 2.72 - 2.59 (m, 2H), 2.44 - 2.26 (m, 2H), 2.15 - 1.96 (m, 1H). LCMS (ESI) calcd. for C₂₉H₂₈Cl₂F₂N₅O₄⁺ [M+H]⁺: 618.15, found, 618.2.

### Example 76: Preparation of 1-(5-((4-(4-(6-fluorobenzo[d]isoxazol-3-yl)piperazin-1-yl)piperidin-1-yl)methyl)-1-oxoisoindolin-2-yl)dihydropyrimidine-2,4(1H,3H)-dione (GT-09356)

With reference to the method of Scheme 1, the target compound (GT-09356) was obtained as a white solid (19 mg, yield 36%). ¹H NMR (400 MHz, DMSO) δ 10.70 (s, 1H), 8.10 (dd, *J* = 8.9, 5.2 Hz, 1H), 7.88 (d, *J* = 7.8 Hz, 2H), 7.77 (d, *J* = 7.3 Hz, 1H), 7.61 (dd, *J* = 9.0, 2.0 Hz, 1H), 7.26 (dd, *J* = 10.0, 8.1 Hz, 1H), 4.82 (d, *J* = 16.5 Hz, 1H), 4.58 (d, *J* = 16.6 Hz, 1H), 4.44 (s, 1H), 4.11 (brs, 2H), 3.79 (t, *J* = 6.9 Hz, 2H), 3.66 - 3.47 (m, 6H), 3.230 - 3.22 (m, 4H), 3.09 - 2.93 (m, 2H), 2.90 - 2.83 (m, 1H), 2.80 - 2.75 (m, 1H), 2.41 - 2.28 (m, 2H), 2.27 - 2.09 (m, 2H). LCMS (ESI) calcd. for C₂₉H₃₃FN₇O₄⁺ [M+H]⁺: 562.26, found, 562.3.

### Example 77: Preparation of 2-(2,4-dioxotetrahydropyrimidin-1(2H)-yl)-5-((4-(4-(6-fluorobenzo[d]isoxazol-3-yl)piperazin-1-yl)piperidin-1-yl)methyl)isoindoline-1,3-dione (GT-09359)

With reference to the method of Scheme 1, the target compound (GT-09359) was obtained as a white solid (17 mg, yield 33%). ¹H NMR (400 MHz, DMSO) δ 10.93 (s, 1H), 8.34 - 8.20 (m, 1H), 8.11 - 8.08 (m, 2H), 7.86 - 7.80 (m, 1H), 7.64 - 7.58 (m, 1H), 7.26 (t, *J* = 8.9 Hz, 1H), 4.61 - 4.52 (m, 1H), 4.47 - 4.32 (m, 1H), 4.22 - 3.98 (m, 2H), 3.72 - 3.64 (m, 2H), 3.62 - 3.47 (m, 8H), 3.25 - 3.18 (m, 4H), 3.07 - 2.91 (m, 2H), 2.41 - 2.32 (m, 1H), 2.21 - 1.98 (m, 2H). LCMS (ESI) calcd. for C₂₉H₃₁FN₇O₅⁺ [M+H]⁺: 576.24, found, 576.3.

### Example 78: Preparation of 1-(5-((3,3-difluoro-4-(4-(6-fluorobenzo[d]isoxazol-3-yl)piperazin-1-yl)piperidin-1-yl)methyl)-1-oxoisoindolin-2-yl)dihydropyrimidine-2,4(1H,3H)-dione (GT-09207)

With reference to the method of Scheme 1, the target compound (GT-09207) was obtained as a white solid (49 mg, yield 65%). ¹H NMR (400 MHz, DMSO) δ 10.68 (s, 1H), 8.06 (dd, *J* = 8.9, 5.3 Hz, 1H), 7.83 (d, *J* = 7.7 Hz, 1H), 7.71 (brs, 1H), 7.63 (brs, 1H), 7.57 (dd, *J* = 9.1, 2.1 Hz, 1H), 7.21 (td, *J* = 9.1, 2.1 Hz, 1H), 4.79 (d, *J* = 16.2 Hz, 1H), 4.57 (d, *J* = 16.4 Hz, 1H), 4.32 - 3.97 (m, 2H), 3.81 - 3.77 (m, 4H), 3.57 - 3.44 (m, 4H), 3.40 - 2.98 (m, 6H), 2.90 - 2.84 (m, 2H), 2.81 - 2.73 (m, 1H), 2.26 - 1.92 (m, 2H). LCMS (ESI) calcd. for C₂₉H₃₁F₃N₇O₄⁺ [M+H]⁺: 598.24, found, 598.3.

### Example 79: Preparation of 5-((3,3-difluoro-4-(4-(6-fluorobenzo[d]isoxazol-3-yl)piperazin-1-yl)piperidin-1-yl)methyl)-2-(2,4-dioxotetrahydropyrimidin-1(2H)-yl)isoindoline-1,3-dione (GT-09360)

With reference to the method of Scheme 1, the target compound (GT-09360) was obtained as a white solid (38 mg, yield 69%). ¹H NMR (400 MHz, DMSO) δ 10.92 (s, 1H), 8.16 - 7.99 (m, 3H), 7.95 (s, 1H), 7.58 (dd, *J* = 9.1, 2.2 Hz, 1H), 7.22 (td, *J* = 9.1, 2.1 Hz, 1H), 4.04 (brs, 2H), 3.82 (t, *J* = 6.8 Hz, 2H), 3.74 - 3.56 (m, 6H), 3.27 - 3.17 (m, 4H), 3.17 - 2.99 (m, 3H), 2.86 (t, *J* = 6.8 Hz, 2H), 2.22 - 2.10 (m, 1H), 2.08 - 1.93 (m, 1H). LCMS (ESI) calcd. for C₂₉H₂₉F₃N₇O₅⁺ [M+H]⁺: 612.22, found, 612.2.

### Example 80: Preparation of 1-(5-((3',3'-difluoro-4-(6-fluorobenzo[d]isoxazol-3-yl)-[1,4'-bipiperidin]-1'-yl)methyl)-1-oxoisoindolin-2-yl)dihydropyrimidine-2,4(1H,3H)-dione (GT-09208)

With reference to the method of Scheme 1, the target compound (GT-09208) was obtained as a white solid (34 mg, yield 46%). ¹H NMR (400 MHz, DMSO) δ 10.68 (s, 1H), 8.23 (s, 1H), 7.79 (d, *J* = 7.8 Hz, 1H), 7.73 (d, *J* = 9.0 Hz, 1H), 7.63 (s, 1H), 7.54 (s, 1H), 7.34 (t, *J* = 9.0 Hz, 1H), 4.78 (d, *J* = 16.7 Hz, 1H), 4.55 (d, *J* = 16.7 Hz, 1H), 4.20 - 3.98 (m, 1H), 3.89 (s, 2H), 3.79 (t, *J* = 7.4 Hz, 3H), 3.75 - 3.65 (m, 2H), 3.35 - 3.21 (m, 3H), 3.19 - 3.00 (m, 2H), 2.97 - 2.82 (m, 2H), 2.82 - 2.70 (m, 2H), 2.47 - 2.37 (m, 3H), 2.27 - 2.18 (m, 2H). LCMS (ESI) calcd. for C₃₀H₃₂F₃N₆O₄⁺ [M+H]⁺: 597.24, found, 597.3.

### Example 81: Preparation of 5-((3',3'-difluoro-4-(6-fluorobenzo[d]isoxazol-3-yl)-[1,4'-bipiperidin]-1'-yl)methyl)-2-(2,4-dioxotetrahydropyrimidin-1(2H)-yl)isoindoline-1,3-dione (GT-09361)

With reference to the method of Scheme 1, the target compound (GT-09361) was obtained as a white solid (22 mg, yield 40%). ¹H NMR (400 MHz, DMSO) δ 10.91 (s, 1H), 8.24 (s, 1H), 8.01 (d, *J* = 7.7 Hz, 1H), 7.97 (s, 1H), 7.91 (d, *J* = 7.7 Hz, 1H), 7.73 (dd, *J* = 9.1, 2.1 Hz, 1H), 7.34 (td, *J* = 9.1, 2.1 Hz, 1H), 4.18 - 3.99 (m, 1H), 3.92 (s, 2H), 3.83 (t, *J* = 6.7 Hz, 2H), 3.78 - 3.65 (m, 2H), 3.61 - 3.54 (m, 4H), 3.33 - 3.20 (m, 2H), 3.10 - 3.02 (m, 1H), 2.85 (t, *J* = 6.8 Hz, 2H), 2.48 - 2.42 (m, 2H), 2.39 - 2.33 (m, 1H), 2.23 - 2.19(m, 2H), 2.12 - 2.05 (m, 1H). LCMS (ESI) calcd. for C₃₀H₃₀F₃N₆O₅⁺ [M+H]⁺: 611.22, found, 611.2.

### Example 82: Preparation of 3-(6-((4-(4-benzhydrylpiperazin-1-yl)-3,3-difluoropiperidin-1-yl)methyl)-1-oxoisoindolin-2-yl)piperidine-2,6-dione (GT-09188)

With reference to the method of Scheme 1, the target compound (GT-09188) was obtained as a white solid (27 mg, yield 46 %). ¹H NMR (400 MHz, DMSO) δ 11.95 (s, 1H), 11.01 (s, 1H), 7.96 - 7.83 (m, 5H), 7.79 (d, *J* = 7.8 Hz, 1H), 7.70 (d, *J* = 7.8 Hz, 1H), 7.43 (t, *J* = 7.5 Hz, 4H), 7.35 (t, *J* = 7.3 Hz, 2H), 5.60 (s, 1H), 5.13 (dd, *J* = 13.2, 5.0 Hz, 1H), 4.51 (d, *J* = 17.7 Hz, 1H), 4.38 (d, *J* = 17.4 Hz, 2H), 3.59 (s, 2H), 3.32 - 3.24 (m, 5H), 3.11 (s, 2H), 3.05 - 2.86 (m, 6H), 2.61 (d, *J* = 17.4 Hz, 1H), 2.46 - 2.37 (m, 1H), 2.08 - 1.89 (m, 3H). LCMS (ESI) calcd. for C₃₆H₄₀F₂N₅O₃ ⁺ [M+H]⁺: 628.31, found, 628.3.

### Example 83: Preparation of 3-(7-((4-(4-benzhydrylpiperazin-1-yl)-3,3-difluoropiperidin-1-yl)methyl)-1-oxoisoindolin-2-yl)piperidine-2,6-dione (GT-09189)

With reference to the method of Scheme 1, the target compound (GT-09189) was obtained as a white solid (20 mg, yield 34 %). ¹H NMR (400 MHz, DMSO) δ 12.01 (s, 1H), 11.04 (s, 1H), 7.91 (d, *J* = 7.3 Hz, 4H), 7.72 (s, 3H), 7.43 (t, *J* = 7.6 Hz, 4H), 7.35 (t, *J* = 7.3 Hz, 2H), 5.60 (s, 1H), 5.18 - 5.07 (m, 1H), 4.88 (d, *J* = 37.2 Hz, 1H), 4.78 - 4.61 (m, 1H), 4.53 (d, *J* = 18.0 Hz, 1H), 4.48 - 4.36 (m, 1H), 3.72 (d, *J* = 30.8 Hz, 2H), 3.35 - 3.25 (m, 4H), 3.09 (d, *J* = 17.8 Hz, 3H), 3.07 - 2.91 (m, 5H), 2.62 (d, *J* = 16.9 Hz, 1H), 2.46 - 2.36 (m, 1H), 2.09 - 1.90 (m, 3H). LCMS (ESI) calcd. for C₃₆H₄₀F₂N₅O₃ ⁺ [M+H]⁺: 628.31, found, 628.3.

### Example 84: Preparation of 3-(5-(2-(4-(4-benzhydrylpiperazin-1-yl)-3,3-difluoropiperidin-1-yl)ethyl)-1-oxoisoindolin-2-yl)piperidine-2,6-dione (GT-09190)

With reference to the method of Scheme 1, the target compound (GT-09190) was obtained as a white solid (10 mg, yield 17 %). ¹H NMR (400 MHz, DMSO) δ 11.91 (s, 1H), 10.99 (s, 1H), 7.90 (d, *J* = 6.9 Hz, 4H), 7.71 (d, *J* = 7.8 Hz, 1H), 7.52 (s, 1H), 7.44 (t, *J* = 7.6 Hz, 5H), 7.36 (t, 2H), 5.61 (s, 1H), 5.14 - 5.04 (m, 1H), 4.45 (d, *J* = 17.6 Hz, 1H), 4.32 (d, *J* = 17.5 Hz, 1H), 3.96 (s, 1H), 3.58 (s, 2H), 3.34 - 3.32 (m, 2H), 3.23 - 3.12 (m, 5H), 3.02 (d, *J* = 10.8 Hz, 4H), 2.96 - 2.80 (m, 2H), 2.70 - 2.54 (m, 2H), 2.46 - 2.32 (m, 2H), 2.11 - 1.98 (m, 3H). LCMS (ESI) calcd. for C₃₇H₄₂F₂N₅O₃ ⁺ [M+H]⁺: 642.33, found, 642.4.

### Example 85: Preparation of 3-(5-(3-(4-(4-benzhydrylpiperazin-1-yl)-3,3-difluoropiperidin-1-yl)propyl)-1-oxoisoindolin-2-yl)piperidine-2,6-dione (GT-09191)

With reference to the method of Scheme 1, the target compound (GT-09191) was obtained as a white solid (12 mg, yield 15 %). ¹H NMR (400 MHz, DMSO) δ 11.90 (s, 1H), 10.99 (s, 1H), 10.75 (s, 1H), 7.90 (d, *J* = 7.2 Hz, 4H), 7.68 (d, *J* = 7.8 Hz, 1H), 7.51 - 7.34 (m, 8H), 5.60 (s, 1H), 5.16 - 4.99 (m, 1H), 4.43 (d, *J* = 17.3 Hz, 1H), 4.30 (d, *J* = 17.3 Hz, 1H), 3.91 (s, 1H), 3.30 (s, 4H), 3.16 - 2.89 (m, 11H), 2.75 (t, *J* = 7.4 Hz, 2H), 2.60 (d, *J* = 17.6 Hz, 1H), 2.47 - 2.31 (m, 2H), 2.15 - 1.94 (m, 5H). LCMS (ESI) calcd. for C₃₈H₄₄F₂N₅O₃ ⁺ [M+H]⁺: 656.34, found, 656.4.

### Example 86: Preparation of 5-((4-(4-benzhydrylpiperazin-1-yl)-3,3-difluoropiperidin-1-yl)methyl)-2-(2,6-dioxopiperidin-3-yl)isoindoline-1,3-dione (GT-09192)

With reference to the method of Scheme 1, the target compound (GT-09192) was obtained as a white solid (21 mg, yield 35 %). ¹H NMR (400 MHz, DMSO) δ 11.47 (s, 1H), 11.07 (s, 1H), 7.89 (d, *J* = 7.8 Hz, 2H), 7.86 - 7.66 (m, 5H), 7.37 (t, *J* = 7.4 Hz, 4H), 7.29 (t, *J* = 7.3 Hz, 2H), 5.51 (s, 1H), 5.09 (dd, *J* = 12.8, 5.5 Hz, 1H), 4.01 (s, 2H), 3.16 (s, 3H), 2.99 (d, *J* = 31.9 Hz, 8H), 2.88 - 2.76 (m, 2H), 2.61 - 2.47 (m, 3H), 2.04 - 1.95 (m, 1H), 1.81 (s, 2H). LCMS (ESI) calcd. for C₃₆H₃₈F₂N₅O₄ ⁺ [M+H]⁺: 642.29, found, 642.3.

### Example 87: Preparation of 4-((4-(4-benzhydrylpiperazin-1-yl)-3,3-difluoropiperidin-1-yl)methyl)-2-(2,6-dioxopiperidin-3-yl)isoindoline-1,3-dione (GT-09193)

With reference to the method of Scheme 1, the target compound (GT-09193) was obtained as a white solid (26 mg, yield 43 %). ¹H NMR (400 MHz, DMSO) δ 11.58 (s, 1H), 11.07 (s, 1H), 7.91 (s, 1H), 7.86 - 7.82 (m, 2H), 7.78 (d, *J* = 5.9 Hz, 4H), 7.37 (t, *J* = 7.5 Hz, 4H), 7.29 (t, *J* = 7.3 Hz, 2H), 5.51 (s, 1H), 5.08 (dd, *J* = 12.8, 5.4 Hz, 1H), 4.25 (s, 2H), 3.18 (s, 4H), 3.09 - 2.89 (m, 8H), 2.88 - 2.78 (m, 2H), 2.62 - 2.51 (m, 2H), 2.02 - 1.92 (m, 1H), 1.90 - 1.73 (m, 2H). LCMS (ESI) calcd. for C₃₆H₃₈F₂N₅O₄ ⁺ [M+H]⁺: 642.29, found, 642.3.

### Example 88: Preparation of 3-(7-((4-(4-benzhydrylpiperazin-1-yl)piperidin-1-yl)methyl)-1-oxoisoindolin-2-yl)piperidine-2,6-dione (GT-09194)

With reference to the method of Scheme 1, the target compound (GT-09194) was obtained as a white solid (21 mg, yield 34 %). ¹H NMR (400 MHz, DMSO) δ 11.03 (s, 1H), 10.55 (s, 1H), 7.91 - 7.61 (m, 7H), 7.39 (t, *J* = 7.4 Hz, 4H), 7.29 (t, *J* = 12.7 Hz, 2H), 5.12 (dd, *J* = 13.2, 5.1 Hz, 1H), 4.85 (d, *J* = 11.6 Hz, 1H), 4.71 (d, *J* = 12.6 Hz, 1H), 4.51 (d, *J* = 17.8 Hz, 1H), 4.39 (d, 1H), 3.60 - 3.55 (m, 6H), 3.21 - 2.98 (m, 6H), 2.95 - 2.87 (m, 1H), 2.70 - 2.56 (m, 2H), 2.49 - 2.36 (m, 2H), 2.29 (d, *J* = 11.1 Hz, 2H), 2.18 - 1.97 (m, 3H). LCMS (ESI) calcd. for C₃₆H₄₂N₅O₃ ⁺ [M+H]⁺: 592.33, found, 592.4.

### Example 89: Preparation of 3-(5-(2-(4-(4-benzhydrylpiperazin-1-yl)piperidin-1-yl)ethyl)-1-oxoisoindolin-2-yl)piperidine-2,6-dione (GT-09195)

With reference to the method of Scheme 1, the target compound (GT-09195) was obtained as a white solid (20 mg, yield 32 %). ¹H NMR (400 MHz, DMSO) δ 10.92 (s, 1H), 10.75 (s, 1H), 7.65 (d, *J* = 7.8 Hz, 1H), 7.50 (d, *J* = 22.2 Hz, 4H), 7.46 (s, 1H), 7.37 (d, *J* = 8.1 Hz, 1H), 7.29 (t, *J* = 7.4 Hz, 5H), 7.20 (t, *J* = 6.9 Hz, 2H), 5.07 - 4.98 (m, 1H), 4.38 (d, *J* = 17.5 Hz, 1H), 4.25 (d, *J* = 17.5 Hz, 1H), 3.65 (d, *J* = 11.0 Hz, 2H), 3.26 - 3.18 (m, 6H), 3.16 - 3.11 (m, 3H), 3.00 - 2.88 (m, 4H), 2.86 - 2.78 (m, 2H), 2.52 (d, 2H), 2.39 - 2.23 (m, 4H), 2.12 - 2.00 (m, 2H), 1.96 - 1.90 (m, 1H). LCMS (ESI) calcd. for C₃₇H₄₄N₅O₃ ⁺ [M+H]⁺: 606.34, found, 606.4.

### Example 90: Preparation of 3-(5-(3-(4-(4-benzhydrylpiperazin-1-yl)piperidin-1-yl)propyl)-1-oxoisoindolin-2-yl)piperidine-2,6-dione (GT-09196)

With reference to the method of Scheme 1, the target compound (GT-09196) was obtained as a yellow solid (16 mg, yield 19 %). ¹H NMR (400 MHz, DMSO) δ 10.99 (s, 1H), 10.56 (s, 1H), 7.68 (d, *J* = 7.7 Hz, 5H), 7.49 (s, 1H), 7.44 - 7.33 (m, 6H), 7.30 (t, *J* = 7.2 Hz, 2H), 5.11 (dd, *J* = 13.2, 5.0 Hz, 1H), 4.44 (d, *J* = 17.3 Hz, 1H), 4.31 (d, *J* = 17.4 Hz, 1H), 3.63 (d, *J* = 11.8 Hz, 2H), 3.28 - 3.20 (m, 3H), 3.09 - 2.99 (m, 4H), 2.97 - 2.87 (m, 4H), 2.77 (t, *J* = 7.2 Hz, 2H), 2.61 (dd, *J* = 34.8, 17.6 Hz, 2H), 2.46 - 2.36 (m, 2H), 2.35 - 2.21 (m, 3H), 2.19 - 1.94 (m, 6H). LCMS (ESI) calcd. for C₃₈H₄₆N₅O₃ ⁺ [M+H]⁺: 620.36, found, 620.4.

### Example 91: Preparation of 5-((4-(4-benzhydrylpiperazin-1-yl)piperidin-1-yl)methyl)-2-(2,6-dioxopiperidin-3-yl)isoindoline-1,3-dione (GT-09334)

With reference to the method of Scheme 1, the target compound (GT-09334) was obtained as a white solid (19 mg, yield 31 %). ¹H NMR (400 MHz, DMSO) δ 11.08 (s, 2H), 8.14 (s, 1H), 8.02 (d, *J* = 7.6 Hz, 1H), 7.96 (d, *J* = 7.6 Hz, 1H), 7.56 (s, 4H), 7.30 (t, *J* = 7.3 Hz, 4H), 7.23 - 7.17 (m, 2H), 5.12 (dd, *J* = 12.7, 5.4 Hz, 1H), 4.44 (s, 2H), 3.44 (d, 4H), 3.43 - 3.41 (m, 3H), 3.30 - 3.29 (m, 2H), 3.00 - 2.88 (m, 4H), 2.86 - 2.77 (m, 2H), 2.58 - 2.47 (m, 2H), 2.26 - 2.18 (m, 2H), 2.09 - 1.96 (m, 3H). LCMS (ESI) calcd. for C₃₆H₄₀N₅O₄ ⁺ [M+H]⁺: 606.31, found, 606.3.

### Example 92: Preparation of 4-((4-(4-benzhydrylpiperazin-1-yl)piperidin-1-yl)methyl)-2-(2,6-dioxopiperidin-3-yl)isoindoline-1,3-dione (GT-09335)

With reference to the method of Scheme 1, the target compound (GT-09335) was obtained as a white solid (23 mg, yield 37 %). ¹H NMR (400 MHz, DMSO) δ 11.09 (s, 1H), 10.83 (s, 1H), 8.10 (d, *J* = 7.6 Hz, 1H), 7.96 (d, *J* = 6.8 Hz, 1H), 7.89 (t, *J* = 7.6 Hz, 1H), 7.51 (s, 4H), 7.29 (t, *J* = 7.3 Hz, 4H), 7.20 (t, *J* = 7.0 Hz, 2H), 5.11 (dd, *J* = 12.7, 5.4 Hz, 1H), 4.63 (s, 2H), 3.54 (s, 3H), 3.28 - 3.22 (m, 5H), 3.09 - 2.99 (m, 3H), 2.97 - 2.76 (m, 4H), 2.61 - 2.49 (m, 2H), 2.24 - 2.16 (m, 2H), 2.10 - 1.96 (m, 3H). LCMS (ESI) calcd. for C₃₆H₄₀N₅O₄ ⁺ [M+H]⁺: 606.31, found, 606.3.

### Example 93: Preparation of 3-(5-((3,3-difluoro-4-(4-(phenyl(pyridin-2-yl)methyl)piperazin-1-yl)piperidin-1-yl)methyl)-4-fluoro-1-oxoisoindolin-2-yl)piperidine-2,6-dione (GT-09336)

With reference to the method of Scheme 1, the target compound (GT-09336) was obtained as a white solid (27 mg, yield 45 %). ¹H NMR (400 MHz, DMSO) δ 11.03 (s, 1H), 8.74 (d, *J* = 4.7 Hz, 1H), 7.92 (t, 1H), 7.80 (s, 1H), 7.70 - 7.61 (m, 4H), 7.49 - 7.38 (m, 4H), 5.85 (s, 1H), 5.14 (dd, *J* = 13.3, 5.1 Hz, 1H), 4.59 (d, *J* = 15.7 Hz, 1H), 4.43 (d, *J* = 17.6 Hz, 1H), 4.27 (s, 2H), 3.36 - 3.28 (m, 3H), 3.16 - 3.00 (m, 9H), 2.94 - 2.82 (m, 2H), 2.61 (d, *J* = 16.5 Hz, 1H), 2.46 - 2.40 (m, 1H), 2.06 - 1.91 (m, 3H). LCMS (ESI) calcd. for C₃₅H₃₈F₃N₆O₃ ⁺ [M+H]⁺: 647.30, found, 647.3.

### Example 94: Preparation of 3-(5-((3,3-difluoro-4-(4-(phenyl(pyridin-2-yl)methyl)piperazin-1-yl)piperidin-1-yl)methyl)-6-fluoro-1-oxoisoindolin-2-yl)piperidine-2,6-dione (GT-09337)

With reference to the method of Scheme 1, the target compound (GT-09337) was obtained as a white solid (16 mg, yield 27 %). ¹H NMR (400 MHz, DMSO) δ 11.02 (s, 1H), 8.74 (d, *J* = 4.8 Hz, 1H), 7.99 - 7.83 (m, 2H), 7.72 - 7.60 (m, 4H), 7.50 - 7.36 (m, 4H), 5.86 (s, 1H), 5.13 (dd, *J* = 13.3, 5.1 Hz, 1H), 4.49 (d, *J* = 17.4, 3.8 Hz, 1H), 4.36 (d, *J* = 17.5, 4.7 Hz, 1H), 4.28 (s, 2H), 3.33 - 3.23 (m, 4H), 3.13 - 3.03 (m, 7H), 2.98 - 2.81 (m, 3H), 2.61 (d, *J* = 17.4 Hz, 1H), 2.45 - 2.39 (m, 1H), 2.07 - 1.92 (m, 3H). LCMS (ESI) calcd. for C₃₅H₃₈F₃N₆O₃ ⁺ [M+H]⁺: 647.30, found, 647.3.

### Example 95: Preparation of 3-(5-((3,3-difluoro-4-(4-(phenyl(pyridin-2-yl)methyl)piperazin-1-yl)piperidin-1-yl)methyl)-7-fluoro-1-oxoisoindolin-2-yl)piperidine-2,6-dione (GT-09338)

With reference to the method of Scheme 1, the target compound (GT-09338) was obtained as a white solid (20 mg, yield 33 %). ¹H NMR (400 MHz, DMSO) δ 11.02 (s, 1H), 8.74 (d, *J* = 4.7 Hz, 1H), 7.91 (t, *J* = 7.7, 3.9 Hz, 1H), 7.68 (d, *J* = 7.0 Hz, 2H), 7.64 (d, *J* = 7.8 Hz, 1H), 7.59 (s, 1H), 7.54 (d, *J* = 9.5 Hz, 1H), 7.50 - 7.39 (m, 4H), 5.86 (s, 1H), 5.10 (dd, *J* = 13.2, 5.0 Hz, 1H), 4.52 (d, *J* = 17.5 Hz, 1H), 4.39 (d, *J* = 18.1 Hz, 1H), 4.29 (s, 2H), 3.39 - 3.34 (m, 3H), 3.15 - 3.10 (m, 2H), 3.10 - 2.98 (m, 6H), 2.97 - 2.87 (m, 2H), 2.61 (d, *J* = 17.3 Hz, 1H), 2.47 - 2.30 (m, 2H), 2.14 - 2.04 (m, 1H), 2.03 - 1.94 (m, 2H). LCMS (ESI) calcd. for C₃₅H₃₈F₃N₆O₃ ⁺ [M+H]⁺: 647.30, found, 647.3.

### Example 96: Preparation of 3-(4-((3,3-difluoro-4-(4-(phenyl(pyridin-2-yl)methyl)piperazin-1-yl)piperidin-1-yl)methyl)-1-oxoisoindolin-2-yl)piperidine-2,6-dione (GT-09339)

With reference to the method of Scheme 1, the target compound (GT-09339) was obtained as a yellow solid (19 mg, yield 32 %). ¹H NMR (400 MHz, DMSO) δ 11.03 (s, 1H), 8.75 (d, *J* = 4.4 Hz, 1H), 7.91 (t, *J* = 7.7, 1.6 Hz, 1H), 7.76 (d, *J* = 7.2 Hz, 2H), 7.66 (d, *J* = 6.8 Hz, 2H), 7.59 (d, *J* = 7.9 Hz, 2H), 7.49 - 7.37 (m, 5H), 5.82 (s, 1H), 5.20 - 5.13 (m, 1H), 4.63 - 4.57 (m, 1H), 4.40 (d, 1H), 4.08 (s, 2H), 3.14 - 3.01 (m, 10H), 2.96 - 2.88 (m, 2H), 2.65 - 2.60 (m, 1H), 2.41 - 2.28 (m, 2H), 1.98 (d, *J* = 35.9 Hz, 4H). LCMS (ESI) calcd. for C₃₅H₃₉F₂N₆O₃ ⁺ [M+H]⁺: 629.30, found, 629.3.

### Example 97: Preparation of 3-(6-((3,3-difluoro-4-(4-(phenyl(pyridin-2-yl)methyl)piperazin-1-yl)piperidin-1-yl)methyl)-1-oxoisoindolin-2-yl)piperidine-2,6-dione (GT-09340)

With reference to the method of Scheme 1, the target compound (GT-09340) was obtained as a white solid (12 mg, yield 21 %). ¹H NMR (400 MHz, DMSO) δ 11.01 (s, 1H), 8.74 (d, *J* = 4.5 Hz, 1H), 7.96 (s, 1H), 7.91 (t, *J* = 7.6 Hz, 1H), 7.83 (d, *J* = 6.9 Hz, 1H), 7.78 - 7.65 (m, 4H), 7.63 (d, *J* = 7.9 Hz, 1H), 7.53 - 7.36 (m, 5H), 5.86 (s, 1H), 5.13 (dd, *J* = 13.2, 5.0 Hz, 1H), 4.51 (d, *J* = 17.7 Hz, 1H), 4.41 (s, 1H), 4.36 (d, 1H), 3.34 - 3.30 (m, 2H), 3.14 (s, 3H), 3.06 - 3.00 (m, 6H), 2.96 - 2.86 (m, 2H), 2.61 (d, *J* = 17.0 Hz, 2H), 2.45 - 2.32 (m, 2H), 2.08 - 1.95 (m, 3H). LCMS (ESI) calcd. for C₃₅H₃₉F₂N₆O₃ ⁺ [M+H]⁺: 629.30, found, 629.4.

### Example 98: 3-(7-((3,3-difluoro-4-(4-(phenyl(pyridin-2-yl)methyl)piperazin-1-yl)piperidin-1-yl)methyl)-1-oxoisoindolin-2-yl)piperidine-2,6-dione (GT-09341) Preparation of

With reference to the method of Scheme 1, the target compound (GT-09341) was obtained as a yellow solid (13 mg, yield 22 %). ¹H NMR (400 MHz, DMSO) δ 11.04 (s, 1H), 8.74 (d, *J* = 4.9 Hz, 1H), 7.91 (t, *J* = 7.7, 1.5 Hz, 1H), 7.74 (s, 3H), 7.69 (d, *J* = 7.2 Hz, 2H), 7.64 (d, *J* = 7.8 Hz, 1H), 7.49 - 7.39 (m, 4H), 5.87 (s, 1H), 5.16 - 5.09 (m, 1H), 4.98 - 4.81 (m, 1H), 4.77 - 4.64 (m, 1H), 4.53 (d, *J* = 17.8 Hz, 1H), 3.75 (s, 2H), 3.42 - 3.40 (m, 2H), 3.19 - 2.98 (m, 10H), 2.89 (t, *J* = 11.7 Hz, 1H), 2.62 (d, *J* = 17.2 Hz, 1H), 2.45 - 2.39 (m, 1H), 2.03 (s, 3H). LCMS (ESI) calcd. for C₃₅H₃₉F₂N₆O₃ ⁺ [M+H]⁺: 629.30, found, 629.3.

### Example 99: Preparation of 3-(5-(2-(3,3-difluoro-4-(4-(phenyl(pyridin-2-yl)methyl)piperazin-1-yl)piperidin-1-yl)ethyl)-1-oxoisoindolin-2-yl)piperidine-2,6-dione (GT-09342)

With reference to the method of Scheme 1, the target compound (GT-09342) was obtained as a yellow solid (18 mg, yield 23 %). ¹H NMR (400 MHz, DMSO) δ 10.99 (s, 1H), 8.75 (d, *J* = 4.6 Hz, 1H), 7.92 (t, *J* = 7.8 Hz, 1H), 7.75 - 7.64 (m, 4H), 7.53 (s, 1H), 7.50 - 7.36 (m, 5H), 5.88 (s, 1H), 5.10 (dd, *J* = 13.3, 5.0 Hz, 1H), 4.45 (d, *J* = 17.5 Hz, 1H), 4.32 (d, *J* = 17.5 Hz, 1H), 4.00 (s, 2H), 3.43 - 3.35 (m, 4H), 3.27 - 3.15 (m, 5H), 3.10 - 3.04 (m, 4H), 2.95 - 2.88 (m, 1H), 2.60 (d, *J* = 16.4 Hz, 2H), 2.39 (dd, *J* = 17.5, 8.8 Hz, 2H), 2.17 - 1.97 (m, 3H). LCMS (ESI) calcd. for C₃₆H₄₁F₂N₆O₃ ⁺ [M+H]⁺: 643.32, found, 643.3.

### Example 100: Preparation of 3-(5-(3-(3,3-difluoro-4-(4-(phenyl(pyridin-2-yl)methyl)piperazin-1-yl)piperidin-1-yl)propyl)-1-oxoisoindolin-2-yl)piperidine-2,6-dione (GT-09343)

With reference to the method of Scheme 1, the target compound (GT-09343) was obtained as a yellow solid (18 mg, yield 23 %). ¹H NMR (400 MHz, DMSO) δ 11.14 (s, 1H), 10.99 (s, 1H), 8.74 (d, *J* = 4.7 Hz, 1H), 7.91 (t, *J* = 7.6 Hz, 1H), 7.75 - 7.61 (m, 4H), 7.52 - 7.35 (m, 6H), 5.88 (s, 1H), 5.11 (dd, *J* = 13.3, 5.0 Hz, 1H), 4.43 (d, *J* = 17.2 Hz, 1H), 4.30 (d, *J* = 17.4 Hz, 1H), 3.91 (s, 1H), 3.37 - 3.26 (m, 4H), 3.15 - 3.00 (m, 10H), 2.96 - 2.87 (m, 1H), 2.75 (t, *J* = 7.2 Hz, 2H), 2.60 (d, *J* = 16.6 Hz, 1H), 2.44 - 2.38 (m, 1H), 2.16 - 1.94 (m, 5H). LCMS (ESI) calcd. for C₃₇H₄₃F₂N₆O₃ ⁺ [M+H]⁺: 657.34, found, 657.4.

### Example 101: Preparation of 5-((3,3-difluoro-4-(4-(phenyl(pyridin-2-yl)methyl)piperazin-1-yl)piperidin-1-yl)methyl)-2-(2,6-dioxopiperidin-3-yl)isoindoline-1,3-dione (GT-09344)

With reference to the method of Scheme 1, the target compound (GT-09344) was obtained as a white solid (14 mg, yield 24 %). ¹H NMR (400 MHz, DMSO) δ 11.14 (s, 1H), 8.74 (d, *J* = 4.1 Hz, 1H), 8.11 (s, 1H), 8.05 - 7.97 (m, 2H), 7.95 - 7.88 (m, 1H), 7.72 - 7.62 (m, 3H), 7.50 - 7.36 (m, 4H), 5.86 (s, 1H), 5.18 (dd, *J* = 12.7, 5.4 Hz, 1H), 4.32 (s, 2H), 3.34 - 3.25 (m, 2H), 3.22 - 3.00 (m, 10H), 2.95 - 2.82 (m, 2H), 2.65 - 2.53 (m, 2H), 2.15 - 2.01 (m, 2H), 1.98 (s, 1H). LCMS (ESI) calcd. for C₃₅H₃₇F₂N₆O₄ ⁺ [M+H]⁺: 643.28, found, 643.3.

### Example 102: Preparation of 4-((3,3-difluoro-4-(4-(phenyl(pyridin-2-yl)methyl)piperazin-1-yl)piperidin-1-yl)methyl)-2-(2,6-dioxopiperidin-3-yl)isoindoline-1,3-dione (GT-09345)

With reference to the method of Scheme 1, the target compound (GT-09345) was obtained as a white solid (w23 mg, yield 38 %). ¹H NMR (400 MHz, DMSO) δ 11.14 (s, 1H), 8.74 (d, *J* = 4.7 Hz, 1H), 8.05 (s, 1H), 7.98 - 7.86 (m, 3H), 7.73 - 7.57 (m, 3H), 7.50 - 7.33 (m, 4H), 5.84 (s, 1H), 5.16 (dd, *J* = 12.7, 5.4 Hz, 1H), 4.42 (s, 2H), 3.24 (s, 2H), 3.20 - 2.98 (m, 10H), 2.97 - 2.82 (m, 2H), 2.69 - 2.55 (m, 2H), 2.09 - 1.96 (m, 2H), 1.94 (s, 1H). LCMS (ESI) calcd. for C₃₅H₃₇F₂N₆O₄ ⁺ [M+H]⁺: 643.28, found, 643.3.

### Example 103: Preparation of 3-(5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-6-fluoro-1-oxoisoindolin-2-yl)piperidine-2,6-dione (GT-09346)

With reference to the method of Scheme 1, the target compound (GT-09346) was obtained as a white solid (26 mg, yield 50 %). ¹H NMR (400 MHz, DMSO) δ 11.02 (s, 1H), 7.73 (s, 1H), 7.58 (d, *J* = 8.6 Hz, 1H), 7.38 (d, *J* = 8.5 Hz, 2H), 7.24 (d, *J* = 8.5 Hz, 2H), 5.13 (dd, *J* = 13.3, 5.0 Hz, 1H), 4.45 (s, 1H), 4.35 (d, *J* = 17.4 Hz, 1H), 3.99 (s, 2H), 3.27 - 3.22 (m, 2H), 3.14 - 3.02 (m, 3H), 2.95 - 2.89 (m, 1H), 2.61 (d, *J* = 16.6 Hz, 2H), 2.44 - 2.32 (m, 4H), 2.12 - 1.97 (m, 2H), 1.96 - 1.65 (m, 4H), 1.48 - 1.37 (m, 2H), 0.98 (d, *J* = 14.0 Hz, 6H). LCMS (ESI) calcd. for C₃₈H₄₄ClF₃N₅O₄ ⁺ [M+H]⁺: 726.30, found, 726.3.

### Example 104: Preparation of 3-(5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-7-fluoro-1-oxoisoindolin-2-yl)piperidine-2,6-dione (GT-09347)

With reference to the method of Scheme 1, the target compound (GT-09347) was obtained as a white solid (22 mg, yield 42 %). ¹H NMR (400 MHz, DMSO) δ 11.01 (s, 1H), 7.47 (s, 1H), 7.38 (d, *J* = 8.5 Hz, 3H), 7.25 (d, *J* = 8.5 Hz, 2H), 5.09 (dd, *J* = 13.2, 5.1 Hz, 1H), 4.50 (d, *J* = 17.6 Hz, 1H), 4.37 (d, *J* = 17.9 Hz, 1H), 4.03 (s, 2H), 3.26 - 2.99 (m, 7H), 2.97 - 2.86 (m, 2H), 2.84 - 2.67 (m, 2H), 2.61 (d, *J* = 17.0 Hz, 2H), 2.46 - 2.35 (m, 4H), 2.14 - 1.77 (m, 6H), 1.47 - 1.37 (m, 2H), 0.98 (d, *J* = 13.9 Hz, 6H). LCMS (ESI) calcd. for C₃₈H₄₄ClF₃N₅O₄ ⁺ [M+H]⁺: 726.30, found, 726.3.

### Example 105: Preparation of 3-(4-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-oxoisoindolin-2-yl)piperidine-2,6-dione (GT-09348)

With reference to the method of Scheme 1, the target compound (GT-09348) was obtained as a white solid (14 mg, yield 28 %). ¹H NMR (400 MHz, DMSO) δ 11.03 (s, 1H), 7.77 - 7.69 (m, 1H), 7.64 (s, 1H), 7.55 (t, *J* = 7.2 Hz, 1H), 7.37 (d, *J* = 8.4 Hz, 2H), 7.24 (d, *J* = 8.5 Hz, 2H), 5.15 (dd, *J* = 13.2, 5.1 Hz, 1H), 4.54 (d, *J* = 16.9 Hz, 1H), 4.39 (d, *J* = 17.3 Hz, 1H), 3.87 (s, 2H), 3.30 - 3.12 (m, 5H), 3.05 (s, 2H), 2.99 - 2.88 (m, 2H), 2.68 (s, 2H), 2.64 (d, *J* = 17.3 Hz, 2H), 2.44 - 2.32 (m, 4H), 2.13 - 2.01 (m, 2H), 1.90 (d, *J* = 17.0 Hz, 1H), 1.80 (s, 2H), 1.48 - 1.37 (m, 2H), 0.98 (d, *J* = 13.9 Hz, 6H). LCMS (ESI) calcd. for C₃₈H₄₅ClF₂N₅O₄ ⁺ [M+H]⁺: 708.31, found, 708.3.

### Example 106: Preparation of 3-(6-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-oxoisoindolin-2-yl)piperidine-2,6-dione (GT-09349)

With reference to the method of Scheme 1, the target compound (GT-09349) was obtained as a white solid (white solid, 24 mg, yield 47 %). ¹H NMR (400 MHz, DMSO) δ 11.01 (s, 1H), 7.89 (s, 1H), 7.73 (s, 1H), 7.68 (d, *J* = 7.7 Hz, 1H), 7.37 (d, *J* = 8.5 Hz, 2H), 7.24 (d, *J* = 8.5 Hz, 2H), 5.13 (dd, *J* = 13.3, 5.1 Hz, 1H), 4.50 (d, *J* = 17.6 Hz, 1H), 4.37 (d, *J* = 17.6 Hz, 1H), 4.27 (s, 2H), 3.25 - 3.08 (m, 6H), 3.01 (s, 2H), 2.96 - 2.87 (m, 2H), 2.61 (d, *J* = 16.8 Hz, 2H), 2.45 - 2.34 (m, 4H), 2.31 - 2.22 (m, 1H), 2.13 - 1.96 (m, 3H), 1.95 - 1.80 (m, 3H), 1.46 - 1.36 (m, 2H), 0.98 (d, *J* = 13.1 Hz, 6H). LCMS (ESI) calcd. for C₃₈H₄₅ClF₂N₅O₄ ⁺ [M+H]⁺: 708.31, found, 708.3.

### Example 107: Preparation of 3-(7-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-oxoisoindolin-2-yl)piperidine-2,6-dione (GT-09350)

With reference to the method of Scheme 1, the target compound (GT-09350) was obtained as a white solid (22 mg, yield 43 %). ¹H NMR (400 MHz, DMSO) δ 11.04 (s, 1H), 7.70 (s, 3H), 7.37 (d, *J* = 8.5 Hz, 2H), 7.24 (d, *J* = 8.3 Hz, 2H), 5.21 - 5.04 (m, 1H), 4.77 (s, 1H), 4.55 (s, 1H), 4.52 (d, *J* = 17.9 Hz, 1H), 4.45 - 4.36 (m, 1H), 3.37 - 3.27 (m, 3H), 3.18 (s, 3H), 3.07 - 2.86 (m, 4H), 2.62 (d, *J* = 17.4 Hz, 3H), 2.45 - 2.32 (m, 4H), 2.19 - 1.99 (m, 3H), 1.90 (d, *J* = 17.3 Hz, 3H), 1.49 - 1.36 (m, 2H), 0.98 (d, *J* = 13.5 Hz, 6H). LCMS (ESI) calcd. for C₃₈H₄₅ClF₂N₅O₄ ⁺ [M+H]⁺: 708.31, found, 708.3.

### Example 108: Preparation of 3-(5-(2-(4-(4-(4'-chloro-5,5-dimethyl-3,4,5,6-tetrahydro-[1,1'-biphenyl]-2-carbonyl)piperazin-1-yl)-3,3-difluoropiperidin-1-yl)ethyl)-1-oxoisoindolin-2-yl)piperidine-2,6-dione (GT-09351)

With reference to the method of Scheme 1, the target compound (GT-09351) was obtained as a white solid (23 mg, yield 33 %). ¹H NMR (400 MHz, DMSO) δ 10.99 (s, 1H), 7.71 (d, *J* = 7.8 Hz, 1H), 7.51 (s, 1H), 7.42 (d, *J* = 7.9 Hz, 1H), 7.38 (d, *J* = 8.5 Hz, 2H), 7.26 (d, *J* = 8.5 Hz, 2H), 5.11 (dd, *J* = 13.3, 5.0 Hz, 1H), 4.45 (d, *J* = 17.4 Hz, 1H), 4.32 (d, *J* = 17.5 Hz, 1H), 3.90 (s, 1H), 3.33 - 3.27 (m, 3H), 3.25 - 3.06 (m, 8H), 3.04 - 2.88 (m, 3H), 2.61 (d, *J* = 16.2 Hz, 2H), 2.45 - 2.36 (m, 4H), 2.26 (s, 1H), 2.13 - 1.83 (m, 6H), 1.44 (d, *J* = 7.8 Hz, 2H), 0.99 (d, *J* = 13.3 Hz, 6H). LCMS (ESI) calcd. for C₃₉H₄₇ClF₂N₅O₄ ⁺ [M+H]⁺: 722.33, found, 722.4.

### Example 109: Preparation of 3-(5-(3-(4-(4-(4'-chloro-5,5-dimethyl-3,4,5,6-tetrahydro-[1,1'-biphenyl]-2-carbonyl)piperazin-1-yl)-3,3-difluoropiperidin-1-yl)propyl)-1-oxoisoindolin-2-yl)piperidine-2,6-dione (GT-09378)

With reference to the method of Scheme 1, the target compound (GT-09378) was obtained as a white solid (14 mg, yield 20 %). ¹H NMR (400 MHz, DMSO) δ 10.99 (s, 1H), 10.65 (s, 1H), 7.71 - 7.64 (m, 1H), 7.48 (s, 1H), 7.42 - 7.33 (m, 3H), 7.25 (d, *J* = 8.5 Hz, 2H), 5.11 (dd, *J* = 13.3, 5.0 Hz, 1H), 4.43 (d, *J* = 17.3 Hz, 1H), 4.30 (d, *J* = 17.3 Hz, 1H), 3.83 (s, 1H), 3.32 - 3.23 (m, 2H), 3.18 - 2.82 (m, 9H), 2.74 (t, *J* = 7.3 Hz, 2H), 2.60 (d, *J* = 17.5 Hz, 2H), 2.46 - 2.29 (m, 5H), 2.21 (s, 1H), 2.14 - 1.97 (m, 4H), 1.93 - 1.72 (m, 3H), 1.50 - 1.35 (m, 2H), 0.98 (d, *J* = 12.4 Hz, 6H). LCMS (ESI) calcd. for C₄₀H₄₉ClF₂N₅O₄ ⁺ [M+H]⁺: 736.34, found, 736.3.

### Example 110: Preparation of 5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-2-(2,6-dioxopiperidin-3-yl)isoindoline-1,3-dione (GT-09352)

With reference to the method of Scheme 1, the target compound (GT-09352) was obtained as a white solid (23 mg, yield 44 %). ¹H NMR (400 MHz, DMSO) δ 11.14 (s, 1H), 7.94 (t, *J* = 11.4 Hz, 2H), 7.90 (s, 1H), 7.38 (d, *J* = 8.5 Hz, 2H), 7.25 (d, *J* = 8.5 Hz, 2H), 5.17 (dd, *J* = 12.7, 5.3 Hz, 1H), 4.06 (s, 2H), 3.26 - 2.96 (m, 7H), 2.95 - 2.83 (m, 2H), 2.83 - 2.67 (m, 2H), 2.67 - 2.57 (m, 3H), 2.43 - 2.33 (m, 3H), 2.11 - 2.00 (m, 2H), 1.97 - 1.58 (m, 4H), 1.52 - 1.39 (m, 2H), 0.98 (d, *J* = 13.6 Hz, 6H). LCMS (ESI) calcd. for C₃₈H₄₃ClF₂N₅O₅ ⁺ [M+H]⁺: 722.29, found, 722.3.

### Example 111: Preparation of 4-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-2-(2,6-dioxopiperidin-3-yl)isoindoline-1,3-dione (GT-09353)

With reference to the method of Scheme 1, the target compound (GT-09353) was obtained as a white solid (16 mg, yield 31 %). ¹H NMR (400 MHz, DMSO) δ 11.13 (s, 1H), 7.97 - 7.91 (m, 1H), 7.88 (d, *J* = 4.9 Hz, 2H), 7.38 (d, *J* = 8.5 Hz, 2H), 7.25 (d, *J* = 8.5 Hz, 2H), 5.15 (dd, *J* = 12.8, 5.4 Hz, 1H), 4.21 (s, 2H), 3.33 - 3.11 (m, 7H), 3.06 (s, 2H), 2.96 - 2.84 (m, 2H), 2.82 - 2.65 (m, 2H), 2.61 (d, *J* = 19.2 Hz, 2H), 2.44 - 2.35 (m, 3H), 2.12 - 2.01 (m, 2H), 1.90 (d, *J* = 16.2 Hz, 1H), 1.77 (s, 2H), 1.50 - 1.40 (m, 2H), 0.98 (d, *J* = 14.2 Hz, 6H). LCMS (ESI) calcd. for C₃₈H₄₃ClF₂N₅O₅ ⁺ [M+H]⁺: 722.29, found, 722.3.

### Example 112: Preparation of 3-(5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-4-fluoro-1-oxoisoindolin-2-yl)piperidine-2,6-dione (GT-09379)

With reference to the method of Scheme 1, the target compound (GT-09379) was obtained as a white solid (18 mg, yield 33 %). ¹H NMR (400 MHz, DMSO) δ 11.02 (s, 1H), 10.05 (s, 1H), 7.71 (s, 1H), 7.63 (d, 1H), 7.44 (d, *J* = 8.4 Hz, 2H), 7.14 (d, *J* = 8.4 Hz, 2H), 5.13 (dd, *J* = 13.2, 5.0 Hz, 1H), 4.58 (d, *J* = 17.8 Hz, 1H), 4.42 (d, *J* = 17.4 Hz, 1H), 4.10 (s, 2H), 3.58 - 3.52 (m, 5H), 3.21 (d, *J* = 10.5 Hz, 2H), 3.11 - 3.01 (m, 3H), 2.97 - 2.77 (m, 4H), 2.61 (d, *J* = 13.8 Hz, 3H), 2.47 - 2.42 (m, 1H), 2.32 (s, 2H), 2.06 - 1.95 (m, 3H), 1.93 - 1.83 (m, 1H), 1.80 (s, 1H), 1.45 (t, *J* = 6.1 Hz, 2H), 0.95 (s, 6H). LCMS (ESI) calcd. for C₃₈H₄₆ClF₃N₅O₃ ⁺ [M+H]⁺: 712.32, found, 712.4.

### Example 113: Preparation of 3-(5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-6-fluoro-1-oxoisoindolin-2-yl)piperidine-2,6-dione (GT-09380)

With reference to the method of Scheme 1, the target compound (GT-09380) was obtained as a white solid (17 mg, yield 31 %). ¹H NMR (400 MHz, DMSO) δ 11.01 (s, 1H), 9.92 (s, 1H), 7.73 (s, 1H), 7.57 (d, *J* = 8.6 Hz, 1H), 7.44 (d, *J* = 8.4 Hz, 2H), 7.14 (d, *J* = 8.4 Hz, 2H), 5.13 (dd, *J* = 13.2, 4.9 Hz, 1H), 4.47 (d, *J* = 16.7 Hz, 1H), 4.34 (d, *J* = 17.4 Hz, 1H), 3.96 (s, 2H), 3.53 (s, 3H), 3.21 (d, *J* = 11.1 Hz, 2H), 3.09 - 2.99 (m, 3H), 2.98 - 2.76 (m, 4H), 2.66 - 2.54 (m, 4H), 2.47 - 2.37 (m, 2H), 2.30 (s, 2H), 2.02 (s, 3H), 1.90 - 1.65 (m, 2H), 1.46 (t, *J* = 6.1 Hz, 2H), 0.95 (s, 6H). LCMS (ESI) calcd. for C₃₈H₄₆ClF₃N₅O₃ ⁺ [M+H]⁺: 712.32, found, 712.3.

### Example 114: Preparation of 3-(5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-7-fluoro-1-oxoisoindolin-2-yl)piperidine-2,6-dione (GT-09381)

With reference to the method of Scheme 1, the target compound (GT-09381) was obtained as a white solid (16 mg, yield 29 %). ¹H NMR (400 MHz, DMSO) δ 11.01 (s, 1H), 9.98 (s, 1H), 7.55 - 7.46 (m, 1H), 7.44 (d, *J* = 8.4 Hz, 3H), 7.14 (d, *J* = 8.4 Hz, 2H), 5.09 (dd, *J* = 13.3, 5.1 Hz, 1H), 4.50 (d, *J* = 17.8 Hz, 1H), 4.37 (d, *J* = 17.9 Hz, 1H), 4.05 (s, 2H), 3.52 (d, *J* = 10.4 Hz, 4H), 3.22 (d, *J* = 10.9 Hz, 3H), 3.07 (d, *J* = 10.5 Hz, 3H), 2.92 - 2.77 (m, 3H), 2.60 (d, *J* = 13.8 Hz, 3H), 2.47 - 2.35 (m, 2H), 2.31 (s, 2H), 2.05 - 1.97 (m, 3H), 1.97 - 1.68 (m, 2H), 1.46 (t, *J* = 6.1 Hz, 2H), 0.96 (s, 6H). LCMS (ESI) calcd. for C₃₈H₄₆ClF₃N₅O₃ ⁺ [M+H]⁺: 712.32, found, 712.3.

### Example 115: Preparation of 3-(4-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-oxoisoindolin-2-yl)piperidine-2,6-dione (GT-09382)

With reference to the method of Scheme 1, the target compound (GT-09382) was obtained as a white solid (20 mg, yield 37 %). ¹H NMR (400 MHz, DMSO) δ 11.02 (s, 1H), 9.93 (s, 1H), 7.87 - 7.59 (m, 2H), 7.57 - 7.48 (m, 1H), 7.44 (d, *J* = 8.4 Hz, 2H), 7.14 (d, *J* = 8.4 Hz, 2H), 5.15 (dd, *J* = 13.2, 5.0 Hz, 1H), 4.65 - 4.51 (m, 1H), 4.40 (d, *J* = 17.5 Hz, 1H), 3.83 (s, 1H), 3.52 (s, 3H), 3.22 (d, *J* = 10.8 Hz, 2H), 3.01 (d, *J* = 23.7 Hz, 4H), 2.97 - 2.78 (m, 4H), 2.68 - 2.54 (m, 4H), 2.44 - 2.25 (m, 4H), 2.03 (s, 3H), 1.91 - 1.62 (m, 2H), 1.46 (t, *J* = 6.1 Hz, 2H), 0.96 (s, 6H). LCMS (ESI) calcd. for C₃₈H₄₇ClF₂N₅O₃ ⁺ [M+H]⁺: 694.33, found, 694.4.

### Example 116: Preparation of 3-(6-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-oxoisoindolin-2-yl)piperidine-2,6-dione (GT-09383)

With reference to the method of Scheme 1, the target compound (GT-09383) was obtained as a white solid (24 mg, yield 45 %). ¹H NMR (400 MHz, DMSO) δ 11.01 (s, 1H), 10.14 (s, 1H), 7.90 (s, 1H), 7.82 - 7.72 (m, 1H), 7.71 - 7.63 (m, 1H), 7.44 (d, *J* = 8.3 Hz, 2H), 7.14 (d, *J* = 8.3 Hz, 2H), 5.13 (dd, *J* = 13.2, 4.8 Hz, 1H), 4.50 (d, *J* = 17.6 Hz, 1H), 4.37 (d, 1H), 4.32 - 4.18 (m, 1H), 3.53 (s, 3H), 3.21 (d, *J* = 11.0 Hz, 3H), 3.08 (d, *J* = 10.0 Hz, 3H), 2.97 - 2.76 (m, 4H), 2.68 - 2.53 (m, 4H), 2.48 - 2.37 (m, 2H), 2.32 (s, 2H), 2.11 - 1.95 (m, 4H), 1.87 (s, 1H), 1.45 (t, *J* = 6.1 Hz, 2H), 0.95 (s, 6H). LCMS (ESI) calcd. for C₃₈H₄₇ClF₂N₅O₃ ⁺ [M+H]⁺: 694.33, found, 694.4.

### Example 117: Preparation of 3-(7-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-oxoisoindolin-2-yl)piperidine-2,6-dione (GT-09384)

With reference to the method of Scheme 1, the target compound (GT-09384) was obtained as a white solid (23 mg, yield 43 %). ¹H NMR (400 MHz, DMSO) δ 11.04 (s, 1H), 10.19 (s, 1H), 7.71 (s, 3H), 7.44 (d, *J* = 8.4 Hz, 2H), 7.14 (d, *J* = 8.3 Hz, 2H), 5.16 - 5.06 (m, 1H), 5.01 - 4.56 (m, 2H), 4.51 (d, *J* = 18.0 Hz, 1H), 4.45 - 4.35 (m, 1H), 3.53 (s, 3H), 3.22 (d, *J* = 10.4 Hz, 3H), 3.09 (s, 2H), 2.93 - 2.76 (m, 3H), 2.68 - 2.57 (m, 3H), 2.48 - 2.36 (m, 2H), 2.33 (s, 2H), 2.03 (s, 4H), 1.89 (s, 1H), 1.45 (t, *J* = 6.1 Hz, 2H), 0.95 (s, 6H). LCMS (ESI) calcd. for C₃₈H₄₇ClF₂N₅O₃ ⁺ [M+H]⁺: 694.33, found, 694.4.

### Example 118: Preparation of 3-(5-(2-(4-(4-((4'-chloro-5,5-dimethyl-3,4,5,6-tetrahydro-[1,1'-biphenyl]-2-yl)methyl)piperazin-1-yl)-3,3-difluoropiperidin-1-yl)ethyl)-1-oxoisoindolin-2-yl)piperidine-2,6-dione (GT-09385)

With reference to the method of Scheme 1, the target compound (GT-09385) was obtained as a yellow solid (22 mg, yield 31 %). ¹H NMR (400 MHz, DMSO) δ 10.99 (s, 1H), 10.12 (s, 1H), 7.71 (d, *J* = 7.8 Hz, 1H), 7.51 (s, 1H), 7.44 (t, *J* = 8.5 Hz, 3H), 7.15 (d, *J* = 8.4 Hz, 2H), 5.11 (dd, *J* = 13.2, 5.1 Hz, 1H), 4.45 (d, *J* = 17.5 Hz, 1H), 4.32 (d, *J* = 17.4 Hz, 1H), 3.92 (s, 1H), 3.55 (s, 4H), 3.24 (d, *J* = 11.2 Hz, 6H), 3.15 - 3.06 (m, 3H), 2.94 - 2.83 (m, 3H), 2.68 - 2.57 (m, 4H), 2.43 - 2.38 (m, 1H), 2.34 (s, 2H), 2.08 - 1.93 (m, 5H), 1.46 (t, *J* = 6.1 Hz, 2H), 0.96 (s, 6H). LCMS (ESI) calcd. for C₃₉H₄₉ClF₂N₅O₃ ⁺ [M+H]⁺: 708.35, found, 708.4.

### Example 119: Preparation of 3-(5-(3-(4-(4-((4'-chloro-5,5-dimethyl-3,4,5,6-tetrahydro-[1,1'-biphenyl]-2-yl)methyl)piperazin-1-yl)-3,3-difluoropiperidin-1-yl)propyl)-1-oxoisoindolin-2-yl)piperidine-2,6-dione (GT-09386)

With reference to the method of Scheme 1, the target compound (GT-09386) was obtained as a yellow solid (24 mg, yield 32 %). ¹H NMR (400 MHz, DMSO) δ 11.21 (s, 1H), 10.99 (s, 1H), 10.41 (s, 1H), 7.73 - 7.63 (m, 1H), 7.52 - 7.42 (m, 3H), 7.39 (d, *J* = 7.9 Hz, 1H), 7.15 (d, *J* = 8.4 Hz, 2H), 5.11 (dd, *J* = 13.3, 5.1 Hz, 1H), 4.43 (d, *J* = 17.4 Hz, 1H), 4.31 (d, 1H), 3.90 (s, 1H), 3.46 - 3.27 (m, 4H), 3.22 (d, *J* = 11.0 Hz, 3H), 3.17 - 3.02 (m, 5H), 2.97 - 2.79 (m, 3H), 2.78 - 2.73 (m, 2H), 2.65 - 2.55 (m, 3H), 2.47 - 2.39 (m, 1H), 2.36 (s, 2H), 2.16 - 2.01 (m, 5H), 2.01 - 1.88 (m, 2H), 1.45 (t, *J* = 6.1 Hz, 2H), 0.96 (s, 6H). LCMS (ESI) calcd. for C₄₀H₅₁ClF₂N₅O₃ ⁺ [M+H]⁺: 722.36, found, 722.4.

### Example 120: Preparation of 5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-2-(2,6-dioxopiperidin-3-yl)isoindoline-1,3-dione (GT-09387)

With reference to the method of Scheme 1, the target compound (GT-09387) was obtained as a white solid (15 mg, yield 28 %). ¹H NMR (400 MHz, DMSO) δ 11.14 (s, 1H), 9.96 (s, 1H), 8.04 - 7.87 (m, 3H), 7.44 (d, *J* = 8.3 Hz, 2H), 7.14 (d, *J* = 8.3 Hz, 2H), 5.17 (dd, *J* = 12.8, 5.4 Hz, 1H), 4.08 (s, 2H), 3.54 (s, 3H), 3.21 (d, *J* = 10.6 Hz, 3H), 3.06 (d, *J* = 10.4 Hz, 4H), 2.92 - 2.81 (m, 3H), 2.68 - 2.54 (m, 5H), 2.31 (s, 2H), 2.10 - 1.99 (m, 3H), 1.89 (s, 1H), 1.78 (s, 1H), 1.46 (t, *J* = 6.1 Hz, 2H), 0.96 (s, 6H). LCMS (ESI) calcd. for C₃₈H₄₅ClF₂N₅O₄ ⁺ [M+H]⁺: 708.31, found, 708.3.

### Example 121: Preparation of 4-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-2-(2,6-dioxopiperidin-3-yl)isoindoline-1,3-dione (GT-09388)

With reference to the method of Scheme 1, the target compound (GT-09388) was obtained as a white solid (12 mg, yield 22 %). ¹H NMR (400 MHz, DMSO) δ 11.14 (s, 1H), 9.98 (s, 1H), 7.98 (s, 1H), 7.93 - 7.85 (m, 2H), 7.44 (d, *J* = 8.3 Hz, 2H), 7.14 (d, *J* = 8.3 Hz, 2H), 5.15 (dd, *J* = 12.8, 5.4 Hz, 1H), 4.27 (s, 2H), 3.54 (s, 3H), 3.22 (d, *J* = 10.6 Hz, 3H), 3.08 (s, 4H), 2.91 - 2.81 (m, 3H), 2.68 - 2.53 (m, 5H), 2.31 (s, 2H), 2.08 - 1.99 (m, 3H), 1.95 - 1.81 (m, 1H), 1.76 (s, 1H), 1.46 (t, *J* = 6.1 Hz, 2H), 0.96 (s, 6H). LCMS (ESI) calcd. for C₃₈H₄₅ClF₂N₅O₄ ⁺ [M+H]⁺: 708.31, found, 708.3.

### Example 122: Preparation of 3-(7-((4-(4-(2,3-dichlorophenyl)piperazin-1-yl)piperidin-1-yl)methyl)-1-oxoisoindolin-2-yl)piperidine-2,6-dione (GT-09389)

With reference to the method of Scheme 1, the target compound (GT-09389) was obtained as a white solid (23 mg, yield 38 %). ¹H NMR (400 MHz, DMSO) δ 11.67 (s, 1H), 11.02 (s, 1H), 10.64 (s, 1H), 7.90 - 7.67 (m, 3H), 7.41 - 7.35 (m, 2H), 7.23 - 7.14 (m, 1H), 5.13 (dd, *J* = 13.1, 5.0 Hz, 1H), 4.86 (d, *J* = 11.5 Hz, 1H), 4.73 (d, *J* = 12.6 Hz, 1H), 4.52 (d, *J* = 17.9 Hz, 1H), 4.39 (d, 1H), 3.63 - 3.54 (m, 4H), 3.49 - 3.45 (m, 2H), 3.29 - 3.25 (m, 2H), 3.19 - 3.05 (m, 4H), 3.00 - 2.84 (m, 2H), 2.64 (d, *J* = 16.5 Hz, 1H), 2.46 - 2.35 (m, 3H), 2.22 - 1.99 (m, 3H). LCMS (ESI) calcd. for C₂₉H₃₄Cl₂N₅O₃ ⁺ [M+H]⁺: 570.20, found, 570.3.

### Example 123: Preparation of 3-(5-(2-(4-(4-(2,3-dichlorophenyl)piperazin-1-yl)piperidin-1-yl)ethyl)-1-oxoisoindolin-2-yl)piperidine-2,6-dione (GT-09390)

With reference to the method of Scheme 1, the target compound (GT-09390) was obtained as a white solid (9 mg, yield 11 %). ¹H NMR (400 MHz, DMSO) δ 11.45 (s, 1H), 10.99 (s, 1H), 10.78 (s, 1H), 7.73 (d, *J* = 7.7 Hz, 1H), 7.54 (s, 1H), 7.46 - 7.36 (m, 2H), 7.21 (d, *J* = 4.4 Hz, 1H), 5.17 - 5.03 (m, 1H), 4.46 (d, *J* = 17.4 Hz, 1H), 4.33 (d, *J* = 17.6 Hz, 1H), 3.78 - 3.72 (m, 1H), 3.61 (s, 2H), 3.54 - 3.44 (m, 3H), 3.30 - 3.08 (m, 8H), 3.05 - 2.83 (m, 3H), 2.70 - 2.57 (m, 2H), 2.46 - 2.34 (m, 3H), 2.33 - 2.10 (m, 2H), 2.04 - 1.94 (m, 1H). LCMS (ESI) calcd. for C₃₀H₃₆Cl₂N₅O₃ ⁺ [M+H]⁺: 584.22, found, 584.3.

### Example 124: Preparation of 3-(5-(3-(4-(4-(2,3-dichlorophenyl)piperazin-1-yl)piperidin-1-yl)propyl)-1-oxoisoindolin-2-yl)piperidine-2,6-dione (GT-09391)

With reference to the method of Scheme 1, the target compound (GT-09391) was obtained as a white solid (31 mg, yield 37 %). ¹H NMR (400 MHz, DMSO) δ 11.57 (s, 1H), 10.99 (s, 1H), 10.73 (s, 1H), 7.69 (d, *J* = 7.8 Hz, 1H), 7.50 (s, 1H), 7.44 - 7.36 (m, 3H), 7.23 - 7.17 (m, 1H), 5.11 (dd, *J* = 13.3, 5.1 Hz, 1H), 4.43 (d, *J* = 17.2, 8.4 Hz, 1H), 4.31 (d, 1H), 3.69 - 3.57 (m, 4H), 3.47 - 3.40 (m, 4H), 3.28 - 3.19 (m, 3H), 3.05 (s, 2H), 2.99 - 2.86 (m, 3H), 2.81 - 2.71 (m, 2H), 2.61 (d, *J* = 18.2 Hz, 2H), 2.44 - 2.32 (m, 3H), 2.24 - 2.18 (m, 1H), 2.12 - 1.97 (m, 3H). LCMS (ESI) calcd. for C₃₁H₃₈Cl₂N₅O₃ ⁺ [M+H]⁺: 598.23, found, 598.3.

### Example 125: Preparation of 5-((4-(4-(2,3-dichlorophenyl)piperazin-1-yl)piperidin-1-yl)methyl)-2-(2,6-dioxopiperidin-3-yl)isoindoline-1,3-dione (GT-09392)

With reference to the method of Scheme 1, the target compound (GT-09392) was obtained as a white solid (15 mg, yield 25 %). ¹H NMR (400 MHz, DMSO) δ 11.51 (s, 1H), 11.26 (s, 1H), 11.16 (s, 1H), 8.23 (s, 1H), 8.11 (d, *J* = 7.0 Hz, 1H), 8.04 (d, *J* = 7.6 Hz, 1H), 7.43 - 7.27 (m, 2H), 7.24 - 7.10 (m, 1H), 5.19 (dd, *J* = 12.8, 5.4 Hz, 1H), 4.52 (s, 2H), 3.62 - 3.50 (m, 4H), 3.44 (s, 2H), 3.30 - 3.11 (m, 5H), 3.11 - 2.94 (m, 2H), 2.94 - 2.86 (m, 1H), 2.65 - 2.53 (m, 2H), 2.42 - 2.31 (m, 2H), 2.30 - 2.13 (m, 2H), 2.12 - 2.03 (m, 1H). LCMS (ESI) calcd. for C₂₉H₃₂Cl₂N₅O₄ ⁺ [M+H]⁺: 584.18, found, 584.2.

### Example 126: Preparation of 4-((4-(4-(2,3-dichlorophenyl)piperazin-1-yl)piperidin-1-yl)methyl)-2-(2,6-dioxopiperidin-3-yl)isoindoline-1,3-dione (GT-09393)

With reference to the method of Scheme 1, the target compound (GT-09393) was obtained as a yellow solid (20 mg, yield 33 %). ¹H NMR (400 MHz, DMSO) δ 11.47 (s, 1H), 11.17 (s, 1H), 10.97 (s, 1H), 8.15 (d, *J* = 8.5 Hz, 1H), 8.03 (d, *J* = 7.3 Hz, 1H), 8.01 - 7.95 (m, 1H), 7.48 - 7.32 (m, 2H), 7.26 - 7.14 (m, 1H), 5.19 (dd, *J* = 12.8, 5.4 Hz, 1H), 4.71 (s, 2H), 3.68 - 3.55 (m, 4H), 3.47 - 3.43 (m, 2H), 3.27 - 3.08 (m, 6H), 2.94 - 2.85 (m, 2H), 2.73 (s, 1H), 2.69 - 2.55 (m, 2H), 2.42 - 2.33 (m, 2H), 2.23 - 2.14 (m, 1H), 2.12 - 2.03 (m, 1H). LCMS (ESI) calcd. for C₂₉H₃₂Cl₂N₅O₄ ⁺ [M+H]⁺: 584.18, found, 584.2.

### Example 127: Preparation of 3-(5-((4-(4-(2,3-dichlorophenyl)piperazin-1-yl)-3,3-difluoropiperidin-1-yl)methyl)-4-fluoro-1-oxoisoindolin-2-yl)piperidine-2,6-dione (GT-09417)

With reference to the method of Scheme 1, the target compound (GT-09417) was obtained as a white solid (35 mg, yield 59 %). ¹H NMR (400 MHz, DMSO) δ 11.03 (s, 1H), 7.72 - 7.62 (m, 2H), 7.38 - 7.31 (m, 2H), 7.21 - 7.13 (m, 1H), 5.14 (dd, *J* = 13.2, 5.0 Hz, 1H), 4.59 (d, *J* = 17.5 Hz, 1H), 4.43 (d, *J* = 17.4 Hz, 1H), 4.00 (s, 2H), 3.83 - 3.71 (m, 3H), 3.32 - 3.21 (m, 6H), 3.15 (s, 2H), 2.95 - 2.89 (m, 1H), 2.83 - 2.71 (m, 1H), 2.61 (d, *J* = 17.3 Hz, 2H), 2.47 - 2.41 (m, 1H), 2.33 (s, 1H), 2.12 - 2.00 (m, 2H). LCMS (ESI) calcd. for C₂₉H₃₁Cl₂F₃N₅O₃ ⁺ [M+H]⁺: 624.18, found, 624.2.

### Example 128: Preparation of 3-(5-((4-(4-(2,3-dichlorophenyl)piperazin-1-yl)-3,3-difluoropiperidin-1-yl)methyl)-6-fluoro-1-oxoisoindolin-2-yl)piperidine-2,6-dione (GT-09418)

With reference to the method of Scheme 1, the target compound (GT-09418) was obtained as a white solid (31 mg, yield 52 %). ¹H NMR (400 MHz, DMSO) δ 11.02 (s, 1H), 7.74 (d, *J* = 5.5 Hz, 1H), 7.58 (d, *J* = 8.6 Hz, 1H), 7.41 - 7.28 (m, 2H), 7.23 - 7.11 (m, 1H), 5.13 (dd, *J* = 13.3, 5.1 Hz, 1H), 4.49 (d, *J* = 17.4 Hz, 1H), 4.36 (d, *J* = 17.4 Hz, 1H), 3.96 (s, 2H), 3.82 - 3.61 (m, 3H), 3.31 - 3.20 (m, 6H), 3.12 (s, 2H), 2.97 - 2.89 (m, 1H), 2.68 (s, 1H), 2.66 - 2.59 (m, 1H), 2.49 - 2.39 (m, 2H), 2.29 (s, 1H), 2.11 - 1.98 (m, 2H). LCMS (ESI) calcd. for C₂₉H₃₁Cl₂F₃N₅O₃ ⁺ [M+H]⁺: 624.18, found, 624.2.

### Example 129: Preparation of 3-(5-((4-(4-(2,3-dichlorophenyl)piperazin-1-yl)-3,3-difluoropiperidin-1-yl)methyl)-7-fluoro-1-oxoisoindolin-2-yl)piperidine-2,6-dione (GT-09419)

With reference to the method of Scheme 1, the target compound (GT-09419) was obtained as a white solid (24 mg, yield 40 %). ¹H NMR (400 MHz, DMSO) δ 11.02 (s, 1H), 7.48 (s, 1H), 7.42 - 7.30 (m, 3H), 7.22 - 7.14 (m, 1H), 5.09 (dd, *J* = 13.3, 5.1 Hz, 1H), 4.51 (d, *J* = 17.9 Hz, 1H), 4.38 (d, *J* = 17.9 Hz, 1H), 4.00 (s, 2H), 3.65 (s, 3H), 3.34 - 3.16 (m, 9H), 2.95 - 2.88 (m, 1H), 2.85 - 2.71 (m, 1H), 2.63 - 2.57 (m, 1H), 2.45 - 2.35 (m, 1H), 2.35 - 2.25 (m, 1H), 2.15 - 2.06 (m, 1H), 2.06 - 1.95 (m, 1H). LCMS (ESI) calcd. for C₂₉H₃₁Cl₂F₃N₅O₃ ⁺ [M+H]⁺: 624.18, found, 624.2.

### Example 130: Preparation of 3-(4-((4-(4-(2,3-dichlorophenyl)piperazin-1-yl)-3,3-difluoropiperidin-1-yl)methyl)-1-oxoisoindolin-2-yl)piperidine-2,6-dione (GT-09420)

With reference to the method of Scheme 1, the target compound (GT-09420) was obtained as a white solid (31 mg, yield 54 %). ¹H NMR (400 MHz, DMSO) δ 11.03 (s, 1H), 7.72 (d, *J* = 7.4 Hz, 1H), 7.66 (s, 1H), 7.55 (t, *J* = 7.4 Hz, 1H), 7.39 - 7.31 (m, 2H), 7.21 - 7.14 (m, 1H), 5.16 (dd, *J* = 13.1, 5.0 Hz, 1H), 4.63 - 4.51 (m, 1H), 4.41 (d, *J* = 17.6 Hz, 1H), 3.89 (s, 3H), 3.67 - 3.58 (m, 3H), 3.42 - 3.37 (m, 2H), 3.36 - 3.20 (m, 6H), 3.09 (s, 1H), 2.98 - 2.89 (m, 1H), 2.63 (d, *J* = 16.9 Hz, 1H), 2.43 - 2.24 (m, 2H), 2.15 - 1.99 (m, 2H). LCMS (ESI) calcd. for C₂₉H₃₂Cl₂F₂N₅O₃ ⁺ [M+H]⁺: 606.18, found, 606.2.

### Example 131: Preparation of 3-(6-((4-(4-(2,3-dichlorophenyl)piperazin-1-yl)-3,3-difluoropiperidin-1-yl)methyl)-1-oxoisoindolin-2-yl)piperidine-2,6-dione (GT-09421)

With reference to the method of Scheme 1, the target compound (GT-09421) was obtained as a white solid (39 mg, yield 67 %). ¹H NMR (400 MHz, DMSO) δ 11.02 (s, 1H), 7.90 (s, 1H), 7.78 (d, *J* = 6.9 Hz, 1H), 7.69 (d, *J* = 7.8 Hz, 1H), 7.33 (d, *J* = 6.8 Hz, 2H), 7.16 (t, 1H), 5.14 (dd, *J* = 13.2, 5.0 Hz, 1H), 4.51 (d, *J* = 17.6 Hz, 1H), 4.38 (d, *J* = 17.6 Hz, 1H), 4.26 (s, 2H), 3.80 - 3.73 (m, 2H), 3.35 (s, 2H), 3.27 - 3.10 (m, 8H), 2.96 - 2.88 (m, 1H), 2.81 (s, 1H), 2.62 (d, *J* = 17.1 Hz, 1H), 2.45 - 2.36 (m, 1H), 2.32 (d, *J* = 10.2 Hz, 1H), 2.20 (s, 1H), 2.06 - 1.95 (m, 1H). LCMS (ESI) calcd. for C₂₉H₃₂Cl₂F₂N₅O₃ ⁺ [M+H]⁺: 606.18, found, 606.2.

### Example 132: Preparation of 3-(7-((4-(4-(2,3-dichlorophenyl)piperazin-1-yl)-3,3-difluoropiperidin-1-yl)methyl)-1-oxoisoindolin-2-yl)piperidine-2,6-dione (GT-09422)

With reference to the method of Scheme 1, the target compound (GT-09422) was obtained as a white solid (27 mg, yield 47 %). ¹H NMR (400 MHz, DMSO) δ 11.03 (s, 1H), 7.77 - 7.62 (m, 3H), 7.40 - 7.25 (m, 2H), 7.20 - 7.09 (m, 1H), 5.17 - 5.08 (m, 1H), 4.85 - 4.38 (m, 4H), 3.71 - 3.66 (m, 2H), 3.41 - 3.37 (m, 2H), 3.18 (s, 8H), 2.98 - 2.86 (m, 2H), 2.62 (d, *J* = 17.6 Hz, 1H), 2.47 - 2.37 (m, 1H), 2.35 - 2.26 (m, 1H), 2.23 - 2.11 (m, 1H), 2.08 - 1.99 (m, 1H). LCMS (ESI) calcd. for C₂₉H₃₂Cl₂F₂N₅O₃ ⁺ [M+H]⁺: 606.18, found, 606.2.

### Example 133: Preparation of 3-(5-(2-(4-(4-(2,3-dichlorophenyl)piperazin-1-yl)-3,3-difluoropiperidin-1-yl)ethyl)-1-oxoisoindolin-2-yl)piperidine-2,6-dione (GT-09430)

With reference to the method of Scheme 1, the target compound (GT-09430) was obtained as a white solid (23 mg, yield 39 %). ¹H NMR (400 MHz, DMSO) δ 11.00 (s, 1H), 7.72 (d, *J* = 7.8 Hz, 1H), 7.53 (s, 1H), 7.44 (d, *J* = 7.8 Hz, 1H), 7.39 - 7.31 (m, 2H), 7.22 - 7.12 (m, 1H), 5.12 (dd, *J* = 13.2, 5.1 Hz, 1H), 4.46 (d, *J* = 17.5 Hz, 1H), 4.33 (d, *J* = 17.5 Hz, 1H), 4.03 (s, 1H), 3.66 (s, 3H), 3.39 - 3.32 (m, 3H), 3.23 - 3.09 (m, 10H), 2.97 - 2.89 (m, 1H), 2.61 (d, *J* = 16.9 Hz, 1H), 2.44 - 2.20 (m, 3H), 2.06 - 1.97 (m, 1H). LCMS (ESI) calcd. for C₃₀H₃₄Cl₂F₂N₅O₃ ⁺ [M+H]⁺: 620.20, found, 620.3.

### Example 134: Preparation of 3-(5-(3-(4-(4-(2,3-dichlorophenyl)piperazin-1-yl)-3,3-difluoropiperidin-1-yl)propyl)-1-oxoisoindolin-2-yl)piperidine-2,6-dione (GT-09431)

With reference to the method of Scheme 1, the target compound (GT-09431) was obtained as a white solid (18 mg, yield 31 %). ¹H NMR (400 MHz, DMSO) δ 10.99 (s, 1H), 7.68 (d, *J* = 7.8 Hz, 1H), 7.50 (s, 1H), 7.41 (d, *J* = 8.0 Hz, 1H), 7.35 - 7.30 (m, 2H), 7.19 - 7.13 (m, 1H), 5.14 - 5.05 (m, 1H), 4.44 (d, *J* = 17.4 Hz, 1H), 4.31 (d, *J* = 17.4 Hz, 1H), 3.96 (s, 1H), 3.65 - 3.64 (m, 1H), 3.08 (s, 10H), 2.98 - 2.84 (m, 2H), 2.77 (t, *J* = 7.4 Hz, 2H), 2.67 - 2.53 (m, 2H), 2.49 - 2.30 (m, 2H), 2.29 - 2.16 (m, 2H), 2.15 - 2.05 (m, 2H), 2.04 - 1.97 (m, 1H). LCMS (ESI) calcd. for C₃₁H₃₆Cl₂F₂N₅O₃ ⁺ [M+H]⁺: 634.22, found, 634.3.

### Example 135: Preparation of 5-((4-(4-(2,3-dichlorophenyl)piperazin-1-yl)-3,3-difluoropiperidin-1-yl)methyl)-2-(2,6-dioxopiperidin-3-yl)isoindoline-1,3-dione (GT-09432)

With reference to the method of Scheme 1, the target compound (GT-09432) was obtained as a white solid (27 mg, yield 46 %). ¹H NMR (400 MHz, DMSO) δ 11.15 (s, 1H), 7.96 (d, *J* = 7.4 Hz, 2H), 7.90 (d, *J* = 7.6 Hz, 1H), 7.37 - 7.32 (m, 2H), 7.20 - 7.15 (m, 1H), 5.17 (dd, *J* = 12.8, 5.3 Hz, 1H), 4.03 (s, 2H), 3.82 - 3.60 (m, 3H), 3.29 - 3.20 (m, 6H), 3.17 - 3.08 (m, 2H), 2.94 - 2.86 (m, 1H), 2.77 (s, 1H), 2.67 - 2.53 (m, 3H), 2.27 (s, 1H), 2.13 - 2.01 (m, 2H). LCMS (ESI) calcd. for C₂₉H₃₀Cl₂F₂N₅O₄ ⁺ [M+H]⁺: 620.16, found, 620.2.

### Example 136: Preparation of 4-((4-(4-(2,3-dichlorophenyl)piperazin-1-yl)-3,3-difluoropiperidin-1-yl)methyl)-2-(2,6-dioxopiperidin-3-yl)isoindoline-1,3-dione (GT-09433)

With reference to the method of Scheme 1, the target compound (GT-09433) was obtained as a white solid (30 mg, yield 51 %). ¹H NMR (400 MHz, DMSO) δ 11.14 (s, 1H), 7.99 - 7.92 (m, 1H), 7.92 - 7.85 (m, 2H), 7.38 - 7.33 (m, 2H), 7.20 - 7.14 (m, 1H), 5.16 (dd, *J* = 12.7, 5.4 Hz, 1H), 4.23 (s, 2H), 3.97 - 3.61 (m, 3H), 3.28 (s, 6H), 3.12 (s, 2H), 2.95 - 2.86 (m, 1H), 2.67 (s, 1H), 2.65 - 2.54 (m, 2H), 2.49 - 2.37 (m, 1H), 2.33 (s, 1H), 2.11 - 2.01 (m, 2H). LCMS (ESI) calcd. for C₂₉H₃₀Cl₂F₂N₅O₄ ⁺ [M+H]⁺: 620.16, found, 620.2.

### Example 137: Preparation of 3-(5-((4-(4-(2,3-dichlorophenyl)-3,6-dihydropyridin-1(2H)-yl)-3,3-difluoropiperidin-1-yl)methyl)-4-fluoro-1-oxoisoindolin-2-yl)piperidine-2,6-dione (GT-09599)

With reference to the method of Scheme 1, the target compound (GT-09599) was obtained as a white solid (20 mg, yield 34 %). ¹H NMR (400 MHz, DMSO) δ 11.02 (s, 1H), 7.68 - 7.58 (m, 3H), 7.39 (t, *J* = 7.9 Hz, 1H), 7.26 (dd, *J* = 7.7, 1.5 Hz, 1H), 5.76 (s, 1H), 5.14 (dd, *J* = 13.3, 5.1 Hz, 1H), 4.59 (d, *J* = 17.5 Hz, 1H), 4.42 (d, *J* = 17.4 Hz, 1H), 3.96 (s, 5H), 3.45 - 3.35 (m, 3H), 3.11 (s, 1H), 2.97 - 2.88 (m, 1H), 2.77 - 2.69 (m, 1H), 2.61 (d, *J* = 16.6 Hz, 2H), 2.48 - 2.32 (m, 3H), 2.12 - 1.97 (m, 2H). LCMS (ESI) calcd. for C₃₀H₃₀Cl₂F₃N₄O₃ ⁺ [M+H]⁺: 621.16, found, 621.2.

### Example 138: Preparation of 3-(5-((4-(4-(2,3-dichlorophenyl)-3,6-dihydropyridin-1(2H)-yl)-3,3-difluoropiperidin-1-yl)methyl)-6-fluoro-1-oxoisoindolin-2-yl)piperidine-2,6-dione (GT-09600)

With reference to the method of Scheme 1, the target compound (GT-09600) was obtained as a white solid (20 mg, yield 34 %). ¹H NMR (400 MHz, DMSO) δ 11.02 (s, 1H), 7.72 (d, *J* = 5.6 Hz, 1H), 7.65 - 7.54 (m, 2H), 7.39 (t, *J* = 7.9 Hz, 1H), 7.30 - 7.21 (m, 1H), 5.76 (s, 1H), 5.19 - 5.06 (m, 1H), 4.49 (d, *J* = 17.4 Hz, 1H), 4.36 (d, *J* = 17.2 Hz, 1H), 3.93 (s, 5H), 3.36 - 3.25 (m, 2H), 3.10 (s, 1H), 2.96 - 2.87 (m, 1H), 2.83 - 2.66 (m, 2H), 2.65 - 2.55 (m, 2H), 2.47 - 2.31 (m, 3H), 2.14 - 1.98 (m, 2H). LCMS (ESI) calcd. for C₃₀H₃₀Cl₂F₃N₄O₃ ⁺ [M+H]⁺: 621.16, found, 621.2.

### Example 139: Preparation of 3-(5-((4-(4-(2,3-dichlorophenyl)-3,6-dihydropyridin-1(2H)-yl)-3,3-difluoropiperidin-1-yl)methyl)-7-fluoro-1-oxoisoindolin-2-yl)piperidine-2,6-dione (GT-09601)

With reference to the method of Scheme 1, the target compound (GT-09601) was obtained as a white solid (16 mg, yield 28 %). ¹H NMR (400 MHz, DMSO) δ 11.02 (s, 1H), 7.64 - 7.59 (m, 1H), 7.45 (s, 1H), 7.39 (t, *J* = 7.9 Hz, 1H), 7.32 (d, *J* = 10.0 Hz, 1H), 7.29 - 7.24 (m, 1H), 5.76 (s, 1H), 5.09 (dd, *J* = 13.3, 5.1 Hz, 1H), 4.51 (d, *J* = 17.6 Hz, 1H), 4.37 (d, *J* = 17.9 Hz, 1H), 3.91 (s, 5H), 3.19 - 3.00 (m, 2H), 2.98 - 2.83 (m, 2H), 2.82 - 2.56 (m, 4H), 2.44 - 2.23 (m, 3H), 2.15 - 1.96 (m, 2H). LCMS (ESI) calcd. for C₃₀H₃₀Cl₂F₃N₄O₃ ⁺ [M+H]⁺: 621.16, found, 621.2.

### Example 140: Preparation of 3-(4-((4-(4-(2,3-dichlorophenyl)-3,6-dihydropyridin-1(2H)-yl)-3,3-difluoropiperidin-1-yl)methyl)-1-oxoisoindolin-2-yl)piperidine-2,6-dione (GT-09602)

With reference to the method of Scheme 1, the target compound (GT-09602) was obtained as a white solid (6 mg, yield 11 %). ¹H NMR (400 MHz, DMSO) δ 11.02 (s, 1H), 7.70 (d, *J* = 7.5 Hz, 1H), 7.66 - 7.58 (m, 2H), 7.54 (t, *J* = 7.5 Hz, 1H), 7.40 (t, *J* = 10.2, 5.4 Hz, 1H), 7.32 - 7.19 (m, 1H), 5.76 (s, 1H), 5.16 (dd, *J* = 13.2, 5.1 Hz, 1H), 4.63 - 4.48 (m, 1H), 4.40 (d, *J* = 17.7 Hz, 1H), 4.17 - 3.58 (m, 6H), 3.30 - 3.15 (m, 2H), 3.14 - 2.87 (m, 3H), 2.63 (d, *J* = 17.5 Hz, 2H), 2.44 - 2.27 (m, 3H), 2.09 - 1.95 (m, 2H). LCMS (ESI) calcd. for C₃₀H₃₁Cl₂F₂N₄O₃ ⁺ [M+H]⁺: 603.17, found, 603.2.

### Example 141: Preparation of 3-(6-((4-(4-(2,3-dichlorophenyl)-3,6-dihydropyridin-1(2H)-yl)-3,3-difluoropiperidin-1-yl)methyl)-1-oxoisoindolin-2-yl)piperidine-2,6-dione (GT-09635)

With reference to the method of Scheme 1, the target compound (GT-09635) was obtained as a white solid (18 mg, yield 31 %). ¹H NMR (400 MHz, DMSO) δ 11.01 (s, 1H), 7.90 - 7.78 (m, 1H), 7.76 - 7.65 (m, 2H), 7.60 (d, *J* = 8.1 Hz, 1H), 7.38 (t, *J* = 7.8 Hz, 1H), 7.30 - 7.19 (m, 1H), 5.74 (s, 1H), 5.14 (dd, *J* = 13.2, 5.0 Hz, 1H), 4.50 (d, *J* = 17.6 Hz, 1H), 4.37 (d, *J* = 17.5 Hz, 1H), 4.14 (s, 2H), 3.91 - 3.79 (m, 3H), 3.42 - 3.18 (m, 4H), 2.98 - 2.82 (m, 2H), 2.67 - 2.57 (m, 3H), 2.45 - 2.34 (m, 2H), 2.19 (s, 1H), 2.05 - 1.93 (m, 1H). LCMS (ESI) calcd. for C₃₀H₃₁Cl₂F₂N₄O₃ ⁺ [M+H]⁺: 603.17, found, 603.3.

### Example 142: Preparation of 3-(7-((4-(4-(2,3-dichlorophenyl)-3,6-dihydropyridin-1(2H)-yl)-3,3-difluoropiperidin-1-yl)methyl)-1-oxoisoindolin-2-yl)piperidine-2,6-dione (GT-09636)

With reference to the method of Scheme 1, the target compound (GT-09636) was obtained as a white solid (23 mg, yield 41 %). ¹H NMR (400 MHz, DMSO) δ 11.02 (d, *J* = 5.3 Hz, 1H), 9.31 (d, *J* = 6.6 Hz, 1H), 8.45 (d, *J* = 6.9 Hz, 1H), 7.68 - 7.64 (m, 2H), 7.61 - 7.57 (m, 2H), 5.74 (s, 1H), 5.14 - 5.10 (m, 1H), 4.51 - 4.33 (m, 4H), 3.81 (s, 3H), 3.40 - 3.14 (m, 4H), 2.95 - 2.85 (m, 2H), 2.81 - 2.73 (m, 1H), 2.69 - 2.56 (m, 3H), 2.46 - 2.38 (m, 2H), 2.05 - 2.00 (m, 1H). LCMS (ESI) calcd. for C₃₀H₃₁Cl₂F₂N₄O₃ ⁺ [M+H]⁺: 603.17, found, 603.3.

### Example 143: Preparation of 3-(5-(2-(4-(4-(2,3-dichlorophenyl)-3,6-dihydropyridin-1(2H)-yl)-3,3-difluoropiperidin-1-yl)ethyl)-1-oxoisoindolin-2-yl)piperidine-2,6-dione (GT-09637)

With reference to the method of Scheme 1, the target compound (GT-09637) was obtained as a white solid (14 mg, yield 25 %). ¹H NMR (400 MHz, DMSO) δ 11.00 (s, 1H), 9.33 (d, *J* = 6.6 Hz, 1H), 8.48 (d, *J* = 9.5 Hz, 1H), 7.74 - 7.58 (m, 3H), 7.53 (s, 1H), 7.47 - 7.43 (m, 1H), 5.74 (s, 1H), 5.12 (dd, *J* = 13.2, 5.1 Hz, 1H), 4.45 (d, *J* = 17.2 Hz, 1H), 4.33 (d, 1H), 3.94 - 3.60 (m, 5H), 3.35 - 3.14 (m, 5H), 3.08 - 2.86 (m, 5H), 2.64 - 2.55 (m, 2H), 2.46 - 2.35 (m, 2H), 2.03 - 1.96 (m, 1H). LCMS (ESI) calcd. for C₃₁H₃₃Cl₂F₂N₄O₃ ⁺ [M+H]⁺: 617.19, found, 617.3.

### Example 144: Preparation of 3-(5-(3-(4-(4-(2,3-dichlorophenyl)-3,6-dihydropyridin-1(2H)-yl)-3,3-difluoropiperidin-1-yl)propyl)-1-oxoisoindolin-2-yl)piperidine-2,6-dione (GT-09638)

With reference to the method of Scheme 1, the target compound (GT-09638) was obtained as a white solid (17 mg, yield 29 %). ¹H NMR (400 MHz, DMSO) δ 10.99 (s, 1H), 9.31 (d, *J* = 6.4 Hz, 1H), 8.47 (d, *J* = 6.8 Hz, 1H), 7.73 - 7.63 (m, 2H), 7.64 - 7.57 (m, 1H), 7.50 (s, 1H), 7.44 - 7.40 (m, 1H), 5.72 (s, 1H), 5.11 (dd, *J* = 13.2, 5.0 Hz, 1H), 4.44 (d, *J* = 17.1 Hz, 1H), 4.31 (d, *J* = 17.3 Hz, 1H), 3.93 (s, 1H), 3.72 - 3.55 (m, 6H), 3.09 (s, 3H), 2.96 - 2.87 (m, 2H), 2.82 - 2.73 (m, 3H), 2.61 (d, *J* = 17.7 Hz, 2H), 2.46 - 2.34 (m, 2H), 2.12 - 2.05 (m, 1H), 2.04 - 1.96 (m, 2H). LCMS (ESI) calcd. for C₃₂H₃₅Cl₂F₂N₄O₃ ⁺ [M+H]⁺: 631.20, found, 631.3.

### Example 145: Preparation of 5-((4-(4-(2,3-dichlorophenyl)-3,6-dihydropyridin-1(2H)-yl)-3,3-difluoropiperidin-1-yl)methyl)-2-(2,6-dioxopiperidin-3-yl)isoindoline-1,3-dione (GT-09639)

With reference to the method of Scheme 1, the target compound (GT-09639) was obtained as a white solid (10 mg, yield 18 %). ¹H NMR (400 MHz, DMSO) δ 11.14 (s, 1H), 7.97 - 7.91 (m, 2H), 7.88 (s, 1H), 7.61 (d, *J* = 7.7 Hz, 1H), 7.39 (t, *J* = 7.9 Hz, 1H), 7.26 (dd, *J* = 7.7, 1.5 Hz, 1H), 5.75 (s, 1H), 5.17 (dd, *J* = 12.9, 5.3 Hz, 1H), 3.97 (d, *J* = 7.7 Hz, 5H), 3.10 (d, *J* = 27.5 Hz, 2H), 2.90 (ddd, *J* = 30.4, 16.3, 9.4 Hz, 2H), 2.68 - 2.53 (m, 4H), 2.49 - 2.27 (m, 3H), 2.10 - 2.00 (m, 2H). LCMS (ESI) calcd. for C₃₀H₂₉Cl₂F₂N₄O₄ ⁺ [M+H]⁺: 617.15, found, 617.2.

### Example 146: Preparation of 4-((4-(4-(2,3-dichlorophenyl)-3,6-dihydropyridin-1(2H)-yl)-3,3-difluoropiperidin-1-yl)methyl)-2-(2,6-dioxopiperidin-3-yl)isoindoline-1,3-dione (GT-09640)

With reference to the method of Scheme 1, the target compound (GT-09640) was obtained as a white solid (12 mg, yield 21 %). ¹H NMR (400 MHz, DMSO) δ 11.14 (s, 1H), 7.94 - 7.85 (m, 3H), 7.64 - 7.55 (m, 1H), 7.39 (t, *J* = 7.8 Hz, 1H), 7.26 (d, *J* = 7.5 Hz, 1H), 5.76 (s, 1H), 5.15 (dd, *J* = 12.7, 5.4 Hz, 1H), 4.20 (d, *J* = 23.9 Hz, 2H), 3.93 (s, 2H), 3.36 - 3.20 (m, 4H), 3.06 (s, 1H), 2.93 - 2.86 (m, 1H), 2.71 - 2.53 (m, 4H), 2.46 - 2.24 (m, 2H), 2.12 - 1.98 (m, 2H). LCMS (ESI) calcd. for C₃₀H₂₉Cl₂F₂N₄O₄ ⁺ [M+H]⁺: 617.15, found, 617.2.

### Example 147: Preparation of 3-(4-fluoro-5-((4-(4-(6-fluorobenzo[d]isoxazol-3-yl)piperazin-1-yl)piperidin-1-yl)methyl)-1-oxoisoindolin-2-yl)piperidine-2,6-dione (GT-09641)

With reference to the method of Scheme 1, the target compound (GT-09641) was obtained as a white solid (16 mg, yield 26 %). ¹H NMR (400 MHz, DMSO) δ 11.59 (s, 1H), 11.02 (d, *J* = 13.9 Hz, 2H), 8.14 - 8.06 (m, 1H), 7.88 (s, 1H), 7.71 (d, *J* = 7.6 Hz, 1H), 7.63 - 7.59 (m, 1H), 7.31 - 7.21 (m, 1H), 5.18 - 5.11 (m, 1H), 4.66 - 4.56 (m, 2H), 4.55 - 4.40 (m, 3H), 4.11 (s, 2H), 3.67 - 3.53 (m, 6H), 3.15 - 3.00 (m, 3H), 2.96 - 2.90 (m, 1H), 2.65 - 2.56 (m, 2H), 2.47 - 2.39 (m, 2H), 2.38 - 2.31 (m, 2H), 2.18 (s, 1H), 2.03 - 1.99 (m, 1H). LCMS (ESI) calcd. for C₃₀H₃₃F₂N₆O₄ ⁺ [M+H]⁺: 579.25, found, 579.3.

### Example 148: Preparation of 3-(6-fluoro-5-((4-(4-(6-fluorobenzo[d]isoxazol-3-yl)piperazin-1-yl)piperidin-1-yl)methyl)-1-oxoisoindolin-2-yl)piperidine-2,6-dione (GT-09642)

With reference to the method of Scheme 1, the target compound (GT-09642) was obtained as a white solid (27 mg, yield 43 %). ¹H NMR (400 MHz, DMSO) δ 11.82 (s, 1H), 11.26 (s, 1H), 11.02 (d, *J* = 13.2 Hz, 1H), 8.10 (dd, *J* = 8.8, 5.1 Hz, 1H), 7.98 (dd, *J* = 17.1, 5.3 Hz, 1H), 7.68 (d, *J* = 8.5 Hz, 1H), 7.61 (d, *J* = 9.0 Hz, 1H), 7.26 (t, *J* = 9.1 Hz, 1H), 5.18 - 5.11 (m, 1H), 4.88 (s, 1H), 4.52 - 4.33 (m, 4H), 4.11 (s, 2H), 3.64 - 3.47 (m, 6H), 3.14 - 3.01 (m, 3H), 2.96 - 2.89 (m, 1H), 2.69 - 2.55 (m, 2H), 2.47 - 2.34 (m, 3H), 2.20 (s, 2H), 2.04 - 1.98 (m, 1H). LCMS (ESI) calcd. for C₃₀H₃₃F₂N₆O₄ ⁺ [M+H]⁺: 579.25, found, 579.3.

### Example 149: Preparation of 3-(7-fluoro-5-((4-(4-(6-fluorobenzo[d]isoxazol-3-yl)piperazin-1-yl)piperidin-1-yl)methyl)-1-oxoisoindolin-2-yl)piperidine-2,6-dione (GT-09643)

With reference to the method of Scheme 1, the target compound (GT-09643) was obtained as a white solid (11 mg, yield 18 %). ¹H NMR (400 MHz, DMSO) δ 11.56 (s, 1H), 11.26 (s, 1H), 11.02 (s, 1H), 8.10 (dd, *J* = 8.8, 5.2 Hz, 1H), 7.72 - 7.55 (m, 3H), 7.26 (t, *J* = 8.9 Hz, 1H), 5.11 (dd, *J* = 13.2, 5.1 Hz, 1H), 4.60 - 4.38 (m, 4H), 4.12 (s, 2H), 3.62 - 3.48 (m, 6H), 3.32 - 3.28 (m, 2H), 3.07 - 2.87 (m, 3H), 2.70 - 2.55 (m, 2H), 2.45 - 2.31 (m, 3H), 2.24 (d, *J* = 18.5 Hz, 2H), 2.05 - 1.97 (m, 1H). LCMS (ESI) calcd. for C₃₀H₃₃F₂N₆O₄ ⁺ [M+H]⁺: 579.25, found, 579.3.

### Example 150: Preparation of 3-(4-((4-(4-(6-fluorobenzo[d]isoxazol-3-yl)piperazin-1-yl)piperidin-1-yl)methyl)-1-oxoisoindolin-2-yl)piperidine-2,6-dione (GT-09644)

With reference to the method of Scheme 1, the target compound (GT-09644) was obtained as a white solid (14 mg, yield 23 %). ¹H NMR (400 MHz, DMSO) δ 11.07 (s, 1H), 10.99 (s, 1H), 8.10 (dd, *J* = 8.9, 5.2 Hz, 1H), 7.94 (s, 1H), 7.84 (d, *J* = 7.2 Hz, 1H), 7.68 - 7.56 (m, 3H), 7.31 - 7.22 (m, 1H), 5.25 - 5.16 (m, 1H), 4.79 (d, *J* = 17.6 Hz, 1H), 4.62 (s, 1H), 4.55 - 4.42 (m, 2H), 4.36 (d, *J* = 17.3 Hz, 2H), 4.12 (s, 2H), 3.62 - 3.55 (m, 4H), 3.11 (s, 2H), 3.01 - 2.86 (m, 2H), 2.65 (d, *J* = 17.0 Hz, 2H), 2.39 - 2.29 (m, 3H), 2.23 (s, 2H), 2.12 - 1.94 (m, 2H). LCMS (ESI) calcd. for C₃₀H₃₄FN₆O₄ ⁺ [M+H]⁺: 561.26, found, 561.3.

### Example 151: Preparation of 3-(6-((4-(4-(6-fluorobenzo[d]isoxazol-3-yl)piperazin-1-yl)piperidin-1-yl)methyl)-1-oxoisoindolin-2-yl)piperidine-2,6-dione (GT-09645)

With reference to the method of Scheme 1, the target compound (GT-09645) was obtained as a white solid (12 mg, yield 20 %). ¹H NMR (400 MHz, DMSO) δ 11.47 (s, 1H), 11.02 (s, 1H), 10.83 (s, 1H), 8.09 (dd, *J* = 9.0, 5.3 Hz, 1H), 8.03 - 7.96 (m, 1H), 7.85 (d, *J* = 7.6 Hz, 1H), 7.73 (d, *J* = 7.7 Hz, 1H), 7.62 (d, *J* = 9.1 Hz, 1H), 7.26 (t, *J* = 8.3 Hz, 1H), 5.13 (dd, *J* = 13.3, 5.1 Hz, 1H), 4.54 - 4.37 (m, 4H), 4.11 (s, 1H), 3.53 (d, *J* = 22.9 Hz, 6H), 3.28 - 3.13 (m, 3H), 3.01 - 2.87 (m, 4H), 2.62 (d, *J* = 16.2 Hz, 2H), 2.40 - 2.31 (m, 2H), 2.16 (s, 2H), 2.05 - 1.99 (m, 1H). LCMS (ESI) calcd. for C₃₀H₃₄FN₆O₄ ⁺ [M+H]⁺: 561.26, found, 561.3.

### Example 152: Preparation of 3-(7-((4-(4-(6-fluorobenzo[d]isoxazol-3-yl)piperazin-1-yl)piperidin-1-yl)methyl)-1-oxoisoindolin-2-yl)piperidine-2,6-dione (GT-09646)

With reference to the method of Scheme 1, the target compound (GT-09646) was obtained as a white solid (14 mg, yield 23 %). ¹H NMR (400 MHz, DMSO) δ 11.78 (s, 1H), 11.03 (s, 1H), 10.65 (s, 1H), 8.10 (dd, *J* = 8.9, 5.2 Hz, 1H), 7.81 - 7.70 (m, 3H), 7.62 (dd, *J* = 9.1, 2.2 Hz, 1H), 7.29 - 7.23 (m, 1H), 5.17 - 5.08 (m, 1H), 4.86 (d, *J* = 12.1 Hz, 1H), 4.73 (d, *J* = 12.8 Hz, 1H), 4.47 (dd, *J* = 42.8, 17.9 Hz, 2H), 4.11 (d, *J* = 12.9 Hz, 1H), 3.65 - 3.53 (m, 6H), 3.34 - 3.23 (m, 4H), 3.14 - 3.04 (m, 2H), 2.92 - 2.85 (m, 1H), 2.63 (d, *J* = 17.6 Hz, 1H), 2.45 - 2.31 (m, 3H), 2.17 (s, 2H), 2.07 - 1.98 (m, 1H). LCMS (ESI) calcd. for C₃₀H₃₄FN₆O₄ ⁺ [M+H]⁺: 561.26, found, 561.3.

### Example 153: Preparation of 3-(5-(2-(4-(4-(6-fluorobenzo[d]isoxazol-3-yl)piperazin-1-yl)piperidin-1-yl)ethyl)-1-oxoisoindolin-2-yl)piperidine-2,6-dione (GT-09647)

With reference to the method of Scheme 1, the target compound (GT-09647) was obtained as a white solid (15 mg, yield 24 %). ¹H NMR (400 MHz, DMSO) δ 11.68 (s, 1H), 11.00 (s, 1H), 10.92 (s, 1H), 8.11 (dd, *J* = 8.8, 5.2 Hz, 1H), 7.72 (d, *J* = 7.8 Hz, 1H), 7.64 - 7.59 (m, 1H), 7.54 (s, 1H), 7.45 (d, *J* = 7.7 Hz, 1H), 7.31 - 7.22 (m, 1H), 5.12 (dd, *J* = 13.2, 5.1 Hz, 1H), 4.46 (d, *J* = 17.4 Hz, 1H), 4.33 (d, *J* = 17.4 Hz, 1H), 4.13 (s, 1H), 3.75 (d, *J* = 11.7 Hz, 2H), 3.70 - 3.48 (m, 5H), 3.36 (s, 5H), 3.25 - 3.20 (m, 2H), 3.10 - 2.96 (m, 2H), 2.94 - 2.85 (m, 1H), 2.61 (d, *J* = 16.6 Hz, 1H), 2.47 - 2.34 (m, 3H), 2.28 - 2.09 (m, 2H), 2.05 - 1.94 (m, 1H). LCMS (ESI) calcd. for C₃₁H₃₆FN₆O₄ ⁺ [M+H]⁺: 575.28, found, 575.3.

### Example 154: Preparation of 3-(5-(3-(4-(4-(6-fluorobenzo[d]isoxazol-3-yl)piperazin-1-yl)piperidin-1-yl)propyl)-1-oxoisoindolin-2-yl)piperidine-2,6-dione (GT-09648)

With reference to the method of Scheme 1, the target compound (GT-09648) was obtained as a white solid (18 mg, yield 15 %). ¹H NMR (400 MHz, DMSO) δ 11.62 (s, 1H), 10.99 (s, 1H), 10.67 (s, 1H), 8.10 (dd, *J* = 8.9, 5.2 Hz, 1H), 7.69 (d, *J* = 7.8 Hz, 1H), 7.65 - 7.58 (m, 1H), 7.50 (s, 1H), 7.41 (d, *J* = 7.8 Hz, 1H), 7.30 - 7.22 (m, 1H), 5.11 (dd, *J* = 13.2, 5.0 Hz, 1H), 4.44 (d, *J* = 17.4 Hz, 1H), 4.31 (d, *J* = 17.3 Hz, 1H), 4.16 - 4.09 (m, 1H), 3.70 - 3.52 (m, 6H), 3.41 (s, 2H), 3.30 - 3.24 (m, 2H), 3.05 (s, 2H), 2.99 - 2.86 (m, 3H), 2.78 (t, *J* = 7.4 Hz, 2H), 2.61 (d, *J* = 17.2 Hz, 1H), 2.45 - 2.31 (m, 3H), 2.29 - 2.14 (m, 2H), 2.13 - 2.05 (m, 2H), 2.04 - 1.96 (m, 1H). LCMS (ESI) calcd. for C₃₂H₃₈FN₆O₄ ⁺ [M+H]⁺: 589.29, found, 589.3.

### Example 155: Preparation of 2-(2,6-dioxopiperidin-3-yl)-5-((4-(4-(6-fluorobenzo[d]isoxazol-3-yl)piperazin-1-yl)piperidin-1-yl)methyl)isoindoline-1,3-dione (GT-09649)

With reference to the method of Scheme 1, the target compound (GT-09649) was obtained as a white solid (10 mg, yield 16 %). ¹H NMR (400 MHz, DMSO) δ 11.61 (s, 1H), 11.26 (s, 1H), 11.16 (s, 1H), 8.22 (s, 1H), 8.13 - 7.99 (m, 3H), 7.62 (d, *J* = 9.1 Hz, 1H), 7.26 (t, *J* = 9.0 Hz, 1H), 5.19 (dd, *J* = 12.8, 5.3 Hz, 1H), 4.52 (s, 1H), 4.11 (s, 1H), 3.78 - 3.46 (m, 8H), 3.30 - 3.23 (m, 2H), 3.17 - 3.08 (m, 1H), 3.07 - 2.84 (m, 3H), 2.59 (d, *J* = 23.0, 11.0 Hz, 4H), 2.33 (s, 2H), 2.19 (s, 2H), 2.12 - 2.05 (m, 1H). LCMS (ESI) calcd. for C₃₀H₃₂FN₆O₅ ⁺ [M+H]⁺: 575.24, found, 575.3.

### Example 156: Preparation of 2-(2,6-dioxopiperidin-3-yl)-4-((4-(4-(6-fluorobenzo[d]isoxazol-3-yl)piperazin-1-yl)piperidin-1-yl)methyl)isoindoline-1,3-dione (GT-09650)

With reference to the method of Scheme 1, the target compound (GT-09650) was obtained as a yellow solid (10 mg, yield 16 %). ¹H NMR (400 MHz, DMSO) δ 11.67 (s, 1H), 11.16 (s, 1H), 11.08 (s, 1H), 8.22 - 8.15 (m, 1H), 8.10 (dd, *J* = 8.8, 5.3 Hz, 1H), 8.03 (d, *J* = 7.1 Hz, 1H), 7.97 (t, *J* = 7.5 Hz, 1H), 7.62 (dd, *J* = 9.1, 2.1 Hz, 1H), 7.30 - 7.22 (m, 1H), 5.19 (dd, *J* = 12.7, 5.3 Hz, 1H), 4.71 (s, 2H), 4.10 (s, 2H), 3.58 (s, 6H), 3.31 - 3.20 (m, 3H), 3.18 - 3.03 (m, 2H), 2.94 - 2.85 (m, 1H), 2.66 - 2.56 (m, 2H), 2.40 - 2.30 (m, 2H), 2.30 - 2.11 (m, 2H), 2.12 - 2.02 (m, 1H). LCMS (ESI) calcd. for C₃₀H₃₂FN₆O₅ ⁺ [M+H]⁺: 575.24, found, 575.3.

### Example 157: Preparation of 3-(5-((3,3-difluoro-4-(4-(6-fluorobenzo[d]isoxazol-3-yl)piperazin-1-yl)piperidin-1-yl)methyl)-4-fluoro-1-oxoisoindolin-2-yl)piperidine-2,6-dione (GT-09655)

With reference to the method of Scheme 1, the target compound (GT-09655) was obtained as a white solid (35 mg, yield 59 %). ¹H NMR (400 MHz, DMSO) δ 11.03 (s, 1H), 8.07 (dd, *J* = 8.9, 5.2 Hz, 1H), 7.78 - 7.68 (m, 1H), 7.65 (d, *J* = 7.7 Hz, 1H), 7.58 (dd, *J* = 9.1, 2.2 Hz, 1H), 7.27 - 7.17 (m, 1H), 5.14 (dd, *J* = 13.3, 5.1 Hz, 1H), 4.60 (d, *J* = 17.5 Hz, 1H), 4.43 (d, *J* = 17.5 Hz, 1H), 4.09 (s, 2H), 3.67 - 3.62 (m, 4H), 3.54 - 3.46 (m, 3H), 3.32 (s, 4H), 3.22 (s, 1H), 3.00 - 2.82 (m, 2H), 2.61 (d, *J* = 17.2 Hz, 1H), 2.48 - 2.39 (m, 1H), 2.34 - 2.23 (m, 1H), 2.16 - 2.06 (m, 1H), 2.05 - 1.97 (m, 1H). LCMS (ESI) calcd. for C₃₀H₃₁F₄N₆O₄ ⁺ [M+H]⁺: 615.23, found, 615.3.

### Example 158: Preparation of 3-(5-((3,3-difluoro-4-(4-(6-fluorobenzo[d]isoxazol-3-yl)piperazin-1-yl)piperidin-1-yl)methyl)-6-fluoro-1-oxoisoindolin-2-yl)piperidine-2,6-dione (GT-09656)

With reference to the method of Scheme 1, the target compound (GT-09656) was obtained as a white solid (26 mg, yield 44 %). ¹H NMR (400 MHz, DMSO) δ 11.02 (s, 1H), 8.07 (dd, *J* = 8.9, 5.2 Hz, 1H), 7.77 (d, *J* = 4.9 Hz, 1H), 7.64 - 7.52 (m, 2H), 7.23 (t, *J* = 9.1, 2.2 Hz, 1H), 5.14 (dd, *J* = 13.3, 5.1 Hz, 1H), 4.49 (d, *J* = 17.3 Hz, 1H), 4.36 (d, *J* = 17.5 Hz, 1H), 4.03 (s, 2H), 3.69 (s, 6H), 3.33 - 3.23 (m, 4H), 3.18 (s, 1H), 3.00 - 2.83 (m, 2H), 2.68 - 2.54 (m, 2H), 2.45 - 2.36 (m, 1H), 2.30 - 2.17 (m, 1H), 2.10 - 1.98 (m, 2H). LCMS (ESI) calcd. for C₃₀H₃₁F₄N₆O₄ ⁺ [M+H]⁺: 615.23, found, 615.3.

### Example 159: Preparation of 3-(5-((3,3-difluoro-4-(4-(6-fluorobenzo[d]isoxazol-3-yl)piperazin-1-yl)piperidin-1-yl)methyl)-7-fluoro-1-oxoisoindolin-2-yl)piperidine-2,6-dione (GT-09657)

With reference to the method of Scheme 1, the target compound (GT-09657) was obtained as a white solid (28 mg, yield 47 %). ¹H NMR (400 MHz, DMSO) δ 11.02 (s, 1H), 8.07 (dd, *J* = 8.9, 5.3 Hz, 1H), 7.58 (dd, *J* = 9.1, 2.1 Hz, 1H), 7.52 (s, 1H), 7.43 (d, *J* = 9.4 Hz, 1H), 7.23 (t, *J* = 9.0, 2.1 Hz, 1H), 5.10 (dd, *J* = 13.3, 5.1 Hz, 1H), 4.52 (d, *J* = 17.9 Hz, 1H), 4.39 (d, *J* = 18.0 Hz, 1H), 4.11 (s, 2H), 3.66 (d, *J* = 13.0 Hz, 8H), 3.27 (s, 5H), 2.96 - 2.87 (m, 1H), 2.61 (d, *J* = 17.2 Hz, 1H), 2.44 - 2.34 (m, 1H), 2.27 (s, 1H), 2.18 - 2.07 (m, 1H), 2.05 - 1.97 (m, 1H). LCMS (ESI) calcd. for C₃₀H₃₁F₄N₆O₄ ⁺ [M+H]⁺: 615.23, found, 615.3.

### Example 160: Preparation of 3-(4-((3,3-difluoro-4-(4-(6-fluorobenzo[d]isoxazol-3-yl)piperazin-1-yl)piperidin-1-yl)methyl)-1-oxoisoindolin-2-yl)piperidine-2,6-dione (GT-09658)

With reference to the method of Scheme 1, the target compound (GT-09658) was obtained as a white solid (36 mg, yield 62 %). ¹H NMR (400 MHz, DMSO) δ 11.04 (s, 1H), 8.07 (dd, *J* = 8.9, 5.2 Hz, 1H), 7.83 - 7.65 (m, 2H), 7.62 - 7.52 (m, 2H), 7.23 (t, *J* = 9.0, 2.0 Hz, 1H), 5.16 (dd, *J* = 13.2, 5.1 Hz, 1H), 4.68 - 4.54 (m, 1H), 4.43 (d, *J* = 17.5 Hz, 1H), 3.95 (s, 2H), 3.77 - 3.66 (m, 6H), 3.34 (s, 6H), 3.14 (s, 1H), 2.98 - 2.90 (m, 1H), 2.64 (d, *J* = 17.2 Hz, 1H), 2.43 - 2.34 (m, 1H), 2.27 (s, 1H), 2.14 - 1.98 (m, 2H). LCMS (ESI) calcd. for C₃₀H₃₂F₃N₆O₄ ⁺ [M+H]⁺: 597.24, found, 597.3.

### Example 161: Preparation of 3-(6-((3,3-difluoro-4-(4-(6-fluorobenzo[d]isoxazol-3-yl)piperazin-1-yl)piperidin-1-yl)methyl)-1-oxoisoindolin-2-yl)piperidine-2,6-dione (GT-09659)

With reference to the method of Scheme 1, the target compound (GT-09659) was obtained as a white solid (28 mg, yield 499 %). ¹H NMR (400 MHz, DMSO) δ 11.02 (s, 1H), 8.06 (dd, *J* = 8.9, 5.3 Hz, 1H), 7.95 (s, 1H), 7.82 (d, *J* = 7.6 Hz, 1H), 7.71 (d, *J* = 7.8 Hz, 1H), 7.57 (dd, *J* = 9.1, 2.2 Hz, 1H), 7.21 (t, *J* = 9.1, 2.2 Hz, 1H), 5.15 (dd, *J* = 13.3, 5.1 Hz, 1H), 4.50 (d, *J* = 14.8 Hz, 1H), 4.43 - 4.28 (m, 3H), 3.63 (s, 8H), 3.42 (s, 1H), 3.18 (s, 4H), 2.96 - 2.88 (m, 1H), 2.62 (d, *J* = 16.7 Hz, 1H), 2.48 - 2.36 (m, 1H), 2.35 - 2.15 (m, 2H), 2.05 - 1.98 (m, 1H). LCMS (ESI) calcd. for C₃₀H₃₂F₃N₆O₄ ⁺ [M+H]⁺: 597.24, found, 597.3.

### Example 162: Preparation of 3-(7-((3,3-difluoro-4-(4-(6-fluorobenzo[d]isoxazol-3-yl)piperazin-1-yl)piperidin-1-yl)methyl)-1-oxoisoindolin-2-yl)piperidine-2,6-dione (GT-09660)

With reference to the method of Scheme 1, the target compound (GT-09660) was obtained as a white solid (24 mg, yield 42 %). ¹H NMR (400 MHz, DMSO) δ 11.04 (s, 1H), 8.06 (dd, *J* = 8.9, 5.3 Hz, 1H), 7.72 (s, 3H), 7.57 (dd, *J* = 9.1, 2.2 Hz, 1H), 7.21 (t, *J* = 9.1, 2.2 Hz, 1H), 5.19 - 5.10 (m, 1H), 4.82 (d, *J* = 37.2 Hz, 1H), 4.71 - 4.58 (m, 1H), 4.52 (d, *J* = 17.9 Hz, 1H), 4.47 - 4.38 (m, 1H), 3.65 - 3.55 (m, 6H), 3.45 (s, 2H), 3.15 (s, 4H), 3.03 - 2.84 (m, 2H), 2.63 (d, *J* = 17.0 Hz, 1H), 2.47 - 2.37 (m, 1H), 2.30 - 2.10 (m, 2H), 2.09 - 2.00 (m, 1H). LCMS (ESI) calcd. for C₃₀H₃₂F₃N₆O₄ ⁺ [M+H]⁺: 597.24, found, 597.3.

### Example 163: Preparation of 3-(5-(2-(3,3-difluoro-4-(4-(6-fluorobenzo[d]isoxazol-3-yl)piperazin-1-yl)piperidin-1-yl)ethyl)-1-oxoisoindolin-2-yl)piperidine-2,6-dione (GT-09661)

With reference to the method of Scheme 1, the target compound (GT-09661) was obtained as a white solid (24 mg, yield 41 %). ¹H NMR (400 MHz, DMSO) δ 11.00 (s, 1H), 8.06 (dd, *J* = 8.9, 5.3 Hz, 1H), 7.72 (d, *J* = 7.8 Hz, 1H), 7.61 - 7.50 (m, 2H), 7.44 (d, *J* = 7.8 Hz, 1H), 7.21 (t, *J* = 9.1, 2.2 Hz, 1H), 5.12 (dd, *J* = 13.3, 5.1 Hz, 1H), 4.46 (d, *J* = 17.5 Hz, 1H), 4.33 (d, *J* = 17.4 Hz, 1H), 4.02 (s, 1H), 3.66 (s, 3H), 3.36 (s, 3H), 3.29 - 3.12 (m, 4H), 3.07 (s, 4H), 2.96 - 2.88 (m, 1H), 2.69 - 2.53 (m, 2H), 2.49 - 2.30 (m, 2H), 2.23 (s, 2H), 2.05 - 1.96 (m, 1H). LCMS (ESI) calcd. for C₃₁H₃₄F₃N₆O₄ ⁺ [M+H]⁺: 611.26, found, 611.3.

### Example 164: Preparation of 3-(5-(3-(3,3-difluoro-4-(4-(6-fluorobenzo[d]isoxazol-3-yl)piperazin-1-yl)piperidin-1-yl)propyl)-1-oxoisoindolin-2-yl)piperidine-2,6-dione (GT-09662)

With reference to the method of Scheme 1, the target compound (GT-09662) was obtained as a white solid (47 mg, yield 39 %). ¹H NMR (400 MHz, DMSO) δ 10.99 (s, 1H), 8.05 (dd, *J* = 8.8, 5.3 Hz, 1H), 7.69 (d, *J* = 7.8 Hz, 1H), 7.57 (dd, *J* = 9.1, 2.1 Hz, 1H), 7.50 (s, 1H), 7.41 (d, *J* = 7.8 Hz, 1H), 7.21 (t, *J* = 9.0, 2.1 Hz, 1H), 5.11 (dd, *J* = 13.3, 5.1 Hz, 1H), 4.44 (d, *J* = 17.3 Hz, 1H), 4.31 (d, *J* = 17.3 Hz, 1H), 3.97 (s, 1H), 3.69 (s, 3H), 3.55 (s, 6H), 3.16 - 3.00 (m, 6H), 2.95 - 2.87 (m, 1H), 2.77 (t, *J* = 7.2 Hz, 2H), 2.66 - 2.56 (m, 1H), 2.44 - 2.34 (m, 1H), 2.21 (s, 1H), 2.17 - 2.05 (m, 2H), 2.04 - 1.95 (m, 1H). LCMS (ESI) calcd. for C₃₂H₃₆F₃N₆O₄ ⁺ [M+H]⁺: 625.27, found, 625.3.

### Example 165: Preparation of 5-((3,3-difluoro-4-(4-(6-fluorobenzo[d]isoxazol-3-yl)piperazin-1-yl)piperidin-1-yl)methyl)-2-(2,6-dioxopiperidin-3-yl)isoindoline-1,3-dione (GT-09663)

With reference to the method of Scheme 1, the target compound (GT-09663) was obtained as a white solid (32 mg, yield 54 %). ¹H NMR (400 MHz, DMSO) δ 11.15 (s, 1H), 8.07 (dd, *J* = 8.9, 5.3 Hz, 1H), 8.04 - 7.89 (m, 3H), 7.58 (dd, *J* = 9.1, 2.1 Hz, 1H), 7.22 (t, *J* = 9.1, 2.1 Hz, 1H), 5.18 (dd, *J* = 12.8, 5.3 Hz, 1H), 4.13 (s, 2H), 3.66 (s, 6H), 3.27 (s, 5H), 2.98 - 2.82 (m, 2H), 2.69 - 2.54 (m, 3H), 2.23 (s, 1H), 2.14 - 2.02 (m, 2H). LCMS (ESI) calcd. for C₃₀H₃₀F₃N₆O₅ ⁺ [M+H]⁺: 611.22, found, 611.3.

### Example 166: Preparation of 4-((3,3-difluoro-4-(4-(6-fluorobenzo[d]isoxazol-3-yl)piperazin-1-yl)piperidin-1-yl)methyl)-2-(2,6-dioxopiperidin-3-yl)isoindoline-1,3-dione (GT-09664)

With reference to the method of Scheme 1, the target compound (GT-09664) was obtained as a white solid (34 mg, yield 57 %). ¹H NMR (400 MHz, DMSO) δ 11.15 (s, 1H), 8.08 (dd, *J* = 8.9, 5.2 Hz, 1H), 8.02 - 7.95 (m, 1H), 7.92 (d, *J* = 6.2 Hz, 2H), 7.59 (dd, *J* = 9.1, 2.1 Hz, 1H), 7.23 (t, *J* = 9.1, 2.0 Hz, 1H), 5.19 - 5.14 (m, 1H), 4.29 (s, 2H), 3.84 - 3.71 (m, 6H), 3.37 (s, 6H), 3.16 (s, 1H), 2.93 - 2.87 (m, 1H), 2.66 - 2.56 (m, 2H), 2.34 - 2.24 (m, 1H), 2.15 - 2.03 (m, 2H). LCMS (ESI) calcd. for C₃₀H₃₀F₃N₆O₅ ⁺ [M+H]⁺: 611.22, found, 611.3.

### Example 167: Preparation of 3-(5-((3',3'-difluoro-4-(6-fluorobenzo[d]isoxazol-3-yl)-[1,4'-bipiperidin]-1'-yl)methyl)-4-fluoro-1-oxoisoindolin-2-yl)piperidine-2,6-dione (GT-09665)

With reference to the method of Scheme 1, the target compound (GT-09665) was obtained as a white solid (20 mg, yield 34 %). ¹H NMR (400 MHz, DMSO) δ 11.64 (s, 1H), 11.03 (s, 1H), 8.28 (s, 1H), 7.73 (dd, *J* = 9.0, 2.0 Hz, 1H), 7.68 - 7.62 (m, 2H), 7.34 (t, *J* = 9.1, 2.1 Hz, 1H), 5.14 (dd, *J* = 13.2, 5.0 Hz, 1H), 4.59 (d, *J* = 17.5 Hz, 1H), 4.42 (d, *J* = 17.5 Hz, 1H), 4.09 (s, 1H), 3.93 (s, 2H), 3.73 (s, 2H), 3.51 - 3.32 (m, 4H), 3.11 (d, *J* = 9.7 Hz, 1H), 2.97 - 2.87 (m, 1H), 2.79 - 2.56 (m, 4H), 2.48 - 2.36 (m, 3H), 2.19 (s, 3H), 2.04 - 1.96 (m, 1H). LCMS (ESI) calcd. for C₃₁H₃₂F₄N₅O₄ ⁺ [M+H]⁺: 614.24, found, 614.3.

### Example 168: Preparation of 3-(5-((3',3'-difluoro-4-(6-fluorobenzo[d]isoxazol-3-yl)-[1,4'-bipiperidin]-1'-yl)methyl)-6-fluoro-1-oxoisoindolin-2-yl)piperidine-2,6-dione (GT-09666)

With reference to the method of Scheme 1, the target compound (GT-09666) was obtained as a white solid (20 mg, yield 34 %). ¹H NMR (400 MHz, DMSO) δ 11.50 (s, 1H), 11.02 (s, 1H), 8.27 (s, 1H), 7.76 - 7.66 (m, 2H), 7.57 (d, *J* = 8.7 Hz, 1H), 7.34 (t, *J* = 9.1, 2.1 Hz, 1H), 5.13 (dd, *J* = 13.2, 5.0 Hz, 1H), 4.49 (d, *J* = 17.2 Hz, 1H), 4.35 (d, *J* = 17.4 Hz, 1H), 4.06 (s, 1H), 3.89 (s, 2H), 3.79 - 3.70 (m, 2H), 3.55 - 3.30 (m, 6H), 3.08 (s, 1H), 2.96 - 2.85 (m, 1H), 2.75 - 2.56 (m, 3H), 2.43 - 2.34 (m, 2H), 2.20 (s, 2H), 2.14 - 1.98 (m, 2H). LCMS (ESI) calcd. for C₃₁H₃₂F₄N₅O₄ ⁺ [M+H]⁺: 614.24, found, 614.3.

### Example 169: Preparation of 3-(5-((3',3'-difluoro-4-(6-fluorobenzo[d]isoxazol-3-yl)-[1,4'-bipiperidin]-1'-yl)methyl)-7-fluoro-1-oxoisoindolin-2-yl)piperidine-2,6-dione (GT-09667)

With reference to the method of Scheme 1, the target compound (GT-09667) was obtained as a white solid (16 mg, yield 28 %). ¹H NMR (400 MHz, DMSO) δ 11.56 (s, 1H), 11.02 (s, 1H), 8.28 (s, 1H), 7.73 (dd, *J* = 9.1, 2.0 Hz, 1H), 7.46 (s, 1H), 7.38 - 7.26 (m, 2H), 5.09 (dd, *J* = 13.3, 5.1 Hz, 1H), 4.51 (d, *J* = 17.9 Hz, 1H), 4.37 (d, *J* = 17.9 Hz, 1H), 4.07 (s, 1H), 3.90 (s, 3H), 3.75 (s, 2H), 3.53 - 3.33 (m, 5H), 3.11 (s, 1H), 2.98 - 2.86 (m, 1H), 2.75 - 2.57 (m, 3H), 2.44 - 2.32 (m, 2H), 2.28 - 2.12 (m, 3H), 2.05 - 1.96 (m, 1H). LCMS (ESI) calcd. for C₃₁H₃₂F₄N₅O₄ ⁺ [M+H]⁺: 614.24, found, 614.3.

### Example 170: Preparation of 3-(4-((3',3'-difluoro-4-(6-fluorobenzo[d]isoxazol-3-yl)-[1,4'-bipiperidin]-1'-yl)methyl)-1-oxoisoindolin-2-yl)piperidine-2,6-dione (GT-09668)

With reference to the method of Scheme 1, the target compound (GT-09668) was obtained as a white solid (23 mg, yield 40 %). ¹H NMR (400 MHz, DMSO) δ 11.58 (s, 1H), 11.02 (s, 1H), 8.28 (s, 1H), 7.77 - 7.67 (m, 2H), 7.62 (d, *J* = 7.2 Hz, 1H), 7.54 (t, *J* = 7.5 Hz, 1H), 7.34 (t, *J* = 9.0, 1.9 Hz, 1H), 5.15 (dd, *J* = 13.2, 5.1 Hz, 1H), 4.63 - 4.50 (m, 1H), 4.40 (d, *J* = 17.5 Hz, 1H), 4.13 (s, 1H), 3.85 - 3.69 (m, 5H), 3.48 - 3.42 (m, 2H), 3.26 (s, 1H), 3.04 (s, 1H), 3.00 - 2.89 (m, 1H), 2.81 - 2.70 (m, 1H), 2.69 - 2.56 (m, 3H), 2.48 - 2.32 (m, 3H), 2.21 (s, 2H), 2.14 - 1.97 (m, 2H). LCMS (ESI) calcd. for C₃₁H₃₃F₃N₅O₄ ⁺ [M+H]⁺: 596.25, found, 596.3.

### Example 171: Preparation of 3-(6-((3',3'-difluoro-4-(6-fluorobenzo[d]isoxazol-3-yl)-[1,4'-bipiperidin]-1'-yl)methyl)-1-oxoisoindolin-2-yl)piperidine-2,6-dione (GT-09669)

With reference to the method of Scheme 1, the target compound (GT-09669) was obtained as a white solid (19 mg, yield 34 %). ¹H NMR (400 MHz, DMSO) δ 11.01 (s, 1H), 8.24 (s, 1H), 7.82 (s, 1H), 7.76 - 7.63 (m, 3H), 7.38 - 7.29 (m, 1H), 5.14 (dd, *J* = 13.3, 5.1 Hz, 1H), 4.49 (d, *J* = 17.5 Hz, 1H), 4.36 (d, *J* = 17.5 Hz, 1H), 4.06 (s, 3H), 3.73 - 3.63 (m, 5H), 3.25 (s, 2H), 2.99 - 2.84 (m, 2H), 2.68 - 2.53 (m, 4H), 2.48 - 2.38 (m, 2H), 2.22 (s, 3H), 2.06 - 1.98 (m, 1H). LCMS (ESI) calcd. for C₃₁H₃₃F₃N₅O₄ ⁺ [M+H]⁺: 596.25, found, 596.3.

### Example 172: Preparation of 3-(7-((3',3'-difluoro-4-(6-fluorobenzo[d]isoxazol-3-yl)-[1,4'-bipiperidin]-1'-yl)methyl)-1-oxoisoindolin-2-yl)piperidine-2,6-dione (GT-09670)

With reference to the method of Scheme 1, the target compound (GT-09670) was obtained as a white solid (26 mg, yield 45 %). ¹H NMR (400 MHz, DMSO) δ 11.45 (s, 1H), 11.02 (s, 1H), 8.24 (s, 1H), 7.72 (dd, *J* = 9.1, 2.0 Hz, 1H), 7.70 - 7.64 (m, 1H), 7.64 - 7.54 (m, 2H), 7.34 (t, *J* = 9.2, 2.1 Hz, 1H), 5.11 (d, *J* = 10.9 Hz, 1H), 4.56 - 4.27 (m, 4H), 4.12 (s, 1H), 3.75 - 3.58 (m, 5H), 3.36 - 3.07 (m, 4H), 2.98 - 2.77 (m, 2H), 2.62 (d, *J* = 17.3 Hz, 2H), 2.45 - 2.32 (m, 2H), 2.20 (s, 3H), 2.08 - 1.97 (m, 1H). LCMS (ESI) calcd. for C₃₁H₃₃F₃N₅O₄ ⁺ [M+H]⁺: 596.25, found, 596.3.

**Example 173: Preparation of 3-(5-(2-(3',3'-difluoro-4-(6-fluorobenzo[d]isoxazol-3-yl)-[1,4'-bipiperidin]-1'-yl)ethyl)-1-oxoisoindolin-2-yl)piperidine-2,6-dione (GT-09671)**

With reference to the method of Scheme 1, the target compound (GT-09671) was obtained as a white solid (12 mg, yield 21 %). ¹H NMR (400 MHz, DMSO) δ 10.99 (s, 1H), 8.20 (s, 1H), 7.78 - 7.64 (m, 2H), 7.52 (s, 1H), 7.43 (d, *J* = 8.0 Hz, 1H), 7.34 (t, *J* = 9.1, 2.1 Hz, 1H), 5.17 - 5.06 (m, 1H), 4.45 (d, *J* = 17.4 Hz, 1H), 4.32 (d, *J* = 17.3 Hz, 1H), 3.70 - 3.54 (m, 5H), 3.36 - 3.19 (m, 6H), 3.10 (s, 3H), 2.95 - 2.88 (m, 1H), 2.61 (d, *J* = 17.0 Hz, 2H), 2.48 - 2.31 (m, 3H), 2.22 (s, 3H), 2.07 - 1.97 (m, 1H). LCMS (ESI) calcd. for C₃₂H₃₅F₃N₅O₄ ⁺ [M+H]⁺: 610.26, found, 610.3.

### Example 174: Preparation of 3-(5-(3-(3',3'-difluoro-4-(6-fluorobenzo[d]isoxazol-3-yl)-[1,4'-bipiperidin]-1'-yl)propyl)-1-oxoisoindolin-2-yl)piperidine-2,6-dione (GT-09672)

With reference to the method of Scheme 1, the target compound (GT-09672) was obtained as a white solid (35 mg, yield 29 %). ¹H NMR (400 MHz, DMSO) δ 10.99 (s, 1H), 8.14 (s, 1H), 7.76 - 7.62 (m, 2H), 7.50 (s, 1H), 7.41 (d, *J* = 7.9 Hz, 1H), 7.33 (t, *J* = 9.1, 1.9 Hz, 1H), 5.11 (dd, *J* = 13.2, 5.1 Hz, 1H), 4.44 (d, *J* = 17.3 Hz, 1H), 4.31 (d, *J* = 17.3 Hz, 1H), 4.00 (s, 2H), 3.71 - 3.54 (m, 6H), 3.03 (s, 3H), 2.98 - 2.83 (m, 2H), 2.77 (t, *J* = 7.4 Hz, 2H), 2.61 (d, *J* = 17.6 Hz, 2H), 2.48 - 2.28 (m, 4H), 2.21 (s, 2H), 2.12 - 1.95 (m, 3H). LCMS (ESI) calcd. for C₃₃H₃₇F₃N₅O₄ ⁺ [M+H]⁺: 624.28, found, 624.3.

### Example 175: Preparation of 5-((3',3'-difluoro-4-(6-fluorobenzo[d]isoxazol-3-yl)-[1,4'-bipiperidin]-1'-yl)methyl)-2-(2,6-dioxopiperidin-3-yl)isoindoline-1,3-dione (GT-09673)

With reference to the method of Scheme 1, the target compound (GT-09673) was obtained as a white solid (13 mg, yield 22 %). ¹H NMR (400 MHz, DMSO) δ 11.14 (s, 2H), 8.23 (s, 1H), 7.99 - 7.88 (m, 2H), 7.84 (d, *J* = 7.6 Hz, 1H), 7.73 (dd, *J* = 9.1, 2.0 Hz, 1H), 7.34 (t, *J* = 9.1, 2.1 Hz, 1H), 5.17 (dd, *J* = 12.9, 5.4 Hz, 1H), 4.07 (s, 1H), 3.89 (s, 2H), 3.72 (s, 2H), 3.63 - 3.47 (m, 3H), 3.32 - 3.20 (m, 2H), 3.03 (s, 1H), 2.94 - 2.85 (m, 1H), 2.66 - 2.54 (m, 3H), 2.48 - 2.33 (m, 3H), 2.22 (s, 2H), 2.13 - 2.01 (m, 2H). LCMS (ESI) calcd. for C₃₁H₃₁F₃N₅O₅ ⁺ [M+H]⁺: 610.23, found, 610.3.

### Example 176: Preparation of 4-((3',3'-difluoro-4-(6-fluorobenzo[d]isoxazol-3-yl)-[1,4'-bipiperidin]-1'-yl)methyl)-2-(2,6-dioxopiperidin-3-yl)isoindoline-1,3-dione (GT-09674)

With reference to the method of Scheme 1, the target compound (GT-09674) was obtained as a white solid (11 mg, yield 19 %). ¹H NMR (400 MHz, DMSO) δ 11.14 (s, 2H), 8.23 (s, 1H), 7.92 - 7.83 (m, 3H), 7.73 (dd, *J* = 9.1, 2.0 Hz, 1H), 7.34 (t, *J* = 9.1, 2.1 Hz, 1H), 5.15 (dd, *J* = 12.7, 5.4 Hz, 1H), 4.21 - 4.10 (m, 2H), 3.68 (s, 2H), 3.64 - 3.47 (m, 3H), 3.32 - 3.26 (m, 2H), 3.04 (s, 1H), 2.95 - 2.82 (m, 2H), 2.69 - 2.54 (m, 3H), 2.47 - 2.33 (m, 3H), 2.22 (s, 2H), 2.12 - 1.96 (m, 2H). LCMS (ESI) calcd. for C₃₁H₃₁F₃N₅O₅ ⁺ [M+H]⁺: 610.23, found, 610.3.

### Example 177: Preparation of 3-(5-(4-(4-benzhydrylpiperazin-1-yl)-3,3-difluoropiperidine-1-carbonyl)-1-oxoisoindolin-2-yl)piperidine-2,6-dione (GT-09675)

With reference to the method of Scheme 4, the target compound (GT-09675) was obtained as a white solid (16 mg, yield 43 %). ¹H NMR (400 MHz, DMSO) δ 11.75 (s, 1H), 11.01 (s, 1H), 7.87 (d, *J* = 7.0 Hz, 4H), 7.80 (d, *J* = 7.8 Hz, 1H), 7.63 (s, 1H), 7.49 (d, *J* = 7.7 Hz, 1H), 7.44 (t, *J* = 7.5 Hz, 4H), 7.36 (t, *J* = 7.3 Hz, 2H), 5.60 (s, 1H), 5.14 (dd, *J* = 13.3, 5.0 Hz, 1H), 4.76 - 4.48 (m, 2H), 4.42 - 4.34 (m, 1H), 3.36 - 3.21 (m, 4H), 3.15 - 3.00 (m, 6H), 2.99 - 2.88 (m, 2H), 2.61 (d, *J* = 16.8 Hz, 1H), 2.45 - 2.33 (m, 1H), 2.04 - 1.97 (m, 1H), 1.92 - 1.61 (m, 2H), 1.26 - 1.09 (m, 1H). LCMS (ESI) calcd. for C₃₆H₃₈F₂N₅O₄ ⁺ [M+H]⁺: 642.29, found, 642.3.

### Example 178: Preparation of 3-(5-(4-(4-benzhydrylpiperazin-1-yl)-3,3-difluoropiperidine-1-carbonyl)-7-fluoro-1-oxoisoindolin-2-yl)piperidine-2,6-dione (GT-09676)

With reference to the method of Scheme 4, the target compound (GT-09676) was obtained as a white solid (10 mg, yield 26 %). ¹H NMR (400 MHz, DMSO) δ 11.69 (s, 1H), 11.02 (s, 1H), 7.87 (d, *J* = 6.6 Hz, 4H), 7.44 (t, *J* = 7.5 Hz, 5H), 7.40 - 7.30 (m, 3H), 5.61 (d, *J* = 8.1 Hz, 1H), 5.10 (dd, *J* = 13.3, 5.1 Hz, 1H), 4.53 (d, *J* = 18.3 Hz, 1H), 4.40 (d, *J* = 18.1, 4.2 Hz, 1H), 3.83 - 3.58 (m, 2H), 3.31 - 3.17 (m, 4H), 3.15 - 3.09 (m, 2H), 3.07 - 2.95 (m, 5H), 2.60 (d, *J* = 16.6 Hz, 1H), 2.43 - 2.30 (m, 1H), 2.05 - 1.96 (m, 1H), 1.92 - 1.66 (m, 2H). LCMS (ESI) calcd. for C₃₆H₃₇F₃N₅O₄ ⁺ [M+H]⁺: 660.28, found, 660.3.

### Example 179: Preparation of 3-(6-(4-(4-benzhydrylpiperazin-1-yl)-3,3-difluoropiperidine-1-carbonyl)-1-oxoisoindolin-2-yl)piperidine-2,6-dione (GT-09677)

With reference to the method of Scheme 4, the target compound (GT-09677) was obtained as a white solid (6 mg, yield 17 %). ¹H NMR (400 MHz, DMSO) δ 11.44 (s, 1H), 11.01 (s, 1H), 7.83 (d, *J* = 7.1 Hz, 4H), 7.72 (d, *J* = 7.8 Hz, 1H), 7.68 - 7.61 (m, 2H), 7.44 (t, *J* = 7.4 Hz, 4H), 7.37 (t, *J* = 7.1 Hz, 2H), 5.61 (d, *J* = 8.9 Hz, 1H), 5.13 (dd, *J* = 13.3, 5.1 Hz, 1H), 4.53 (d, *J* = 17.9 Hz, 1H), 4.39 (d, *J* = 17.8 Hz, 1H), 3.30 - 3.09 (m, 7H), 3.08 - 2.98 (m, 5H), 2.98 - 2.84 (m, 2H), 2.61 (d, *J* = 17.1 Hz, 1H), 2.47 - 2.36 (m, 1H), 2.05 - 1.98 (m, 1H), 1.77 (s, 2H). LCMS (ESI) calcd. for C₃₆H₃₈F₂N₅O₄ ⁺ [M+H]⁺: 642.29, found, 642.3.

### Example 180: Preparation of 3-(4-(4-(4-benzhydrylpiperazin-1-yl)-3,3-difluoropiperidine-1-carbonyl)-1-oxoisoindolin-2-yl)piperidine-2,6-dione (GT-09678)

With reference to the method of Scheme 4, the target compound (GT-09678) was obtained as a white solid (13 mg, yield 35 %). ¹H NMR (400 MHz, DMSO) δ 11.77 (s, 1H), 10.98 (d, *J* = 4.7 Hz, 1H), 7.90 - 7.84 (m, 4H), 7.66 - 7.56 (m, 2H), 7.44 (t, *J* = 7.5 Hz, 4H), 7.36 (t, *J* = 7.3 Hz, 2H), 5.61 (d, *J* = 8.2 Hz, 1H), 5.19 - 5.07 (m, 1H), 4.72 - 4.15 (m, 3H), 3.70 - 3.57 (m, 2H), 3.38 - 3.20 (m, 4H), 3.14 - 2.95 (m, 6H), 2.89 (t, 1H), 2.57 (d, *J* = 17.7 Hz, 1H), 2.47 - 2.33 (m, 1H), 2.03 - 1.95 (m, 1H), 1.94 - 1.67 (m, 2H). LCMS (ESI) calcd. for C₃₆H₃₈F₂N₅O₄ ⁺ [M+H]⁺: 642.29, found, 642.4.

### Example 181: Preparation of 3-(5-(4-(4-benzhydrylpiperazin-1-yl)-3,3-difluoropiperidine-1-carbonyl)-6-fluoro-1-oxoisoindolin-2-yl)piperidine-2,6-dione (GT-09710)

With reference to the method of Scheme 4, the target compound (GT-09710) was obtained as a white solid (7 mg, yield 19 %). ¹H NMR (400 MHz, DMSO) δ 11.46 (s, 1H), 11.02 (s, 1H), 7.84 (d, *J* = 6.6 Hz, 4H), 7.67 (d, *J* = 7.5 Hz, 1H), 7.44 (t, *J* = 7.4 Hz, 4H), 7.37 (t, *J* = 7.3 Hz, 2H), 5.60 (d, *J* = 8.9 Hz, 1H), 5.14 (dd, *J* = 13.3, 4.7 Hz, 1H), 4.81 - 4.54 (m, 1H), 4.42 (dd, *J* = 48.5, 17.2 Hz, 2H), 3.62 - 3.45 (m, 2H), 3.30 - 3.17 (m, 4H), 3.16 - 3.10 (m, 2H), 3.07 - 2.98 (m, 4H), 2.94 - 2.87 (m, 1H), 2.60 (d, *J* = 17.7 Hz, 1H), 2.49 - 2.29 (m, 2H), 2.05 - 1.98 (m, 1H), 1.76 - 1.70 (m, 1H). LCMS (ESI) calcd. for C₃₆H₃₇F₃N₅O₄ ⁺ [M+H]⁺: 660.28, found, 660.3.

### Example 182: Preparation of 3-(7-(4-(4-benzhydrylpiperazin-1-yl)-3,3-difluoropiperidine-1-carbonyl)-1-oxoisoindolin-2-yl)piperidine-2,6-dione (GT-09711)

With reference to the method of Scheme 4, the target compound (GT-09711) was obtained as a white solid (11 mg, yield 29 %). ¹H NMR (400 MHz, DMSO) δ 11.35 (s, 1H), 10.99 (s, 1H), 7.82 (d, *J* = 6.6 Hz, 4H), 7.70 - 7.66 (m, 2H), 7.44 (t, *J* = 7.3 Hz, 4H), 7.37 (d, *J* = 7.2 Hz, 2H), 7.33 - 7.29 (m, 1H), 5.60 (d, *J* = 9.5 Hz, 1H), 5.09 - 4.96 (m, 1H), 4.79 - 4.65 (m, 1H), 4.54 - 4.38 (m, 2H), 3.36 - 3.22 (m, 4H), 3.22 - 3.08 (m, 6H), 3.02 (s, 4H), 2.94 - 2.82 (m, 2H), 2.61 (d, *J* = 16.5 Hz, 1H), 2.48 - 2.30 (m, 2H), 2.03 - 1.93 (m, 1H), 1.93 - 1.77 (m, 1H), 1.69 - 1.55 (m, 1H). LCMS (ESI) calcd. for C₃₆H₃₈F₂N₅O₄ ⁺ [M+H]⁺: 642.29, found, 642.4.

### Example 183: Preparation of 4-(4-(4-benzhydrylpiperazin-1-yl)-3,3-difluoropiperidine-1-carbonyl)-2-(2,6-dioxopiperidin-3-yl)isoindoline-1,3-dione (GT-09712)

With reference to the method of Scheme 4, the target compound (GT-09712) was obtained as a white solid (8 mg, yield 21 %). ¹H NMR (400 MHz, DMSO) δ 11.29 (s, 1H), 11.17 - 11.05 (m, 1H), 8.01 - 7.91 (m, 2H), 7.85 - 7.64 (m, 5H), 7.45 (t, *J* = 7.4 Hz, 4H), 7.37 (t, *J* = 7.1 Hz, 2H), 5.60 (d, *J* = 9.3 Hz, 1H), 5.13 (dd, *J* = 12.5, 5.3 Hz, 1H), 4.79 - 4.59 (m, 1H), 3.31 - 3.22 (m, 2H), 3.21 - 3.08 (m, 5H), 3.07 - 2.96 (m, 4H), 2.96 - 2.76 (m, 2H), 2.60 (d, *J* = 17.2 Hz, 1H), 2.45 (s, 1H), 2.05 (s, 1H), 1.90 - 1.63 (m, 2H). LCMS (ESI) calcd. for C₃₆H₃₆F₂N₅O₅ ⁺ [M+H]⁺: 656.27, found, 656.3.

### Example 184: Preparation of 5-(4-(4-benzhydrylpiperazin-1-yl)-3,3-difluoropiperidine-1-carbonyl)-2-(2,6-dioxopiperidin-3-yl)isoindoline-1,3-dione (GT-09713)

With reference to the method of Scheme 4, the target compound (GT-09713) was obtained as a white solid (9 mg, yield 24 %). ¹H NMR (400 MHz, DMSO) δ 11.53 (s, 1H), 11.15 (s, 1H), 8.01 (d, *J* = 8.1 Hz, 1H), 7.85 (d, *J* = 7.0 Hz, 6H), 7.44 (t, *J* = 7.4 Hz, 4H), 7.37 (t, *J* = 7.2 Hz, 2H), 5.61 (d, *J* = 9.2 Hz, 1H), 5.18 (dd, *J* = 12.7, 5.4 Hz, 1H), 4.79 - 4.47 (m, 1H), 3.66 - 3.56 (m, 1H), 3.29 - 3.18 (m, 4H), 3.14 - 3.09 (m, 2H), 3.03 (s, 4H), 2.94 - 2.87 (m, 1H), 2.65 - 2.53 (m, 2H), 2.10 - 2.01 (m, 1H), 1.92 - 1.72 (m, 2H). LCMS (ESI) calcd. for C₃₆H₃₆F₂N₅O₅ ⁺ [M+H]⁺: 656.27, found, 656.3.

### Example 185: Preparation of 3-(5-(3,3-difluoro-4-(4-(phenyl(pyridin-2-yl)methyl)piperazin-1-yl)piperidine-1-carbonyl)-1-oxoisoindolin-2-yl)piperidine-2,6-dione (GT-09714)

With reference to the method of Scheme 4, the target compound (GT-09714) was obtained as a white solid (11 mg, yield 29 %). ¹H NMR (400 MHz, DMSO) δ 11.02 (s, 1H), 8.74 (d, *J* = 4.8 Hz, 1H), 7.92 (t, *J* = 12.1, 4.4 Hz, 1H), 7.85 - 7.77 (m, 1H), 7.69 (d, *J* = 7.0 Hz, 2H), 7.64 (d, *J* = 7.6 Hz, 2H), 7.52 - 7.39 (m, 5H), 5.86 (s, 1H), 5.14 (dd, *J* = 13.2, 5.0 Hz, 1H), 4.79 - 4.55 (m, 1H), 4.51 (d, *J* = 17.7 Hz, 1H), 4.39 (d, *J* = 17.6, 4.2 Hz, 1H), 3.96 - 3.84 (m, 2H), 3.44 - 3.27 (m, 2H), 3.18 - 2.99 (m, 8H), 2.95 - 2.88 (m, 1H), 2.61 (d, *J* = 17.2 Hz, 1H), 2.46 - 2.35 (m, 1H), 2.06 - 1.99 (m, 1H), 1.95 - 1.71 (m, 2H). LCMS (ESI) calcd. for C₃₅H₃₇F₂N₆O₄ ⁺ [M+H]⁺: 643.28, found, 643.3.

### Example 186: Preparation of 3-(5-(3,3-difluoro-4-(4-(phenyl(pyridin-2-yl)methyl)piperazin-1-yl)piperidine-1-carbonyl)-7-fluoro-1-oxoisoindolin-2-yl)piperidine-2,6-dione (GT-09715)

With reference to the method of Scheme 4, the target compound (GT-09715) was obtained as a white solid (11 mg, yield 29 %). ¹H NMR (400 MHz, DMSO) δ 11.02 (s, 1H), 8.75 (d, *J* = 4.8 Hz, 1H), 7.91 (t, *J* = 7.8, 6.5 Hz, 1H), 7.66 (d, *J* = 8.2 Hz, 2H), 7.59 (d, *J* = 7.8 Hz, 1H), 7.51 - 7.37 (m, 5H), 7.28 (s, 1H), 5.85 (s, 1H), 5.10 (dd, *J* = 13.3, 4.7 Hz, 1H), 4.76 - 4.47 (m, 2H), 4.39 (d, *J* = 18.0 Hz, 1H), 3.79 - 3.65 (m, 2H), 3.38 - 3.27 (m, 2H), 3.16 - 2.98 (m, 8H), 2.95 - 2.87 (m, 1H), 2.60 (d, *J* = 17.1 Hz, 1H), 2.43 - 2.30 (m, 1H), 2.05 - 1.95 (m, 1H), 1.84 (d, *J* = 60.3 Hz, 2H). LCMS (ESI) calcd. for C₃₅H₃₆F₃N₆O₄ ⁺ [M+H]⁺: 661.27, found, 661.3.

### Example 187: Preparation of 3-(5-(3,3-difluoro-4-(4-(phenyl(pyridin-2-yl)methyl)piperazin-1-yl)piperidine-1-carbonyl)-6-fluoro-1-oxoisoindolin-2-yl)piperidine-2,6-dione (GT-09716)

With reference to the method of Scheme 4, the target compound (GT-09716) was obtained as a white solid (15 mg, yield 39 %). ¹H NMR (400 MHz, DMSO) δ 11.02 (s, 1H), 8.75 (d, *J* = 6.7 Hz, 1H), 7.92 (t, *J* = 7.7, 1.3 Hz, 1H), 7.72 - 7.60 (m, 5H), 7.51 - 7.36 (m, 4H), 5.86 (s, 1H), 5.14 (dd, *J* = 13.2, 4.8 Hz, 1H), 4.75 - 4.58 (m, 1H), 4.49 (d, *J* = 17.6 Hz, 1H), 4.36 (d, *J* = 17.4 Hz, 1H), 3.92 - 3.89 (m, 1H), 3.57 - 3.50 (m, 1H), 3.39 - 3.24 (m, 2H), 3.19 - 2.97 (m, 8H), 2.96 - 2.87 (m, 1H), 2.61 (d, *J* = 17.4 Hz, 1H), 2.46 - 2.35 (m, 1H), 2.08 - 1.98 (m, 1H), 1.96 - 1.63 (m, 2H). LCMS (ESI) calcd. for C₃₅H₃₆F₃N₆O₄ ⁺ [M+H]⁺: 661.27, found, 661.3.

### Example 188: Preparation of 3-(4-(3,3-difluoro-4-(4-(phenyl(pyridin-2-yl)methyl)piperazin-1-yl)piperidine-1-carbonyl)-1-oxoisoindolin-2-yl)piperidine-2,6-dione (GT-09717)

With reference to the method of Scheme 4, the target compound (GT-09717) was obtained as a white solid (13 mg, yield 35 %). ¹H NMR (400 MHz, DMSO) δ 10.98 (d, *J* = 4.6 Hz, 1H), 8.74 (d, *J* = 4.9 Hz, 1H), 7.93 (t, *J* = 7.7 Hz, 1H), 7.85 (d, *J* = 8.6 Hz, 1H), 7.72 - 7.58 (m, 5H), 7.50 - 7.38 (m, 4H), 5.87 (s, 1H), 5.20 - 5.06 (m, 1H), 4.77 - 4.30 (m, 3H), 3.74 - 3.53 (m, 2H), 3.47 - 3.26 (m, 2H), 3.21 - 3.02 (m, 8H), 2.94 - 2.83 (m, 1H), 2.57 (d, *J* = 17.0 Hz, 1H), 2.46 - 2.30 (m, 1H), 2.04 - 1.70 (m, 3H). LCMS (ESI) calcd. for C₃₅H₃₇F₂N₆O₄ ⁺ [M+H]⁺: 643.28, found, 643.3.

### Example 189: Preparation of 3-(6-(3,3-difluoro-4-(4-(phenyl(pyridin-2-yl)methyl)piperazin-1-yl)piperidine-1-carbonyl)-1-oxoisoindolin-2-yl)piperidine-2,6-dione (GT-09718)

With reference to the method of Scheme 4, the target compound (GT-09718) was obtained as a white solid (5 mg, yield 14 %). ¹H NMR (400 MHz, DMSO) δ 11.02 (s, 1H), 8.75 (d, *J* = 4.7 Hz, 1H), 7.91 (t, *J* = 7.7, 3.9 Hz, 1H), 7.74 - 7.61 (m, 6H), 7.48 - 7.33 (m, 4H), 5.86 (s, 1H), 5.13 (dd, *J* = 13.2, 5.1 Hz, 1H), 4.90 - 4.56 (m, 1H), 4.53 (d, *J* = 17.8 Hz, 1H), 4.40 (d, *J* = 17.8 Hz, 1H), 4.00 - 3.83 (m, 2H), 3.41 - 3.26 (m, 2H), 3.17 - 3.00 (m, 8H), 2.96 - 2.87 (m, 1H), 2.61 (d, *J* = 17.1 Hz, 1H), 2.46 - 2.38 (m, 1H), 2.06 - 1.75 (m, 3H). LCMS (ESI) calcd. for C₃₅H₃₇F₂N₆O₄ ⁺ [M+H]⁺: 643.28, found, 643.4.

### Example 190: Preparation of 3-(7-(3,3-difluoro-4-(4-(phenyl(pyridin-2-yl)methyl)piperazin-1-yl)piperidine-1-carbonyl)-1-oxoisoindolin-2-yl)piperidine-2,6-dione (GT-09719)

With reference to the method of Scheme 4, the target compound (GT-09719) was obtained as a white solid (12 mg, yield 32 %). ¹H NMR (400 MHz, DMSO) δ 10.98 (d, *J* = 18.3 Hz, 1H), 8.74 (d, *J* = 4.7 Hz, 1H), 7.90 (t, *J* = 11.0, 5.5 Hz, 1H), 7.73 - 7.64 (m, 4H), 7.61 (d, *J* = 7.5 Hz, 1H), 7.49 - 7.38 (m, 4H), 7.35 - 7.28 (m, 1H), 5.83 (s, 1H), 5.09 - 4.93 (m, 1H), 4.78 - 4.65 (m, 1H), 4.54 - 4.34 (m, 2H), 3.44 - 3.17 (m, 4H), 3.16 - 3.00 (m, 8H), 2.92 - 2.83 (m, 1H), 2.61 (d, *J* = 15.5 Hz, 1H), 2.47 - 2.33 (m, 1H), 2.04 - 1.58 (m, 3H). LCMS (ESI) calcd. for C₃₅H₃₇F₂N₆O₄ ⁺ [M+H]⁺: 643.28, found, 643.3.

### Example 191: Preparation of 4-(3,3-difluoro-4-(4-(phenyl(pyridin-2-yl)methyl)piperazin-1-yl)piperidine-1-carbonyl)-2-(2,6-dioxopiperidin-3-yl)isoindoline-1,3-dione (GT-09720)

With reference to the method of Scheme 4, the target compound (GT-09720) was obtained as a white solid (8 mg, yield 21 %). ¹H NMR (400 MHz, DMSO) δ 11.19 - 11.09 (m, 1H), 11.07 - 10.38 (m, 1H), 8.75 (d, *J* = 6.6 Hz, 1H), 8.02 - 7.88 (m, 3H), 7.77 - 7.64 (m, 3H), 7.61 (d, *J* = 7.7 Hz, 1H), 7.49 - 7.39 (m, 4H), 5.84 (s, 1H), 5.18 - 5.06 (m, 1H), 4.79 - 4.59 (m, 1H), 3.49 - 3.21 (m, 4H), 3.16 - 2.98 (m, 8H), 2.91 - 2.82 (m, 1H), 2.60 (d, *J* = 17.5 Hz, 1H), 2.49 - 2.41 (m, 1H), 2.09 - 1.99 (m, 1H), 1.97 - 1.64 (m, 2H). LCMS (ESI) calcd. for C₃₅H₃₅F₂N₆O₅ ⁺ [M+H]⁺: 657.26, found, 657.3.

### Example 192: Preparation of 5-(3,3-difluoro-4-(4-(phenyl(pyridin-2-yl)methyl)piperazin-1-yl)piperidine-1-carbonyl)-2-(2,6-dioxopiperidin-3-yl)isoindoline-1,3-dione (GT-09721)

With reference to the method of Scheme 4, the target compound (GT-09721) was obtained as a white solid (9 mg, yield 24 %). ¹H NMR (400 MHz, DMSO) δ 11.13 (s, 1H), 8.74 (d, *J* = 4.2 Hz, 1H), 8.01 (d, *J* = 8.1 Hz, 1H), 7.96 - 7.79 (m, 3H), 7.69 (d, *J* = 6.9 Hz, 2H), 7.63 (d, *J* = 7.9 Hz, 1H), 7.49 - 7.37 (m, 4H), 5.85 (s, 1H), 5.18 (dd, *J* = 12.7, 5.4 Hz, 1H), 4.62 (d, *J* = 53.8 Hz, 1H), 3.60 - 3.51 (m, 2H), 3.43 - 3.27 (m, 2H), 3.17 - 3.00 (m, 8H), 2.96 - 2.86 (m, 1H), 2.58 (d, 2H), 2.13 - 2.02 (m, 1H), 2.01 - 1.64 (m, 2H). LCMS (ESI) calcd. for C₃₅H₃₅F₂N₆O₅ ⁺ [M+H]⁺: 657.26, found, 657.3.

### Example 193: Preparation of 3-(5-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-1-oxoisoindolin-2-yl)piperidine-2,6-dione (GT-09722)

With reference to the method of Scheme 4, the target compound (GT-09722) was obtained as a white solid (18 mg, yield 55 %). ¹H NMR (400 MHz, DMSO) δ 11.01 (s, 1H), 7.80 (d, *J* = 12.0 Hz, 1H), 7.64 (s, 1H), 7.51 (d, *J* = 7.7 Hz, 1H), 7.39 (d, *J* = 8.5 Hz, 2H), 7.25 (d, *J* = 8.5 Hz, 2H), 5.14 (dd, *J* = 13.2, 5.0 Hz, 1H), 4.73 - 4.37 (m, 3H), 3.46 - 3.01 (m, 6H), 2.97 - 2.86 (m, 2H), 2.78 (s, 1H), 2.61 (d, *J* = 17.2 Hz, 1H), 2.49 - 2.31 (m, 4H), 2.16 - 1.62 (m, 6H), 1.53 - 1.35 (m, 2H), 0.98 (d, *J* = 14.3 Hz, 6H). LCMS (ESI) calcd. for C₃₈H₄₃ClF₂N₅O₅ ⁺ [M+H]⁺: 722.29, found, 722.3.

### Example 194: Preparation of 3-(5-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-7-fluoro-1-oxoisoindolin-2-yl)piperidine-2,6-dione (GT-09723)

With reference to the method of Scheme 4, the target compound (GT-09723) was obtained as a white solid (6 mg, yield 19 %). ¹H NMR (400 MHz, DMSO) δ 11.02 (s, 1H), 7.45 (s, 1H), 7.38 (d, *J* = 8.4 Hz, 2H), 7.35 - 7.27 (m, 1H), 7.25 (d, *J* = 8.5 Hz, 2H), 5.10 (dd, *J* = 13.3, 5.0 Hz, 1H), 4.68 - 4.35 (m, 3H), 3.37 - 3.08 (m, 6H), 3.07 - 2.85 (m, 3H), 2.81 - 2.55 (m, 4H), 2.43 - 2.34 (m, 3H), 2.14 - 1.98 (m, 2H), 1.91 (d, *J* = 17.4 Hz, 1H), 1.78 - 1.57 (m, 3H), 1.51 - 1.41 (m, 2H), 0.98 (d, *J* = 12.9 Hz, 6H). LCMS (ESI) calcd. for C₃₈H₄₂ClF₃N₅O₅ ⁺ [M+H]⁺: 740.28, found, 740.3.

### Example 195: Preparation of 3-(5-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-6-fluoro-1-oxoisoindolin-2-yl)piperidine-2,6-dione (GT-09724)

With reference to the method of Scheme 4, the target compound (GT-09724) was obtained as a white solid (13 mg, yield 39 %). ¹H NMR (400 MHz, DMSO) δ 11.02 (s, 1H), 7.75 - 7.58 (m, 2H), 7.38 (d, *J* = 8.0 Hz, 2H), 7.25 (d, *J* = 8.3 Hz, 2H), 5.14 (dd, *J* = 13.2, 4.9 Hz, 1H), 4.69 - 4.34 (m, 3H), 3.16 (s, 4H), 3.01 - 2.88 (m, 2H), 2.74 - 2.54 (m, 4H), 2.46 - 2.26 (m, 4H), 2.12 - 2.00 (m, 2H), 1.90 (d, *J* = 16.9 Hz, 1H), 1.73 - 1.54 (m, 2H), 1.50 - 1.39 (m, 2H), 0.98 (d, *J* = 13.3 Hz, 6H). LCMS (ESI) calcd. for C₃₈H₄₂ClF₃N₅O₅ ⁺ [M+H]⁺: 740.28, found, 740.3.

### Example 196: Preparation of 3-(4-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-1-oxoisoindolin-2-yl)piperidine-2,6-dione (GT-09733)

With reference to the method of Scheme 4, the target compound (GT-09733) was obtained as a white solid (14 mg, yield 43 %). ¹H NMR (400 MHz, DMSO) δ 11.00 (d, *J* = 4.1 Hz, 1H), 7.86 (d, *J* = 8.3 Hz, 1H), 7.68 - 7.54 (m, 2H), 7.38 (d, *J* = 8.4 Hz, 2H), 7.25 (d, *J* = 8.5 Hz, 2H), 5.19 - 5.08 (m, 1H), 4.68 - 4.25 (m, 3H), 3.84 - 3.64 (m, 4H), 3.21 (s, 4H), 3.06 (s, 1H), 2.96 - 2.88 (m, 1H), 2.80 - 2.68 (m, 1H), 2.59 (d, *J* = 17.1 Hz, 2H), 2.47 - 2.26 (m, 4H), 2.12 - 1.98 (m, 2H), 1.90 (d, *J* = 17.0 Hz, 1H), 1.79 - 1.60 (m, 2H), 1.51 - 1.38 (m, 2H), 0.98 (d, *J* = 13.8 Hz, 6H). LCMS (ESI) calcd. for C₃₈H₄₃ClF₂N₅O₅ ⁺ [M+H]⁺: 722.29, found, 722.3.

### Example 197: Preparation of 3-(6-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-1-oxoisoindolin-2-yl)piperidine-2,6-dione (GT-09725)

With reference to the method of Scheme 4, the target compound (GT-09725) was obtained as a white solid (15 mg, yield 46 %). ¹H NMR (400 MHz, DMSO) δ 11.02 (s, 1H), 7.73 (d, *J* = 7.7 Hz, 1H), 7.69 - 7.60 (m, 2H), 7.39 (d, *J* = 6.5 Hz, 2H), 7.25 (d, *J* = 8.4 Hz, 2H), 5.14 (dd, *J* = 13.2, 5.0 Hz, 1H), 4.75 - 4.48 (m, 2H), 4.40 (d, *J* = 17.8 Hz, 1H), 3.34 - 3.21 (m, 3H), 3.07 (s, 1H), 3.00 - 2.85 (m, 2H), 2.81 (s, 1H), 2.61 (d, *J* = 16.8 Hz, 2H), 2.45 - 2.34 (m, 3H), 2.15 - 2.01 (m, 2H), 1.90 (d, *J* = 16.9 Hz, 1H), 1.78 - 1.58 (m, 4H), 1.50 - 1.40 (m, 2H), 1.32 - 1.04 (m, 3H), 0.98 (d, *J* = 14.1 Hz, 6H). LCMS (ESI) calcd. for C₃₈H₄₃ClF₂N₅O₅ ⁺ [M+H]⁺: 722.29, found, 722.3.

### Example 198: Preparation of 3-(7-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-1-oxoisoindolin-2-yl)piperidine-2,6-dione (GT-09726)

With reference to the method of Scheme 4, the target compound (GT-09726) was obtained as a white solid (16 mg, yield 49 %). ¹H NMR (400 MHz, DMSO) δ 11.00 (s, 1H), 7.76 - 7.65 (m, 2H), 7.40 - 7.30 (m, 3H), 7.27 - 7.21 (m, 2H), 5.08 - 4.94 (m, 1H), 4.77 - 4.60 (m, 1H), 4.55 - 4.37 (m, 2H), 3.34 - 2.95 (m, 8H), 2.90 - 2.81 (m, 1H), 2.61 (d, 3H), 2.36 (d, 4H), 2.10 - 1.86 (m, 3H), 1.83 - 1.40 (m, 5H), 0.98 (d, *J* = 13.5 Hz, 6H). LCMS (ESI) calcd. for C₃₈H₄₃ClF₂N₅O₅ ⁺ [M+H]⁺: 722.29, found, 722.3.

### Example 199: Preparation of 4-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-2-(2,6-dioxopiperidin-3-yl)isoindoline-1,3-dione (GT-09727)

With reference to the method of Scheme 4, the target compound (GT-09727) was obtained as a white solid (11 mg, yield 33 %). ¹H NMR (400 MHz, DMSO) δ 11.20 - 11.04 (m, 1H), 8.04 - 7.91 (m, 2H), 7.78 - 7.63 (m, 1H), 7.41 - 7.33 (m, 2H), 7.25 (d, *J* = 8.4 Hz, 2H), 5.19 - 5.06 (m, 1H), 4.80 - 4.53 (m, 1H), 3.44 - 3.00 (m, 8H), 2.97 - 2.80 (m, 2H), 2.78 - 2.56 (m, 3H), 2.37 (d, *J* = 16.6 Hz, 3H), 2.15 - 2.00 (m, 2H), 1.98 - 1.50 (m, 4H), 1.50 - 1.36 (m, 2H), 0.98 (d, *J* = 13.9 Hz, 6H). LCMS (ESI) calcd. for C₃₈H₄₁ClF₂N₅O₆ ⁺ [M+H]⁺: 736.27, found, 736.3.

### Example 200: Preparation of 5-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-2-(2,6-dioxopiperidin-3-yl)isoindoline-1,3-dione (GT-09728)

With reference to the method of Scheme 4, the target compound (GT-09728) was obtained as a white solid (11 mg, yield 33 %). ¹H NMR (400 MHz, DMSO) δ 11.15 (s, 1H), 8.01 (d, *J* = 7.4 Hz, 1H), 7.85 (s, 2H), 7.38 (d, *J* = 8.2 Hz, 2H), 7.25 (d, *J* = 8.4 Hz, 2H), 5.19 (dd, *J* = 12.7, 5.4 Hz, 1H), 4.59 (d, *J* = 43.1 Hz, 1H), 3.50 - 3.30 (m, 5H), 3.26 - 3.10 (m, 3H), 3.04 (s, 1H), 2.94 - 2.86 (m, 1H), 2.79 - 2.54 (m, 4H), 2.47 - 2.22 (m, 3H), 2.15 - 2.02 (m, 2H), 1.90 (d, *J* = 16.7 Hz, 1H), 1.80 - 1.62 (m, 2H), 1.50 - 1.37 (m, 2H), 0.98 (d, *J* = 13.6 Hz, 6H). LCMS (ESI) calcd. for C₃₈H₄₁ClF₂N₅O₆ ⁺ [M+H]⁺: 736.27, found, 736.3.

### Example 201: Preparation of 3-(5-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-1-oxoisoindolin-2-yl)piperidine-2,6-dione (GT-09729)

With reference to the method of Scheme 4, the target compound (GT-09729) was obtained as a white solid (10 mg, yield 29 %). ¹H NMR (400 MHz, DMSO) δ 11.01 (s, 1H), 10.02 (s, 1H), 7.81 (d, *J* = 7.8 Hz, 1H), 7.63 (s, 1H), 7.50 (d, *J* = 7.7 Hz, 1H), 7.44 (d, *J* = 8.4 Hz, 2H), 7.14 (d, *J* = 8.4 Hz, 2H), 5.14 (dd, *J* = 13.3, 5.1 Hz, 1H), 4.74 - 4.48 (m, 2H), 4.45 - 4.34 (m, 1H), 3.57 (s, 3H), 3.31 - 3.17 (m, 4H), 3.16 - 2.83 (m, 6H), 2.61 (d, *J* = 15.7 Hz, 3H), 2.46 - 2.36 (m, 1H), 2.32 (s, 2H), 2.02 (s, 3H), 1.88 - 1.60 (m, 2H), 1.46 (t, *J* = 6.2 Hz, 2H), 0.96 (s, 6H). LCMS (ESI) calcd. for C₃₈H₄₅ClF₂N₅O₄ ⁺ [M+H]⁺: 708.31, found, 708.4.

### Example 202: Preparation of 3-(5-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-7-fluoro-1-oxoisoindolin-2-yl)piperidine-2,6-dione (GT-09730)

With reference to the method of Scheme 4, the target compound (GT-09730) was obtained as a white solid (13 mg, yield 36 %). ¹H NMR (400 MHz, DMSO) δ 11.02 (s, 1H), 10.05 (s, 1H), 7.44 (d, *J* = 8.4 Hz, 3H), 7.32 (s, 1H), 7.14 (d, *J* = 8.4 Hz, 2H), 5.11 (dd, *J* = 13.2, 5.1 Hz, 1H), 4.76 - 4.50 (m, 2H), 4.46 - 4.36 (m, 1H), 3.66 (s, 1H), 3.31 - 3.04 (m, 7H), 2.96 - 2.82 (m, 4H), 2.69 - 2.56 (m, 4H), 2.44 - 2.36 (m, 1H), 2.33 (s, 2H), 2.09 - 1.98 (m, 3H), 1.86 - 1.64 (m, 2H), 1.46 (t, *J* = 6.2 Hz, 2H), 0.96 (s, 6H). LCMS (ESI) calcd. for C₃₈H₄₄ClF₃N₅O₄ ⁺ [M+H]⁺: 726.30, found, 726.3.

### Example 203: Preparation of 3-(5-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-6-fluoro-1-oxoisoindolin-2-yl)piperidine-2,6-dione (GT-09731)

With reference to the method of Scheme 4, the target compound was obtained as a white solid (GT-09731) (white solid, 10 mg, yield 28 %). ¹H NMR (400 MHz, DMSO) δ 11.02 (s, 1H), 9.88 (s, 1H), 7.72 - 7.55 (m, 2H), 7.44 (d, *J* = 8.4 Hz, 2H), 7.14 (d, 2H), 5.14 (dd, *J* = 13.1, 5.0 Hz, 1H), 4.72 - 4.31 (m, 3H), 3.59 - 3.51 (m, 3H), 3.30 - 3.18 (m, 4H), 3.15 - 2.76 (m, 6H), 2.65 - 2.55 (m, 3H), 2.43 - 2.25 (m, 3H), 2.04 (s, 3H), 1.87 - 1.60 (m, 2H), 1.46 (t, *J* = 6.2 Hz, 2H), 0.96 (s, 6H). LCMS (ESI) calcd. for C₃₈H₄₄ClF₃N₅O₄ ⁺ [M+H]⁺: 726.30, found, 726.3.

### Example 204: Preparation of 3-(4-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-1-oxoisoindolin-2-yl)piperidine-2,6-dione (GT-09732)

With reference to the method of Scheme 4, the target compound (GT-09732) was obtained as a white solid (11 mg, yield 31 %). ¹H NMR (400 MHz, DMSO) δ 10.99 (d, *J* = 4.0 Hz, 1H), 9.92 (s, 1H), 7.85 (d, *J* = 8.6 Hz, 1H), 7.66 - 7.55 (m, 2H), 7.44 (d, *J* = 8.4 Hz, 2H), 7.14 (d, *J* = 8.4 Hz, 2H), 5.20 - 5.06 (m, 1H), 4.75 - 4.27 (m, 3H), 3.55 (s, 3H), 3.30 - 3.16 (m, 4H), 3.10 - 3.01 (m, 2H), 2.92 - 2.80 (m, 3H), 2.66 - 2.54 (m, 4H), 2.47 - 2.37 (m, 1H), 2.31 (s, 2H), 2.07 - 1.96 (m, 3H), 1.74 (s, 2H), 1.46 (t, *J* = 6.2 Hz, 2H), 0.96 (s, 6H). LCMS (ESI) calcd. for C₃₈H₄₅ClF₂N₅O₄ ⁺ [M+H]⁺: 708.31, found, 708.4.

### Example 205: Preparation of 3-(6-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-1-oxoisoindolin-2-yl)piperidine-2,6-dione (GT-09828)

With reference to the method of Scheme 4, the target compound (GT-09828) was obtained as a white solid (11 mg, yield 32 %). ¹H NMR (400 MHz, DMSO) δ 11.02 (s, 1H), 9.90 (s, 1H), 7.72 (d, *J* = 7.8 Hz, 1H), 7.69 - 7.62 (m, 2H), 7.44 (d, *J* = 8.4 Hz, 2H), 7.14 (d, *J* = 8.4 Hz, 2H), 5.13 (dd, *J* = 13.3, 5.1 Hz, 1H), 4.80 - 4.49 (m, 2H), 4.40 (d, *J* = 17.8 Hz, 1H), 3.66 - 3.58 (m, 4H), 3.31 - 3.17 (m, 4H), 3.10 - 3.01 (m, 2H), 2.95 - 2.82 (m, 3H), 2.65 - 2.57 (m, 3H), 2.47 - 2.36 (m, 1H), 2.31 (s, 2H), 2.06 - 1.98 (m, 3H), 1.83 - 1.62 (m, 2H), 1.46 (t, *J* = 6.2 Hz, 2H), 0.96 (s, 6H). LCMS (ESI) calcd. for C₃₈H₄₅ClF₂N₅O₄ ⁺ [M+H]⁺: 708.31, found, 708.4.

### Example 206: Preparation of 3-(7-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-1-oxoisoindolin-2-yl)piperidine-2,6-dione (GT-09829)

With reference to the method of Scheme 4, the target compound (GT-09829) was obtained as a white solid (10 mg, yield 29 %). ¹H NMR (400 MHz, DMSO) δ 11.00 (d, *J* = 4.3 Hz, 1H), 9.94 (s, 1H), 7.74 - 7.63 (m, 2H), 7.49 - 7.38 (m, 2H), 7.37 - 7.27 (m, 1H), 7.14 (d, *J* = 7.1 Hz, 2H), 5.11 - 4.93 (m, 1H), 4.80 - 4.61 (m, 1H), 4.61 - 4.35 (m, 2H), 3.60 (s, 2H), 3.42 - 3.16 (m, 5H), 3.13 - 2.98 (m, 3H), 2.93 - 2.79 (m, 3H), 2.61 (d, *J* = 15.1 Hz, 3H), 2.47 - 2.36 (m, 1H), 2.31 (s, 2H), 2.12 - 1.94 (m, 3H), 1.87 - 1.67 (m, 1H), 1.57 (s, 1H), 1.46 (t, *J* = 6.1 Hz, 2H), 0.95 (s, 6H). LCMS (ESI) calcd. for C₃₈H₄₅ClF₂N₅O₄ ⁺ [M+H]⁺: 708.31, found, 708.3.

### Example 207: Preparation of 4-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-2-(2,6-dioxopiperidin-3-yl)isoindoline-1,3-dione (GT-09830)

With reference to the method of Scheme 4, the target compound (GT-09830) was obtained as a white solid (9 mg, yield 26 %). ¹H NMR (400 MHz, DMSO) δ 11.17 - 11.08 (m, 1H), 9.75 (s, 1H), 8.12 - 7.87 (m, 2H), 7.81 - 7.59 (m, 1H), 7.43 (d, *J* = 8.3 Hz, 2H), 7.14 (d, *J* = 8.3 Hz, 2H), 5.20 - 5.07 (m, 1H), 4.81 - 4.48 (m, 1H), 3.59 - 3.51 (m, 2H), 3.33 - 3.09 (m, 6H), 3.06 - 2.96 (m, 2H), 2.94 - 2.80 (m, 3H), 2.60 (d, *J* = 13.9 Hz, 3H), 2.34 - 2.25 (m, 2H), 2.09 - 1.99 (m, 3H), 1.88 - 1.56 (m, 2H), 1.46 (t, *J* = 6.1 Hz, 2H), 1.28 (dd, *J* = 11.1, 6.5 Hz, 1H), 0.96 (s, 6H). LCMS (ESI) calcd. for C₃₈H₄₃ClF₂N₅O₅ ⁺ [M+H]⁺: 722.29, found, 722.3.

### Example 208: Preparation of 5-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-2-(2,6-dioxopiperidin-3-yl)isoindoline-1,3-dione (GT-09831)

With reference to the method of Scheme 4, the target compound (GT-09831) was obtained as a white solid (9 mg, yield 26 %). ¹H NMR (400 MHz, DMSO) δ 11.15 (s, 1H), 9.62 (s, 1H), 8.01 (d, *J* = 8.1 Hz, 1H), 7.85 (d, 2H), 7.44 (d, *J* = 8.4 Hz, 2H), 7.14 (d, *J* = 8.4 Hz, 2H), 5.19 (dd, *J* = 12.8, 5.4 Hz, 1H), 4.60 (d, *J* = 52.1 Hz, 1H), 3.63 - 3.50 (m, 3H), 3.22 (d, *J* = 9.0 Hz, 4H), 3.08 - 2.97 (m, 2H), 2.96 - 2.85 (m, 3H), 2.70 - 2.53 (m, 4H), 2.33 - 2.25 (m, 2H), 2.13 - 2.00 (m, 3H), 1.83 - 1.68 (m, 2H), 1.66 - 1.57 (m, 1H), 1.46 (t, *J* = 6.2 Hz, 2H), 0.96 (s, 6H). LCMS (ESI) calcd. for C₃₈H₄₃ClF₂N₅O₅ ⁺ [M+H]⁺: 722.29, found, 722.3.

### Example 209: Preparation of 3-(5-(4-(4-(2,3-dichlorophenyl)piperazin-1-yl)piperidine-1-carbonyl)-1-oxoisoindolin-2-yl)piperidine-2,6-dione (GT-09832)

With reference to the method of Scheme 4, the target compound (GT-09832) was obtained as a white solid (8 mg, yield 21 %). ¹H NMR (400 MHz, DMSO) δ 11.04 (d, *J* = 15.2 Hz, 2H), 7.81 (d, *J* = 7.8 Hz, 1H), 7.66 (s, 1H), 7.55 (d, *J* = 8.8 Hz, 1H), 7.43 - 7.31 (m, 2H), 7.28 - 7.17 (m, 1H), 5.15 (dd, *J* = 13.3, 5.1 Hz, 1H), 4.66 (s, 1H), 4.52 (d, *J* = 17.6 Hz, 1H), 4.40 (d, *J* = 17.6 Hz, 1H), 3.75 - 3.53 (m, 4H), 3.47 (d, *J* = 9.7 Hz, 2H), 3.28 - 3.11 (m, 5H), 2.98 - 2.78 (m, 2H), 2.61 (d, *J* = 16.2 Hz, 1H), 2.46 - 2.36 (m, 1H), 2.32 (dd, *J* = 11.6, 9.8 Hz, 1H), 2.19 - 2.06 (m, 1H), 2.06 - 1.97 (m, 1H), 1.86 - 1.70 (m, 2H). LCMS (ESI) calcd. for C₂₉H₃₂Cl₂N₅O₄ ⁺ [M+H]⁺: 584.18, found, 584.2.

### Example 210: Preparation of 3-(5-(4-(4-(2,3-dichlorophenyl)piperazin-1-yl)piperidine-1-carbonyl)-7-fluoro-1-oxoisoindolin-2-yl)piperidine-2,6-dione (GT-09833)

With reference to the method of Scheme 4, the target compound (GT-09833) was obtained as a white solid (10 mg, yield 25 %). ¹H NMR (400 MHz, DMSO) δ 11.11 (s, 1H), 11.03 (s, 1H), 7.49 (s, 1H), 7.43 - 7.31 (m, 3H), 7.26 - 7.17 (m, 1H), 5.11 (dd, *J* = 13.2, 5.1 Hz, 1H), 4.79 - 4.59 (m, 1H), 4.54 (d, *J* = 18.1 Hz, 1H), 4.41 (d, *J* = 18.1 Hz, 1H), 3.72 - 3.53 (m, 4H), 3.47 (d, *J* = 10.2 Hz, 2H), 3.29 - 3.13 (m, 5H), 2.98 - 2.79 (m, 2H), 2.61 (d, *J* = 16.6 Hz, 1H), 2.45 - 2.26 (m, 2H), 2.14 - 1.97 (m, 2H), 1.86 - 1.71 (m, 2H). LCMS (ESI) calcd. for C₂₉H₃₁Cl₂FN₅O₄ ⁺ [M+H]⁺: 602.17, found, 602.2.

### Example 211: Preparation of 3-(5-(4-(4-(2,3-dichlorophenyl)piperazin-1-yl)piperidine-1-carbonyl)-6-fluoro-1-oxoisoindolin-2-yl)piperidine-2,6-dione (GT-09834)

With reference to the method of Scheme 4, the target compound (GT-09834) was obtained as a white solid (11 mg, yield 28 %). ¹H NMR (400 MHz, DMSO) δ 11.50 - 10.74 (m, 2H), 7.78 - 7.59 (m, 2H), 7.44 - 7.34 (m, 2H), 7.21 (dd, *J* = 7.1, 2.4 Hz, 1H), 5.23 - 5.03 (m, 1H), 4.69 (d, *J* = 12.1 Hz, 1H), 4.50 (d, *J* = 17.4 Hz, 1H), 4.37 (d, *J* = 17.6 Hz, 1H), 3.58 (s, 4H), 3.46 (d, *J* = 10.2 Hz, 2H), 3.28 - 3.10 (m, 5H), 2.97 - 2.81 (m, 2H), 2.61 (d, *J* = 17.4 Hz, 1H), 2.47 - 2.38 (m, 1H), 2.35 - 2.27 (m, 1H), 2.13 (s, 1H), 2.07 - 1.99 (m, 1H), 1.70 (s, 2H). LCMS (ESI) calcd. for C₂₉H₃₁Cl₂FN₅O₄ ⁺ [M+H]⁺: 602.17, found, 602.2.

### Example 212: Preparation of 3-(4-(4-(4-(2,3-dichlorophenyl)piperazin-1-yl)piperidine-1-carbonyl)-1-oxoisoindolin-2-yl)piperidine-2,6-dione (GT-09835)

With reference to the method of Scheme 4, the target compound (GT-09835) was obtained as a white solid (5 mg, yield 14 %). ¹H NMR (400 MHz, DMSO) δ 11.16 - 10.91 (m, 2H), 7.85 (dd, *J* = 7.0, 1.5 Hz, 1H), 7.69 - 7.56 (m, 2H), 7.43 - 7.29 (m, 2H), 7.26 - 7.13 (m, 1H), 5.15 (dd, *J* = 13.2, 4.9 Hz, 1H), 4.80 - 4.17 (m, 3H), 3.66 - 3.53 (m, 3H), 3.50 - 3.42 (m, 2H), 3.29 - 3.02 (m, 6H), 2.97 - 2.78 (m, 2H), 2.58 (d, *J* = 17.4 Hz, 1H), 2.47 - 2.36 (m, 1H), 2.19 (s, 2H), 2.04 - 1.96 (m, 1H), 1.85 - 1.69 (m, 2H). LCMS (ESI) calcd. for C₂₉H₃₂Cl₂N₅O₄ ⁺ [M+H]⁺: 584.18, found, 584.3.

### Example 213: Preparation of 3-(6-(4-(4-(2,3-dichlorophenyl)piperazin-1-yl)piperidine-1-carbonyl)-1-oxoisoindolin-2-yl)piperidine-2,6-dione (GT-09836)

With reference to the method of Scheme 4, the target compound (GT-09836) was obtained as a white solid (9 mg, yield 24 %). ¹H NMR (400 MHz, DMSO) δ 11.10 (s, 1H), 11.02 (s, 1H), 7.73 (d, *J* = 8.3 Hz, 2H), 7.70 - 7.65 (m, 1H), 7.40 - 7.34 (m, 2H), 7.21 (dd, *J* = 7.1, 2.5 Hz, 1H), 5.14 (dd, *J* = 13.3, 5.1 Hz, 1H), 4.66 (s, 1H), 4.53 (d, *J* = 17.8 Hz, 1H), 4.40 (d, *J* = 17.8 Hz, 1H), 3.79 - 3.53 (m, 4H), 3.46 (d, *J* = 9.9 Hz, 2H), 3.30 - 3.16 (m, 5H), 2.99 - 2.78 (m, 2H), 2.62 (d, *J* = 16.7 Hz, 1H), 2.46 - 2.36 (m, 1H), 2.34 - 2.08 (m, 2H), 2.06 - 2.00 (m, 1H), 1.84 - 1.70 (m, 2H). LCMS (ESI) calcd. for C₂₉H₃₂Cl₂N₅O₄ ⁺ [M+H]⁺: 584.18, found, 584.3.

### Example 214: Preparation of 3-(7-(4-(4-(2,3-dichlorophenyl)piperazin-1-yl)piperidine-1-carbonyl)-1-oxoisoindolin-2-yl)piperidine-2,6-dione (GT-09837)

With reference to the method of Scheme 4, the target compound (GT-09837) was obtained as a white solid (6 mg, yield 16 %). ¹H NMR (400 MHz, DMSO) δ 11.20 - 10.58 (m, 2H), 7.76 - 7.60 (m, 2H), 7.45 - 7.29 (m, 3H), 7.26 - 7.10 (m, 1H), 5.09 - 4.86 (m, 1H), 4.73 (t, 1H), 4.56 - 4.48 (m, 1H), 4.46 - 4.34 (m, 1H), 3.64 - 3.53 (m, 2H), 3.50 - 3.41 (m, 3H), 3.30 - 3.14 (m, 5H), 3.05 - 2.91 (m, 1H), 2.91 - 2.71 (m, 2H), 2.61 (d, *J* = 17.3 Hz, 1H), 2.46 - 2.36 (m, 1H), 2.27 (dd, *J* = 31.4, 13.4 Hz, 1H), 2.13 - 1.91 (m, 2H), 1.91 - 1.62 (m, 2H). LCMS (ESI) calcd. for C₂₉H₃₂Cl₂N₅O₄ ⁺ [M+H]⁺: 584.18, found, 584.3.

### Example 215: Preparation of 4-(4-(4-(2,3-dichlorophenyl)piperazin-1-yl)piperidine-1-carbonyl)-2-(2,6-dioxopiperidin-3-yl)isoindoline-1,3-dione (GT-09838)

With reference to the method of Scheme 4, the target compound (GT-09838) was obtained as a white solid (7 mg, yield 18 %). ¹H NMR (400 MHz, DMSO) δ 11.23 - 10.57 (m, 2H), 8.07 - 7.91 (m, 2H), 7.90 - 7.70 (m, 1H), 7.43 - 7.30 (m, 2H), 7.26 - 7.12 (m, 1H), 5.23 - 5.05 (m, 1H), 4.72 (d, *J* = 12.6 Hz, 1H), 3.65 - 3.40 (m, 6H), 3.30 - 2.96 (m, 6H), 2.94 - 2.79 (m, 2H), 2.64 - 2.56 (m, 1H), 2.34 - 2.24 (m, 1H), 2.13 - 1.94 (m, 2H), 1.81 - 1.45 (m, 2H). LCMS (ESI) calcd. for C₂₉H₃₀Cl₂N₅O₅ ⁺ [M+H]⁺: 598.16, found, 598.2.

### Example 216: Preparation of 5-(4-(4-(2,3-dichlorophenyl)piperazin-1-yl)piperidine-1-carbonyl)-2-(2,6-dioxopiperidin-3-yl)isoindoline-1,3-dione (GT-09839)

With reference to the method of Scheme 4, the target compound (GT-09839) was obtained as a white solid (13 mg, yield 33 %). ¹H NMR (400 MHz, DMSO) δ 11.22 (s, 1H), 11.16 (s, 1H), 8.02 (d, *J* = 7.7 Hz, 1H), 7.95 (s, 1H), 7.93 - 7.87 (m, 1H), 7.42 - 7.33 (m, 2H), 7.25 - 7.19 (m, 1H), 5.19 (dd, *J* = 12.8, 5.4 Hz, 1H), 4.64 (s, 1H), 3.59 (s, 4H), 3.46 (d, *J* = 10.0 Hz, 2H), 3.29 - 3.10 (m, 5H), 2.97 - 2.81 (m, 2H), 2.69 - 2.53 (m, 2H), 2.35 - 2.21 (m, 1H), 2.13 - 2.02 (m, 2H), 1.81 (d, *J* = 9.7 Hz, 2H). LCMS (ESI) calcd. for C₂₉H₃₀Cl₂N₅O₅ ⁺ [M+H]⁺: 598.16, found, 598.2.

### Example 217: Preparation of 3-(5-(4-(4-(2,3-dichlorophenyl)piperazin-1-yl)-3,3-difluoropiperidine-1-carbonyl)-1-oxoisoindolin-2-yl)piperidine-2,6-dione (GT-09840)

With reference to the method of Scheme 4, the target compound (GT-09840) was obtained as a white solid (22 mg, yield 60 %). ¹H NMR (400 MHz, DMSO) δ 11.02 (s, 1H), 7.83 (d, *J* = 7.8 Hz, 1H), 7.67 (s, 1H), 7.54 (d, *J* = 7.8 Hz, 1H), 7.32 (d, *J* = 6.7 Hz, 2H), 7.22 - 7.09 (m, 1H), 5.15 (dd, *J* = 13.4, 4.6 Hz, 1H), 4.69 (d, *J* = 70.8 Hz, 1H), 4.57 - 4.49 (m, 1H), 4.46 - 4.34 (m, 1H), 3.91 - 3.56 (m, 4H), 3.15 (s, 8H), 2.95 - 2.87 (m, 1H), 2.61 (d, *J* = 17.1 Hz, 1H), 2.45 - 2.33 (m, 1H), 2.06 - 1.86 (m, 2H). LCMS (ESI) calcd. for C₂₉H₃₀Cl₂F₂N₅O₄ ⁺ [M+H]⁺: 620.16, found, 620.2.

### Example 218: Preparation of 3-(5-(4-(4-(2,3-dichlorophenyl)piperazin-1-yl)-3,3-difluoropiperidine-1-carbonyl)-7-fluoro-1-oxoisoindolin-2-yl)piperidine-2,6-dione (GT-09841)

With reference to the method of Scheme 4, the target compound (GT-09841) was obtained as a white solid (21 mg, yield 56 %). ¹H NMR (400 MHz, DMSO) δ 11.03 (s, 1H), 7.50 (s, 1H), 7.43 - 7.25 (m, 3H), 7.17 (t, 1H), 5.11 (dd, *J* = 13.2, 3.9 Hz, 1H), 4.83 - 4.38 (m, 3H), 3.76 (d, *J* = 63.6 Hz, 2H), 3.34 - 2.98 (m, 10H), 2.97 - 2.85 (m, 1H), 2.61 (d, *J* = 16.8 Hz, 1H), 2.44 - 2.32 (m, 1H), 2.31 - 1.85 (m, 3H). LCMS (ESI) calcd. for C₂₉H₂₉Cl₂F₃N₅O₄ ⁺ [M+H]⁺: 638.15, found, 638.2.

### Example 219: Preparation of 3-(5-(4-(4-(2,3-dichlorophenyl)piperazin-1-yl)-3,3-difluoropiperidine-1-carbonyl)-6-fluoro-1-oxoisoindolin-2-yl)piperidine-2,6-dione (GT-09842)

With reference to the method of Scheme 4, the target compound (GT-09842) was obtained as a white solid (17 mg, yield 45 %). ¹H NMR (400 MHz, DMSO) δ 11.03 (s, 1H), 7.78 - 7.60 (m, 2H), 7.32 (d, *J* = 6.6 Hz, 2H), 7.17 (t, *J* = 4.8 Hz, 1H), 5.15 (dd, *J* = 13.3, 4.9 Hz, 1H), 4.85 - 4.60 (m, 1H), 4.50 (d, *J* = 17.4 Hz, 1H), 4.38 (d, *J* = 17.4 Hz, 1H), 3.79 - 3.63 (m, 2H), 3.34 - 2.99 (m, 10H), 2.96 - 2.86 (m, 1H), 2.61 (d, *J* = 16.8 Hz, 1H), 2.45 - 2.30 (m, 1H), 2.30 - 1.97 (m, 2H), 1.91 (s, 1H). LCMS (ESI) calcd. for C₂₉H₂₉Cl₂F₃N₅O₄ ⁺ [M+H]⁺: 638.15, found, 638.2.

### Example 220: Preparation of 3-(4-(4-(4-(2,3-dichlorophenyl)piperazin-1-yl)-3,3-difluoropiperidine-1-carbonyl)-1-oxoisoindolin-2-yl)piperidine-2,6-dione (GT-09843)

With reference to the method of Scheme 4, the target compound (GT-09843) was obtained as a white solid (15 mg, yield 41 %). ¹H NMR (400 MHz, DMSO) δ 11.00 (d, *J* = 2.6 Hz, 1H), 7.87 (t, 1H), 7.65 (d, *J* = 5.0 Hz, 2H), 7.32 (d, *J* = 6.7 Hz, 2H), 7.18 (t, *J* = 9.1, 4.4 Hz, 1H), 5.21 - 5.08 (m, 1H), 4.91 - 4.27 (m, 3H), 3.58 (s, 3H), 3.28 - 2.99 (m, 8H), 2.96 - 2.86 (m, 1H), 2.58 (d, *J* = 17.5 Hz, 1H), 2.47 - 2.34 (m, 1H), 2.31 - 1.84 (m, 3H). LCMS (ESI) calcd. for C₂₉H₃₀Cl₂F₂N₅O₄ ⁺ [M+H]⁺: 620.16, found, 620.2.

### Example 221: Preparation of 3-(6-(4-(4-(2,3-dichlorophenyl)piperazin-1-yl)-3,3-difluoropiperidine-1-carbonyl)-1-oxoisoindolin-2-yl)piperidine-2,6-dione (GT-09844)

With reference to the method of Scheme 4, the target compound (GT-09844) was obtained as a white solid (20 mg, yield 55 %). ¹H NMR (400 MHz, DMSO) δ 11.02 (s, 1H), 7.77 - 7.63 (m, 3H), 7.33 (d, *J* = 7.3, 6.2 Hz, 2H), 7.18 (t, *J* = 9.3, 4.2 Hz, 1H), 5.15 (dd, *J* = 13.3, 5.1 Hz, 1H), 4.87 - 4.37 (m, 3H), 3.66 (s, 2H), 3.08 (d, *J* = 79.5 Hz, 10H), 2.96 - 2.87 (m, 1H), 2.62 (d, *J* = 16.6 Hz, 1H), 2.47 - 2.32 (m, 1H), 2.20 (s, 1H), 2.08 - 1.87 (m, 2H). LCMS (ESI) calcd. for C₂₉H₃₀Cl₂F₂N₅O₄ ⁺ [M+H]⁺: 620.16, found, 620.3.

### Example 222: Preparation of 3-(7-(4-(4-(2,3-dichlorophenyl)piperazin-1-yl)-3,3-difluoropiperidine-1-carbonyl)-1-oxoisoindolin-2-yl)piperidine-2,6-dione (GT-09845)

With reference to the method of Scheme 4, the target compound (GT-09845) was obtained as a white solid (18 mg, yield 49 %). ¹H NMR (400 MHz, DMSO) δ 11.06 - 10.95 (m, 1H), 7.80 - 7.65 (m, 2H), 7.41 - 7.28 (m, 3H), 7.21 - 7.10 (m, 1H), 5.10 - 4.98 (m, 1H), 4.93 - 4.69 (m, 1H), 4.55 - 4.36 (m, 2H), 3.42 - 3.04 (m, 13H), 2.92 - 2.83 (m, 1H), 2.61 (d, *J* = 17.0 Hz, 1H), 2.46 - 2.36 (m, 1H), 2.05 - 1.84 (m, 2H). LCMS (ESI) calcd. for C₂₉H₃₀Cl₂F₂N₅O₄ ⁺ [M+H]⁺: 620.16, found, 620.2.

### Example 223: Preparation of 4-(4-(4-(2,3-dichlorophenyl)piperazin-1-yl)-3,3-difluoropiperidine-1-carbonyl)-2-(2,6-dioxopiperidin-3-yl)isoindoline-1,3-dione (GT-09846)

With reference to the method of Scheme 4, the target compound (GT-09846) was obtained as a white solid (13 mg, yield 35 %). ¹H NMR (400 MHz, DMSO) δ 11.14 (d, *J* = 4.9 Hz, 1H), 8.07 - 7.89 (m, 2H), 7.83 - 7.64 (m, 1H), 7.33 (d, *J* = 4.6 Hz, 2H), 7.22 - 7.12 (m, 1H), 5.23 - 5.09 (m, 1H), 4.93 - 4.61 (m, 1H), 3.58 (s, 2H), 3.36 - 2.96 (m, 11H), 2.94 - 2.84 (m, 1H), 2.61 (d, *J* = 17.8 Hz, 1H), 2.37 - 1.95 (m, 2H), 1.95 - 1.76 (m, 1H). LCMS (ESI) calcd. for C₂₉H₂₈Cl₂F₂N₅O₅ ⁺ [M+H]⁺: 634.14, found, 634.2.

### Example 224: Preparation of 5-(4-(4-(2,3-dichlorophenyl)piperazin-1-yl)-3,3-difluoropiperidine-1-carbonyl)-2-(2,6-dioxopiperidin-3-yl)isoindoline-1,3-dione (GT-09847)

With reference to the method of Scheme 4, the target compound (GT-09847) was obtained as a white solid (15 mg, yield 40 %). ¹H NMR (400 MHz, DMSO) δ 11.16 (s, 1H), 8.00 (t, *J* = 18.3 Hz, 1H), 7.89 (s, 2H), 7.32 (t, *J* = 6.6 Hz, 2H), 7.17 (t, *J* = 4.8 Hz, 1H), 5.20 (dd, *J* = 12.7, 5.4 Hz, 1H), 4.68 (d, *J* = 57.1 Hz, 1H), 3.61 (s, 2H), 3.11 (s, 10H), 2.95 - 2.85 (m, 1H), 2.70 - 2.53 (m, 2H), 2.36 - 1.84 (m, 3H). LCMS (ESI) calcd. for C₂₉H₂₈Cl₂F₂N₅O₅ ⁺ [M+H]⁺: 634.14, found, 634.2.

### Example 225: Preparation of 3-(5-(4-(4-(6-fluorobenzo[d]isoxazol-3-yl)piperazin-1-yl)piperidine-1-carbonyl)-1-oxoisoindolin-2-yl)piperidine-2,6-dione (GT-09848)

With reference to the method of Scheme 4, the target compound (GT-09848) was obtained as a white solid (11 mg, yield 23 %). ¹H NMR (400 MHz, DMSO) δ 11.37 (s, 1H), 11.02 (s, 1H), 8.09 (dd, *J* = 8.9, 5.2 Hz, 1H), 7.81 (d, *J* = 7.8 Hz, 1H), 7.66 (s, 1H), 7.62 (dd, *J* = 9.1, 2.2 Hz, 1H), 7.54 (d, *J* = 7.7 Hz, 1H), 7.32 - 7.20 (m, 1H), 5.15 (dd, *J* = 13.3, 5.1 Hz, 1H), 4.66 (s, 1H), 4.52 (d, *J* = 17.6 Hz, 1H), 4.39 (d, *J* = 17.6 Hz, 1H), 4.13 (d, *J* = 13.0 Hz, 2H), 3.77 - 3.51 (m, 6H), 3.30 (d, *J* = 10.9 Hz, 2H), 3.15 (d, *J* = 15.7 Hz, 1H), 2.98 - 2.77 (m, 2H), 2.61 (d, *J* = 16.9 Hz, 1H), 2.45 - 2.31 (m, 1H), 2.30 - 1.97 (m, 3H), 1.76 (s, 2H). LCMS (ESI) calcd. for C₃₀H₃₂FN₆O₅ ⁺ [M+H]⁺: 575.24, found, 575.3.

### Example 226: Preparation of 3-(7-fluoro-5-(4-(4-(6-fluorobenzo[d]isoxazol-3-yl)piperazin-1-yl)piperidine-1-carbonyl)-1-oxoisoindolin-2-yl)piperidine-2,6-dione (GT-09849)

With reference to the method of Scheme 4, the target compound (GT-09849) was obtained as a white solid (13 mg, yield 26 %). ¹H NMR (400 MHz, DMSO) δ 11.47 (s, 1H), 11.03 (s, 1H), 8.09 (dd, *J* = 8.9, 5.2 Hz, 1H), 7.62 (dd, *J* = 9.1, 2.2 Hz, 1H), 7.49 (s, 1H), 7.36 (d, *J* = 8.3 Hz, 1H), 7.31 - 7.20 (m, 1H), 5.11 (dd, *J* = 13.3, 5.1 Hz, 1H), 4.75 - 4.58 (m, 1H), 4.54 (d, 1H), 4.43 (d, *J* = 14.6 Hz, 1H), 4.13 (d, *J* = 13.3 Hz, 2H), 3.68 - 3.50 (m, 6H), 3.33 - 3.24 (m, 2H), 3.13 (s, 1H), 2.95 - 2.73 (m, 2H), 2.61 (d, *J* = 16.8 Hz, 1H), 2.45 - 2.33 (m, 1H), 2.26 (s, 1H), 2.12 - 1.96 (m, 2H), 1.84 - 1.68 (m, 2H). LCMS (ESI) calcd. for C₃₀H₃₁F₂N₆O₅ ⁺ [M+H]⁺: 593.23, found, 593.3.

### Example 227: Preparation of 3-(6-fluoro-5-(4-(4-(6-fluorobenzo[d]isoxazol-3-yl)piperazin-1-yl)piperidine-1-carbonyl)-1-oxoisoindolin-2-yl)piperidine-2,6-dione (GT-09850)

With reference to the method of Scheme 4, the target compound (GT-09850) was obtained as a white solid (7 mg, yield 15 %). ¹H NMR (400 MHz, DMSO) δ 11.20 (d, *J* = 75.5 Hz, 1H), 11.03 (s, 1H), 8.09 (dd, *J* = 8.9, 5.2 Hz, 1H), 7.76 - 7.65 (m, 2H), 7.62 (dd, *J* = 9.1, 2.2 Hz, 1H), 7.31 - 7.21 (m, 1H), 5.15 (dd, *J* = 13.1, 4.9 Hz, 1H), 4.69 (d, *J* = 12.3 Hz, 1H), 4.50 (d, *J* = 17.3 Hz, 1H), 4.37 (d, *J* = 15.4 Hz, 1H), 4.13 (d, *J* = 13.0 Hz, 2H), 3.70 - 3.48 (m, 6H), 3.31 - 3.23 (m, 2H), 3.14 (s, 1H), 2.98 - 2.78 (m, 2H), 2.61 (d, *J* = 16.9 Hz, 1H), 2.46 - 2.25 (m, 2H), 2.17 - 1.95 (m, 2H), 1.68 (s, 2H). LCMS (ESI) calcd. for C₃₀H₃₁F₂N₆O₅ ⁺ [M+H]⁺: 593.23, found, 593.3.

### Example 228: Preparation of 3-(4-(4-(4-(6-fluorobenzo[d]isoxazol-3-yl)piperazin-1-yl)piperidine-1-carbonyl)-1-oxoisoindolin-2-yl)piperidine-2,6-dione (GT-09851)

With reference to the method of Scheme 4, the target compound (GT-09851) was obtained as a white solid (10 mg, yield 19 %). ¹H NMR (400 MHz, DMSO) δ 11.38 (s, 1H), 11.00 (s, 1H), 8.18 - 8.03 (m, 1H), 7.89 - 7.76 (m, 1H), 7.72 - 7.55 (m, 3H), 7.30 - 7.17 (m, 1H), 5.15 (dd, *J* = 13.2, 5.1 Hz, 1H), 4.77 - 4.29 (m, 3H), 4.13 (d, *J* = 13.0 Hz, 2H), 3.84 - 3.49 (m, 6H), 3.29 (d, *J* = 9.9 Hz, 2H), 3.15 (s, 1H), 2.98 - 2.69 (m, 2H), 2.59 (d, *J* = 16.9 Hz, 1H), 2.49 - 2.38 (m, 1H), 2.34 - 2.06 (m, 2H), 2.04 - 1.96 (m, 1H), 1.86 - 1.67 (m, 2H). LCMS (ESI) calcd. for C₃₀H₃₂FN₆O₅ ⁺ [M+H]⁺: 575.24, found, 575.3.

### Example 229: Preparation of 3-(6-(4-(4-(6-fluorobenzo[d]isoxazol-3-yl)piperazin-1-yl)piperidine-1-carbonyl)-1-oxoisoindolin-2-yl)piperidine-2,6-dione (GT-09896)

With reference to the method of Scheme 4, the target compound (GT-09896) was obtained as a white solid (17 mg, yield 35 %). ¹H NMR (400 MHz, DMSO) δ 11.24 (s, 1H), 11.02 (s, 1H), 8.10 (dd, *J* = 8.9, 5.2 Hz, 1H), 7.78 - 7.70 (m, 2H), 7.69 - 7.65 (m, 1H), 7.62 (dd, *J* = 9.1, 2.2 Hz, 1H), 7.31 - 7.20 (m, 1H), 5.14 (dd, *J* = 13.3, 5.1 Hz, 1H), 4.68 - 4.33 (m, 3H), 4.13 (d, *J* = 13.0 Hz, 2H), 3.69 - 3.53 (m, 6H), 3.30 (d, *J* = 10.0 Hz, 2H), 3.19 - 3.08 (m, 1H), 2.97 - 2.80 (m, 2H), 2.61 (d, *J* = 17.6 Hz, 1H), 2.45 - 2.36 (m, 1H), 2.26 - 2.00 (m, 3H), 1.77 (d, *J* = 8.0 Hz, 2H). LCMS (ESI) calcd. for C₃₀H₃₂FN₆O₅ ⁺ [M+H]⁺: 575.24, found, 575.3.

### Example 230: Preparation of 3-(7-(4-(4-(6-fluorobenzo[d]isoxazol-3-yl)piperazin-1-yl)piperidine-1-carbonyl)-1-oxoisoindolin-2-yl)piperidine-2,6-dione (GT-09897)

With reference to the method of Scheme 4, the target compound (GT-09897) was obtained as a white solid (8 mg, yield 17 %). ¹H NMR (400 MHz, DMSO) δ 11.37 - 10.86 (m, 2H), 8.14 - 8.05 (m, 1H), 7.74 - 7.65 (m, 2H), 7.64 - 7.59 (m, 1H), 7.29 - 7.21 (m, 1H), 5.12 - 4.94 (m, 1H), 4.72 (s, 1H), 4.59 - 4.36 (m, 2H), 4.20 - 4.02 (m, 2H), 3.58 (t, *J* = 13.1 Hz, 4H), 3.49 - 3.44 (m, 2H), 3.29 (d, *J* = 10.3 Hz, 3H), 3.09 - 2.75 (m, 3H), 2.67 - 2.58 (m, 1H), 2.48 - 2.35 (m, 1H), 2.30 - 2.14 (m, 1H), 2.05 - 1.98 (m, 1H), 1.92 - 1.65 (m, 2H). LCMS (ESI) calcd. for C₃₀H₃₂FN₆O₅ ⁺ [M+H]⁺: 575.24, found, 575.3.

### Example 231: Preparation of 2-(2,6-dioxopiperidin-3-yl)-4-(4-(4-(6-fluorobenzo[d]isoxazol-3-yl)piperazin-1-yl)piperidine-1-carbonyl)isoindoline-1,3-dione (GT-09898)

With reference to the method of Scheme 4, the target compound (GT-09898) was obtained as a white solid (14 mg, yield 28 %). ¹H NMR (400 MHz, DMSO) δ 11.52 - 11.06 (m, 2H), 8.14 - 8.03 (m, 1H), 8.03 - 7.89 (m, 2H), 7.85 - 7.71 (m, 1H), 7.62 (dd, *J* = 9.1, 2.1 Hz, 1H), 7.26 (t, *J* = 9.0, 1.9 Hz, 1H), 5.24 - 5.03 (m, 1H), 4.72 (d, *J* = 11.4 Hz, 1H), 4.12 (d, *J* = 13.0 Hz, 2H), 3.70 - 3.51 (m, 4H), 3.50 - 3.38 (m, 2H), 3.31 - 3.19 (m, 2H), 3.13 - 2.94 (m, 1H), 2.94 - 2.79 (m, 2H), 2.69 - 2.52 (m, 2H), 2.34 - 2.20 (m, 1H), 2.13 - 1.91 (m, 2H), 1.82 - 1.42 (m, 2H). LCMS (ESI) calcd. for C₃₀H₃₀FN₆O₆ ⁺ [M+H]⁺: 589.22, found, 589.3.

### Example 232: Preparation of 2-(2,6-dioxopiperidin-3-yl)-5-(4-(4-(6-fluorobenzo[d]isoxazol-3-yl)piperazin-1-yl)piperidine-1-carbonyl)isoindoline-1,3-dione (GT-09899)

With reference to the method of Scheme 4, the target compound (GT-09899) was obtained as a white solid (15 mg, yield 30 %). ¹H NMR (400 MHz, DMSO) δ 11.15 (s, 2H), 8.08 (dd, *J* = 20.2, 10.1 Hz, 1H), 8.02 (d, *J* = 7.7 Hz, 1H), 7.95 (s, 1H), 7.93 - 7.87 (m, 1H), 7.62 (dd, *J* = 9.1, 2.1 Hz, 1H), 7.32 - 7.15 (m, 1H), 5.19 (dd, *J* = 12.8, 5.4 Hz, 1H), 4.64 (s, 1H), 4.14 (d, *J* = 12.6 Hz, 2H), 3.83 - 3.49 (m, 6H), 3.33 - 3.29 (m, 2H), 3.16 (s, 1H), 2.97 - 2.78 (m, 2H), 2.65 - 2.52 (m, 2H), 2.35 - 2.17 (m, 1H), 2.15 - 1.96 (m, 2H), 1.78 (s, 2H). LCMS (ESI) calcd. for C₃₀H₃₀FN₆O₆ ⁺ [M+H]⁺: 589.22, found, 589.3.

### Example 233: Preparation of 3-(5-(3,3-difluoro-4-(4-(6-fluorobenzo[d]isoxazol-3-yl)piperazin-1-yl)piperidine-1-carbonyl)-1-oxoisoindolin-2-yl)piperidine-2,6-dione (GT-09900)

With reference to the method of Scheme 4, the target compound (GT-09900) was obtained as a white solid (27 mg, yield 58 %). ¹H NMR (400 MHz, DMSO) δ 11.02 (s, 1H), 8.07 (dd, *J* = 8.9, 5.3 Hz, 1H), 7.83 (d, *J* = 7.8 Hz, 1H), 7.67 (s, 1H), 7.61 - 7.49 (m, 2H), 7.28 - 7.12 (m, 1H), 5.15 (dd, *J* = 12.9, 4.8 Hz, 1H), 4.81 - 4.33 (m, 3H), 3.72 (s, 6H), 3.29 (s, 6H), 2.96 - 2.89 (m, 1H), 2.62 (d, *J* = 16.9 Hz, 1H), 2.46 - 2.37 (m, 1H), 2.33 - 2.08 (m, 1H), 2.07 - 1.91 (m, 2H). LCMS (ESI) calcd. for C₃₀H₃₀F₃N₆O₅ ⁺ [M+H]⁺: 611.22, found, 611.3.

### Example 234: Preparation of 3-(5-(3,3-difluoro-4-(4-(6-fluorobenzo[d]isoxazol-3-yl)piperazin-1-yl)piperidine-1-carbonyl)-7-fluoro-1-oxoisoindolin-2-yl)piperidine-2,6-dione (GT-09901)

With reference to the method of Scheme 4, the target compound (GT-09901) was obtained as a white solid (27 mg, yield 57 %). ¹H NMR (400 MHz, DMSO) δ 11.03 (s, 1H), 8.07 (dd, *J* = 8.9, 5.3 Hz, 1H), 7.58 (dd, *J* = 9.1, 2.2 Hz, 1H), 7.50 (s, 1H), 7.36 (s, 1H), 7.22 (t, *J* = 9.1, 2.2 Hz, 1H), 5.11 (dd, *J* = 13.3, 4.1 Hz, 1H), 4.80 - 4.37 (m, 3H), 3.90 - 3.72 (m, 5H), 3.41 - 3.09 (m, 6H), 2.98 - 2.83 (m, 2H), 2.61 (d, *J* = 17.3 Hz, 1H), 2.43 - 2.31 (m, 1H), 2.30 - 2.06 (m, 1H), 2.05 - 1.88 (m, 2H). LCMS (ESI) calcd. for C₃₀H₂₉F₄N₆O₅ ⁺ [M+H]⁺: 629.21, found, 629.3.

### Example 235: Preparation of 3-(5-(3,3-difluoro-4-(4-(6-fluorobenzo[d]isoxazol-3-yl)piperazin-1-yl)piperidine-1-carbonyl)-6-fluoro-1-oxoisoindolin-2-yl)piperidine-2,6-dione (GT-09902)

With reference to the method of Scheme 4, the target compound (GT-09902) was obtained as a white solid (21 mg, yield 45 %). ¹H NMR (400 MHz, DMSO) δ 11.03 (s, 1H), 8.10 - 7.95 (m, 1H), 7.75 - 7.61 (m, 2H), 7.57 (dd, *J* = 9.1, 2.1 Hz, 1H), 7.21 (t, *J* = 9.1, 2.1 Hz, 1H), 5.15 (dd, *J* = 13.2, 5.0 Hz, 1H), 4.86 - 4.59 (m, 1H), 4.44 (dd, *J* = 49.1, 17.6 Hz, 2H), 3.78 - 3.63 (m, 4H), 3.43 - 3.29 (m, 4H), 3.16 (s, 3H), 3.09 - 2.99 (m, 1H), 2.96 - 2.86 (m, 1H), 2.61 (d, *J* = 17.0 Hz, 1H), 2.46 - 2.36 (m, 1H), 2.22 - 1.98 (m, 2H), 1.88 (s, 1H). LCMS (ESI) calcd. for C₃₀H₂₉F₄N₆O₅ ⁺ [M+H]⁺: 629.21, found, 629.3.

### Example 236: Preparation of 3-(4-(3,3-difluoro-4-(4-(6-fluorobenzo[d]isoxazol-3-yl)piperazin-1-yl)piperidine-1-carbonyl)-1-oxoisoindolin-2-yl)piperidine-2,6-dione (GT-09903)

With reference to the method of Scheme 4, the target compound (GT-09903) was obtained as a white solid (23 mg, yield 50 %). ¹H NMR (400 MHz, DMSO) δ 11.01 (d, *J* = 4.1 Hz, 1H), 8.06 (dd, *J* = 8.9, 5.3 Hz, 1H), 7.93 - 7.81 (m, 1H), 7.65 (d, *J* = 5.0 Hz, 2H), 7.58 (dd, *J* = 9.1, 2.2 Hz, 1H), 7.22 (t, *J* = 9.1, 2.2 Hz, 1H), 5.18 - 5.11 (m, 1H), 4.83 - 4.48 (m, 1H), 4.49 - 4.21 (m, 2H), 3.95 - 3.66 (m, 6H), 3.36 - 3.04 (m, 6H), 2.97 - 2.85 (m, 1H), 2.59 (d, *J* = 17.3 Hz, 1H), 2.47 - 2.37 (m, 1H), 2.25 - 1.86 (m, 3H). LCMS (ESI) calcd. for C₃₀H₃₀F₃N₆O₅ ⁺ [M+H]⁺: 611.22, found, 611.3.

### Example 237: Preparation of 3-(6-(3,3-difluoro-4-(4-(6-fluorobenzo[d]isoxazol-3-yl)piperazin-1-yl)piperidine-1-carbonyl)-1-oxoisoindolin-2-yl)piperidine-2,6-dione (GT-09904)

With reference to the method of Scheme 4, the target compound (GT-09904) was obtained as a white solid (30 mg, yield 53 %). ¹H NMR (400 MHz, DMSO) δ 11.02 (s, 1H), 8.06 (dd, *J* = 8.9, 5.3 Hz, 1H), 7.78 - 7.62 (m, 3H), 7.58 (dd, *J* = 9.1, 2.2 Hz, 1H), 7.22 (t, *J* = 9.1, 2.2 Hz, 1H), 5.15 (dd, *J* = 13.3, 5.1 Hz, 1H), 4.48 (dd, *J* = 52.3, 17.8 Hz, 3H), 3.71 - 3.62 (m, 6H), 3.35 - 3.05 (m, 6H), 2.99 - 2.88 (m, 1H), 2.62 (d, *J* = 16.6 Hz, 1H), 2.47 - 2.34 (m, 1H), 2.28 - 1.88 (m, 3H). LCMS (ESI) calcd. for C₃₀H₃₀F₃N₆O₅ ⁺ [M+H]⁺: 611.22, found, 611.3.

### Example 238: Preparation of 3-(7-(3,3-difluoro-4-(4-(6-fluorobenzo[d]isoxazol-3-yl)piperazin-1-yl)piperidine-1-carbonyl)-1-oxoisoindolin-2-yl)piperidine-2,6-dione (GT-09905)

With reference to the method of Scheme 4, the target compound (GT-09905) was obtained as a white solid (31 mg, yield 55 %). ¹H NMR (400 MHz, DMSO) δ 11.00 (s, 1H), 8.12 - 7.99 (m, 1H), 7.78 - 7.66 (m, 2H), 7.62 - 7.51 (m, 1H), 7.44 - 7.31 (m, 1H), 7.22 (t, 1H), 5.13 - 4.95 (m, 1H), 4.92 - 4.69 (m, 1H), 4.56 - 4.34 (m, 2H), 3.74 - 3.61 (m, 6H), 3.35 - 3.15 (m, 6H), 2.96 - 2.85 (m, 1H), 2.62 (d, *J* = 17.1 Hz, 1H), 2.49 - 2.13 (m, 2H), 2.02 - 1.63 (m, 2H). LCMS (ESI) calcd. for C₃₀H₃₀F₃N₆O₅ ⁺ [M+H]⁺: 611.22, found, 611.3.

### Example 239: Preparation of 4-(3,3-difluoro-4-(4-(6-fluorobenzo[d]isoxazol-3-yl)piperazin-1-yl)piperidine-1-carbonyl)-2-(2,6-dioxopiperidin-3-yl)isoindoline-1,3-dione (GT-09906)

With reference to the method of Scheme 4, the target compound (GT-09906) was obtained as a white solid (28 mg, yield 49 %). ¹H NMR (400 MHz, DMSO) δ 11.23 - 11.10 (m, 1H), 8.13 - 7.91 (m, 3H), 7.85 - 7.67 (m, 1H), 7.64 - 7.49 (m, 1H), 7.22 (t, *J* = 9.0 Hz, 1H), 5.21 - 5.08 (m, 1H), 4.90 - 4.62 (m, 1H), 3.84 - 3.64 (m, 6H), 3.39 - 3.13 (m, 6H), 3.12 - 2.97 (m, 1H), 2.95 - 2.83 (m, 1H), 2.61 (d, *J* = 17.8 Hz, 1H), 2.26 - 1.76 (m, 3H). LCMS (ESI) calcd. for C₃₀H₂₈F₃N₆O₆ ⁺ [M+H]⁺: 625.20, found, 625.3.

### Example 240: Preparation of 5-(3,3-difluoro-4-(4-(6-fluorobenzo[d]isoxazol-3-yl)piperazin-1-yl)piperidine-1-carbonyl)-2-(2,6-dioxopiperidin-3-yl)isoindoline-1,3-dione (GT-09907)

With reference to the method of Scheme 4, the target compound (GT-09907) was obtained as a white solid (25 mg, yield 44 %). ¹H NMR (400 MHz, DMSO) δ 11.15 (s, 1H), 8.11 - 7.97 (m, 2H), 7.89 (s, 2H), 7.57 (dd, *J* = 9.1, 2.2 Hz, 1H), 7.21 (t, *J* = 9.1, 2.2 Hz, 1H), 5.20 (dd, *J* = 12.7, 5.4 Hz, 1H), 4.68 (d, *J* = 53.2 Hz, 1H), 3.78 - 3.67 (m, 2H), 3.49 - 2.97 (m, 10H), 2.95 - 2.84 (m, 1H), 2.67 - 2.54 (m, 2H), 2.25 - 1.85 (m, 3H). LCMS (ESI) calcd. for C₃₀H₂₈F₃N₆O₆ ⁺ [M+H]⁺: 625.20, found, 625.3.

### Example 241: Preparation of 3-(5-(3',3'-difluoro-4-(6-fluorobenzo[d]isoxazol-3-yl)-[1,4'-bipiperidine]-1'-carbonyl)-1-oxoisoindolin-2-yl)piperidine-2,6-dione (GT-09908)

With reference to the method of Scheme 4, the target compound (GT-09908) was obtained as a white solid (16 mg, yield 29 %). ¹H NMR (400 MHz, DMSO) δ 11.56 (s, 1H), 11.02 (s, 1H), 8.24 (s, 1H), 7.84 (d, *J* = 7.8 Hz, 1H), 7.75 - 7.64 (m, 2H), 7.57 (d, *J* = 7.8 Hz, 1H), 7.34 (t, *J* = 9.1, 2.0 Hz, 1H), 5.17 - 5.11 (m, 1H), 4.89 - 4.25 (m, 5H), 4.06 - 3.45 (m, 10H), 2.96 - 2.89 (m, 1H), 2.59 (d, 1H), 2.44 - 2.40 (m, 1H), 2.25 - 2.01 (m, 4H). LCMS (ESI) calcd. for C₃₁H₃₁F₃N₅O₅ ⁺ [M+H]⁺: 610.23, found, 610.3.

### Example 242: Preparation of 3-(5-(3',3'-difluoro-4-(6-fluorobenzo[d]isoxazol-3-yl)-[1,4'-bipiperidine]-1'-carbonyl)-7-fluoro-1-oxoisoindolin-2-yl)piperidine-2,6-dione (GT-09909)

With reference to the method of Scheme 4, the target compound was obtained as a white solid (GT-09909) (white solid, 24 mg, yield 42 %). ¹H NMR (400 MHz, DMSO) δ 11.58 (s, 1H), 11.03 (s, 1H), 8.23 (s, 1H), 7.73 (d, 1H), 7.52 - 7.30 (m, 3H), 5.12 (dd, *J* = 13.1, 4.9 Hz, 1H), 4.73 (d, *J* = 83.3 Hz, 1H), 4.59 - 4.36 (m, 3H), 4.12 - 3.44 (m, 9H), 3.12 - 2.85 (m, 2H), 2.59 (d, 1H), 2.42 - 2.37 (m, 1H), 2.31 - 1.97 (m, 5H). LCMS (ESI) calcd. for C₃₁H₃₀F₄N₅O₅ ⁺ [M+H]⁺: 628.22, found, 628.3.

### Example 243: Preparation of 3-(5-(3',3'-difluoro-4-(6-fluorobenzo[d]isoxazol-3-yl)-[1,4'-bipiperidine]-1'-carbonyl)-6-fluoro-1-oxoisoindolin-2-yl)piperidine-2,6-dione (GT-09910)

With reference to the method of Scheme 4, the target compound (GT-09910) was obtained as a white solid (13 mg, yield 23 %). ¹H NMR (400 MHz, DMSO) δ 11.55 (s, 1H), 11.03 (s, 1H), 8.19 (s, 1H), 7.77 - 7.64 (m, 3H), 7.34 (t, *J* = 8.9 Hz, 1H), 5.15 (dd, 1H), 4.94 - 4.66 (m, 1H), 4.61 - 4.30 (m, 3H), 3.97 - 3.45 (m, 8H), 3.10 - 2.83 (m, 2H), 2.66 - 2.56 (m, 2H), 2.43 - 2.38 (m, 1H), 2.27 - 1.87 (m, 5H). LCMS (ESI) calcd. for C₃₁H₃₀F₄N₅O₅ ⁺ [M+H]⁺: 628.22, found, 628.3.

### Example 244: Preparation of 3-(4-(3',3'-difluoro-4-(6-fluorobenzo[d]isoxazol-3-yl)-[1,4'-bipiperidine]-1'-carbonyl)-1-oxoisoindolin-2-yl)piperidine-2,6-dione (GT-09911)

With reference to the method of Scheme 4, the target compound (GT-09911) was obtained as a white solid (17 mg, yield 31 %). ¹H NMR (400 MHz, DMSO) δ 11.68 (s, 1H), 11.00 (d, *J* = 3.8 Hz, 1H), 8.24 (s, 1H), 7.88 (d, *J* = 8.5 Hz, 1H), 7.78 - 7.51 (m, 3H), 7.34 (t, *J* = 9.1, 2.1 Hz, 1H), 5.23 - 5.08 (m, 1H), 4.95 - 4.21 (m, 4H), 4.02 - 3.45 (m, 8H), 3.24 - 3.05 (m, 1H), 2.90 (t, *J* = 20.2, 8.7 Hz, 1H), 2.59 (d, *J* = 17.7 Hz, 2H), 2.48 - 2.35 (m, 2H), 2.26 - 1.94 (m, 4H). LCMS (ESI) calcd. for C₃₁H₃₁F₃N₅O₅ ⁺ [M+H]⁺: 610.23, found, 610.3.

### Example 245: Preparation of 3-(6-(3',3'-difluoro-4-(6-fluorobenzo[d]isoxazol-3-yl)-[1,4'-bipiperidine]-1'-carbonyl)-1-oxoisoindolin-2-yl)piperidine-2,6-dione (GT-09912)

With reference to the method of Scheme 4, the target compound (GT-09912) was obtained as a white solid (20 mg, yield 36 %). ¹H NMR (400 MHz, DMSO) δ 11.49 - 10.96 (m, 2H), 8.21 (s, 1H), 7.77 - 7.69 (m, 4H), 7.35 (t, *J* = 9.1 Hz, 1H), 5.16 (dd, 1H), 4.58 - 4.38 (m, 3H), 4.08 - 3.46 (m, 8H), 3.21 - 3.02 (m, 1H), 2.95 - 2.88 (m, 1H), 2.68 - 2.58 (m, 2H), 2.46 - 2.37 (m, 2H), 2.26 - 1.96 (m, 5H). LCMS (ESI) calcd. for C₃₁H₃₁F₃N₅O₅ ⁺ [M+H]⁺: 610.23, found, 610.3.

### Example 246: Preparation of 3-(7-(3',3'-difluoro-4-(6-fluorobenzo[d]isoxazol-3-yl)-[1,4'-bipiperidine]-1'-carbonyl)-1-oxoisoindolin-2-yl)piperidine-2,6-dione (GT-09913)

With reference to the method of Scheme 4, the target compound (GT-09913) was obtained as a white solid (25 mg, yield 45 %). ¹H NMR (400 MHz, DMSO) δ 11.02 (s, 1H), 8.21 (s, 1H), 7.80 - 7.64 (m, 4H), 7.41 - 7.31 (m, 2H), 5.11 - 4.65 (m, 3H), 4.57 - 4.39 (m, 3H), 3.75 - 3.52 (m, 4H), 2.99 - 2.82 (m, 2H), 2.63 (d, *J* = 14.2 Hz, 2H), 2.47 - 2.32 (m, 3H), 2.29 - 1.86 (m, 6H). LCMS (ESI) calcd. for C₃₁H₃₁F₃N₅O₅ ⁺ [M+H]⁺: 610.23, found, 610.3.

### Example 247: Preparation of 4-(3',3'-difluoro-4-(6-fluorobenzo[d]isoxazol-3-yl)-[1,4'-bipiperidine]-1'-carbonyl)-2-(2,6-dioxopiperidin-3-yl)isoindoline-1,3-dione (GT-09914)

With reference to the method of Scheme 4, the target compound (GT-09914) was obtained as a white solid (20 mg, yield 36 %). ¹H NMR (400 MHz, DMSO) δ 11.88 - 10.99 (m, 2H), 8.21 (s, 1H), 8.05 - 7.92 (m, 2H), 7.85 - 7.66 (m, 2H), 7.33 (t, *J* = 8.2 Hz, 1H), 5.21 - 5.12 (m, 1H), 4.81 (d, *J* = 64.4 Hz, 1H), 4.38 (s, 1H), 3.84 - 3.46 (m, 6H), 3.32 - 2.82 (m, 4H), 2.61 (d, *J* = 18.6 Hz, 2H), 2.44 - 1.85 (m, 6H). LCMS (ESI) calcd. for C₃₁H₂₉F₃N₅O₆ ⁺ [M+H]⁺: 624.21, found, 624.3.

### Example 248: Preparation of 5-(3',3'-difluoro-4-(6-fluorobenzo[d]isoxazol-3-yl)-[1,4'-bipiperidine]-1'-carbonyl)-2-(2,6-dioxopiperidin-3-yl)isoindoline-1,3-dione (GT-09915)

With reference to the method of Scheme 4, the target compound (GT-09915) was obtained as a white solid (24 mg, yield 42 %). ¹H NMR (400 MHz, DMSO) δ 12.05 - 11.01 (m, 2H), 8.37 - 8.11 (m, 1H), 8.04 (d, *J* = 7.6 Hz, 1H), 7.99 - 7.84 (m, 2H), 7.72 (d, *J* = 9.0, 1.9 Hz, 1H), 7.34 (t, 1H), 5.20 (dd, *J* = 12.8, 5.4 Hz, 1H), 4.74 (d, *J* = 79.7 Hz, 1H), 4.57 - 4.08 (m, 1H), 4.02 - 3.42 (m, 8H), 3.17 - 2.98 (m, 1H), 2.93 - 2.88 (m, 1H), 2.67 - 2.53 (m, 3H), 2.46 - 2.30 (m, 1H), 2.27 - 2.03 (m, 4H). LCMS (ESI) calcd. for C₃₁H₂₉F₃N₅O₆ ⁺ [M+H]⁺: 624.21, found, 624.3.

### Example 249: Preparation of 3-(5-(4-(4-benzhydrylpiperazin-1-yl)piperidine-1-carbonyl)-1-oxoisoindolin-2-yl)piperidine-2,6-dione (GT-09944)

With reference to the method of Scheme 4, the target compound (GT-09944) was obtained as a white solid (22 mg, yield 37 %). ¹H NMR (400 MHz, DMSO) δ 11.01 (s, 1H), 7.90 - 7.59 (m, 5H), 7.52 (d, *J* = 8.2 Hz, 1H), 7.49 - 7.14 (m, 7H), 5.14 (dd, *J* = 13.3, 5.1 Hz, 1H), 4.71 - 4.45 (m, 2H), 4.38 (d, *J* = 17.6 Hz, 1H), 3.79 - 3.57 (m, 4H), 3.47 - 3.30 (m, 4H), 3.11 (s, 3H), 2.98 - 2.86 (m, 2H), 2.81 (s, 1H), 2.61 (d, *J* = 17.2 Hz, 1H), 2.44 - 2.37 (m, 1H), 2.21 (s, 1H), 2.08 - 1.94 (m, 2H), 1.71 (s, 2H). LCMS (ESI) calcd. for C₃₆H₄₀N₅O₄ ⁺ [M+H]⁺: 606.31, found, 606.4.

### Example 250: Preparation of 3-(5-(4-(4-benzhydrylpiperazin-1-yl)piperidine-1-carbonyl)-7-fluoro-1-oxoisoindolin-2-yl)piperidine-2,6-dione (GT-09945)

With reference to the method of Scheme 4, the target compound (GT-09945) was obtained as a white solid (16 mg, yield 26 %). ¹H NMR (400 MHz, DMSO) δ 11.02 (s, 1H), 8.09 - 7.48 (m, 4H), 7.46 (s, 1H), 7.45 - 7.23 (m, 8H), 5.10 (dd, *J* = 13.3, 5.1 Hz, 1H), 4.62 - 4.37 (m, 3H), 3.69 - 3.55 (m, 3H), 3.44 - 3.21 (m, 4H), 3.11 (t, 3H), 2.97 - 2.87 (m, 2H), 2.84 - 2.76 (m, 1H), 2.58 (d, 1H), 2.47 - 2.29 (m, 2H), 2.20 (s, 1H), 2.05 - 1.95 (m, 2H), 1.77 - 1.65 (m, 2H). LCMS (ESI) calcd. for C₃₆H₃₉FN₅O₄ ⁺ [M+H]⁺: 624.30, found, 624.4.

### Example 251: Preparation of 3-(5-(4-(4-benzhydrylpiperazin-1-yl)piperidine-1-carbonyl)-6-fluoro-1-oxoisoindolin-2-yl)piperidine-2,6-dione (GT-09946)

With reference to the method of Scheme 4, the target compound (GT-09946) was obtained as a white solid (20 mg, yield 33 %). ¹H NMR (400 MHz, DMSO) δ 11.02 (s, 1H), 8.01 - 7.57 (m, 5H), 7.56 - 7.20 (m, 7H), 5.18 - 5.09 (m, 1H), 4.65 (d, *J* = 12.3 Hz, 1H), 4.54 - 4.32 (m, 2H), 3.65 - 3.53 (m, 4H), 3.42 - 3.22 (m, 3H), 3.13 (s, 3H), 2.98 - 2.78 (m, 3H), 2.61 (d, *J* = 16.6 Hz, 1H), 2.49 - 2.30 (m, 2H), 2.28 - 2.18 (m, 1H), 2.09 - 1.94 (m, 2H), 1.78 - 1.55 (m, 2H). LCMS (ESI) calcd. for C₃₆H₃₉FN₅O₄ ⁺ [M+H]⁺: 624.30, found, 624.3.

### Example 252: Preparation of 3-(4-(4-(4-benzhydrylpiperazin-1-yl)piperidine-1-carbonyl)-1-oxoisoindolin-2-yl)piperidine-2,6-dione (GT-09947)

With reference to the method of Scheme 4, the target compound (GT-09947) was obtained as a white solid (17 mg, yield 29 %). ¹H NMR (400 MHz, DMSO) δ 10.99 (s, 1H), 7.83 (dd, *J* = 6.3, 2.3 Hz, 1H), 7.80 - 7.44 (m, 6H), 7.44 - 7.21 (m, 7H), 5.13 (dd, *J* = 12.8, 4.6 Hz, 1H), 4.66 - 4.52 (m, 1H), 4.47 - 4.30 (m, 2H), 3.74 - 3.57 (m, 4H), 3.47 - 3.32 (m, 3H), 3.29 - 2.97 (m, 4H), 2.95 - 2.85 (m, 2H), 2.54 (d, 1H), 2.47 - 2.39 (m, 1H), 2.36 - 1.91 (m, 4H), 1.77 - 1.65 (m, 2H). LCMS (ESI) calcd. for C₃₆H₄₀N₅O₄ ⁺ [M+H]⁺: 606.31, found, 606.4.

### Example 253: Preparation of 3-(6-(4-(4-benzhydrylpiperazin-1-yl)piperidine-1-carbonyl)-1-oxoisoindolin-2-yl)piperidine-2,6-dione (GT-09948)

With reference to the method of Scheme 4, the target compound (GT-09948) was obtained as a white solid (19 mg, yield 35 %). ¹H NMR (400 MHz, DMSO) δ 11.01 (s, 1H), 8.16 - 7.45 (m, 7H), 7.43 - 7.18 (m, 6H), 5.14 (dd, *J* = 13.3, 5.1 Hz, 1H), 4.67 - 4.36 (m, 3H), 3.76 - 3.55 (m, 4H), 3.46 - 3.24 (m, 4H), 3.23 - 2.98 (m, 3H), 2.97 - 2.71 (m, 3H), 2.61 (d, *J* = 16.9 Hz, 1H), 2.46 - 2.35 (m, 1H), 2.24 - 1.92 (m, 3H), 1.73 (s, 2H). LCMS (ESI) calcd. for C₃₆H₄₀N₅O₄ ⁺ [M+H]⁺: 606.31, found, 606.3.

### Example 254: Preparation of 3-(7-(4-(4-benzhydrylpiperazin-1-yl)piperidine-1-carbonyl)-1-oxoisoindolin-2-yl)piperidine-2,6-dione (GT-09949)

With reference to the method of Scheme 4, the target compound (GT-09949) was obtained as a white solid (17 mg, yield 29 %). ¹H NMR (400 MHz, DMSO) δ 10.99 (d, *J* = 7.2 Hz, 1H), 8.17 - 7.44 (m, 6H), 7.44 - 7.25 (m, 7H), 5.14 - 4.91 (m, 1H), 4.68 (t, *J* = 11.6 Hz, 1H), 4.56 - 4.33 (m, 2H), 3.59 (s, 4H), 3.46 - 3.12 (m, 5H), 3.11 - 2.70 (m, 5H), 2.61 (d, *J* = 16.6 Hz, 1H), 2.41 - 2.31 (m, 1H), 2.23 - 2.14 (m, 1H), 2.02 - 1.91 (m, 1H), 1.90 - 1.44 (m, 3H). LCMS (ESI) calcd. for C₃₆H₄₀N₅O₄ ⁺ [M+H]⁺: 606.31, found, 606.4.

### Example 255: Preparation of 4-(4-(4-benzhydrylpiperazin-1-yl)piperidine-1-carbonyl)-2-(2,6-dioxopiperidin-3-yl)isoindoline-1,3-dione (GT-09950)

With reference to the method of Scheme 4, the target compound (GT-09950) was obtained as a white solid (22 mg, yield 36 %). ¹H NMR (400 MHz, DMSO) δ 11.18 - 11.07 (m, 1H), 8.01 - 7.92 (m, 2H), 7.82 - 7.48 (m, 4H), 7.46 - 7.10 (m, 7H), 5.17 - 5.09 (m, 1H), 4.68 (d, *J* = 9.8 Hz, 1H), 3.62 - 3.48 (m, 6H), 3.36 - 2.76 (m, 8H), 2.60 (d, *J* = 17.7 Hz, 1H), 2.49 - 2.41 (m, 1H), 2.23 (d, *J* = 10.2 Hz, 1H), 2.10 - 1.90 (m, 2H), 1.77 - 1.42 (m, 2H). LCMS (ESI) calcd. for C₃₆H₃₈N₅O₅ ⁺ [M+H]⁺: 620.29, found, 620.3.

### Example 256: Preparation of 5-(4-(4-benzhydrylpiperazin-1-yl)piperidine-1-carbonyl)-2-(2,6-dioxopiperidin-3-yl)isoindoline-1,3-dione (GT-09951)

With reference to the method of Scheme 4, the target compound (GT-09951) was obtained as a white solid (16 mg, yield 27 %). ¹H NMR (400 MHz, DMSO) δ 11.15 (s, 1H), 8.01 (d, *J* = 7.6 Hz, 1H), 7.93 - 7.85 (m, 2H), 7.70 - 7.19 (m, 9H), 5.19 (dd, *J* = 12.7, 5.4 Hz, 1H), 4.86 - 4.11 (m, 2H), 3.68 - 3.54 (m, 4H), 3.45 - 3.38 (m, 2H), 3.36 - 2.97 (m, 4H), 2.96 - 2.75 (m, 3H), 2.66 - 2.55 (m, 2H), 2.26 - 2.14 (m, 1H), 2.10 - 1.94 (m, 2H), 1.74 (s, 2H). LCMS (ESI) calcd. for C₃₆H₃₈N₅O₅ ⁺ [M+H]⁺: 620.29, found, 620.4.

### Example 257: Preparation of 3-(5-(4-(4-(2,3-dichlorophenyl)-3,6-dihydropyridin-1(2H)-yl)-3,3-difluoropiperidine-1-carbonyl)-1-oxoisoindolin-2-yl)piperidine-2,6-dione (GT-09952)

With reference to the method of Scheme 4, the target compound (GT-09952) was obtained as a white solid (18 mg, yield 33 %). ¹H NMR (400 MHz, DMSO) δ 11.02 (s, 1H), 7.84 (d, *J* = 7.8 Hz, 1H), 7.72 - 7.51 (m, 3H), 7.38 (t, *J* = 7.8 Hz, 1H), 7.26 (d, *J* = 14.0, 7.0 Hz, 1H), 5.76 (s, 1H), 5.15 (dd, 1H), 4.85 - 4.39 (m, 3H), 4.00 - 3.60 (m, 5H), 3.43 - 3.26 (m, 3H), 3.13 - 2.84 (m, 2H), 2.66 - 2.56 (m, 2H), 2.44 - 2.36 (m, 1H), 2.11 - 1.96 (m, 2H). LCMS (ESI) calcd. for C₃₀H₂₉Cl₂F₂N₄O₄ ⁺ [M+H]⁺: 617.15, found, 617.2.

### Example 258: Preparation of 3-(5-(4-(4-(2,3-dichlorophenyl)-3,6-dihydropyridin-1(2H)-yl)-3,3-difluoropiperidine-1-carbonyl)-7-fluoro-1-oxoisoindolin-2-yl)piperidine-2,6-dione (GT-09953)

With reference to the method of Scheme 4, the target compound (GT-09953) was obtained as a white solid (19 mg, yield 34 %). ¹H NMR (400 MHz, DMSO) δ 11.03 (s, 1H), 7.61 (d, *J* = 8.0, 1.2 Hz, 1H), 7.51 (s, 1H), 7.42 - 7.31 (m, 2H), 7.30 - 7.22 (m, 1H), 5.76 (s, 1H), 5.12 (dd, *J* = 13.3, 4.3 Hz, 1H), 4.93 - 4.59 (m, 1H), 4.55 (d, *J* = 18.2 Hz, 1H), 4.42 (d, *J* = 18.0, 3.1 Hz, 1H), 4.16 - 3.60 (m, 6H), 3.39 - 3.22 (m, 2H), 3.07 (s, 1H), 2.96 - 2.87 (m, 1H), 2.61 (d, *J* = 16.7 Hz, 2H), 2.46 - 2.37 (m, 1H), 2.15 - 1.94 (m, 2H). LCMS (ESI) calcd. for C₃₀H₂₈Cl₂F₃N₄O₄ ⁺ [M+H]⁺: 635.14, found, 635.2.

### Example 259: Preparation of 3-(5-(4-(4-(2,3-dichlorophenyl)-3,6-dihydropyridin-1(2H)-yl)-3,3-difluoropiperidine-1-carbonyl)-6-fluoro-1-oxoisoindolin-2-yl)piperidine-2,6-dione (GT-09954)

With reference to the method of Scheme 4, the target compound (GT-09954) was obtained as a white solid (12 mg, yield 22 %). ¹H NMR (400 MHz, DMSO) δ 11.03 (s, 1H), 7.74 - 7.63 (m, 2H), 7.63 - 7.58 (m, 1H), 7.38 (t, *J* = 7.9 Hz, 1H), 7.31 - 7.19 (m, 1H), 5.76 (s, 1H), 5.15 (dd, *J* = 13.2, 5.0 Hz, 1H), 4.91 - 4.61 (m, 1H), 4.45 (dd, *J* = 48.4, 17.4 Hz, 2H), 4.21 - 3.55 (m, 7H), 3.22 - 3.01 (m, 1H), 2.97 - 2.87 (m, 1H), 2.61 (d, *J* = 16.7 Hz, 2H), 2.48 - 2.20 (m, 2H), 2.11 - 1.89 (m, 2H). LCMS (ESI) calcd. for C₃₀H₂₈Cl₂F₃N₄O₄ ⁺ [M+H]⁺: 635.14, found, 635.2.

### Example 260: Preparation of 3-(4-(4-(4-(2,3-dichlorophenyl)-3,6-dihydropyridin-1(2H)-yl)-3,3-difluoropiperidine-1-carbonyl)-1-oxoisoindolin-2-yl)piperidine-2,6-dione (GT-09955)

With reference to the method of Scheme 4, the target compound (GT-09955) was obtained as a white solid (17 mg, yield 31 %). ¹H NMR (400 MHz, DMSO) δ 11.00 (d, *J* = 3.2 Hz, 1H), 7.94 - 7.82 (m, 1H), 7.74 - 7.51 (m, 3H), 7.39 (t, *J* = 7.8 Hz, 1H), 7.27 (d, *J* = 7.6, 1.4 Hz, 1H), 5.76 (s, 1H), 5.24 - 5.06 (m, 1H), 4.52 - 4.34 (m, 2H), 3.86 (s, 3H), 3.70 - 3.60 (m, 2H), 3.45 - 3.09 (m, 4H), 2.99 - 2.84 (m, 2H), 2.59 (d, *J* = 17.5 Hz, 2H), 2.41 (d, *J* = 13.5 Hz, 1H), 2.13 - 1.93 (m, 2H). LCMS (ESI) calcd. for C₃₀H₂₉Cl₂F₂N₄O₄ ⁺ [M+H]⁺: 617.15, found, 617.3.

### Example 261: Preparation of 3-(6-(4-(4-(2,3-dichlorophenyl)-3,6-dihydropyridin-1(2H)-yl)-3,3-difluoropiperidine-1-carbonyl)-1-oxoisoindolin-2-yl)piperidine-2,6-dione (GT-09956)

With reference to the method of Scheme 4, the target compound (GT-09956) was obtained as a white solid (14 mg, yield 26 %). ¹H NMR (400 MHz, DMSO) δ 11.02 (s, 1H), 7.80 - 7.65 (m, 3H), 7.61 (d, *J* = 8.0, 1.4 Hz, 1H), 7.39 (t, *J* = 7.8 Hz, 1H), 7.27 (d, *J* = 7.7, 1.5 Hz, 1H), 5.76 (s, 1H), 5.15 (dd, *J* = 13.2, 5.1 Hz, 1H), 4.48 (dd, *J* = 52.1, 17.8 Hz, 2H), 4.02 - 3.69 (m, 4H), 3.65 - 3.55 (m, 3H), 3.32 - 3.03 (m, 2H), 3.01 - 2.87 (m, 2H), 2.69 - 2.56 (m, 2H), 2.46 - 2.38 (m, 1H), 2.14 - 1.99 (m, 2H). LCMS (ESI) calcd. for C₃₀H₂₉Cl₂F₂N₄O₄ ⁺ [M+H]⁺: 617.15, found, 617.2.

### Example 262: Preparation of 3-(7-(4-(4-(2,3-dichlorophenyl)-3,6-dihydropyridin-1(2H)-yl)-3,3-difluoropiperidine-1-carbonyl)-1-oxoisoindolin-2-yl)piperidine-2,6-dione (GT-09957)

With reference to the method of Scheme 4, the target compound (GT-09957) was obtained as a white solid (15 mg, yield 28 %). ¹H NMR (400 MHz, DMSO) δ 11.01 (d, *J* = 5.0 Hz, 1H), 7.80 - 7.66 (m, 2H), 7.60 (d, *J* = 8.0 Hz, 1H), 7.46 - 7.32 (m, 2H), 7.26 (d, *J* = 7.5 Hz, 1H), 5.74 (s, 1H), 5.09 - 4.97 (m, 1H), 4.95 - 4.70 (m, 1H), 4.56 - 4.37 (m, 2H), 4.26 - 3.70 (m, 3H), 3.52 (s, 2H), 3.40 - 3.19 (m, 4H), 2.93 - 2.84 (m, 1H), 2.62 (d, *J* = 16.8 Hz, 2H), 2.46 - 2.36 (m, 1H), 2.12 - 1.90 (m, 2H). LCMS (ESI) calcd. for C₃₀H₂₉Cl₂F₂N₄O₄ ⁺ [M+H]⁺: 617.15, found, 617.2.

### Example 263: Preparation of 4-(4-(4-(2,3-dichlorophenyl)-3,6-dihydropyridin-1(2H)-yl)-3,3-difluoropiperidine-1-carbonyl)-2-(2,6-dioxopiperidin-3-yl)isoindoline-1,3-dione (GT-09958)

With reference to the method of Scheme 4, the target compound (GT-09958) was obtained as a white solid (20 mg, yield 36 %). ¹H NMR (400 MHz, DMSO) δ 11.14 (d, *J* = 5.5 Hz, 1H), 8.09 - 7.89 (m, 2H), 7.83 - 7.70 (m, 1H), 7.60 (d, *J* = 8.0 Hz, 1H), 7.37 (t, *J* = 7.8 Hz, 1H), 7.26 (d, *J* = 6.9 Hz, 1H), 5.74 (s, 1H), 5.27 - 5.05 (m, 1H), 4.95 - 4.62 (m, 1H), 4.33 - 3.52 (m, 6H), 3.36 - 2.99 (m, 4H), 2.96 - 2.84 (m, 1H), 2.61 (d, *J* = 18.9 Hz, 2H), 2.14 - 1.88 (m, 2H). LCMS (ESI) calcd. for C₃₀H₂₇Cl₂F₂N₄O₅ ⁺ [M+H]⁺: 631.13, found, 631.2.

### Example 264: Preparation of 5-(4-(4-(2,3-dichlorophenyl)-3,6-dihydropyridin-1(2H)-yl)-3,3-difluoropiperidine-1-carbonyl)-2-(2,6-dioxopiperidin-3-yl)isoindoline-1,3-dione (GT-09959)

With reference to the method of Scheme 4, the target compound (GT-09959) was obtained as a white solid (16 mg, yield 29 %). ¹H NMR (400 MHz, DMSO) δ 11.16 (s, 1H), 8.04 (d, *J* = 7.8 Hz, 1H), 7.91 (s, 2H), 7.60 (d, *J* = 8.0 Hz, 1H), 7.38 (t, *J* = 7.8 Hz, 1H), 7.31 - 7.21 (m, 1H), 5.75 (s, 1H), 5.20 (dd, *J* = 12.7, 5.4 Hz, 1H), 4.72 (d, *J* = 76.6 Hz, 1H), 4.05 - 3.59 (m, 5H), 3.41 - 3.19 (m, 3H), 3.14 - 2.84 (m, 2H), 2.66 - 2.53 (m, 3H), 2.13 - 1.96 (m, 2H). LCMS (ESI) calcd. for C₃₀H₂₇Cl₂F₂N₄O₅ ⁺ [M+H]⁺: 631.13, found, 631.2.

### Biological activity assay

### Reagents and Materials:

| Reagents and materials | Suppliers or Source |
|---|---|
| Human multiple myeloma cell line: MM.1S | ATCC (American Type Culture Collection) |
| Human multiple myeloma cell line resistant to dexamethasone: MM.1R | ATCC |
| Human mantle cell lymphoma cell line: JEKO-1 | ATCC |
| Human T lymphocytic leukemia cell line: Jurkat | ATCC |
| Human T cell acute lymphoblastic leukemia cell line: CCRF-CEM | Dalian Meilun Biotech Co., Ltd. |
| Human acute myeloid leukemia cell line: Kasumi-1 | ATCC |
| Human liver cancer cell: HEP3B2.1-7 | Dalian Meilun Biotech Co., Ltd. |
| Human breast cancer cell line: MCF-7 | ATCC |
| Human myeloid monocytic leukemia cell line MV-4-11 | ATCC |
| Human monocytic leukemia cell line THP-1 | ATCC |
| Human colon cancer cell line SW620 | ATCC |
| Human diffuse large B-cell lymphoma cell line TMD8 | Kyinno Biotechnology Co., Ltd |
| Human breast ductal cancer cell T47D | ATCC |
| Human prostate cancer cell Vcap | ATCC |
| Human acute T lymphocytic leukemia cell line Molt4 | Dalian Meilun Biotech Co., Ltd. |
| RPMI-1640 Medium | ATCC |
| MEM Medium | Dalian Meilun Biotech Co., Ltd. |
| IMDM Medium | Gibco Company |
| EMEM Medium | ATCC |
| DMEM high-glucose medium | ATCC |
| L-15 Medium | ATCC |
| Meilun Cell Counting Kit-8 | Dalian Meilun Biotech Co., Ltd. |
| Fetal bovine serum (FBS) | Sunrise Science Products Company |
| Penicillin-Streptomycin | |
| | Beijing TransGen Biotech Company |
| Mycoplasma detection kit | Beijing TransGen Biotech Company |
| STR genotype detection | Shanghai Biowing Applied Biotechnology Co. Ltd. |

### Methods:

### Cell cultures

The tumor cells used herein were cultured in a cell culture medium listed in table 2 at 37°C in an incubator with 5% CO₂. Prior to experimentation, all cell lines used were correctly identified by STR profiling, and tested negative for mycoplasma contamination using a mycoplasma detection kit through routine screenings.

**Table 2. Tumor cells and cell culture medium used in the present disclosure**

| Cell line | Cell culture medium used |
|---|---|
| MM.1S | RPMI 1640 culture medium supplemented with 10% FBS, as well as penicillin at a final concentration of 100 U/mL and streptomycin at a final concentration of 100 µg/mL |
| MM.1R | |
| CCRF-CEM | |
| Kasumi-1 | |
| Jurkat | |
| Molt4 | |
| JEKO-1 | |
| THP-1 | |
| TMD8 | |
| T47D | |
| HEP3B2.1-7 | MEM culture medium supplemented with 10% FBS, as well as penicillin at a final concentration of 100 U/mL and streptomycin at a final concentration of 100 µg/mL |
| MV-4-11 | IMDM culture medium supplemented with 10% FBS, as well as penicillin at a final concentration of 100 U/mL and streptomycin at a final concentration of 100 µg/mL |
| MCF-7 | EMEM culture medium supplemented with 10% FBS, as well as penicillin at a final concentration of 100 U/mL and streptomycin at a final concentration of 100 µg/mL |
| SW620 | L-15 culture medium supplemented with 10% FBS, as well as penicillin at a final concentration of 100 U/mL and streptomycin at a final concentration of 100 µg/mL |
| Vcap | DMEM high-glucose medium supplemented with 10% FBS, as well as penicillin at a final concentration of 100 U/mL and streptomycin at a final concentration of 100 µg/mL |

### I. Determination of half inhibitory concentration (IC₅₀) of the compounds of the present disclosure against tumor cells

The IC₅₀ values of the compounds of the present disclosure (including the compounds in Table 1 and Examples) were determined using the CellTiter-Meiluncell Luminescent Cell Viability Assay Kit from Dalian Meilun Biotech Co., Ltd. The detailed procedure is as follows: Tumor cells were seeded in 96-well cell culture plates at the densities specified in Table 3. On the following day, the test compounds of the present disclosure were subjected to serial dilution. The dilution can be performed according to one of the following two methods:
(1) The first dilution method: The test compounds of the present disclosure (including the compounds in Table 1 and Examples) were subjected to 5-fold serial dilutions starting from a highest concentration of 2000 µM, resulting in a total of 9 concentrations from high to low (2000, 400, 80, 16, 3.2, 0.64, 0.128, 0.0256, 0.00512 µM). Subsequently, 0.5 µL of the test compound dilution was transferred into the 96-well plate containing the cells, with each well containing 100 µL of cell culture medium. This resulted in final test compound concentrations of 10, 2, 0.4, 0.08, 0.016, 0.0032, 0.00064, 0.000128, and 0.0000256 µM, respectively, or
(2) As an alternative, a second dilution method may be used: The test compounds of the present disclosure (including the compounds in Table 1 and Examples) were subjected to a 50-fold gradient dilution starting from a highest concentration of 100 µM, resulting in 2 concentrations (100 µM, 2 µM). In a 96-well plate, the diluted test compounds of the present disclosure and the corresponding detection reagent were added to each well such that the final test concentrations of the compounds in each well reach 500 nM and 10 nM, respectively, in order to evaluate the inhibitory activity of the test compounds on tumor cell proliferation at these two concentration levels. Below, the diluted 2 µM test compounds were used as an example to illustrate the experimental procedure (the operating steps for the 100 µM concentration and for the gradient concentrations obtained by the first dilution method were similar to those for the 2 µM test compounds).

0.5 µL of the aforementioned diluted compound of the present disclosure (including the compounds in Table 1 and Examples) was added to the seeded cells in 100 µL of medium, yielding a final test compound concentration of 10 nM. After the cells were treated with the compounds of the present disclosure for a specified period, the cell viability assay reagent was added to the culture medium according to the kit's instructions to determine cell viability. The negative control was DMSO, and the reference controls were corresponding commercial inhibitors immunomodulatory agents (including lenalidomide and pomalidomide). All controls were treated using the same procedure as the compounds of the present disclosure. The growth inhibition curves for the compounds of the present disclosure were plotted using Prism GraphPad software, and the IC₅₀ values were calculated therefrom.

**Table 3. Seeding protocol and treatment time for tumor cells**

| Cells | Cells seeding protocol | The times for treating cells with the compounds of the present disclosure (hours) |
|---|---|---|
| MM.1S | cells were seeded at a density of 10,000 cells per well in 100 µL of serum-containing RPMI-1640 medium per well | 96 hours |
| MM.1R | | |
| Jurkat | | |
| THP-1 | | |
| Molt4 | | |
| TMD8 | | |
| Vcap | cells were seeded at a density of 4,000 cells per well in 100 µL of serum-containing DMEM high-glucose medium per well | 96 hours |
| JEKO-1 | cells were seeded at a density of 4,000 cells per well in 100 µL of serum-containing RPMI-1640 medium per well | 96 hours |
| CCRF-CEM | | |
| T47D | | |
| Kasumi-1 | cells were seeded at a density of 10,000 cells per well in 100 µL of serum-containing RPMI-1640 medium per well | 72 hours |
| SW620 | cells were seeded at a density of 4,000 cells per well in 100 µL of serum-containing L-15 medium per well | 96 hours |
| MV-4-11 | cells were seeded at a density of 10,000 cells per well in 100 µL of serum-containing IMDM medium per well | 96 hours |
| MCF-7 | cells were seeded at a density of 4,000 cells per well in 100 µL of serum-containing EMEM medium per well | 96 hours |
| HEP3B2.1-7 | cells were seeded at a density of 2,000 cells per well in 100 µL of serum-containing MEM medium per well | 96 hours |

The results of tumor cell proliferation inhibition are presented in Tables I-1 through I-8 below.

**Table I-1. The Inhibitory activity (Inhibition rate %) of the compounds of the present disclosure on the proliferation of Human myeloid monocytic leukemia cell (MV-4-11)**

| Comp. No. /names | Inhibition Rate at 500 nM (%) | Inhibition Rate at 10 nM (%) | Comp. No. /names | Inhibition Rate at 500 nM (%) | Inhibition Rate at 10 nM (%) | Comp. No. /names | Inhibition Rate at 500 nM (%) | Inhibition Rate at 10 nM (%) |
|---|---|---|---|---|---|---|---|---|
| lenalidomide | 1a5 | 1a4 | GT-09600 | 1a10 | 1a11 | GT-09939 | 1a7 | 1a3 |
| pomalidomide | 1a5 | 1a3 | GT-09601 | 1a7 | 1a7 | GT-09896 | 1a7 | 1a5 |
| GT-05212 | 1a10 | 1a6 | GT-09602 | 1a11 | 1a7 | GT-09897 | 1a8 | 1a8 |
| GT-05333 | 1a10 | 1a5 | GT-09638 | 1a10 | 1a8 | GT-09900 | 1a10 | 1a6 |
| GT-09337 | 1a10 | 1a4 | GT-09645 | 1a7 | 1a6 | GT-09902 | 1a10 | 1a8 |
| GT-09338 | 1a10 | 1a8 | GT-09688 | 1a9 | 1a5 | GT-09903 | 1a9 | 1a5 |
| GT-09341 | 1a9 | 1a7 | GT-09656 | 1a11 | 1a10 | GT-09910 | 1a10 | 1a7 |
| GT-09343 | 1a7 | 1a7 | GT-09666 | 1a10 | 1a10 | GT-09911 | 1a10 | 1a8 |
| GT-09346 | 1a10 | 1a9 | GT-09668 | 1a8 | 1a4 | GT-09944 | 1a9 | 1a7 |
| GT-09349 | 1a7 | 1a7 | GT-09710 | 1a7 | 1a3 | GT-09946 | 1a10 | 1a7 |
| GT-09380 | 1a10 | 1a9 | GT-09835 | 1a8 | 1a5 | GT-09947 | 1a9 | 1a5 |
| GT-09417 | 1a10 | 1a6 | GT-09842 | 1a9 | 1a5 | GT-09952 | 1a7 | 1a4 |
| GT-09418 | 1a10 | 1a10 | GT-09850 | 1a8 | 1a5 | GT-09954 | 1a10 | 1a5 |
| GT-09431 | 1a8 | 1a4 | GT-09851 | 1a10 | 1a5 | GT-09955 | 1a10 | 1a3 |
| GT-09614 | 1a10 | 1a4 | GT-09934 | 1a9 | 1a7 | GT-09957 | 1a9 | 1a7 |
| GT-09599 | 1a10 | 1a10 | GT-09935 | 1a9 | 1a8 | | | |

| | | | | | | | | |
|---|---|---|---|---|---|---|---|---|
| Note: in table I-1, the symbols 1a1, 1a2, 1a3, 1a4, 1a5, 1a6, 1a7, 1a8, 1a9, 1a10, and 1a11 are defined as follows: 0 < 1a1 < 5%, 5% ≤ 1a2 < 10%, 10% ≤ 1a3 < 20%, 20% ≤ 1a4 < 30%, 30% ≤ 1a5 ≤ 40%, 40% < 1a6 < 50%, 50% ≤ 1a7 < 60%, 60% ≤ 1a8 < 70%, 70% ≤ 1a9 < 80%, 80% ≤ 1a10 < 100%, 100% ≤ 1a11 ≤ 105%. | | | | | | | | |

**Table I-2. The Inhibitory activity (Inhibition rate %) of the compounds of the present disclosure on the proliferation of Human multiple myeloma cell (MM.1S)**

| Comp. No. /names | Inhibition Rate at 500 nM (%) | Inhibition Rate at 10 nM (%) | Comp. No. /names | Inhibition Rate at 500 nM (%) | Inhibition Rate at 10 nM (%) | Comp. No. /names | Inhibition Rate at 500 nM (%) | Inhibition Rate at 10 nM (%) |
|---|---|---|---|---|---|---|---|---|
| lenalidomide | 1b5 | 1b4 | GT-09600 | 1b10 | 1b10 | GT-09657 | 1b9 | 1b4 |
| GT-05212 | 1b10 | 1b7 | GT-09602 | 1b10 | 1b7 | GT-09665 | 1b9 | 1b9 |
| GT-05333 | 1b10 | 1b7 | GT-09638 | 1b10 | 1b8 | GT-09666 | 1b10 | 1b10 |
| GT-09337 | 1b10 | 1b4 | GT-09645 | 1b10 | 1b10 | GT-09668 | 1b8 | 1b5 |
| GT-09346 | 1b9 | 1b4 | GT-09646 | 1b10 | 1b10 | GT-09835 | 1b7 | 1b4 |
| GT-09380 | 1b10 | 1b7 | GT-09679 | 1b9 | 1b5 | GT-09843 | 1b9 | 1b3 |
| GT-09417 | 1b10 | 1b6 | GT-09687 | 1b7 | 1b6 | GT-09902 | 1b10 | 1b4 |
| GT-09418 | 1b10 | 1b10 | GT-09695 | 1b7 | 1b5 | GT-09954 | 1b10 | 1b5 |
| GT-09431 | 1b8 | 1b4 | GT-09655 | 1b8 | 1b5 | GT-09955 | 1b9 | 1b4 |
| GT-09599 | 1b10 | 1b10 | GT-09656 | 1b10 | 1b10 | | | |

| | | | | | | | | |
|---|---|---|---|---|---|---|---|---|
| Note: in table I-2, the symbols 1b1, 1b2, 1b3, 1b4, 1b5, 1b6, 1b7, 1b8, 1b9, and 1b10 are defined as follows: 0 < 1b1 < 5%, 5% ≤ 1b2 < 10%, 10% ≤ 1b3 < 20%, 20% ≤ 1b4 < 32%, 32% ≤ 1b5 < 42%, 42% ≤ 1b6 < 50%, 50% ≤ 1b7 < 60%, 60% ≤ 1b8 < 70%, 70% ≤ 1b9 < 80%, 80% ≤ 1b10 ≤ 100%. | | | | | | | | |

**Table I-3. The Inhibitory activity (Inhibition rate %) of the compounds of the present disclosure on the proliferation of Human acute T lymphocytic leukemia cell (Molt4)**

| Comp. No. /names | Inhibitio n Rate at 500 nM (%) | Inhibitio n Rate at 10 nM (%) | Comp. No. /names | Inhibiti on Rate at 500 nM (%) | Inhibitio n Rate at 10 nM (%) | Comp. No. /names | Inhibiti on Rate at 500 nM (%) | Inhibitio n Rate at 10 nM (%) |
|---|---|---|---|---|---|---|---|---|
| lenalidom ide | 1c3 | 1c3 | GT-09418 | 1c11 | 1c11 | GT-09710 | 1c7 | 1c2 |
| pomalido mide | 1c1 | 1c1 | GT-09614 | 1c8 | 1c3 | GT-09842 | 1c11 | 1c7 |
| GT-05212 | 1c11 | 1c5 | GT-09599 | 1c11 | 1c11 | GT-09851 | 1c7 | 1c6 |
| GT-05333 | 1c11 | 1c6 | GT-09600 | 1c11 | 1c12 | GT-09900 | 1c8 | 1c3 |
| GT-09337 | 1c11 | 1c4 | GT-09602 | 1c11 | 1c4 | GT-09902 | 1c11 | 1c6 |
| GT-09346 | 1c9 | 1c5 | GT-09638 | 1c11 | 1c8 | GT-09910 | 1c9 | 1c4 |
| GT-09380 | 1c10 | 1c9 | GT-09656 | 1c11 | 1c11 | GT-09954 | 1c11 | 1c5 |
| GT-09417 | 1c10 | 1c6 | GT-09666 | 1c11 | 1c11 | GT-09955 | 1c11 | 1c4 |

| | | | | | | | | |
|---|---|---|---|---|---|---|---|---|
| Note: in table I-3, the symbols 1c1, 1c2, 1c3, 1c4, 1c5, 1c6, 1c7, 1c8, 1c9, 1c10, 1c11, and 1c12 are defined as follows: -7% < 1c1 < 0, 0 < 1c2 < 5%, 5% ≤ 1c3 < 10%, 10% ≤ 1c4 < 20%, 20% ≤ 1c5 < 30%, 30% ≤ 1c6 < 40%, 40% ≤ 1c7 < 50%, 50% ≤ 1c8 < 60%, 60% ≤ 1c9 < 70%, 70% ≤ 1c10 < 80%, 80% ≤ 1c11 ≤ 100%, 100% < 1c12 ≤ 105%. | | | | | | | | |

**Table I-4. The Inhibitory activity (Inhibition rate %) of the compounds of the present disclosure on the proliferation of Human colon cancer cell (SW620)**

| Comp. No. /names | Inhibitio n Rate at 500 nM (%) | Inhibitio n Rate at 10 nM (%) | Comp. No. /names | Inhibitio n Rate at 500 nM (%) | Inhibitio n Rate at 10 nM (%) | Comp. No. /names | Inhibitio n Rate at 500 nM (%) | Inhibitio n Rate at 10 nM (%) |
|---|---|---|---|---|---|---|---|---|
| lenalidom ide | 1d3 | 1d3 | GT-09417 | 1d9 | 1d3 | GT-09638 | 1d6 | 1d5 |
| pomalido mide | 1d1 | 1d1 | GT-09418 | 1d10 | 1d9 | GT-09656 | 1d10 | 1d9 |
| GT-05212 | 1d6 | 1d1 | GT-09431 | 1d6 | 1d3 | GT-09666 | 1d10 | 1d10 |
| GT-05333 | 1d7 | 1d6 | GT-09614 | 1d6 | 1d3 | GT-09902 | 1d8 | 1d2 |
| GT-09337 | 1d9 | 1d4 | GT-09599 | 1d9 | 1d7 | GT-09954 | 1d6 | 1d2 |
| GT-09346 | 1d7 | 1d3 | GT-09600 | 1d10 | 1d10 | GT-09955 | 1d6 | 1d1 |
| GT-09380 | 1d9 | 1d7 | GT-09602 | 1d10 | 1d4 | | | |

| | | | | | | | | |
|---|---|---|---|---|---|---|---|---|
| Note: in table I-4, the symbols 1d1, 1d2, 1d3, 1d4, 1d5, 1d6, 1d7, 1d8, 1d9, and 1d10 are defined as follows: 0 < 1d1 < 5%, 5% ≤ 1d2 < 10%, 10% ≤ 1d3 < 20%, 20% ≤ 1d4 < 30%, 30% ≤ 1d5 < 40%, 40% ≤ 1d6 < 50%, 50% ≤ 1d7 < 60%, 60% ≤ 1d8 < 70%, 70% ≤ 1d9 < 80%, 80% ≤ 1d10 ≤ 100%. | | | | | | | | |

**Table I-5. The Inhibitory activity (Inhibition rate %) of the compounds of the present disclosure on the proliferation of Human breast cancer cell (MCF-7)**

| Comp. No. /names | Inhibition Rate at 500 nM (%) | Inhibition Rate at 10 nM (%) | Comp. No. /names | Inhibition Rate at 500 nM (%) | Inhibition Rate at 10 nM (%) |
|---|---|---|---|---|---|
| lenalidomide | 1e5 | 1e5 | GT-09600 | 1e10 | 1e8 |
| pomalidomide | 1e1 | 1e1 | GT-09602 | 1e10 | 1e5 |
| GT-09380 | 1e8 | 1e5 | GT-09656 | 1e7 | 1e5 |
| GT-09417 | 1e8 | 1e6 | GT-09666 | 1e9 | 1e8 |
| GT-09418 | 1e11 | 1e9 | GT-09835 | 1e7 | 1e5 |

| | | | | | |
|---|---|---|---|---|---|
| Note: in table I-5, the symbols 1e1, 1e2, 1e3, 1e4, 1e5, 1e6, 1e7, 1e8, 1e9, 1e10, and 1e11 are defined as follows: -10% < 1e1 < 0, 0 < 1e2 < 5%, 5% ≤ 1e3 < 10%, 10% ≤ 1e4 < 20%, 20% ≤ 1e5 < 30%, 30% ≤ 1e6 < 40%, 40% ≤ 1e7 < 50%, 50% ≤ 1e8 < 60%, 60% ≤ 1e9 < 70%, 70% ≤ 1e10 < 80%, 80% ≤ 1e11 ≤ 100%. | | | | | |

**Table I-6. The Inhibitory activity (Inhibition rate %) of the compounds of the present disclosure on the proliferation of Human diffuse large B-cell lymphoma cell (TMD8)**

| Comp. No. /names | Inhibition Rate at 500 nM (%) | Inhibition Rate at 10 nM (%) | Comp. No. /names | Inhibition Rate at 500 nM (%) | Inhibition Rate at 10 nM (%) |
|---|---|---|---|---|---|
| lenalidomide | 1f3 | 1f3 | GT-09666 | 1f8 | 1f4 |
| pomalidomide | 1f4 | 1f2 | GT-09732 | 1f7 | 1f3 |
| GT-09418 | 1f10 | 1f4 | GT-09835 | 1f7 | 1f7 |
| GT-09600 | 1f6 | 1f3 | GT-09843 | 1f8 | 1f4 |
| GT-09649 | 1f10 | 1f9 | GT-09851 | 1f8 | 1f6 |

| | | | | | |
|---|---|---|---|---|---|
| Note: in table I-6, the symbols 1f1, 1f2, 1f3, 1f4, 1f5, 1f6, 1f7, 1f8, 1f9, and 1f10 are defined as follows: 0 < 1f1 < 5%, 5% ≤ 1f2 < 10%, 10% ≤ 1f3 < 20%, 20% ≤ 1f4 < 30%, 30% ≤ 1f5 < 40%, 40% ≤ 1f6 < 50%, 50% ≤ 1f7 < 60%, 60% ≤ 1f8 < 70%, 70% ≤ 1f9 < 80%, 80% ≤ 1f10 ≤ 100%. | | | | | |

**Table I-7. The Inhibitory activity (Inhibition rate %) of the compounds of the present disclosure on the proliferation of Human mantle cell lymphoma cell line (JEKO-1)**

| Comp. No. /names | Inhibiti on Rate at 500 nM (%) | Inhibitio n Rate at 10 nM (%) | Comp. No. /names | Inhibitio n Rate at 500 nM (%) | Inhibitio n Rate at 10 nM (%) | Comp. No. /names | Inhibiti on Rate at 500 nM (%) | Inhibitio n Rate at 10 nM (%) |
|---|---|---|---|---|---|---|---|---|
| lenalidomi de | 1g3 | 1g3 | GT-09380 | 1g6 | 1g4 | GT-09666 | 1g9 | 1g8 |
| pomalido mide | 1g4 | 1g2 | GT-09417 | 1g8 | 1g3 | GT-09842 | 1g7 | 1g6 |
| GT-05212 | 1g6 | 1g2 | GT-09418 | 1g10 | 1g8 | GT-09843 | 1g6 | 1g5 |
| GT-05333 | 1g6 | 1g5 | GT-09600 | 1g9 | 1g8 | GT-09902 | 1g6 | 1g3 |
| GT-09337 | 1g6 | 1g2 | GT-09602 | 1g10 | 1g4 | | | |

| | | | | | | | | |
|---|---|---|---|---|---|---|---|---|
| Note: in table I-7, the symbols 1g1, 1g2, 1g3, 1g4, 1g5, 1g6, 1g7, 1g8, 1g9, and 1g10 are defined as follows: 0 < 1g1 < 5%, 5% ≤ 1g2 < 10%, 10% ≤ 1g3 < 20%, 20% ≤ 1g4 < 30%, 30% ≤ 1g5 < 40%, 40% ≤ 1g6 < 50%, 50% ≤ 1g7 < 60%, 60% ≤ 1g8 < 70%, 70% ≤ 1g9 < 80%, 80% ≤ 1g10 ≤ 100%. | | | | | | | | |

**Table 1-8. The Inhibitory activity of the compounds of the present disclosure against tumor cells proliferation (IC₅₀, nM)**

| Comp. No. /names | MM.1 S | MM.1 R | CCRF-CEM | Kasum i-1 | Jurkat | Vcap | T47D | HEP3 B2.1-7 | THP-1 |
|---|---|---|---|---|---|---|---|---|---|
| lenalidomide | F | H | H | H | H | H | H | H | H |
| lenalidomide | D | H | H | H | H | H | H | H | H |
| GT-04376 | A++ | A++ | A++ | A | A+ | A | C | A++ | - |
| GT-04377 | A | A | A | D | B | C | - | A | - |
| GT-04378 | A++ | A++ | A+++ | A++ | A+++ | A+ | A+ | A++ | B |
| GT-04931 | C | - | - | - | - | - | - | - | - |
| GT-04932 | A+ | - | - | - | - | - | - | - | - |

| | | | | | | | | | |
|---|---|---|---|---|---|---|---|---|---|
| Note: in table 1-8, the symbols A+++, A++, A+, A, B, C, D, E, F, and G are defined as follows: 0 nM < A++++ ≤ 2 nM; 2 nM < A+++ ≤ 5 nM; 5 nM < A++ ≤ 10 nM; 10 nM < A+ ≤ 20 nM; 20 nM < A ≤ 50 nM; 50 nM < B ≤ 100 nM; 100 nM < C ≤ 200 nM; 200 nM < D ≤ 500 nM; 500 nM < E ≤ 1000 nM; 1000 nM < F ≤ 5000 nM; 5000 nM < G ≤ 10000 nM; 10000 nM<H. The symbol "-" indicates "not tested". | | | | | | | | | |

The experimental results demonstrate that the compounds of the present disclosure (including those listed in Table 1 and in Examples) can effectively inhibit the proliferation of tumor cells proliferation (as shown in Tables I-1 through 1-8), which are superior to those of the corresponding positive control drugs.

### II. Western Blot Assay

Conventional Western blot assay was performed to evaluate the effects of the compounds of the present disclosure on the expression of target proteins in cells.
(1) Cell Seeding and Protein Harvesting: Human peripheral blood mononuclear cells (hPBMCs) at a density of 1×10⁶ cells/mL were thawed according to the PBMC Recovery Protocol (Milecell Biotechnologies Inc, Shanghai). The cells were then treated with the compounds of the present disclosure (including the compounds listed in Table 1 and the Examples compounds) at concentrations of 50 nM and 500 nM. Controls included a negative control (DMSO) and a positive control (the commercial immunomodulatory drug lenalidomide), both processed following the identical experimental protocol as the compounds of the present disclosure. After 24 hours of compounds treatment, cells were harvested and lysed in RIPA protein lysate containing protease inhibitors on ice for 30 min, followed by centrifugation. The supernatant was aspirated as the extracted total cell protein. The protein concentration of the supernatant was determined using the conventional BCA (Bicinchoninic Acid) assay.
(2) To the collected supernatant, 5X SDS sample loading buffer was added, followed by denaturation at 95°C for 5 minutes. The denatured samples were then separated by SDS-PAGE electrophoresis on a polyacrylamide gel, transferred onto a nitrocellulose membrane (0.45 µm NC membrane), blocked with blocking buffer at room temperature for 1 hour, and subjected to antibody incubation and development procedures according to the antibody manufacturer's instructions (Cell Signaling Technology Company).

Half-Maximal Degradation Concentration value (the drug concentration required for degrading proteins by 50%, abbreviated as DC₅₀) reads method: comparing the grayscale values of Western blotting bands from drug-treated samples with those from blank DMSO-treated controls, and identifying the drug concentration range at which the grayscale value is half that of the DMSO-treated control bands.

DC₅₀ values can be calculated using ImageJ software to measure grayscale values of Western blotting bands from drug-treated samples. The concentration-response curve is fitted to estimate the drug concentration corresponding to half the grayscale value.

Target protein remaining (%) refers to the percentage of target protein remaining in cells after treatment with protein degrader compounds.

Calculation method for target protein remaining (%): Use ImageJ software to measure grayscale values of Western blotting bands from drug (protein degrader)-treated samples. The grayscale value of drug-treated bands is divided by that of internal control protein bands, and the resulting quotient is normalized to obtain the relative percentage of remaining target protein after protein degrader treatment.$Target protein degradation rate \left(%\right) = 1 - target protein remaining \left(%\right) .$

The effects of the compounds of the present disclosure (including the compounds listed in Table 1 and the Examples compounds) on substrate proteins expression in cells were shown in Table II-1 below.

**Table II-1 Degradation of substrate proteins WEE1, IKZF1, IKZF2, IKZF3, CK1α and GSPT1 by the compounds of the present disclosure**

| Comp. No./names | WEE1 | IKZF 1 | IKZF2 | IKZF3 | CK1a | GSPT1 |
|---|---|---|---|---|---|---|
| Lenalidomide | B | A | B | A | B | B |
| GT-04329 | B | B | A | A | A | A |
| GT-04330 | B | B | A | A | A | A |
| GT-04331 | B | B | A | B | B | A |
| GT-04376 | A | A | A | A | A | A |
| GT-04377 | A | A | A | A | A | A |
| GT-04378 | A | A | A | A | A | A |
| GT-04379 | A | A | B | A | B | A |
| GT-04380 | A | A | B | A | B | A |
| GT-04595 | A | A | A | A | B | A |
| GT-04596 | B | A | A | A | B | A |
| GT-04597 | B | B | A | A | B | A |
| GT-04598 | B | A | A | A | B | A |
| GT-04599 | B | B | A | A | B | A |
| GT-04604 | A | A | B | A | B | B |
| GT-04605 | A | A | B | A | B | B |
| GT-04606 | A | A | A | B | B | A |
| GT-04607 | B | A | A | B | B | A |
| GT-04609 | B | A | A | B | B | A |
| GT-04608 | B | A | A | B | B | A |
| GT-04931 | B | B | B | A | B | A |
| GT-04932 | A | A | A | A | B | A |
| GT-04941 | B | B | A | A | B | B |
| GT-04942 | B | B | B | B | B | A |
| GT-04989 | A | A | B | A | B | A |
| GT-05037 | A | A | A | A | B | A |
| GT-05082 | B | B | B | A | A | A |
| GT-05160 | B | B | B | B | B | A |
| GT-05208 | B | B | B | A | B | B |
| GT-05212 | A | A | A | A | B | A |
| GT-05257 | A | B | B | B | B | B |
| GT-05258 | B | B | A | B | B | B |
| GT-05333 | B | B | B | B | B | A |
| GT-05335 | B | B | B | B | B | A |
| GT-05270 | B | B | B | A | B | A |
| GT-05271 | B | B | A | A | B | A |
| GT-09356 | B | B | B | B | B | A |
| GT-09357 | A | B | B | B | B | A |
| GT-09358 | B | B | B | B | B | A |
| GT-09360 | A | A | A | B | B | B |
| GT-09361 | A | A | A | B | B | B |
| GT-09334 | B | B | B | A | B | B |
| GT-09335 | B | B | B | A | B | B |
| GT-09336 | B | B | B | A | B | A |
| GT-09337 | A | A | A | A | A | A |
| GT-09338 | B | B | B | B | A | A |
| GT-09339 | B | B | B | B | A | A |
| GT-09340 | B | B | B | B | A | A |
| GT-09341 | B | B | B | B | A | A |
| GT-09342 | B | B | B | B | A | A |
| GT-09343 | B | B | B | B | B | A |
| GT-09344 | B | B | B | B | B | A |
| GT-09345 | B | B | B | B | A | A |
| GT-09346 | A | B | A | B | A | A |
| GT-09347 | B | B | B | B | A | A |
| GT-09348 | B | B | B | A | A | A |
| GT-09350 | B | B | B | B | B | A |
| GT-09351 | A | B | B | B | A | A |
| GT-09352 | A | B | A | B | A | A |
| GT-09353 | A | B | A | A | A | A |
| GT-09378 | B | B | A | A | A | A |
| GT-09379 | B | B | B | A | B | B |
| GT-09380 | A | A | B | B | A | A |
| GT-09381 | B | B | B | B | B | A |
| GT-09382 | B | B | B | B | A | A |
| GT-09383 | B | B | B | B | A | A |
| GT-09384 | B | B | B | B | A | A |
| GT-09385 | A | B | B | B | A | A |
| GT-09386 | B | B | A | B | B | A |
| GT-09387 | A | B | A | B | B | A |
| GT-09388 | A | B | A | B | B | A |
| GT-09389 | A | B | B | B | B | A |
| GT-09390 | B | B | B | B | B | A |
| GT-09391 | A | A | A | A | B | A |
| GT-09392 | B | B | B | A | B | B |
| GT-09208 | A | A | B | B | B | A |
| GT-09189 | A | B | B | B | B | A |
| GT-09190 | B | B | B | B | B | A |
| GT-09191 | B | B | B | B | B | A |
| GT-09192 | B | B | A | A | A | A |
| GT-09193 | B | B | A | B | B | B |
| GT-09195 | B | A | A | A | B | B |
| GT-09196 | B | B | A | B | B | B |
| GT-09417 | A | A | A | A | A | A |
| GT-09418 | A | A | A | A | A | A |
| GT-09419 | B | B | B | B | B | A |
| GT-09420 | B | B | B | A | B | A |
| GT-09422 | A | A | B | B | A | A |
| GT-09430 | A | A | B | B | A | A |
| GT-09431 | A | A | B | B | A | A |
| GT-09432 | A | A | B | B | A | A |
| GT-09433 | A | A | B | B | A | A |
| GT-09575 | A | B | B | B | B | A |
| GT-09576 | A | B | B | B | B | A |
| GT-09614 | A | B | B | A | A | A |
| GT-09615 | A | B | B | B | B | A |
| GT-09616 | B | B | B | B | B | A |
| GT-09618 | A | B | B | B | B | B |
| GT-09619 | B | B | B | A | B | A |
| GT-09599 | A | A | A | A | A | A |
| GT-09600 | A | A | A | A | A | A |
| GT-09601 | B | B | B | B | B | A |
| GT-09602 | A | A | A | A | A | A |
| GT-09635 | B | B | B | B | B | A |
| GT-09636 | B | B | B | B | B | A |
| GT-09637 | B | B | B | B | B | A |
| GT-09638 | B | B | B | B | B | A |
| GT-09639 | B | B | B | B | B | A |
| GT-09640 | B | B | B | B | B | A |
| GT-09642 | A | B | B | B | B | B |
| GT-09644 | B | B | B | B | A | B |
| GT-09680 | A | A | B | B | B | A |
| GT-09687 | A | A | B | B | B | A |
| GT-09688 | A | B | A | A | B | A |
| GT-09689 | A | B | B | A | B | B |
| GT-09690 | A | B | B | A | B | B |
| GT-09691 | B | B | B | A | B | B |
| GT-09693 | B | B | A | B | B | B |
| GT-09694 | B | B | A | B | B | A |
| GT-09695 | B | B | B | B | B | A |
| GT-09655 | A | A | A | A | A | A |
| GT-09656 | A | A | A | A | A | A |
| GT-09657 | A | B | B | A | A | A |
| GT-09658 | A | B | B | B | A | A |
| GT-09659 | B | B | B | B | B | A |
| GT-09661 | B | B | B | A | A | A |
| GT-09662 | B | B | A | A | B | B |
| GT-09663 | B | B | A | A | B | B |
| GT-09664 | B | B | A | A | B | B |
| GT-09665 | B | A | A | A | B | A |
| GT-09666 | A | A | A | A | A | A |
| GT-09667 | A | A | A | A | A | A |
| GT-09668 | A | A | A | A | A | A |
| GT-09669 | B | B | B | B | B | A |
| GT-09670 | B | B | B | B | B | A |
| GT-09671 | A | B | A | A | A | A |
| GT-09672 | A | B | A | A | A | A |
| GT-09673 | B | B | B | B | B | A |
| GT-09674 | B | B | B | B | B | A |
| GT-09675 | A | A | B | B | B | A |
| GT-09676 | A | A | B | B | B | A |
| GT-09677 | A | A | B | A | A | A |
| GT-09678 | A | A | B | A | A | A |
| GT-09712 | B | B | A | A | B | A |
| GT-09713 | B | B | A | A | B | A |
| GT-09714 | B | B | A | A | B | A |
| GT-09715 | B | B | A | A | A | A |
| GT-09716 | B | B | A | A | A | A |
| GT-09717 | A | A | A | A | B | A |
| GT-09718 | B | B | B | A | B | A |
| GT-09719 | B | B | B | A | B | A |
| GT-09720 | B | B | B | B | B | A |
| GT-09721 | A | B | B | B | B | A |
| GT-09733 | B | B | B | B | B | A |
| GT-09726 | B | B | B | B | B | A |
| GT-09727 | B | B | B | B | B | A |
| GT-09728 | B | B | B | B | B | A |
| GT-09729 | B | B | B | B | B | A |
| GT-09731 | B | B | B | B | A | A |
| GT-09732 | B | B | B | B | A | A |
| GT-09863 | B | B | B | A | B | A |
| GT-09864 | B | B | B | B | A | A |
| GT-09828 | B | B | B | A | B | B |
| GT-09829 | B | B | B | A | B | B |
| GT-09830 | B | B | B | A | B | B |
| GT-09831 | B | B | A | A | B | B |
| GT-09832 | B | B | B | B | B | A |
| GT-09833 | B | B | B | B | B | A |
| GT-09834 | B | B | B | B | B | A |
| GT-09835 | B | B | A | A | A | A |
| GT-09836 | B | B | B | B | B | A |
| GT-09837 | B | B | B | B | A | A |
| GT-09838 | A | B | B | B | A | A |
| GT-09839 | B | B | B | B | A | A |
| GT-09840 | B | B | B | B | A | A |
| GT-09841 | B | B | B | B | A | A |
| GT-09842 | A | B | A | B | B | A |
| GT-09843 | B | A | A | A | A | A |
| GT-09847 | B | B | B | B | B | A |
| GT-09848 | A | B | B | B | B | A |
| GT-09849 | A | B | B | B | A | A |
| GT-09850 | A | B | B | B | A | A |
| GT-09851 | A | B | B | B | A | A |
| GT-09934 | A | B | B | A | B | A |
| GT-09936 | B | B | B | B | B | A |
| GT-09938 | B | B | B | A | A | A |
| GT-09939 | B | B | A | B | A | A |
| GT-09940 | B | B | B | B | A | A |
| GT-09941 | B | B | B | B | B | A |
| GT-09896 | B | B | B | B | B | A |
| GT-09897 | B | B | B | B | B | A |
| GT-09898 | B | A | B | B | B | A |
| GT-09899 | B | A | B | B | B | A |
| GT-09900 | B | A | B | B | B | A |
| GT-09901 | B | B | B | A | B | A |
| GT-09902 | A | B | B | B | A | A |
| GT-09903 | A | B | B | B | A | A |
| GT-09906 | B | B | A | B | B | B |
| GT-09907 | A | B | B | B | B | A |
| GT-09908 | A | B | B | B | B | B |
| GT-09909 | A | B | B | B | B | B |
| GT-099 10 | A | B | B | B | B | A |
| GT-09911 | B | B | A | B | A | A |
| GT-09912 | B | B | A | B | B | A |
| GT-09913 | B | B | A | B | B | A |
| GT-09914 | B | A | B | B | B | A |
| GT-09915 | B | B | A | B | B | A |
| GT-09944 | A | A | B | B | B | A |
| GT-09945 | B | B | B | B | B | A |
| GT-09946 | B | B | B | B | B | A |
| GT-09947 | B | B | B | B | B | A |
| GT-09948 | B | A | A | B | A | A |
| GT-09949 | B | A | A | B | A | A |
| GT-09950 | B | A | B | B | A | A |
| GT-09951 | A | A | A | B | A | A |
| GT-09952 | A | A | A | B | A | A |
| GT-09953 | A | A | A | A | A | A |
| GT-09954 | A | A | A | A | A | A |
| GT-09955 | A | A | A | A | A | A |
| GT-09956 | A | A | A | A | A | A |
| GT-09957 | A | A | A | A | A | A |
| GT-09958 | A | B | B | B | B | A |
| GT-09959 | A | B | B | B | B | A |

| | | | | | | |
|---|---|---|---|---|---|---|
| Note: In Table II-1, "A" is defined as 0% ≤ substrate protein remaining % ≤ 80%, and "B" is defined as 80%< substrate protein remaining %≤ 100%. | | | | | | |

The degradation effects of the compounds of the present invention on the target substrate proteins were shown in Table II-1 and Figure 1. From the experimental results in Table II-1 and Figure 1, it can be concluded that the compounds of the present invention significantly degrade the target substrate proteins (e.g., proteins WEE1, IKZF1/2/3, CK1α, and GSPT1 etc.). Compared to lenalidomide, the compounds of the present invention exhibited significantly enhanced efficiency in inducing substrate protein degradation and also showed selective induction of degradation, thereby potentially enabling their use in treating indications associated with these substrate proteins.

### III. Determination of CRBN-binding affinity of compounds of the present disclosure:

The CEREBLON BINDING kits (specification: 10,000 tests; product number: Catalog # 64BDCRBNPEH; CISBIO company) was used to determine the CRBN binding ability of the compounds to be tested through a HTRF method (homogeneous time-resolved fluorescence). The specific methods are as follows:
**1.** According to the instructions of the CEREBLON BINDING KITS, serial dilutions were performed for the test compounds of the present disclosure (including the compounds in Table 1 and Examples) and for lenalidomide. Specifically, diluent #9 (1X) solution was used as the diluent, and multiple concentration gradients were set. The dilution operation may be performed according to one of the following two methods:
   (1) The first dilution method: The test compounds of the present disclosure (including the compounds in Table 1 and Examples) were subjected to 5-fold serial dilutions starting from a highest concentration of 40 µM, resulting in a total of 7 concentrations from high to low (40, 8, 1.6, 0.32, 0.064, 0.0128, 0.00256 µM). Subsequently, the diluted test compounds and corresponding detection reagents were added to each well of a 96-well plate, resulting in final test compound concentrations of 10, 2, 0.4, 0.08, 0.016, 0.0032, and 0.00064 µM, respectively.
   (2) As an alternative, a second dilution method may be used: The test compounds of the present disclosure (including the compounds in Table 1 and Examples) were subjected to 4-fold gradient dilutions starting from a highest concentration of 8 µM, resulting in 2 concentrations (8 µM, 2 µM). In a 96-well plate, the diluted test compounds of the present disclosure and corresponding detection reagents were added to each well such that the final test concentrations of the compounds in each well reach 2 µM and 0.5 µM, respectively. The experimental procedure is described below using the diluted 8 µM test compound and lenalidomide solutions as examples.
**2.** 2.5 µL of the above 8 µM of the tested compounds and lenalidomide solution, as well as the same volume of diluent #9 (1X) solution (solvent control group, Std0) were added to each well of a 96-well plate, respectively. Then, 2.5 µL of human Cereblon WT GST-tagged protein solution was added to each well. Finally, 5 µL of the thoroughly mixed Thalidomide-Re reagent and GST Eu antibody working solution were added to each of the aforementioned wells. The final concentration of the tested compounds and lenalidomide in each well was 2 µM.
**3.** The blank control wells were sequentially added with 2.5 µL of diluent #9 (1X) solution, 2.5 µL of binding buffer, and 5 µL of thoroughly mixed Thalidomide-Re reagent and GST Eu antibody working solution.
**4.** After sealing and incubating the solutions in the aforementioned wells at room temperature for 3 hours, the absorbance values at emission wavelengths of 620 nm and 665 nm were detected by the HTRF method using a Spark microplate reader (V3.1 SP1).

The corresponding CRBN binding inhibition rate was calculated using the following method: ${R}_{compound} = OD 665 {nm}_{compound} / OD 620 {nm}_{compound} - OD 665 {nm}_{control} / OD 620 {nm}_{control}$ ${R}_{Std 0} = OD 665 {nm}_{Std 0} / OD 620 {nm}_{Std 0} - OD 665 {nm}_{control} / OD 620 {nm}_{control}$ $inhibition rate \left(%\right) = \left(1-{R}_{compound}/{R}_{Std 0}\right) * 100$
Note: OD 665 nm compound is the absorbance value of each well of the compounds to be tested of the present disclosure at an emission wavelength of 665 nm.
OD 620 nm compound is the absorbance value of each well of the compounds to be tested of the present disclosure at an emission wavelength of 620 nm.
OD 665 nm control is the absorbance value of the blank control well at an emission wavelength of 665 nm.
OD 620 nm control is the absorbance value of the blank control well at an emission wavelength of 620 nm.
OD 665 nm Std0 is the absorbance value of the solvent control well at an emission wavelength of 665 nm.
OD 620 nm Std0 is the absorbance value of the solvent control well at an emission wavelength of 620 nm.

The test results were shown in Tables III-1 and III-2 below.

**Table III-1. HTRF inhibitory activity (IC₅₀, µM) of the compounds of the present disclosure (including but not limited to the compounds in Table 1 and the Examples compounds)**

| Comp. No. | HTRF IC₅₀ (µM) | Comp. No. | HTRF IC₅₀ (µM) | Comp. No. | HTRF IC₅₀ (µM) | Comp. No. | HTRF IC₅₀ (µM) |
|---|---|---|---|---|---|---|---|
| Lenalidomide | e | GT-04379 | b | GT-04606 | c | GT-05042 | c |
| GT-04329 | b | GT-04595 | a | GT-04607 | b | GT-05105 | a |
| GT-04330 | b | GT-04596 | b | GT-04609 | c | GT-05160 | c |
| GT-04331 | c | GT-04597 | b | GT-04931 | a | GT-05208 | b |
| GT-04376 | c | GT-04598 | d | GT-04932 | a | GT-05212 | a |
| GT-04377 | c | GT-04604 | c | GT-04989 | a | GT-05333 | a |
| GT-04378 | d | GT-04605 | b | GT-05037 | b | GT-05335 | d |

| | | | | | | | |
|---|---|---|---|---|---|---|---|
| Note: in Table III-1, the symbols a1, a, b, c, d, and e are defined as follows: 0<a0≤0.1µM, 0.1µM<a≤0.5µM, 0.5µM<b≤1µM, 1µM<c≤1.5µM, 1.5µM<d<1.9µM, 1.9µM ≤ e ≤10µM, 10µM < f. | | | | | | | |

**Table III-2: Screening of CRBN Binding Affinity of the compounds of the present disclosure (including but not limited to the compounds in Table 1 and Examples Compounds) at Concentrations of 2 µM and 0.5 µM**

| Comp. No. | Inhibitio n Rate at 2 µM (%) | Inhibitio n Rate at 0.5 µM (%) | Comp. No. | Inhibitio n Rate at 2 µM (%) | Inhibitio n Rate at 0.5 µM (%) | Comp. No. | Inhibitio n Rate at 2 µM (%) | Inhibiti on Rate at 0.5 µM (%) |
|---|---|---|---|---|---|---|---|---|
| Lenalidomi de | a4 | a2 | GT-09637 | a8 | a4 | GT-09732 | a6 | a3 |
| GT-09336 | a7 | a6 | GT-09638 | a7 | a5 | GT-09861 | a8 | a4 |
| GT-09337 | a7 | a5 | GT-09641 | a7 | a4 | GT-09862 | a7 | a3 |
| GT-09338 | a8 | a5 | GT-09642 | a6 | a4 | GT-09863 | a6 | a3 |
| GT-09339 | a8 | a6 | GT-09643 | a7 | a4 | GT-09864 | a6 | a2 |
| GT-09340 | a7 | a3 | GT-09644 | a5 | a2 | GT-09829 | a5 | a1 |
| GT-09341 | a6 | a3 | GT-09645 | a5 | a2 | GT-09830 | a5 | a2 |
| GT-09342 | a8 | a7 | GT-09647 | a8 | a6 | GT-09833 | a5 | a2 |
| GT-09343 | a8 | a7 | GT-09648 | a8 | a7 | GT-09834 | a5 | a2 |
| GT-09346 | a8 | a6 | GT-09679 | a8 | a5 | GT-09835 | a6 | a3 |
| GT-09347 | a8 | a5 | GT-09680 | a5 | a2 | GT-09836 | a5 | a3 |
| GT-09348 | a8 | a5 | GT-09687 | a8 | a4 | GT-09841 | a7 | a4 |
| GT-09349 | a8 | a6 | GT-09688 | a8 | a4 | GT-09842 | a5 | a2 |
| GT-09350 | a8 | a3 | GT-09689 | a8 | a5 | GT-09843 | a5 | a2 |
| GT-09351 | a8 | a6 | GT-09690 | a8 | a5 | GT-09844 | a6 | a3 |
| GT-09352 | a6 | a2 | GT-09691 | a7 | a4 | GT-09845 | a5 | a2 |
| GT-09353 | a5 | a1 | GT-09692 | a8 | a4 | GT-09848 | a6 | a3 |
| GT-09378 | a8 | a6 | GT-09693 | a6 | a3 | GT-09849 | a6 | a3 |
| GT-09379 | a7 | a3 | GT-09694 | a7 | a4 | GT-09851 | a6 | a3 |
| GT-09380 | a6 | a2 | GT-09695 | a7 | a4 | GT-09934 | a8 | a4 |
| GT-09381 | a7 | a4 | GT-09655 | a6 | a5 | GT-09935 | a8 | a4 |
| GT-09383 | a6 | a3 | GT-09657 | a8 | a5 | GT-09936 | a6 | a4 |
| GT-09385 | a8 | a7 | GT-09660 | a8 | a4 | GT-09938 | a5 | a2 |
| GT-09386 | a7 | a2 | GT-09661 | a8 | a5 | GT-09940 | a5 | a3 |
| GT-09389 | a5 | a3 | GT-09662 | a7 | a4 | GT-09941 | a8 | a6 |
| GT-09390 | a8 | a8 | GT-09663 | a5 | a2 | GT-09900 | a6 | a4 |
| GT-09391 | a8 | a6 | GT-09669 | a5 | a3 | GT-09901 | a7 | a3 |
| GT-09188 | a5 | a2 | GT-09671 | a8 | a8 | GT-09902 | a7 | a3 |
| GT-09190 | a8 | a7 | GT-09675 | a6 | a3 | GT-09903 | a8 | a4 |
| GT-09191 | a8 | a4 | GT-09676 | a7 | a3 | GT-09904 | a7 | a3 |
| GT-09195 | a8 | a6 | GT-09677 | a6 | a2 | GT-09905 | a6 | a3 |
| GT-09196 | a8 | a5 | GT-09678 | a6 | a2 | GT-09908 | a8 | a6 |
| GT-09419 | a7 | a4 | GT-09710 | a6 | a3 | GT-09909 | a8 | a5 |
| GT-09422 | a5 | a2 | GT-09714 | a6 | a4 | GT-09910 | a8 | a4 |
| GT-09430 | a5 | a4 | GT-09715 | a6 | a3 | GT-09911 | a8 | a6 |
| GT-09431 | a6 | a4 | GT-09716 | a6 | a3 | GT-09912 | a7 | a4 |
| GT-09575 | a8 | a6 | GT-09717 | a6 | a3 | GT-09913 | a7 | a4 |
| GT-09576 | a6 | a3 | GT-09718 | a6 | a3 | GT-09944 | a6 | a3 |
| GT-09614 | a7 | a4 | GT-09722 | a8 | a6 | GT-09945 | a6 | a3 |
| GT-09615 | a8 | a5 | GT-09723 | a8 | a6 | GT-09946 | a6 | a4 |
| GT-09616 | a8 | a5 | GT-09724 | a8 | a5 | GT-09947 | a6 | a3 |
| GT-09617 | a6 | a4 | GT-09733 | a8 | a7 | GT-09948 | a5 | a2 |
| GT-09618 | a8 | a5 | GT-09725 | a8 | a6 | GT-09952 | a6 | a3 |
| GT-09619 | a8 | a5 | GT-09726 | a7 | a5 | GT-09953 | a7 | a4 |
| GT-09599 | a5 | a4 | GT-09727 | a5 | a2 | GT-09954 | a6 | a3 |
| GT-09600 | a5 | a3 | GT-09728 | a6 | a3 | GT-09955 | a7 | a3 |
| GT-09601 | a8 | a4 | GT-09729 | a6 | a1 | GT-09956 | a7 | a3 |
| GT-09636 | a6 | a4 | GT-09730 | a6 | a2 | GT-09957 | a6 | a3 |

| | | | | | | | | |
|---|---|---|---|---|---|---|---|---|
| Note: in table III-2, the symbols a1, a2, a3, a4, a5, a6, and a7 are defined as follows: 0% < a ≤ 10%, 10% < a1 ≤ 20%, 20% < a2 ≤ 30%, 30% < a3 ≤ 40%, 40% < a4 ≤ 53%, 53% < a5 ≤ 60%, 60% < a6 ≤ 70%, 70% < a7 ≤80%, 80% < a8 ≤100%. | | | | | | | | |

The results in Table III-1 and Table III-2 demonstrate that the compounds of the present disclosure (including but not limited to the compounds in Table 1 and Examples) exhibit lower or comparable IC₅₀ values, or higher inhibition rates compared to lenalidomide, indicating stronger binding affinity to CRBN.

### IV. TNF-α Activity Inhibition Assay

PBMCs cells were cultured at 37°C with 5% CO₂ and seeded in 96-well plates at 1×10⁷ cells/well. The compounds to be tested (including those listed in Table 1 and Examples compounds) were dissolved in DMSO and diluted to appropriate concentrations, ensuring that the final DMSO concentration in cell culture did not exceed 0.5%. After 1-hour incubation in medium with or without the compounds, the cells were stimulated with Lipopolysaccharide (LPS, 1 ng/ml) and continued to be cultured for 18-20 hours. Culture supernatants were then collected, diluted with serum-free medium, and analyzed for TNF-α levels using an ELISA kit. IC₅₀ values were calculated using GraphPad Prism 7.0.

TNF-α downregulation is a key mechanism for the anti-tumor action of immunomodulators. The compounds of the present disclosure demonstrate dose-dependent inhibition of TNF-α levels.

### V. Pharmacokinetic Study of the compounds of the present disclosure

The pharmacokinetic properties of the compounds of the present disclosure were evaluated using conventional pharmacokinetic experimental methods.

The test compounds of the present disclosure (including the compounds in Table 1 and the Examples compounds) were orally administered to experimental animals to evaluate their pharmacokinetic properties. The experimental animals used may be those commonly used in pharmacokinetic experiments, e.g., adult and healthy rodents (e.g., mice, rats (such as Sprague-Dawley (SD) rats), guinea pigs) or non-rodents (rabbits, dogs, and monkeys). The experimental animals used in this study were mice.

The experimental animals were divided into two groups: a control group (for collecting blank plasma) and an oral administration group (administered at a dose of 10 mg/kg or 30mg/kg). Blood samples were collected at predetermined time points after administration, such as 0.25 h, 0.5 h, 1 h, 2 h, 4 h, 8 h, and 24 h post-dose. Blank plasma was collected from the control group.

Prior to analysis, plasma samples were processed as follows: To 10 µL of plasma sample were added 10 µL of 50% acetonitrile and 200 µL of internal standard solution (containing 50 ng/mL dexamethasone in acetonitrile). For blank control plasma samples, no internal standard was added, instead an equivalent volume of acetonitrile was supplemented. The processed samples were vortex-mixed, centrifuged, and the supernatant was subjected to LC-MS/MS analysis.

After preparation of calibration curves and QC samples, the concentrations of compounds in plasma samples were determined by LC-MS/MS analysis. The pharmacokinetic parameters of the test compounds were calculated using the pharmacokinetic calculation software WinNonlin v8.1 with non-compartmental analysis based on the plasma concentration data obtained from LC-MS/MS.

The results demonstrate that the compounds of the present disclosure administered via oral route exhibited favorable drug metabolism and pharmacokinetic (DMPK) properties, indicating their potential as therapeutic agents for cancer patients.

### VI. Effects of the compounds of the present disclosure on the release of inflammatory cytokines (including TNF-α, IL-2, IL-5, IL-10, IL-1α, IL-1β, GM-CSF) from PBMCs induced by phytohemagglutinin or the eBioscience^{™} Cell Stimulation Mixture (PMA + Ionomycin)

The present inventors examined the effects of the compounds of the present disclosure on the release of a panel of inflammatory cytokines from peripheral blood mononuclear cells (PBMCs) triggered by phytohemagglutinin or the eBioscience^{™} Cell Stimulation Complex (containing PMA and Ionomycin). These inflammatory cytokines include TNF-α, IL-2, IL-5, IL-10, IL-1α, IL-6, IL-1β, granulocyte-macrophage colony-stimulating factor (GM-CSF), among others. Published studies suggest that these inflammatory cytokines may play important roles in the development and progression of cancer, autoimmune diseases, infectious diseases, and inflammatory diseases. In autoimmune diseases such as rheumatoid arthritis (RA), psoriatic arthritis (PsA), and ankylosing spondylitis (AS), the expression level of TNF-α is significantly elevated and contributes to disease pathogenesis by stimulating synovial cell proliferation and promoting inflammatory responses. IL-1α is associated with cancer, autoimmune diseases, infectious diseases, and other inflammatory disorders, and participates in the inflammatory processes of autoimmune diseases. In diseases such as rheumatoid arthritis and systemic lupus erythematosus, elevated IL-1β levels can reflect the degree and activity of the inflammatory response. IL-2, IL-5, IL-10, and GM-CSF may also be associated with various inflammatory and autoimmune diseases.

The following materials were used in this study:

### VI.1 Reagents and Consumable materials

| Name | Supplier or Source |
|---|---|
| Human PBMC cell | Milecell Biotechnologies Inc, Shanghai |
| eBioscience^{™} Cell Stimulation Mixture | Invitrogen |
| Phytohemagglutinin (PHA) | MCE |
| RPMI 1640 Medium | Gibco |
| Human IFN-γ CBA Flex Set E7 100Tst | Becton Dickinson Medical |
| | Devices (shanghai) co., Ltd. |
| Human IL-2 CBA Flex Set A4 100Tst | Becton Dickinson Medical |
| | Devices (shanghai) co., Ltd. |
| Human TNF-α CBA Flex Set D9 100Tst | Becton Dickinson Medical |
| | Devices (shanghai) co., Ltd. |
| Human IL-10 CBA Flex Set B7 100Tst | Becton Dickinson Medical |
| | Devices (shanghai) co., Ltd. |
| Human IL-6 CBA Flex Set A7 100Tst | Becton Dickinson Medical |
| | Devices (shanghai) co., Ltd. |
| Human IL-8 CBA Flex Set A9 100Tst | Becton Dickinson Medical |
| | Devices (shanghai) co., Ltd. |
| Human IL-1β CBA Flex Set B4 100Tst | Becton Dickinson Medical |
| | Devices (shanghai) co., Ltd. |
| Human IL-12/IL-23p40 CBA Flex Set E5 100Tst | Becton Dickinson Medical |
| | Devices (shanghai) co., Ltd. |
| Human IL-17A CBA Flex Set B5 100Tst | Becton Dickinson Medical |
| | Devices (shanghai) co., Ltd. |
| Human IL-1α CBA Flex Set D6 100Tst | Becton Dickinson Medical |
| | Devices (shanghai) co., Ltd. |
| Human IL-5 CBA Flex Set A6 100Tst | Becton Dickinson Medical |
| | Devices (shanghai) co., Ltd. |
| Human IL-13 CBA Flex Set E6 100Tst | Becton Dickinson Medical |
| | Devices (shanghai) co., Ltd. |
| Human IL-4 CBA Flex Set A5 100Tst | Becton Dickinson Medical |
| | Devices (shanghai) co., Ltd. |
| Human GM-CSF CBA Flex Set C9 100Tst | Becton Dickinson Medical |
| | Devices (shanghai) co., Ltd. |
| Sheath Fluid for Flow Cytometry Analysis (20L) | Becton Dickinson Medical |
| | Devices (shanghai) co., Ltd. |
| Human Solbl Ptein CBA Buf Kit 500Tst | Becton Dickinson Medical |
| | Devices (shanghai) co., Ltd. |

### VI.2 Methods

### VI.2.1 Cell Thawing and Plating

A complete medium was prepared and pre-warmed to 37°C. Cryovials containing the cells were retrieved from liquid nitrogen, and the area immediately below the cap-notch interface was firmly gripped with forceps. The cryovials were immersed in a 37°C water bath, and shook occasionally to facilitate thawing. The cell suspension was aspirated and transferred to a centrifuge tube containing 10 mL complete medium, followed by thorough mixing and centrifugation at 400 × g for 10 min at room temperature. The supernatant was discarded, and the pellet was resuspended in fresh complete medium. After cell counting and the cell density adjustment, the cell suspension was seeded in 96-well plates at a density of 1×10⁵ cells/well (75 µL/well) and incubated in an incubator for 2 h at 37°C with 5% CO₂ under high humidity conditions.

### VI.2.2 Compound Preparation and Addition to Cell Plates

1) The test compounds (including the compounds in Table 1 and the Examples compounds) were prepared as 10 mM stock solutions in DMSO.
2) The test compounds were serially diluted 10-fold in DMSO starting from 1 mM (highest concentration) to generate 2 concentration gradients.
3) The test compounds were further diluted in culture medium to five times their final working concentration intended for use.
4) The diluted compounds were added to designated wells according to the plate layout at 25 µL/well, achieving final concentrations starting from 1 µM with 10-fold serial dilution (2 concentration points in total).
5) After 1 h incubation, PHA or PMA+Ionomycin was added to the cell wells at 25 µL/well to achieve the following final concentrations: PHA at 1 µg/mL (for TNF-α, IL-10, IL-6, IL-1β, IFN-γ, IL-2, IL-12p40, IL-1α, IL-8), or PMA at 81 nM/mL (for IL-4, IL-5, IL-13, GM-CSF, and IL-17A), or Ionomycin at 1.34 µM/mL (for IL-4, IL-5, IL-13, GM-CSF, and IL-17A).
6) The plates were incubated in an incubator for 24 h at 37°C with 5% CO₂ under high humidity.

### Cell plate layout:

| | 1 | 2 | 3 | 4 | 5 | 6 | 7 | 8 | 9 | 10 | 11 | 12 |
|---|---|---|---|---|---|---|---|---|---|---|---|---|
| A | H₂O | | | | | | | | | | | |
| B | DMSO | PHA/ PMA+Ionomycin | Cmpd 1-5# (1 µM and 0.1µM, 2 points) | | | | | | | | | |
| C | Cmpd 6-11# (1 µM and 0.1µM, 2 points) | | | | | | | | | | | |
| D | Cmpd 12-17# (1 µM and 0.1µM, 2 points) | | | | | | | | | | | |
| E | Cmpd 18-23# (1 µM and 0.1µM, 2 points) | | | | | | | | | | | |
| F | Cmpd 24-29# (1 µM and 0.1µM, 2 points) | | | | | | | | | | | |
| G | Cmpd 30-35# (1 µM and 0.1µM, 2 points) | | | | | | | | | | | |
| H | Cmpd 36-41# (1 µM and 0.1µM, 2 points) | | | | | | | | | | | |

### VI.2.3 Detection

(1) The cell plate was centrifuged and the culture supernatant was aspirated. TNF-α, IL-10, IL-6, IL-1β, IFN-γ, IL-2, IL-12p40, IL-1α, IL-8, IL-4, IL-5, IL-13, GM-CSF, and IL-17A cytokines in the culture supernatant were detected according to the flow cytometry CBA assay instructions.
(2) The flow cytometry CBA assay was performed as follows. The standards and samples were diluted according to a certain ratio to allow accurate quantitative analysis in the detection. Mixing of capture beads: the capture beads were mixed with the samples or standards and incubated for 1 hour in the dark. Mixing of detection antibodies: the detection antibodies were mixed with the samples or standards and incubated for 2 hours in the dark. These two steps are the core of the flow cytometry CBA technology, by which the quantitative detection of cytokines was achieved through the binding of the beads to the target molecules. Flow cytometry detection: the mixture was detected using a flow cytometer. Fluorescent labels on the beads were excited by the laser of the flow cytometer, and the fluorescent signals were collected and analyzed. Thereby, the concentrations of cytokines in the samples were quantitatively determined.
(3) The data collected by the flow cytometer needed to be further analyzed. The software FACP was typically used for data processing to generate a detailed report of cytokine concentrations (TNF-α, IL-10, IL-6, IL-1β, IFN-γ, IL-2, IL-12p40, IL-1α, IL-8, IL-4, IL-5, IL-13, GM-CSF, and IL-17A). The inhibition rate (%) was calculated as [(Ac-As)/(Ac-Ab)]×100%.
   As: OA of the samples (cells + PHA/ PMA + Ionomycin + test compound)
   Ac: OA of positive cell control (cells + PHA/ PMA + Ionomycin + DMSO)
   Ab: OA of blank control (cells + culture medium + DMSO)
(4) IC₅₀ value was calculated as follows: using GraphPad Prism 6 software and applying the calculation formula XY-analysis/Nonlinear regression (curve fit)/Dose response-Inhibition/log (inhibitor) vs. response-Variable slope (four parameters) to perform IC₅₀ curve fitting and determine the IC₅₀ values.

The results were shown in Table IV below.

**Table IV: Inhibition Rate (%) of the Compounds of the present disclosure (including but not limited to the compounds in Table 1 and the Example compounds) on Inflammatory Cytokines at Concentrations of 1 µM and 0.1 µM**

| Comp. No. | **IL-2** | | **TNF-α** | | **GM-CSF** | | **IL-5** | | **IL-10** | | **IL-1α** | | **IL-1β** | |
|---|---|---|---|---|---|---|---|---|---|---|---|---|---|---|
| | 1µ M | 0.1µ M | 1µ M | 0.1µ M | 1µ M | 0.1µ M | 1µ M | 0.1µ M | 1µ M | 0.1µ M | 1µ M | 0.1µ M | 1µ M | 0.1µ M |
| lenalidom ide | IV1 | IV2 | IV4 | IV6 | IV4 | IV4 | IV7 | IV6 | IV4 | IV5 | IV1 0 | IV10 | IV1 2 | IV12 |
| GT-05105 | IV9 | IV4 | IV1 2 | IV4 | - | - | - | - | - | - | IV1 3 | IV8 | IV1 4 | IV9 |
| GT-05160 | IV7 | IV9 | - | - | - | - | - | - | - | - | - | - | - | - |
| GT-05208 | IV1 0 | IV12 | IV9 | IV9 | - | - | - | - | - | - | - | - | - | - |
| GT-05255 | IV1 1 | IV11 | IV7 | IV10 | - | - | - | - | - | - | - | - | - | - |
| GT-05256 | IV1 1 | IV11 | - | - | - | - | - | - | - | - | - | - | - | - |
| GT-05257 | IV8 | IV9 | - | - | - | - | - | - | - | - | - | - | - | - |
| GT-09355 | IV1 0 | IV11 | IV8 | IV9 | - | - | - | - | - | - | - | - | - | - |
| GT-09356 | IV1 0 | IV11 | IV9 | IV9 | - | - | IV8 | IV9 | - | - | - | - | - | - |
| GT-09358 | IV1 0 | IV10 | IV9 | IV10 | - | - | - | - | - | - | - | - | - | - |
| GT-09359 | IV9 | IV9 | - | - | - | - | IV9 | IV7 | - | - | - | - | - | - |
| GT-09361 | IV8 | IV9 | - | - | - | - | - | - | - | - | - | - | - | - |
| GT-09334 | IV9 | IV11 | IV8 | IV11 | IV8 | IV9 | IV8 | IV10 | IV7 | IV9 | IV5 | IV11 | - | - |
| GT-09335 | IV1 2 | IV11 | IV1 1 | IV8 | - | - | - | - | IV1 0 | IV6 | IV1 1 | IV4 | - | - |
| GT-09336 | - | - | IV1 2 | IV11 | - | - | IV1 0 | IV8 | IV1 0 | IV9 | IV1 3 | IV11 | - | - |
| GT-09338 | IV1 0 | IV9 | - | - | - | - | - | - | - | - | - | - | - | - |
| GT-09341 | IV7 | IV10 | - | - | IV9 | IV10 | IV8 | IV9 | - | - | - | - | - | - |
| GT-09342 | IV1 1 | IV9 | IV1 2 | IV8 | IV9 | IV9 | IV9 | IV10 | IV9 | IV6 | IV1 2 | IV6 | | |
| GT-09343 | IV1 0 | IV9 | - | - | IV1 0 | IV10 | IV9 | IV9 | - | - | - | - | - | - |
| GT-09344 | IV9 | IV8 | - | - | IV9 | IV8 | - | - | - | - | - | - | - | - |
| GT-09348 | IV8 | IV9 | IV1 0 | IV8 | IV8 | IV9 | - | - | IV9 | IV7 | - | - | - | - |
| GT-09349 | IV1 1 | IV11 | IV6 | IV9 | IV8 | IV10 | - | - | - | - | - | - | - | - |
| GT-09350 | IV9 | IV9 | - | - | IV9 | IV9 | - | - | - | - | - | - | - | - |
| GT-09351 | - | - | - | - | IV9 | IV9 | - | - | - | - | - | - | - | - |
| GT-09379 | - | - | - | - | IV8 | IV9 | IV8 | IV9 | - | - | - | - | - | - |
| GT-09381 | - | - | - | - | IV9 | IV9 | - | - | - | - | - | - | - | - |
| GT- | - | - | - | - | IV9 | IV9 | - | - | - | - | - | - | - | - |
| 09382 | | | | | | | | | | | | | | |
| GT-09383 | IV9 | IV9 | - | - | IV9 | IV8 | IV9 | IV8 | - | - | - | - | - | - |
| GT-09390 | - | - | IV9 | IV9 | - | - | - | - | - | - | - | - | - | - |
| GT-09391 | - | - | IV1 0 | IV9 | - | - | - | - | - | - | - | - | - | - |
| GT-09392 | IV7 | IV9 | IV8 | IV9 | - | - | - | - | - | - | - | - | - | - |
| GT-09208 | IV9 | IV10 | IV1 0 | IV8 | - | - | - | - | - | - | - | - | - | - |
| GT-09188 | IV9 | IV10 | IV9 | IV9 | - | - | - | - | - | - | - | - | - | - |
| GT-09189 | IV9 | IV9 | - | - | - | - | - | - | - | - | - | - | - | - |
| GT-09192 | IV1 0 | IV10 | IV9 | IV9 | IV7 | IV9 | - | - | - | - | - | - | - | - |
| GT-09193 | IV9 | IV10 | IV1 0 | IV10 | IV9 | IV10 | - | - | - | - | IV1 1 | IV10 | - | - |
| GT-09194 | IV1 0 | IV8 | IV1 0 | IV9 | IV9 | IV9 | - | - | IV9 | IV8 | - | - | - | - |
| GT-09195 | - | - | IV1 1 | IV10 | - | - | - | - | IV9 | IV8 | IV1 1 | IV11 | | |
| GT-09421 | IV9 | IV9 | - | - | IV9 | IV9 | - | - | - | - | - | - | - | - |
| GT-09430 | IV1 1 | IV9 | IV9 | IV8 | IV1 0 | IV10 | - | - | - | - | - | - | - | - |
| GT-09617 | - | - | IV1 0 | IV9 | IV9 | IV8 | - | - | - | - | IV1 1 | IV9 | - | - |
| GT-09641 | - | - | IV9 | IV9 | - | - | - | - | - | - | - | - | - | - |
| GT-09646 | IV8 | IV9 | - | - | - | - | - | - | - | - | - | - | - | - |
| GT-09647 | - | - | IV1 0 | IV12 | - | - | - | - | - | - | IV1 0 | IV13 | - | - |
| GT-09648 | - | - | IV1 0 | IV9 | - | - | - | - | - | - | - | - | - | - |
| GT-09649 | IV8 | IV9 | - | - | - | - | - | - | - | - | - | - | - | - |
| GT-09679 | - | - | IV9 | IV5 | - | - | - | - | - | - | IV1 3 | IV11 | IV1 3 | IV10 |
| GT-09660 | IV9 | IV10 | - | - | - | - | - | - | - | - | - | - | - | - |
| GT-09710 | IV1 0 | IV10 | - | - | - | - | - | - | - | - | - | - | - | - |
| GT-09711 | IV9 | IV9 | - | - | - | - | - | - | - | - | - | - | - | - |
| GT-09722 | IV1 0 | IV7 | - | - | - | - | - | - | - | - | - | - | - | - |
| GT-09724 | - | - | IV1 0 | IV5 | - | - | - | - | - | - | - | - | - | - |
| GT-09861 | IV7 | IV9 | - | - | - | - | - | - | - | - | - | - | - | - |
| GT-09862 | IV9 | IV9 | - | - | - | - | - | - | - | - | - | - | - | - |
| GT-09844 | IV9 | IV7 | IV9 | IV7 | - | - | - | - | - | - | - | - | - | - |
| GT- | - | - | - | - | - | - | IV9 | IV8 | - | - | - | - | - | - |
| 09905 | | | | | | | | | | | | | | |

| | | | | | | | | | | | | | | |
|---|---|---|---|---|---|---|---|---|---|---|---|---|---|---|
| Note: in table IV, the symbols IV1, IV2, IV3, IV4, IV5, IV6, IV7, IV8, IV9, IV10, IV11, IV12, IV13, and IV14 are defined as follows: -320% ≤ IV1 < -200%, -200% ≤ IV2 < -100%, -100% ≤ IV3 < - 50%, -50% ≤ IV4 < 0%, 0% < IV5 ≤ 10%, 10% < IV6 ≤ 20%, 20% < IV7 ≤ 30%, 30% < IV8 ≤ 40%, 40% < IV9 ≤ 50%, 50% < IV 10 ≤ 60%, 60% < IV11 ≤ 70%, 70% < IV12 ≤ 80%, 80% < IV13 ≤ 90%, 90% < IV14 ≤ 100%. The symbol "-" indicates "not tested". | | | | | | | | | | | | | | |

The experimental results show that the compounds of the present disclosure (including the compounds in Table 1 and the Example compounds) exhibit excellent regulatory capability in modulating the production levels of cytokines associated with inflammatory diseases and autoimmune diseases, with their inhibitory effects being more significant compared to the positive control drug lenalidomide. Lenalidomide elevates IL-2 or IL-10 levels, thereby inducing inflammatory responses and posing risks of adverse effects such as skin rash in clinical practice. In contrast, the compounds of the present disclosure inhibit or do not significantly stimulate IL-2 and IL-10, demonstrating greater advantages in treating immune system disorders. The compounds of the present disclosure (including the compounds in Table 1 and the Example compounds) hold broad prospects as potential therapeutics for various inflammatory diseases and autoimmune diseases, such as rheumatoid arthritis, ankylosing spondylitis, psoriasis and psoriatic arthritis, systemic lupus erythematosus, atopic dermatitis, and hidradenitis suppurativa.

### VII. Efficacy experiment of the compounds of the present disclosure on Psoriasis-like skin lesions induced by Imiquimod in Mice

The compounds of the present disclosure (test compounds, including the compounds in Table 1 and Examples) were administered orally via gavage once daily for 7 consecutive days to mice with imiquimod-induced psoriasis-like skin lesions on the back, in order to investigate the efficacy of the compounds on such lesions.

A total of 60 female C57BL/6J mice (purchased from Shanghai SLAC Laboratory Animal Co., Ltd.) were randomly divided into 6 groups (n=10 per group) based on body weight:

| Grou p | Drug | Number of Animals | Dose (mg/kg) | Administrat ion volume (mL/kg) | Administrati on concentration (mg/mL) | Route and Duration |
|---|---|---|---|---|---|---|
| 1 | Control: Vehicle | 10 | NA | 10 | NA | ig. qd. 7 days |
| 2 | Model: Vehicle | 10 | NA | 10 | NA | ig. qd. 7 days |
| 3 | Positive Control | 10 | 3 | 10 | 0.3 | sc. qd. 7 days |
| 4 | Low-dose test compounds group | 10 | 3 | 10 | 0.3 | ig. qd. 7 days |
| 5 | Medium-dose test compounds group | 10 | 10 | 10 | 1 | ig. qd. 7 days |
| 6 | High-dose test compounds group | 10 | 30 | 10 | 3 | ig. qd. 7 days |

| | | | | | | |
|---|---|---|---|---|---|---|
| Note: ig., oral gavage; sc., subcutaneous injection; qd., once daily. | | | | | | |

After the experimental animals were divided into groups, the back of all mice was shaved. Except for the control group, all other groups received topical application of imiquimod cream once daily for 7 consecutive days. Meanwhile, the vehicle control, positive control drug, or test compound was administered to each group according to the above table, once daily for 7 consecutive days.

All clinical symptoms of each animal were observed at the beginning and throughout the experiment. The animals' weights were measured and recorded every 2 days.

### Scoring

Gross Observation Scoring of Skin Lesions (PASI Method): during the last 3 days of modeling and drug administration, the changes in skin lesions of mice in each group were observed once daily. PASI Method: the erythema, scaling, and thickening/infiltration of the lesions were scored separately, and the scores were summed to obtain the total score. PASI Scoring Criteria: 0 = none; 1 = mild; 2 = moderate; 3 = severe; 4 = very severe.

The skin lesions were photographed once daily during the last 3 days of modeling and drug administration.

After the experiment, the mice were euthanized by CO2 inhalation. Three different areas of skin from the lesion site on the back were collected using a 6 mm punch, weighed, and recorded, and the average weight was calculated.

The skin tissue from the lesion site was homogenized and the levels of IL-23p19, IL-12p40, IL-17, and TNF-α were determined.

The local lesion tissue was fixed, stained with HE, and subjected to pathological scoring (based on epidermal thickness, degree of epidermal keratinization, thickness of the basal layer, and degree of dermal inflammatory cell infiltration). Scoring Criteria: 0 = none; 1 = mild; 2 = moderate; 3 = severe; 4 = very severe.

Experimental data were presented as Mean ± SD. Statistical analysis between two groups was performed using SPSS, with p < 0.05 considered statistically significant.

The experimental results demonstrated that the compounds of the present disclosure exhibited ameliorative effects on psoriasis-like skin lesions on the back of mice induced by imiquimod and can be used for the treatment of psoriasis.

### VIII. Efficacy Experiment of the compounds of the present disclosure on the CIA Model in Rats Induced by Type II Collagen

The compounds of the present disclosure (test compounds, including the compounds in Table 1 and Examples) were administered orally via gavage once daily for 21 consecutive days to Wistar rats with collagen-induced arthritis (CIA) that was induced using bovine type II collagen (Chondrex, Inc.), in order to investigate their efficacy in the rat arthritis model.

### Preparation of the Modeling Agent:

10 mg of CII (Immunization Grade Bovine Type II Collagen, purchased from Chondrex, Inc.) was dissolved in 5 mL of 0.05M acetic acid solution, allowing it to be fully dissolved, thereby preparing a solution with a concentration of 2 mg/mL, which was then stored at 4°C in the dark overnight. On the day of the experiment, the 2 mg/mL CII solution was mixed with an equal volume of a 4 mg/mL solution of Complete Freund's Adjuvant (CFA, also purchased from Chondrex, Inc.) on ice, and was thoroughly emulsified into an emulsion.

On day 0, 10 Wistar rats (purchased from Beijing Vital River Laboratory Animal Technology Co., Ltd.) were randomly selected as the blank control group and were not immunized. The remaining 100 Wistar rats were intradermally injected at the base of the tail with an emulsion prepared by mixing equal volumes of CII (2 mg/mL) and CFA (4 mg/mL) for the first immunization. A second booster immunization was administered to them on day 7. Between days 10 and 14, i.e., 3 to 7 days after the second booster immunization, 50 rats with an arthritis index (AI) score ranging from 1 to 2 were selected from the modeled animals and were divided into 5 groups based on their body weight, foot volume, and AI score, with 10 rats per group:

| Grou p | Drug | Number of Animals | Dose (mg/kg) | Administr ation volume (mL/kg) | Administr ation concentrat ion (mg/mL) | Route and Duration |
|---|---|---|---|---|---|---|
| 1 | Control: Vehicle | 10 | NA | 10 | NA | ig. qd. 21 days |
| 2 | Model: Vehicle | 10 | NA | 10 | NA | ig. qd. 21 days |
| 3 | Positive Control | 10 | 0.1 | 10 | 0.01 | sc. qd. 21 days |
| 4 | Low-dose test compounds group | 10 | 3 | 10 | 0.3 | ig. qd. 21 days |
| 5 | Medium-dose test compounds group | 10 | 10 | 10 | 1 | ig. qd. 21 days |
| 6 | High-dose test compounds group | 10 | 30 | 10 | 3 | ig. qd. 21 days |

| | | | | | | |
|---|---|---|---|---|---|---|
| Note: ig. = oral gavage; sc. = subcutaneous injection | | | | | | |

Between 3 and 7 days after the second booster immunization, when the AI score of the affected paws reached 1-2, administration was commenced. According to the table above, animals in each group received daily oral gavage (or subcutaneous injection) of either the vehicle control, positive control drug, or the test compounds once a day for 21 consecutive days.

Beginning on day 3 after the second booster immunization, the volume of both hind paws (or toes) was measured and recorded twice weekly.

Foot Volume Measurement: Before the measurement, a line was marked on the rat's ankle joint with a marker pen to indicate the position. After clean water was added to the instrument, the instrument's reading was reset to zero in preparation for the measurement. The hind limb of the rat was placed in the water so that the marked line at the ankle joint was at the surface of the liquid. The reading obtained by pressing the foot pedal at this time was the foot volume of the rat. After the measurement was completed, the foot pedal was pressed again to reset the instrument to zero, preparing it for the measurement of the next rat.

### Scoring

Arthritis Index (AI): The foot volume was measured twice a week, and the swelling of the four toes was scored simultaneously. Each foot was scored on a scale from 0 to 4, with a maximum score of 16 for each rat.

### Arthritis Index (AI) Scoring Criteria:

| Score | Degree |
|---|---|
| 0 | No swelling, normal appearance |
| 1 | Mild swelling or redness of the ankle, wrist, or finger joints |
| 2 | Moderate swelling or redness of the ankle, wrist, or finger joints |
| 3 | Severe swelling or redness of the ankle, wrist, or finger joints |
| 4 | Very severe swelling or redness of the ankle, wrist, or finger joints |

The day of starting drug administration is recorded as Day 0. Five days after starting drug administration, i.e., 2 hours after Day 5 administration, blood was collected from the jugular vein of the experimental animals, allowed to stand for more than 30 minutes, centrifuged to separate the serum, which was stored at -80°C. The levels of TNF-α, IL-1β, and IL-6 in serum were measured by ELISA.

After the experiment, the animals were euthanized by CO₂ inhalation. The spleen and thymus of the animals were collected and weighed, and the organ coefficients were calculated.

Pathological Examination: One side of the foot joint tissue was fixed in 10% neutral formalin solution, followed by decalcification and paraffin embedding to prepare pathological sections. The sections were then stained with hematoxylin and eosin (HE) for pathological observation.

### Observation Indices:

1. Infiltration of synovial inflammatory cells (lymphocytes, plasma cells);
2. Synovial tissue hyperplasia;
3. Synovial pannus;
4. Fibrinoid necrosis on the synovial surface.
Scoring and Counting Method: 0, 1, 2, 3, and 4 levels, where "0" indicates no observation; "1" is mild; "2" is moderate; "3" is severe; and "4" is extremely severe.

Experimental data were presented as Mean ± SEM. Data between two groups were analyzed using Excel-T test, with p<0.05 considered statistically significant. Scoring data were analyzed using the non-parametric Mann-Whitney U test in SPSS, with p<0.05 considered statistically significant.

The experimental results demonstrate that the compounds of the present disclosure regulate the serum inflammatory cytokines levels in the bovine type II collagen-induced rat model of collagen-induced arthritis (CIA), significantly ameliorate limb swelling in the rats, and are therefore applicable for the treatment of arthritis.

The above descriptions have shown and described the basic principles, main features, and advantages of the present invention. It should be understood by those skilled in the art that the present invention is not limited to the above Examples, and that various changes and modifications may be made therein without departing from the spirit and scope of the present invention. Such changes and modifications all fall within the scope of the present invention as claimed. The scope of protection of the present invention is defined by the appended claims and their equivalents.

## Claims

1. A compound of Formula (I), 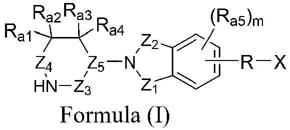 or salts, enantiomers, diastereoisomers, isotopically enriched analogs, solvates, or polymorphs thereof, wherein
Z₁ represents C(O), C(S), CH₂, or CD₂, and Z₂, Z₃, and Z₄ each independently represent C(O) or C(S);
Z₅ represents CH or N;
Rₐ₁, Rₐ₂, Rₐ₃, and Rₐ₄ each independently represent hydrogen, deuterium, halogen, C₁₋₆ alkyl, halogenated C₁₋₆ alkyl, deuterated C₁₋₆ alkyl, C₁₋₆ alkoxy, deuterated C₁₋₆ alkoxy or halogenated C₁₋₆ alkoxy;
(Rₐ₅)ₘ indicates that the isoindoline ring to which it is attached is optionally substituted with m Rₐ₅, with each Rₐ₅ being identical or different and each independently representing deuterium, halogen, hydroxy, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkenyl, or C₂₋₆ alkynyl;
m represents an integer of 0, 1, 2, or 3;
R represents C(O) or optionally substituted linear or branched C₁₋₅ alkylene;
X represents: 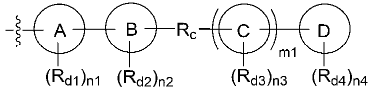
wherein ring A represents nitrogen-containing heterocyclylene, and (R_{d1})ₙ₁ indicates that the ring A is optionally substituted with n1 R_{d1} groups, wherein each R_{d1} independently represents deuterium, C₁₋₆ alkyl, deuterated C₁₋₆ alkyl, halogenated C₁₋₆ alkyl, C₁₋₆ alkoxy, halogenated C₁₋₆ alkoxy, deuterium, halogen, amino, hydroxy, mercapto, nitro, cyano, oxo, C₂₋₆ alkynyl, or C₂₋₆ alkenyl, and n1 represents an integer of 0-20;
ring B represents nitrogen-containing heterocyclylene, and (R_{d2})ₙ₂ indicates that the ring B is optionally substituted with n2 R_{d2} groups, wherein each R_{d2} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n2 represents an integer of 0-20;
R_{c} represents C(O), CR_{c1}R_{c2}, or a bond, wherein R_{c1} and R_{c2} each independently represent H, deuterium, halogen, optionally substituted linear or branched alkyl, optionally substituted cycloalkyl, optionally substituted heterocyclyl, optionally substituted aryl, or optionally substituted heteroaryl;
ring C represents cycloalkylene, heterocyclylene, arylene, or heteroarylene, m1 represents an integer of 0 or 1, and (R_{d3})ₙ₃ indicates that the ring C is optionally substituted with n3 R_{d3} groups, wherein each R_{d3} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n3 represents an integer of 0-20; and
ring D represents heterocyclyl, cycloalkyl, aryl, or heteroaryl, and (R_{d4})ₙ₄ indicates that the ring D is optionally substituted with n4 R_{d4} groups, and each R_{d4} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n4 represents an integer of 0-20.

2. The compound of Formula (I) or salts, enantiomers, diastereoisomers, solvates, or polymorphs thereof of claim 1, wherein
(i) Rₐ₁, Rₐ₂, Rₐ₃, and Rₐ₄ each independently represent H; and/or
(ii) R represents C(O) or optionally substituted linear or branched C₁₋₃ alkylene; and/or
(iii) X represents: 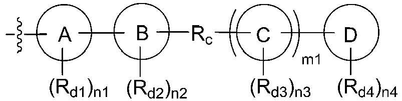
wherein the ring A represents 4- to 30-membered nitrogen-containing heterocyclylene (including 4- to 20-membered nitrogen-containing heterocyclylene, and 4- to 15-membered nitrogen-containing heterocyclylene), and (R_{d1})ₙ₁ indicates that the ring A is optionally substituted with n1 R_{d1} groups, wherein each R_{d1} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n1 represents an integer of 0-20;
the ring B represents 4- to 30-membered nitrogen-containing heterocyclylene, and (R_{d2})ₙ₂ indicates that the ring B is optionally substituted with n2 R_{d2} groups, wherein each R_{d2} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n2 represents an integer of 0-20;
R_{c} represents C(O), CR_{c1}R_{c2}, or a bond, wherein R_{c1} and R_{c2} each independently represent H, deuterium, halogen, optionally substituted linear or branched C₁₋₁₀ alkyl, optionally substituted C₃₋₃₀ cycloalkyl, optionally substituted C₅₋₃₀ aryl, optionally substituted 4-to 30-membered heterocyclyl, or optionally substituted 5- to 30-membered heteroaryl;
the ring C represents 4- to 30-membered heterocyclylene, C₃₋₃₀ cycloalkylene, C₅₋₃₀ arylene, or 5- to 30-membered heteroarylene, m1 represents an integer of 0 or 1, and (R_{d3})ₙ₃ indicates that the ring C is optionally substituted with n3 R_{d3} groups, wherein each R_{d3} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n3 represents an integer of 0-20; and/or
the ring D represents C₃₋₃₀ cycloalkyl, 4- to 30-membered heterocyclyl, C₅₋₃₀ aryl, or 5- to 30-membered heteroaryl, and (R_{d4})ₙ₄ indicates that the ring D is optionally substituted with n4 R_{d4} groups, wherein each R_{d4} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n4 represents an integer of 0-20.

3. The compound of Formula (I) or salts, enantiomers, diastereoisomers, solvates, or polymorphs thereof of claim 1 or 2, wherein
(i) the ring A represents 4- to 20-membered nitrogen-containing heterocyclylene (including 4-to 15-membered nitrogen-containing heterocyclylene), and (R_{d1})ₙ₁ indicates that the ring A is optionally substituted with n1 R_{d1} groups, wherein each R_{d1} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n1 represents an integer of 0-20; and/or
(ii) the ring B represents 4- to 20-membered nitrogen-containing heterocyclylene (including 4-to 15-membered nitrogen-containing heterocyclylene), and (R_{d2})ₙ₂ indicates that the ring B is optionally substituted with n2 R_{d2} groups, wherein each R_{d2} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n2 represents an integer of 0-20; and/or
(iii) the R_{c1} and R_{c2} each independently represent H, deuterium, halogen, optionally substituted linear or branched C₁₋₆ alkyl, optionally substituted C₃₋₂₀ cycloalkyl (including optionally substituted C₃₋₁₅ cycloalkyl), optionally substituted C₅₋₂₀ aryl (including optionally substituted C₅₋₁₅ aryl), optionally substituted 4- to 20-membered heterocyclyl (including optionally substituted 4- to 15-membered heterocyclyl), or optionally substituted 5- to 20-membered heteroaryl (including optionally substituted 5- to 15-membered heteroaryl) ; and/or
(iv) the ring C represents 4- to 20-membered heterocyclylene (including 4- to 15-membered heterocyclylene), C₃₋₂₀ cycloalkylene (including C₃₋₁₅ cycloalkylene), C₅₋₂₀ arylene (including C₅₋₁₅ arylene), or 5- to 20-membered heteroarylene (including 5- to 15-membered heteroarylene), m1 represents an integer of 0 or 1, and (R_{d3})ₙ₃ indicates that the ring C is optionally substituted with n3 R_{d3} groups, wherein each R_{d3} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n3 represents an integer of 0-20; and/or
(v) the ring D represents C₃₋₂₀ cycloalkyl (including C₃₋₁₅ cycloalkyl), 4- to 20-membered heterocyclyl (including 4- to 15-membered heterocyclyl), C₅₋₂₀ aryl (including C₅₋₁₅ aryl), or 5- to 20-membered heteroaryl (including 5- to 15-membered heteroaryl), and (R_{d4})ₙ₄ indicates that the ring D is optionally substituted with n4 R_{d4} groups, wherein each R_{d4} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, or C₂₋₆ alkenyl, and n4 represents an integer of 0-20.

4. The compound of Formula (I) or salts, enantiomers, diastereoisomers, solvates, or polymorphs thereof of any one of claims 1-3, wherein
(i) R represents C(O) or optionally substituted methylene; and/or
(ii) the ring A and the ring B each independently represent azetidinylene, pyrrolidinylene, imidazolidinylene, pyrazolidylene, piperidinylene, piperazinylene, azacycloheptanylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, quinuclidinylene, 2,6-diazaspiro[3.3]heptanylene, 2,7-diazaspiro[3.5]nonanylene, 2,8-diazaspiro[4.5]decanylene, 3,9-diazaspiro[5.5]undecanylene, 3-azaspiro[5.5]undecanylene, 7-azaspiro[3.5]nonanylene, 8-azaspiro[4.5]decanylene, or octahydropyrrolo[3,4-c]pyrrolylene, each optionally substituted with one or more (e.g., 1-10) substituents selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, and C₂₋₆ alkenyl; and/or
(iii) R_{c1} and R_{c2} each independently represent:
H, deuterium, halogen, or optionally substituted linear or branched C₁₋₁₀ alkyl; or
cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., C₅-C₂₀ spiro-cycloalkyl, such as spiro[3.3]heptyl, spiro[2.5]octyl, spiro[3.5]nonyl, spiro[4.4]nonyl, spiro[4.5]decyl, and spiro[5.5]undecyl), *p*-menthanyl, *m-*menthanyl, or bridged cycloalkyl (e.g., C₆-C₂₀ bridged cycloalkyl, such as adamantanyl, noradamantanyl, bornyl, norbornyl, bicyclo[2.2.1]heptanyl, 2-oxobicyclo[2.2.1]heptyl, or bicyclo[2.2.1]heptenyl), each optionally substituted with one or more (e.g., 1-10) substituents selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, and C₂₋₆ alkenyl; or
azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, tetrahydropyridinyl, dihydroxy-piperidinyl, difluoro-piperidinyl, morpholinyl, thiomorpholinyl, azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), and azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl, each optionally substituted with one or more (e.g., 1-10) substituents selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, and C₂₋₆ alkenyl; or
phenyl or naphthyl, each optionally substituted with one or more (e.g., 1-7) substituents selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, C₂₋₆ alkynyl, and C₂₋₆ alkenyl; or
furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, indolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl,quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno[2,3-d]pyrimidinyl, each optionally substituted with one or more (e.g., 1-10) substituents selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, C₂₋₆ alkynyl, and C₂₋₆ alkenyl; and/or
(iv) the ring C represents:
cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro-cycloalkylene (e.g., C₅-C₂₀ spiro-cycloalkylene, such as spiro[3.3]heptylene, spiro[2.5]octylene, spiro[3.5]nonylene, spiro[4.4]nonylene, spiro[4.5]decylene, spiro[5.5]undecylene), *p*-menthanylene, *m*-menthanylene, or bridged cycloalkylene (e.g., C₆-C₂₀ bridged cycloalkylene, such as adamantanylene, noradamantanylene, bornylene, norbornylene, bicyclo[2.2.1]heptylene, 2-oxobicyclo[2.2.1]heptylene, or bicyclo[2.2.1]heptentylene), each optionally substituted with one or more (e.g., 1-20) substituents selected from the group consisting of deuterium, halogen, hydroxy, mercapto, nitro, amino, cyano, oxo, C₁₋₆ alkyl, halogenated C₁₋₆ alkyl, deuterated C₁₋₆ alkyl, hydroxy-substituted C₁₋₆ alkyl, amino-substituted C₁₋₆ alkyl, C₁₋₆ alkoxy, deuterated C₁₋₆ alkoxy, halogenated C₁₋₆ alkoxy, C₂₋₆ alkenyl, and C₂₋₆ alkynyl; or
azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, tetrahydropyridinylene, dihydroxy-piperidinylene, difluoro-piperidinylene, morpholinylene, thiomorpholinylene, azacycloheptanylene, azacyclooctylene, dioxacyclohexylene, azacycloheptanylene, azacyclooctylene, diazacycloheptanylene (e.g., 1,4-diazacycloheptanylene, 4,5-diazacycloheptanylene, 1,3-diazacycloheptanylene), diazacyclooctylene, bridged heterocyclylene (e.g., 6- to 20-membered bridged heterocyclylene, such as 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and quinuclidinylene), azaspirocycloalkylene (e.g., 5- to 20-membered azaspirocycloalkylene, such as 2,6-diazaspiro[3.3]heptanylene, 2,7-diazaspiro[3.5]nonanylene, 2,8-diazaspiro[4.5]decanylene, 3,9-diazaspiro[5.5]undecanylene, 3-azaspiro[5.5]undecanylene, and 7-azaspiro[3.5]nonanylene), or octahydropyrrolo[3,4-c]pyrrolylene, each optionally substituted with one or more (e.g., 1-20) substituents selected from the group consisting of deuterium, halogen, hydroxy, mercapto, nitro, amino, cyano, oxo, C₁₋₆ alkyl, halogenated C₁₋₆ alkyl, deuterated C₁₋₆ alkyl, hydroxy-substituted C₁₋₆ alkyl, amino-substituted C₁₋₆ alkyl, C₁₋₆ alkoxy, deuterated C₁₋₆ alkoxy, halogenated C₁₋₆ alkoxy, C₂₋₆ alkenyl, and C₂₋₆ alkynyl; or
phenylene or naphthylene, each optionally substituted with one or more (e.g., 1-6) substituents selected from the group consisting of deuterium, halogen, hydroxy, mercapto, nitro, amino, cyano, C₁₋₆ alkyl, halogenated C₁₋₆ alkyl, deuterated C₁₋₆ alkyl, hydroxy-substituted C₁₋₆ alkyl, amino-substituted C₁₋₆ alkyl, C₁₋₆ alkoxy, deuterated C₁₋₆ alkoxy, halogenated C₁₋₆ alkoxy, C₂₋₆ alkenyl, and C₂₋₆ alkynyl; or
furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, triazinylene, indolylene, isoindolylene, indolinylene, benzofuranylene, chromanylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, 2-oxo-2,3-dihydro-1H-benzo[d]imidazolylene, benzo[b][1,4]oxazinylene, 3,4-dihydro-2H-benzo[b][1,4]oxazinylene, quinolinylene, isoquinolinylene, 1,2,3,4-tetrahydroquinolinylene, naphthyridinylene, cinnolinylene, quinazolinylene, quinoxalinylene, 1,2,3,4-tetrahydroquinoxalinylene, phthalazinylene, 5,6,7,8-tetrahydro-[1,2,4]triazolo[4,3-a]pyrazinylene, 4,5,6,7-tetrahydrothieno[3,2-c]pyridinylene, 5-oxo-6,7-dihydrothieno[3,2-d]pyrimidinylene, thieno[2,3-d]pyrimidinylene, thieno[3,2-d]pyrimidinylene, isoxazolo[4,5-c]pyridinylene, isoxazolo[4,5-c]pyrimidinylene, isoxazolo[4,5-d]pyrimidinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, imidazo[2,1-b]thiazolylene, 5,6,7,8-tetrahydrobenzo[4,5]thieno[2,3-d]pyrimidinylene, or 6,7-dihydro-5H-cyclopenta[4,5]thieno[2,3-d]pyrimidinylene, each optionally substituted with one or more (e.g., 1-20) substituents selected from the group consisting of deuterium, halogen, hydroxy, mercapto, nitro, amino, cyano, C₁₋₆ alkyl, halogenated C₁₋₆ alkyl, deuterated C₁₋₆ alkyl, hydroxy-substituted C₁₋₆ alkyl, amino-substituted C₁₋₆ alkyl, C₁₋₆ alkoxy, deuterated C₁₋₆ alkoxy, halogenated C₁₋₆ alkoxy, C₂₋₆ alkenyl, and C₂₋₆ alkynyl; and/or
(v) the ring D represents:
cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., C₅-C₂₀ spiro-cycloalkyl, such as spiro[3.3]heptyl, spiro[2.5]octyl, spiro[3.5]nonyl, spiro[4.4]nonyl, spiro[4.5]decyl, and spiro[5.5]undecyl), *p*-menthanyl, *m-*menthanyl, or bridged cycloalkyl (e.g., C₆-C₂₀ bridged cycloalkyl, such as adamantanyl, noradamantanyl, bornyl, norbornyl, bicyclo[2.2.1]heptanyl, 2-oxobicyclo[2.2.1]heptyl, or bicyclo[2.2.1]heptenyl), each optionally substituted with one or more (e.g., 1-20) substituents selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, and C₂₋₆ alkenyl; or
azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, tetrahydropyridinyl, dihydroxy-piperidinyl, difluoro-piperidinyl, morpholinyl, thiomorpholinyl, azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), and azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl, each optionally substituted with one or more (e.g., 1-20) substituents selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, and C₂₋₆ alkenyl; or
phenyl or naphthyl, each optionally substituted with one or more (e.g., 1-7) substituents selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, C₂₋₆ alkynyl, and C₂₋₆ alkenyl; or
furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, indolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl,quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno[2,3-d]pyrimidinyl, each optionally substituted with one or more (e.g., 1-20) substituents selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, C₂₋₆ alkynyl, and C₂₋₆ alkenyl.

5. The compound of Formula (I) or salts, enantiomers, diastereoisomers, solvates, or polymorphs thereof of any one of claims 1-4, wherein R represents:
-CH₂-, -(CH₂)₂-, -(CH₂)₃-, -(CH₂)₄-, or -(CH₂)₅-;
wherein the above groups are optionally substituted with a substituent selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, oxo, C₂₋₆ alkynyl, and C₂₋₆ alkenyl.

6. The compound of Formula (I) or salts, enantiomers, diastereoisomers, solvates, or polymorphs thereof of any one of claims 1-5, wherein X represents:

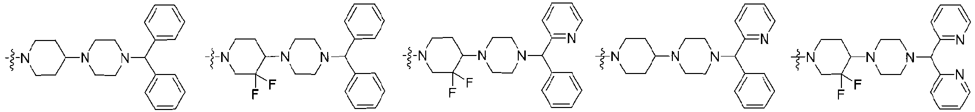

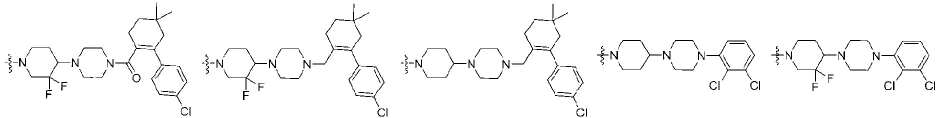

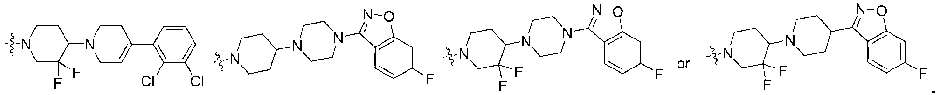

7. The compound of Formula (I) or salts, enantiomers, diastereoisomers, solvates, or polymorphs thereof of claim 1, which is selected from:
3-(5-((4-(4-benzhydrylpiperazin-1-yl)piperidin-1-yl)methyl)-1-oxoisoindolin-2-yl)piperidine-2,6-dione;
3-(5-((4-(4-benzhydrylpiperazin-1-yl)piperidin-1-yl)methyl)-4-fluoro-1-oxoisoindolin-2-yl)piperidine-2,6-dione;
3-(5-((4-(4-benzhydrylpiperazin-1-yl)piperidin-1-yl)methyl)-6-fluoro-1-oxoisoindolin-2-yl)piperidine-2,6-dione;
3-(5-((4-(4-benzhydrylpiperazin-1-yl)piperidin-1-yl)methyl)-7-fluoro-1-oxoisoindolin-2-yl)piperidine-2,6-dione;
3-(4-((4-(4-benzhydrylpiperazin-1-yl)piperidin-1-yl)methyl)-1-oxoisoindolin-2-yl)piperidine-2,6-dione;
3-(6-((4-(4-benzhydrylpiperazin-1-yl)piperidin-1-yl)methyl)-1-oxoisoindolin-2-yl)piperidine-2,6-dione;
3-(7-((4-(4-benzhydrylpiperazin-1-yl)piperidin-1-yl)methyl)-1-oxoisoindolin-2-yl)piperidine-2,6-dione;
3-(5-(2-(4-(4-benzhydrylpiperazin-1-yl)piperidin-1-yl)ethyl)-1-oxoisoindolin-2-yl)piperidine-2,6-dione;
3-(5-(3-(4-(4-benzhydrylpiperazin-1-yl)piperidin-1-yl)propyl)-1-oxoisoindolin-2-yl)piperidine-2,6-dione;
4-((4-(4-benzhydrylpiperazin-1-yl)piperidin-1-yl)methyl)-2-(2,6-dioxopiperidin-3-yl)isoindoline-1,3-dione;
5-((4-(4-benzhydrylpiperazin-1-yl)piperidin-1-yl)methyl)-2-(2,6-dioxopiperidin-3-yl)isoindoline-1,3-dione;
3-(5-((4-(4-benzhydrylpiperazin-1-yl)-3,3-difluoropiperidin-1-yl)methyl)-1-oxoisoindolin-2-yl)piperidine-2,6-dione;
3-(5-((4-(4-benzhydrylpiperazin-1-yl)-3,3-difluoropiperidin-1-yl)methyl)-4-fluoro-1-oxoisoindolin-2-yl)piperidine-2,6-dione;
3-(5-((4-(4-benzhydrylpiperazin-1-yl)-3,3-difluoropiperidin-1-yl)methyl)-6-fluoro-1-oxoisoindolin-2-yl)piperidine-2,6-dione;
3-(5-((4-(4-benzhydrylpiperazin-1-yl)-3,3-difluoropiperidin-1-yl)methyl)-7-fluoro-1-oxoisoindolin-2-yl)piperidine-2,6-dione;
3-(4-((4-(4-benzhydrylpiperazin-1-yl)-3,3-difluoropiperidin-1-yl)methyl)-1-oxoisoindolin-2-yl)piperidine-2,6-dione;
3-(6-((4-(4-benzhydrylpiperazin-1-yl)-3,3-difluoropiperidin-1-yl)methyl)-1-oxoisoindolin-2-yl)piperidine-2,6-dione;
3-(7-((4-(4-benzhydrylpiperazin-1-yl)-3,3-difluoropiperidin-1-yl)methyl)-1-oxoisoindolin-2-yl)piperidine-2,6-dione;
3-(5-(2-(4-(4-benzhydrylpiperazin-1-yl)-3,3-difluoropiperidin-1-yl)ethyl)-1-oxoisoindolin-2-yl)piperidine-2,6-dione;
3-(5-(3-(4-(4-benzhydrylpiperazin-1-yl)-3,3-difluoropiperidin-1-yl)propyl)-1-oxoisoindolin-2-yl)piperidine-2,6-dione;
4-((4-(4-benzhydrylpiperazin-1-yl)-3,3-difluoropiperidin-1-yl)methyl)-2-(2,6-dioxopiperidin-3-yl)isoindoline-1,3-dione;
5-((4-(4-benzhydrylpiperazin-1-yl)-3,3-difluoropiperidin-1-yl)methyl)-2-(2,6-dioxopiperidin-3-yl)isoindoline-1,3-dione;
3-(5-((3,3-difluoro-4-(4-(phenyl(pyridin-2-yl)methyl)piperazin-1-yl)piperidin-1-yl)methyl)-1-oxoisoindolin-2-yl)piperidine-2,6-dione;
3-(5-((3,3-difluoro-4-(4-(phenyl(pyridin-2-yl)methyl)piperazin-1-yl)piperidin-1-yl)methyl)-4-fluoro-1-oxoisoindolin-2-yl)piperidine-2,6-dione;
3-(5-((3,3-difluoro-4-(4-(phenyl(pyridin-2-yl)methyl)piperazin-1-yl)piperidin-1-yl)methyl)-6-fluoro-1-oxoisoindolin-2-yl)piperidine-2,6-dione;
3-(5-((3,3-difluoro-4-(4-(phenyl(pyridin-2-yl)methyl)piperazin-1-yl)piperidin-1-yl)methyl)-7-fluoro-1-oxoisoindolin-2-yl)piperidine-2,6-dione;
3-(4-((3,3-difluoro-4-(4-(phenyl(pyridin-2-yl)methyl)piperazin-1-yl)piperidin-1-yl)methyl)-1-oxoisoindolin-2-yl)piperidine-2,6-dione;
3-(6-((3,3-difluoro-4-(4-(phenyl(pyridin-2-yl)methyl)piperazin-1-yl)piperidin-1-yl)methyl)-1-oxoisoindolin-2-yl)piperidine-2,6-dione;
3-(7-((3,3-difluoro-4-(4-(phenyl(pyridin-2-yl)methyl)piperazin-1-yl)piperidin-1-yl)methyl)-1-oxoisoindolin-2-yl)piperidine-2,6-dione;
3-(5-(2-(3,3-difluoro-4-(4-(phenyl(pyridin-2-yl)methyl)piperazin-1-yl)piperidin-1-yl)ethyl)-1-oxoisoindolin-2-yl)piperidine-2,6-dione;
3-(5-(3-(3,3-difluoro-4-(4-(phenyl(pyridin-2-yl)methyl)piperazin-1-yl)piperidin-1-yl)propyl)-1-oxoisoindolin-2-yl)piperidine-2,6-dione;
4-((3,3-difluoro-4-(4-(phenyl(pyridin-2-yl)methyl)piperazin-1-yl)piperidin-1-yl)methyl)-2-(2,6-dioxopiperidin-3-yl)isoindoline-1,3-dione;
5-((3,3-difluoro-4-(4-(phenyl(pyridin-2-yl)methyl)piperazin-1-yl)piperidin-1-yl)methyl)-2-(2,6-dioxopiperidin-3-yl)isoindoline-1,3-dione;
3-(5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-oxoisoindolin-2-yl)piperidine-2,6-dione;
3-(5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-4-fluoro-1-oxoisoindolin-2-yl)piperidine-2,6-dione;
3-(5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-6-fluoro-1-oxoisoindolin-2-yl)piperidine-2,6-dione;
3-(5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-7-fluoro-1-oxoisoindolin-2-yl)piperidine-2,6-dione;
3-(4-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-oxoisoindolin-2-yl)piperidine-2,6-dione;
3-(6-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-oxoisoindolin-2-yl)piperidine-2,6-dione;
3-(7-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-oxoisoindolin-2-yl)piperidine-2,6-dione;
3-(5-(2-(4-(4-(4'-chloro-5,5-dimethyl-3,4,5,6-tetrahydro-[1,1'-biphenyl]-2-carbonyl)piperazin-1-yl)-3,3-difluoropiperidin-1-yl)ethyl)-1-oxoisoindolin-2-yl)piperidine-2,6-dione;
3-(5-(3-(4-(4-(4'-chloro-5,5-dimethyl-3,4,5,6-tetrahydro-[1,1'-biphenyl]-2-carbonyl)piperazin-1-yl)-3,3-difluoropiperidin-1-yl)propyl)-1-oxoisoindolin-2-yl)piperidine-2,6-dione;
5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-2-(2,6-dioxopiperidin-3-yl)isoindoline-1,3-dione;
4-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-2-(2,6-dioxopiperidin-3-yl)isoindoline-1,3-dione;
3-(5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-oxoisoindolin-2-yl)piperidine-2,6-dione;
3-(5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-4-fluoro-1-oxoisoindolin-2-yl)piperidine-2,6-dione;
3-(5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-6-fluoro-1-oxoisoindolin-2-yl)piperidine-2,6-dione;
3-(5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-7-fluoro-1-oxoisoindolin-2-yl)piperidine-2,6-dione;
3-(4-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-oxoisoindolin-2-yl)piperidine-2,6-dione;
3-(6-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-oxoisoindolin-2-yl)piperidine-2,6-dione;
3-(7-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-oxoisoindolin-2-yl)piperidine-2,6-dione;
3-(5-(2-(4-(4-((4'-chloro-5,5-dimethyl-3,4,5,6-tetrahydro-[1,1'-biphenyl]-2-yl)methyl)piperazin-1-yl)-3,3-difluoropiperidin-1-yl)ethyl)-1-oxoisoindolin-2-yl)piperidine-2,6-dione;
3-(5-(3-(4-(4-((4'-chloro-5,5-dimethyl-3,4,5,6-tetrahydro-[1,1'-biphenyl]-2-yl)methyl)piperazin-1-yl)-3,3-difluoropiperidin-1-yl)propyl)-1-oxoisoindolin-2-yl)piperidine-2,6-dione;
5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-2-(2,6-dioxopiperidin-3-yl)isoindoline-1,3-dione;
4-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-2-(2,6-dioxopiperidin-3-yl)isoindoline-1,3-dione;
3-(5-((4-(4-(2,3-dichlorophenyl)piperazin-1-yl)piperidin-1-yl)methyl)-1-oxoisoindolin-2-yl)piperidine-2,6-dione;
3-(5-((4-(4-(2,3-dichlorophenyl)piperazin-1-yl)piperidin-1-yl)methyl)-4-fluoro-1-oxoisoindolin-2-yl)piperidine-2,6-dione;
3-(5-((4-(4-(2,3-dichlorophenyl)piperazin-1-yl)piperidin-1-yl)methyl)-6-fluoro-1-oxoisoindolin-2-yl)piperidine-2,6-dione;
3-(5-((4-(4-(2,3-dichlorophenyl)piperazin-1-yl)piperidin-1-yl)methyl)-7-fluoro-1-oxoisoindolin-2-yl)piperidine-2,6-dione;
3-(4-((4-(4-(2,3-dichlorophenyl)piperazin-1-yl)piperidin-1-yl)methyl)-1-oxoisoindolin-2-yl)piperidine-2,6-dione;
3-(6-((4-(4-(2,3-dichlorophenyl)piperazin-1-yl)piperidin-1-yl)methyl)-1-oxoisoindolin-2-yl)piperidine-2,6-dione;
3-(7-((4-(4-(2,3-dichlorophenyl)piperazin-1-yl)piperidin-1-yl)methyl)-1-oxoisoindolin-2-yl)piperidine-2,6-dione;
3-(5-(2-(4-(4-(2,3-dichlorophenyl)piperazin-1-yl)piperidin-1-yl)ethyl)-1-oxoisoindolin-2-yl)piperidine-2,6-dione;
3-(5-(3-(4-(4-(2,3-dichlorophenyl)piperazin-1-yl)piperidin-1-yl)propyl)-1-oxoisoindolin-2-yl)piperidine-2,6-dione;
4-((4-(4-(2,3-dichlorophenyl)piperazin-1-yl)piperidin-1-yl)methyl)-2-(2,6-dioxopiperidin-3-yl)isoindoline-1,3-dione;
5-((4-(4-(2,3-dichlorophenyl)piperazin-1-yl)piperidin-1-yl)methyl)-2-(2,6-dioxopiperidin-3-yl)isoindoline-1,3-dione;
3-(5-((4-(4-(2,3-dichlorophenyl)piperazin-1-yl)-3,3-difluoropiperidin-1-yl)methyl)-1-oxoisoindolin-2-yl)piperidine-2,6-dione;
3-(5-((4-(4-(2,3-dichlorophenyl)piperazin-1-yl)-3,3-difluoropiperidin-1-yl)methyl)-4-fluoro-1-oxoisoindolin-2-yl)piperidine-2,6-dione;
3-(5-((4-(4-(2,3-dichlorophenyl)piperazin-1-yl)-3,3-difluoropiperidin-1-yl)methyl)-6-fluoro-1-oxoisoindolin-2-yl)piperidine-2,6-dione;
3-(5-((4-(4-(2,3-dichlorophenyl)piperazin-1-yl)-3,3-difluoropiperidin-1-yl)methyl)-7-fluoro-1-oxoisoindolin-2-yl)piperidine-2,6-dione;
3-(4-((4-(4-(2,3-dichlorophenyl)piperazin-1-yl)-3,3-difluoropiperidin-1-yl)methyl)-1-oxoisoindolin-2-yl)piperidine-2,6-dione;
3-(6-((4-(4-(2,3-dichlorophenyl)piperazin-1-yl)-3,3-difluoropiperidin-1-yl)methyl)-1-oxoisoindolin-2-yl)piperidine-2,6-dione;
3-(7-((4-(4-(2,3-dichlorophenyl)piperazin-1-yl)-3,3-difluoropiperidin-1-yl)methyl)-1-oxoisoindolin-2-yl)piperidine-2,6-dione;
3-(5-(2-(4-(4-(2,3-dichlorophenyl)piperazin-1-yl)-3,3-difluoropiperidin-1-yl)ethyl)-1-oxoisoindolin-2-yl)piperidine-2,6-dione;
3-(5-(3-(4-(4-(2,3-dichlorophenyl)piperazin-1-yl)-3,3-difluoropiperidin-1-yl)propyl)-1-oxoisoindolin-2-yl)piperidine-2,6-dione;
4-((4-(4-(2,3-dichlorophenyl)piperazin-1-yl)-3,3-difluoropiperidin-1-yl)methyl)-2-(2,6-dioxopiperidin-3-yl)isoindoline-1,3-dione;
5-((4-(4-(2,3-dichlorophenyl)piperazin-1-yl)-3,3-difluoropiperidin-1-yl)methyl)-2-(2,6-dioxopiperidin-3-yl)isoindoline-1,3-dione;
3-(5-((4-(4-(2,3-dichlorophenyl)-3,6-dihydropyridin-1(2H)-yl)-3,3-difluoropiperidin-1-yl)methyl)-1-oxoisoindolin-2-yl)piperidine-2,6-dione;
3-(5-((4-(4-(2,3-dichlorophenyl)-3,6-dihydropyridin-1(2H)-yl)-3,3-difluoropiperidin-1-yl)methyl)-4-fluoro-1-oxoisoindolin-2-yl)piperidine-2,6-dione;
3-(5-((4-(4-(2,3-dichlorophenyl)-3,6-dihydropyridin-1(2H)-yl)-3,3-difluoropiperidin-1-yl)methyl)-6-fluoro-1-oxoisoindolin-2-yl)piperidine-2,6-dione;
3-(5-((4-(4-(2,3-dichlorophenyl)-3,6-dihydropyridin-1(2H)-yl)-3,3-difluoropiperidin-1-yl)methyl)-7-fluoro-1-oxoisoindolin-2-yl)piperidine-2,6-dione;
3-(4-((4-(4-(2,3-dichlorophenyl)-3,6-dihydropyridin-1(2H)-yl)-3,3-difluoropiperidin-1-yl)methyl)-1-oxoisoindolin-2-yl)piperidine-2,6-dione;
3-(6-((4-(4-(2,3-dichlorophenyl)-3,6-dihydropyridin-1(2H)-yl)-3,3-difluoropiperidin-1-yl)methyl)-1-oxoisoindolin-2-yl)piperidine-2,6-dione;
3-(7-((4-(4-(2,3-dichlorophenyl)-3,6-dihydropyridin-1(2H)-yl)-3,3-difluoropiperidin-1-yl)methyl)-1-oxoisoindolin-2-yl)piperidine-2,6-dione;
3-(5-(2-(4-(4-(2,3-dichlorophenyl)-3,6-dihydropyridin-1(2H)-yl)-3,3-difluoropiperidin-1-yl)ethyl)-1-oxoisoindolin-2-yl)piperidine-2,6-dione;
3-(5-(3-(4-(4-(2,3-dichlorophenyl)-3,6-dihydropyridin-1(2H)-yl)-3,3-difluoropiperidin-1-yl)propyl)-1-oxoisoindolin-2-yl)piperidine-2,6-dione;
4-((4-(4-(2,3-dichlorophenyl)-3,6-dihydropyridin-1(2H)-yl)-3,3-difluoropiperidin-1-yl)methyl)-2-(2,6-dioxopiperidin-3-yl)isoindoline-1,3-dione;
5-((4-(4-(2,3-dichlorophenyl)-3,6-dihydropyridin-1(2H)-yl)-3,3-difluoropiperidin-1-yl)methyl)-2-(2,6-dioxopiperidin-3-yl)isoindoline-1,3-dione;
3-(5-((4-(4-(6-fluorobenzo[d]isoxazol-3-yl)piperazin-1-yl)piperidin-1-yl)methyl)-1-oxoisoindolin-2-yl)piperidine-2,6-dione;
3-(4-fluoro-5-((4-(4-(6-fluorobenzo[d]isoxazol-3-yl)piperazin-1-yl)piperidin-1-yl)methyl)-1-oxoisoindolin-2-yl)piperidine-2,6-dione;
3-(6-fluoro-5-((4-(4-(6-fluorobenzo[d]isoxazol-3-yl)piperazin-1-yl)piperidin-1-yl)methyl)-1-oxoisoindolin-2-yl)piperidine-2,6-dione;
3-(7-fluoro-5-((4-(4-(6-fluorobenzo[d]isoxazol-3-yl)piperazin-1-yl)piperidin-1-yl)methyl)-1-oxoisoindolin-2-yl)piperidine-2,6-dione;
3-(4-((4-(4-(6-fluorobenzo[d]isoxazol-3-yl)piperazin-1-yl)piperidin-1-yl)methyl)-1-oxoisoindolin-2-yl)piperidine-2,6-dione;
3-(6-((4-(4-(6-fluorobenzo[d]isoxazol-3-yl)piperazin-1-yl)piperidin-1-yl)methyl)-1-oxoisoindolin-2-yl)piperidine-2,6-dione;
3-(7-((4-(4-(6-fluorobenzo[d]isoxazol-3-yl)piperazin-1-yl)piperidin-1-yl)methyl)-1-oxoisoindolin-2-yl)piperidine-2,6-dione;
3-(5-(2-(4-(4-(6-fluorobenzo[d]isoxazol-3-yl)piperazin-1-yl)piperidin-1-yl)ethyl)-1-oxoisoindolin-2-yl)piperidine-2,6-dione;
3-(5-(3-(4-(4-(6-fluorobenzo[d]isoxazol-3-yl)piperazin-1-yl)piperidin-1-yl)propyl)-1-oxoisoindolin-2-yl)piperidine-2,6-dione;
2-(2,6-dioxopiperidin-3-yl)-4-((4-(4-(6-fluorobenzo[d]isoxazol-3-yl)piperazin-1-yl)piperidin-1-yl)methyl)isoindoline-1,3-dione;
2-(2,6-dioxopiperidin-3-yl)-5-((4-(4-(6-fluorobenzo[d]isoxazol-3-yl)piperazin-1-yl)piperidin-1-yl)methyl)isoindoline-1,3-dione;
3-(5-((3,3-difluoro-4-(4-(6-fluorobenzo[d]isoxazol-3-yl)piperazin-1-yl)piperidin-1-yl)methyl)-1-oxoisoindolin-2-yl)piperidine-2,6-dione;
3-(5-((3,3-difluoro-4-(4-(6-fluorobenzo[d]isoxazol-3-yl)piperazin-1-yl)piperidin-1-yl)methyl)-4-fluoro-1-oxoisoindolin-2-yl)piperidine-2,6-dione;
3-(5-((3,3-difluoro-4-(4-(6-fluorobenzo[d]isoxazol-3-yl)piperazin-1-yl)piperidin-1-yl)methyl)-6-fluoro-1-oxoisoindolin-2-yl)piperidine-2,6-dione;
3-(5-((3,3-difluoro-4-(4-(6-fluorobenzo[d]isoxazol-3-yl)piperazin-1-yl)piperidin-1-yl)methyl)-7-fluoro-1-oxoisoindolin-2-yl)piperidine-2,6-dione;
3-(4-((3,3-difluoro-4-(4-(6-fluorobenzo[d]isoxazol-3-yl)piperazin-1-yl)piperidin-1-yl)methyl)-1-oxoisoindolin-2-yl)piperidine-2,6-dione;
3-(6-((3,3-difluoro-4-(4-(6-fluorobenzo[d]isoxazol-3-yl)piperazin-1-yl)piperidin-1-yl)methyl)-1-oxoisoindolin-2-yl)piperidine-2,6-dione;
3-(7-((3,3-difluoro-4-(4-(6-fluorobenzo[d]isoxazol-3-yl)piperazin-1-yl)piperidin-1-yl)methyl)-1-oxoisoindolin-2-yl)piperidine-2,6-dione;
3-(5-(2-(3,3-difluoro-4-(4-(6-fluorobenzo[d]isoxazol-3-yl)piperazin-1-yl)piperidin-1-yl)ethyl)-1-oxoisoindolin-2-yl)piperidine-2,6-dione;
3-(5-(3-(3,3-difluoro-4-(4-(6-fluorobenzo[d]isoxazol-3-yl)piperazin-1-yl)piperidin-1-yl)propyl)-1-oxoisoindolin-2-yl)piperidine-2,6-dione;
4-((3,3-difluoro-4-(4-(6-fluorobenzo[d]isoxazol-3-yl)piperazin-1-yl)piperidin-1-yl)methyl)-2-(2,6-dioxopiperidin-3-yl)isoindoline-1,3-dione;
5-((3,3-difluoro-4-(4-(6-fluorobenzo[d]isoxazol-3-yl)piperazin-1-yl)piperidin-1-yl)methyl)-2-(2,6-dioxopiperidin-3-yl)isoindoline-1,3-dione;
3-(5-((3',3'-difluoro-4-(6-fluorobenzo[d]isoxazol-3-yl)-[1,4'-bipiperidin]-1'-yl)methyl)-1-oxoisoindolin-2-yl)piperidine-2,6-dione;
3-(5-((3',3'-difluoro-4-(6-fluorobenzo[d]isoxazol-3-yl)-[1,4'-bipiperidin]-1'-yl)methyl)-4-fluoro-1-oxoisoindolin-2-yl)piperidine-2,6-dione;
3-(5-((3',3'-difluoro-4-(6-fluorobenzo[d]isoxazol-3-yl)-[1,4'-bipiperidin]-1'-yl)methyl)-6-fluoro-1-oxoisoindolin-2-yl)piperidine-2,6-dione;
3-(5-((3',3'-difluoro-4-(6-fluorobenzo[d]isoxazol-3-yl)-[1,4'-bipiperidin]-1'-yl)methyl)-7-fluoro-1-oxoisoindolin-2-yl)piperidine-2,6-dione;
3-(4-((3',3'-difluoro-4-(6-fluorobenzo[d]isoxazol-3-yl)-[1,4'-bipiperidin]-1'-yl)methyl)-1-oxoisoindolin-2-yl)piperidine-2,6-dione;
3-(6-((3',3'-difluoro-4-(6-fluorobenzo[d]isoxazol-3-yl)-[1,4'-bipiperidin]-1'-yl)methyl)-1-oxoisoindolin-2-yl)piperidine-2,6-dione;
3-(7-((3',3'-difluoro-4-(6-fluorobenzo[d]isoxazol-3-yl)-[1,4'-bipiperidin]-1'-yl)methyl)-1-oxoisoindolin-2-yl)piperidine-2,6-dione;
3-(5-(2-(3',3'-difluoro-4-(6-fluorobenzo[d]isoxazol-3-yl)-[1,4'-bipiperidin]-1'-yl)ethyl)-1-oxoisoindolin-2-yl)piperidine-2,6-dione;
3-(5-(3-(3',3'-difluoro-4-(6-fluorobenzo[d]isoxazol-3-yl)-[1,4'-bipiperidin]-1'-yl)propyl)-1-oxoisoindolin-2-yl)piperidine-2,6-dione;
4-((3',3'-difluoro-4-(6-fluorobenzo[d]isoxazol-3-yl)-[1,4'-bipiperidin]-1'-yl)methyl)-2-(2,6-dioxopiperidin-3-yl)isoindoline-1,3-dione;
5-((3',3'-difluoro-4-(6-fluorobenzo[d]isoxazol-3-yl)-[1,4'-bipiperidin]-1'-yl)methyl)-2-(2,6-dioxopiperidin-3-yl)isoindoline-1,3-dione;
3-(5-(4-(4-benzhydrylpiperazin-1-yl)piperidine-1-carbonyl)-1-oxoisoindolin-2-yl)piperidine-2,6-dione;
3-(5-(4-(4-benzhydrylpiperazin-1-yl)piperidine-1-carbonyl)-6-fluoro-1-oxoisoindolin-2-yl)piperidine-2,6-dione;
3-(5-(4-(4-benzhydrylpiperazin-1-yl)piperidine-1-carbonyl)-7-fluoro-1-oxoisoindolin-2-yl)piperidine-2,6-dione;
3-(4-(4-(4-benzhydrylpiperazin-1-yl)piperidine-1-carbonyl)-1-oxoisoindolin-2-yl)piperidine-2,6-dione;
3-(6-(4-(4-benzhydrylpiperazin-1-yl)piperidine-1-carbonyl)-1-oxoisoindolin-2-yl)piperidine-2,6-dione;
3-(7-(4-(4-benzhydrylpiperazin-1-yl)piperidine-1-carbonyl)-1-oxoisoindolin-2-yl)piperidine-2,6-dione;
4-(4-(4-benzhydrylpiperazin-1-yl)piperidine-1-carbonyl)-2-(2,6-dioxopiperidin-3-yl)isoindoline-1,3-dione;
5-(4-(4-benzhydrylpiperazin-1-yl)piperidine-1-carbonyl)-2-(2,6-dioxopiperidin-3-yl)isoindoline-1,3-dione;
3-(5-(4-(4-benzhydrylpiperazin-1-yl)-3,3-difluoropiperidine-1-carbonyl)-1-oxoisoindolin-2-yl)piperidine-2,6-dione;
3-(5-(4-(4-benzhydrylpiperazin-1-yl)-3,3-difluoropiperidine-1-carbonyl)-6-fluoro-1-oxoisoindolin-2-yl)piperidine-2,6-dione;
3-(5-(4-(4-benzhydrylpiperazin-1-yl)-3,3-difluoropiperidine-1-carbonyl)-7-fluoro-1-oxoisoindolin-2-yl)piperidine-2,6-dione;
3-(4-(4-(4-benzhydrylpiperazin-1-yl)-3,3-difluoropiperidine-1-carbonyl)-1-oxoisoindolin-2-yl)piperidine-2,6-dione;
3-(6-(4-(4-benzhydrylpiperazin-1-yl)-3,3-difluoropiperidine-1-carbonyl)-1-oxoisoindolin-2-yl)piperidine-2,6-dione;
3-(7-(4-(4-benzhydrylpiperazin-1-yl)-3,3-difluoropiperidine-1-carbonyl)-1-oxoisoindolin-2-yl)piperidine-2,6-dione;
4-(4-(4-benzhydrylpiperazin-1-yl)-3,3-difluoropiperidine-1-carbonyl)-2-(2,6-dioxopiperidin-3-yl)isoindoline-1,3-dione;
5-(4-(4-benzhydrylpiperazin-1-yl)-3,3-difluoropiperidine-1-carbonyl)-2-(2,6-dioxopiperidin-3-yl)isoindoline-1,3-dione;
3-(5-(3,3-difluoro-4-(4-(phenyl(pyridin-2-yl)methyl)piperazin-1-yl)piperidine-1-carbonyl)-1-oxoisoindolin-2-yl)piperidine-2,6-dione;
3-(5-(3,3-difluoro-4-(4-(phenyl(pyridin-2-yl)methyl)piperazin-1-yl)piperidine-1-carbonyl)-6-fluoro-1-oxoisoindolin-2-yl)piperidine-2,6-dione;
3-(5-(3,3-difluoro-4-(4-(phenyl(pyridin-2-yl)methyl)piperazin-1-yl)piperidine-1-carbonyl)-7-fluoro-1-oxoisoindolin-2-yl)piperidine-2,6-dione;
3-(4-(3,3-difluoro-4-(4-(phenyl(pyridin-2-yl)methyl)piperazin-1-yl)piperidine-1-carbonyl)-1-oxoisoindolin-2-yl)piperidine-2,6-dione;
3-(6-(3,3-difluoro-4-(4-(phenyl(pyridin-2-yl)methyl)piperazin-1-yl)piperidine-1-carbonyl)-1-oxoisoindolin-2-yl)piperidine-2,6-dione;
3-(7-(3,3-difluoro-4-(4-(phenyl(pyridin-2-yl)methyl)piperazin-1-yl)piperidine-1-carbonyl)-1-oxoisoindolin-2-yl)piperidine-2,6-dione;
4-(3,3-difluoro-4-(4-(phenyl(pyridin-2-yl)methyl)piperazin-1-yl)piperidine-1-carbonyl)-2-(2,6-dioxopiperidin-3-yl)isoindoline-1,3-dione;
5-(3,3-difluoro-4-(4-(phenyl(pyridin-2-yl)methyl)piperazin-1-yl)piperidine-1-carbonyl)-2-(2,6-dioxopiperidin-3-yl)isoindoline-1,3-dione;
3-(5-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-1-oxoisoindolin-2-yl)piperidine-2,6-dione;
3-(5-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-6-fluoro-1-oxoisoindolin-2-yl)piperidine-2,6-dione;
3-(5-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-7-fluoro-1-oxoisoindolin-2-yl)piperidine-2,6-dione;
3-(4-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-1-oxoisoindolin-2-yl)piperidine-2,6-dione;
3-(6-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-1-oxoisoindolin-2-yl)piperidine-2,6-dione;
3-(7-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-1-oxoisoindolin-2-yl)piperidine-2,6-dione;
4-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-2-(2,6-dioxopiperidin-3-yl)isoindoline-1,3-dione;
5-(4-(4-(4'-chloro-5,5-dimethyl-3,4,5,6-tetrahydro-[1,1'-biphenyl]-2-carbonyl)piperazin-1-yl)-3,3-difluoropiperidine-1-carbonyl)-2-(2,6-dioxopiperidin-3-yl)isoindoline-1,3-dione;
3-(5-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-1-oxoisoindolin-2-yl)piperidine-2,6-dione;
3-(5-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-6-fluoro-1-oxoisoindolin-2-yl)piperidine-2,6-dione;
3-(5-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-7-fluoro-1-oxoisoindolin-2-yl)piperidine-2,6-dione;
3-(4-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-1-oxoisoindolin-2-yl)piperidine-2,6-dione;
3-(6-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-1-oxoisoindolin-2-yl)piperidine-2,6-dione;
3-(7-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-1-oxoisoindolin-2-yl)piperidine-2,6-dione;
4-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-2-(2,6-dioxopiperidin-3-yl)isoindoline-1,3-dione;
5-(4-(4-((4'-chloro-5,5-dimethyl-3,4,5,6-tetrahydro-[1,1'-biphenyl]-2-yl)methyl)piperazin-1-yl)-3,3-difluoropiperidine-1-carbonyl)-2-(2,6-dioxopiperidin-3-yl)isoindoline-1,3-dione;
3-(5-(4-(4-(2,3-dichlorophenyl)piperazin-1-yl)piperidine-1-carbonyl)-1-oxoisoindolin-2-yl)piperidine-2,6-dione;
3-(5-(4-(4-(2,3-dichlorophenyl)piperazin-1-yl)piperidine-1-carbonyl)-6-fluoro-1-oxoisoindolin-2-yl)piperidine-2,6-dione;
3-(5-(4-(4-(2,3-dichlorophenyl)piperazin-1-yl)piperidine-1-carbonyl)-7-fluoro-1-oxoisoindolin-2-yl)piperidine-2,6-dione;
3-(4-(4-(4-(2,3-dichlorophenyl)piperazin-1-yl)piperidine-1-carbonyl)-1-oxoisoindolin-2-yl)piperidine-2,6-dione;
3-(6-(4-(4-(2,3-dichlorophenyl)piperazin-1-yl)piperidine-1-carbonyl)-1-oxoisoindolin-2-yl)piperidine-2,6-dione;
3-(7-(4-(4-(2,3-dichlorophenyl)piperazin-1-yl)piperidine-1-carbonyl)-1-oxoisoindolin-2-yl)piperidine-2,6-dione;
4-(4-(4-(2,3-dichlorophenyl)piperazin-1-yl)piperidine-1-carbonyl)-2-(2,6-dioxopiperidin-3-yl)isoindoline-1,3-dione;
5-(4-(4-(2,3-dichlorophenyl)piperazin-1-yl)piperidine-1-carbonyl)-2-(2,6-dioxopiperidin-3-yl)isoindoline-1,3-dione;
3-(5-(4-(4-(2,3-dichlorophenyl)piperazin-1-yl)-3,3-difluoropiperidine-1-carbonyl)-1-oxoisoindolin-2-yl)piperidine-2,6-dione;
3-(5-(4-(4-(2,3-dichlorophenyl)piperazin-1-yl)-3,3-difluoropiperidine-1-carbonyl)-6-fluoro-1-oxoisoindolin-2-yl)piperidine-2,6-dione;
3-(5-(4-(4-(2,3-dichlorophenyl)piperazin-1-yl)-3,3-difluoropiperidine-1-carbonyl)-7-fluoro-1-oxoisoindolin-2-yl)piperidine-2,6-dione;
3-(4-(4-(4-(2,3-dichlorophenyl)piperazin-1-yl)-3,3-difluoropiperidine-1-carbonyl)-1-oxoisoindolin-2-yl)piperidine-2,6-dione;
3-(6-(4-(4-(2,3-dichlorophenyl)piperazin-1-yl)-3,3-difluoropiperidine-1-carbonyl)-1-oxoisoindolin-2-yl)piperidine-2,6-dione;
3-(7-(4-(4-(2,3-dichlorophenyl)piperazin-1-yl)-3,3-difluoropiperidine-1-carbonyl)-1-oxoisoindolin-2-yl)piperidine-2,6-dione;
4-(4-(4-(2,3-dichlorophenyl)piperazin-1-yl)-3,3-difluoropiperidine-1-carbonyl)-2-(2,6-dioxopiperidin-3-yl)isoindoline-1,3-dione;
5-(4-(4-(2,3-dichlorophenyl)piperazin-1-yl)-3,3-difluoropiperidine-1-carbonyl)-2-(2,6-dioxopiperidin-3-yl)isoindoline-1,3-dione;
3-(5-(4-(4-(2,3-dichlorophenyl)-3,6-dihydropyridin-1(2H)-yl)-3,3-difluoropiperidine-1-carbonyl)-1-oxoisoindolin-2-yl)piperidine-2,6-dione;
3-(5-(4-(4-(2,3-dichlorophenyl)-3,6-dihydropyridin-1(2H)-yl)-3,3-difluoropiperidine-1-carbonyl)-6-fluoro-1-oxoisoindolin-2-yl)piperidine-2,6-dione;
3-(5-(4-(4-(2,3-dichlorophenyl)-3,6-dihydropyridin-1(2H)-yl)-3,3-difluoropiperidine-1-carbonyl)-7-fluoro-1-oxoisoindolin-2-yl)piperidine-2,6-dione;
3-(4-(4-(4-(2,3-dichlorophenyl)-3,6-dihydropyridin-1(2H)-yl)-3,3-difluoropiperidine-1-carbonyl)-1-oxoisoindolin-2-yl)piperidine-2,6-dione;
3-(6-(4-(4-(2,3-dichlorophenyl)-3,6-dihydropyridin-1(2H)-yl)-3,3-difluoropiperidine-1-carbonyl)-1-oxoisoindolin-2-yl)piperidine-2,6-dione;
3-(7-(4-(4-(2,3-dichlorophenyl)-3,6-dihydropyridin-1(2H)-yl)-3,3-difluoropiperidine-1-carbonyl)-1-oxoisoindolin-2-yl)piperidine-2,6-dione;
4-(4-(4-(2,3-dichlorophenyl)-3,6-dihydropyridin-1(2H)-yl)-3,3-difluoropiperidine-1-carbonyl)-2-(2,6-dioxopiperidin-3-yl)isoindoline-1,3-dione;
5-(4-(4-(2,3-dichlorophenyl)-3,6-dihydropyridin-1(2H)-yl)-3,3-difluoropiperidine-1-carbonyl)-2-(2,6-dioxopiperidin-3-yl)isoindoline-1,3-dione;
3-(5-(4-(4-(6-fluorobenzo[d]isoxazol-3-yl)piperazin-1-yl)piperidine-1-carbonyl)-1-oxoisoindolin-2-yl)piperidine-2,6-dione;
3-(6-fluoro-5-(4-(4-(6-fluorobenzo[d]isoxazol-3-yl)piperazin-1-yl)piperidine-1-carbonyl)-1-oxoisoindolin-2-yl)piperidine-2,6-dione;
3-(7-fluoro-5-(4-(4-(6-fluorobenzo[d]isoxazol-3-yl)piperazin-1-yl)piperidine-1-carbonyl)-1-oxoisoindolin-2-yl)piperidine-2,6-dione;
3-(4-(4-(4-(6-fluorobenzo[d]isoxazol-3-yl)piperazin-1-yl)piperidine-1-carbonyl)-1-oxoisoindolin-2-yl)piperidine-2,6-dione;
3-(6-(4-(4-(6-fluorobenzo[d]isoxazol-3-yl)piperazin-1-yl)piperidine-1-carbonyl)-1-oxoisoindolin-2-yl)piperidine-2,6-dione;
3-(7-(4-(4-(6-fluorobenzo[d]isoxazol-3-yl)piperazin-1-yl)piperidine-1-carbonyl)-1-oxoisoindolin-2-yl)piperidine-2,6-dione;
2-(2,6-dioxopiperidin-3-yl)-4-(4-(4-(6-fluorobenzo[d]isoxazol-3-yl)piperazin-1-yl)piperidine-1-carbonyl)isoindoline-1,3-dione;
2-(2,6-dioxopiperidin-3-yl)-5-(4-(4-(6-fluorobenzo[d]isoxazol-3-yl)piperazin-1-yl)piperidine-1-carbonyl)isoindoline-1,3-dione;
3-(5-(3,3-difluoro-4-(4-(6-fluorobenzo[d]isoxazol-3-yl)piperazin-1-yl)piperidine-1-carbonyl)-1-oxoisoindolin-2-yl)piperidine-2,6-dione;
3-(5-(3,3-difluoro-4-(4-(6-fluorobenzo[d]isoxazol-3-yl)piperazin-1-yl)piperidine-1-carbonyl)-6-fluoro-1-oxoisoindolin-2-yl)piperidine-2,6-dione;
3-(5-(3,3-difluoro-4-(4-(6-fluorobenzo[d]isoxazol-3-yl)piperazin-1-yl)piperidine-1-carbonyl)-7-fluoro-1-oxoisoindolin-2-yl)piperidine-2,6-dione;
3-(4-(3,3-difluoro-4-(4-(6-fluorobenzo[d]isoxazol-3-yl)piperazin-1-yl)piperidine-1-carbonyl)-1-oxoisoindolin-2-yl)piperidine-2,6-dione;
3-(6-(3,3-difluoro-4-(4-(6-fluorobenzo[d]isoxazol-3-yl)piperazin-1-yl)piperidine-1-carbonyl)-1-oxoisoindolin-2-yl)piperidine-2,6-dione;
3-(7-(3,3-difluoro-4-(4-(6-fluorobenzo[d]isoxazol-3-yl)piperazin-1-yl)piperidine-1-carbonyl)-1-oxoisoindolin-2-yl)piperidine-2,6-dione;
4-(3,3-difluoro-4-(4-(6-fluorobenzo[d]isoxazol-3-yl)piperazin-1-yl)piperidine-1-carbonyl)-2-(2,6-dioxopiperidin-3-yl)isoindoline-1,3-dione;
5-(3,3-difluoro-4-(4-(6-fluorobenzo[d]isoxazol-3-yl)piperazin-1-yl)piperidine-1-carbonyl)-2-(2,6-dioxopiperidin-3-yl)isoindoline-1,3-dione;
3-(5-(3',3'-difluoro-4-(6-fluorobenzo[d]isoxazol-3-yl)-[1,4'-bipiperidine]-1'-carbonyl)-1-oxoisoindolin-2-yl)piperidine-2,6-dione;
3-(5-(3',3'-difluoro-4-(6-fluorobenzo[d]isoxazol-3-yl)-[1,4'-bipiperidine]-1'-carbonyl)-6-fluoro-1-oxoisoindolin-2-yl)piperidine-2,6-dione;
3-(5-(3',3'-difluoro-4-(6-fluorobenzo[d]isoxazol-3-yl)-[1,4'-bipiperidine]-1'-carbonyl)-7-fluoro-1-oxoisoindolin-2-yl)piperidine-2,6-dione;
3-(4-(3',3'-difluoro-4-(6-fluorobenzo[d]isoxazol-3-yl)-[1,4'-bipiperidine]-1'-carbonyl)-1-oxoisoindolin-2-yl)piperidine-2,6-dione;
3-(6-(3',3'-difluoro-4-(6-fluorobenzo[d]isoxazol-3-yl)-[1,4'-bipiperidine]-1'-carbonyl)-1-oxoisoindolin-2-yl)piperidine-2,6-dione;
3-(7-(3',3'-difluoro-4-(6-fluorobenzo[d]isoxazol-3-yl)-[1,4'-bipiperidine]-1'-carbonyl)-1-oxoisoindolin-2-yl)piperidine-2,6-dione;
4-(3',3'-difluoro-4-(6-fluorobenzo[d]isoxazol-3-yl)-[1,4'-bipiperidine]-1'-carbonyl)-2-(2,6-dioxopiperidin-3-yl)isoindoline-1,3-dione;
5-(3',3'-difluoro-4-(6-fluorobenzo[d]isoxazol-3-yl)-[1,4'-bipiperidine]-1'-carbonyl)-2-(2,6-dioxopiperidin-3-yl)isoindoline-1,3-dione;
3-(5-((4-(4-benzhydrylpiperazin-1-yl)piperidin-1-yl)methyl)-1-thioxoisoindolin-2-yl)piperidine-2,6-dione;
3-(4-((4-(4-benzhydrylpiperazin-1-yl)piperidin-1-yl)methyl)-1-thioxoisoindolin-2-yl)piperidine-2,6-dione;
3-(6-((4-(4-benzhydrylpiperazin-1-yl)piperidin-1-yl)methyl)-1-thioxoisoindolin-2-yl)piperidine-2,6-dione;
3-(5-((4-(4-benzhydrylpiperazin-1-yl)-3,3-difluoropiperidin-1-yl)methyl)-1-thioxoisoindolin-2-yl)piperidine-2,6-dione;
3-(4-((4-(4-benzhydrylpiperazin-1-yl)-3,3-difluoropiperidin-1-yl)methyl)-1-thioxoisoindolin-2-yl)piperidine-2,6-dione;
3-(6-((4-(4-benzhydrylpiperazin-1-yl)-3,3-difluoropiperidin-1-yl)methyl)-1-thioxoisoindolin-2-yl)piperidine-2,6-dione;
3-(5-((3,3-difluoro-4-(4-(phenyl(pyridin-2-yl)methyl)piperazin-1-yl)piperidin-1-yl)methyl)-1-thioxoisoindolin-2-yl)piperidine-2,6-dione;
3-(4-((3,3-difluoro-4-(4-(phenyl(pyridin-2-yl)methyl)piperazin-1-yl)piperidin-1-yl)methyl)-1-thioxoisoindolin-2-yl)piperidine-2,6-dione;
3-(6-((3,3-difluoro-4-(4-(phenyl(pyridin-2-yl)methyl)piperazin-1-yl)piperidin-1-yl)methyl)-1-thioxoisoindolin-2-yl)piperidine-2,6-dione;
3-(5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-thioxoisoindolin-2-yl)piperidine-2,6-dione;
3-(4-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-thioxoisoindolin-2-yl)piperidine-2,6-dione;
3-(6-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-thioxoisoindolin-2-yl)piperidine-2,6-dione;
3-(5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-thioxoisoindolin-2-yl)piperidine-2,6-dione;
3-(4-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-thioxoisoindolin-2-yl)piperidine-2,6-dione;
3-(6-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-thioxoisoindolin-2-yl)piperidine-2,6-dione;
3-(5-((4-(4-(2,3-dichlorophenyl)piperazin-1-yl)piperidin-1-yl)methyl)-1-thioxoisoindolin-2-yl)piperidine-2,6-dione;
3-(4-((4-(4-(2,3-dichlorophenyl)piperazin-1-yl)piperidin-1-yl)methyl)-1-thioxoisoindolin-2-yl)piperidine-2,6-dione;
3-(6-((4-(4-(2,3-dichlorophenyl)piperazin-1-yl)piperidin-1-yl)methyl)-1-thioxoisoindolin-2-yl)piperidine-2,6-dione;
3-(5-((4-(4-(2,3-dichlorophenyl)piperazin-1-yl)-3,3-difluoropiperidin-1-yl)methyl)-1-thioxoisoindolin-2-yl)piperidine-2,6-dione;
3-(4-((4-(4-(2,3-dichlorophenyl)piperazin-1-yl)-3,3-difluoropiperidin-1-yl)methyl)-1-thioxoisoindolin-2-yl)piperidine-2,6-dione;
3-(6-((4-(4-(2,3-dichlorophenyl)piperazin-1-yl)-3,3-difluoropiperidin-1-yl)methyl)-1-thioxoisoindolin-2-yl)piperidine-2,6-dione;
3-(5-((4-(4-(2,3-dichlorophenyl)-3,6-dihydropyridin-1(2H)-yl)-3,3-difluoropiperidin-1-yl)methyl)-1-thioxoisoindolin-2-yl)piperidine-2,6-dione;
3-(4-((4-(4-(2,3-dichlorophenyl)-3,6-dihydropyridin-1(2H)-yl)-3,3-difluoropiperidin-1-yl)methyl)-1-thioxoisoindolin-2-yl)piperidine-2,6-dione;
3-(6-((4-(4-(2,3-dichlorophenyl)-3,6-dihydropyridin-1(2H)-yl)-3,3-difluoropiperidin-1-yl)methyl)-1-thioxoisoindolin-2-yl)piperidine-2,6-dione;
3-(5-((4-(4-(6-fluorobenzo[d]isoxazol-3-yl)piperazin-1-yl)piperidin-1-yl)methyl)-1-thioxoisoindolin-2-yl)piperidine-2,6-dione;
3-(4-((4-(4-(6-fluorobenzo[d]isoxazol-3-yl)piperazin-1-yl)piperidin-1-yl)methyl)-1-thioxoisoindolin-2-yl)piperidine-2,6-dione;
3-(6-((4-(4-(6-fluorobenzo[d]isoxazol-3-yl)piperazin-1-yl)piperidin-1-yl)methyl)-1-thioxoisoindolin-2-yl)piperidine-2,6-dione;
3-(5-((3,3-difluoro-4-(4-(6-fluorobenzo[d]isoxazol-3-yl)piperazin-1-yl)piperidin-1-yl)methyl)-1-thioxoisoindolin-2-yl)piperidine-2,6-dione;
3-(4-((3,3-difluoro-4-(4-(6-fluorobenzo[d]isoxazol-3-yl)piperazin-1-yl)piperidin-1-yl)methyl)-1-thioxoisoindolin-2-yl)piperidine-2,6-dione;
3-(6-((3,3-difluoro-4-(4-(6-fluorobenzo[d]isoxazol-3-yl)piperazin-1-yl)piperidin-1-yl)methyl)-1-thioxoisoindolin-2-yl)piperidine-2,6-dione;
3-(5-((3',3'-difluoro-4-(6-fluorobenzo[d]isoxazol-3-yl)-[1,4'-bipiperidin]-1'-yl)methyl)-1-thioxoisoindolin-2-yl)piperidine-2,6-dione;
3-(4-((3',3'-difluoro-4-(6-fluorobenzo[d]isoxazol-3-yl)-[1,4'-bipiperidin]-1'-yl)methyl)-1-thioxoisoindolin-2-yl)piperidine-2,6-dione;
3-(6-((3',3'-difluoro-4-(6-fluorobenzo[d]isoxazol-3-yl)-[1,4'-bipiperidin]-1'-yl)methyl)-1-thioxoisoindolin-2-yl)piperidine-2,6-dione;
1-(5-((4-(4-benzhydrylpiperazin-1-yl)piperidin-1-yl)methyl)-1-oxoisoindolin-2-yl)dihydropyrimidine-2,4(1H,3H)-dione;
5-((4-(4-benzhydrylpiperazin-1-yl)piperidin-1-yl)methyl)-2-(2,4-dioxotetrahydropyrimidin-1(2H)-yl)isoindoline-1,3-dione;
1-(5-((4-(4-benzhydrylpiperazin-1-yl)-3,3-difluoropiperidin-1-yl)methyl)-1-oxoisoindolin-2-yl)dihydropyrimidine-2,4(1H,3H)-dione;
5-((4-(4-benzhydrylpiperazin-1-yl)-3,3-difluoropiperidin-1-yl)methyl)-2-(2,4-dioxotetrahydropyrimidin-1(2H)-yl)isoindoline-1,3-dione;
1-(5-((3,3-difluoro-4-(4-(phenyl(pyridin-2-yl)methyl)piperazin-1-yl)piperidin-1-yl)methyl)-1-oxoisoindolin-2-yl)dihydropyrimidine-2,4(1H,3H)-dione;
5-((3,3-difluoro-4-(4-(phenyl(pyridin-2-yl)methyl)piperazin-1-yl)piperidin-1-yl)methyl)-2-(2,4-dioxotetrahydropyrimidin-1(2H)-yl)isoindoline-1,3-dione;
1-(5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-1-oxoisoindolin-2-yl)dihydropyrimidine-2,4(1H,3H)-dione;
5-((4-(4-(4'-chloro-5,5-dimethyl-3,4,5,6-tetrahydro-[1,1'-biphenyl]-2-carbonyl)piperazin-1-yl)-3,3-difluoropiperidin-1-yl)methyl)-2-(2,4-dioxotetrahydropyrimidin-1(2H)-yl)isoindoline-1,3-dione;
1-(5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-1-oxoisoindolin-2-yl)dihydropyrimidine-2,4(1H,3H)-dione;
5-((4-(4-((4'-chloro-5,5-dimethyl-3,4,5,6-tetrahydro-[1,1'-biphenyl]-2-yl)methyl)piperazin-1-yl)-3,3-difluoropiperidin-1-yl)methyl)-2-(2,4-dioxotetrahydropyrimidin-1(2H)-yl)isoindoline-1,3-dione;
1-(5-((4-(4-(2,3-dichlorophenyl)piperazin-1-yl)piperidin-1-yl)methyl)-1-oxoisoindolin-2-yl)dihydropyrimidine-2,4(1H,3H)-dione;
5-((4-(4-(2,3-dichlorophenyl)piperazin-1-yl)piperidin-1-yl)methyl)-2-(2,4-dioxotetrahydropyrimidin-1(2H)-yl)isoindoline-1,3-dione;
1-(5-((4-(4-(2,3-dichlorophenyl)piperazin-1-yl)-3,3-difluoropiperidin-1-yl)methyl)-1-oxoisoindolin-2-yl)dihydropyrimidine-2,4(1H,3H)-dione;
5-((4-(4-(2,3-dichlorophenyl)piperazin-1-yl)-3,3-difluoropiperidin-1-yl)methyl)-2-(2,4-dioxotetrahydropyrimidin-1(2H)-yl)isoindoline-1,3-dione;
1-(5-((4-(4-(2,3-dichlorophenyl)-3,6-dihydropyridin-1(2H)-yl)-3,3-difluoropiperidin-1-yl)methyl)-1-oxoisoindolin-2-yl)dihydropyrimidine-2,4(1H,3H)-dione;
5-((4-(4-(2,3-dichlorophenyl)-3,6-dihydropyridin-1(2H)-yl)-3,3-difluoropiperidin-1-yl)methyl)-2-(2,4-dioxotetrahydropyrimidin-1(2H)-yl)isoindoline-1,3-dione;
1-(5-((4-(4-(6-fluorobenzo[d]isoxazol-3-yl)piperazin-1-yl)piperidin-1-yl)methyl)-1-oxoisoindolin-2-yl)dihydropyrimidine-2,4(1H,3H)-dione;
2-(2,4-dioxotetrahydropyrimidin-1(2H)-yl)-5-((4-(4-(6-fluorobenzo[d]isoxazol-3-yl)piperazin-1-yl)piperidin-1-yl)methyl)isoindoline-1,3-dione;
1-(5-((3,3-difluoro-4-(4-(6-fluorobenzo[d]isoxazol-3-yl)piperazin-1-yl)piperidin-1-yl)methyl)-1-oxoisoindolin-2-yl)dihydropyrimidine-2,4(1H,3H)-dione;
5-((3,3-difluoro-4-(4-(6-fluorobenzo[d]isoxazol-3-yl)piperazin-1-yl)piperidin-1-yl)methyl)-2-(2,4-dioxotetrahydropyrimidin-1(2H)-yl)isoindoline-1,3-dione;
1-(5-((3',3'-difluoro-4-(6-fluorobenzo[d]isoxazol-3-yl)-[1,4'-bipiperidin]-1'-yl)methyl)-1-oxoisoindolin-2-yl)dihydropyrimidine-2,4(1H,3H)-dione; and
5-((3',3'-difluoro-4-(6-fluorobenzo[d]isoxazol-3-yl)-[1,4'-bipiperidin]-1'-yl)methyl)-2-(2,4-dioxotetrahydropyrimidin-1(2H)-yl)isoindoline-1,3-dione.

8. The compound of Formula (I) or salts, enantiomers, stereoisomers, solvates, or polymorphs thereof of any one of claims 1-7, which is sulfate, hydrohalide (including hydrochloride, hydrobromide), maleate, sulfonate, citrate, lactate, lactobionate, L-tartrate, fumarate, L-malate, L-lactate, α-ketoglutarate, hippurate, D-glucuronate, D-gluconate, α-D-glucoheptonate, glycolate, mucate, L-ascorbate, orotate, picrate, glycinate, alaninate, arginate, cinnamate, laurate, pamoate, sebacate, besylate, methanesulfonate, ethanesulfonate, edisylate, formate, acetate, 2,2-dichloroacetate, trimethylacetate, propionate, valerate, palmitate, triphenylacetate, 2-ethyl-butane-1,4-dioate, iodate, nicotinate, L-pyroglutamate, L-prolinate, ferulate, 2-hydroxyethanesulfonate, nitrate, gentisate, cholate, salicylate, terephthalate, glutarate, adipate, stearate, oleate, undecylenate, camphorate, camsylate, dodecylsulfate, phosphate, thiocyanate, dihydrogen phosphate, pyrophosphate, metaphosphate, oxalate, carbonate, malonate, benzoate, mandelate, succinate, pyruvate, 4-chlorobenzenesulfonate, 1,5-naphthalenedisulfonate, 3-hydroxy-2-naphthoate, 1-hydroxy-2-naphthoate, 2-naphthalenesulfonate, glycolate, trifluoroacetate, terephthalate, or 4-methylbenzenesulfonate of the compound of Formula (I).

9. A pharmaceutical composition, comprising: the compound of Formula (I) or pharmaceutically acceptable salts thereof of any one of claims 1-8, and at least one pharmaceutically acceptable carrier or excipient.

10. The pharmaceutical composition of claim 9, further comprising a second therapeutic agent, e.g., an anticancer agent.

11. The compound of Formula (I) or pharmaceutically acceptable salts thereof of any one of claims 1-8, for use in the prevention or treatment of a disease or disorder associated with cereblon protein.

12. The compound of Formula (I) or pharmaceutically acceptable salts thereof of any one of claims 1-8, for use in the prevention or treatment of a disease or disorder selected from the group consisting of: tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, and diabetes.

13. The compound of Formula (I) or pharmaceutically acceptable salts thereof of claim 11 or 12, wherein the disease or disorder is selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myeloid leukemia (CML), chronic myelogenous leukemia, acute lymphoblastic leukemia (ALL), chronic lymphocytic leukemia (CLL), B-cell chronic lymphocytic leukemia, leukemia-associated anemia, acute myeloid leukemia (AML), acute lymphoblastic leukemia (including acute B-lymphoblastic leukemia, T-lymphoblastic leukemia, acute T-lymphoblastic leukemia, chronic lymphocytic leukemia, lymphoma cell leukemia, monocytic leukemia, and myelomonocytic leukemia); lymphoma, including diffuse large B-cell lymphoma, non-Hodgkin's lymphoma, Hodgkin's lymphoma, anaplastic lymphoma, anaplastic large cell lymphoma, immunoblastic T-Cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; Burkitt's lymphoma (Burkitt's lymphoma); thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, psoriatic arthritis, systemic lupus erythematosus, multiple sclerosis, recurrent oral ulcers, Kawasaki disease, polymyositis/dermatomyositis, Sjogren's syndrome, atopic dermatitis, hidradenitis suppurativa, gout, type I diabetes, urticaria, inflammatory bowel disease (including Crohn's disease and ulcerative colitis); keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; and acute liver failure.

14. Use of the compound of Formula (I) or pharmaceutically acceptable salts thereof of any one of claims 1-8, or the pharmaceutical composition of claim 9 or 10 for the manufacture of a medicament for the prevention or treatment of a disease or disorder associated with cereblon protein.

15. Use of the compound of Formula (I) or pharmaceutically acceptable salts thereof of any one of claims 1-8, or the pharmaceutical composition of claim 9 or 10 for the manufacture of a medicament for the prevention or treatment of a disease or disorder selected from the group consisting of: tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, and diabetes.

16. A method for treating or preventing a disease or disorder associated with cereblon protein in a subject, comprising administering to the subject a therapeutically effective amount of the compound of Formula (I) or pharmaceutically acceptable salts thereof of any one of claims 1-8, or the pharmaceutical composition of claim 9 or 10.

17. A method for treating or preventing a disease or disorder in a subject, comprising administering to the subject a therapeutically effective amount of the compound of Formula (I) or pharmaceutically acceptable salts thereof of any one of claims 1-8, or the pharmaceutical composition of claim 9 or 10, wherein the disease or disorder is selected from the group consisting of: tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, and diabetes.

18. The use of claim 14 or 15, or the method of claim 16 or 17, wherein the disease or disorder is selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myeloid leukemia (CML), chronic myelogenous leukemia, acute lymphoblastic leukemia (ALL), chronic lymphocytic leukemia (CLL), B-cell chronic lymphocytic leukemia, leukemia-associated anemia, acute B-lymphoblastic leukemia, T-lymphoblastic leukemia, acute T-lymphoblastic leukemia, lymphoma cell leukemia, monocytic leukemia, myelomonocytic leukemia; lymphoma, including diffuse large B-cell lymphoma, non-Hodgkin's lymphoma, Hodgkin's lymphoma, anaplastic lymphoma, anaplastic large cell lymphoma, immunoblastic T-Cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; Burkitt's lymphoma (Burkitt's lymphoma); thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, psoriatic arthritis, systemic lupus erythematosus, multiple sclerosis, recurrent oral ulcers, Kawasaki disease, polymyositis/dermatomyositis, Sjogren's syndrome, atopic dermatitis, hidradenitis suppurativa, gout, type I diabetes, urticaria, inflammatory bowel disease (including Crohn's disease and ulcerative colitis); keratoconjunctivitis; inflammatory diseases, including pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; tuberculosis; bacterial meningitis; chronic obstructive pulmonary disease; asthma; hemorrhagic shock; organ (including kidney, heart, lung) or tissue transplantation rejection; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; and acute liver failure.

19. A method for preparing the compound of Formula (I) of claim 1, comprising reacting a compound of Formula (M1) with a compound of Formula (M2) to obtain the compound of Formula (I): 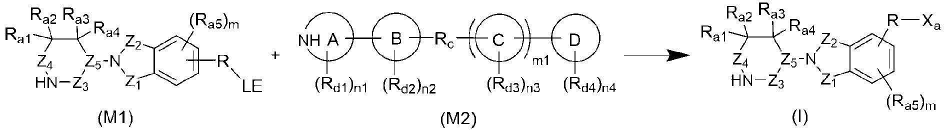
wherein Rₐ₁, Rₐ₂, Rₐ₃, Rₐ₄, (Rₐ₅)ₘ, Z₁, Z₂, Z₃, Z₄, Z₅, (R_{d1})ₙ₁, ring B, (R_{d2})ₙ₂, R_{c}, ring C, m1, (R_{d3})ₙ₃, ring D, and (R_{d4})ₙ₄ are as defined in claim 1;
nitrogen-containing heterocycle A represents a 4- to 30-membered nitrogen-containing heterocycle (preferably, a 4- to 20-membered nitrogen-containing heterocycle; more preferably, a 4-to 15-membered nitrogen-containing heterocycle);
R represents optionally substituted linear or branched C₁₋₅ alkylene;
LE represents Cl, Br, I, methanesulfonyloxy, p-toluenesulfonyloxy, o-nitrobenzenesulfonyl, C(O)Cl, or COOH; and
Xₐ of the compound of Formula (I) correspondingly represents a structure represented by the following formula: 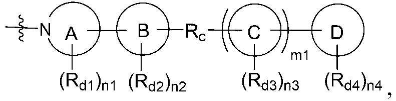
wherein nitrogen-containing heterocyclylene A is a divalent group obtained by removing a hydrogen atom from the nitrogen atom of said nitrogen-containing heterocycle A, and said nitrogen-containing heterocyclylene A represents a 4- to 30-membered nitrogen-containing heterocyclylene (preferably a 4- to 20-membered nitrogen-containing heterocyclylene; more preferably a 4- to 15-membered nitrogen-containing heterocyclylene), and
(R_{d1})ₙ₁, ring B, (Ra₂)ₙ₂, R_{c}, ring C, m1, (R_{d3})ₙ₃, ring D, and (R_{d4})ₙ₄ are as defined in any one of claims 1-8.

20. The method of claim 19, wherein (1) when the group LE represents methanesulfonyloxy, the compound of Formula (M1) is prepared by reacting a compound of Formula (M3) with methanesulfonic anhydride or methanesulfonyl chloride: 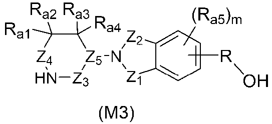
wherein Rₐ₁, Rₐ₂, Rₐ₃, Rₐ₄, (Rₐ₅)ₘ, Z₁, Z₂, Z₃, Z₄, Z₅, and R are as defined in claim 19; or
wherein (2) when the group LE represents Cl, Br, or I, the compound of Formula (M1) is prepared by subjecting the compound of Formula (M3) to a halogenation reaction with a hydrohalic acid.

21. The method of claim 20 or 21, wherein R represents CH₂ in each of the compound of Formula (M1), the compound of Formula (I), and the compound of Formula (M3).

22. The method of claim 21, wherein the compound of Formula (M3) is prepared by subjecting a compound of Formula (M4) to a coupling reaction with (tributylstannyl)methanol in the presence of a palladium catalyst: 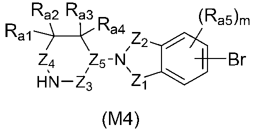 wherein Rₐ₁, Rₐ₂, Rₐ₃, Rₐ₄, (Rₐ₅)ₘ, Z₁, Z₂, Z₃, Z₄, and Z₅ are as defined in claim 19.

23. The method of claim 22, wherein when Z₅ in the compound of Formula (M4) represents N, and Z₁ represents C(O), CH₂, or CD₂, and Z₂, Z₃, and Z₄ each independently represent C(O), the compound of Formula (M4) is prepared by subjecting a compound of Formula (M5) and a compound of Formula (M6) to an amine-ester exchange reaction: 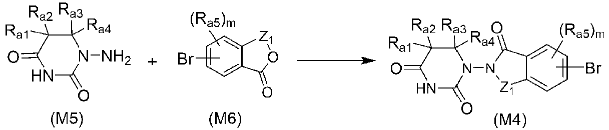 wherein Rₐ₁, Rₐ₂, Rₐ₃, Rₐ₄, and (Rₐ₅)ₘ are as defined in claim 19.

24. The method of claim 22, wherein when Z₁ in the compound of Formula (M4) represents C(O), C(S), CH₂, or CD₂; Z₂, Z₃, and Z₄ each independently represent C(O) or C(S); and at least one of Z₁, Z₂, Z₃, and Z₄ represents C(S), the compound of Formula (M4) is prepared by subjecting a compound of Formula (M7) to a thionation reaction with a thionating reagent: 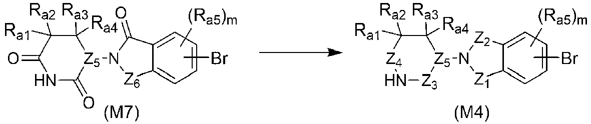
wherein Z₆ in the compound of Formula (M7) represents C(O), CH₂, or CD₂; and
Rₐ₁, Rₐ₂, Rₐ₃, Rₐ₄, and (Rₐ₅)ₘ are as defined in claim 19.

25. The method of claim 24, wherein the thionating reagent comprises carbon disulfide, hexamethyldisilathiane, sulfur, thiourea, hydrogen sulfide, phosphorus pentasulfide, Lawesson's reagent, Belleau's reagent, and Davy's reagent.
